# EUROPEAN PATENT SPECIFICATION

(11) **EP 2 750 678 B1**
(45) Date of publication and mention of the grant of the patent: **22.03.2017**
(21) Application number: 12826819.0
(22) Date of filing: 29.08.2012
(51) Int. Cl.: A61K 31/473, C07D 221/14, C07D 221/16, C07D 471/04, C07D 491/04, C07D 471/14, C07D 495/04

(54) **ANTIBACTERIAL COMPOUNDS AND METHODS FOR USE**
ANTIBAKTERIELLE VERBINDUNGEN UND VERWENDUNGSVERFAHREN DAFÜR
COMPOSÉS ANTIBACTÉRIENS ET PROCÉDÉS POUR LEUR UTILISATION

(30) Priority: 29.08.2011 US 201161528609 P
(43) Date of publication of application: 09.07.2014
(73) Proprietor: PTC Therapeutics, Inc., South Plainfield, NJ 07080 (US)
(72) Inventor: BRANSTROM, Arthur, East Windsor, NJ 08520 (US); JOSYULA, Vara Prasad, Venkata Nagendra, Superior Township, MI 48198 (US); ARNOLD, Michael, Andrew, Flemington, NJ 08822 (US); GERASYUTO, Aleksey, I., Piscataway, NJ 08854 (US); KARP, Gary, Princeton Junction, NJ 08550 (US); WANG, Jiashi, Monmouth Junction, NJ 08852 (US); CHEN, Guangming, Bridgewater, NJ 08807 (US); GINZBURG, Olya, Bloomfield, NJ 07003 (US); HUANG, Song, San Leandro, CA 94579 (US); PEDDI, Srinivasa, Piscataway, NJ 08854 (US); WOLL, Matthew,G., Dunellen, NJ 08812 (US); SMITH, Sean Wesley, Hamilton, NJ 08619 (US); ZHANG, Xiaoyan, Belle Mead, NJ 08502 (US); ZHANG, Nanjing, Princeton, NJ 08540 (US); TURPOFF, Antony Allan, Hillsborough, NJ 08844 (US); NARASIMHAN, Jana, Scotch Plains, NJ 07076 (US)
(74) Representative: Nederlandsch Octrooibureau
(86) International application number: PCT/US2012/052882
(87) International publication number: WO 2013/033228

(56) References cited:
- US-A- 4 474 786
- US-A1- 2007 249 583
- US-A1- 2008 312 292
- US-A1- 2008 312 292
- US-A1- 2010 069 380
- US-A1- 2010 261 706
- FOSSA, PAOLA ET AL: "Synthesis and pharmacological characterization of functionalized 2-pyridones structurally related to the cardiotonic agent milrinone", BIOORGANIC & MEDICINAL CHEMISTRY CODEN: BMECEP, vol. 11, no. 22, 2003, pages 4749-4759, XP002733994, ISSN: 0968-0896, DOI: 10.1016/S0968-0896(03)00528-5
- BONDAVALLI, FRANCESCO ET AL: "An efficient synthesis of functionalized 2-pyridones by direct route or via amide/enolate ammonium salt intermediates", SYNTHESIS CODEN: SYNTBF, no. 7, 1999, pages 1169-1174, XP009078054, ISSN: 0039-7881, DOI: 10.1055/S-1999-3531
- IVANOV, I. ET AL: "A facile synthesis of [1]benzopyrano[4,3-b]pyridin-5-ones", LIEBIGS ANNALEN DER CHEMIE CODEN: LACHDL, no. 3, 1992, pages 203-207, XP002733996, ISSN: 0170-2041
- POTTS, KEVIN T. ET AL: "Carbon-carbon bond formation via intramolecular cycloadditions: use of the thiocarbonyl ylide dipole in anhydro-4-hydroxythiazolium hydroxides", JOURNAL OF ORGANIC CHEMISTRY CODEN: JOCEAH, vol. 54, no. 5, 1989, pages 1077-1088, XP002733997, ISSN: 0022-3263, DOI: 10.1021/JO00266A016
- SCHMIDT, HANS WERNER ET AL: "Syntheses with nitriles. LXVII. (Ethoxymethylene)malononitrile as a synthon for the preparation of fused heterocyclic pyrano and pyrido systems", LIEBIGS ANNALEN DER CHEMIE CODEN: LACHDL, no. 4, 1983, pages 695-704, XP002733998, ISSN: 0170-2041
- ISMAIL, MOSTAFA M. ET AL: "Quinolones substituted by different moieties. Part 2. Reactions of 1,2-dihydro-4-hydroxy-1-methyl-2-oxoquinol ine-3-carbaldehyde with acyclic active methylene compounds", JOURNAL OF THE SERBIAN CHEMICAL SOCIETY CODEN: JSCSEN, vol. 62, no. 10, 1997, pages 977-986, XP009181783, ISSN: 0352-5139
- UKAWA, KIYOSHI ET AL: "Synthesis of 5-oxo-5H-[1]benzopyrano[4,3-b]pyridine derivatives", HETEROCYCLES CODEN: HTCYAM, vol. 24, no. 7, 1986, pages 1931-1941, XP009181781, ISSN: 0385-5414
- VAIDYA, V. P. ET AL: "Synthetic studies in benzofurans: Part XI - Synthesis of some benzofuro[3,2-b]pyridine derivatives", INDIAN JOURNAL OF CHEMISTRY, SECTION B: ORGANIC CHEMISTRY INCLUDING MEDICINAL CHEMISTRY CODEN: IJSBDB, vol. 20B, no. 5, 1981, pages 391-393, XP009181780, ISSN: 0376-4699
- VELEZHEVA, V. S. ET AL: "New synthesis of .delta.-carbolines. Reaction of 3-indolyl isocyanates with CH-acids", ZHURNAL ORGANICHESKOI KHIMII CODEN: ZORKAE, vol. 14, no. 8, 1978, pages 1712-1723, XP009181791, ISSN: 0514-7492
- WALSER, ARMIN ET AL: "2-Benzazepines. 10. New d-fused 2-benzazepines", JOURNAL OF HETEROCYCLIC CHEMISTRY CODEN: JHTCAD, vol. 26, no. 5, 1989, pages 1299-1304, XP002733999, ISSN: 0022-152X, DOI: 10.1002/JHET.5570260516
- RAMESH, D. ET AL: "Synthesis of novel naphtho[2,1-b]furo[3,2-b]pyridine derivatives as potential antimicrobial agents", INDIAN JOURNAL OF CHEMISTRY, SECTION B: ORGANIC CHEMISTRY INCLUDING MEDICINAL CHEMISTRY CODEN: IJSBDB, vol. 47B, no. 5, 2008, pages 753-758, XP009181774, ISSN: 0376-4699

## Description

### FIELD OF THE INVENTION

The present description relates to compounds and forms and pharmaceutical compositions thereof and methods of using such compounds, forms or compositions thereof for treating or ameliorating a bacterial infection. More particularly, the present description relates to compounds and forms and pharmaceutical compositions thereof and compounds for use in methods of using such compounds, forms or compositions thereof for treating or ameliorating a bacterial infection, or for treating or ameliorating a multi-drug resistant (MDR) bacterial infection.

### BACKGROUND OF THE INVENTION

Currently marketed antimicrobial agents inhibit bacterial DNA synthesis by acting on the two key enzymes of DNA gyrase and topoisomerase IV (see, Mitscher, L.A. Bacterial topoisomerase inhibitors: quinolone and pyridone antibacterial agents, Chem. Rev. 2005, 105, 559-592; Hooper, D. C.; Rubinstein, E. Quinolone antimicrobial agents / edited by David C. Hooper and Ethan Rubinstein; or De Souza, M. V. New fluoroquinolones: a class of potent antibiotics. Mini. Rev. Med. Chem. 2005, 5 (11), 1009-1017).

The DNA gyrase and topoisomerase IV enzymes are both type II topoisomerases, consisting of two protein subunits active as heterodimers (A₂B₂). The ATPase domain resides on one polypeptide (GyrB in DNA gyrase, ParE in topoisomerase IV), while the DNA cleavage core lies on a second polypeptide (GyrA in DNA gyrase, ParC in topoisomerase IV). Current therapies, including the aminocoumarin novobiocin, function as competitive inhibitors of energy transduction of DNA gyrase by binding to the ATPase active site in GyrB (see, Maxwell, A. The interaction between coumarin drugs and DNA gyrase. Mol. Microbiol. 1993, 9 (4), 681-686; Flatman, R. H.; Eustaquio, A.; Li, S. M.; Heide, L.; Maxwell, A. Structure-activity relationships of aminocoumarin-type gyrase and topoisomerase IV inhibitors obtained by combinatorial biosynthesis. Antimicrob. Agents Chemother. 2006, 50 (4), 1136-1142).

In contrast, the nalidixic acid, ciprofloxacin and moxifloxacin preferentially bind these enzymes at the cleavage core (GyrA and ParC) and prevent decatenation of replicating DNA (see, Hooper, D. C. Quinolone mode of action. Drugs 1995, 49 Suppl 2, 10-15). Although first site resistance mutations generally occur in *gyr*A, mutations in *gyr*B also have been shown to reduce susceptibility to quinolones (see, Yoshida, H.; Bogaki, M.; Nakamura, M.; Yamanaka, L. M.; Nakamura, S. Quinolone resistance-determining region in the DNA gyrase gyrB gene of Escherichia coli. Antimicrob. Agents Chemother. 1991, 35 (8), 1647-1650).

Bacterial DNA synthesis inhibitors (e.g., fluoroquinolones) have been used to treat primarily Gram-negative infections and have historically achieved outstanding clinical outcomes (see, Emmerson, A.M.; Jones, A.M. The quinolones: decades of development and use. J. Antimicrobial Chemotherapy, 2003, 51 (S1), 13-20). A wealth of knowledge exists for the quinolone class of compounds (see, Hooper, D. C.; Rubinstein, E. Quinolone antimicrobial agents / edited by David C. Hooper and Ethan Rubinstein), including bioavailability, tissue distribution, PK/PD relationships and photoxicity. Structurally, quinolone antibiotics possess a bicyclic (ciprofloxacin and moxifloxacin) or tricyclic ring structure (levofloxacin) with an aryl side chain containing an acyclic ring incorporating an amine functionality.

Other ring structures such as the 2-pyridones (monocyclic and bicyclic)(see, Chu, D. T. Recent progress in novel macrolides, quinolones, and 2-pyridones to overcome bacterial resistance. Med. Res. Rev. 1999, 19 (6), 497-520), quinazolinediones (see, Ellsworth, E. L.; Tran, T. P.; Showalter, H. D.; Sanchez, J. P.; Watson, B. M.; Stier, M. A.; Domagala, J. M.; Gracheck, S. J.; Joannides, E. T.; Shapiro, M. A.; Dunham, S. A.; Hanna, D. L.; Huband, M. D.; Gage, J. W.; Bronstein, J. C.; Liu, J. Y.; Nguyen, D. Q.; Singh, R. 3-aminoquinazolinediones as a new class of antibacterial agents demonstrating excellent antibacterial activity against wild-type and multidrug resistant organisms. J. Med. Chem. 2006, 49 (22), 6435-6438; and, Tran, T. P.; Ellsworth, E. L.; Stier, M. A.; Domagala, J. M.; Hollis Showalter, H. D.; Gracheck, S. J.; Shapiro, M. A.; Joannides, T. E.; Singh, R. Synthesis and structural-activity relationships of 3-hydroxyquinazoline-2,4-dione antibacterial agents. Bioorg. Med. Chem. Lett. 2004, 14 (17), 4405-4409) and tricyclic isoquinolones have been described in the literature.

Though some of these molecules, such the 2-pyridone and 4-pyridones (e.g., Ro-13-5478), isoquinolones and quinazolinediones have progressed to the late preclinical stage, none have reached the market. In the 1980s, monocyclic 2-pyridone and 4-pyridones were reported to inhibit DNA gyrase (see, Georgopapadakou, N. H.; Dix, B. A.; Angehrn, P.; Wick, A.; Olson, G. L. Monocyclic and tricyclic analogs of quinolones: mechanism of action. Antimicrob. Agents Chemother. 1987, 31 (4), 614-616).

The monocyclic 4-pyridone class of molecules generally exhibited poor activity against quinolone-resistant (quin^{R}) strains, possessed attendant CNS side effects, and in most cases, had only limited *in vivo* efficacy. Recent studies on monocyclic-4-pyridone analogs (see, Laursen, J. B.; Nielsen, J.; Haack, T.; Pusuluri, S.; David, S.; Balakrishna, R.; Zeng, Y.; Ma, Z.; Doyle, T. B.; Mitscher, L. A. Further exploration of antimicrobial ketodihydronicotinic acid derivatives by multiple parallel syntheses. Comb. Chem. High Throughout. Screen. 2006, 9 (9), 663-681) demonstrate that these compounds elicit cross-resistance to ciprofloxacin and possess poor antibacterial activity against *E. coli.*

More recently, antibacterial spiro-tricyclic barbituric acid derivatives (QPT-1) (see, Miller, A.A.; Bundy, G.L.; Mott, J.E.; Skepner, J.E.; Boyle, T.P.; Harris, D.W.; Hromockyj, A.E.; Marrotti, K.R.; Zurenko, G.E.; Munzner, J.B.; Sweeney, M.T.; Bammert, G.F.; Hamel, J.C.; Ford, C.W.; Zhong, W-Z.; Graber, D.R.; Martin, G.E.; Han, F.; Dolak, L.A.; Seest, E.P.; Ruble, J.C.; Kamilar, G.M.; Palmer, J.R.; Banitt, L.S.; Hurd, A.R.; Barbachyn, M.R. Discovery and characterization of QPT-1, the progenitor of a new class of bacterial topoisomerase inhibitors. Antimicrob. Agents Chemother. 2008, 52 (8), 2806-2812; and, Ruble, J.C.; Hurd, A.R.; Johnson, T.A.; Sherry, D.A.; Barbachyn, M.R.; Toogood, P.L.; Bundy, G.L.; Graber, D.R.; Kamilar, G.M. Synthesis of (-)-PNU-286607 by asymmetric cyclization of alkylidene barbiturates. J. Am. Chem. Soc. 2009, 131 (11), 3991-3997), inhibitors possessing a tetrahydroindazole and piperidine motif and a 6-methoxyquinoline moiety (*e.g.*, NXL101 and GSK299423) (see, Black, M. T.; Stachyra, T.; Platel, D.; Girard, A. M.; Claudon, M.; Bruneau, J. M.; Miossec, C. Mechanism of action of the antibiotic NXL101, a novel nonfluoroquinolone inhibitor of bacterial type II topoisomerases. Antimicrob. Agents Chemother. 2008, 52 (9), 3339-3349; Bax, B. D.; Chan, P. F.; Eggleston, D. S.; Fosberry, A.; Gentry, D. R.; Gorrec, F.; Giordano, I.; Hann, M. M.; Hennessy, A.; Hibbs, M.; Huang, J.; Jones, E.; Jones, J.; Brown, K. K.; Lewis, C. J.; May, E. W.; Saunders, M. R.; Singh, O.; Spitzfaden, C. E.; Shen, C.; Shillings, A.; Theobald, A. J.; Wohlkonig, A.; Pearson, N. D.; Gwynn, M. N. Type IIA topoisomerase inhibition by a new class of antibacterial agents. Nature 2010, 466 (7309), 935-940; Gomez, L.; Hack, M. D.; Wu, J.; Wiener, J. J.; Venkatesan, H.; Santillan, A., Jr.; Pippel, D. J.; Mani, N.; Morrow, B. J.; Motley, S. T.; Shaw, K. J.; Wolin, R.; Grice, C. A.; Jones, T. K. Novel pyrazole derivatives as potent inhibitors of type II topoisomerases. Part 1: synthesis and preliminary SAR analysis. Bioorg. Med. Chem. Lett. 2007, 17 (10), 2723-2727; and, Wiener, J. J.; Gomez, L.; Venkatesan, H.; Santillan, A., Jr.; Allison, B. D.; Schwarz, K. L.; Shinde, S.; Tang, L.; Hack, M. D.; Morrow, B. J.; Motley, S. T.; Goldschmidt, R. M.; Shaw, K. J.; Jones, T. K.; Grice, C. A. Tetrahydroindazole inhibitors of bacterial type II topoisomerases. Part 2: SAR development and potency against multidrug-resistant strains. Bioorg. Med. Chem. Lett. 2007, 17 (10), 2718-2722) and isothiazoloquinolones (*e.g*., ACH-702)(see, Kim, H.Y.; Wiles, J.A.; Wang, Q.; Pais, G.C.G.; Lucien, E.; Hashimoto, A.; Nelson, D.M.; Thanassi, J.A.; Podos, S.D.; Deshpande, M.; Pucci, M.J.; Bradbury, B.J. Exploration of the activity of 7-pyrrolidino-8-methoxyisothiazoloquinolones against methicillin-resistant Staphylococcus aureus (MRSA). J. Med. Chem., 2010, 54(9), 3268-3282) have been described as new classes of bacterial topoisomerase inhibitors. The X-ray crystallographic structure of GSK299423 bound to DNA gyrase has also been reported (Bax, B. D., et al., **2010**).

Structurally, most of the known inhibitors (with the exception of QPT-1, the tetrahydroindazoles, NXL101, GSK299423 and ACH-702) possess a keto-acid functionality, either a carboxylic acid (ciprofloxacin and moxifloxacin, levofloxacin, the monocyclic and bicyclic 2-pyridone and 4-pyridones), hydroxylamine (quinazolinediones and tricyclic isoquinolones), or a hydrazine (quinazolinediones) group, which relate to DNA gyrase and topoisomerase activity and presumably bind to a divalent cation in the activated complex (see, Laponogov, I.; Sohi, M. K.; Veselkov, D. A.; Pan, X. S.; Sawhney, R.; Thompson, A. W.; McAuley, K. E.; Fisher, L. M.; Sanderson, M. R. Structural insight into the quinolone-DNA cleavage complex of type IIA topoisomerases. Nat. Struct. Mol. Biol. 2009, 16 (6), 667-669).

Most inhibitors also possess an amine functional group attached to the core heterocycle, making these compounds zwitterionic in nature. Monocyclic 2-pyridone and 4-pyridone (*e.g*., Ro-13-5478) inhibitors possess this amine functionality attached to a phenyl group (see, Tesfaye, B.; Heck, J.V.; Thorsett, E.D. European Patent Application 0308022 A2, 1987**;** Narita, H.; Konishi, Y.; Nitta, J.; Misumi, S.; Nagaki, H.; Kitayama, I.; Nagai, Y.; Watanbe, Y.; Matsubare, N.; Minami, S.; Saikawa, I.; UK Patent Application GB2130580, 1983**;** and, Narita, H.; Konishi, Y.; Nitta, J.; Misumi, S.; Nagaki, H.; Kitayama, I.; Nagai, Y.; Watanbe, Y.; Matsubare, N.; Minami, S.; Saikawa, I. US Patent 4,698,352; 1987).

The zwitterionic nature of these inhibitors relate to the permeation of these compounds into the Gram-negative cell using porin channels (see, Nikaido, H.; Thanassi, D. G. Penetration of lipophilic agents with multiple protonation sites into bacterial cells: tetracyclines and fluoroquinolones as examples. Antimicrob. Agents Chemother. 1993, 37 (7), 1393-1399; and, Tieleman, D. P.; Berendsen, H. J. A molecular dynamics study of the pores formed by Escherichia coli OmpF porin in a fully hydrated palmitoyloleoylphosphatidylcholine bilayer. Biophys. J. 1998, 74 (6), 2786-2801).

Due to increasing resistance of multiple bacteria to marketed antibiotics in hospital as well as in community settings, the discovery of new and especially novel antibiotics is urgently needed (see, Bonhoeffer, S.; Lipsitch, M.; Levin, B. R. Evaluating treatment protocols to prevent antibiotic resistance. Proc. Natl. Acad. Sci. U. S. A 1997, 94 (22), 12106-12111; Wang, Y. C.; Lipsitch, M. Upgrading antibiotic use within a class: tradeoff between resistance and treatment success. Proc. Natl. Acad. Sci. U. S. A 2006, 103 (25), 9655-9660; and, Payne, D. J.; Gwynn, M. N.; Holmes, D. J.; Pompliano, D. L. Drugs for bad bugs: confronting the challenges of antibacterial discovery. Nat. Rev. Drug Discov. 2007, 6 (1), 29-40).

Approximately 70% of bacterial strains causing nosocomial infections are resistant to at least one of the drugs most commonly used to treat such infections, and 25% of bacterial pneumonia cases have been shown to be resistant to penicillin (Todar, K. Todar's Online textbook of Bacteriology, htttp://www.textbookofbacteriology.net/). Recently, there has been a dramatic decrease in the number of new antibiotic approvals, where only two new entities have been approved in the past two years.

There are some antibiotics available that have had success against MRSA (see, Perry, C. M.; Jarvis, B. Linezolid: a review of their use in the management of serious Gram-positive infections. Drugs 2001, 61 (4), 525-551; Peterson, L. R. A review of tigecycline--the first glycylcycline. Int. J. Antimicrob. Agents 2008, 32 Suppl 4, S215-S222; Chu, D. T. Recent developments in macrolides and ketolides. Curr. Opin. Microbiol. 1999, 2 (5), 467-474; Kahne, D.; Leimkuhler, C.; Lu, W.; Walsh, C. Glycopeptide and lipoglycopeptide antibiotics. Chem. Rev. 2005, 105 (2), 425-448; and, Zhanel, G. G.; Lam, A.; Schweizer, F.; Thomson, K.; Walkty, A.; Rubinstein, E.; Gin, A. S.; Hoban, D. J.; Noreddin, A. M.; Karlowsky, J. A. Ceftobiprole: a review of a broad-spectrum and anti-MRSA cephalosporin. Am. J. Clin. Dermatol. 2008, 9 (4), 245-254), but there have been no new clinically approved agents targeting Gram-negative bacteria.

Quinolones have been shown to be highly effective in the clinic, but wide-scale deployment of these current drugs, partly due to generic usage of the effective second generation quinolones (*e.g*., ciprofloxacin), jeopardizes their future long-term utility. Quinolone resistance is already rising in both hospitals and the community at large. Therefore, new drugs targeting MDR Gram-negative pathogens would be expected to help address this important unmet medical need (see, Talbot, G. H.; Bradley, J.; Edwards, J. E., Jr.; Gilbert, D.; Scheld, M.; Bartlett, J. G. Bad bugs need drugs: an update on the development pipeline from the Antimicrobial Availability Task Force of the Infectious Diseases Society of America. Clin. Infect. Dis. 2006, 42 (5), 657-668; and, Rice, L. B. Unmet medical needs in antibacterial therapy. Biochem. Pharmacol. 2006, 71 (7), 991-995).

As resistance to marketed antibiotics continues to increase, and new antibacterials have not been readily forthcoming from the pharmaceutical industry, the availability of new antibiotic and antibacterials agents is essential to overcome pre-existing and burgeoning resistance. As an effective monotherapy, novel compounds active against MDR strains of *E. coli* and *A. baumannii* pathogens, as well as other bacterial strains of great interest are needed, including those potentially employable as bioterror agents. New compounds that bind differently than existing DNA synthesis inhibitors and new therapies with combinations of antibacterial and antibiotic agents having additive or synergistic activities, including combinations with current quinolone antibiotics, would enable longer clinical lifetimes for proven antibacterial agents against a mechanistically validated target. Accordingly, the availability of such compounds and therapies would provide a significant current and future human health benefit with a high probability of success on several fronts for the control of difficult bacterial infections for a number of years to come.

6-methoxyquinoline based compounds for use as antibacterial topoisomerase inhibitors possessing Gram-negative activities have been reported by Glaxo-SmithKline, Johnson & Johnson and Novexel. Achillion and Rib-x Pharmaceuticals have also reported isothiazoloquinolones and quinolone (delafloxacin), respectively, that possess activity against resistant Gram-positive strains, including MRSA. Other examples in the literature include AM-1954 (Kyorin), DC-159a and DX-619 (Diaiichi), JNJ-Q2 (Johnson & Johnson), WQ-3813 (Wakunaga). However, all these compounds are derived from a quinolone moiety. Pfizer, Astra Zeneca, Achaogen and Targanta further describe quinolone-based compounds that possess an expanded spectrum of activity, especially against Gram-positive strains. Recently, the literature from 2005 to 2010 has been surveyed for new quinolone antibiotics (see, Wiles, J.A.; Bradbury, B.J.; Pucci, M.J. New quinolone antibiotics: a survey of the literature from 2005 to 2010. Expect Opin. Ther. Patents, 2010, 20(10), 1295-1319), including the development of compounds by AstraZeneca, Vertex Pharmaceuticals and Pfizer that act on the gyrase B sub-unit of the enzyme.

Despite the availability of quinolone based agents, the pre-existing and burgeoning resistance to such agents requires the availability of new antibiotic and antibacterials agents. However, the high conservation of sequence identity between DNA gyrase and topoisomerase IV enzymes continues to provide an opportunity for the discovery and development of non-quinolone inhibitors possessing a broad spectrum of activity against these targets. The present description relates to compounds having activity toward wild-type and MDR bacteria. The present description also relates to compounds having activity against quinolone-resistant Gram-negative strains (including MDR strains) as well as antibacterial activity to MDR resistant Gram-positive pathogens (including MRSA strains). The present description also relates to compounds with selectivity between bacterial topoisomerase IV and DNA gyrase enzyme inhibition compared to human topoisomerase II enzyme inhibition. The present description further relates to compounds that may be combined with known antibacterial agents to provide additive or synergistic activity, thus enabling the development of a combination product for the treatment of Gram-negative (especially MDR strains) and Gram-positive infections.

### SUMMARY OF THE INVENTION

The present description relates to a compound of Formula (I), Formula (II) or Formula (III): 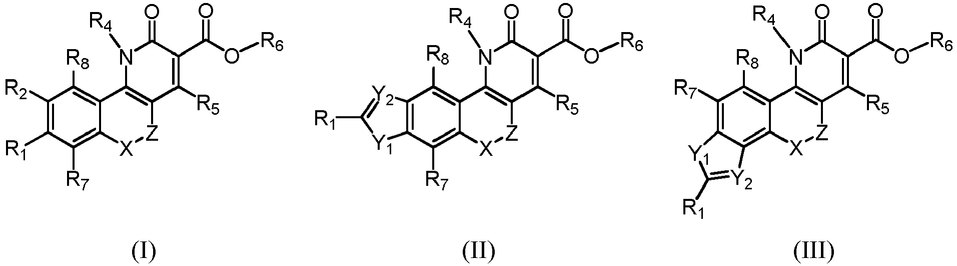 wherein R₁, R₂, R₄, R₅, R₆, R₇, R₈, Y₁, Y₂, X and Z are as defined herein, and forms and compositions thereof, and also relates to uses of a compound of Formula (I), Formula (II) or Formula (III) and methods of treating or ameliorating a bacterial infection, or for treating or ameliorating a multi-drug resistant (MDR) bacterial infection.

The present description further relates to a compound of Formula (I) having activity toward wild-type and MDR bacteria. The present description also relates to a compound of Formula (I), Formula (II) or Formula (III) having activity against quinolone-resistant Gram-negative strains (including MDR strains) as well as antibacterial activity to MDR resistant Gram-positive pathogens (including MRSA strains). The present description also relates to a compound of Formula (I), Formula (II) or Formula (III) having selectivity between bacterial topoisomerase IV and DNA gyrase enzyme inhibition compared to human topoisomerase II enzyme inhibition. The present description further relates to a compound of Formula (I), Formula (II) or Formula (III) that may be combined with known antibacterial agents to provide additive or synergistic activity, thus enabling the development of a combination product for the treatment of Gram-negative (especially MDR strains) and Gram-positive infections.

### DETAILED DESCRIPTION

The present description relates to a compound of Formula (I), Formula (II) or Formula (III): 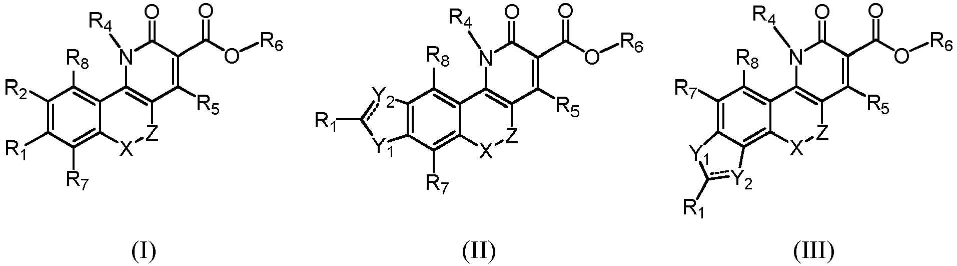 or a form thereof, wherein the form of the compound is selected from a free acid, free base, salt, hydrate, solvate, clathrate, isotopologue, racemate, enantiomer, diastereomer, stereoisomer, polymorph or tautomer form thereof, wherein
X is a bond, N(R₁₄),S, O, -CH(R₉)-, -CH(R₉)-CH(R₁₀)-, -CH(R₉)-CH(R₁₀)-CH(R₁₁)-, -C(R₉)=C(R₁₀)-, -C(R₉)=C(R₁₀)-CH(R₁₁)-, -CH(R₉)-C(R₁₀)=C(R₁₁)-, -O-CH(R₁₀)-, -CH(R₉)-O-, -N(R₁₄)-CH(R₁₀)-, -CH(R₉)-N(R₁₄)-, -S-CH(R₁₀)-, -CH(R₉)-S-, -O-CH(R₁₀)-CH(R₁₁)-, -CH(R₉)-O-CH(R₁₁)-, -CH(R₉)-CH(R₁₀)-O-, -N(R₁₄)-CH(R₁₀)-CH(R₁₁)-, -CH(R₉)-N(R₁₄)-CH(R₁₁)-, -CH(R₉)-CH(R₁₀)-N(R₁₄)-, -S-CH(R₁₀)-CH(R₁₁)-, -CH(R₉)-S-CH(R₁₁)-, -CH(R₉)-CH(R₁₀)-S-; -O-C(O)-CH(R₁₁)-, -C(O)-O-CH(R₁₁)-, -CH(R₉)-O-C(O)-, -CH(R₉)-C(O)-O-, -N(R₁₄)-C(O)-CH(₁₁)-, -C(O)-N(R₁₄)-CH(R₁₁)-, -CH(R₉)-N(R₁₄)-C(O)-, -CH(R₉)-C(O)-N(R₁₄)-, -S-C(O)-CH(R₁₁)-, -C(O)-S-CH(R₁₁)-, -CH(R₉)-S-C(O)- or -CH(R₉)-C(O)-S-;
Y₁ is -N(R₁₂)- or -O-;
Y₂ is -C(R₁₃)-, -N(R₁₂)- or -O-; wherein the dashed line represents a double bond that is present when Y₂ is -C(R₁₃)- and absent when Y₂ is -N(R₁₂)- or -O-;
Z is N(R₁₄), S, O, C(O) or -CH(R₃)-;
R₁ is halogen, hydroxyl, oxo, cyano, nitro, C₁₋₈alkyl, hydroxyl-C₁₋₈alkyl, halo-C₁₋₈alkyl, C₁₋₈alkoxy, halo-C₁₋₈alkoxy, C₁₋₈alkyl-thio, carboxyl, C₁₋₈alkyl-carbonyl, C₁₋₈alkoxy-carbonyl, amino-carbonyl, amino, C₁₋₈alkyl-amino, (C₁₋₈alkyl)₂-amino, C₂₋₈alkenyl-amino, (C₂₋₈alkenyl)₂-amino, C₂₋₈alkynyl-amino, (C₂₋₈alkynyl)₂-amino, amino-C₁₋₈alkyl, C₁₋₁₀alkyl-amino-C₁₋₈alkyl, (C₁₋₈alkyl)₂-amino-C₁₋₈alkyl, C₂₋₈alkenyl-amino-C₁₋₈alkyl, (C₂₋₈alkenyl)₂-amino-C₁₋₈alkyl, C₂₋₈alkynyl-amino-C₁₋₈alkyl, (C₂₋₈alkynyl)₂-amino-C₁₋₈alkyl, halo-C₁₋₈alkyl-amino, (halo-C₁-salkyl)₂-amino, halo-C₁₋₈alkyl-amino-C₁₋₈alkyl, (halo-C₁₋₈alkyl)₂-amino-C₁₋₈alkyl, C₁₋₈alkoxy-C₁₋₈alkyl-amino, (C₁₋₈alkoxy-C₁₋₈alkyl,C₁₋₈alkyl)-amino, (C₁₋₈alkoxy-C₁₋₈alkyl)₂-amino, C₁₋₈alkoxy-C₁₋₈alkyl-amino-C₁₋₈alkyl, (C₁₋₈alkoxy-C₁₋₈alkyl,C₁₋₈alkyl)-amino-C₁₋₈alkyl, (C₁₋₈alkoxy-C₁₋₈alkyl)₂-amino-C₁₋₈alkyl, amino-C₁₋₈alkyl-amino, (amino-C₁₋₈alkyl,C₁₋₈alkyl)amino, C₁₋₈alkyl-amino-C₁₋₈alkyl-amino, (C₁₋₈alkyl-amino-C₁₋₈alkyl,C₁₋₈alkyl)amino, (C₁₋₈alkyl)₂-amino-C₁₋₈alkyl-amino, [(C₁₋₈alkyl)₂-amino-C₁₋₈alkyl,C₁₋₈alkyl]amino, amino-C₁₋₈alkyl-amino-C₁₋₈alkyl, (amino-C₁₋₈alkyl,C₁₋₈alkyl)amino-C₁₋₈alkyl, C₁₋₈alkyl-amino-C₁₋₈alkyl-amino-C₁₋₈alkyl, (C₁₋₈alkyl-amino-C₁₋₈alkyl,C₁₋₈alkyl)amino-C₁₋₈alkyl, (C₁₋₈alkyl)₂-amino-C₁₋₈alkyl-amino-C₁₋₈alkyl, [(C₁₋₈alkyl)₂-amino-C₁₋₈-salkyl,C₁₋₈alkyl]amino-C₁₋₈alkyl, hydroxyl-amino, hydroxyl-C₁₋₈alkyl-amino, (hydroxyl-C₁₋₈alkyl,C₁₋₈alkyl)amino, (hydroxyl-C₁₋₈alkyl)₂-amino, hydroxyl-C₁₋₈alkyl-amino-C₁₋₈alkyl, (hydroxyl-C₁₋₈alkyl,C₁₋₈alkyl)amino-C₁₋₈alkyl, hydroxyl-C₁₋₈alkyl-amino-C₁₋₈alkyl-amino, (hydroxyl-C₁₋₈alkyl-amino-C₁₋₈alkyl,C₁₋₈alkyl)amino, (hydroxyl-C₁₋₈alkyl,C₁₋₈alkyl)amino-C₁₋₈alkyl-amino, [(hydroxyl-C₁₋₈alkyl,C₁₋₈alkyl)amino-C₁₋₈alkyl,C₁₋₈alkyl]amino, (C₁₋₈alkyl-carbonyl,C₁₋₈alkyl)amino-C₁₋₈alkyl, C₁₋₈alkyl-amino-carbonyl, (C₁₋₈alkyl)₂-amino-carbonyl, (C₁₋₈alkyl)₂-amino-carbonyl-C₁₋₈alkyl-amino-C₁₋₈alkyl, C₃₋₁₄cycloalkyl, C₃₋₁₄cycloalkyl-C₁₋₈alkyl, C₃₋₁₄cycloalkyl-oxy, C₃₋₁₄cycloalkyl-C₁₋₈alkoxy, C₃₋₁₄cycloalkyl-amino, C₃₋₁₄cycloalkyl-amino-C₁₋₈alkyl, (C₃₋₁₄cycloalkyl,C₁₋₈alkyl)amino-C₁₋₈alkyl, (C₃₋₁₄cycloalkyl)₂-amino-C₁₋₈alkyl, C₃₋₁₄cycloalkyl-C₁₋₈alkyl-amino-C₁₋₈alkyl, (C₃₋₁₄cycloalkyl-C₁₋₈alkyl,C₁₋₈alkyl)amino-C₁₋₈alkyl, (C₃₋₁₄cycloalkyl-C₁₋₈alkyl)₂-amino-C₁₋₈alkyl, aryl, aryl-C₁₋₈alkyl, aryl-C₁₋₈alkoxy, aryl-amino, (aryl,C₁₋₈alkyl)amino, (aryl)₂-amino, aryl-amino-C₁₋₈alkyl, (aryl,C₁₋₈alkyl)amino-C₁₋₈alkyl, (aryl)₂-amino-C₁₋₈alkyl, aryl-C₁₋₈alkyl-amino, (aryl-C₁₋₈alkyl,C₁₋₈alkyl)amino, (aryl-C₁₋₈alkyl)₂-amino, aryl-C₁₋₈alkyl-amino-C₁₋₈alkyl, (aryl-C₁₋₈alkyl,C₁₋₈alkyl)amino-C₁₋₈alkyl, (aryl-C₁₋₈alkyl)₂-amino-C₁₋₈alkyl, heteroaryl selected from the group consisting of pyrrolyl, thiazolyl, 1H-1,2,3-triazolyl, 1H-tetrazolyl, 2H-tetrazolyl, imidazolyl, and pyridinyl, heteroaryl-C₁₋₈alkyl, heteroaryl-amino, heteroaryl-C₁₋₈alkyl-amino, (heteroaryl-C₁₋₈alkyl,C₁₋₈alkyl)amino, (heteroaryl-C₁₋₈alkyl)₂-amino, heteroaryl-C₁₋₈alkyl-amino-C₁₋₈alkyl, (heteroaryl-C₁₋₈alkyl,C₁₋₈alkyl)amino-C₁₋₈alkyl, heterocyclyl selected from the group consisting of azetidinyl, pyrrolidinyl, tetrahydrofuranyl, piperidinyl, piperazinyl, morpholinyl, 1,4-diazepanyl, 1,3-dioxolanyl, 2,5-dihydro-1H-pyrrolyl, dihydro-1H-imidazolyl, 1,4,5,6-tetrahydropyrimidiny, 1,2,3,6-tetrahydropyridinyl, tetrahydro-2H-pyranyl, indolinyl, 2,3-dihydrobenzo[d]oxazolyl, 3,4-dihydro-2H-benzo[b][1,4]oxazinyl, 3,4-dihydroisoquinolin-(1H)-yl, 1,2,3,4-tetrahydroisoquinolinyl, 1,2,3,4-tetrahydroquinoxalinyl, hexahydropyrrolo[3,4-b][1,4]oxazin-(2H)-yl, (4aR,7aS)-hexahydropyrrolo[3,4-b][1,4]oxazin-(4aH)-yl, 3,4-dihydro-2H-pyrido[3,2-b][1,4]oxazinyl, (cis)-octahydrocyclopenta[c]pyrrolyl, hexahydropyrrolo[3,4-b]pyrrol-(1H)-yl, (3aR,6aR)-hexahydropyrrolo[3,4-b]pyrrol-(1H)-yl, (3aR,6aS)-hexahydropyrrolo[3,4-c]pyrrol-(1H)-yl, 5H-pyrrolo[3,4-b]pyridin-(7H)-yl, 5,7-dihydro-6H-pyrrolo[3,4-b]pyridinyl, tetrahydro-1H-pyrrolo[3,4-b]pyridin-(2H,7H,7aH)-yl, hexahydro-1H-pyrrolo[3,4-b]pyridin-(2H)-yl, (4aR,7aR)-hexahydro-1H-pyrrolo[3,4-b]pyridin-(2H)-yl, octahydro-6H-pyrrolo[3,4-b]pyridinyl, 2,3,4,9-tetrahydro-1H-carbazolyl, 1,2,3,4-tetrahydropyrazino[1,2-a]indol 8 yl, 2,3-dihydro-1H-pyrrolo[1,2-a]indolyl, (3aR,6aR)-hexahydrocyclopenta[c]pyrrol-(1H)-yl, (3aR,4R,6aS)-hexahydrocyclopenta[c]pyrrol-(1H)-yl, (3aR,4S,6aS)-hexahydrocyclopenta[c]pyrrol-(1H)-yl, (3aR,5r,6aS)-hexahydrocyclopenta[c]pyrrol-(1H)-yl, 1,3-dihydro-2H-isoindolyl, octahydro-2H-isoindolyl, (3aS)-1,3,3a,4,5,6-hexahydro-2H-isoindolyl, (3aR,4R,7aS)-1H-isoindol-(3H,3aH,4H,5H,6H,7H,7aH)-yl, (3aR,7aS)-octahydro-2H-isoindolyl, (3aR,4R,7aS)-octahydro-2H-isoindolyl, (3aR,4S,7aS)-octahydro-2H-isoindolyl, 2,5-diazabicyclo[2.2.1]heptanyl, 2-azabicyclo[2.2.1]hept-5-enyl, 3-azabicyclo[3.1.0]hexanyl, (1R,5S,6s)-3-azabicyclo[3.1.0]hexanyl, (cis,cis)-3-azabicyclo[3.1.0]hexanyl, 3,6-diazabicyclo[3.1.0]hexanyl, (1S,5R,6R)-3-azabicyclo[3.2.0]heptanyl, (1S,5R,6S)-3-azabicyclo[3.2.0]heptanyl, 5-azaspiro[2.4]heptanyl, 2,6-diazaspiro[3.3]heptanyl, 2,5-diazaspiro[3.4]octanyl, 2,6-diazaspiro[3.4]octanyl, 2,7-diazaspiro[3.5]nonanyl, 2,7-diazaspiro[4.4]nonanyl, 2-azaspiro[4.5]decanyl, and 2,8-diazaspiro[4.5]decanyl, heterocyclyl-C₁₋₈alkyl, heterocyclyl-oxy, heterocyclyl-C₁₋₈alkoxy, heterocyclyl-amino, (heterocyclyl,C₁₋₈alkyl)amino, (heterocyclyl)₂-amino, heterocyclyl-amino-C₁₋₈alkyl, (heterocyclyl,C₁₋₈alkyl)amino-C₁₋₈alkyl, (heterocyclyl)₂-amino-C₁₋₈alkyl, (heterocyclyl,C₃₋₁₄cycloalkyl-C₁₋₈alkyl)amino-C₁₋₈alkyl, heterocyclyl-C₁₋₈alkyl-amino-C₁₋₈alkyl, (heterocyclyl-C₁₋₈alkyl,C₁₋₈alkyl)amino-C₁₋₈alkyl, (heterocyclyl-C₁₋₈alkyl)₂-amino-C₁₋₈alkyl, heterocyclyl-oxy-amino, (heterocyclyl-oxy,C₁₋₈alkyl)amino, (heterocyclyl-oxy)₂-amino, (heterocyclyl-oxy-C₁₋₈alkyl,C₁₋₈alkyl)amino, heterocyclyl-carbonyl or heterocyclyl-carbonyl-oxy;
wherein each instance of C₃₋₁₄cycloalkyl, aryl, heterocyclyl and heteroaryl is optionally substituted with one, two or three substituents each selected from R₁₅; and,
wherein each instance of aryl, heterocyclyl or heteroaryl is optionally substituted with one additional substituent selected from R₁₆;
R₂ is hydrogen, halogen, hydroxyl, C₁₋₈alkyl, amino, C₁₋₈alkyl-amino or (C₁₋₈alkyl)₂-amino;
R₃ is hydrogen, halogen, hydroxyl, C₁₋₈alkyl, amino, C₁₋₈alkyl-amino or (C₁₋₈alkyl)₂-amino;
R₄ is hydrogen, hydroxyl, aryl-C₁₋₈alkyl, wherein aryl is optionally substituted with one additional substituent selected from C₁₋₈alkyl, halo-C₁₋₈alkyl, C₁₋₈alkoxy or C₁₋₈alkoxy-C₁₋₈alkyl;
R₅ is hydrogen, halogen, hydroxyl, C₁₋₈alkyl, C₁₋₈alkoxy, carboxyl, amino, C₁₋₈alkyl-amino, (C₁₋₈alkyl)₂-amino or C₁₋₈alkyl-SO₂-amino;
R₆ is hydrogen or C₁₋₈alkyl;
R₇ is hydrogen, halogen, hydroxyl, C₁₋₈alkyl, amino, C₁₋₈alkyl-amino or (C₁₋₈alkyl)₂-amino;
R₈ is hydrogen, halogen, hydroxyl, C₁₋₈alkyl, amino, C₁₋₈alkyl-amino or (C₁₋₈alkyl)₂-amino;
R₉ is hydrogen, halogen, hydroxyl, C₁₋₈alkyl, amino, C₁₋₈alkyl-amino or (C₁₋₈alkyl)₂-amino;
R₁₀ is hydrogen, halogen, hydroxyl, C₁₋₈alkyl, amino, C₁₋₈alkyl-amino or (C₁₋₈alkyl)₂-amino;
R₁₁ is hydrogen, halogen, hydroxyl, C₁₋₈alkyl, amino, C₁₋₈alkyl-amino or (C₁₋₈alkyl)₂-amino;
R₁₂ is hydrogen, C₁₋₈alkyl, aryl or heteroaryl, wherein aryl and heteroaryl is optionally substituted with one additional substituent selected from C₁₋₈alkyl, halo-C₁-₈alkyl, C₁₋₈alkoxy or C₁₋₈alkoxy-C₁₋₈alkyl;
R₁₃ is hydrogen, cyano, halogen, hydroxyl or C₁₋₈alkyl;
R₁₄ is hydrogen, C₁₋₈alkyl, C₁₋₈alkyl-carbonyl, C₁₋₈alkoxy-carbonyl, aryl or heteroaryl, wherein aryl and heteroaryl is optionally substituted with one additional substituent selected from C₁₋₈alkyl, halo-C₁₋₈alkyl, C₁₋₈alkoxy or C₁₋₈alkoxy-C₁₋₈alkyl;
R₁₅ is azido, halogen, hydroxyl, oxo, cyano, nitro, C₁₋₈alkyl, halo-C₁₋₈alkyl, hydroxyl-C₁₋₈alkyl, C₁₋₈alkoxy-C₁₋₈alkyl, C₁₋₈alkoxy, halo-C₁₋₈alkoxy, hydroxyl-C₁₋₈alkoxy, carboxyl, C₁₋₈alkyl-carbonyl, C₁₋₈alkoxy-carbonyl, amino, C₁₋₈alkyl-amino, (C₁₋₈alkyl)₂-amino, halo-C₁₋₈alkyl-amino, (halo-C₁₋₈alkyl)₂-amino, halo-C₁₋₈alkyl-amino-C₁₋₈alkyl, (halo-C₁₋₈alkyl)₂-amino-C₁₋₈alkyl, amino-C₁₋₈alkyl, C₁₋₈alkyl-amino-C₁₋₈alkyl, (C₁₋₈alkyl)₂-amino-C₁₋₈alkyl, [(C₁₋₈alkyl)₂-amino-C₁₋₈alkyl,C₁₋₈alkyl]amino-C₁₋₈alkyl C₁₋₈alkyl-thio, amino-carbonyl, C₁₋₈alkyl-amino-carbonyl, (C₁₋₈alkyl)₂-amino-carbonyl, C₁₋₈alkyl-carbonyl-amino or (carboxyl-C₁₋₈alkyl,C₁₋₈alkyl)amino-carbonyl-amino;
R₁₆ is C₃₋₁₄cycloalkyl, C₃₋₁₄cycloalkyl-amino, aryl, aryl-C₁₋₈alkyl, aryl-amino, (aryl,C₁₋₈alkyl)amino, (aryl)₂-amino, aryl-C₁₋₈alkyl-amino, (aryl-C₁₋₈alkyl,C₁₋₈alkyl)amino, (aryl-C₁₋₈alkyl)₂-amino, aryl-C₁₋₈alkyl-amino-C₁₋₈alkyl, (aryl-C₁₋₈alkyl,C₁₋₈alkyl)amino-C₁₋₈alkyl, (aryl-C₁₋₈alkyl)₂-amino-C₁₋₈alkyl, aryl-amino-C₁₋₈alkyl, (aryl,C₁₋₈alkyl)amino-C₁₋₈alkyl, (aryl)₂-amino-C₁₋₈alkyl, aryl-amino-carbonyl, aryl-C₁₋₈alkoxy, aryl-C₁₋₈alkoxy-carbonyl-amino, heteroaryl, heteroaryl-C₁₋₈alkyl, heteroaryl-amino, (heteroaryl)₂-amino, heteroaryl-C₁₋₈alkyl-amino, (heteroaryl-C₁₋₈alkyl,C₁₋₈alkyl)amino, (heteroaryl-C₁₋₈alkyl)₂-amino, heteroaryl-C₁₋₈alkyl-amino-C₁₋₈alkyl, (heteroaryl-C₁₋₈alkyl,C₁₋₈alkyl)amino-C₁₋₈alkyl, (heteroaryl-C₁₋₈alkyl)₂-amino-C₁₋₈alkyl, heterocyclyl, heterocyclyl-C₁₋₈alkyl, heterocyclyl-amino-C₁₋₈alkyl or heterocyclyl-oxy;
wherein each instance of C₃₋₁₄cycloalkyl is optionally substituted with one substituent selected from R₁₈;
wherein each instance of aryl is optionally substituted with one substituent selected from R₁₉;R₁₈ is amino, C₁₋₈alkyl-amino, (C₁₋₈alkyl)₂-amino, amino-C₁₋₈alkyl, C₁₋₈alkyl-amino-C₁₋₈alkyl, (C₁₋₈alkyl)₂-amino-C₁₋₈alkyl or aryl-C₁₋₈alkyl-amino; and
R₁₉ is halogen.Disclosed are such substances wherein each instance of heterocyclyl and heteroaryl is optionally substituted with one substituent selected from R₂₀, wherein R₂₀ is halogen, hydroxyl, C₁₋₈alkyl, halo-C₁₋₈alkyl, C₁₋₈alkoxy, C₁₋₈alkoxy-C₁₋₈alkyl, halo-C₁₋₈alkoxy, amino, C₁₋₈alkyl-amino or (C₁₋₈alkyl)₂-amino.

One embodiment of the present description includes a compound of Formula (I) or a form thereof, wherein the form of the compound is selected from a free acid, free base, salt, hydrate, solvate, clathrate, isotopologue, racemate, enantiomer, diastereomer, stereoisomer, polymorph or tautomer form thereof, wherein R₁ is
C₃₋₁₄cycloalkyl selected in each instance, when present, from cyclopropyl, cyclobutyl, cyclopentyl, cyclohexyl or cycloheptyl;
aryl selected in each instance, when present, from phenyl;
heteroaryl selected in each instance, when present, from pyrrolyl, thiazolyl, 1H-1,2,3-triazolyl, 1H-tetrazolyl, 2H-tetrazolyl, imidazolyl or pyridinyl;
heterocyclyl selected in each instance, when present, from azetidinyl, pyrrolidinyl, tetrahydrofuranyl, piperidinyl, piperazinyl, morpholinyl, 1,4-diazepanyl, 1,3-dioxolanyl, 2,5-dihydro-1H-pyrrolyl, dihydro-1H-imidazolyl, 1,4,5,6-tetrahydropyrimidinyl, 1,2,3,6-tetrahydropyridinyl, tetrahydro-2H-pyranyl, indolinyl, 2,3-dihydrobenzo[d]oxazolyl, 3,4-dihydro-2H-benzo[b][1,4]oxazinyl, 3,4-dihydroisoquinolin-(1H)-yl, 1,2,3,4-tetrahydroisoquinolinyl, 1,2,3,4-tetrahydroquinoxalinyl, hexahydropyrrolo[3,4-b][1,4]oxazin-(2H)-yl, (4aR,7aS)-hexahydropyrrolo[3,4-b][1,4]oxazin-(4aH)-yl, 3,4-dihydro-2H-pyrido[3,2-b][1,4]oxazinyl, (cis)-octahydrocyclopenta[c]pyrrolyl, hexahydropyrrolo[3,4-b]pyrrol-(1H)-yl, (3aR,6aR)-hexahydropyrrolo[3,4-b]pyrrol-(1H)-yl, (3aR,6aS)-hexahydropyrrolo[3,4-c]pyrrol-(1H)-yl, 5H-pyrrolo[3,4-b]pyridin-(7H)-yl, 5,7-dihydro-6H-pyrrolo[3,4-b]pyridinyl, tetrahydro-1H-pyrrolo[3,4-b]pyridin-(2H,7H,7aH)-yl, hexahydro-1H-pyrrolo[3,4-b]pyridin-(2H)-yl, (4aR,7aR)-hexahydro-1H-pyrrolo[3,4-b]pyridin-(2H)-yl, octahydro-6H-pyrrolo[3,4-b]pyridinyl, 2,3,4,9-tetrahydro-1H-carbazolyl, 1,2,3,4-tetrahydropyrazino[1,2-a]indolyl, 2,3-dihydro-1H-pyrrolo[1,2-a]indolyl, (3aR,6aR)-hexahydrocyclopenta[c]pyrrol-(1H)-yl, (3aR,4R,6aS)-hexahydrocyclopenta[c]pyrrol-(1H)-yl, (3aR,4S,6aS)-hexahydrocyclopenta[c]pyrrol-(1H)-yl, (3aR,5r,6aS)-hexahydrocyclopenta[c]pyrrol-(1H)-yl, 1,3-dihydro-2H-isoindolyl, octahydro-2H-isoindolyl, (3aS)-1,3,3a,4,5,6-hexahydro-2H-isoindolyl, (3aR,4R,7aS)-1H-isoindol-(3H,3aH,4H,5H,6H,7H,7aH)-yl, (3aR,7aS)-octahydro-2H-isoindolyl, (3aR,4R,7aS)-octahydro-2H-isoindolyl, (3aR,4S,7aS)-octahydro-2H-isoindolyl, 2,5-diazabicyclo[2.2.1]heptanyl, 2-azabicyclo[2.2.1]hept-5-enyl, 3-azabicyclo[3.1.0]hexanyl, (1R,5S,6s)-3-azabicyclo[3.1.0]hexanyl, (cis,cis)-3-azabicyclo[3.1.0]hexanyl, 3,6-diazabicyclo[3.1.0]hexanyl, (1S,5R,6R)-3-azabicyclo[3.2.0]heptanyl, (1S,5R,6S)-3-azabicyclo[3.2.0]heptanyl, 5-azaspiro[2.4]heptanyl, 2,6-diazaspiro[3.3]heptanyl, 2,5-diazaspiro[3.4]octanyl, 2,6-diazaspiro[3.4]octanyl, 2,7-diazaspiro[3.5]nonanyl, 2,7-diazaspiro[4.4]nonanyl, 2-azaspiro[4.5]decanyl or 2,8-diazaspiro[4.5]decanyl.
As part of the disclosure, R₁ can additionally be hydrogen, any heteroaryl, or any heterocyclyl, and R₄ can additionally be C₁₋₈alkyl.

One embodiment of the present description includes a compound of Formula (I) or a form thereof, wherein R₁ is
heteroaryl selected in each instance, when present, from pyrrol-1-yl, thiazol-2-yl, 1H-1,2,3-triazol-1-yl, 1H-tetrazol-5-yl, 2H-tetrazol-2-yl, imidazol-1-yl, pyridin-2-yl, pyridin-3-yl or pyridin-4-yl;
heterocyclyl selected in each instance, when present, from azetidin-1-yl, pyrrolidin-1-yl, tetrahydrofuran-2-yl, pyrrolidin-2-yl, pyrrolidin-3-yl, piperidin-1-yl, piperidin-2-yl, piperidin-3-yl, piperidin-4-yl, piperazin-1-yl, piperazin-2-yl, morpholin-4-yl, 1,4-diazepan-1-yl, 1,3-dioxolan-2-yl, 2,5-dihydro-1H-pyrrol-1-yl, dihydro-1H-imidazol-2-yl, 1,4,5,6-tetrahydropyrimidin-2-yl, 1,2,3,6-tetrahydropyridin-4-yl, tetrahydro-2H-pyran-2-yl, tetrahydro-2H-pyran-4-yl, 3,4-dihydroisoquinolin-2(1H)-yl, 1,2,3,4-tetrahydroisoquinolin-1-yl, hexahydropyrrolo[3,4-b][1,4]oxazin-6(2H)-yl, (4aR,7aS)-hexahydropyrrolo[3,4-b][1,4]oxazin-4(4aH)-yl, (cis)-octahydrocyclopenta[c]pyrrol-4-yl, hexahydropyrrolo[3,4-b]pyrrol-5(1H)-yl, (3aR,6aR)-hexahydropyrrolo[3,4-b]pyrrol-5(1H)-yl, (3aR,6aS)-hexahydropyrrolo[3,4-c]pyrrol-2(1H)-yl, 5H-pyrrolo[3,4-b]pyridin-6(7H)-yl, 5,7-dihydro-6H-pyrrolo[3,4-b]pyridin-6-yl, tetrahydro-1H-pyrrolo[3,4-b]pyridin-6(2H,7H,7aH)-yl, hexahydro-1H-pyrrolo[3,4-b]pyridin-6(2H)-yl, (4aR,7aR)-hexahydro-1H-pyrrolo[3,4-b]pyridin-6(2H)-yl, octahydro-6H-pyrrolo[3,4-b]pyridin-6-yl, (3aR,6aR)-hexahydrocyclopenta[c]pyrrol-3a(1H)-yl, (3aR,4R,6aS)-hexahydrocyclopenta[c]pyrrol-2(1H)-yl, (3aR,4S,6aS)-hexahydrocyclopenta[c]pyrrol-2(1H)-yl, (3aR,5r,6aS)-hexahydrocyclopenta[c]pyrrol-2(1H)-yl, 1,3-dihydro-2H-isoindol-2-yl, octahydro-2H-isoindol-2-yl, (3aS)-1,3,3a,4,5,6-hexahydro-2H-isoindol-2-yl, (3aR,4R,7aS)-1H-isoindol-2(3H,3aH,4H,5H,6H,7H,7aH)-yl, (3aR,7aS)-octahydro-2H-isoindol-2-yl, (3aR,4R,7aS)-octahydro-2H-isoindol-2-yl, (3aR,4S,7aS)-octahydro-2H-isoindol-2-yl, 2,5-diazabicyclo[2.2.1]heptan-2-yl, 2-azabicyclo[2.2.1]hept-5-en-2-yl, 3-azabicyclo[3.1.0]hexan-3-yl, (1R,5S,6s)-3-azabicyclo[3.1.0]hexan-3-yl, (1R,5S,6s)-3-azabicyclo[3.1.0]hexan-6-yl, (cis,cis)-3-azabicyclo[3.1.0]hexan-3-yl, 3,6-diazabicyclo[3.1.0]hexan-3-yl, (1S,5R,6R)-3-azabicyclo[3.2.0]heptan-3-yl, (1S,5R,6S)-3-azabicyclo[3.2.0]heptan-3-yl, 5-azaspiro[2.4]heptan-5-yl, 2,6-diazaspiro[3.3]heptan-2-yl, 2,5-diazaspiro[3.4]octan-2-yl, 2,6-diazaspiro[3.4]octan-6-yl, 2,7-diazaspiro[3.5]nonan-2-yl, 2,7-diazaspiro[4.4]nonan-2-yl, 2-azaspiro[4.5]decan-2-yl or 2,8-diazaspiro[4.5]decan-2-yl.

One embodiment of the present description includes a compound of Formula (I) or a form thereof, wherein R₁ is
heteroaryl selected in each instance, when present, from pyridinyl;
heterocyclyl selected in each instance, when present, from azetidinyl, pyrrolidinyl, tetrahydrofuranyl, piperidinyl, piperazinyl, morpholinyl, 1,4-diazepanyl, 1,3-dioxolanyl, dihydro-1H-imidazolyl, 1,4,5,6-tetrahydropyrimidinyl, 1,2,3,6-tetrahydropyridinyl, tetrahydro-2H-pyranyl, 3,4-dihydroisoquinolin-(1H)-yl, 1,2,3,4-tetrahydroisoquinolinyl, 5H-pyrrolo[3,4-b]pyridin-(7H)-yl, tetrahydro-1H-pyrrolo[3,4-b]pyridin-(2H,7H,7aH)-yl, (3aR,4R,6aS)-hexahydrocyclopenta[c]pyrrol-(1H)-yl, (3aR,4R,7aS)-1H-isoindol-(3H,3aH,4H,5H,6H,7H,7aH)-yl, 2,5-diazabicyclo[2.2.1]heptanyl or (1R,5S,6s)-3-azabicyclo[3.1.0]hexanyl.

One embodiment of the present description includes a compound of Formula (I) or a form thereof, wherein R₁ is
heteroaryl selected in each instance, when present, from pyridin-2-yl, pyridin-3-yl or pyridin-4-yl;
heterocyclyl selected in each instance, when present, from azetidin-1-yl, pyrrolidin-1-yl, pyrrolidin-2-yl, pyrrolidin-3-yl, tetrahydrofuran-2-yl, piperidin-1-yl, piperidin-2-yl, piperidin-3-yl, piperidin-4-yl, piperazin-1-yl, piperazin-2-yl, morpholin-4-yl, 1,4-diazepan-1-yl, 1,3-dioxolan-2-yl, dihydro-1H-imidazol-2-yl, 1,4,5,6-tetrahydropyrimidin-2-yl, 1,2,3,6-tetrahydropyridin-4-yl, tetrahydro-2H-pyran-2-yl, tetrahydro-2H-pyran-4-yl, 3,4-dihydroisoquinolin-2(1H)-yl, 1,2,3,4-tetrahydroisoquinolin-1-yl, 5H-pyrrolo[3,4-b]pyridin-6(7H)-yl, tetrahydro-1H-pyrrolo[3,4-b]pyridin-6(2H,7H,7aH)-yl, (3aR,4R,6aS)-hexahydrocyclopenta[c]pyrrol-2(1H)-yl, (3aR,4R,7aS)-1H-isoindol-2(3H,3aH,4H,5H,6H,7H,7aH)-yl, 2,5-diazabicyclo[2.2.1]heptan-2-yl, (1R,5S,6s)-3-azabicyclo[3.1.0]hexan-3-yl or (1R,5S,6s)-3-azabicyclo[3.1.0]hexan-6-yl.

One embodiment of the present description includes a compound of Formula (I) or a form thereof, wherein R₁ is
heteroaryl selected in each instance, when present, from pyrrolyl, imidazolyl, 1H-tetrazolyl or 2H-tetrazolyl;
heterocyclyl selected in each instance, when present, from azetidinyl, pyrrolidinyl, tetrahydrofuranyl, piperidinyl, piperazinyl, morpholinyl, 1,4-diazepanyl, 1,3-dioxolanyl, 2,5-dihydro-1H-pyrrolyl, dihydro-1H-imidazolyl, 1,4,5,6-tetrahydropyrimidinyl, 1,2,3,6-tetrahydropyridinyl, tetrahydro-2H-pyranyl, 3,4-dihydroisoquinolin-(1H)-yl, 1,2,3,4-tetrahydroisoquinolinyl, hexahydropyrrolo[3,4-b][1,4]oxazin-(2H)-yl, (4aR,7aS)-hexahydropyrrolo[3,4-b][1,4]oxazin-(4aH)-yl, (cis)-octahydrocyclopenta[c]pyrrolyl, hexahydropyrrolo[3,4-b]pyrrol-(1H)-yl, (3aR,6aR)-hexahydropyrrolo[3,4-b]pyrrol-(1H)-yl, (3aR,6aS)-hexahydropyrrolo[3,4-c]pyrrol-(1H)-yl, 5H-pyrrolo[3,4-b]pyridin-(7H)-yl, 5,7-dihydro-6H-pyrrolo[3,4-b]pyridinyl, tetrahydro-1H-pyrrolo[3,4-b]pyridin-(2H,7H,7aH)-yl, hexahydro-1H-pyrrolo[3,4-b]pyridin-(2H)-yl, (4aR,7aR)-hexahydro-1H-pyrrolo[3,4-b]pyridin-(2H)-yl, octahydro-6H-pyrrolo[3,4-b]pyridinyl, (3aR,6aR)-hexahydrocyclopenta[c]pyrrol-(1H)-yl, (3aR,4R,6aS)-hexahydrocyclopenta[c]pyrrol-(1H)-yl, (3aR,4S,6aS)-hexahydrocyclopenta[c]pyrrol-(1H)-yl, (3aR,5r,6aS)-hexahydrocyclopenta[c]pyrrol-(1H)-yl, 1,3-dihydro-2H-isoindolyl, octahydro-2H-isoindolyl, (3aS)-1,3,3a,4,5,6-hexahydro-2H-isoindolyl, (3aR,4R,7aS)-1H-isoindol-(3H,3aH,4H,5H,6H,7H,7aH)-yl, (3aR,7aS)-octahydro-2H-isoindolyl, (3aR,4R,7aS)-octahydro-2H-isoindolyl, (3aR,4S,7aS)-octahydro-2H-isoindolyl, 2,5-diazabicyclo[2.2.1]heptanyl, 2-azabicyclo[2.2.1]hept-5-enyl, 3-azabicyclo[3.1.0]hexanyl, 3,6-diazabicyclo[3.1.0]hexanyl, (1R,5S,6s)-3-azabicyclo[3.1.0]hexanyl, (1S,5R,6R)-3-azabicyclo[3.2.0]heptanyl, (1S,5R,6S)-3-azabicyclo[3.2.0]heptanyl, 5-azaspiro[2.4]heptanyl, 2,6-diazaspiro[3.3]heptanyl, 2,5-diazaspiro[3.4]octanyl, 2,6-diazaspiro[3.4]octanyl, 2,7-diazaspiro[3.5]nonanyl, 2,7-diazaspiro[4.4]nonanyl, 2-azaspiro[4.5]decanyl or 2,8-diazaspiro[4.5]decanyl.

One embodiment of the present description includes a compound of Formula (I) or a form thereof, wherein R₁ is
heteroaryl selected in each instance, when present, from 1H-tetrazol-5-yl, imidazol-1-yl, pyrrol-1-yl or 2H-tetrazol-2-yl;
heterocyclyl selected in each instance, when present, from azetidin-1-yl, pyrrolidin-1-yl, tetrahydrofuran-2-yl, piperidin-1-yl, piperazin-1-yl, morpholin-4-yl, 2,5-dihydro-1H-pyrrol-1-yl, hexahydropyrrolo[3,4-b][1,4]oxazin-6(2H)-yl, (4aR,7aS)-hexahydropyrrolo[3,4-b][1,4]oxazin-4(4aH)-yl, (3aR,6aR)-hexahydropyrrolo[3,4-b]pyrrol-5(1H)-yl, (3aR,6aS)-hexahydropyrrolo[3,4-c]pyrrol-2(1H)-yl, 5,7-dihydro-6H-pyrrolo[3,4-b]pyridin-6-yl, octahydro-6H-pyrrolo[3,4-b]pyridin-6-yl, (3aR,6aR)-hexahydrocyclopenta[c]pyrrol-3a(1H)-yl, (3aR,4R,6aS)-hexahydrocyclopenta[c]pyrrol-2(1H)-yl, (3aR,4S,6aS)-hexahydrocyclopenta[c]pyrrol-2(1H)-yl, (3aR,5r,6aS)-hexahydrocyclopenta[c]pyrrol-2(1H)-yl, 1,3-dihydro-2H-isoindol-2-yl, octahydro-2H-isoindol-2-yl, (3aS)-1,3,3a,4,5,6-hexahydro-2H-isoindol-2-yl, (3aR,7aS)-octahydro-2H-isoindol-2-yl, (3aR,4R,7aS)-octahydro-2H-isoindol-2-yl, (3aR,4S,7aS)-octahydro-2H-isoindol-2-yl, 2,5-diazabicyclo[2.2.1]heptan-2-yl, 2-azabicyclo[2.2.1]hept-5-en-2-yl, 3-azabicyclo[3.1.0]hexan-3-yl, 3,6-diazabicyclo[3.1.0]hexan-3-yl, (1R,5S,6s)-3-azabicyclo[3.1.0]hexan-3-yl, (1R,5S,6s)-3-azabicyclo[3.1.0]hexan-6-yl, (1S,5R,6R)-3-azabicyclo[3.2.0]heptan-3-yl, (1S,5R,6S)-3-azabicyclo[3.2.0]heptan-3-yl, 5-azaspiro[2.4]heptan-5-yl, 2,6-diazaspiro[3.3]heptan-2-yl, 2,5-diazaspiro[3.4]octan-2-yl, 2,6-diazaspiro[3.4]octan-6-yl, 2,7-diazaspiro[3.5]nonan-2-yl, 2,7-diazaspiro[4.4]nonan-2-yl, 2-azaspiro[4.5]decan-2-yl or 2,8-diazaspiro[4.5]decan-2-yl.

One embodiment of the present description includes a compound of Formula (I) or a form thereof, wherein
R₂ is hydrogen, C₁₋₈alkyl, amino, C₁₋₈alkyl-amino or (C₁₋₈alkyl)₂-amino;
R₃ is hydrogen, halogen, hydroxyl, C₁₋₈alkyl, amino, C₁₋₈alkyl-amino or (C₁₋₈alkyl)₂-amino; and,
R₄ is hydrogen or C₁₋₈alkyl.

One embodiment of the present description includes a compound of Formula (I) or a form thereof, wherein
R₂ is hydrogen;
R₃ is hydrogen or C₁₋₈alkyl; and,
R₄ is hydrogen.

One embodiment of the present description includes a compound of Formula (I) or a form thereof, wherein R₁ is
C₃₋₁₄cycloalkyl-amino-C₁₋₈alkyl, wherein C₃₋₁₄cycloalkyl is selected from cyclopropyl, cyclobutyl, cyclopentyl or cyclohexyl;
(C₃₋₁₄cycloalkyl,C₁₋₈alkyl)amino-C₁₋₈alkyl, wherein C₃₋₁₄cycloalkyl is selected from cyclopropyl, cyclobutyl or cyclopentyl;
C₃₋₁₄cycloalkyl-C₁₋₁₄alkyl-amino-C₁₋₈alkyl, wherein C₃₋₁₄cycloalkyl is selected from cyclopropyl, cyclobutyl, cyclopentyl, cyclohexyl or cycloheptyl;
aryl, wherein aryl is selected from phenyl;
aryl-amino, wherein aryl is selected from phenyl;
(aryl,C₁₋₈alkyl)amino, wherein aryl is selected from phenyl;
aryl-amino-C₁₋₈alkyl, wherein aryl is selected from phenyl;
aryl-C₁₋₈alkyl-amino-C₁₋₈alkyl, wherein aryl is selected from phenyl;
(aryl-C₁₋₈alkyl,C₁₋₈alkyl)amino-C₁₋₈alkyl, wherein aryl is selected from phenyl;
(aryl-C₁₋₈alkyl)₂-amino-C₁₋₈alkyl, wherein aryl is selected from phenyl;
heteroaryl, wherein heteroaryl is selected from pyrrolyl, thiazolyl, 1H-1,2,3-triazolyl, 1H-tetrazolyl, 2H-tetrazolyl, imidazolyl or pyridinyl;
heteroaryl-C₁₋₈alkyl-amino-C₁₋₈alkyl, wherein heteroaryl is selected from pyridin-2-yl, pyridin-3-yl or pyridin-4-yl;
(heteroaryl-C₁₋₈alkyl,C₁₋₈alkyl)amino-C₁₋₈alkyl, wherein heteroaryl is selected from pyridin-3-yl or pyridin-4-yl;
heterocyclyl, wherein heterocyclyl is selected from azetidinyl, pyrrolidinyl, tetrahydrofuranyl, piperidinyl, piperazinyl, morpholinyl, 1,4-diazepanyl, 1,3-dioxolanyl, 2,5-dihydro-1H-pyrrolyl, dihydro-1H-imidazolyl, 1,4,5,6-tetrahydropyrimidinyl, 1,2,3,6-tetrahydropyridinyl, tetrahydro-2H-pyranyl, 3,4-dihydroisoquinolin-(1H)-yl, 1,2,3,4-tetrahydroisoquinolinyl, hexahydropyrrolo[3,4-b][1,4]oxazin-(2H)-yl, (4aR,7aS)-hexahydropyrrolo[3,4-b][1,4]oxazin-(4aH)-yl, (cis)-octahydrocyclopenta[c]pyrrolyl, hexahydropyrrolo[3,4-b]pyrrol-(1H)-yl, (3aR,6aR)-hexahydropyrrolo[3,4-b]pyrrol-(1H)-yl, (3aR,6aS)-hexahydropyrrolo[3,4-c]pyrrol-(1H)-yl, 5H-pyrrolo[3,4-b]pyridin-(7H)-yl, 5,7-dihydro-6H-pyrrolo[3,4-b]pyridinyl, tetrahydro-1H-pyrrolo[3,4-b]pyridin-(2H,7H,7aH)-yl, hexahydro-1H-pyrrolo[3,4-b]pyridin-(2H)-yl, (4aR,7aR)-hexahydro-1H-pyrrolo[3,4-b]pyridin-(2H)-yl, octahydro-6H-pyrrolo[3,4-b]pyridinyl, (3aR,6aR)-hexahydrocyclopenta[c]pyrrol-(1H)-yl, (3aR,4R,6aS)-hexahydrocyclopenta[c]pyrrol-(1H)-yl, (3aR,4S,6aS)-hexahydrocyclopenta[c]pyrrol-(1H)-yl, (3aR,5r,6aS)-hexahydrocyclopenta[c]pyrrol-2(1H)-yl, 1,3-dihydro-2H-isoindolyl, octahydro-2H-isoindolyl, (3aS)-1,3,3a,4,5,6-hexahydro-2H-isoindolyl, (3aR,4R,7aS)-1H-isoindol-(3H,3aH,4H,5H,6H,7H,7aH)-yl, (3aR,7aS)-octahydro-2H-isoindolyl, (3aR,4R,7aS)-octahydro-2H-isoindolyl, (3aR,4S,7aS)-octahydro-2H-isoindolyl, 2,5-diazabicyclo[2.2.1]heptanyl, 2-azabicyclo[2.2.1]hept-5-enyl, 3-azabicyclo[3.1.0]hexanyl, (1R,5S,6s)-3-azabicyclo[3.1.0]hexanyl, (cis,cis)-3-azabicyclo[3.1.0]hexanyl, 3,6-diazabicyclo[3.1.0]hexanyl, (1S,5R,6R)-3-azabicyclo[3.2.0]heptanyl, (1S,5R,6S)-3-azabicyclo[3.2.0]heptanyl, 5-azaspiro[2.4]heptanyl, 2,6-diazaspiro[3.3]heptanyl, 2,5-diazaspiro[3.4]octanyl, 2,6-diazaspiro[3.4]octanyl, 2,7-diazaspiro[3.5]nonanyl, 2,7-diazaspiro[4.4]nonanyl, 2-azaspiro[4.5]decanyl or 2,8-diazaspiro[4.5]decanyl;
heterocyclyl-C₁₋₈alkyl, wherein heterocyclyl is selected from azetidinyl, pyrrolidinyl, tetrahydrofuranyl, piperidinyl, piperazinyl, morpholinyl, 1,4-diazepanyl, 1,3-dioxolanyl, 2,5-dihydro-1H-pyrrolyl, dihydro-1H-imidazolyl, 1,4,5,6-tetrahydropyrimidinyl, 1,2,3,6-tetrahydropyridinyl, tetrahydro-2H-pyranyl, 3,4-dihydroisoquinolin-(1H)-yl, 1,2,3,4-tetrahydroisoquinolinyl, hexahydropyrrolo[3,4-b][1,4]oxazin-(2H)-yl, (4aR,7aS)-hexahydropyrrolo[3,4-b][1,4]oxazin-(4aH)-yl, (cis)-octahydrocyclopenta[c]pyrrolyl, hexahydropyrrolo[3,4-b]pyrrol-(1H)-yl, (3aR,6aR)-hexahydropyrrolo[3,4-b]pyrrol-(1H)-yl, (3aR,6aS)-hexahydropyrrolo[3,4-c]pyrrol-(1H)-yl, 5H-pyrrolo[3,4-b]pyridin-(7H)-yl, 5,7-dihydro-6H-pyrrolo[3,4-b]pyridinyl, tetrahydro-1H-pyrrolo[3,4-b]pyridin-(2H,7H,7aH)-yl, hexahydro-1H-pyrrolo[3,4-b]pyridin-(2H)-yl, (4aR,7aR)-hexahydro-1H-pyrrolo[3,4-b]pyridin-(2H)-yl, octahydro-6H-pyrrolo[3,4-b]pyridinyl, (3aR,6aR)-hexahydrocyclopenta[c]pyrrol-(1H)-yl, (3aR,4R,6aS)-hexahydrocyclopenta[c]pyrrol-(1H)-yl, (3aR,4S,6aS)-hexahydrocyclopenta[c]pyrrol-(1H)-yl, (3aR,5r,6aS)-hexahydrocyclopenta[c]pyrrol-2(1H)-yl, 1,3-dihydro-2H-isoindolyl, octahydro-2H-isoindolyl, (3aS)-1,3,3a,4,5,6-hexahydro-2H-isoindolyl, (3aR,4R,7aS)-1H-isoindol-(3H,3aH,4H,5H,6H,7H,7aH)-yl, (3aR,7aS)-octahydro-2H-isoindolyl, (3aR,4R,7aS)-octahydro-2H-isoindolyl, (3aR,4S,7aS)-octahydro-2H-isoindolyl, 2,5-diazabicyclo[2.2.1]heptanyl, 2-azabicyclo[2.2.1]hept-5-enyl, 3-azabicyclo[3.1.0]hexanyl, (1R,5S,6s)-3-azabicyclo[3.1.0]hexanyl, (cis,cis)-3-azabicyclo[3.1.0]hexanyl, 3,6-diazabicyclo[3.1.0]hexanyl, (1S,5R,6R)-3-azabicyclo[3.2.0]heptanyl, (1S,5R,6S)-3-azabicyclo[3.2.0]heptanyl, 5-azaspiro[2.4]heptanyl, 2,6-diazaspiro[3.3]heptanyl, 2,5-diazaspiro[3.4]octanyl, 2,6-diazaspiro[3.4]octanyl, 2,7-diazaspiro[3.5]nonanyl, 2,7-diazaspiro[4.4]nonanyl, 2-azaspiro[4.5]decanyl or 2,8-diazaspiro[4.5]decanyl;
heterocyclyl-amino-C₁₋₈alkyl, wherein heterocyclyl is selected from azetidin-1-yl or piperidin-4-yl;
(heterocyclyl,C₁₋₈alkyl)amino-C₁₋₈alkyl, wherein heterocyclyl is selected from piperidin-3-yl or piperidin-4-yl;
(heterocyclyl,C₃₋₁₄cycloalkyl-C₁₋₈alkyl)amino-C₁₋₈alkyl, wherein heterocyclyl is selected from piperidin-3-yl or piperidin-4-yl;
heterocyclyl-C₁₋₈alkyl-amino-C₁₋₈alkyl, wherein heterocyclyl is selected from pyrrolidin-2-yl, piperidin-2-yl, piperidin-3-yl, piperidin-4-yl or tetrahydro-2H-pyran-4-yl; and
(heterocyclyl-oxy-C₁₋₈alkyl,C₁₋₈alkyl)amino selected from tetrahydro-2H-pyran-2-yl-oxy-C₁₋₈alkyl,C₁₋₈alkyl)amino.

One embodiment of the present description includes a compound of Formula (I) or a form thereof, wherein R₁ is
heteroaryl, wherein heteroaryl is selected from 1H-tetrazolyl, imidazolyl, pyrrolyl or 2H-tetrazolyl; and,
heterocyclyl, wherein heterocyclyl is selected from azetidinyl, pyrrolidinyl, tetrahydrofuranyl, piperidinyl, piperazinyl, morpholinyl, 2,5-dihydro-1H-pyrrolyl, hexahydropyrrolo[3,4-b][1,4]oxazin-(2H)-yl, (4aR,7aS)-hexahydropyrrolo[3,4-b][1,4]oxazin-(4aH)-yl, (3aR,6aR)-hexahydropyrrolo[3,4-b]pyrrol-(1H)-yl, (3aR,6aS)-hexahydropyrrolo[3,4-c]pyrrol-(1H)-yl, 5,7-dihydro-6H-pyrrolo[3,4-b]pyridinyl, octahydro-6H-pyrrolo[3,4-b]pyridinyl, (3aR,6aR)-hexahydrocyclopenta[c]pyrrol-3a(1H)-yl, (3aR,4R,6aS)-hexahydrocyclopenta[c]pyrrol-(1H)-yl, (3aR,4S,6aS)-hexahydrocyclopenta[c]pyrrol-(1H)-yl, (3aR,5r,6aS)-hexahydrocyclopenta[c]pyrrol-(1H)-yl, 1,3-dihydro-2H-isoindolyl, octahydro-2H-isoindolyl, (3aS)-1,3,3a,4,5,6-hexahydro-2H-isoindolyl, (3aR,7aS)-octahydro-2H-isoindolyl, (3aR,4R,7aS)-octahydro-2H-isoindolyl, (3aR,4S,7aS)-octahydro-2H-isoindolyl, 2,5-diazabicyclo[2.2.1]heptanyl, 2-azabicyclo[2.2.1]hept-5-enyl, 3-azabicyclo[3.1.0]hexanyl, 3,6-diazabicyclo[3.1.0]hexanyl, (1R,5S,6s)-3-azabicyclo[3.1.0]hexanyl, (1S,5R,6R)-3-azabicyclo[3.2.0]heptanyl, (1S,5R,6S)-3-azabicyclo[3.2.0]heptanyl, 5-azaspiro[2.4]heptanyl, 2,6-diazaspiro[3.3]heptanyl, 2,5-diazaspiro[3.4]octanyl, 2,6-diazaspiro[3.4]octanyl, 2,7-diazaspiro[3.5]nonanyl, 2,7-diazaspiro[4.4]nonanyl, 2-azaspiro[4.5]decanyl or 2,8-diazaspiro[4.5]decanyl.

One embodiment of the present description includes a compound of Formula (I) or a form thereof, wherein R₁ is
heteroaryl, wherein heteroaryl is selected from 1H-tetrazol-5-yl, imidazol-1-yl, pyrrol-1-yl or 2H-tetrazol-2-yl; and,
heterocyclyl, wherein heterocyclyl is selected from azetidin-1-yl, pyrrolidin-1-yl, piperidin-1-yl, tetrahydrofuran-2-yl, piperazin-1-yl, morpholin-4-yl, 2,5-dihydro-1H-pyrrol-1-yl, hexahydropyrrolo[3,4-b][1,4]oxazin-6(2H)-yl, (4aR,7aS)-hexahydropyrrolo[3,4-b][1,4]oxazin-4(4aH)-yl, (3aR,6aR)-hexahydropyrrolo[3,4-b]pyrrol-5(1H)-yl, (3aR,6aS)-hexahydropyrrolo[3,4-c]pyrrol-2(1H)-yl, 5,7-dihydro-6H-pyrrolo[3,4-b]pyridin-6-yl, octahydro-6H-pyrrolo[3,4-b]pyridin-6-yl, (3aR,6aR)-hexahydrocyclopenta[c]pyrrol-3a(1H)-yl, (3aR,4R,6aS)-hexahydrocyclopenta[c]pyrrol-2(1H)-yl, (3aR,4S,6aS)-hexahydrocyclopenta[c]pyrrol-2(1H)-yl, (3aR,5r,6aS)-hexahydrocyclopenta[c]pyrrol-2(1H)-yl, 1,3-dihydro-2H-isoindol-2-yl, octahydro-2H-isoindol-2-yl, (3aS)-1,3,3a,4,5,6-hexahydro-2H-isoindol-2-yl, (3aR,7aS)-octahydro-2H-isoindol-2-yl, (3aR,4R,7aS)-octahydro-2H-isoindol-2-yl, (3aR,4S,7aS)-octahydro-2H-isoindol-2-yl, 2,5-diazabicyclo[2.2.1]heptan-2-yl, 2-azabicyclo[2.2.1]hept-5-en-2-yl, 3-azabicyclo[3.1.0]hexan-3-yl, 3,6-diazabicyclo[3.1.0]hexan-3-yl, (1R,5S,6s)-3-azabicyclo[3.1.0]hexan-3-yl, (1R,5S,6s)-3-azabicyclo[3.1.0]hexan-6-yl, (1S,5R,6R)-3-azabicyclo[3.2.0]heptan-3-yl, (1S,5R,6S)-3-azabicyclo[3.2.0]heptan-3-yl, 5-azaspiro[2.4]heptan-5-yl, 2,6-diazaspiro[3.3]heptan-2-yl, 2,5-diazaspiro[3.4]octan-2-yl, 2,6-diazaspiro[3.4]octan-6-yl, 2,7-diazaspiro[3.5]nonan-2-yl, 2,7-diazaspiro[4.4]nonan-2-yl, 2-azaspiro[4.5]decan-2-yl or 2,8-diazaspiro[4.5]decan-2-yl.

One embodiment of the present description includes a compound of Formula (I) or a form thereof, wherein R₁ is
heteroaryl, wherein heteroaryl is selected from pyridinyl; and,
heterocyclyl, wherein heterocyclyl is selected from azetidinyl, pyrrolidinyl, tetrahydrofuranyl, piperidinyl, piperazinyl, morpholinyl, 1,4-diazepanyl, 1,3-dioxolanyl, dihydro-1H-imidazolyl, 1,4,5,6-tetrahydropyrimidinyl, 1,2,3,6-tetrahydropyridinyl, tetrahydro-2H-pyranyl, 3,4-dihydroisoquinolin-(1H)-yl, 1,2,3,4-tetrahydroisoquinolinyl, 5H-pyrrolo[3,4-b]pyridin-(7H)-yl, tetrahydro-1H-pyrrolo[3,4-b]pyridin-(2H,7H,7aH)-yl, (3aR,4R,6aS)-hexahydrocyclopenta[c]pyrrol-(1H)-yl, (3aR,4R,7aS)-1H-isoindol-(3H,3aH,4H,5H,6H,7H,7aH)-yl, 2,5-diazabicyclo[2.2.1]heptanyl or (1R,5S,6s)-3-azabicyclo[3.1.0]hexanyl.

One embodiment of the present description includes a compound of Formula (I) or a form thereof, wherein R₁ is
heteroaryl selected in each instance, when present, from pyridin-2-yl, pyridin-3-yl or pyridin-4-yl;
heterocyclyl selected in each instance, when present, from azetidin-1-yl, pyrrolidin-1-yl, pyrrolidin-2-yl, pyrrolidin-3-yl, tetrahydrofuran-2-yl, piperidin-1-yl, piperidin-2-yl, piperidin-3-yl, piperidin-4-yl, piperazin-1-yl, piperazin-2-yl, morpholin-4-yl, 1,4-diazepan-1-yl, 1,3-dioxolan-2-yl, dihydro-1H-imidazol-2-yl, 1,4,5,6-tetrahydropyrimidin-2-yl, 1,2,3,6-tetrahydropyridin-4-yl, tetrahydro-2H-pyran-2-yl, tetrahydro-2H-pyran-4-yl, 3,4-dihydroisoquinolin-2(1H)-yl, 1,2,3,4-tetrahydroisoquinolin-1-yl, 5H-pyrrolo[3,4-b]pyridin-6(7H)-yl, tetrahydro-1H-pyrrolo[3,4-b]pyridin-6(2H,7H,7aH)-yl, (3aR,4R,6aS)-hexahydrocyclopenta[c]pyrrol-2(1H)-yl, (3aR,4R,7aS)-1H-isoindol-2(3H,3aH,4H,5H,6H,7H,7aH)-yl, 2,5-diazabicyclo[2.2.1]heptan-2-yl, (1R,5S,6s)-3-azabicyclo[3.1.0]hexan-3-yl or (1R,5S,6s)-3-azabicyclo[3.1.0]hexan-6-yl.

One embodiment of the present description includes a compound of Formula (I) or a form thereof, wherein R₁₆ is
C₃₋₁₄cycloalkyl, wherein C₃₋₁₄cycloalkyl is selected from cyclopropyl or cyclobutyl;
C₃₋₁₄cycloalkyl-amino, wherein C₃₋₁₄cycloalkyl is selected from cyclopropyl;
aryl, wherein aryl is selected from phenyl;
aryl-C₁₋₈alkyl, wherein aryl is selected from phenyl;
aryl-amino, wherein aryl is selected from phenyl;
(aryl,C₁₋₈alkyl)amino, wherein aryl is selected from phenyl;
aryl-C₁₋₈alkyl-amino, wherein aryl is selected from phenyl;
(aryl-C₁₋₈alkyl,C₁₋₈alkyl)amino, wherein aryl is selected from phenyl;
(aryl-C₁₋₈alkyl)₂-amino, wherein aryl is selected from phenyl;
aryl-C₁₋₈alkyl-amino-C₁₋₈alkyl, wherein aryl is selected from phenyl;
(aryl-C₁₋₈alkyl,C₁₋₈alkyl)amino-C₁₋₈alkyl, wherein aryl is selected from phenyl;
(aryl-C₁₋₈alkyl)₂-amino-C₁₋₈alkyl, wherein aryl is selected from phenyl;
aryl-C₁₋₈alkoxy, wherein aryl is selected from phenyl;
aryl-C₁₋₈alkoxy-carbonyl-amino, wherein aryl is selected from phenyl;
heteroaryl, wherein heteroaryl is selected from pyridin-2-yl, pyridin-4-yl, thiazol-2-yl or 1H-1,2,3-triazol-1-yl;
heteroaryl-C₁₋₈alkyl-amino-C₁₋₈alkyl, wherein heteroaryl is selected from pyridin-2-yl, pyridin-3-yl or pyridin-4-yl;
heterocyclyl, wherein heterocyclyl is selected from pyrrolidin-1-yl or morpholin-4-yl;
heterocyclyl-C₁₋₈alkyl, wherein heterocyclyl is selected from pyrrolidin-1-yl; and,
heterocyclyl-oxy, wherein heterocyclyl is selected from tetrahydro-2H-pyran-2-yl-oxy.

One embodiment of the present description includes a compound of Formula (I) or a form thereof, wherein R₁₆ is
aryl, wherein aryl is selected from phenyl;
(aryl-C₁₋₈alkyl,C₁₋₈alkyl)amino, wherein aryl is selected from phenyl;
(aryl-C₁₋₈alkyl)₂-amino, wherein aryl is selected from phenyl;
aryl-amino-C₁₋₈alkyl, wherein aryl is selected from phenyl;
heteroaryl, wherein heteroaryl is selected from pyridin-2-yl or pyridin-4-yl;
heterocyclyl, wherein heterocyclyl is selected from piperidin-1-yl; and,
heterocyclyl-amino-C₁₋₈alkyl, wherein heterocyclyl is selected from (1R,5S,6s)-3-azabicyclo[3.1.0]hexan-6-yl.

In one embodiment, the compound of Formula (I), Formula (II) or Formula (III) is a compound of Formula (Ia), Formula (IIa) or Formula (IIIa), respectively:

| | | |
|---|---|---|
| 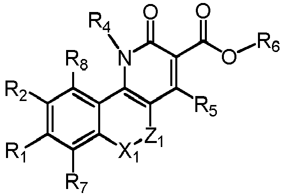 | 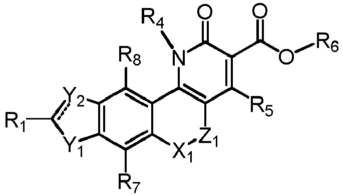 | 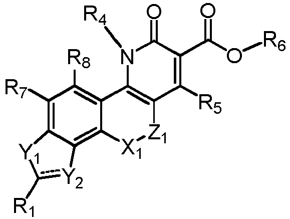 |
| (Ia) | (IIa) | (IIIa) |

or a form thereof, wherein
the dashed line in Formula (IIa) and Formula (IIIa) represents an optionally present double bond;
X₁ is a bond, N(R₁₄), S, O or -CH(R₉)-;
Z₁ is N(R₁₄), S, O, C(O) or -CH(R₃)- when X₁ is a bond or -CH(R₉)- or, Z₁ is -CH(R₃)- when X₁ is N(R₁₄), S, or O; and,
all other variables are as previously defined.

In a disclosure, the compound of Formula (I), Formula (II) or Formula (III) is a compound of Formula (Ib), Formula (IIb) or Formula (IIIb), respectively:

| | | |
|---|---|---|
| 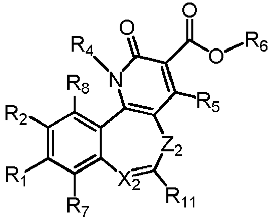 | 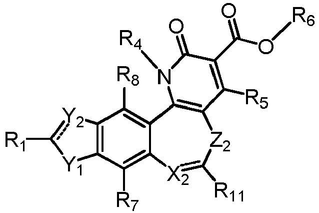 | 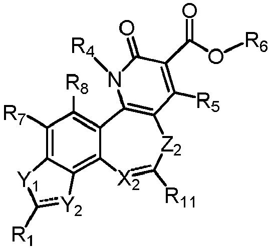 |
| (Ib) | (IIb) | (IIIb) |

or a form thereof, wherein
the dashed lines in Formula (Ib), Formula (IIb) and Formula (IIIb) represent optionally present double bonds;
X₂ is N(R₁₄), S, O or -CH(R₉)- when a double bond is absent; and, X₂ is N or -C(R₉)- when a double bond is present, wherein X₂ is -CH(R₉)- when Z₂ is N(R₁₄), S, O or C(O);
Z₂ is N(R₁₄), S, O, C(O) or -CH(R₃)-, wherein Z₂ is -CH(R₃)- when X₂ is N(R₁₄), S or O; and, all other variables are as previously defined.

In a disclosure, the compound of Formula (I), Formula (II) or Formula (III) is a compound of Formula (Ic), Formula (IIc) or Formula (IIIc), respectively:

| | | |
|---|---|---|
| 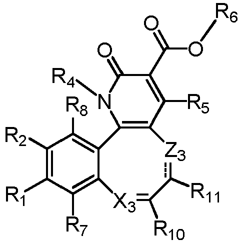 | 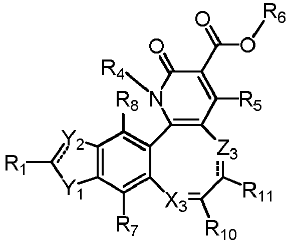 | 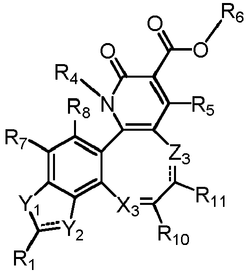 |
| (Ic) | (IIc) | (IIIc) |

or a form thereof, wherein
the dashed lines in Formula (Ic), Formula (IIc) and Formula (IIIc) represent optionally present double bonds;
X₃ is N(R₁₄), S, O or -CH(R₉)- when a double bond is absent; and, X₃ is N, S, O or -C(R₉)- when a double bond is present, wherein X₂ is -CH(R₉)- when Z₂ is N(R₁₄), S, O or C(O);
Z₃ is N(R₁₄), S, O, C(O) or -CH(R₃)- when a double bond is absent; and, Z₃ is N or -CH(R₃)- when a double bond is present, wherein Z₂ is -CH(R₃)- when X₂ is N(R₁₄), S or O; and,
all other variables are as previously defined.

In another embodiment of the present description, a compound or a form thereof is selected from: 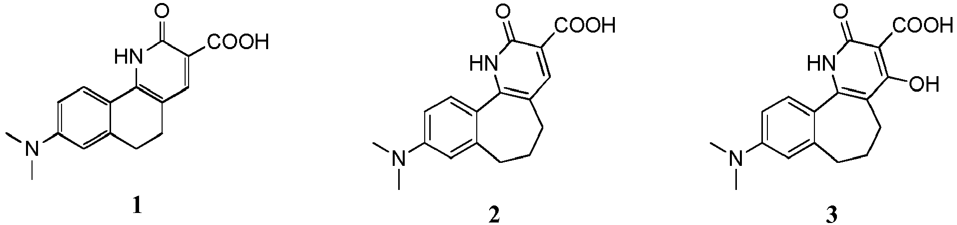 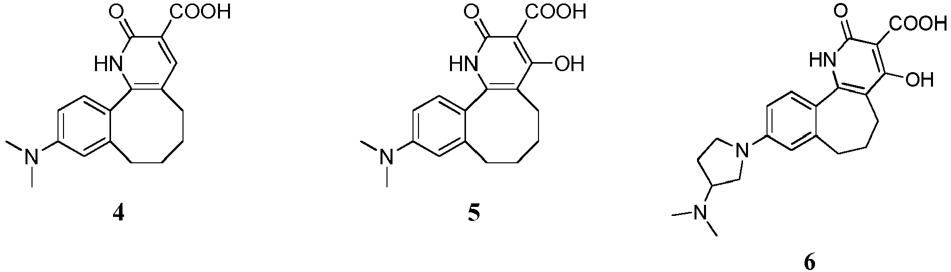 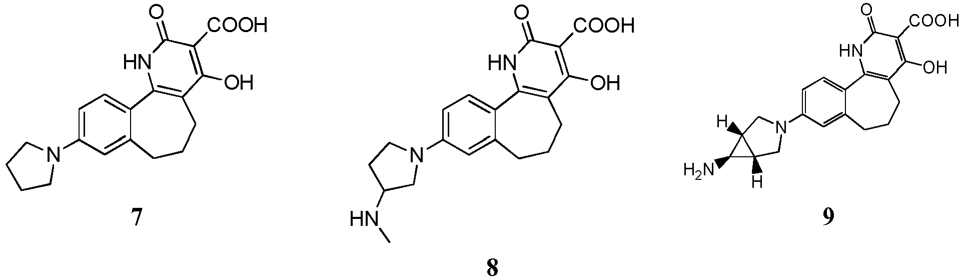 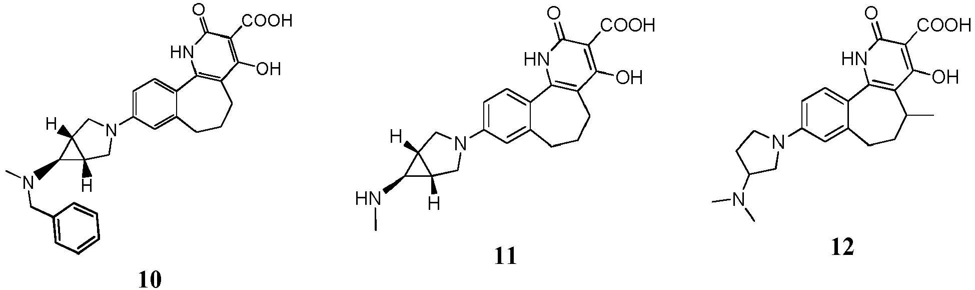 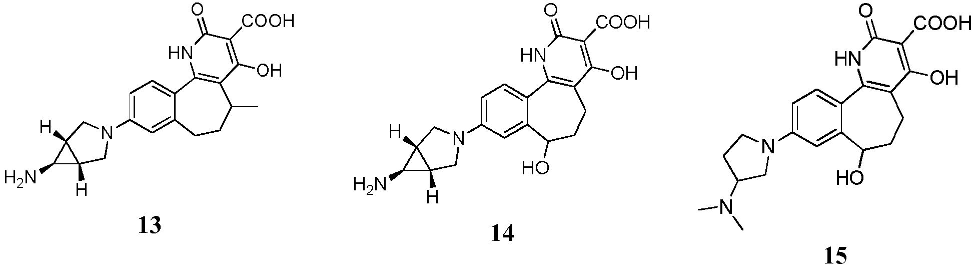 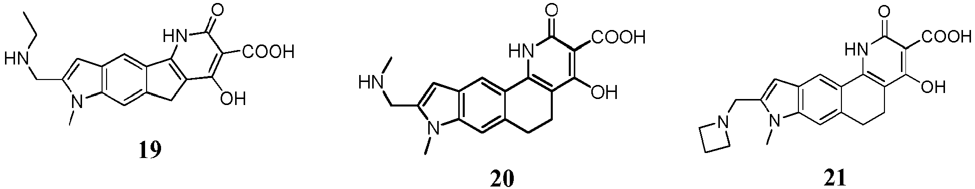 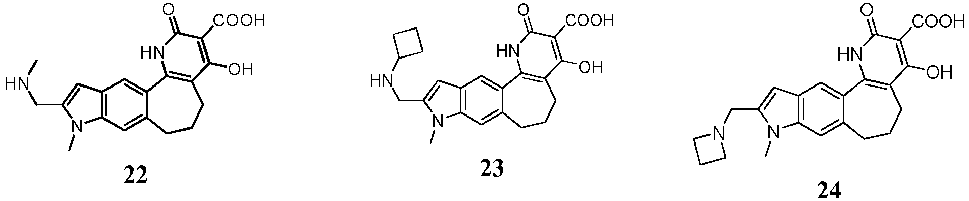 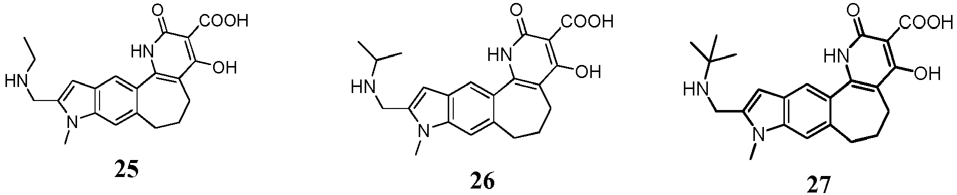 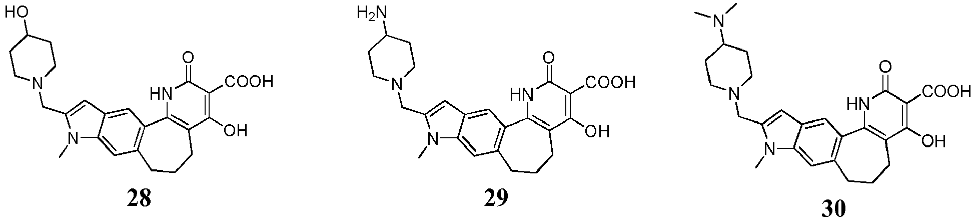 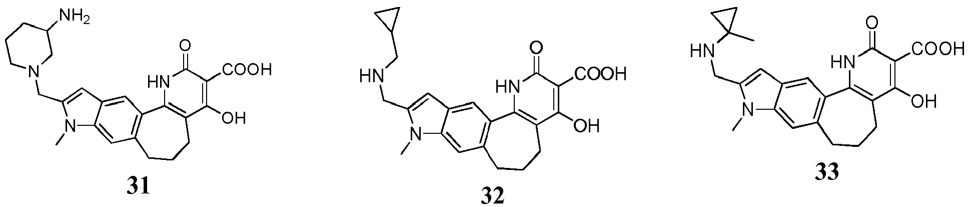 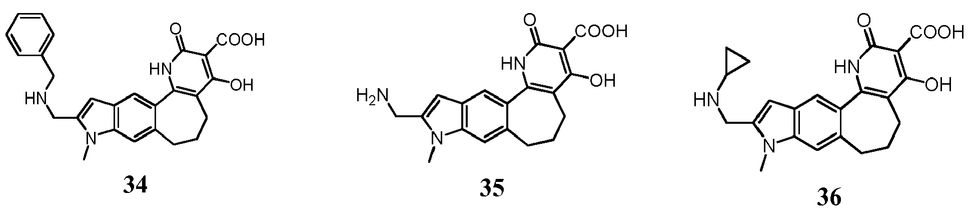 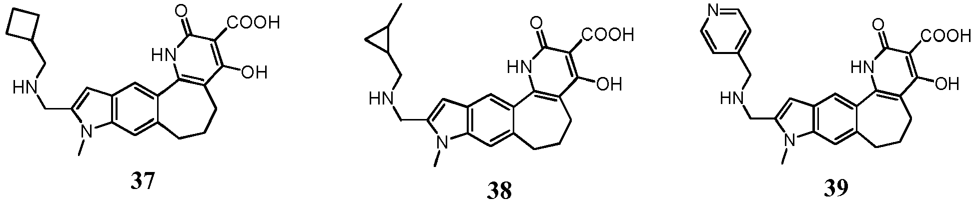 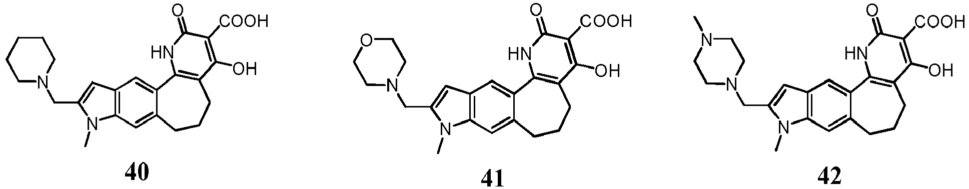 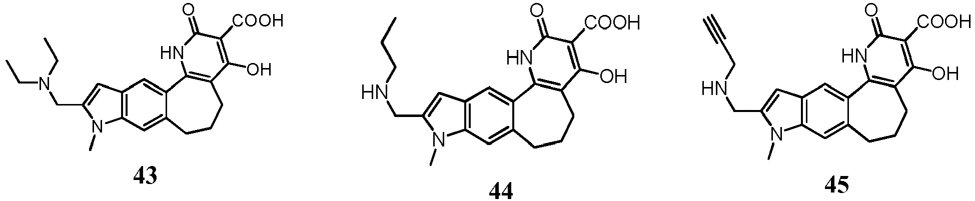 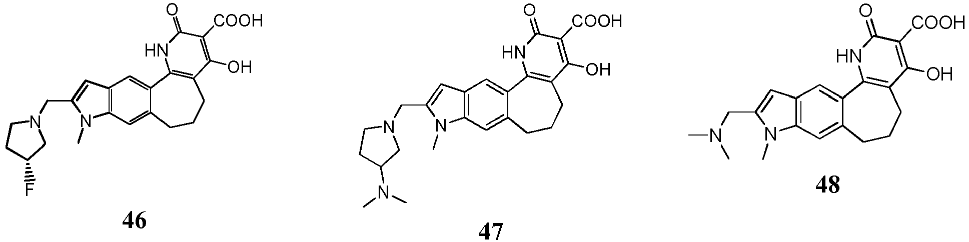 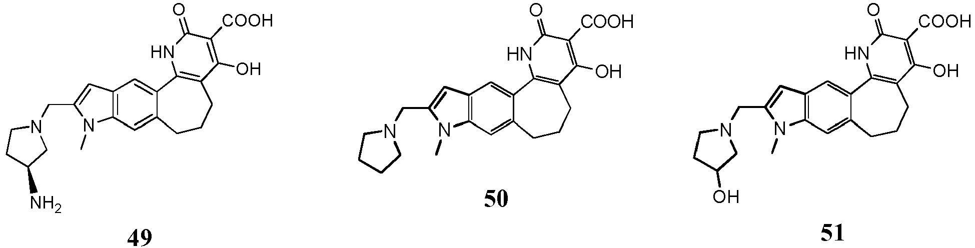 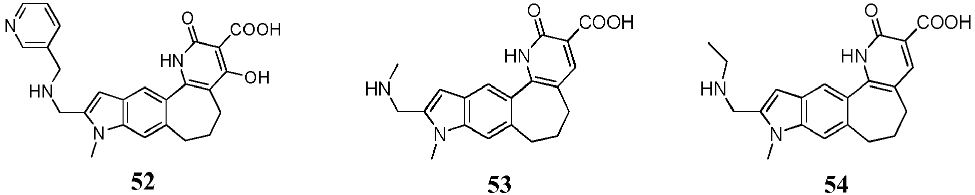 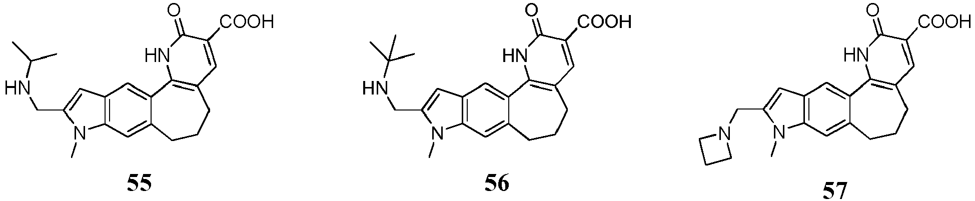 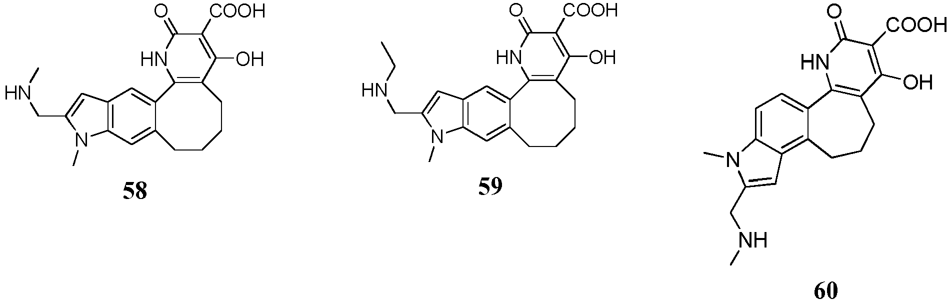 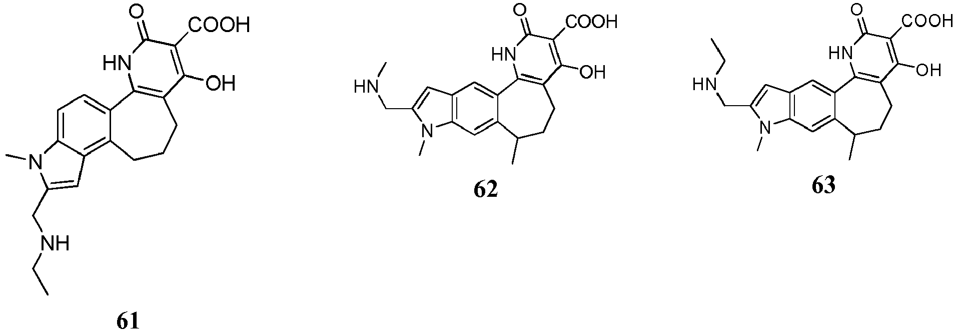 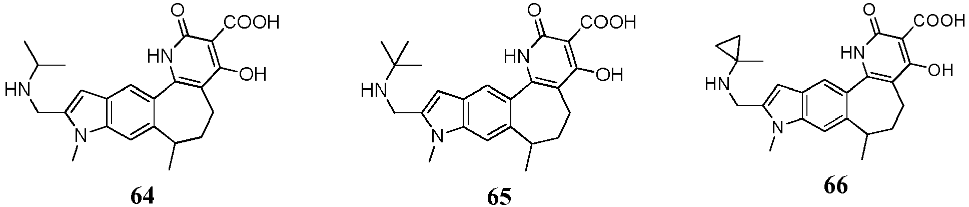 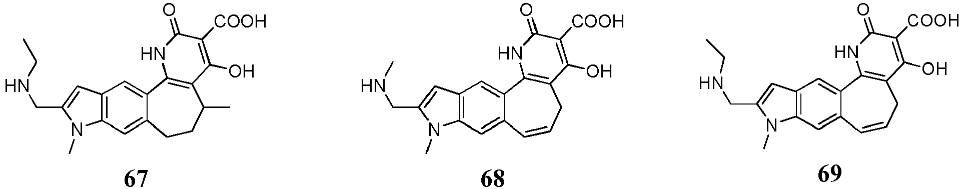 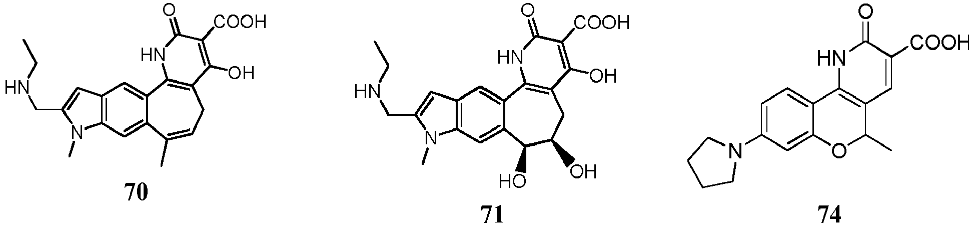 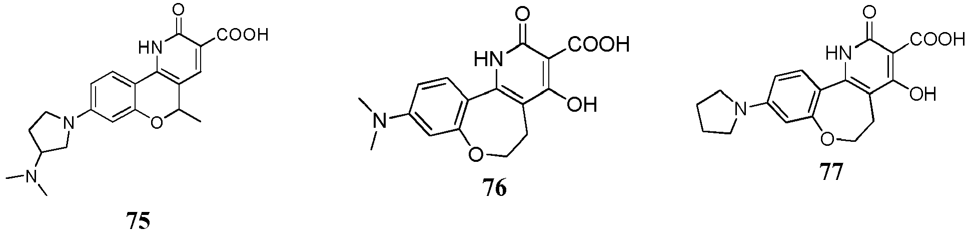 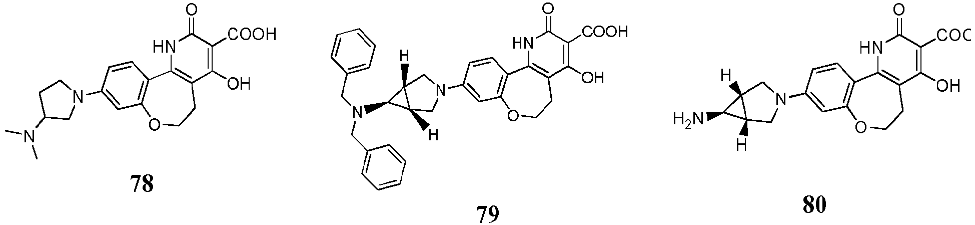 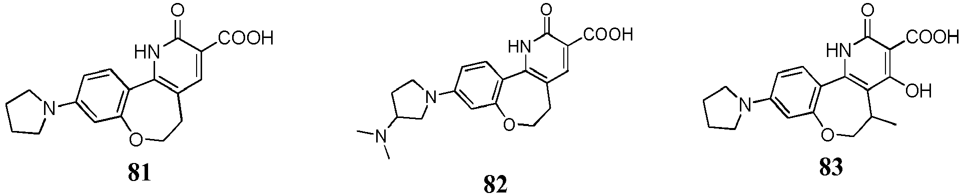 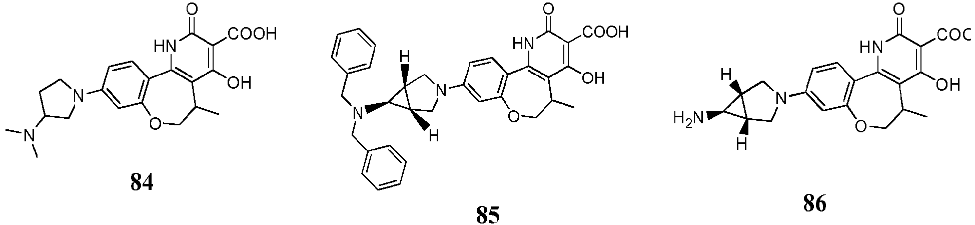 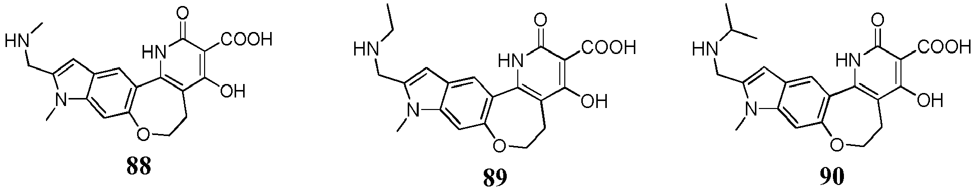 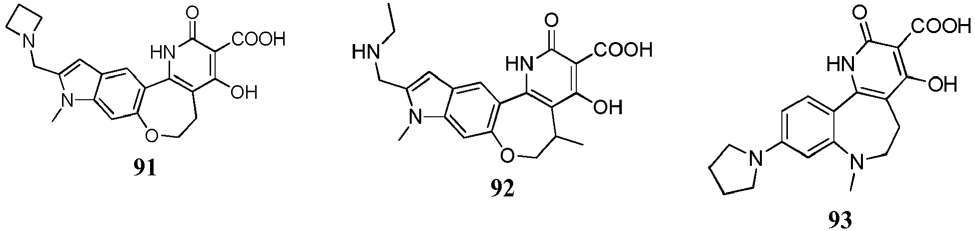 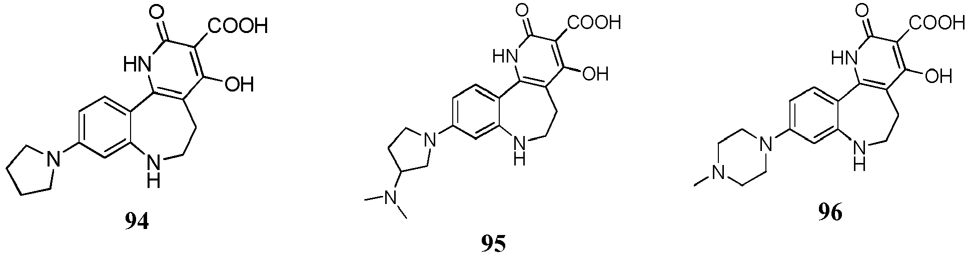 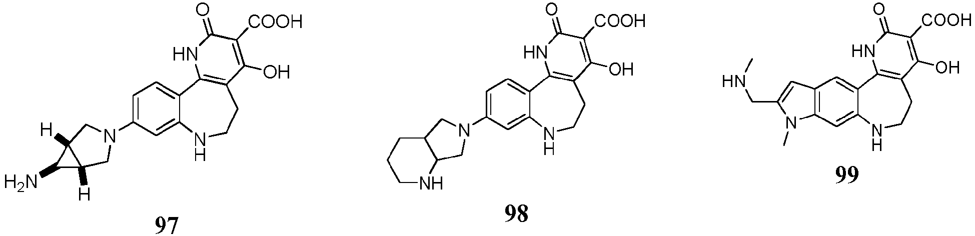 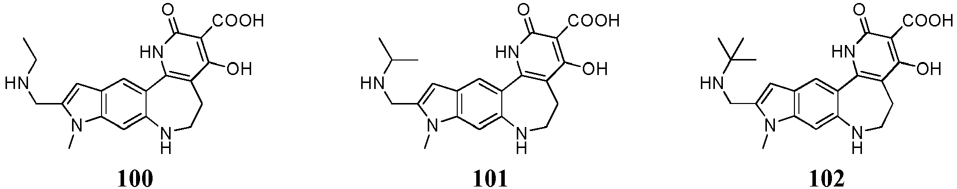 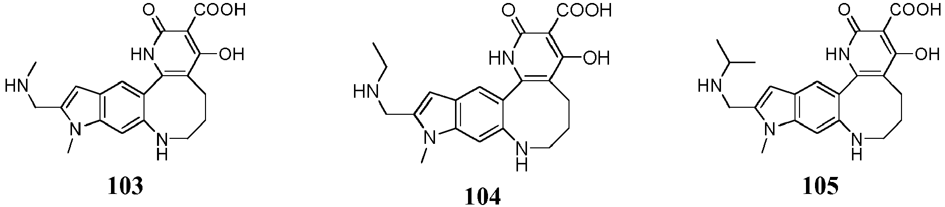 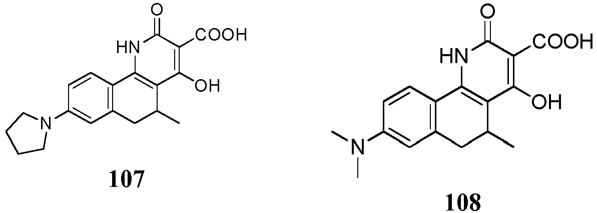 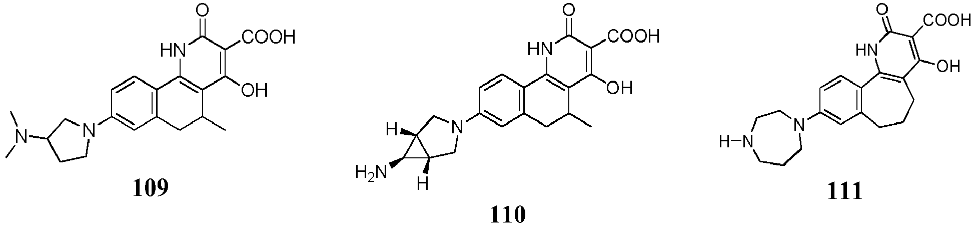 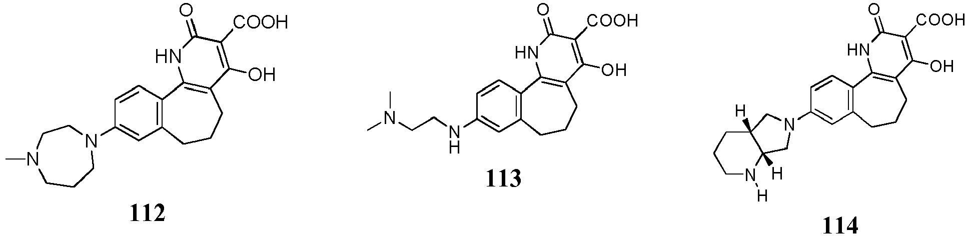 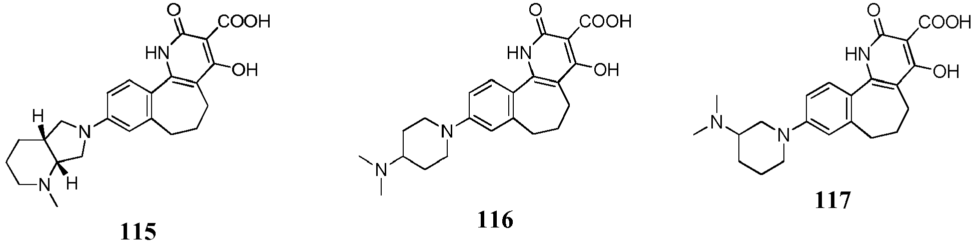 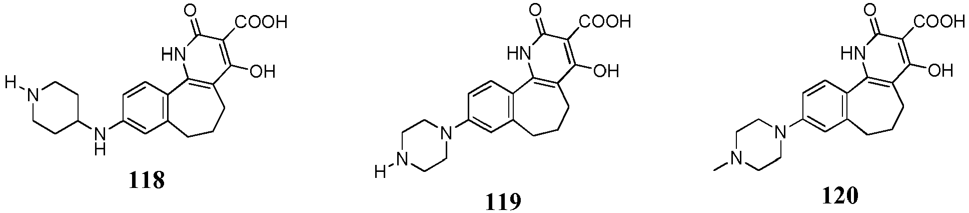 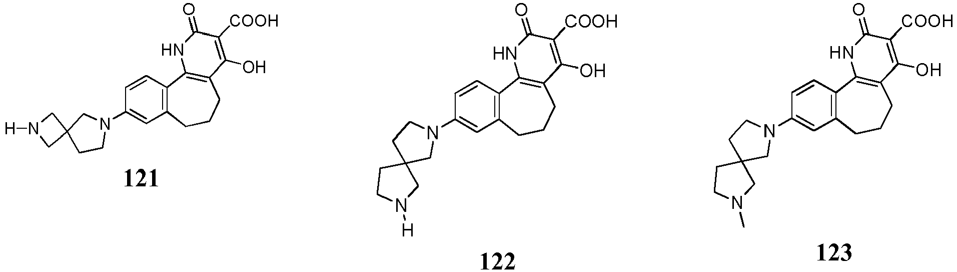 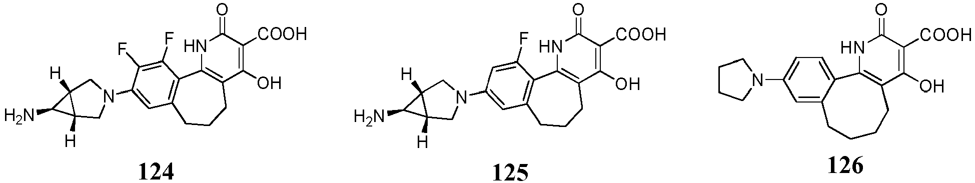 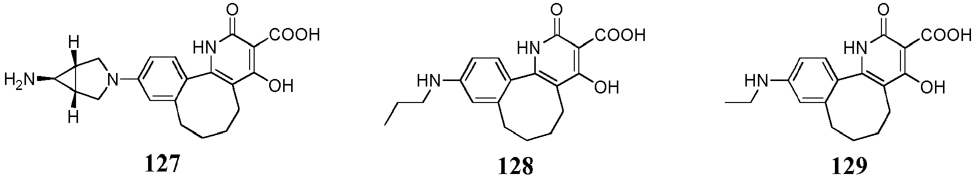 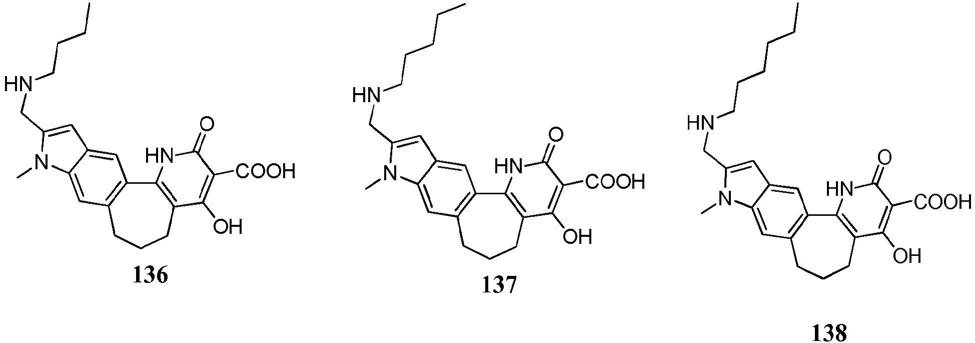 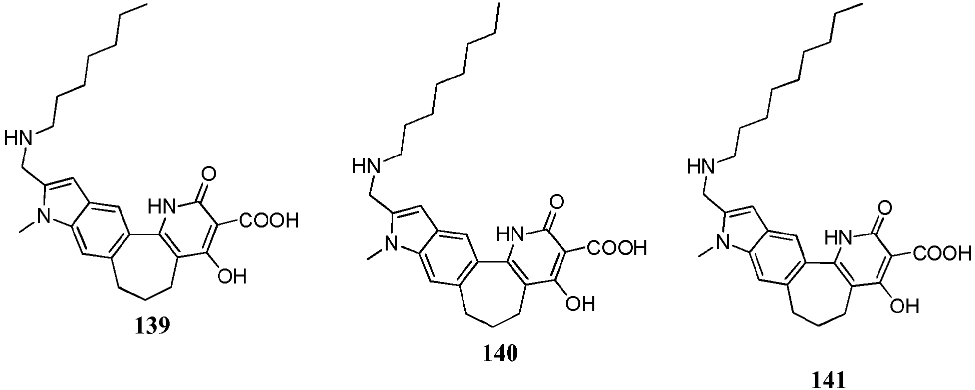 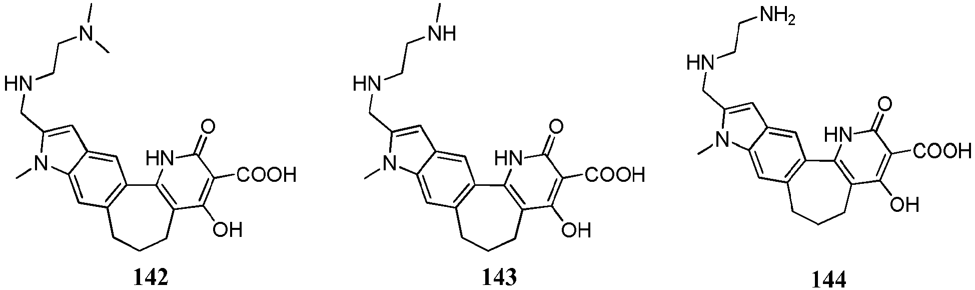 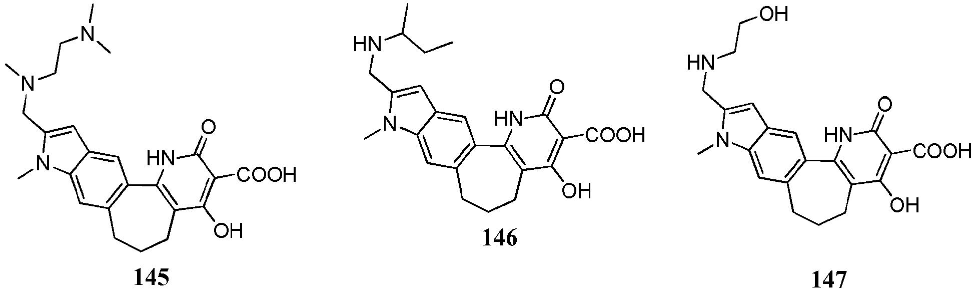 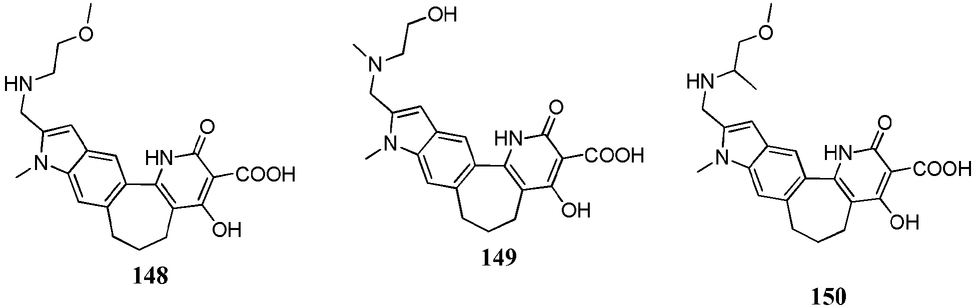 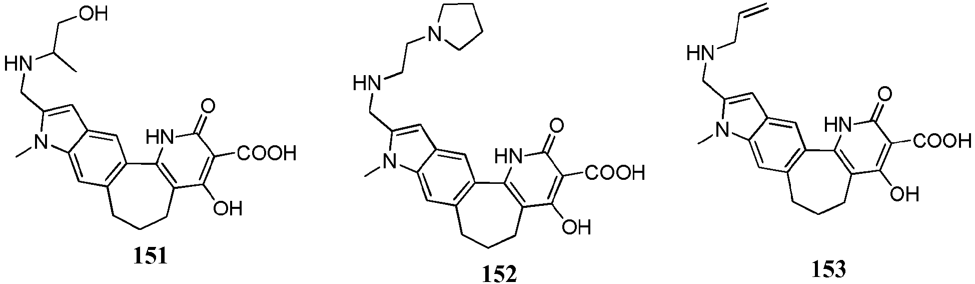 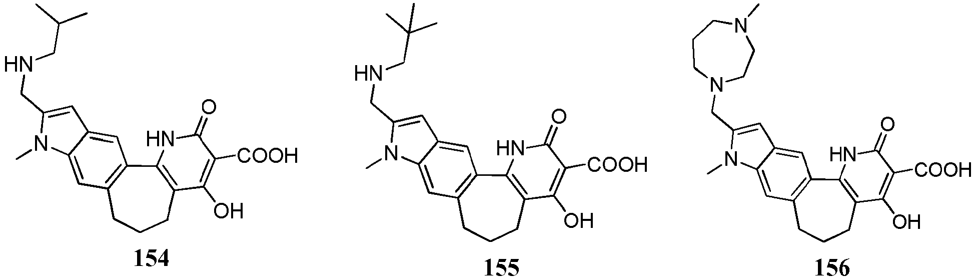 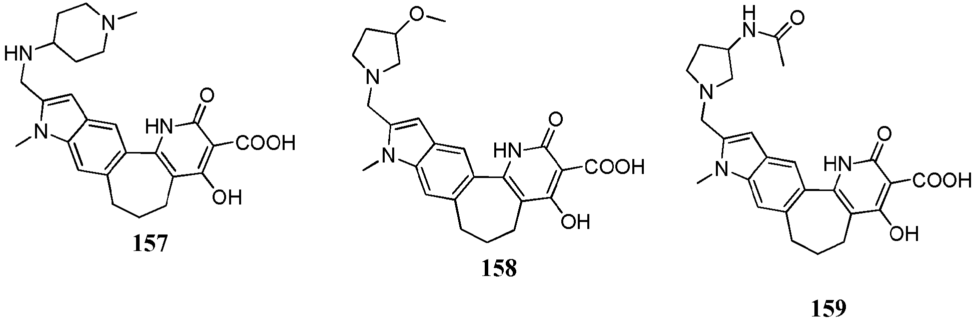 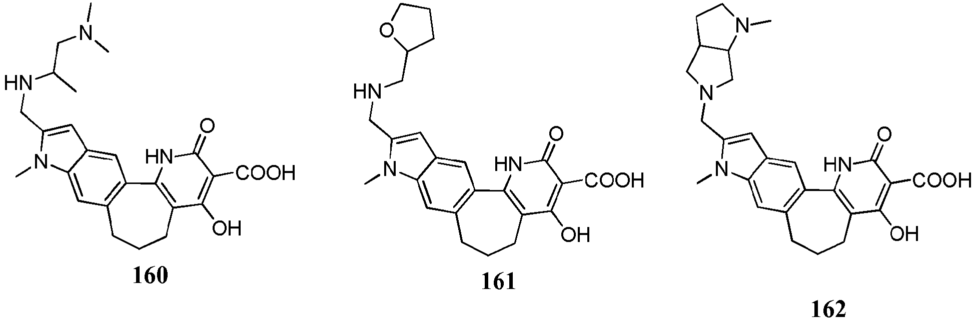 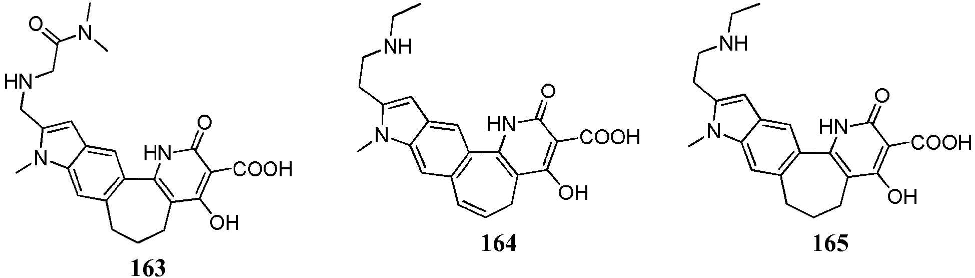 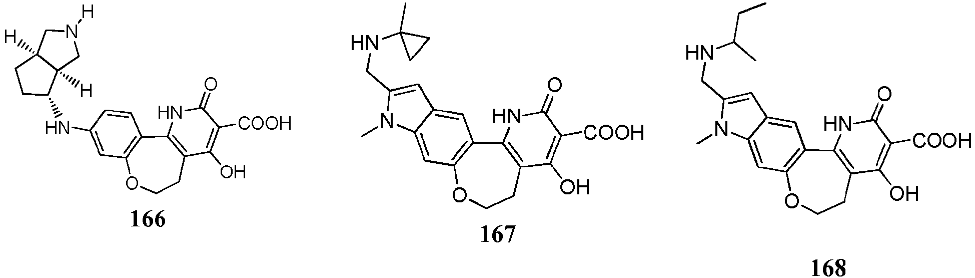 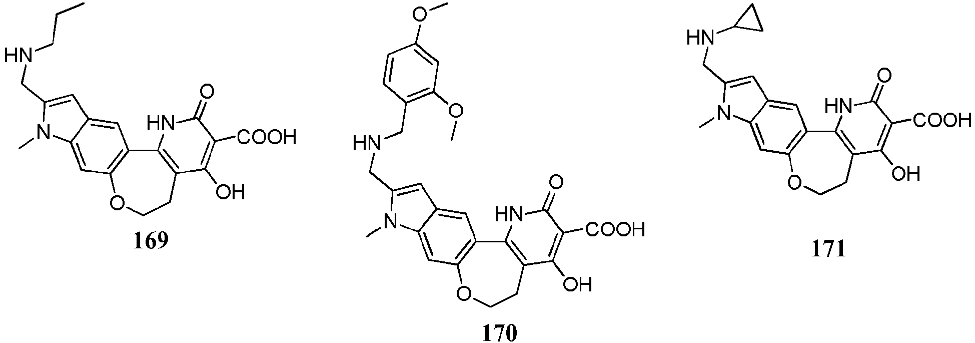 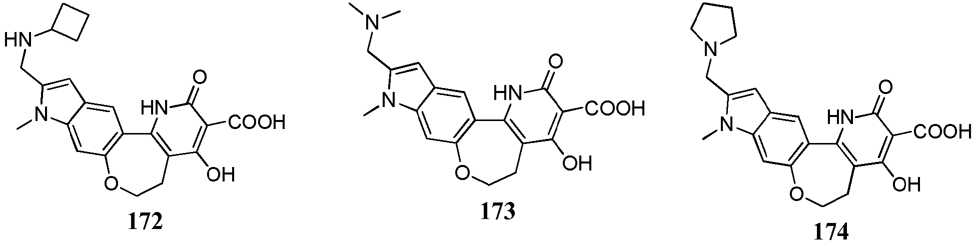 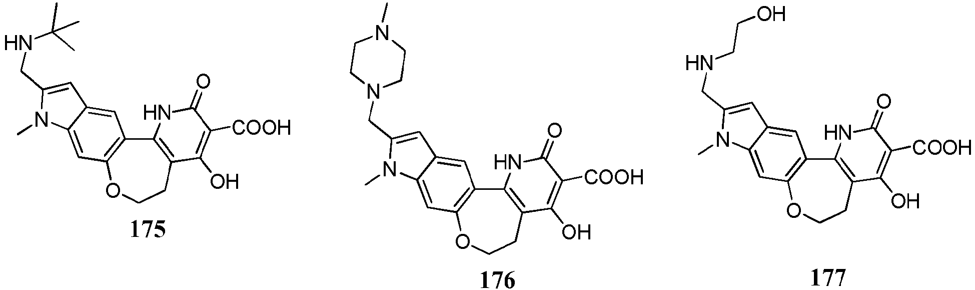 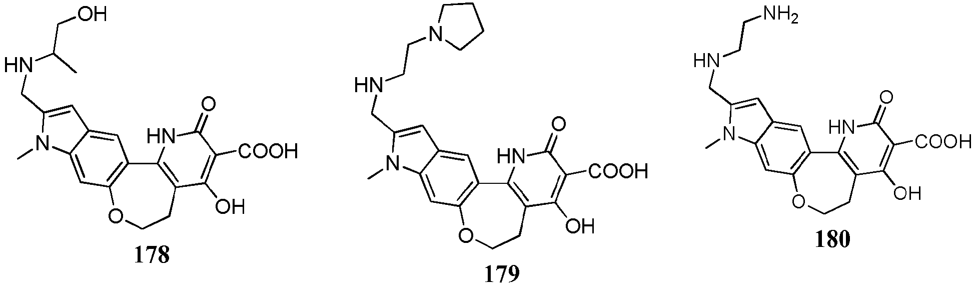 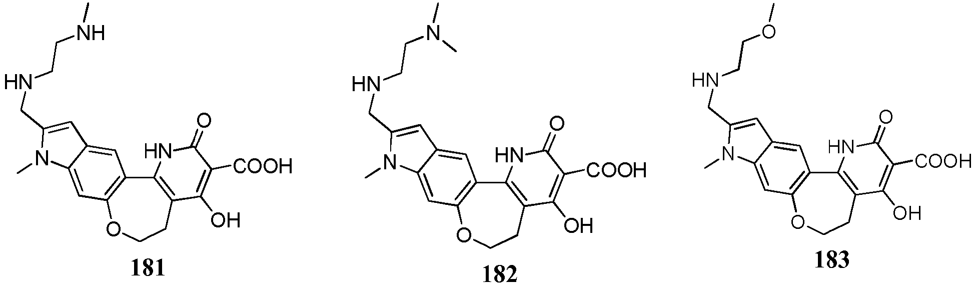 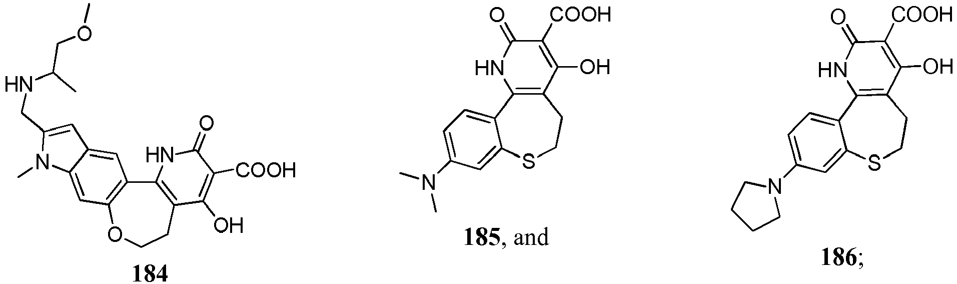 wherein the form of the compound is selected from a free acid, free base, salt, hydrate, solvate, clathrate, isotopologue, racemate, enantiomer, diastereomer, stereoisomer, polymorph or tautomer form thereof, and wherein the form is isolated for use.

In another embodiment of the present description, a compound or a form thereof is selected from:

| **Cpd** | **Name** |
|---|---|
| **1** | 8-(dimethylamino)-2-oxo-1,2,5,6-tetrahydrobenzo[h]quinoline-3-carboxylic acid |
| **2** | 9-(dimethylamino)-2-oxo-2,5,6,7-tetrahydro-1H-benzo[6,7]cyclohepta[1,2-b]pyridine-3-carboxylic acid |
| **3** | 9-(dimethylamino)-4-hydroxy-2-oxo-2,5,6,7-tetrahydro-1H-benzo[6,7]cyclohepta[1,2-b]pyridine-3-carboxylic acid |
| **4** | 10-(dimethylamino)-2-oxo-1,2,5,6,7,8-hexahydrobenzo[7,8]cycloocta[1,2-b]pyridine-3-carboxylic acid |
| **5** | 10-(dimethylamino)-4-hydroxy-2-oxo-1,2,5,6,7,8-hexahydrobenzo[7,8]cycloocta[1,2-b]pyridine-3-carboxylic acid |
| **7** | 4-hydroxy-2-oxo-9-(pyrrolidin-1-yl)-2,5,6,7-tetrahydro-1H-benzo[6,7]cyclohepta[1,2-b]pyridine-3-carboxylic acid |
| **74** | 5-methyl-2-oxo-8-(pyrrolidin-1-yl)-2,5-dihydro-1H-chromeno[4,3-b]pyridine-3-carboxylic acid |
| **76** | 9-(dimethylamino)-4-hydroxy-2-oxo-1,2,5,6-tetrahydrobenzo[2,3]oxepino[4,5-b]pyridine-3-carboxylic acid |
| **77** | 4-hydroxy-2-oxo-9-(pyrrolidin-1-yl)-1,2,5,6-tetrahydrobenzo[2,3]oxepino[4,5-b]pyridine-3-carboxylic acid |
| **81** | 2-oxo-9-(pyrrolidin-1-yl)-1,2,5,6-tetrahydrobenzo[2,3]oxepino[4,5-b]pyridine-3-carboxylic acid |
| **83** | 4-hydroxy-5-methyl-2-oxo-9-(pyrrolidin-1-yl)-1,2,5,6-tetrahydrobenzo[2,3]oxepino[4,5-b]pyridine-3-carboxylic acid |
| **93** | 4-hydroxy-7-methyl-2-oxo-9-(pyrrolidin-1-yl)-2,5,6,7-tetrahydro-1H-benzo[b]pyrido[2,3-d]azepine-3-carboxylic acid |
| **94** | 4-hydroxy-2-oxo-9-(pyrrolidin-1-yl)-2,5,6,7-tetrahydro-1H-benzo[b]pyrido[2,3-d]azepine-3-carboxylic acid |
| **107** | 4-hydroxy-5-methyl-2-oxo-8-(pyrrolidin-1-yl)-1,2,5,6-tetrahydrobenzo[h]quinoline-3-carboxylic acid |
| **108** | 8-(dimethylamino)-4-hydroxy-5-methyl-2-oxo-1,2,5,6-tetrahydrobenzo[h]quinoline-3-carboxylic acid |
| **126** | 4-hydroxy-2-oxo-10-(pyrrolidin-1-yl)-1,2,5,6,7,8-hexahydrobenzo[7,8]cycloocta[1,2-b]pyridine-3-carboxylic acid |
| **128** | 4-hydroxy-2-oxo-10-(propylamino)-1,2,5,6,7,8-hexahydrobenzo[7,8]cycloocta[1,2-b]pyridine-3-carboxylic acid |
| **129** | 10-(ethylamino)-4-hydroxy-2-oxo-1,2,5,6,7,8-hexahydrobenzo[7,8]cycloocta[1,2-b]pyridine-3-carboxylic acid |
| **185** | 9-(dimethylamino)-4-hydroxy-2-oxo-1,2,5,6-tetrahydrobenzo[2,3]thiepino[4,5-b]pyridine-3-carboxylic acid, and |
| **186** | 4-hydroxy-2-oxo-9-(pyrrolidin-1-yl)-1,2,5,6-tetrahydrobenzo[2,3]thiepino[4,5-b]pyridine-3-carboxylic acid; |

wherein the form of the compound is selected from a free acid, free base, salt, hydrate, solvate, clathrate, isotopologue, racemate, enantiomer, diastereomer, stereoisomer, polymorph or tautomer form thereof.
As part of the disclosure, a compound or a form thereof is selected from:

| | |
|---|---|
| **16** | 4-hydroxy-9-methyl-2-oxo-1,2,5,9-tetrahydropyrido[3',2':6,7]cyclohepta[1,2-f]indole-3-carboxylic acid |
| **17** | 4-hydroxy-9-methyl-2-oxo-1,2,5,6,7,9-hexahydropyrido[3',2':6,7]cyclohepta[1,2-f]indole-3-carboxylic acid |
| **18** | 8-fluoro-4-hydroxy-9-methyl-2-oxo-1,2,5,6,7,9-hexahydropyrido[3',2':6,7]cyclohepta[1,2-f]indole-3-carboxylic acid |
| **72** | 1-benzyl-8-chloro-5-methyl-2-oxo-2,5-dihydro-1H-chromeno[4,3-b]pyridine-3-carboxylic acid |
| **73** | 5-methyl-2-oxo-2,5-dihydro-1H-chromeno[4,3-b]pyridine-3-carboxylic acid |
| **87** | 4-hydroxy-9-methyl-2-oxo-2,5,6,9-tetrahydro-1H-pyrido[2',3':4,5]oxepino[3,2-f]indole-3-carboxylic acid |
| **106** | 4-hydroxy-5-methyl-2-oxo-1,2,5,6-tetrahydro-[1,3]dioxolo[4',5':4,5]benzo[1,2-h]quinoline-3-carboxylic acid |
| **130** | 1,9-dimethyl-2-oxo-1,2,5,9-tetrahydropyrido[3',2':6,7]cyclohepta[1,2-f]indole-3-carboxylic acid |
| **131** | 4-hydroxy-1,9-dimethyl-2-oxo-1,2,5,9-tetrahydropyrido[3',2':6,7]cyclohepta[1,2-f]indole-3-carboxylic acid |
| **132** | 1-ethyl-4-hydroxy-9-methyl-2-oxo-1,2,5,9-tetrahydropyrido[3',2':6,7]cyclohepta[1,2-f]indole-3-carboxylic acid |
| **133** | 1,9-dimethyl-2-oxo-1,2,5,6,7,9-hexahydropyrido[3',2':6,7]cyclohepta[1,2-f]indole-3-carboxylic acid |
| **134** | 4-hydroxy-1,9-dimethyl-2-oxo-1,2,5,6,7,9-hexahydropyrido[3',2':6,7]cyclohepta[1,2-f]indole-3-carboxylic acid |
| **135** | 4-hydroxy-1,9-dimethyl-2-oxo-1,2,5,6,7,9,10,11-octahydropyrido[3',2':6,7]cyclohepta[1,2-f]indole-3-carboxylic acid |

wherein the form of the compound is selected from a free acid, free base, salt, hydrate, solvate, clathrate, isotopologue, racemate, enantiomer, diastereomer, stereoisomer, polymorph or tautomer form thereof.

In another embodiment of the present description, the form is isolated for use.

In another embodiment of the present description, a compound or a form thereof is selected from:

| **Cpd** | **Name** |
|---|---|
| **6** | 9-(3-(dimethylamino)pyrrolidin-1-yl)-4-hydroxy-2-oxo-2,5,6,7-tetrahydro-1H-benzo[6,7]cyclohepta[1,2-b]pyridine-3-carboxylic acid hydrochloride |
| **8** | 4-hydroxy-9-(3-(methylamino)pyrrolidin-1-yl)-2-oxo-2,5,6,7-tetrahydro-1H-benzo[6,7]cyclohepta[1,2-b]pyridine-3-carboxylic acid hydrochloride |
| **9** | 9-((cis,cis)-6-amino-3-azabicyclo[3.1.0]hexan-3-yl)-4-hydroxy-2-oxo-2,5,6,7-tetrahydro-1H-benzo[6,7]cyclohepta[1,2-b]pyridine-3-carboxylic acid trifluoroacetate |
| **10** | 9-((cis,cis)-6-(benzyl(methyl)amino)-3-azabicyclo[3.1.0]hexan-3-yl)-4-hydroxy-2-oxo-2,5,6,7-tetrahydro-1H-benzo[6,7]cyclohepta[1,2-b]pyridine-3-carboxylic acid hydrochloride |
| **11** | 4-hydroxy-9-((cis,cis)-6-(methylamino)-3-azabicyclo[3.1.0]hexan-3-yl)-2-oxo-2,5,6,7-tetrahydro-1H-benzo[6,7]cyclohepta[1,2-b]pyridine-3-carboxylic acid hydrochloride |
| **12** | 9-(3-(dimethylamino)pyrrolidin-1-yl)-4-hydroxy-5-methyl-2-oxo-2,5,6,7-tetrahydro-1H-benzo[6,7]cyclohepta[1,2-b]pyridine-3-carboxylic acid hydrochloride |
| **13** | 9-((cis,cis)-6-amino-3-azabicyclo[3.1.0]hexan-3-yl)-4-hydroxy-5-methyl-2-oxo-2,5,6,7-tetrahydro-1H-benzo[6,7]cyclohepta[1,2-b]pyridine-3-carboxylic acid hydrochloride |
| **14** | 9-((cis,cis)-6-amino-3-azabicyclo[3.1.0]hexan-3-yl)-4,7-dihydroxy-2-oxo-2,5,6,7-tetrahydro-1H-benzo[6,7]cyclohepta[1,2-b]pyridine-3-carboxylic acid trifluoroacetate |
| **15** | 9-(3-(dimethylamino)pyrrolidin-1-yl)-4,7-dihydroxy-2-oxo-2,5,6,7-tetrahydro-1H-benzo[6,7]cyclohepta[1,2-b]pyridine-3-carboxylic acid hydrochloride |
| **19** | 2-((ethylamino)methyl)-8-hydroxy-1-methyl-6-oxo-1,5,6,9-tetrahydropyrido[3',2':4,5]cyclopenta[1,2-f]indole-7-carboxylic acid hydrochloride |
| **20** | 4-hydroxy-8-methyl-9-((methylamino)methyl)-2-oxo-2,5,6,8-tetrahydro-1H-indolo[6,5-h]quinoline-3-carboxylic acid hydrochloride |
| **21** | 9-(azetidin-1-ylmethyl)-4-hydroxy-8-methyl-2-oxo-2,5,6,8-tetrahydro-1H-indolo[6,5-h]quinoline-3-carboxylic acid hydrochloride |
| **22** | 4-hydroxy-9-methyl-10-((methylamino)methyl)-2-oxo-1,2,5,6,7,9-hexahydropyrido[3',2':6,7]cyclohepta[1,2-f]indole-3-carboxylic acid hydrochloride |
| **23** | 10-((cyclobutylamino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **24** | 10-(azetidin-1-ylmethyl)-4-hydroxy-9-methyl-2-oxo-1,2,5,6,7,9-hexahydropyrido[3',2':6,7]cyclohepta[1,2-f]indole-3-carboxylic acid hydrochloride |
| **25** | 10-((ethylamino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **26** | 4-hydroxy-10-((isopropylamino)methyl)-9-methyl-2-oxo-1,2,5,6,7,9-hexahydropyrido[3',2':6,7]cyclohepta[1,2-f]indole-3-carboxylic acid hydrochloride |
| **27** | 10-((tert-butylamino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **28** | 4-hydroxy-10-((4-hydroxypiperidin-1-yl)methyl)-9-methyl-2-oxo-1,2,5,6,7,9-exahydropyrido[3',2':6,7]cyclohepta[1,2-f]indole-3-carboxylic acid hydrochloride |
| **29** | 10-((4-aminopiperidin-1-yl)methyl)-4-hydroxy-9-methyl-2-oxo-1,2,5,6,7,9-exahydropyrido[3',2':6,7]cyclohepta[1,2-f]indole-3-carboxylic acid dihydrochloride |
| **30** | 10-((4-(dimethylamino)piperidin-1-yl)methyl)-4-hydroxy-9-methyl-2-oxo-1,2,5,6,7,9-hexahydropyrido[3',2':6,7]cyclohepta[1,2-f]indole-3-carboxylic acid dihydrochloride |
| **31** | 10-((3-aminopiperidin-1-yl)methyl)-4-hydroxy-9-methyl-2-oxo-1,2,5,6,7,9-hexahydropyrido[3',2':6,7]cyclohepta[1,2-f]indole-3-carboxylic acid dihydrochloride |
| **32** | 10-(((cyclopropylmethyl)amino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **33** | 4-hydroxy-9-methyl-10-(((1-methylcyclopropyl)amino)methyl)-2-oxo-1,2,5,6,7,9-hexahydropyrido[3',2':6,7]cyclohepta[1,2-f]indole-3-carboxylic acid hydrochloride |
| **34** | 10-((benzylamino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **35** | 10-(aminomethyl)-4-hydroxy-9-methyl-2-oxo-1,2,5,6,7,9-hexahydropyrido[3',2':6,7]cyclohepta[1,2-f]indole-3-carboxylic acid hydrochloride |
| **36** | 10-((cyclopropylamino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **37** | 10-(((cyclobutylmethyl)amino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **38** | 4-hydroxy-9-methyl-10-((((2-methylcyclopropyl)methyl)amino)methyl)-2-oxo-1,2,5,6,7,9-hexahydropyrido[3',2':6,7]cyclohepta[1,2-f]indole-3-carboxylic acid hydrochloride |
| **39** | 4-hydroxy-9-methyl-2-oxo-10-(((pyridin-4-ylmethyl)amino)methyl)-1,2,5,6,7,9-hexahydropyrido[3',2':6,7]cyclohepta[1,2-f]indole-3-carboxylic acid hydrochloride |
| **40** | 4-hydroxy-9-methyl-2-oxo-10-(piperidin-1-ylmethyl)-1,2,5,6,7,9-hexahydropyrido[3',2':6,7]cyclohepta[1,2-f]indole-3-carboxylic acid hydrochloride |
| **41** | 4-hydroxy-9-methyl-10-(morpholinomethyl)-2-oxo-1,2,5,6,7,9-hexahydropyrido[3',2':6,7]cyclohepta[1,2-f]indole-3-carboxylic acid hydrochloride |
| **42** | 4-hydroxy-9-methyl-10-((4-methylpiperazin-1-yl)methyl)-2-oxo-1,2,5,6,7,9-hexahydropyrido[3',2':6,7]cyclohepta[1,2-f]indole-3-carboxylic acid dihydrochloride |
| **43** | 10-((diethylamino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **44** | 4-hydroxy-9-methyl-2-oxo-10-((propylamino)methyl)-1,2,5,6,7,9-hexahydropyrido[3',2':6,7]cyclohepta[1,2-f]indole-3-carboxylic acid hydrochloride |
| **45** | 4-hydroxy-9-methyl-2-oxo-10-((prop-2-yn-1-ylamino)methyl)-1,2,5,6,7,9-hexahydropyrido[3',2':6,7]cyclohepta[1,2-f]indole-3-carboxylic acid hydrochloride |
| **46** | (R)-10-((3-fluoropyrrolidin-1-yl)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **47** | 10-((3-(dimethylamino)pyrrolidin-1-yl)methyl)-4-hydroxy-9-methyl-2-oxo-1,2,5,6,7,9-hexahydropyrido[3',2':6,7]cyclohepta[1,2-f]indole-3-carboxylic acid dihydrochloride |
| **48** | 10-((dimethylamino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **49** | (S)-10-((3-aminopyrrolidin-1-yl)methyl)-4-hydroxy-9-methyl-2-oxo-1,2,5,6,7,9-hexahydropyrido[3',2':6,7]cyclohepta[1,2-f]indole-3-carboxylic acid dihydrochloride |
| **50** | 4-hydroxy-9-methyl-2-oxo-10-(pyrrolidin-1-ylmethyl)-1,2,5,6,7,9-hexahydropyrido[3',2':6,7]cyclohepta[1,2-f]indole-3-carboxylic acid hydrochloride |
| **51** | 4-hydroxy-10-((3-hydroxypyrrolidin-1-yl)methyl)-9-methyl-2-oxo-1,2,5,6,7,9-hexahydropyrido[3',2':6,7]cyclohepta[1,2-f]indole-3-carboxylic acid hydrochloride |
| **52** | 4-hydroxy-9-methyl-2-oxo-10-(((pyridin-3-ylmethyl)amino)methyl)-1,2,5,6,7,9-hexahydropyrido[3',2':6,7]cyclohepta[1,2-f]indole-3-carboxylic acid hydrochloride |
| **53** | 9-methyl-10-((methylamino)methyl)-2-oxo-1,2,5,6,7,9-hexahydropyrido[3',2':6,7]cyclohepta[1,2-f]indole-3-carboxylic acid hydrochloride |
| **54** | 10-((ethylamino)methyl)-9-methyl-2-oxo-1,2,5,6,7,9-hexahydropyrido[3',2':6,7]cyclohepta[1,2-f]indole-3-carboxylic acid hydrochloride |
| **55** | 10-((isopropylamino)methyl)-9-methyl-2-oxo-1,2,5,6,7,9-hexahydropyrido[3',2':6,7]cyclohepta[1,2-f]indole-3-carboxylic acid hydrochloride |
| **56** | 10-((tert-butylamino)methyl)-9-methyl-2-oxo-1,2,5,6,7,9-hexahydropyrido[3',2':6,7]cyclohepta[1,2-f]indole-3-carboxylic acid hydrochloride |
| **57** | 10-(azetidin-1-ylmethyl)-9-methyl-2-oxo-1,2,5,6,7,9-hexahydropyrido[3',2':6,7]cyclohepta[1,2-f]indole-3-carboxylic acid hydrochloride |
| **58** | 4-hydroxy-10-methyl-11-((methylamino)methyl)-2-oxo-2,5,6,7,8,10-hexahydro-1H-pyrido[3',2':7,8]cycloocta[1,2-f]indole-3-carboxylic acid hydrochloride |
| **59** | 11-((ethylamino)methyl)-4-hydroxy-10-methyl-2-oxo-2,5,6,7,8,10-hexahydro-1H-pyrido[3',2':7,8]cycloocta[1,2-f]indole-3-carboxylic acid hydrochloride |
| **60** | 7-hydroxy-1-methyl-2-((methylamino)methyl)-9-oxo-1,4,5,6,9,10-hexahydropyrido[2',3':3,4]cyclohepta[1,2-e]indole-8-carboxylic acid hydrochloride |
| **61** | 2-((ethylamino)methyl)-7-hydroxy-1-methyl-9-oxo-1,4,5,6,9,10-hexahydropyrido[2',3':3,4]cyclohepta[1,2-e]indole-8-carboxylic acid hydrochloride |
| **62** | 4-hydroxy-7,9-dimethyl-10-((methylamino)methyl)-2-oxo-1,2,5,6,7,9-hexahydropyrido[3',2':6,7]cyclohepta[1,2-f]indole-3-carboxylic acid hydrochloride |
| **63** | 10-((ethylamino)methyl)-4-hydroxy-7,9-dimethyl-2-oxo-1,2,5,6,7,9-hexahydropyrido[3',2':6,7]cyclohepta[1,2-f]indole-3-carboxylic acid hydrochloride |
| **64** | 4-hydroxy-10-((isopropylamino)methyl)-7,9-dimethyl-2-oxo-1,2,5,6,7,9-hexahydropyrido[3',2':6,7]cyclohepta[1,2-f]indole-3-carboxylic acid hydrochloride |
| **65** | 10-((tert-butylamino)methyl)-4-hydroxy-7,9-dimethyl-2-oxo-1,2,5,6,7,9-hexahydropyrido[3',2':6,7]cyclohepta[1,2-f]indole-3-carboxylic acid hydrochloride |
| **66** | 4-hydroxy-7,9-dimethyl-10-(((1-methylcyclopropyl)amino)methyl)-2-oxo-1,2,5,6,7,9-hexahydropyrido[3',2':6,7]cyclohepta[1,2-f]indole-3-carboxylic acid hydrochloride |
| **67** | 10-((ethylamino)methyl)-4-hydroxy-5,9-dimethyl-2-oxo-1,2,5,6,7,9-hexahydropyrido[3',2':6,7]cyclohepta[1,2-f]indole-3-carboxylic acid hydrochloride |
| **68** | 4-hydroxy-9-methyl-10-((methylamino)methyl)-2-oxo-1,2,5,9-tetrahydropyrido[3',2':6,7]cyclohepta[1,2-f]indole-3-carboxylic acid hydrochloride |
| **69** | 10-((ethylamino)methyl)-4-hydroxy-9-methyl-2-oxo-1,2,5,9-tetrahydropyrido[3',2':6,7]cyclohepta[1,2-f]indole-3-carboxylic acid hydrochloride |
| **70** | 10-((ethylamino)methyl)-4-hydroxy-7,9-dimethyl-2-oxo-1,2,5,9-tetrahydropyrido[3',2':6,7]cyclohepta[1,2-f]indole-3-carboxylic acid hydrochloride |
| **71** | (cis)-10-((ethylamino)methyl)-4,6,7-trihydroxy-9-methyl-2-oxo-1,2,5,6,7,9-hexahydropyrido[3',2':6,7]cyclohepta[1,2-f]indole-3-carboxylic acid hydrochloride |
| **75** | 8-(3-(dimethylamino)pyrrolidin-1-yl)-5-methyl-2-oxo-2,5-dihydro-1H-chromeno[4,3-b]pyridine-3-carboxylic acid hydrochloride |
| **78** | 9-(3-(dimethylamino)pyrrolidin-1-yl)-4-hydroxy-2-oxo-1,2,5,6-tetrahydrobenzo[2,3]oxepino[4,5-b]pyridine-3-carboxylic acid hydrochloride |
| **79** | 9-((cis,cis)-6-(dibenzylamino)-3-azabicyclo[3.1.0]hexan-3-yl)-4-hydroxy-2-oxo-1,2,5,6-tetrahydrobenzo[2,3]oxepino[4,5-b]pyridine-3-carboxylic acid trifluoroacetate |
| **80** | 9-((cis,cis)-6-amino-3-azabicyclo[3.1.0]hexan-3-yl)-4-hydroxy-2-oxo-1,2,5,6-tetrahydrobenzo[2,3]oxepino[4,5-b]pyridine-3-carboxylic acid hydrochloride |
| **82** | 9-(3-(dimethylamino)pyrrolidin-1-yl)-2-oxo-1,2,5,6-tetrahydrobenzo[2,3]oxepino[4,5-b]pyridine-3-carboxylic acid hydrochloride |
| **84** | 9-(3-(dimethylamino)pyrrolidin-1-yl)-4-hydroxy-5-methyl-2-oxo-1,2,5,6-tetrahydrobenzo[2,3]oxepino[4,5-b]pyridine-3-carboxylic acid hydrochloride |
| **85** | 9-((cis,cis)-6-(dibenzylamino)-3-azabicyclo[3.1.0]hexan-3-yl)-4-hydroxy-5-methyl-2-oxo-1,2,5,6-tetrahydrobenzo[2,3]oxepino[4,5-b]pyridine-3-carboxylic acid hydrochloride |
| **86** | 9-((cis,cis)-6-amino-3-azabicyclo[3.1.0]hexan-3-yl)-4-hydroxy-5-methyl-2-oxo-1,2,5,6-tetrahydrobenzo[2,3]oxepino[4,5-b]pyridine-3-carboxylic acid hydrochloride |
| **88** | 4-hydroxy-9-methyl-10-((methylamino)methyl)-2-oxo-2,5,6,9-tetrahydro-1H-pyrido[2',3':4,5]oxepino[3,2-f]indole-3-carboxylic acid hydrochloride |
| **89** | 10-((ethylamino)methyl)-4-hydroxy-9-methyl-2-oxo-2,5,6,9-tetrahydro-1H-pyrido[2',3':4,5]oxepino[3,2-f]indole-3-carboxylic acid hydrochloride |
| **90** | 4-hydroxy-10-((isopropylamino)methyl)-9-methyl-2-oxo-2,5,6,9-tetrahydro-1H-pyrido[2',3':4,5]oxepino[3,2-f]indole-3-carboxylic acid hydrochloride |
| **91** | 10-(azetidin-1-ylmethyl)-4-hydroxy-9-methyl-2-oxo-2,5,6,9-tetrahydro-1H-pyrido[2',3':4,5]oxepino[3,2-f]indole-3-carboxylic acid hydrochloride |
| **92** | 10-((ethylamino)methyl)-4-hydroxy-5,9-dimethyl-2-oxo-2,5,6,9-tetrahydro-1H-pyrido[2',3':4,5]oxepino[3,2-f]indole-3-carboxylic acid hydrochloride |
| **95** | 9-(3-(dimethylamino)pyrrolidin-1-yl)-4-hydroxy-2-oxo-2,5,6,7-tetrahydro-1H-benzo[b]pyrido[2,3-d]azepine-3-carboxylic acid hydrochloride |
| **96** | 4-hydroxy-9-(4-methylpiperazin-1-yl)-2-oxo-2,5,6,7-tetrahydro-1H-benzo[b]pyrido[2,3-d]azepine-3-carboxylic acid hydrochloride |
| 9**7** | 9-((cis,cis)-6-amino-3-azabicyclo[3.1.0]hexan-3-yl)-4-hydroxy-2-oxo-2,5,6,7-tetrahydro-1H-benzo[b]pyrido[2,3-d]azepine-3-carboxylic acid hydrochloride |
| **98** | 9-(hexahydro-1H-pyrrolo[3,4-b]pyridin-6(2H)-yl)-4-hydroxy-2-oxo-2,5,6,7-tetrahydro-1H-benzo[b]pyrido[2,3-d]azepine-3-carboxylic acid hydrochloride |
| **99** | 4-hydroxy-9-methyl-10-((methylamino)methyl)-2-oxo-1,2,5,6,7,9-hexahydropyrido[2',3':4,5]azepino[3,2-f]indole-3-carboxylic acid hydrochloride |
| **100** | 10-((ethylamino)methyl)-4-hydroxy-9-methyl-2-oxo-1,2,5,6,7,9-hexahydropyrido[2',3':4,5]azepino[3,2-f]indole-3-carboxylic acid hydrochloride |
| **101** | 4-hydroxy-10-((isopropylamino)methyl)-9-methyl-2-oxo-1,2,5,6,7,9-hexahydropyrido[2',3':4,5]azepino[3,2-f]indole-3-carboxylic acid hydrochloride |
| **102** | 10-((tert-butylamino)methyl)-4-hydroxy-9-methyl-2-oxo-1,2,5,6,7,9-hexahydropyrido[2',3':4,5]azepino[3,2-f]indole-3-carboxylic acid hydrochloride |
| **103** | 4-hydroxy-10-methyl-11-((methylamino)methyl)-2-oxo-2,5,6,7,8,10-hexahydro-1H-pyrido[2',3':4,5]azocino[3,2-f]indole-3-carboxylic acid hydrochloride |
| **104** | 11-((ethylamino)methyl)-4-hydroxy-10-methyl-2-oxo-2,5,6,7,8,10-hexahydro-1h-pyrido[2',3':4,5]azocino[3,2-f]indole-3-carboxylic acid hydrochloride |
| **105** | 4-hydroxy-11-((isopropylamino)methyl)-10-methyl-2-oxo-2,5,6,7,8,10-hexahydro-1H-pyrido[2',3':4,5]azocino[3,2-f]indole-3-carboxylic acid hydrochloride |
| **109** | 8-(3-(dimethylamino)pyrrolidin-1-yl)-4-hydroxy-5-methyl-2-oxo-1,2,5,6-tetrahydrobenzo[h]quinoline-3-carboxylic acid hydrochloride |
| **110** | 8-((cis,cis)-6-amino-3-azabicyclo[3.1.0]hexan-3-yl)-4-hydroxy-5-methyl-2-oxo-1,2,5,6-tetrahydrobenzo[h]quinoline-3-carboxylic acid trifluoroacetate |
| **111** | 9-(1,4-diazepan-1-yl)-4-hydroxy-2-oxo-2,5,6,7-tetrahydro-1H-benzo[6,7]cyclohepta[1,2-b]pyridine-3-carboxylic acid hydrochloride |
| **112** | 4-hydroxy-9-(4-methyl-1,4-diazepan-1-yl)-2-oxo-2,5,6,7-tetrahydro-1H-benzo[6,7]cyclohepta[1,2-b]pyridine-3-carboxylic acid hydrochloride |
| **113** | 9-((2-(dimethylamino)ethyl)amino)-4-hydroxy-2-oxo-2,5,6,7-tetrahydro-1H-benzo[6,7]cyclohepta[1,2-b]pyridine-3-carboxylic acid hydrochloride |
| **114** | 9-((4aR,7aR)-hexahydro-1H-pyrrolo[3,4-b]pyridin-6(2H)-yl)-4-hydroxy-2-oxo-2,5,6,7-tetrahydro-1H-benzo[6,7]cyclohepta[1,2-b]pyridine-3-carboxylic acid hydrochloride |
| **115** | 4-hydroxy-9-((4aR,7aR)-1-methylhexahydro-1H-pyrrolo[3,4-b]pyridin-6(2H)-yl)-2-oxo-2,5,6,7-tetrahydro-1H-benzo[6,7]cyclohepta[1,2-b]pyridine-3-carboxylic acid hydrochloride |
| **116** | 9-(4-(dimethylamino)piperidin-1-yl)-4-hydroxy-2-oxo-2,5,6,7-tetrahydro-1H-benzo[6,7]cyclohepta[1,2-b]pyridine-3-carboxylic acid hydrochloride |
| **117** | 9-(3-(dimethylamino)piperidin-1-yl)-4-hydroxy-2-oxo-2,5,6,7-tetrahydro-1H-benzo[6,7]cyclohepta[1,2-b]pyridine-3-carboxylic acid hydrochloride |
| **118** | 4-hydroxy-2-oxo-9-(piperidin-4-ylamino)-2,5,6,7-tetrahydro-1H-benzo[6,7]cyclohepta[1,2-b]pyridine-3-carboxylic acid hydrochloride |
| **119** | 4-hydroxy-2-oxo-9-(piperazin-1-yl)-2,5,6,7-tetrahydro-1H-benzo[6,7]cyclohepta[1,2-b]pyridine-3-carboxylic acid hydrochloride |
| **120** | 4-hydroxy-9-(4-methylpiperazin-1-yl)-2-oxo-2,5,6,7-tetrahydro-1H-benzo[6,7]cyclohepta[1,2-b]pyridine-3-carboxylic acid hydrochloride |
| **121** | 4-hydroxy-2-oxo-9-(2,6-diazaspiro[3.4]octan-6-yl)-2,5,6,7-tetrahydro-1H-benzo[6,7]cyclohepta[1,2-b]pyridine-3-carboxylic acid hydrochloride |
| **122** | 4-hydroxy-2-oxo-9-(2,7-diazaspiro[4.4]nonan-2-yl)-2,5,6,7-tetrahydro-1H-benzo[6,7]cyclohepta[1,2-b]pyridine-3-carboxylic acid hydrochloride |
| **123** | 4-hydroxy-9-(7-methyl-2,7-diazaspiro[4.4]nonan-2-yl)-2-oxo-2,5,6,7-tetrahydro-1H-benzo[6,7]cyclohepta[1,2-b]pyridine-3-carboxylic acid hydrochloride |
| **124** | 9-((cis,cis)-6-amino-3-azabicyclo[3.1.0]hexan-3-yl)-10,11-difluoro-4-hydroxy-2-oxo-2,5,6,7-tetrahydro-1H-benzo[6,7]cyclohepta[1,2-b]pyridine-3-carboxylic acid hydrochloride |
| 1**25** | 9-((cis,cis)-6-amino-3-azabicyclo[3.1.0]hexan-3-yl)-11-fluoro-4-hydroxy-2-oxo-2,5,6,7-tetrahydro-1H-benzo[6,7]cyclohepta[1,2-b]pyridine-3-carboxylic acid hydrochloride |
| **127** | 10-((cis,cis)-6-amino-3-azabicyclo[3.1.0]hexan-3-yl)-4-hydroxy-2-oxo-1,2,5,6,7,8-hexahydrobenzo[7,8]cycloocta[1,2-b]pyridine-3-carboxylic acid hydrochloride |
| **136** | 10-((butylamino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **137** | 4-hydroxy-9-methyl-2-oxo-10-((pentylamino)methyl)-1,2,5,6,7,9-hexahydropyrido[3',2':6,7]cyclohepta[1,2-f]indole-3-carboxylic acid hydrochloride |
| **138** | 10-((hexylamino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **139** | 10-((heptylamino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **140** | 4-hydroxy-9-methyl-10-((octylamino)methyl)-2-oxo-1,2,5,6,7,9-hexahydropyrido[3',2':6,7]cyclohepta[1,2-f]indole-3-carboxylic acid hydrochloride |
| **141** | 4-hydroxy-9-methyl-10-((nonylamino)methyl)-2-oxo-1,2,5,6,7,9-hexahydropyrido[3',2':6,7]cyclohepta[1,2-f]indole-3-carboxylic acid hydrochloride |
| **142** | 10-(((2-(dimethylamino)ethyl)amino)methyl)-4-hydroxy-9-methyl-2-oxo-1,2,5,6,7,9-hexahydropyrido[3',2':6,7]cyclohepta[1,2-f]indole-3-carboxylic acid dihydrochloride |
| **143** | 4-hydroxy-9-methyl-10-(((2-(methylamino)ethyl)amino)methyl)-2-oxo-1,2,5,6,7,9-hexahydropyrido[3',2':6,7]cyclohepta[1,2-f]indole-3-carboxylic acid dihydrochloride |
| **144** | 10-(((2-aminoethyl)amino)methyl)-4-hydroxy-9-methyl-2-oxo-1,2,5,6,7,9-hexahydropyrido[3',2':6,7]cyclohepta[1,2-f]indole-3-carboxylic acid dihydrochloride |
| **145** | 10-(((2-(dimethylamino)ethyl)(methyl)amino)methyl)-4-hydroxy-9-methyl-2-oxo-1,2,5,6,7,9-hexahydropyrido[3',2':6,7]cyclohepta[1,2-f]indole-3-carboxylic acid dihydrochloride |
| **146** | 10-((sec-butylamino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **147** | 4-hydroxy-10-(((2-hydroxyethyl)amino)methyl)-9-methyl-2-oxo-1,2,5,6,7,9-hexahydropyrido[3',2':6,7]cyclohepta[1,2-f]indole-3-carboxylic acid hydrochloride |
| **148** | 4-hydroxy-10-(((2-methoxyethyl)amino)methyl)-9-methyl-2-oxo-1,2,5,6,7,9-hexahydropyrido[3',2':6,7]cyclohepta[1,2-f]indole-3-carboxylic acid hydrochloride |
| **149** | 4-hydroxy-10-(((2-hydroxyethyl)(methyl)amino)methyl)-9-methyl-2-oxo-1,2,5,6,7,9-hexahydropyrido[3',2':6,7]cyclohepta[1,2-f]indole-3-carboxylic acid hydrochloride |
| **150** | 4-hydroxy-10-(((1-methoxypropan-2-yl)amino)methyl)-9-methyl-2-oxo-1,2,5,6,7,9-hexahydropyrido[3',2':6,7]cyclohepta[1,2-f]indole-3-carboxylic acid hydrochloride |
| **151** | 4-hydroxy-10-(((1-hydroxypropan-2-yl)amino)methyl)-9-methyl-2-oxo-1,2,5,6,7,9-hexahydropyrido[3',2':6,7]cyclohepta[1,2-f]indole-3-carboxylic acid hydrochloride |
| **152** | 4-hydroxy-9-methyl-2-oxo-10-(((2-(pyrrolidin-1-yl)ethyl)amino)methyl)-1,2,5,6,7,9-hexahydropyrido[3',2':6,7]cyclohepta[1,2-f]indole-3-carboxylic acid dihydrochloride |
| **153** | 10-((allylamino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **154** | 4-hydroxy-10-((isobutylamino)methyl)-9-methyl-2-oxo-1,2,5,6,7,9-hexahydropyrido[3',2':6,7]cyclohepta[1,2-f]indole-3-carboxylic acid hydrochloride |
| **155** | 4-hydroxy-9-methyl-10-((neopentylamino)methyl)-2-oxo-1,2,5,6,7,9-hexahydropyrido[3',2':6,7]cyclohepta[1,2-f]indole-3-carboxylic acid hydrochloride |
| **156** | 4-hydroxy-9-methyl-10-((4-methyl-1,4-diazepan-1-yl)methyl)-2-oxo-1,2,5,6,7,9-hexahydropyrido[3',2':6,7]cyclohepta[1,2-f]indole-3-carboxylic acid dihydrochloride |
| **157** | 4-hydroxy-9-methyl-10-(((1-methylpiperidin-4-yl)amino)methyl)-2-oxo-1,2,5,6,7,9-hexahydropyrido[3',2':6,7]cyclohepta[1,2-f]indole-3-carboxylic acid dihydrochloride |
| **158** | 4-hydroxy-10-((3-methoxypyrrolidin-1-yl)methyl)-9-methyl-2-oxo-1,2,5,6,7,9-hexahydropyrido[3',2':6,7]cyclohepta[1,2-f]indole-3-carboxylic acid hydrochloride |
| **159** | 10-((3-acetamidopyrrolidin-1-yl)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **160** | 10-(((1-(dimethylamino)propan-2-yl)amino)methyl)-4-hydroxy-9-methyl-2-oxo-1,2,5,6,7,9-hexahydropyrido[3',2':6,7]cyclohepta[1,2-f]indole-3-carboxylic acid dihydrochloride |
| **161** | 4-hydroxy-9-methyl-2-oxo-10-((((tetrahydrofuran-2-yl)methyl)amino)methyl)-1,2,5,6,7,9-hexahydropyrido[3',2':6,7]cyclohepta[1,2-f]indole-3-carboxylic acid hydrochloride |
| **162** | 4-hydroxy-9-methyl-10-((1-methylhexahydropyrrolo[3,4-b]pyrrol-5(1H)-yl)methyl)-2-oxo-1,2,5,6,7,9-hexahydropyrido[3',2':6,7]cyclohepta[1,2-f]indole-3-carboxylic acid dihydrochloride |
| **163** | 10-(((2-(dimethylamino)-2-oxoethyl)amino)methyl)-4-hydroxy-9-methyl-2-oxo-1,2,5,6,7,9-hexahydropyrido[3',2':6,7]cyclohepta[1,2-f]indole-3-carboxylic acid hydrochloride |
| **164** | 10-(2-(ethylamino)ethyl)-4-hydroxy-9-methyl-2-oxo-1,2,5,9-tetrahydropyrido[3',2':6,7]cyclohepta[1,2-f]indole-3-carboxylic acid trifluoroacetate |
| **165** | 10-(2-(ethylamino)ethyl)-4-hydroxy-9-methyl-2-oxo-1,2,5,6,7,9-hexahydropyrido[3',2':6,7]cyclohepta[1,2-f]indole-3-carboxylic acid trifluoroacetate |
| **166** | 4-hydroxy-9-(((cis)-octahydrocyclopenta[c]pyrrol-4-yl)-(cis)-amino)-2-oxo-1,2,5,6-tetrahydrobenzo[2,3]oxepino[4,5-b]pyridine-3-carboxylic acid hydrochloride |
| **167** | 4-hydroxy-9-methyl-10-(((1-methylcyclopropyl)amino)methyl)-2-oxo-2,5,6,9-tetrahydro-1H-pyrido[2',3':4,5]oxepino[3,2-f]indole-3-carboxylic acid hydrochloride |
| **168** | 10-((sec-butylamino)methyl)-4-hydroxy-9-methyl-2-oxo-2,5,6,9-tetrahydro-1H-pyrido[2',3':4,5]oxepino[3,2-f]indole-3-carboxylic acid hydrochloride |
| **169** | 4-hydroxy-9-methyl-2-oxo-10-((propylamino)methyl)-2,5,6,9-tetrahydro-1H-pyrido[2',3':4,5]oxepino[3,2-f]indole-3-carboxylic acid hydrochloride |
| **170** | 10-(((2,4-dimethoxybenzyl)amino)methyl)-4-hydroxy-9-methyl-2-oxo-2,5,6,9-tetrahydro-1H-pyrido[2',3':4,5]oxepino[3,2-f]indole-3-carboxylic acid hydrochloride |
| **171** | 10-((cyclopropylamino)methyl)-4-hydroxy-9-methyl-2-oxo-2,5,6,9-tetrahydro-1H-pyrido[2',3':4,5]oxepino[3,2-f]indole-3-carboxylic acid hydrochloride |
| **172** | 10-((cyclobutylamino)methyl)-4-hydroxy-9-methyl-2-oxo-2,5,6,9-tetrahydro-1H-pyrido[2',3':4,5]oxepino[3,2-f]indole-3-carboxylic acid hydrochloride |
| **173** | 10-((dimethylamino)methyl)-4-hydroxy-9-methyl-2-oxo-2,5,6,9-tetrahydro-1H-pyrido[2',3':4,5]oxepino[3,2-f]indole-3-carboxylic acid hydrochloride |
| **174** | 4-hydroxy-9-methyl-2-oxo-10-(pyrrolidin-1-ylmethyl)-2,5,6,9-tetrahydro-1H-pyrido[2',3':4,5]oxepino[3,2-f]indole-3-carboxylic acid hydrochloride |
| **175** | 10-((tert-butylamino)methyl)-4-hydroxy-9-methyl-2-oxo-2,5,6,9-tetrahydro-1H-pyrido[2',3':4,5]oxepino[3,2-f]indole-3-carboxylic acid hydrochloride |
| **176** | 4-hydroxy-9-methyl-10-((4-methylpiperazin-1-yl)methyl)-2-oxo-2,5,6,9-tetrahydro-1H-pyrido[2',3':4,5]oxepino[3,2-f]indole-3-carboxylic acid dihydrochloride |
| **177** | 4-hydroxy-10-(((2-hydroxyethyl)amino)methyl)-9-methyl-2-oxo-2,5,6,9-tetrahydro-1H-pyrido[2',3':4,5]oxepino[3,2-f]indole-3-carboxylic acid hydrochloride |
| **178** | 4-hydroxy-10-(((1-hydroxypropan-2-yl)amino)methyl)-9-methyl-2-oxo-2,5,6,9-tetrahydro-1H-pyrido[2',3':4,5]oxepino[3,2-f]indole-3-carboxylic acid hydrochloride |
| **179** | 4-hydroxy-9-methyl-2-oxo-10-(((2-(pyrrolidin-1-yl)ethyl)amino)methyl)-2,5,6,9-tetrahydro-1H-pyrido[2',3':4,5]oxepino[3,2-f]indole-3-carboxylic acid dihydrochloride |
| **180** | 10-(((2-aminoethyl)amino)methyl)-4-hydroxy-9-methyl-2-oxo-2,5,6,9-tetrahydro-1H-pyrido[2',3':4,5]oxepino[3,2-f]indole-3-carboxylic acid dihydrochloride |
| **181** | 4-hydroxy-9-methyl-10-(((2-(methylamino)ethyl)amino)methyl)-2-oxo-2,5,6,9-tetrahydro-1H-pyrido[2',3':4,5]oxepino[3,2-f]indole-3-carboxylic acid dihydrochloride |
| **182** | 10-(((2-(dimethylamino)ethyl)amino)methyl)-4-hydroxy-9-methyl-2-oxo-2,5,6,9-tetrahydro-1H-pyrido[2',3':4,5]oxepino[3,2-f]indole-3-carboxylic acid dihydrochloride |
| **183** | 4-hydroxy-10-(((2-methoxyethyl)amino)methyl)-9-methyl-2-oxo-2,5,6,9-tetrahydro-1H-pyrido[2',3':4,5]oxepino[3,2-f]indole-3-carboxylic acid hydrochloride, and |
| **184** | 4-hydroxy-10-(((1-methoxypropan-2-yl)amino)methyl)-9-methyl-2-oxo-2,5,6,9-tetrahydro-1H-pyrido[2',3':4,5]oxepino[3,2-f]indole-3-carboxylic acid hydrochloride; |

wherein the form of the compound is selected from a free acid, free base, hydrate, solvate, clathrate, isotopologue, racemate, enantiomer, diastereomer, stereoisomer, polymorph or tautomer form thereof.

In another embodiment of the present description, the form is isolated for use.

### Chemical Definitions

The chemical terms used above and throughout the description herein, unless specifically defined otherwise, shall be understood by one of ordinary skill in the art to have the following indicated meanings.

As used herein, the term "C₁₋₁₀alkyl" generally refers to saturated hydrocarbon radicals having from one to ten carbon atoms in a straight or branched chain configuration, including, without limitation, methyl, ethyl, n-propyl, isopropyl, n-butyl, isobutyl, sec-butyl, tert-butyl, n-pentyl, n-hexyl, n-heptyl, n-octyl, n-nonyl, n-decyl and the like. In some embodiments, C₁₋₁₀alkyl includes C₁₋₈alkyl, C₁₋₆alkyl, C₁₋₄alkyl and the like. A C₁₋₁₀alkyl radical may be optionally substituted where allowed by available valences.

As used herein, the term "C₂₋₈alkenyl" generally refers to partially unsaturated hydrocarbon radicals having from two to eight carbon atoms in a straight or branched chain configuration and one or more carbon-carbon double bonds therein, including, without limitation, ethenyl, allyl, propenyl and the like. In some embodiments, C₂₋₈alkenyl includes C₂₋₆alkenyl, C₂₋₄alkenyl and the like. A C₂₋₈alkenyl radical may be optionally substituted where allowed by available valences.

As used herein, the term "C₂₋₈alkynyl" generally refers to partially unsaturated hydrocarbon radicals having from two to eight carbon atoms in a straight or branched chain configuration and one or more carbon-carbon triple bonds therein, including, without limitation, ethynyl, propynyl and the like. In some embodiments, C₂₋₈alkynyl includes C₂₋₆alkynyl, C₂₋₄alkynyl and the like. A C₂₋₈alkynyl radical may be optionally substituted where allowed by available valences.

As used herein, the term "C₁₋₈alkoxy" generally refers to saturated hydrocarbon radicals having from one to eight carbon atoms in a straight or branched chain configuration of the formula: -O-C₁₋₈salkyl, including, without limitation, methoxy, ethoxy, n-propoxy, isopropoxy, n-butoxy, isobutoxy, sec-butoxy, tert-butoxy, n-pentoxy, n-hexoxy and the like. In some embodiments, C₁₋₈alkoxy includes C₁₋₆alkoxy, C₁₋₄alkoxy and the like. A C₁₋₈alkoxy radical may be optionally substituted where allowed by available valences.

As used herein, the term "C₃₋₁₄cycloalkyl" generally refers to a saturated monocyclic, bicyclic or polycyclic hydrocarbon radical, including, without limitation, cyclopropyl, cyclobutyl, cyclopentyl, cyclohexyl, cycloheptyl, cyclooctyl, 1H-indanyl, indenyl, tetrahydro-naphthalenyl and the like. In some embodiments, C₃₋₁₄cycloalkyl includes C₃₋₈cycloalkyl, C₅₋₈cycloalkyl, C₃₋₁₀cycloalkyl and the like. A C₃₋₁₄cycloalkyl radical may be optionally substituted where allowed by available valences.

As used herein, the term "C₃₋₁₄cycloalkenyl" generally refers to a partially unsaturated monocyclic, bicyclic or polycyclic hydrocarbon radical having one or more chemically stable carbon-carbon double bonds therein, including, without limitation, cyclopropenyl, cyclobutenyl, cyclopentenyl, cyclohexenyl, cycloheptenyl, cyclooctenyl and the like. In some embodiments, C₃₋₁₄cycloalkenyl includes C₃₋₈cycloalkenyl, C₅₋₈cycloalkenyl, C₃₋₁₀cycloalkenyl and the like. A C₃₋₁₄cycloalkenyl radical may be optionally substituted where allowed by available valences.

As used herein, the term "aryl" generally refers to a monocyclic, bicyclic or polycyclic aromatic carbon atom ring structure radical, including, without limitation, phenyl, naphthyl, anthracenyl, fluorenyl, azulenyl, phenanthrenyl and the like. An aryl radical may be optionally substituted where allowed by available valences.

As used herein, the term "heteroaryl" generally refers to a monocyclic, bicyclic or polycyclic aromatic carbon atom ring structure radical in which one or more carbon atom ring members have been replaced, where allowed by structural stability, with one or more heteroatoms, such as an O, S or N atom, including, without limitation, furanyl, thienyl (also referred to as thiophenyl), pyrrolyl, pyrazolyl, imidazolyl, isoxazolyl, isothiazolyl, oxazolyl, thiazolyl, triazolyl, oxadiazolyl, thiadiazolyl, tetrazolyl, pyranyl, thiopyranyl, pyridinyl, pyrimidinyl, pyrazinyl, pyridazinyl, triazinyl, indolyl, indazolyl, indolizinyl, benzofuranyl, benzothienyl, benzimidazolyl, benzothiazolyl, benzooxazolyl, 9H-purinyl, quinoxalinyl, isoindolyl, quinolinyl, isoquinolinyl, quinazolinyl, acridinyl and the like. A heteroaryl radical may be optionally substituted on a carbon or nitrogen atom ring member where allowed by available valences.

As used herein, the term "heterocyclyl" generally refers to a saturated or partially unsaturated monocyclic, bicyclic or polycyclic carbon atom ring structure radical in which one or more carbon atom ring members have been replaced, where allowed by structural stability, with a heteroatom, such as an O, S or N atom, including, without limitation, oxiranyl, oxetanyl, azetidinyl, dihydrofuranyl, tetrahydrofuranyl, dihydrothienyl, tetrahydrothienyl, pyrrolinyl, pyrrolidinyl, dihydropyrazolyl, pyrazolinyl, pyrazolidinyl, dihydroimidazolyl, imidazolinyl, imidazolidinyl, isoxazolinyl, isoxazolidinyl, isothiazolinyl, isothiazolidinyl, oxazolinyl, oxazolidinyl, thiazolinyl, thiazolidinyl, triazolinyl, triazolidinyl, oxadiazolinyl, oxadiazolidinyl, thiadiazolinyl, thiadiazolidinyl, tetrazolinyl, tetrazolidinyl, dihydro-2H-pyranyl, dihydro-pyridinyl, tetrahydro-pyridinyl, 1,2,3,6-tetrahydropyridinyl, hexahydro-pyridinyl, dihydro-pyrimidinyl, tetrahydro-pyrimidinyl, 1,4,5,6-tetrahydropyrimidinyl, dihydro-pyrazinyl, tetrahydro-pyrazinyl, dihydro-pyridazinyl, tetrahydro-pyridazinyl, piperazinyl, piperidinyl, morpholinyl, thiomorpholinyl, dihydro-triazinyl, tetrahydro-triazinyl, hexahydro-triazinyl, 1,4-diazepanyl, dihydro-indolyl, indolinyl, tetrahydro-indolyl, dihydro-indazolyl, tetrahydro-indazolyl, dihydro-isoindolyl, dihydro-benzofuranyl, tetrahydro-benzofuranyl, dihydro-benzothienyl, tetrahydro-benzothienyl, dihydro-benzimidazolyl, tetrahydro-benzimidazolyl, dihydro-benzooxazolyl, 2,3-dihydrobenzo[d]oxazolyl, tetrahydro-benzooxazolyl, dihydro-benzooxazinyl, 3,4-dihydro-2H-benzo[b][1,4]oxazinyl, tetrahydro-benzooxazinyl, benzo[1,3]dioxolyl, benzo[1,4]dioxanyl, dihydro-purinyl, tetrahydro-purinyl, dihydro-quinolinyl, tetrahydro-quinolinyl, 1,2,3,4-tetrahydroquinolinyl, dihydro-isoquinolinyl, 3,4-dihydroisoquinolin-(1H)-yl, tetrahydro-isoquinolinyl, 1,2,3,4-tetrahydroisoquinolinyl, dihydro-quinazolinyl, tetrahydro-quinazolinyl, dihydro-quinoxalinyl, tetrahydro-quinoxalinyl, 1,2,3,4-tetrahydroquinoxalinyl, 1,3-dioxolanyl, 2,5-dihydro-1H-pyrrolyl, dihydro-1H-imidazolyl, tetrahydro-2H-pyranyl, hexahydropyrrolo[3,4-b][1,4]oxazin-(2H)-yl, (4aR,7aS)-hexahydropyrrolo[3,4-b][1,4]oxazin-(4aH)-yl, 3,4-dihydro-2H-pyrido[3,2-b][1,4]oxazinyl, (cis)-octahydrocyclopenta[c]pyrrolyl, hexahydropyrrolo[3,4-b]pyrrol-(1H)-yl, (3aR,6aR)-hexahydropyrrolo[3,4-b]pyrrol-(1H)-yl, (3aR,6aS)-hexahydropyrrolo[3,4-c]pyrrol-(1H)-yl, 5H-pyrrolo[3,4-b]pyridin-(7H)-yl, 5,7-dihydro-6H-pyrrolo[3,4-b]pyridinyl, tetrahydro-1H-pyrrolo[3,4-b]pyridin-(2H,7H,7aH)-yl, hexahydro-1H-pyrrolo[3,4-b]pyridin-(2H)-yl, (4aR,7aR)-hexahydro-1H-pyrrolo[3,4-b]pyridin-(2H)-yl, octahydro-6H-pyrrolo[3,4-b]pyridinyl, 2,3,4,9-tetrahydro-1H-carbazolyl, 1,2,3,4-tetrahydropyrazino[1,2-a]indolyl, 2,3-dihydro-1H-pyrrolo[1,2-a]indolyl, (3aR,6aR)-hexahydrocyclopenta[c]pyrrol-(1H)-yl, (3aR,4R,6aS)-hexahydrocyclopenta[c]pyrrol-(1H)-yl, (3aR,4S,6aS)-hexahydrocyclopenta[c]pyrrol-(1H)-yl, (3aR,5r,6aS)-hexahydrocyclopenta[c]pyrrol-(1H)-yl, 1,3-dihydro-2H-isoindolyl, octahydro-2H-isoindolyl, (3aS)-1,3,3a,4,5,6-hexahydro-2H-isoindolyl, (3aR,4R,7aS)-1H-isoindol-(3H,3aH,4H,5H,6H,7H,7aH)-yl, (3aR,7aS)-octahydro-2H-isoindolyl, (3aR,4R,7aS)-octahydro-2H-isoindolyl, (3aR,4S,7aS)-octahydro-2H-isoindolyl, 2,5-diazabicyclo[2.2.1]heptanyl, 2-azabicyclo[2.2.1]hept-5-enyl, 3-azabicyclo[3.1.0]hexanyl, 3,6-diazabicyclo[3.1.0]hexanyl, (1R,5S,6s)-3-azabicyclo[3.1.0]hexanyl, (1S,5R,6R)-3-azabicyclo[3.2.0]heptanyl, (1S,5R,6S)-3-azabicyclo[3.2.0]heptanyl, 5-azaspiro[2.4]heptanyl, 2,6-diazaspiro[3.3]heptanyl, 2,5-diazaspiro[3.4]octanyl, 2,6-diazaspiro[3.4]octanyl, 2,7-diazaspiro[3.5]nonanyl, 2,7-diazaspiro[4.4]nonanyl, 2-azaspiro[4.5]decanyl, 2,8-diazaspiro[4.5]decanyl and the like. A heterocyclyl radical may be optionally substituted on a carbon or nitrogen atom ring member where allowed by available valences.

As used herein, the term "C₂₋₈alkenyl-amino" refers to a radical of the formula: -NH-C₂₋₈alkenyl.

As used herein, the term "(C₂₋₈alkenyl)₂-amino" refers to a radical of the formula: -N(C₂₋₈alkenyl)₂.

As used herein, the term "C₂₋₈alkenyl-amino-C₁₋₈alkyl" refers to a radical of the formula: -C₁₋₈alkyl-NH-C₂₋₈alkenyl.

As used herein, the term "(C₂₋₈alkenyl)₂-amino-C₁₋₈alkyl" refers to a radical of the formula: -C₁₋₈alkyl-N(C₂₋₈alkenyl)₂.

As used herein, the term "C₁₋₈alkoxy-C₁₋₈alkyl" refers to a radical of the formula: -C₁₋₈alkyl-O-C₁₋₈alkyl.

As used herein, the term "C₁₋₈alkoxy-C₁₋₈alkyl-amino" refers to a radical of the formula: -O-C₁₋₈alkyl-NH-C₁₋₈alkyl-O-C₁₋₈alkyl.

As used herein, the term "(C₁₋₈alkoxy-C₁₋₈alkyl)₂-amino" refers to a radical of the formula: -N(C₁₋₈alkyl-O-C₁₋₈alkyl)₂.

As used herein, the term "C₁₋₈alkoxy-C₁₋₈alkyl-amino-C₁₋₈alkyl" refers to a radical of the formula: -C₁₋₈alkyl-NH-C₁₋₈alkyl-O-C₁₋₈alkyl.

As used herein, the term "(C₁₋₈alkoxy-C₁₋₈alkyl)₂-amino-C₁₋₈alkyl" refers to a radical of the formula: -C₁₋₈alkyl-N(C₁₋₈alkyl-O-C₁₋₈alkyl)₂.

As used herein, the term "(C₁₋₈alkoxy-C₁₋₈alkyl,C₁₋₈alkyl)-amino" refers to a radical of the formula: -N[(C₁₋₈alkyl)(-C₁₋₈alkyl-O-C₁₋₈alkyl)].

As used herein, the term "(C₁₋₈alkoxy-C₁₋₈alkyl,C₁₋₈alkyl)-amino-C₁₋₈alkyl" refers to a radical of the formula: -C₁₋₈alkyl-N[(C₁₋₈alkyl)(-C₁₋₈alkyl-O-C₁₋₈alkyl)].

As used herein, the term "C₁₋₈alkoxy-carbonyl" refers to a radical of the formula: -C(O)-O-C₁₋₈alkyl.

As used herein, the term "C₁₋₈alkyl-amino" refers to a radical of the formula: -NH-C₁₋₈alkyl.

As used herein, the term "(C₁₋₈alkyl)₂-amino" refers to a radical of the formula: -N(C₁₋₈alkyl)₂.

As used herein, the term "C₁₋₈alkyl-amino-C₁₋₈alkyl" refers to a radical of the formula: -C₁₋₈alkyl-NH-C₁₋₈alkyl.

As used herein, the term "(C₁₋₈alkyl)₂-amino-C₁₋₈alkyl" refers to a radical of the formula: -C₁₋₈alkyl-N(C₁₋₈alkyl)₂.

As used herein, the term "C₁₋₈alkyl-amino-C₁₋₈alkyl-amino" refers to a radical of the formula: -NH-C₁₋₈alkyl-NH-C₁₋₈alkyl.

As used herein, the term "(C₁₋₈alkyl)₂-amino-C₁₋₈alkyl-amino" refers to a radical of the formula: -NH-C₁₋₈alkyl-N(C₁₋₈alkyl)₂.

As used herein, the term "C₁₋₈alkyl-amino-C₁₋₈alkyl-amino-C₁₋₈alkyl" refers to a radical of the formula: -C₁₋₈alkyl-NH-C₁₋₈alkyl-NH-C₁₋₈alkyl.

As used herein, the term "(C₁₋₈alkyl)₂-amino-C₁₋₈alkyl-amino-C₁₋₈alkyl" refers to a radical of the formula: -C₁₋₈alkyl-NH-C₁₋₈alkyl-N(C₁₋₈alkyl)₂.

As used herein, the term "(C₁₋₈alkyl-amino-C₁₋₈alkyl,C₁₋₈alkyl)amino" refers to a radical of the formula: -N[(C₁₋₈alkyl)(-C₁₋₈alkyl-NH-C₁₋₈alkyl)].

As used herein, the term "[(C₁₋₈alkyl)₂-amino-C₁₋₈alkyl,C₁₋₈alkyl]amino" refers to a radical of the formula: -N[(C₁₋₈alkyl)(-C₁₋₈alkyl-N(C₁₋₈alkyl)₂)].

As used herein, the term "(C₁₋₈alkyl-amino-C₁₋₈alkyl,C₁₋₈alkyl)amino-C₁₋₈alkyl,

As used herein, the term "[(C₁₋₈alkyl)₂-amino-C₁₋₈alkyl,C₁₋₈alkyl]amino-C₁₋₈alkyl" refers to a radical of the formula: -C₁₋₈alkyl-N[(C₁₋₈alkyl)(-C₁₋₈alkyl-N(C₁₋₈alkyl)₂)].

As used herein, the term "C₁₋₈alkyl-amino-carbonyl" refers to a radical of the formula: -C(O)-NH-C₁₋₈alkyl.

As used herein, the term "(C₁₋₈alkyl)₂-amino-carbonyl" refers to a radical of the formula: -C(O)-N(C₁₋₈alkyl)₂.

As used herein, the term "C₁₋₈alkyl-carbonyl" refers to a radical of the formula: -C(O)-C₁₋₈alkyl.

As used herein, the term "C₁₋₈alkyl-carbonyl-amino" refers to a radical of the formula: -NH-C(O)-C₁₋₈alkyl.

As used herein, the term "(C₁₋₈alkyl-carbonyl,C₁₋₈alkyl)amino-C₁₋₈alkyl" refers to a radical of the formula: -C₁₋₈alkyl-N[(C₁₋₈alkyl)(-C(O)-C₁₋₈alkyl)].

As used herein, the term "C₁₋₈alkyl-thio" refers to a radical of the formula: -S-C₁₋₈alkyl.

As used herein, the term "C₂₋₈alkynyl-C₁₋₈alkyl" refers to a radical of the formula: -C₁₋₈alkyl-C₂₋₈alkynyl.

As used herein, the term "C₂₋₈alkynyl-amino" refers to a radical of the formula: -NH-C₂₋₈alkynyl.

As used herein, the term "(C₂₋₈alkynyl)₂-amino" refers to a radical of the formula: -N(C₂₋₈alkynyl)₂.

As used herein, the term "C₂₋₈alkynyl-amino-C₁₋₈alkyl" refers to a radical of the formula: -C₁₋₈alkyl-NH-C₂₋₈alkynyl.

As used herein, the term "(C₂₋₈alkynyl)₂-amino-C₁₋₈alkyl" refers to a radical of the formula: -C₁₋₈alkyl-N(C₂₋₈alkynyl)₂.

As used herein, the term "amino" refers to a radical of the formula: -NH₂.

As used herein, the term "amino-C₁₋₈alkyl" refers to a radical of the formula: -C₁₋₈alkyl-NH₂.

As used herein, the term "amino-C₁₋₈alkyl-amino" refers to a radical of the formula: -NH-C₁₋₈alkyl-NH₂.

As used herein, the term "(amino-C₁₋₈alkyl)₂-amino" refers to a radical of the formula: -N(C₁₋₈alkyl-NH₂)₂.

As used herein, the term "amino-C₁₋₈alkyl-amino-C₁₋₈alkyl" refers to a radical of the formula: -C₁₋₈alkyl-NH-C₁₋₈-alkyl-NH₂.

As used herein, the term "(amino-C₁₋₈alkyl,C₁₋₈alkyl)amino" refers to a radical of the formula: -N[(C₁₋₈alkyl)(-C₁₋₈alkyl-NH₂)].

As used herein, the term "(amino-C₁₋₈alkyl,C₁₋₈alkyl)amino-C₁₋₈alkyl" refers to a radical of the formula: -C₁₋₈alkyl-N[(C₁₋₈alkyl)(-C₁₋₈alkyl-NH₂)].

As used herein, the term "amino-carbonyl" refers to a radical of the formula: -C(O)-NH₂.

As used herein, the term "aryl-C₁₋₈alkoxy" refers to a radical of the formula: -O-C₁₋₈alkyl-aryl.

As used herein, the term "aryl-C₁₋₈alkoxy-carbonyl-amino" refers to a radical of the formula: -NH-C(O)-O-C₁₋₈alkyl-aryl.

As used herein, the term "aryl-C₁₋₈alkyl" refers to a radical of the formula: -C₁₋₈alkyl-aryl.

As used herein, the term "aryl-C₁₋₈alkyl-amino" refers to a radical of the formula: -NH-C₁₋₈alkyl-aryl.

As used herein, the term "(aryl-C₁₋₈alkyl)₂-amino" refers to a radical of the formula: -N[(-C₁₋₈alkyl-aryl)_{2]}.

As used herein, the term "aryl-C₁₋₈alkyl-amino-C₁₋₈alkyl" refers to a radical of the formula: -C₁₋₈alkyl-NH-C₁₋₈alkyl-aryl.

As used herein, the term "(aryl-C₁₋₈alkyl)₂-amino-C₁₋₈alkyl" refers to a radical of the formula: -C₁₋₈alkyl-N[(-C₁₋₈alkyl-aryl)₂].

As used herein, the term "(aryl,C₁₋₈alkyl)amino" refers to a radical of the formula: -N[(C₁₋₈alkyl)(aryl)].

As used herein, the term "(aryl,C₁₋₈alkyl)amino-C₁₋₈alkyl" refers to a radical of the formula: -C₁₋₈alkyl-N[(C₁₋₈alkyl)(aryl)].

As used herein, the term "(aryl-C₁₋₈alkyl,C₁₋₈alkyl)amino" refers to a radical of the formula: -N[(C₁₋₈alkyl)(-C₁₋₈alkyl-aryl)].

As used herein, the term "(aryl-C₁₋₈alkyl,C₁₋₈alkyl)amino-C₁₋₈alkyl" refers to a radical of the formula: -C₁₋₈alkyl-N[(C₁₋₈alkyl)(-C₁₋₈alkyl-aryl)].

As used herein, the term "aryl-amino" refers to a radical of the formula: -NH-aryl.

As used herein, the term "(aryl)₂-amino" refers to a radical of the formula: -N[(aryl)₂].

As used herein, the term "aryl-amino-C₁₋₈alkyl" refers to a radical of the formula: -C₁₋₈alkyl-NH-aryl.

As used herein, the term "(aryl)₂-amino-C₁₋₈alkyl" refers to a radical of the formula: -C₁₋₈alkyl-N[(aryl)₂].

As used herein, the term "aryl-amino-carbonyl" refers to a radical of the formula: -C(O)-NH-aryl.

As used herein, the term "azido" refers to a radical of the formula: -N=N⁺=N⁻.

As used herein, the term "carboxyl" refers to a radical of the formula: -COOH, -C(O)OH or -CO₂H.

As used herein, the term "(carboxyl-C₁₋₈alkyl,C₁₋₈alkyl)amino-carbonyl-amino" refers to a radical of the formula: -NH-C(O)-N[(C₁₋₈alkyl)(-C₁₋₈alkyl-CO₂H)].

As used herein, the term "C₃₋₁₄cycloalkyl-C₁₋₈alkoxy" refers to a radical of the formula: -O-C₁₋₈alkyl-C₃₋₁₄cycloalkyl.

As used herein, the term "C₃₋₁₄cycloalkyl-C₁₋₈alkyl" refers to a radical of the formula: -C₁₋₈alkyl-C₃₋₁₄cycloalkyl.

As used herein, the term "C₃₋₁₄cycloalkyl-amino" refers to a radical of the formula: -NH-C₃₋₁₄cycloalkyl.

As used herein, the term "C₃₋₁₄cycloalkyl-amino-C₁₋₈alkyl" refers to a radical of the formula: -C₁₋₈alkyl-NH-C₃₋₁₄cycloalkyl.

As used herein, the term "(C₃₋₁₄cycloalkyl)₂-amino-C₁₋₈alkyl" refers to a radical of the formula: -C₁₋₈alkyl-N[(C₃₋₁₄cycloalkyl)₂].

As used herein, the term "C₃₋₁₄cycloalkyl-C₁₋₈alkyl-amino-C₁₋₈alkyl" refers to a radical of the formula: -C₁₋₈alkyl-NH-C₁₋₈alkyl-C₃₋₁₄cycloalkyl.

As used herein, the term "(C₃₋₁₄cycloalkyl-C₁₋₈alkyl)₂-amino-C₁₋₈alkyl" refers to a radical of the formula: -C₁₋₈alkyl-N[(-C₁₋₈alkyl-C₃₋₁₄cycloalkyl)₂].

As used herein, the term "(C₃₋₁₄cycloalkyl,C₁₋₈alkyl)amino-C₁₋₈alkyl" refers to a radical of the formula: -C₁₋₈alkyl-N[(C₁₋₈alkyl)(C₃₋₁₄cycloalkyl)].

As used herein, the term "(C₃₋₁₄cycloalkyl-C₁₋₈alkyl,C₁₋₈alkyl)amino-C₁₋₈alkyl" refers to a radical of the formula: -C₁₋₈alkyl-N[(C₁₋₈alkyl)(-C₁₋₈alkyl-C₃₋₁₄cycloalkyl)].

As used herein, the term "C₃₋₁₄cycloalkyl-oxy" refers to a radical of the formula: -O-C₃₋₁₄cycloalkyl.

As used herein, the term "formyl" refers to a radical of the formula: -C(O)-H.

As used herein, the term "formyl-C₁₋₈alkyl" refers to a radical of the formula: -C₁₋₈alkyl-C(O)-H, including, without limitation, formylmethyl (also referred to as 2-oxoethyl), 2-formyl-ethyl (also referred to as 3-oxopropyl), 3-formyl-propyl (also referred to as 4-oxobutyl), 4-formyl-butyl (also referred to as 5-oxopentyl) and the like.

As used herein, the term "halo" or "halogen" generally refers to a halogen atom radical, including fluoro, chloro, bromo and iodo.

As used herein, the term "halo-C₁₋₈alkoxy" refers to a radical of the formula: -O-C₁₋₈alkyl-halo, wherein C₁₋₈alkyl may be partially or completely substituted where allowed by available valences with one or more halogen atoms, including, without limitation, fluoromethoxy, difluoromethoxy, trifluoromethoxy, fluoroethoxy, difluoroethoxy or trifluoroethoxy and the like. In some embodiments, difluoroethoxy includes 2,2-difluoroethoxy, 1,2-difluoroethoxy or 1,1-difluoroethoxy and the like. In some embodiments, halo-C₁₋₈alkoxy includes halo-C₁₋₈alkoxy, halo-C₁₋₄alkoxy and the like.

As used herein, the term "halo-C₁₋₈alkyl" refers to a radical of the formula: -C₁₋₈alkyl-halo, wherein C₁₋₈alkyl may be partially or completely substituted where allowed by available valences with one or more halogen atoms, including, without limitation, fluoromethyl, difluoromethyl, trifluoromethyl, fluoroethyl, difluoroethyl, trifluoroethyl, fluoroisopropyl, difluoroisopropyl, trifluoroisopropyl, fluoro-tert-butyl, difluoro-tert-butyl, trifluoro-tert-butyl and the like. In some embodiments, difluoroethyl includes 2,2-difluoroethyl, 1,2-difluoroethyl or 1,1-difluoroethyl and the like; difluoroisopropyl includes 1,3-difluoropropan-2-yl and the like; trifluoroisopropyl includes 1,1,1-trifluoropropan-2-yl and the like; trifluoro-tert-butyl includes 1,1,1-trifluoro-2-methylpropan-2-yl and the like. In some embodiments, halo-C₁₋₈alkyl includes halo-C₁₋₆alkyl, halo-C₁₋₄alkyl and the like.

As used herein, the term "halo-C₁₋₈alkyl-amino" refers to a radical of the formula: -NH-C₁₋₈alkyl-halo.

As used herein, the term "(halo-C₁₋₈alkyl)₂-amino" refers to a radical of the formula: -N(C₁₋₈alkyl-halo)₂.

As used herein, the term "halo-C₁₋₈alkyl-amino-C₁₋₈alkyl" refers to a radical of the formula: -NH-C₁₋₈alkyl-halo.

As used herein, the term "(halo-C₁₋₈alkyl)₂-amino-C₁₋₈alkyl" refers to a radical of the formula: -C₁₋₈alkyl-N(C₁₋₈alkyl-halo)₂.

As used herein, the term "heteroaryl-C₁₋₈alkyl" refers to a radical of the formula: -C₁₋₈alkyl-heteroaryl.

As used herein, the term "heteroaryl-amino" refers to a radical of the formula: -NH-heteroaryl.

As used herein, the term "(heteroaryl)₂-amino" refers to a radical of the formula: -N[(heteroaryl)₂].

As used herein, the term "heteroaryl-C₁₋₈alkyl-amino" refers to a radical of the formula: -NH-C₁₋₈alkyl-heteroaryl.

As used herein, the term "(heteroaryl-C₁₋₈alkyl)₂-amino" refers to a radical of the formula: -N[(-C₁₋₈alkyl-heteroaryl)₂].

As used herein, the term "heteroaryl-C₁₋₈alkyl-amino-C₁₋₈alkyl" refers to a radical of the formula: -C₁₋₈alkyl-NH-C₁₋₈alkyl-heteroaryl.

As used herein, the term "(heteroaryl-C₁₋₈alkyl)₂-amino-C₁₋₈alkyl" refers to a radical of the formula: -C₁₋₈alkyl-N[(-C₁₋₈alkyl-heteroaryl)₂].

As used herein, the term "(heteroaryl-C₁₋₈alkyl,C₁₋₈alkyl)amino" refers to a radical of the formula: -N[(C₁₋₈alkyl)(-C₁₋₈alkyl-heteroaryl)].

As used herein, the term "(heteroaryl-C₁₋₈alkyl,C₁₋₈alkyl)amino-C₁₋₈alkyl" refers to a radical of the formula: -C₁₋₈alkyl-N[(C₁₋₈alkyl)(-C₁₋₈alkyl-heteroaryl)].

As used herein, the term "heterocyclyl-C₁₋₈alkoxy" refers to a radical of the formula: -O-C₁₋₈alkyl-heterocyclyl.

As used herein, the term "heterocyclyl-C₁₋₈alkyl" refers to a radical of the formula: -C₁₋₈alkyl-heterocyclyl.

As used herein, the term "heterocyclyl-amino" refers to a radical of the formula: -NH-heterocyclyl.

As used herein, the term "(heterocyclyl)₂-amino" refers to a radical of the formula: -N[(heterocyclyl)₂].

As used herein, the term "heterocyclyl-amino-C₁₋₈alkyl" refers to a radical of the formula: -C₁₋₈alkyl-NH-heterocyclyl.

As used herein, the term "(heterocyclyl)₂-amino-C₁₋₈alkyl" refers to a radical of the formula: -C₁₋₈alkyl-N[(heterocyclyl)₂].

As used herein, the term "heterocyclyl-C₁₋₈alkyl-amino-C₁₋₈alkyl" refers to a radical of the formula: -C₁₋₈alkyl-NH-C₁₋₈alkyl-heterocyclyl.

As used herein, the term "(heterocyclyl-C₁₋₈alkyl)₂-amino-C₁₋₈alkyl" refers to a radical of the formula: -C₁₋₈alkyl-N[(-C₁₋₈alkyl-heterocyclyl)₂].

As used herein, the term "(heterocyclyl,C₁₋₈alkyl)amino" refers to a radical of the formula: -N[(C₁₋₈alkyl)(heterocyclyl)].

As used herein, the term "(heterocyclyl,C₁₋₈alkyl)amino-C₁₋₈alkyl" refers to a radical of the formula: -C₁₋₈alkyl-N[(C₁₋₈alkyl)(heterocyclyl)].

As used herein, the term "(heterocyclyl-C₁₋₈alkyl,C₁₋₈alkyl)amino-C₁₋₈alkyl" refers to a radical of the formula: -C₁₋₈alkyl-N[(C₁₋₈alkyl)(-C₁₋₈alkyl-heterocyclyl)].

As used herein, the term "(heterocyclyl,C₃₋₁₄cycloalkyl-C₁₋₈alkyl)amino-C₁₋₈alkyl" refers to a radical of the formula: -C₁₋₈alkyl-N[(heterocyclyl)(-C₁₋₈alkyl-C₃₋₁₄cycloalkyl)].

As used herein, the term "heterocyclyl-carbonyl" refers to a radical of the formula: -C(O)-heterocyclyl.

As used herein, the term "heterocyclyl-carbonyl-oxy" refers to a radical of the formula: -O-C(O)-heterocyclyl.

As used herein, the term "heterocyclyl-oxy" refers to a radical of the formula: -O-heterocyclyl.

As used herein, the term "heterocyclyl-oxy-amino" refers to a radical of the formula: -NH-O-heterocyclyl.

As used herein, the term "(heterocyclyl-oxy)₂-amino" refers to a radical of the formula: -N[(-O-heterocyclyl)₂].

As used herein, the term "(heterocyclyl-oxy,C₁₋₈alkyl)amino" refers to a radical of the formula: -N[(C₁₋₈alkyl)(-O-heterocyclyl)].

As used herein, the term "(heterocyclyl-oxy-C₁₋₈alkyl,C₁₋₈alkyl)amino" refers to a radical of the formula: -N[(C₁₋₈alkyl)(-C₁₋₈alkyl-O-heterocyclyl)].

As used herein, the term "hydroxyl-C₁₋₈alkoxy" refers to a radical of the formula: -O-C₁₋₈alkyl-OH, wherein C₁₋₈alkyl may be partially or completely substituted where allowed by available valences with one or more hydroxyl radicals.

As used herein, the term "hydroxyl-C₁₋₈alkyl" refers to a radical of the formula: -C₁₋₈alkyl-OH, wherein C₁₋₈alkyl may be partially or completely substituted where allowed by available valences with one or more hydroxy radicals.

As used herein, the term "hydroxyl-amino" refers to a radical of the formula: -NH-OH.

As used herein, the term "hydroxyl-C₁₋₈alkyl-amino" refers to a radical of the formula: -NH-C₁₋₈alkyl-OH, wherein C₁₋₈alkyl may be partially or completely substituted where allowed by available valences with one or more hydroxyl radicals.

As used herein, the term "(hydroxyl-C₁₋₈alkyl)₂-amino" refers to a radical of the formula: -N(C₁₋₈alkyl-OH)₂, wherein C₁₋₈alkyl may be partially or completely substituted where allowed by available valences with one or more hydroxyl radicals.

As used herein, the term "hydroxyl-C₁₋₈alkyl-amino-C₁₋₈alkyl" refers to a radical of the formula: -C₁₋₈alkyl-NH-C₁₋₈alkyl-OH, wherein C₁₋₈alkyl may be partially or completely substituted where allowed by available valences with one or more hydroxyl radicals.

As used herein, the term "hydroxyl-C₁₋₈alkyl-amino-C₁₋₈alkyl-amino" refers to a radical of the formula: -NH-C₁₋₈alkyl-NH-C₁₋₈alkyl-OH, wherein C₁₋₈alkyl may be partially or completely substituted where allowed by available valences with one or more hydroxyl radicals.

As used herein, the term "(hydroxyl-C₁₋₈alkyl,C₁₋₈alkyl)amino" refers to a radical of the formula: -N[(C₁₋₈alkyl)(C₁₋₈alkyl-OH)], wherein C₁₋₈alkyl may be partially or completely substituted where allowed by available valences with one or more hydroxyl radicals.

As used herein, the term "(hydroxyl-C₁₋₈alkyl,C₁₋₈alkyl)amino-C₁₋₈alkyl" refers to a radical of the formula: -C₁₋₈alkyl-N[(C₁₋₈alkyl)(C₁₋₈alkyl-OH)], wherein C₁₋₈alkyl may be partially or completely substituted where allowed by available valences with one or more hydroxyl radicals.

As used herein, the term "(hydroxyl-C₁₋₈alkyl,C₁₋₈alkyl)amino-C₁₋₈alkyl-amino" refers to a radical of the formula: -NH-C₁₋₈alkyl-N[(C₁₋₈alkyl)(C₁₋₈alkyl-OH)], wherein C₁₋₈alkyl may be partially or completely substituted where allowed by available valences with one or more hydroxyl radicals.

As used herein, the term "[(hydroxyl-C₁₋₈alkyl,C₁₋₈alkyl)amino-C₁₋₈alkyl,C₁₋₈alkyl)amino" refers to a radical of the formula: -N[(C₁₋₈alkyl)(-C₁₋₈alkyl-N[(C₁₋₈alkyl)(C₁₋₈alkyl-OH)])], wherein C₁₋₈alkyl may be partially or completely substituted where allowed by available valences with one or more hydroxyl radicals.

As used herein, the term "(hydroxyl-C₁₋₈alkyl-amino-C₁₋₈alkyl,C₁₋₈alkyl)amino" refers to a radical of the formula: -N[(C₁₋₈alkyl)(-C₁₋₈alkyl-NH-C₁₋₈alkyl-OH)], wherein C₁₋₈alkyl may be partially or completely substituted where allowed by available valences with one or more hydroxyl radicals.

As used herein, the term "substituent" means positional variables on the atoms of a core molecule that are substituted at a designated atom position, replacing one or more hydrogens on the designated atom, provided that the designated atom's normal valency is not exceeded, and that the substitution results in a stable compound. Combinations of substituents and/or variables are permissible only if such combinations result in stable compounds. A person of ordinary skill in the art should note that any carbon as well as heteroatom with valences that appear to be unsatisfied as described or shown herein is assumed to have a sufficient number of hydrogen atom(s) to satisfy the valences described or shown.

As used herein, the term "and the like," with reference to the definitions of chemical terms provided herein, means that variations in chemical structures that could be expected by one skilled in the art include, without limitation, isomers (including chain, branching or positional structural isomers), hydration of ring systems (including saturation or partial unsaturation of monocyclic, bicyclic or polycyclic ring structures) and all other variations where allowed by available valences which result in a stable compound.

For the purposes of this description, where one or more substituent variables for a compound of Formula (I) encompass functionalities incorporated into a compound of Formula (I), each functionality appearing at any location within the disclosed compound may be independently selected, and as appropriate, independently and/or optionally substituted.

As used herein, the terms "independently selected," or "each selected" refer to functional variables in a substituent list that may occur more than once on the structure of Formula (I), the pattern of substitution at each occurrence is independent of the pattern at any other occurrence. Further, the use of a generic substituent variable on any formula or structure for a compound described herein is understood to include the replacement of the generic substituent with species substituents that are included within the particular genus, *e.g.,* aryl may be replaced with phenyl or naphthalenyl and the like, and that the resulting compound is to be included within the scope of the compounds described herein.

As used herein, the term "each instance of" when used in a phrase such as "...aryl, aryl-C₁₋₈alkyl, heterocyclyl and heterocyclyl-C₁₋₈alkyl, wherein *each instance of* aryl and heterocyclyl is optionally substituted with one or two substituents..." (emphasis added) is intended to optionally include substitution on each appearance of aryl and heterocyclyl, whether the ring is a primary or secondary substituent, that is, where the aryl and heterocyclyl rings are the primary (first) substituent or where the primary substituent is C₁₋₈alkyl and the aryl and heterocyclyl rings are the secondary (second) substituent, as in, for example, aryl-C₁₋₈alkyl and heterocyclyl-C₁₋₈alkyl.

As used herein, the term "optionally substituted" means optional substitution with the specified substituent variables, groups, radicals or moieties.

As used herein, the terms "stable compound' or "stable structure" mean a compound that is sufficiently robust to survive isolation to a useful degree of purity from a reaction mixture and formulations thereof into an efficacious therapeutic agent.

Compound names used herein were obtained using the ACD Labs Index Name software provided by ACD Labs; and/or, were provided using the Autonom function of ChemDraw Ultra provided by CambridgeSoft. When the compound name disclosed herein conflicts with the structure depicted, the structure shown will supercede the use of the name to define the compound intended.

### Compound Forms

As used herein, the terms "a compound of Formula (Ia), (IIa), (IIIa), (Ib), (IIb), (IIIb), (Ic), (IIc) or (IIIc)," as defined previously, refer to sub-genuses of the compound of Formula (I) or a form thereof and are defined herein. Rather than repeat embodiments for a compound of Formula (Ia), (IIa), (IIIa), (Ib), (IIb), (IIIb), (Ic), (IIc) or (IIIc), in certain embodiments, the term "a compound of Formula (I) or a form thereof" is used to refer to one or more of a compound of Formula (Ia), (IIa), (IIIa), (Ib), (IIb), (IIIb), (Ic), (IIc) or (IIIc) or a form thereof, wherein the form of the compound is selected from a free acid, free base, salt, hydrate, solvate, clathrate, isotopologue, racemate, enantiomer, diastereomer, stereoisomer, polymorph or tautomer form thereof. Thus, embodiments and references to "a compound of Formula (I)" are intended to include compounds of Formula (Ia), (IIa), (IIIa), (Ib), (IIb), (IIIb), (Ic), (IIc) and (IIIc).

As used herein, the term "form" means a compound of Formula (I) isolated for use selected from a free acid, free base, salt, hydrate, solvate, clathrate, isotopologue, racemate, enantiomer, diastereomer, stereoisomer, polymorph or tautomer form thereof.

In certain embodiments described herein, the form of the compound of Formula (I) is a free acid, free base or salt thereof.

In certain embodiments described herein, the form of the compound of Formula (I) is an isotopologue thereof.

In certain embodiments described herein, the form of the compound of Formula (I) is a stereoisomer, racemate, enantiomer or diastereomer thereof.

In certain embodiments described herein, the form of the compound of Formula (I) is a tautomer thereof.

In certain embodiments described herein, the form of the compound of Formula (I) is a pharmaceutically acceptable form.

In certain embodiments described herein, the compound of Formula (I) or a form thereof is isolated for use.

As used herein, the term "isolated" means the physical state of a compound of Formula (I) after being isolated and/or purified from a synthetic process (e.g., from a reaction mixture) or natural source or combination thereof according to an isolation or purification process or processes described herein or which are well known to the skilled artisan (e.g., chromatography, recrystallization and the like) in sufficient purity to be characterizable by standard analytical techniques described herein or well known to the skilled artisan.

As used herein, the term "protected" means that a functional group in a compound of Formula (I) is in a form modified to preclude undesired side reactions at the protected site when the compound is subjected to a reaction. Suitable protecting groups will be recognized by those with ordinary skill in the art as well as by reference to standard textbooks such as, for example, T. W. Greene et al, Protective Groups in organic Synthesis (1991), Wiley, New York.

Solvates of the compounds described herein are also contemplated.

As used herein, the term "prodrug" means a form of an instant compound (e.g., a drug precursor) that is *transformed in vivo* to yield an active compound of Formula (I) or a form thereof. The transformation may occur by various mechanisms (e.g., by metabolic and/or non-metabolic chemical processes), such as, for example, by hydrolysis and/or metabolism in blood, liver and/or other organs and tissues. A discussion of the use of prodrugs is provided by T. Higuchi and W. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

In one example, when a compound of Formula (I) or a form thereof contains a carboxylic acid functional group, a prodrug can comprise an ester formed by the replacement of the hydrogen atom of the acid group with a functional group such as alkyl and the like. In another example, when a compound of Formula (I) or a form thereof contains an alcohol functional group, a prodrug can be formed by the replacement of the hydrogen atom of the alcohol group with a functional group such as alkyl or carbonyloxy and the like. In another example, when a compound of Formula (I) or a form thereof contains an amine functional group, a prodrug can be formed by the replacement of one or more amine hydrogen atoms with a functional group such as alkyl or substituted carbonyl.

One or more compounds described herein may exist in unsolvated as well as solvated forms with pharmaceutically acceptable solvents such as water, ethanol, and the like, and the description herein is intended to embrace both solvated and unsolvated forms.

As used herein, the term "solvate" means a physical association of a compound described herein with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. As used herein, "solvate" encompasses both solution-phase and isolatable solvates. Non-limiting examples of suitable solvates include ethanolates, methanolates, and the like.

One or more compounds described herein may optionally be converted to a solvate. Preparation of solvates is generally known. The preparation of solvates of the antifungal fluconazole in ethyl acetate as well as from water has been described (see, M. Caira et al, J. Pharmaceutical Sci., 93(3), 601-611 (2004)). Similar preparations of solvates, hemisolvate, hydrates and the like have also been described (see, E. C. van Tonder et al, AAPS PharmSciTech., 5(1), article 12 (2004); and A. L. Bingham et al, Chem. Commun., 603-604 (2001)). A typical, non-limiting process involves dissolving a compound in a desired amount of the desired solvent (organic or water or mixtures thereof) at a higher than ambient temperature, and cooling the solution at a rate sufficient to form crystals which are then isolated by standard methods. Analytical techniques such as, for example infrared spectroscopy, show the presence of the solvent (or water) in the crystals as a solvate (or hydrate).

As used herein, the term "hydrate" means a solvate wherein the solvent molecule is water.

The compounds of Formula (I) can form salts, which are intended to be included within the scope of this description. Reference to a compound of Formula (I) herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a compound of Formula (I) contains both a basic moiety, such as, but not limited to a pyridine or imidazole, and an acidic moiety, such as, but not limited to a carboxylic acid, zwitterions ("inner salts") may be formed and are included within the term "salt(s)" as used herein.

The term "pharmaceutically acceptable salt(s)", as used herein, means those salts of compounds described herein that are safe and effective *(i.e.,* non-toxic, physiologically acceptable) for use in mammals and that possess biological activity, although other salts are also useful. Salts of the compounds of the Formula (I) may be formed, for example, by reacting a compound of Formula (I) with an amount of acid or base, such as an equivalent amount, in a medium such as one in which the salt precipitates or in an aqueous medium followed by lyophilization.

Pharmaceutically acceptable salts include one or more salts of acidic or basic groups present in compounds described herein. Embodiments of acid addition salts include, and are not limited to, acetate, acid phosphate, ascorbate, benzoate, benzenesulfonate, bisulfate, bitartrate, borate, butyrate, chloride, citrate, camphorate, camphorsulfonate, ethanesulfonate, formate, fumarate, gentisinate, gluconate, glucaronate, glutamate, hydrobromide, hydrochloride, dihydrochloride, hydroiodide, isonicotinate, lactate, maleate, methanesulfonate, naphthalenesulfonate, nitrate, oxalate, pamoate, pantothenate, phosphate, propionate, saccharate, salicylate, succinate, sulfate, tartrate, thiocyanate, toluenesulfonate (also known as tosylate), trifluoroacetate salts and the like. Certain embodiments of acid addition salts include chloride, hydrobromide, hydrochloride, dihydrochloride, acetate or trifluoroacetic acid salts.

Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33, 201-217; Anderson et al, The Practice of Medicinal Chemistry (1996), Academic Press, New York; and in The Orange Book (Food & Drug Administration, Washington, D.C. on their website).

Suitable basic salts include, but are not limited to, aluminum, ammonium, calcium, lithium, magnesium, potassium, sodium, zinc, and diethanolamine salts. Certain compounds described herein can also form pharmaceutically acceptable salts with organic bases (for example, organic amines) such as, but not limited to, dicyclohexylamines, t-butyl amines and the like, and with various amino acids such as, but not limited to, arginine, lysine and the like. Basic nitrogen-containing groups may be quarternized with agents such as lower alkyl halides *(e.g.,* methyl, ethyl, and butyl chlorides, bromides and iodides), dialkyl sulfates *(e.g.,* dimethyl, diethyl, and dibutyl sulfates), long chain halides *(e.g.,* decyl, lauryl, and stearyl chlorides, bromides and iodides), aralkyl halides (e.g., benzyl and phenethyl bromides), and others.

All such acid salts and base salts are intended to be included within the scope of pharmaceutically acceptable salts as described herein. In addition, all such acid and base salts are considered equivalent to the free forms of the corresponding compounds for purposes of this description.

Pharmaceutically acceptable prodrugs of compounds of Formula (I) or a form thereof include those compounds substituted with one or more of the following groups: carboxylic acid esters, sulfonate esters, amino acid esters phosphonate esters and mono-, di- or triphosphate esters or alkyl substituents, where appropriate. As described herein, it is understood by a person of ordinary skill in the art that one or more of such substituents may be used to provide a compound of Formula (I) or a form thereof as a prodrug.

Compounds of Formula (I), and forms thereof, may further exist in a tautomeric form (for example, as an amide or imino ether). All such tautomeric forms are contemplated and intended to be included within the scope of the compounds of Formula (I) or a form thereof as described herein.

The compounds of Formula (I) may contain asymmetric or chiral centers, and, therefore, exist in different stereoisomeric forms. The present description is intended toinclude all stereoisomeric forms of the compounds of Formula (I) as well as mixtures thereof, including racemic mixtures.

The compounds described herein may include one or more chiral centers, and as such may exist as racemic mixtures (*R*/*S*) or as substantially pure enantiomers and diastereomers. The compounds may also exist as substantially pure (R) or (*S*) enantiomers (when one chiral center is present). In one embodiment, the compounds described herein are (S) isomers and may exist as enantiomerically pure compositions substantially comprising only the (*S*) isomer. In another embodiment, the compounds described herein are (R) isomers and may exist as enantiomerically pure compositions substantially comprising only the (R) isomer. As one of skill in the art will recognize, when more than one chiral center is present, the compounds described herein may also exist as a (*R*,*R*), (*R,S*)*,* (*S,R*) or (*S*,*S*) isomer, as defined by *IUPAC* Nomenclature Recommendations.

As used herein, the term "substantially pure" refers to compounds consisting substantially of a single isomer in an amount greater than or equal to 90%, in an amount greater than or equal to 92%, in an amount greater than or equal to 95%, in an amount greater than or equal to 98%, in an amount greater than or equal to 99%, or in an amount equal to 100% of the single isomer.

In one aspect of the description, a compound of Formula (I) is a substantially pure (*S*) enantiomer present in an amount greater than or equal to 90%, in an amount greater than or equal to 92%, in an amount greater than or equal to 95%, in an amount greater than or equal to 98%, in an amount greater than or equal to 99%, or in an amount equal to 100%.

In one aspect of the description, a compound of Formula (I) is a substantially pure (R) enantiomer present in an amount greater than or equal to 90%, in an amount greater than or equal to 92%, in an amount greater than or equal to 95%, in an amount greater than or equal to 98%, in an amount greater than or equal to 99%, or in an amount equal to 100%.

As used herein, a "racemate" is any mixture of isometric forms that are not "enantiomerically pure", including mixtures such as, without limitation, in a ratio of about 50/50, about 60/40, about 70/30, or about 80/20.

In addition, the present description embraces all geometric and positional isomers. For example, if a compound of Formula (I) incorporates a double bond or a fused ring, both the cis- and trans-forms, as well as mixtures, are embraced within the scope of the description. Diastereomeric mixtures can be separated into their individual diastereomers on the basis of their physical chemical differences by methods well known to those skilled in the art, such as, for example, by chromatography and/or fractional crystallization. Enantiomers can be separated by use of chiral HPLC column or other chromatographic methods known to those skilled in the art. Enantiomers can also be separated by converting the enantiomeric mixture into a diastereomeric mixture by reaction with an appropriate optically active compound (e.g., chiral auxiliary such as a chiral alcohol or Mosher's acid chloride), separating the diastereomers and converting (e.g., hydrolyzing) the individual diastereomers to the corresponding pure enantiomers. Also, some of the compounds of Formula (I) may be atropisomers (e.g., substituted biaryls) and are considered as part of this description.

It is also possible that the compounds of Formula (I) may exist in different tautomeric forms, and all such forms are embraced within the scope of the description. Also, for example, all keto-enol and imine-enamine forms of the compounds are included in the scope of this description.

All stereoisomers (for example, geometric isomers, optical isomers and the like) of the present compounds (including those of the salts, solvates, esters and prodrugs of the compounds as well as the salts, solvates and esters of the prodrugs), such as those which may exist due to asymmetric carbons on various substituents, including enantiomeric forms (which may exist even in the absence of asymmetric carbons), rotameric forms, atropisomers, and diastereomeric forms, are contemplated within the scope of this description, as are positional isomers (such as, for example, 4-pyridyl and 3-pyridyl). For example, if a compound of Formula (I) incorporates a double bond or a fused ring, both the cis- and trans-forms, as well as mixtures thereof, are embraced herein. Also, for example, all keto-enol and imine-enamine forms of the compounds are included herein. Individual stereoisomers of the compounds described herein may, for example, be substantially free of other isomers, or may be present in a racemic mixture, as described supra.

The use of the terms "salt", "solvate", "ester", "prodrug" and the like, is intended to equally apply to the salt, solvate, ester and prodrug of enantiomers, stereoisomers, rotamers, tautomers, positional isomers, racemates, isotopologues or prodrugs of the instant compounds.

The term "isotopologue" refers to isotopically-enriched compounds described herein which are identical to those recited herein, but for the fact that one or more atoms are replaced by an atom having an atomic mass or mass number different from the atomic mass or mass number usually found in nature. Examples of isotopes that can be incorporated into compounds described herein include isotopes of hydrogen, carbon, nitrogen, oxygen, phosphorus, fluorine and chlorine, such as H², H³, C¹³, C¹⁴, N¹⁵, O¹⁸, O¹⁷, P³¹, P³², S³⁵, F¹⁸, Cl³⁵ and Cl³⁶, respectively, each of which are also within the scope of this description.

Certain isotopically-enriched compounds described herein *(e.g.,* those labeled with H³ and C¹⁴) are useful in compound and/or substrate tissue distribution assays. Tritiated *(i.e.,* H³) and carbon-14 *(i.e.,* C¹⁴) isotopes are particularly preferred for their ease of preparation and detectability. Further, substitution with heavier isotopes such as deuterium *(i.e.,* H²) may afford certain therapeutic advantages resulting from greater metabolic stability (e.g., increased in vivo half-life or reduced dosage requirements) and hence may be preferred in some circumstances.

Polymorphic crystalline and amorphous forms of the compounds of Formula (I), and of the salts, solvates, hydrates, esters and prodrugs of the compounds of Formula (I), are further intended to be included in the present description.

### Use of Compounds

The present description further relates to the compound of Formula (I) for use in methods of treating or ameliorating a bacterial infection, or of treating or ameliorating a multi-drug resistant (MDR) bacterial infection.

The present description further relates to uses of the compound of Formula (I) having activity toward wild-type and MDR bacteria. The present description also relates to uses of the compound of Formula (I) having activity against quinolone-resistant Gram-negative strains (including MDR strains) as well as antibacterial activity to MDR resistant Gram-positive pathogens (including MRSA strains). The present description also relates to uses of the compound of Formula (I) having selectivity between bacterial topoisomerase IV and DNA gyrase enzyme inhibition compared to human topoisomerase II enzyme inhibition. The present description further relates to uses of the compound of Formula (I) that may be combined with known antibacterial agents to provide additive or synergistic activity, thus enabling the development of a combination product for the treatment of Gram-negative (especially MDR strains) and Gram-positive infections.

The compounds of the present description inhibit the clinically validated bacterial targets DNA gyrase and topoisomerase IV, and, thus, may be used for the treatment of infections caused by Gram-negative and Gram-positive pathogens. The instant compounds *possess in vitro* antibacterial activity against a wide spectrum of bacteria which have developed resistance to almost all known treatments, including MDR Gram-negative and MDR Gram-positive pathogens and successfully effect the treatment of bacterial infections compared to current antibacterial agents that target the same enzymes. The compounds are also *effective in vivo* and lack cellular toxicity. In addition to monotherapeutic use, the instant compounds are amenable to combination therapy with current standards of care, having demonstrated additive and synergistic activity with one or more fluoroquinolone based antibacterial agents. The demonstrated additive and synergistic activity of the compounds indicates a mechanistically alternate binding in conjunction with the DNA gyrase and topoisomerase IV targets.

Accordingly, the present description relates to compounds for use in methods of using a compound of Formula (I) for treating or ameliorating a bacterial infection, or for using a compound of Formula (I) for treating or ameliorating a multidrug resistant bacterial infection. In accordance with the present description, compounds that selectively treat or ameliorate a bacterial infection have been identified and methods of using these compounds for treating or ameliorating a bacterial infection or disorders or symptoms associated therewith are provided.

One embodiment of the present description relates to compounds for use in a method of treating or ameliorating a bacterial infection in a subject in need thereof comprising administering an effective amount of a compound of Formula (I) or a form thereof to the subject.

An embodiment of the present description relates to compounds for use in a method of treating or ameliorating a bacterial infection resulting from a bacteria that is a Gram-negative or Gram-positive type in a subject in need thereof comprising administering an effective amount of the compound of Formula (I) or a form thereof to the subject.

An embodiment of the present description relates to compounds for use in a method of treating or ameliorating a bacterial infection resulting from a bacteria that is a resistant Gram-negative or Gram-positive type in a subject in need thereof comprising administering an effective amount of the compound of Formula (I) or a form thereof to the subject.

One embodiment of the present description relates to a compound of Formula (I) or a form thereof for treating or ameliorating a bacterial infection in a subject in need thereof comprising administering an effective amount of the compound to the subject.

An embodiment of the present description relates to a compound of Formula (I) or a form thereof for treating or ameliorating a bacterial infection resulting from a bacteria that is a Gram-negative or Gram-positive type in a subject in need thereof comprising administering an effective amount of the compound to the subject.

An embodiment of the present description relates to a compound of Formula (I) or a form thereof for treating or ameliorating a bacterial infection resulting from a bacteria that is a resistant Gram-negative or Gram-positive type in a subject in need thereof comprising administering an effective amount of the compound to the subject.

An embodiment of the present description relates to a compound of Formula (I) or a form thereof, for use as a medicament.

An embodiment of the present description relates to a use of a compound of Formula (I) or a form thereof in the manufacture of a medicament for treating or ameliorating a bacterial infection in a subject in need thereof comprising administering an effective amount of the medicament to the subject.

An embodiment of the present description relates to a use of a compound of Formula (I) or a form thereof in the manufacture of a medicament for treating or ameliorating a bacterial infection resulting from a bacteria that is a Gram-negative or Gram-positive type in a subject in need thereof comprising administering an effective amount of the compound to the subject.

An embodiment of the present description relates to a use of a compound of Formula (I) or a form thereof in the manufacture of a medicament for treating or ameliorating a bacterial infection resulting from a bacteria that is a resistant Gram-negative or Gram-positive type in a subject in need thereof comprising administering an effective amount of the compound to the subject.

An embodiment of the present description relates to a use of a compound of Formula (I) or a form thereof in the preparation of a pharmaceutical kit comprising the compound of Formula (I) or a form thereof and instructions for administering the compound for treating or ameliorating a bacterial infection in a subject in need thereof.

Another embodiment of the present description relates to a compound of Formula (I) or a form thereof for treating or ameliorating a bacterial infection by selectively inhibiting DNA gyrase and topoisomerase IV compared to human topoisomerase II.

An embodiment of the present description relates to compounds for use in a method of treating or ameliorating a bacterial infection or disorders or symptoms associated therewith in a subject in need thereof comprising administering an effective amount of a compound of Formula (I) or a form thereof to the subject.

An embodiment of the present description relates to the use of a compound of Formula (I) or a form thereof in the manufacture of a medicament for treating or ameliorating bacterial infection or disorders or symptoms associated therewith in a subject in need thereof comprising administering an effective amount of the medicament to the subject.

An embodiment of the present description relates to the use of a compound of Formula (I) or a form thereof in the preparation of a pharmaceutical kit comprising the compound of Formula (I) or a form thereof and instructions for administering the compound for treating or ameliorating a bacterial infection or disorders or symptoms associated therewith in a subject in need thereof.

In one respect, for each of such embodiments, the subject is treatment naive. In another respect, for each of such embodiments, the subject is not treatment naive.

As used herein, the term "treating" refers to: (i) preventing a disease, disorder or condition from occurring in a subject that may be predisposed to the disease, disorder and/or condition but has not yet been diagnosed as having the disease, disorder and/or condition; (ii) inhibiting a disease, disorder or condition, *i.e.,* arresting the development thereof; and/or (iii) relieving a disease, disorder or condition, *i. e.,* causing regression of the disease, disorder and/or condition.

As used herein, the term "subject" refers to an animal or any living organism having sensation and the power of voluntary movement, and which requires oxygen and organic food. Nonlimiting examples include members of the human, equine, porcine, bovine, murine, canine and feline specie. In some embodiments, the subject is a mammal or a warm-blooded vertebrate animal. In other embodiments, the subject is a human. As used herein, the term "patient" may be used interchangeably with "subject" and "human".

Another aspect of the description relates to compounds for use in a method of treating or ameliorating a bacterial infection resulting from a bacteria that is a Gram-negative or Gram-positive type.

Another aspect of the description relates to compounds for use in a method of treating or ameliorating a bacterial infection resulting from bacteria that is a resistant Gram-negative or Gram-positive type.

Another aspect of the description particularly relates to compounds for use in a method of treating or ameliorating a bacterial infection by a wild type bacteria that is resistant to a currently available antibacterial agent, in a subject in need thereof, comprising administering to the subject an effective amount of a compound of Formula (I) or a form thereof.

Examples of bacterial infections intended to be included within the scope of the description include bacterial infections resulting from a bacteria of the phyla including, but not limited to, Acidobacteria; Actinobacteria; Aquificae; Bacteroidetes; Caldiserica; Chlamydiae; Chlorobi; Chloroflexi; Chrysiogenetes; Cyanobacteria; Deferribacteres; Deinococcus-Thermus; Dictyoglomi; Elusimicrobia; Fibrobacteres; Firmicutes; Fusobacteria; Gemmatimonadetes; Lentisphaerae; Nitrospira; Planctomycetes; Proteobacteria; Spirochaetes; Synergistetes; Tenericutes; Firmicutes; Thermodesulfobacteria; Thermomicrobia; Thermotogae; or Verrucomicrobia. The listing of phyla is obtained from the *List of Prokaryotic Names with Standing in Nomenclature (LPSN)* (see, http://www.bacterio.cict.fr/index.html)

Another aspect of the description relates to compounds for use in a method of treating or ameliorating a bacterial infection by a bacteria from a phyla that is a Gram-negative or Gram-positive type.

Another aspect of the description relates to compounds for use in a method of treating or ameliorating a bacterial infection by a bacteria from a phyla that is a drug resistant Gram-negative or Gram-positive type.

Another aspect of the description relates to compounds for use in a method of treating or ameliorating a bacterial infection by a bacteria from a phyla that is a multi-drug resistant Gram-negative or Gram-positive type.

Examples of such bacterial infections intended to be included within the scope of the description particularly include bacterial infections that result from a bacteria of the phyla selected from Proteobacteria, Spirochaetes, Bacteriodetes, Chlamydiae, Firmicutes or Actinobacteria.

In a particular example, the bacterial infections include those resulting from a bacterial species selected from *Acinetobacter baumannii, Bacillus anthracis, Bacillus subtilis, Enterobacter* spp., *Enterococcus faecalis, Enterococcus faecalis, Enterococcus faecium, Escherichia coli, Francisella tularensis, Haemophilus influenzae, Klebsiella pneumoniae, Moraxella catarrhalis, Mycobacterium tuberculosis, Neisseria* spp., *Pseudomonas aeruginosa, Shigella* spp., *Staphylococcus aureus**,** Streptococcus pyogenes*, *Streptococcus pneumoniae* and *Yersinia pestis.*

In another particular example, the bacteria are selected from *Acinetobacter baumannii* BAA747, *Acinetobacter baumannii* MMX2240 (MDR), *Bacillus anthracis* (wild-type), *Bacillus anthracis* T105-R (Cipro^{R}), *Bacillus subtilis* 23857, *Enterococcus faecalis* 29212, *Enterococcus faecalis* 44841 (quin^{R}), *Enterococcus faecium* 49624, *Escherichia coli* BAS849, *Escherichia coli* 25922, *Escherichia coli* LZ3111, *Escherichia coli* LZ3110, *Escherichia coli* ELZ4251 (MDR), *Escherichia coli* NDM-1, *Francisella tularensis* (wild-type), *Haemophilus influenzae* 49247, *Klebsiella pneumoniae* 35657, *Klebsiella pneumoniae* MMX1232 (MDR), *Moraxella catarrhalis* 25238, *Pseudomonas aeruginosa* 27853, *Staphylococcus aureus* 29213, *Staphylococcus aureus* 43300 (MDR), *Staphylococcus aureus* 700789 (MDR), *Streptococcus pneumoniae* 49150 or *Yersinia pestis* (wild-type).

Another aspect of the description relates to compounds for use in a method of treating or ameliorating treating a bacterial infection by a Gram-negative or Gram-positive bacteria.

Another aspect of the description relates to compounds for use in a method of treating or ameliorating treating a bacterial infection by a resistant Gram-negative or Gram-positive bacteria.

Another aspect of the description relates to compounds for use in a method of treating or ameliorating treating a bacterial infection by a drug resistant Gram-negative or Gram-positive bacteria.

Another aspect of the description relates to compounds for use in a method of treating or ameliorating treating a bacterial infection by a multi-drug resistant Gram-negative or Gram-positive bacteria.

As used herein, the terms "effective amount" or "therapeutically effective amount" mean an amount of compound of Formula (I) or a form, composition or medicament thereof effective in inhibiting the above-noted diseases and thus producing the desired therapeutic, ameliorative, inhibitory or preventative effect in a subject in need thereof.

In general, the effective amount will be in a range of from about 0.001 mg/Kg/day to about 500 mg/Kg/day, or about 0.01 mg/Kg/day to about 500 mg/Kg/day, or about 0.1 mg to about 500 mg/Kg/day, or about 1.0 mg/day to about 500 mg/Kg/day, in single, divided, or a continuous dose for a patient or subject having a weight in a range of between about 40 to about 200 Kg (which dose may be adjusted for patients or subjects above or below this range, particularly children under 40 Kg). The typical adult subject is expected to have a median weight in a range of between about 60 to about 100 Kg.

The dose administered to achieve an effective target plasma concentration may also be administered based upon the weight of the subject or patient. Doses administered on a weight basis may be in the range of about 0.01 mg/kg/day to about 50 mg/kg/day, or about 0.015 mg/kg/day to about 20 mg/kg/day, or about 0.02 mg/kg/day to about 10 mg/kg/day, or about 0.025 mg/kg/day to about 10 mg/kg/day, or about 0.03 mg/kg/day to about 10 mg/kg/day, wherein said amount is orally administered once (once in approximately a 24 hour period), twice (once in approximately a 12 hour period) or thrice (once in approximately an 8 hour period) daily according to subject weight.

In another embodiment, where daily doses are adjusted based upon the weight of the subject or patient, compounds described herein may be formulated for delivery at about 0.02, 0.025, 0.03, 0.05, 0.06, 0.075, 0.08, 0.09. 0.10, 0.20, 0.25, 0.30, 0.50, 0.60, 0.75, 0.80, 0.90, 1.0, 1.10, 1.20, 1.25, 1.50, 1.75, 2.0, 5.0, 10, 20 or 50 mg/kg/day. Daily doses adjusted based upon the weight of the subject or patient may be administered as a single, divided, or continuous dose. In embodiments where a dose of compound is given more than once per day, the dose may be administered twice, thrice, or more per day.

Within the scope of the present description, the "effective amount" of a compound of Formula (I) or a form thereof for use and for use in the manufacture of a medicament, the preparation of a pharmaceutical kit or in a method of treating or ameliorating bacterial infection or disorders or symptoms associated therewith in a subject in need thereof, is intended to include an amount in a range of from about 1.0 mg to about 3500 mg administered once daily; 10.0 mg to about 600 mg administered once daily; 0.5 mg to about 2000 mg administered twice daily; or, an amount in a range of from about 5.0 mg to about 300 mg administered twice daily.

For example, the effective amount may be the amount required to treat a bacterial infection, or the amount required to inhibit bacterial replication in a subject or, more specifically, in a human. In some instances, the desired effect can be determined by analyzing the presence of bacterial DNA. The effective amount for a subject will depend upon various factors, including the subject's body weight, size and health. Effective amounts for a given patient can be determined by routine experimentation that is within the skill and judgment of the clinician.

For any compound, the effective amount can be estimated initially either in cell culture assays or in relevant animal models, such as a mouse, chimpanzee, marmoset or tamarin animal model. Relevant animal models may also be used to determine the appropriate concentration range and route of administration. Such information can then be used to determine useful doses and routes for administration in humans. Therapeutic efficacy and toxicity may be determined by standard pharmaceutical procedures in cell cultures or experimental animals, e.g., ED₅₀ (the dose therapeutically effective in 50% of the population) and LD₅₀ (the dose lethal to 50% of the population). The dose ratio between therapeutic and toxic effects is therapeutic index, and can be expressed as the ratio, LD₅₀/ED₅₀. In some embodiments, the effective amount is such that a large therapeutic index is achieved. In further embodiments, the dosage is within a range of circulating concentrations that include an ED₅₀ with little or no toxicity. The dosage may vary within this range depending upon the dosage form employed, sensitivity of the patient, and the route of administration.

More specifically, the concentration-biological effect relationships observed with regard to a compound of Formula (I) or a form thereof indicate an trough target plasma concentration ranging from approximately 0.001 µg/mL to approximately 50 µg/mL, from approximately 0.01 µg/mL to approximately 20 µg/mL, from approximately 0.05 µg/mL to approximately 10 µg/mL, or from approximately 0.1 µg/mL to approximately 5 µg/mL. To achieve such plasma concentrations, the compounds described herein may be administered at doses that vary from 0.1 µg to 100,000 mg, depending upon the route of administration in single, divided, or continuous doses for a patient weighing between about 40 to about 100 kg (which dose may be adjusted for patients above or below this weight range, particularly children under 40 kg).

The exact dosage will be determined by the practitioner, in light of factors related to the subject. Dosage and administration may be adjusted to provide sufficient levels of the active agent(s) or to maintain the desired effect. Factors which may be taken into account include the severity of the disease state, general health of the subject, ethinicity, age, weight, and gender of the subject, diet, time and frequency of administration, drug combination(s), reaction sensitivities, experience with other antibacterial therapies, and tolerance/response to therapy. Long-acting pharmaceutical compositions may be administered every 2, 3 or 4 days, once every week, or once every two weeks depending on half-life and clearance rate of the particular formulation.

The compounds and compositions described herein may be administered to the subject via any drug delivery route known in the art. Nonlimiting examples include oral, ocular, rectal, buccal, topical, nasal, ophthalmic, subcutaneous, intramuscular, intraveneous (bolus and infusion), intracerebral, transdermal, and pulmonary routes of administration.

### Metabolites of the Compounds

Also disclosed in the present description are *the in vivo* metabolic products of the compounds described herein. Such products may result, for example, from the oxidation, reduction, hydrolysis, amidation, esterification and the like of the administered compound, primarily due to enzymatic processes. Accordingly, the description includes compounds produced by a process comprising contacting a compound described herein with a mammalian tissue or a mammal for a period of time sufficient to yield a metabolic product thereof.

Such products typically are identified by preparing a radio-labeled isotopologue (e.g., C¹⁴ or H³) of a compound described herein, administering the radio-labeled compound in a detectable dose (e.g., greater than about 0.5 mg/kg) to a mammal such as a rat, mouse, guinea pig, dog, monkey or human, allowing sufficient time for metabolism to occur (typically about 30 seconds to about 30 hours), and identifying the metabolic conversion products from urine, bile, blood or other biological samples. These products are easily isolated since they are "radio labeled" by virtue of being isotopically-enriched (others are isolated by the use of antibodies capable of binding epitopes surviving in the metabolite). The metabolite structures are determined in conventional fashion, *e.g.,* by MS or NMR analysis. In general, analysis of metabolites may be done in the same way as conventional drug metabolism studies well-known to those skilled in the art. The conversion products, so long as they are not otherwise *found in vivo,* are useful in diagnostic assays for therapeutic dosing of the compounds described herein even if they possess no biological activity of their own.

### Pharmaceutical Compositions

Embodiments of the present description include the compound of Formula (I) or a form thereof for use in a pharmaceutical composition for the prevention or treatment of a bacterial infection comprising an effective amount of a compound of Formula (I) or a form thereof in admixture with a pharmaceutically acceptable excipient.

As used herein, the term "composition" means a product comprising the specified ingredients in the specified amounts, as well as any product which results, directly or indirectly, from combination of the specified ingredients in the specified amounts.

The pharmaceutical composition may be formulated to achieve a physiologically compatible pH, ranging from about pH 3 to about pH 11. In some embodiments, the pharmaceutical composition is formulated to achieve a pH of from about pH 3 to about pH 7. In other embodiments, the pharmaceutical composition is formulated to achieve a pH of from about pH 5 to about pH 8.

The term "pharmaceutically acceptable excipient" refers to an excipient for administration of a pharmaceutical agent, such as the compounds described herein. The term refers to any pharmaceutical excipient that may be administered without undue toxicity. Pharmaceutically acceptable excipients may be determined in part by the particular composition being administered, as well as by the particular mode of administration and/or dosage form. Nonlimiting examples of pharmaceutically acceptable excipients include carriers, solvents, stabilizers, adjuvants, diluents, *etc.* Accordingly, there exists a wide variety of suitable formulations of pharmaceutical compositions for the instant compoounds described herein (*see, e.g.,* Remington's Pharmaceutical Sciences).

Suitable excipients may be carrier molecules that include large, slowly metabolized macromolecules such as proteins, polysaccharides, polylactic acids, polyglycolic acids, polymeric amino acids, amino acid copolymers, and inactive antibodies. Other exemplary excipients include antioxidants such as ascorbic acid; chelating agents such as EDTA; carbohydrates such as dextrin, hydroxyalkylcellulose, hydroxyalkylmethylcellulose, stearic acid; liquids such as oils, water, saline, glycerol and ethanol; wetting or emulsifying agents; pH buffering substances; and the like. Liposomes are also included within the definition of pharmaceutically acceptable excipients.

The pharmaceutical compositions described herein may be formulated in any form suitable for the intended method of administration. Suitable formulations for oral administration include solids, liquid solutions, emulsions and suspensions, while suitable inhaleable formulations for pulmonary administration include liquids and powders. Alternative formulations include syrups, creams, ointments, tablets, and lyophilized solids which can be reconstituted with a physiologically compatible solvent prior to administration.

When intended for oral use for example, tablets, troches, lozenges, aqueous or oil suspensions, non-aqueous solutions, dispersible powders or granules (including micronized particles or nanoparticles), emulsions, hard or soft capsules, syrups or elixirs may be prepared. Compositions intended for oral use may be prepared according to any method known to the art for the manufacture of pharmaceutical compositions, and such compositions may contain one or more agents including sweetening agents, flavoring agents, coloring agents and preserving agents, in order to provide a palatable preparation.

Pharmaceutically acceptable excipients suitable for use in conjunction with tablets include, for example, inert diluents, such as celluloses, calcium or sodium carbonate, lactose, calcium or sodium phosphate; disintegrating agents, such as croscarmellose sodium, crosslinked povidone, maize starch, or alginic acid; binding agents, such as povidone, starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

Formulations for oral use may be also presented as hard gelatin capsules where the active ingredient is mixed with an inert solid diluent, for example celluloses, lactose, calcium phosphate or kaolin, or as soft gelatin capsules wherein the active ingredient is mixed with non-aqueous or oil medium, such as glycerin, propylene glycol, polyethylene glycol, peanut oil, liquid paraffin or olive oil.

In other embodiments, pharmaceutical compositions described herein may be formulated as suspensions comprising a compound of Formula (I) or a form thereof in admixture with at least one pharmaceutically acceptable excipient suitable for the manufacture of a suspension. In yet other embodiments, pharmaceutical compositions described herein may be formulated as dispersible powders and granules suitable for preparation of a suspension by the addition of one or more excipient(s).

Excipients suitable for use in connection with suspensions include suspending agents, such as sodium carboxymethylcellulose, methylcellulose, hydroxypropyl methylcelluose, sodium alginate, polyvinylpyrrolidone, gum tragacanth, gum acacia, dispersing or wetting agents such as a naturally occurring phosphatide (*e.g.*, lecithin), a condensation product of an alkylene oxide with a fatty acid (*e.g.*, polyoxyethylene stearate), a condensation product of ethylene oxide with a long chain aliphatic alcohol (*e.g.*, heptadecaethyleneoxycethanol), a condensation product of ethylene oxide with a partial ester derived from a fatty acid and a hexitol anhydride (*e.g.*, polyoxyethylene sorbitan monooleate); and thickening agents, such as carbomer, beeswax, hard paraffin or cetyl alcohol. The suspensions may also contain one or more preservatives such as acetic acid, methyl and/or n-propyl p-hydroxy-benzoate; one or more coloring agents; one or more flavoring agents; and one or more sweetening agents such as sucrose or saccharin.

The pharmaceutical compositions described herein may also be in the form of oil-in-water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth; naturally occurring phosphatides, such as soybean lecithin, esters or partial esters derived from fatty acids; hexitol anhydrides, such as sorbitan monooleate; and condensation products of these partial esters with ethylene oxide, such as polyoxyethylene sorbitan monooleate. The emulsion may also contain sweetening and flavoring agents. Syrups and elixirs may be formulated with sweetening agents, such as glycerol, sorbitol or sucrose. Such formulations may also contain a demulcent, a preservative, a flavoring or a coloring agent.

Additionally, the pharmaceutical compositions described herein may be in the form of a sterile injectable preparation, such as a sterile injectable aqueous emulsion or oleaginous suspension. Such emulsion or suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as a solution in 1,2-propane-diol. The sterile injectable preparation may also be prepared as a lyophilized powder. Among the acceptable vehicles and solvents that may be employed are water, Ringer's solution, and isotonic sodium chloride solution. In addition, sterile fixed oils may be employed as a solvent or suspending medium. For this purpose any bland fixed oil may be employed including synthetic mono- or di-glycerides. In addition, fatty acids such as oleic acid may likewise be used in the preparation of injectables.

The compounds described herein may be substantially insoluble in water and sparingly soluble in most pharmaceutically acceptable protic solvents and vegetable oils, but generally soluble in medium-chain fatty acids (*e.g.*, caprylic and capric acids) or triglycerides and in propylene glycol esters of medium-chain fatty acids. Thus, contemplated in the description are compounds which have been modified by substitutions or additions of chemical or biochemical moieties which make them more suitable for delivery (*e.g.*, increase solubility, bioactivity, palatability, decrease adverse reactions, *etc.),* for example by esterification, glycosylation, PEGylation, etc.

In some embodiments, the compound described herein is formulated for oral administration in a lipid-based composition suitable for low solubility compounds. Lipid-based formulations can generally enhance the oral bioavailability of such compounds. As such, pharmaceutical compositions described herein may comprise a effective amount of a compound of Formula (I) or a form thereof, together with at least one pharmaceutically acceptable excipient selected from medium chain fatty acids or propylene glycol esters thereof (e.g., propylene glycol esters of edible fatty acids such as caprylic and capric fatty acids) and pharmaceutically acceptable surfactants, such as polyoxyl 40 hydrogenated castor oil.

In other embodiments, the bioavailability of low solubility compounds may be enhanced using particle size optimization techniques including the preparation of nanoparticles or nanosuspensions using techniques known to those skilled in art. The compound forms present in such preparations include amorphous, partially amorphous, partially crystalline or crystalline forms.

In alternative embodiments, the pharmaceutical composition may further comprise one or more aqueous solubility enhancer(s), such as a cyclodextrin. Nonlimiting examples of cyclodextrin include hydroxypropyl, hydroxyethyl, glucosyl, maltosyl and maltotriosyl derivatives of α-, β-, and γ-cyclodextrin, and hydroxypropyl-(β-cyclodextrin (HPBC). In some embodiments, the pharmaceutical composition further comprises HPBC in a range of from about 0.1% to about 20%, from about 1% to about 15%, or from about 2.5% to about 10%. The amount of solubility enhancer employed may depend on the amount of the compound in the composition.

### Preparation of Compounds

### General Synthetic Examples

Methods for preparing certain compounds useful for treating or ameliorating bacterial infections or disorders or symptoms associated therewith are available via standard, well-known synthetic methodologies.

As disclosed herein, methods for preparing the compounds described herein are also available via standard, well-known synthetic methodology. Many of the starting materials used herein are commercially available or can be prepared using the routes described below using techniques known to those skilled in the art.

### General Schemes

Compounds of Formula (I), Formula (II) and Formula (III) can be prepared as described in the Schemes below.

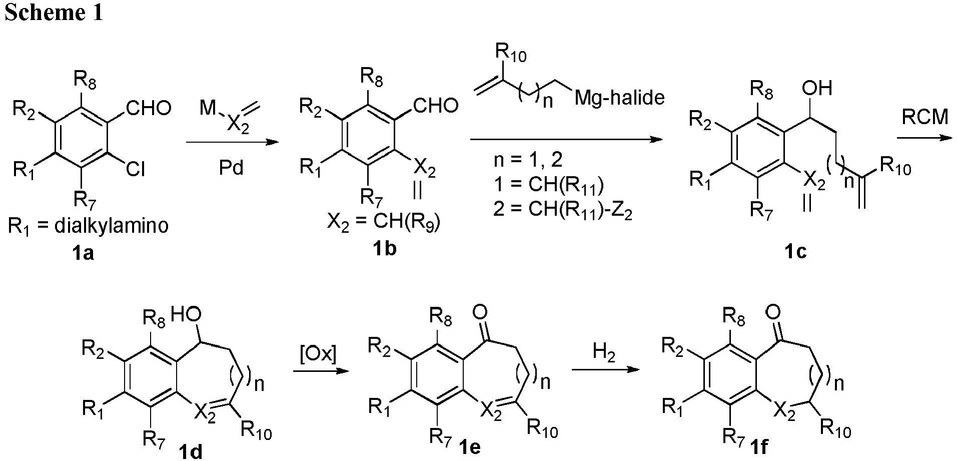

### General Procedures for Scheme 1

Aldehydes of type 1b are prepared from chlorobenzaldehydes of type 1a via Pd catalyzed Suzuki-type coupling with a vinylation reagent (such as potassium vinyl trifluoroborate and the like), in the presence of a phosphine ligand (such as 2-dicyclohexylphosphino-2',6'-dimethoxybiphenyl and the like) in a suitable biphasic mixture (such as a mixture of dioxane/H₂O and the like).

Dienes of type 1c are prepared from aldehydes of type 1b via reaction with alkyl magnesium halides. Ring closing metathesis (RCM) of dienes of type 1c is accomplished in the presence of Grubbs' catalyst (such as a second generation Grubbs' catalyst) in a suitable organic solvent (such as toluene and the like) provides bicyclic anilines of type 1d.

Benzylic alcohols of type 1d are converted to unsaturated ketones of type 1e by reaction with a suitable oxidative reagent (such as DDQ (2,3-Dichloro-5,6-dicyano-1,4-benzoquinone) and the like). Final ketones of type If are prepared by hydrogenation of olefins of type 1e over a Pd catalyst (such as Pd/C and the like).

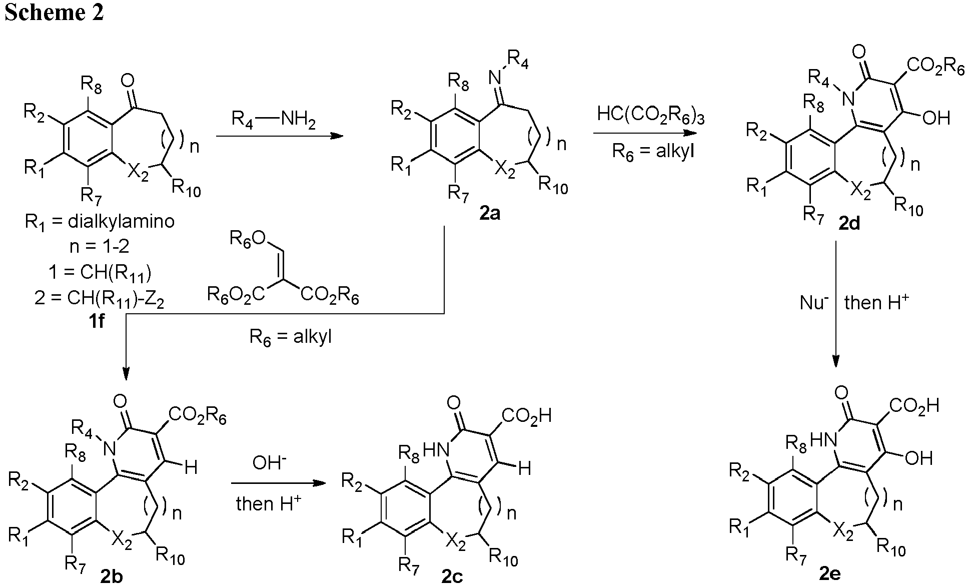

### General Procedures for Scheme 2

Ketones of type If are converted into imines of type 2a by reaction with a benzyl amine (such as 2,4-dimethoxybenzyl amine and the like) in the presence of a suitable dehydrating agent (such as titanium tetrachloride and the like) and a suitable organic base (such as triethylamine and the like) in an organic solvent (such as dichloromethane and the like).

Imines of type 2a are converted into tricyclic 2-pyridones of type 2b by reaction with a dialkyl 2-(alkoxymethylene)malonate in a suitable organic solvent (such as diphenyl ether and the like) at a suitable temperature such as, for example, 180 °C. Hydrolysis of the ester moiety on a 2-pyridone of type 2b is accomplished by reaction with an aqueous inorganic hydroxide (such as LiOH and the like) in a suitable organic solvent (such as THF and the like). The product 2-pyridone of type 2c is prepared by reaction with DMB (2,4-dimethoxybenzyl) under acidic conditions in the presence of a suitable acid (such as TFA (trifluoroacetic acid) and the like) in the presence of a suitable hydride containing reducing reagent (such as i-Pr₃SiH and the like) at a suitable temperature such as, for example, room temperature.

Imines of type 2a may also be converted to hydroxy-2-pyridones of type 2d by reaction with a trialkylmethane tricarboxylate (such as trimethylmethanetricarboxylate and the like) in a suitable organic solvent (such as diphenyl ether and the like) at a suitable temperature such as, for example, 230 °C. The ester moiety on the hydroxy-2-pyridone of type 2d is converted to the corresponding carboxylic acid by reaction with a suitable nucleophilic reagent (such as LiI and the like) in a suitable organic solvent (such as ethyl acetate and the like) at a suitable temperature such as, for example, 60 °C. The product hydroxy-2-pyridone of type 2e is prepared by reaction with DMB under acidic conditions, in the presence of a suitable acid (such as TFA and the like) and a suitable hydride containing reducing reagent (such as i-Pr₃SiH and the like) at a suitable temperature such as, for example, 60 °C.

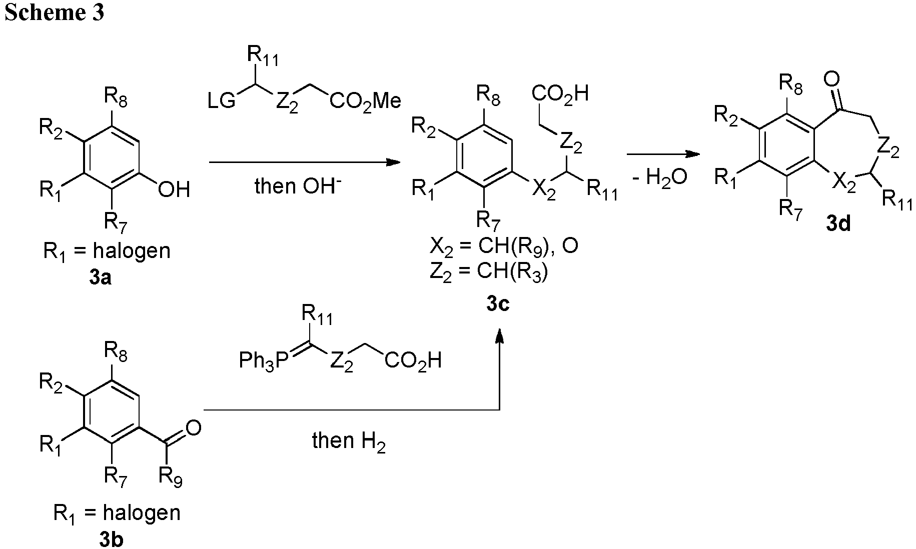

### General Procedures for Scheme 3

Carboxylic acids of type 3c are prepared from phenols of type 3a via alkylation in the presence of an inorganic base (such as K₂CO₃ and the like) followed by hydrolysis in the presence of an inorganic base (such as NaOH and the like). Carboxylic acids of type 3c are also prepared from carbonyl containing compounds of type 3b via Wittig reaction followed by hydrogenation of the intermediate olefin in the presence of a suitable Pd catalyst (such as Pd/C and the like). Ketones of type 3d are prepared from carboxylic acids of type 3c via Friedel-Crafts acylation in the presence of a dehydrating reagent (such as polyphosphoric acid and the like).

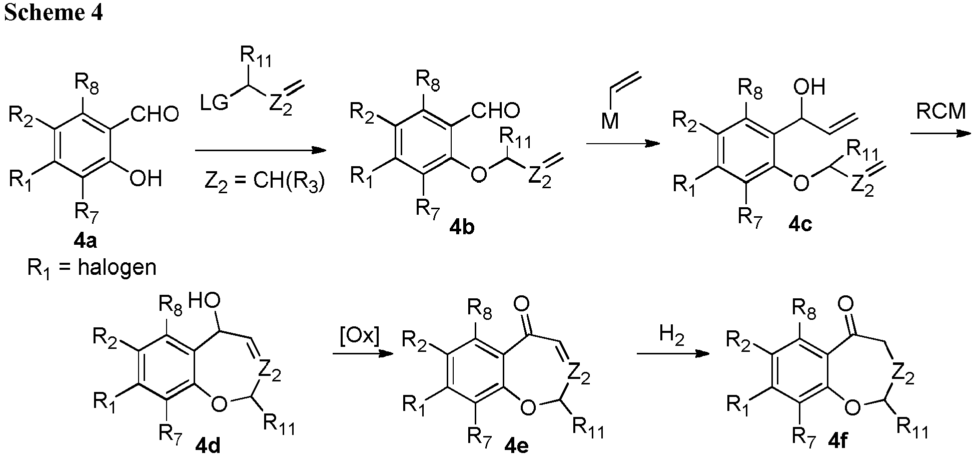

### General Procedures for Scheme 4

Aldehydes of type 4b are prepared from hydroxy-benzaldehydes of type 4a via alkylation in presence of inorganic base (such as K₂CO₃ and the like) in an organic solvent (such as dimethylformamide and the like). Dienes of type 4c are prepared from aldehydes of type 4b via reaction with vinyl metal species (such as vinyl magnesium bromide and the like). RCM of dienes of type 4c is accomplished in the presence of a Grubbs' catalyst (such as a second generation catalyst) in a suitable organic solvent (such as dichloromethane and the like) provides to benzoxapines of type 4d. The benzylic alcohols of type 4d are converted into unsaturated ketones of type 4e by reaction with a suitable oxidant ([Ox]) (such as MnO₂ and the like). Final ketones of type 4f are prepared by hydrogenation of olefin compounds 4e in the presence of a suitable Pt catalyst (such as PtO₂ and the like).

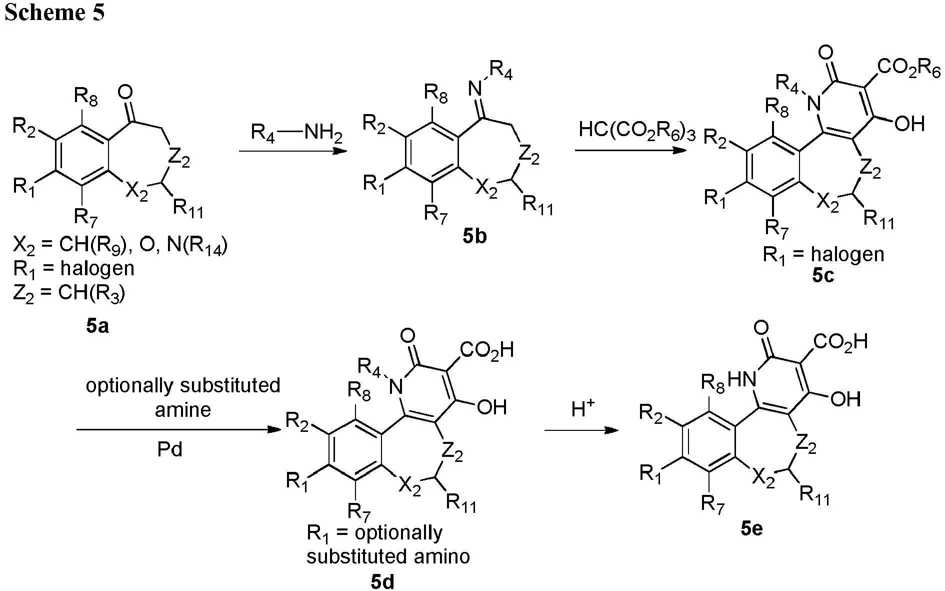

### General Procedures for Scheme 5

The ketones used in Scheme 5 are prepared via the procedures described in Schemes 3 and 4. Ketones of type 5a are converted into imines of type 5b by reaction with a benzyl amine (such as 2,4-dimethoxybenzyl amine and the like) in the presence of a suitable dehydrating agent (such as titanium tetrachloride and the like) and a suitable organic base(such as triethylamine and the like) in a suitable organic solvent (such as dichloromethane and the like). Imines of type 5b are converted into tricyclic hydroxy-2-pyridones of type 5c by reaction with suitable trialkyl methanetricarboxylates (such as trimethylmethanetricarboxylate and the like) in a suitable organic solvent (such as diphenyl ether and the like) at a suitable temperature such as, for example, 230 °C. Aryl-halides of type 5c are converted to anilines of type 5d via amine coupling in the presence of a suitable Pd catalyst (such as 2-(2'-di-*tert*-butylphosphine)biphenylpalladium(II) acetate and the like). Final compounds of type 5e are prepared by reaction with DMB under suitable acidic conditions, in the presence of a suitable acid (such as TFA and the like) and a suitable hydride containing reducing reagent (such as i-Pr₃SiH and the like).

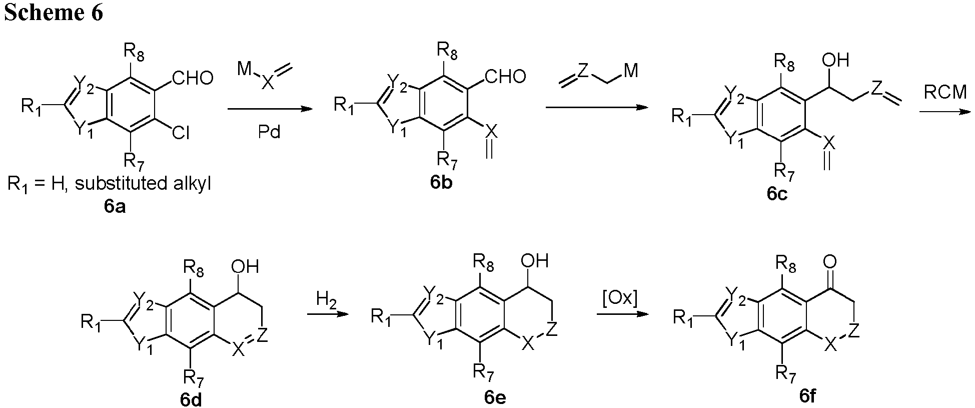

### General Procedures for Scheme 6

Vinyl aldehydes of type 6b are prepared from chloroaldehydes of type 6a via Pd catalyzed Suzuki-type coupling with a suitable vinylation reagent (such as potassium vinyl trifluoroborate and the like) in the presence of a suitable phosphine ligand (such as 2-dicyclohexylphosphino-2',6'-dimethoxybiphenyl and the like) in a suitable biphasic mixture (such as a mixture of dioxane/H₂O and the like). Dienes of type 6c are prepared from aldehydes of type 6b via reaction with alkyl metal halides. RCM of dienes of type 6c is accomplished in the presence of a Grubbs' catalyst (such as a second generation catalyst) in a suitable organic solvent (such as toluene and the like) to provide the tricyclic compound of type 6d. Olefins of type 6d are hydrogenated in the presence of suitable Pd or Pt catalysts (such as Pd/C or PtO₂ and the like) to yield saturated benzylic alcohols of type 6e. Benzylic alcohols of type 6e are converted to ketones of type 6f by reaction with a suitable oxidative reagent (such as TPAP (Tetrapropylammonium perruthenate)/NMO (*N*-Methylmorpholine *N-*oxide) and the like).

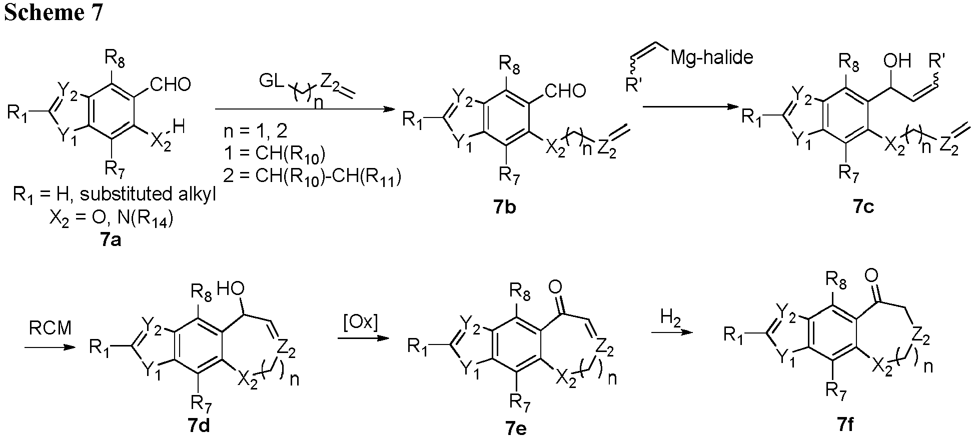

### General Procedures for Scheme 7

Hydroxy substituted aldehydes or anilino aldehydes of type 7a are alkylated in the presence of a suitable inorganic base (such as K₂CO₃ and the like) to yield dienes of type 7b. The bis dienes of type 7c are prepared from aldehydes of type 7b via reaction with a suitable vinyl metal species (such as vinyl magnesium halide and the like). RCM of bis dienes of type 7c is accomplished in the presence of Grubbs' catalyst (such as a second generation catalyst) in a suitable organic solvent (such as toluene and the like) to provide the tricyclic compound of type 7d. Benzylic alcohols of type 7d are converted to ketones of type 7e by reaction with a suitable oxidant (such as MnO₂ and the like). The enones of type 7e are reduced in the presence of suitable Pd or Pt catalysts (such as Pd/C or PtO₂ and the like) to yield saturated ketones 7f.

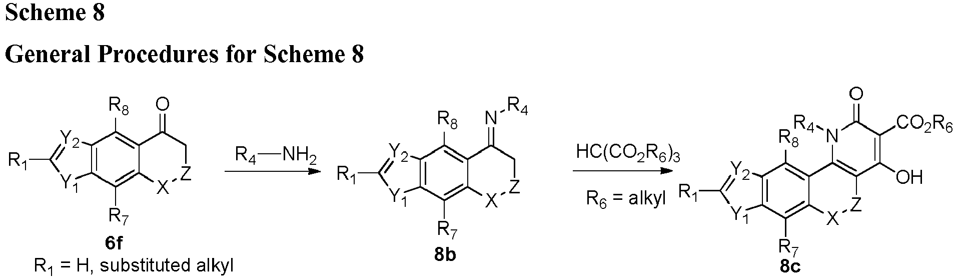

The ketones used in Scheme 8 are prepared via the procedures described in Schemes 6 and 7. Ketones of type 6f are converted into imines of type 8b by reaction with a benzyl amine (such as 2,4-dimethoxybenzyl amine and the like) in the presence of a suitable dehydrating agent (such as titanium tetrachloride and the like) and a suitable organic base (such as triethylamine and the like) in a suitable organic solvent (such as dichloromethane and the like). The imines of type 8b are converted into tetracyclic hydroxy-2-pyridones of type 8c by reaction with a suitable trialkylmethanetricarboxylate (such as trimethylmethanetricarboxylate and the like) in a suitable organic solvent (such as diphenyl ether and the like) at a suitable temperature such as, for example, 230 °C.

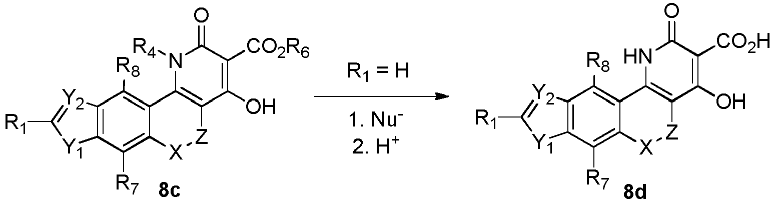

Unsubstituted tetracycles of type 8c (where R₁ is hydrogen) are converted into the pyridones of type 8d via ester group cleavage with a suitable nucleophilic reagent (such as LiI and the like) and subsequent deprotection by removal of DMB under suitable acidic conditions, in the presence of a suitable acid (such as TFA and the like) and a suitable hydride containing reducing reagent (such as i-Pr₃SiH and the like).

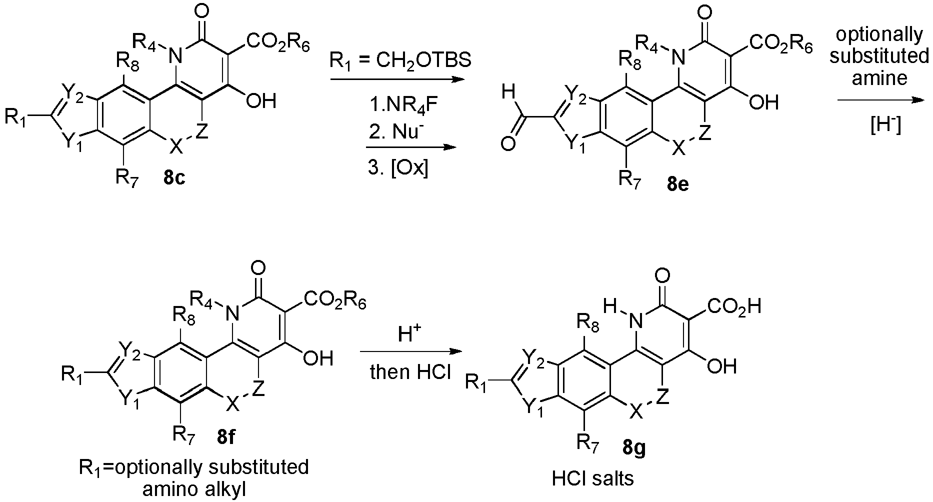

Substituted pyridones of type 8c (where R₁ is CH₂OTBS) (tert-butyldimethylsilyl) are converted into aldehydes of type 8e via a three step process including: deprotection of the TBS-group with a suitable fluoride containing reagent (such as TBAF (tetra-n-butylammonium fluoride) and the like); then, cleavage of the ester group with a suitable nucleophilic reagent (such as LiI and the like; and, conversion of the benzylic hydroxyl to an aldehyde with a suitable oxidant (such as MnO₂ and the like).

Aldehydes of type 8e are converted to amines of type 8f by the reaction with a primary or secondary amine in the presence of a suitable reducing reagent (such as NaBH(OAc)₃ and the like) in a suitable organic solvent (such as dicholoroethane and the like). The product tetracycle of type 8g is deprotected by removal of DMB under suitable acidic conditions, in the presence of a suitable acid (such as TFA and the like) and a suitable hydride containing reducing reagent (such as i-Pr₃SiH and the like), with a subsequent salt exchange using a suitable mineral acid (such as HCl and the like).

### Specific Examples

To assist in understanding the present description, the following Examples are included. The experiments relating to this description should not, of course, be construed as specifically limiting the description and such variations of the description, now known or later developed, which would be within the purview of one skilled in the art are considered to fall within the scope of the description as described herein and hereinafter claimed.

Other than in the working examples, unless indicated to the contrary, all numbers expressing quantities of ingredients, reaction conditions, experimental data, and so forth used in the specification and claims are to be understood as being modified by the term "about". Accordingly, all such numbers represent approximations that may vary depending upon the desired properties sought to be obtained by a reaction or as a result of variable experimental conditions. Therefore, within an expected range of experimental reproducibility, the term "about" in the context of the resulting data, refers to a range for data provided that may vary according to a standard deviation from the mean. As well, for experimental results provided, the resulting data may be rounded up or down to present data consistently, without loss of significant figures. At the very least, and not as an attempt to limit the application of the doctrine of equivalents to the scope of the claims, each numerical parameter should be construed in light of the number of significant digits and ordinary rounding techniques.

While the numerical ranges and parameters setting forth the broad scope of the description are approximations, the numerical values set forth in the working examples are reported as precisely as possible. Any numerical value, however, inherently contains certain errors necessarily resulting from the standard deviation found in their respective testing measurements.

### Synthetic Examples

Greater details of the present description are provided with reference to the following non-limiting examples, which are offered to more fully illustrate the description, but are not to be construed as limiting the scope thereof. The examples illustrate the preparation of certain compounds described herein, and the testing of these *compounds in vitro* and/or *in vivo.* Those of skill in the art will understand that the techniques described in these examples represent techniques described by the inventors to function well in the practice of the description, and as such constitute preferred modes for the practice thereof. However, those of skill in the art should appreciate in light of the present disclosure that many changes can be made to the specific methods that are disclosed and still obtain a like or similar result without departing from the spirit and scope of the description. For example, various conditions were used to obtain LC-MS characterization for the compounds described herein.

As indicated for certain compounds, the 2 Minute Method uses the following column and mobile phase ratios:
Column: Acquity UPLC HSS C18 Column 2.1 x 50 mm, 1.8µm
Mobile Phase A: H₂O/0.1% HCO₂H
Mobile Phase B: Acetonitrile/0.1% HCO₂H

| | Time (min) | Flow (mL/min) | %A | %B |
|---|---|---|---|---|
| 1 | 0 | 0.8 | 100 | 0 |
| 2 | 0.2 | 0.8 | 100 | 0 |
| 3 | 1.5 | 0.8 | 0 | 100 |
| 4 | 2.0 | 0.8 | 100 | 0 |

As indicated for certain compounds, the 1 Minute Method uses the following column and mobile phase ratios:
Column: Acquity UPLC HSS C18 Column 2.1 x 50 mm, 1.8µm
Mobile Phase A: H₂O/0.1% HCO₂H
Mobile Phase B: Acetonitrile/0.1% HCO₂H

| | Time (min) | Flow (mL/min) | %A | %B |
|---|---|---|---|---|
| 1 | 0 | 0.8 | 90 | 10 |
| 2 | 0.1 | 0.8 | 90 | 10 |
| 3 | 0.8 | 0.8 | 5 | 95 |
| 4 | 1.0 | 0.8 | 90 | 10 |

As indicated for certain compounds, the Polar Method uses the following column and mobile phase ratios:
Column: Acquity UPLC HSS C18 Column 2.1 x 50 mm, 1.8µm
Mobile Phase A: H₂O/0.1% HCO₂H
Mobile Phase B: Acetonitrile/0.1% HCO₂H

| | Time (min) | Flow (mL/min) | %A | %B |
|---|---|---|---|---|
| 1 | 0 | 0.8 | 100 | 0 |
| 2 | 0.2 | 0.8 | 100 | 0 |
| 3 | 1.5 | 0.8 | 50 | 50 |
| 4 | 2.0 | 0.8 | 100 | 0 |

As indicated for certain compounds, Method A uses the following column and mobile phase ratios:
Column: HSS T3 Column 2.1 x 50 mm, 1.8 µm
Mobile Phase A: H₂O/0.1% HCO₂H
Mobile Phase B: Acetonitrile/0.1% HCO₂H

| | Time (min) | Flow (ml/min) | %A | %B |
|---|---|---|---|---|
| 1 | 0 | 0.8 | 80 | 20 |
| 2 | 0.2 | 0.8 | 80 | 20 |
| 3 | 1.25 | 0.8 | 5 | 95 |
| 4 | 2.0 | 0.8 | 80 | 20 |

As indicated for certain compounds, Method B uses the following column and mobile phase ratios:
Column: HSS T3 Column 2.1 x 50 mm, 1.8 µm
Mobile Phase A: H₂O/0.1% HCO₂H
Mobile Phase B: Acetonitrile/0.1% HCO₂H

| | Time (min) | Flow (ml/min) | %A | %B |
|---|---|---|---|---|
| 1 | 0 | 0.8 | 95 | 5 |
| 2 | 0.2 | 0.8 | 95 | 5 |
| 3 | 1.5 | 0.8 | 10 | 90 |
| 4 | 2.0 | 0.8 | 95 | 5 |

As indicated for certain compounds, Method C uses the following column and mobile phase ratios:
Column: BEH C18 Column 2.1 x 50 mm, 1.7 µm
Mobile Phase A: NH₄OAc_{aq} 10mM
Mobile Phase B: Acetonitrile

| | Time (min) | Flow (ml/min) | %A | %B |
|---|---|---|---|---|
| 1 | 0 | 0.8 | 95 | 5 |
| 2 | 0.2 | 0.8 | 95 | 5 |
| 3 | 1.5 | 0.8 | 10 | 90 |
| 4 | 2.0 | 0.8 | 95 | 5 |

As used above, and throughout this description, the following abbreviations, unless otherwise indicated, shall be understood to have the following meanings:

| **Abbreviation** | **Meaning** |
|---|---|
| AcOH or HOAc | acetic acid |
| ACN or MeCN | acetonitrile |
| atm | atmosphere |
| Bn | benzyl |
| BnBr | benzyl bromide |
| BnO or OBn | benzyloxy |
| BnOH | benzyl alcohol |
| Boc | *tert-*butoxycarbonyl |
| Boc₂O or (Boc)₂O | di-*tert*-butyl dicarbonate |
| BORSM | based on recovered starting material |
| Cbz | benzyloxycarbonyl |
| CDI | 1,1'-carbonyldiimidazole |
| DCE | dichloroethane |
| DCM | dichloromethane (CH₂Cl₂) |
| DDQ | 2,3-dichloro-5,6-dicyano-1,4-benzoquinone |
| DIAD | diisopropyl azodicarboxylate |
| DIBAL-H | diisobutylaluminium hydride |
| DMF | dimethyl formamide |
| DMA | dimethylacetamide |
| DMAP | 4-dimethylaminopyridine |
| DMB | 2,4-dimethoxybenzyl |
| DMSO | dimethylsulfoxide |
| EA or EtOAc | ethyl acetate |
| EtOH | ethanol |
| Et₂O | diethyl ether |
| HPLC | high performance liquid chromatography |
| h/hr/min/s | hour(h or hr)/minute(min)/second(s) |
| KOAc | potassium acetate |
| LAH | lithium aluminium hydride |
| LC/MS, LCMS or LC-MS | liquid chromatographic mass spectroscopy |
| LDA | lithium diisopropylamide |
| LiOH | lithium hydroxide |
| MeI | methyl iodide |
| MeOH | methanol |
| Me₂NH | N-methylmethanamine |
| MS | mass spectroscopy |
| NaBH(OAc)₃ | sodium triacetoxyborohydride |
| NBS | N-bromosuccinimide |
| NIS | N-iodosuccinimide |
| NMO | N-methylmorpho line-N-oxide |
| n-Bu | n-butyl |
| n-BuLi | n-butyl lithium |
| NMR | nuclear magnetic resonance |
| nPr or n-Pr | n-propyl |
| OsO₄ | osmium tetroxide |
| Pd° | palladium |
| Pd/C° | palladium on carbon |
| Pd₂(dba)₃ | tris(dibenzylideneacetone)dipalladium(0) |
| PdCl₂dppf | [1,1'-bis(diphenylphosphino)ferrocene] dichloropalladium(II) |
| Pd(PPh₃)₄ | tetrakis(triphenylphosphine)palladium |
| Ph₂O | diphenyl ether |
| PPA | 2-phosphonoacetic acid |
| PPh₃ | triphenylphosphine |
| psi | pounds per square inch pressure |
| PTFE | polytetrafluoroethylene |
| PtO₂ | platimum(IV) oxide |
| RT | retention time |
| RCM | ring closing methathesis |
| S-Phos | 2-dicyclohexylphosphino-2',6'-dimethoxybiphenyl |
| TBAF | tetra-n-butylammonium fluoride |
| TBSCl | tert-butyldimethylsilyl chloride |
| t-Bu | tert-butyl |
| t-BuOK | potassium tert-butoxide |
| TEA or NEt₃ | triethylamine |
| TFA | trifluoroacetic acid |
| THF | tetrahydrofuran |
| THP | tetrahydro-2H-pyranyl |
| THPO or OTHP | tetrahydro-2H-pyran-2-yl-oxy |
| TiCl₄ | titanium tetrachloride |
| TIPS-H | triisopropyl silyl-hydrogen |
| TLC | thin layer chromatography |
| TMSI | trimethylsilyl iodide |
| TMSOK | potassium trimethylsilanolate |
| TPAP | tetra-n-propylammonium perruthenate |

### Example 1

### 8-(dimethylamino)-2-oxo-1,2,5,6-tetrahydrobenzo[h]quinoline-3-carboxylic acid (Cpd 1)

A mixture of 6-(dimethylamino)-3,4-dihydronaphthalen-1(2H)-one (1.033 g, 5.45 mmol) and DMF-DMA (3 mL, 22.56 mmol) was microwaved at 200 °C 30 min. After cooling to room temperature, a precipitate was formed, then collected by filtration and washed with Et₂O to provide the product 6-(dimethylamino)-2-((dimethylamino)methylene)-3,4-dihydronaphthalen-1(2H)-one (0.769 g, 58%).
¹H NMR (500 MHz, CHCl₃-*d*) δ ppm 2.80 (t, *J*=7.1 Hz, 2 H) 2.92 (t, *J*=7.1 Hz, 2 H) 3.03 (s, 3 H) 3.09 (s, 3 H) 6.39 (d, *J*=2.7 Hz, 1 H) 6.62 (dd, *J*=8.7, 2.7 Hz, 1 H) 7.62 (s, 1 H) 7.96 (d, *J*=8.7 Hz, 1 H).

To a suspension of NaH (60% oil, 0.320 g, 8.00 mmol) in DMF (6 mL) was added a solution of 6-(dimethylamino)-2-((dimethylamino)methylene)-3,4-dihydronaphthalen-1(2H)-one (0.769 g, 3.15 mmol), cyanoacetamide (0.290 g, 3.45 mmol) and MeOH (0.32 mL, 8.00 mmol) in DMF (6 mL). The reaction was stirred at room temperature for 15 min and then heated to 95 °C overnight. After cooling to room temperature, the reaction was quenched with NH₄Cl (aqueous saturated). The resulting brownish precipitate was filtered, washed several times with H₂O, dried in an N₂-flow and washed with Et₂O. The product 8-(dimethylamino)-2-oxo-1,2,5,6-tetrahydrobenzo[h]quinoline-3-carbonitrile (0.80 g, 90%) was used directly in the next step without further purification. LC-MS 266.3 [M+H]⁺, RT 1.15 min

A mixture of 8-(dimethylamino)-2-oxo-1,2,5,6-tetrahydrobenzo[h]quinoline-3-carbonitrile (0.311 g, 1.17 mmol) with 6M HCl (10 mL) was heated at 100 °C for 36 h. After cooling to room temperature, a precipitate was formed, then collected by filtration and washed with water. The product was stirred vigorously with 1M NaOH (-20 mL) and the insoluble material was filtered off. The mother liquor was acidified with 1M HCl to pH~2. An orange solid was collected by filtration, was washed several times with H₂O, then dried in an N₂-flow and washed with Et₂O. The product 8-(dimethylamino)-2-oxo-1,2,5,6-tetrahydrobenzo[h]quinoline-3-carboxylic acid (0.045 g, 13%) was obtained. ¹H NMR (500 MHz, DMSO-*d*₆) δ ppm 2.67 - 2.75 (m, 2 H) 2.76 - 2.86 (m, 2 H) 3.03 (s, 6 H) 6.59 - 6.73 (m, 2 H) 7.95 (d, *J*=8.8 Hz, 1 H) 8.17 (s, 1 H) 12.91 (br. s., 1 H) 14.88 (br. s., 1 H). LC-MS 282.9 [M-H]⁻, 285.3 [M+H]⁺, RT 1.18 min.

### Example 2

### 9-(dimethylamino)-2-oxo-2,5,6,7-tetrahydro-1H-benzo[6,7]cyclohepta[1,2-b]pyridine-3-carboxylic acid (Cpd 2)

### Step 1: 4-(dimethylamino)-2-vinylbenzaldehyde

2-chloro-4-(dimethylamino)benzaldehyde (4.34 g, 23.63 mmol), potassium vinyltrifluoroborate (6.33 g, 47.26 mmol), Pd(OAc)₂ (160 mg, 0.71 mmol, 3 mol%), S-Phos ligand (580 mg, 1.41 mmol, 6 mol%) and K₂CO₃ (9.80 g, 70.91 mmol) were mixed together in a 250 mL round bottom flask. The flask was placed under vacuum and back filled with Argon, then dioxane (95.0 mL) and H₂O (16.0 mL) were added. The mixture was heated at 85-90 °C for 2.5 h and monitored by LC/MC for complete consumption of starting material. After completion, the reaction was cooled to room temperature, then water (60 mL) was added and the product was extracted with DCM (3x100 mL). The combined organics were washed with NaCl (aqueous saturated, 100 mL) and dried over Na₂SO₄. After concentration of the solvent, the residue was purified by column chromatography (EtOAc/hexanes, 0-40% gradient), affording the product as a pale yellow solid (4.03 g, 97%). ¹H NMR (500 MHz, CHCl₃-*d*) δ ppm 3.10 (s, 6 H) 5.44 (dd, *J*=11.0, 1.6 Hz, 1 H) 5.66 (dd, *J*=17.3, 1.6 Hz, 1 H) 6.66 (dd, *J*=8.7, 2.5 Hz, 1 H) 6.70 (d, *J*=2.5 Hz, 1 H) 7.56 (dd, *J*=17*.*3*,* 11.0 Hz, 1 H) 7.70 (d, *J*=8*.*7 Hz, 1 H) 10.00 (s, 1 H). LC-MS 176.2 [M+H]⁺, RT 1.13 min.

### Step 2: 1-(4-(dimethylamino)-2-vinylphenyl)pent-4-en-1-ol

To a solution of 4-(dimethylamino)-2-vinylbenzaldehyde (2.50 g, 14.27 mmol) in THF (30 mL) at -78 °C was added 3-butenylmagnesium bromide (0.5M in THF, 33.0 mL, 16.5 mmol) dropwise over ∼10 min. The reaction mixture was stirred at -78 °C for 10 min and slowly allowed to warm to 0 °C. After stirring for 30 min, the reaction was quenched by addition of NH₄Cl (aqueous saturated, 40 mL) and the product was extracted with EtOAc (3x75 mL). The combined organics were washed with NaCl (aqueous saturated, 50 mL) and dried over Na₂SO₄. The solvent was removed and the residue was purified by column chromatography (EtOAc/hexanes, 0-40% gradient), affording the product as a pale yellow solid (3.08 g, 93%).
¹H NMR (500 MHz, CHCl₃-*d*) δ ppm 1.70 (d, *J*=3.2 Hz, 1 H) 1.75 - 1.95 (m, 2 H) 2.08 - 2.27 (m, 2 H) 2.98 (s, 6 H) 4.92 - 5.01 (m, 2 H) 5.05 (dq, *J*=17.1, 1.8 Hz, 1 H) 5.31 (dd, *J*=10.9, 1.4 Hz, 1 H) 5.62 (dd, *J*=17*.*3*,* 1.7 Hz, 1 H) 5.86 (ddt, *J*=17.1, 10.2,6.6 Hz, 1 H) 6.60 - 6.87 (m, 2 H) 7.09 (dd, *J*=17.3, 10.9 Hz, 1 H) 7.35 (d, *J*=8.8 Hz, 1 H). LC-MS 232.2 [M+H]⁺, RT 0.96 min.

### Step 3: 2-(dimethylamino)-6,7-dihydro-5H-benzo[7]annulen-5-ol

1-(4-(dimethylamino)-2-vinylphenyl)pent-4-en-1-ol (1.96 g, 8.47 mmol) was dissolved in toluene (170 mL, 0.05M) under Argon. A second generation Grubbs' catalyst (215 mg, 0.25 mmol, 3 mol%) was added and the mixture was heated at 60 °C for 1.5-2 h until the starting material was completely consumed as indicated by LC/MS. After cooling the reaction to room temperature, the toluene was removed under reduced pressure and the residue was purified by column chromatography (EtOAc/hexanes, 0-40% gradient) to provide the product as a pale yellow solid (1.58 g, 92%).
¹H NMR (500 MHz, CHCl₃-*d*) δ ppm 1.77 (d, *J*=5*.*0 Hz, 1 H) 1.96 - 2.09 (m, 1 H) 2.15 - 2.29 (m, 1 H) 2.37 - 2.50 (m, 1 H) 2.58 - 2.72 (m, 1 H) 2.96 (s, 6 H) 4.88 (t, *J*=6.3 Hz, 1 H) 5.94 (ddd, *J*=12.3, 5.4, 4.1 Hz, 1 H) 6.37 (d, *J*=12.3 Hz, 1 H) 6.60 (br. s., 2 H) 7.25 (d, *J*=8.8 Hz, 1 H). LC-MS 204.2 [M+H]⁺, RT 0.59 min.

### Step 4: 2-(dimethylamino)-6,7-dihydro-5H-benzo[7]annulen-5-one

To a solution of 1-(4-(dimethylamino)-2-vinylphenyl)pent-4-en-1-ol (1.57 g, 7.72 mmol) in DCM (60 mL) was added DDQ (2.10 g, 9.25 mmol). The mixture was vigorously stirred at room temperature for 20 min, then the solids were filtered off and washed with DCM (10 mL). The mother liquor was concentrated and the residue was purified by column chromatography (EtOAc/hexanes, 0-40% gradient) to afford 2-(dimethylamino)-6,7-dihydro-5H-benzo[7]annulen-5-one (1.00 g, 64%) as a solid.
¹H NMR (500 MHz, CHCl₃-*d*) δ ppm 2.38 - 2.48 (m, 2 H) 2.76 - 2.96 (m, 2 H) 3.06 (s, 6 H) 6.22 (dt, *J*=11.6, 5.7 Hz, 1 H) 6.42 (s, 1 H) 6.44 (d, *J*=7*.*9 Hz, 1 H) 6.60 (dd, *J*=9.0, 2.7 Hz, 1 H) 8.01 (d, *J*=7.9 Hz, 1 H). LC-MS 202.2 [M+H]⁺, RT 1.18 min.

### Step 5: 2-(dimethylamino)-6,7,8,9-tetrahydro-5H-benzo[7]annulen-5-one

A solution of 2-(dimethylamino)-6,7-dihydro-5H-benzo[7]annulen-5-one (1.20 g, 5.97 mmol) in MeOH (35 mL) and DCM (5 mL) was hydrogenated under 1 atm of H₂ over Pd/C (10%, 180 mg) for 40 min and monitored by LC/MS to avoid reduction of the ketone. After complete consumption of the starting material, the catalyst was filtered off and the filtrate was washed with DCM (10 mL). The mother liquor was concentrated and the residue was purified by column chromatography (EtOAc/hexanes, 0-40% gradient) to provide the product as a solid (1.00 g, 83%).
¹H NMR (500 MHz, CHCl₃-*d*) δ ppm 1.76 - 1.84 (m, 2 H) 1.84 - 1.92 (m, 2 H) 2.65 - 2.75 (m, 2 H) 2.91 (t, *J*=6.4 Hz, 2 H) 3.05 (s, 6 H) 6.42 (d, *J*=2.8 Hz, 1 H) 6.59 (dd, *J*=8.8*,* 2.8 Hz, 1 H) 7.80 (d, *J*=8.8 Hz, 1 H). LC-MS 204.2 [M+H]⁺, RT 1.16 min.

### Step 6: 5-((2,4-dimethoxybenzyl)imino)-N,N-dimethyl-6,7,8,9-tetrahydro-5H-benzo[7]annulen-2-amine

To a solution of 2-(dimethylamino)-6,7,8,9-tetrahydro-5H-benzo[7]annulen-5-one (1.00 g, 4.92 mmol) in DCM (10 mL) was added 2,4-dimethoxybenzylamine (0.85 mL, 5.65 mmol) and NEt₃ (1.80 mL, 12.91 mmol). The mixture was cooled to 0 °C, then a TiCl₄ solution (1M DCM, 3.2 mL, 3.2 mmol) was added dropwise via syringe pump over 30 min. The reaction was allowed to warm to room temperature and stirred overnight. The mixture was diluted with DCM (20 mL) and the reaction was quenched with NaHCO₃ (aqueous saturated, 20 mL). After vigorous shaking, the organic phase was separated using a PTFE phase separator, then dried over Na₂SO₄. The solvent was removed to provide the product (1.70 g, quant) as a yellow oil, which was used directly in the next step without further purification.

### Step 7: methyl 1-(2,4-dimethoxybenzyl)-9-(dimethylamino)-2-oxo-2,5,6,7-tetrahydro-1H-benzo[6,7]cyclohepta[1,2-b]pyridine-3-carboxylate

The obtained product 5-((2,4-dimethoxybenzyl)imino)-N,N-dimethyl-6,7,8,9-tetrahydro-5H-benzo[7]annulen-2-amine (0.85 g, 2.41 mmol) and dimethyl 2-(methoxymethylene)malonate (0.83 g, 4.77 mmol) were mixed together in Ph₂O (5.50 mL). With stirring, the mixture was placed onto a pre-heated heat block at 220 °C and heated for 15 min after the initial bubbling of MeOH was observed (occurs at ∼160 °C internal reaction temperature). The reaction mixture was cooled to room temperature and purified via column chromatography (hexanes followed by EtOAc/hexanes 40-90% gradient) to provide the product as a yellow foam (0.256 g, 23% in 2 steps)
¹H NMR (500 MHz, CHCl₃-*d*) δ ppm 1.94 - 2.08 (m, 3 H) 2.27 - 2.39 (m, 2 H) 2.46 (dd, *J*=13.2, 5.7 Hz, 1 H) 3.00 (s, 6 H) 3.62 (s, 3 H) 3.76 (s, 3 H) 3.91 (s, 3 H) 5.23 (d, *J*=15.8 Hz, 1 H) 5.29 (d, *J*=15.*8* Hz, 1 H) 6.34 (d, *J*=2.5 Hz, 1 H) 6.36 (dd, *J*=8.2, 2.5 Hz, 1 H) 6.39-6.57 (m, 2 H) 6.79 (d, *J*=8.2 Hz, 1 H) 6.99 (d, *J*=8.5 Hz, 1 H) 8.11 (s, 1 H). LC-MS 463.4 [M+H]⁺, RT 1.37 min.

### Step 8: 1-(2,4-dimethoxybenzyl)-9-(dimethylamino)-2-oxo-2,5,6,7-tetrahydro-1H-benzo[6,7]cyclohepta[1,2-b]pyridine-3-carboxylic acid

A mixture of methyl 1-(2,4-dimethoxybenzyl)-9-(dimethylamino)-2-oxo-2,5,6,7-tetrahydro-1H-benzo[6,7]cyclohepta[1,2-b]pyridine-3-carboxylate (0.236 g, 0.51 mmol) and LiOH (1M aq, 1.00 mL, 1.00 mmol) in THF (2 mL) was stirred at room temperature for 45 min, then acidified with 1M HCl to pH~2 and the product was extracted with DCM (3x10 mL). After drying the organic phase over Na₂SO₄, the solvents were removed to afford the product 1-(2,4-dimethoxybenzyl)-9-(dimethylamino)-2-oxo-2,5,6,7-tetrahydro-1H-benzo[6,7]cyclohepta[1,2-b]pyridine-3-carboxylic acid (0.23 g) as a solid in quantitative yield. LC-MS 449.4 [M+H]⁺, RT 1.42 min

### Step 9: 9-(dimethylamino)-2-oxo-2,5,6,7-tetrahydro-1H-benzo[6,7]cyclohepta[1,2-b]pyridine-3-carboxylic acid

A solution of 1-(2,4-dimethoxybenzyl)-9-(dimethylamino)-2-oxo-2,5,6,7-tetrahydro-1H-benzo[6,7]cyclohepta[1,2-b]pyridine-3-carboxylic acid (0.23 g, 0.50 mmol) in DCM (2 mL) was treated with i-Pr₃SiH (0.50 mL) and TFA (1.0 mL). After stirring for 45 min at room temperature, the solvents were removed under reduced pressure and the residue was triturated with Et₂O. The solid was filtered and washed with Et₂O, affording the product 9-(dimethylamino)-2-oxo-2,5,6,7-tetrahydro-1H-benzo[6,7]cyclohepta[1,2-b]pyridine-3-carboxylic acid (0.120 g, 80% 2 steps) as a bright yellow solid.
¹H NMR (500 MHz, DMSO-*d₆*) δ ppm 2.11 (quin, *J*=6.9 Hz, 2 H) 2.31 (t, *J*=6*.*9 Hz, 2 H) 2.49 - 2.54 (m, 2 H) 3.00 (s, 6 H) 6.67 - 6.73 (m, 2 H) 7.37 (d, *J*=9*.*5 Hz, 1 H) 8.26 (s, 1 H) 13.17 (br. s., 1 H) 15.05 (br. s., 1 H). LC-MS 297.3 [M-H]⁻, 299.2 [M+H]⁺, RT 1.07 min.

### Example 3

### 9-(dimethylamino)-4-hydroxy-2-oxo-2,5,6,7-tetrahydro-1H-benzo[6,7]cyclohepta[1,2-b]pyridine-3-carboxylic acid (Cpd 3)

### Step 1: methyl 1-(2,4-dimethoxybenzyl)-9-(dimethylamino)-4-hydroxy-2-oxo-2,5,6,7-tetrahydro-1H-benzo[6,7]cyclohepta[1,2-b]pyridine-3-carboxylate

The previously obtained product 5-((2,4-dimethoxybenzyl)imino)-*N*,*N-*dimethyl-6,7,8,9-tetrahydro-5H-benzo[7]annulen-2-amine (Example 1, step 6, 0.85 g, 2.41 mmol) and trimethyl methanetricarboxylate (0.87 g, 4.58 mmol) were mixed together in Ph₂O (5.5 mL). With stirring, the mixture was placed onto a pre-heated heat block at 220 °C and heated for 15 min after the initial bubbling of MeOH was observed (occurs at ∼160 °C internal reaction temperature). The reaction mixture was cooled to room temperature, then purified by column chromatography (hexanes, followed by EtOAc/hexanes 40-90% gradient) to provide the product as a yellow foam (0.525 g, 45% 2 steps).
¹H NMR (500 MHz, CHCl₃-*d*) δ ppm 1.58 (td, *J*=13*.*6*,* 6.9 Hz, 1 H) 1.86 - 2.08 (m, 2 H) 2.33 (td, *J*=12*.*7*,* 7.7 Hz, 1 H) 2.45 (dd, *J*=13.1*,* 6.1 Hz, 1 H) 2.97 - 3.01 (m, 1 H) 3.00 (s, 6 H) 3.61 (s, 3 H) 3.76 (s, 3 H) 3.97 (s, 3 H) 5.14 (br. s., 2 H) 6.33 (d, *J*=2.2 Hz, 1 H) 6.36 (dd, *J*=8.4*,* 2.2 Hz, 1 H) 6.50 (br. s., 2 H) 6.79 (d, *J*=8*.*4 Hz, 1 H) 7.00 (d, *J*=8.4 Hz, 1 H) 13.65 (s, 1 H). LC-MS 477.4 [M-H]⁻, 479.4 [M+H]⁺, RT 1.46 min.

### Step 2: 1-(2,4-dimethoxybenzyl)-9-(dimethylamino)-4-hydroxy-2-oxo-2,5,6,7-tetrahydro-1H-benzo[6,7]cyclohepta[1,2-b]pyridine-3-carboxylic acid

To a suspension of methyl 1-(2,4-dimethoxybenzyl)-9-(dimethylamino)-4-hydroxy-2-oxo-2,5,6,7-tetrahydro-1H-benzo[6,7]cyclohepta[1,2-b]pyridine-3-carboxylate (0.256 g, 0.53 mmol) in EtOAc (2.0 mL) was added LiI (0.210 g, 1.57 mmol). The reaction mixture was stirred and heated at 60 °C for 1 h until complete consumption of starting material was observed. The mixture was then cooled to room temperature and acidified with HCl (1M aq, 2 mL). The product was extracted with DCM (3x7 mL) and the organic phase was washed with NaCl (aqueous saturated, 7 mL) and dried over Na₂SO₄. The solvent was removed to provide the product as a yellow solid (0.24 g) in quantitative yield. LC-MS 463.4 [M-H]⁻, 465.3 [M+H]⁺, RT 1.46 min.

### Step 3: 9-(dimethylamino)-4-hydroxy-2-oxo-2,5,6,7-tetrahydro-1H-benzo[6,7]cyclohepta[1,2-b]pyridine-3-carboxylic acid

A solution of 1-(2,4-dimethoxybenzyl)-9-(dimethylamino)-4-hydroxy-2-oxo-2,5,6,7-tetrahydro-1H-benzo[6,7]cyclohepta[1,2-b]pyridine-3-carboxylic acid (0.24 g, 0.51 mmol) in DCM (2 mL) was treated with i-Pr₃SiH (1.0 mL) and TFA (1.0 mL). After stirring for 45 min at 45 °C, the solvents were removed under reduced pressure and the residue was triturated with Et₂O. The solid was filtered and washed with Et₂O, affording 9-(dimethylamino)-4-hydroxy-2-oxo-2,5,6,7-tetrahydro-1H-benzo[6,7]cyclohepta[1,2-b]pyridine-3-carboxylic acid (0.139 g, 83% 2 steps) as a bright yellow solid.
¹H NMR (500 MHz, DMSO-*d*₆) δ ppm 2.08 (quin, *J*=7*.*0 Hz, 2 H) 2.21 - 2.30 (m, 2 H) 2.51-2.55 (m, 2 H) 3.00 (s, 6 H) 6.65 - 6.73 (m, 2 H) 7.35 (d, *J*=9.5 Hz, 1 H) 12.68 (s, 1 H) 13.77 (s, 1 H). LC-MS 313.3 [M-H]⁻, 315.3 [M+H]⁺, RT 1.27 min.

### Example 4

### 10-(dimethylamino)-2-oxo-1,2,5,6,7,8-hexahydrobenzo[7,8]cycloocta[1,2-b]pyridine-3-carboxylic acid (Cpd 4)

### Step 1: 1-(4-(dimethylamino)-2-vinylphenyl)hex-5-en-1-ol

To a solution of 4-(dimethylamino)-2-vinylbenzaldehyde (Example 2, step 1, 1.75 g, 10.0 mmol) in THF (20 mL) at -78 °C was added pent-4-en-1-ylmagnesium bromide (0.5M in MeOH-THF, 23.0 mL, 11.5 mmol) dropwise over ∼10 min. The reaction mixture was stirred at -78 °C for 10 min and slowly allowed to warm to 0 °C. After stirring at 0 °C for 30 min, the reaction was quenched by addition of NH₄Cl (aqueous saturated, 40 mL). The product was extracted with EtOAc (3x50 mL), then the combined organics were washed with NaCl (aqueous saturated, 50 mL) and dried over Na₂SO₄. The solvent was removed to provide the product as a pale-yellow oil (2.30 g, 94%), which solidified under high-vacuum. The product was used directly in the next step without further purification.
¹H NMR (500 MHz, CHCl₃-*d*) δ ppm 1.36 - 1.46 (m, 1 H) 1.49 - 1.62 (m, 1 H) 1.62 - 1.87 (m, 2 H) 2.08 (q, *J*=7.3 Hz, 2 H) 2.97 (s, 6 H) 4.90 - 4.97 (m, 2 H) 5.00 (dd, *J*=17.0, 1.9 Hz, 1 H) 5.31 (dd, *J*=11.0, 1.6 Hz, 1 H) 5.61 (dd, *J*=17.2, 1.4 Hz, 1 H) 5.80 (ddt, *J*=17*.*0*,* 10.2, 6.7, 6.7 Hz, 1 H) 6.73 (dd, *J*=8.5, 2.5 Hz, 1 H) 6.81 (d, *J*=2.5 Hz, 1 H) 7.10 (dd, *J*=17*.*2*,* 11.0 Hz, 1 H) 7.33 (d, *J*=8.5 Hz, 1 H). LC-MS 246.3 [M+H]⁺, RT 1.07 min.

### Step 2: 4-(1-((tert-butyldimethylsilyl)oxy)hex-5-en-1-yl)-N,N-dimethyl-3-vinylaniline

To a solution of the crude product 1-(4-(dimethylamino)-2-vinylphenyl)hex-5-en-1-ol (1.38 g, 5.63 mmol) in DCM (20 mL) was added imidazole (0.50 g, 7.34 mmol). The mixture was stirred at room temperature for 5 min, then TBSCl (1.02 g 6.76 mmol) was added in several portions. The reaction mixture was stirred at room temperature for 1.5 h, then diluted with DCM (50 mL) and washed with H₂O (40 mL). The organic phase was dried over Na₂SO₄ and the solvents were removed. The residue was purified by column chromatography (EtOAc/hexanes, 0-10% gradient) to afford 4-(1-((tert-butyldimethylsilyl)oxy)hex-5-en-1-yl)-N,N-dimethyl-3-vinylaniline (1.90 g, 94%) as an oil.
¹H NMR (500 MHz, CHCl₃-*d*) δ ppm 1.70 (d, *J*=3.2 Hz, 1 H) 1.75 - 1.95 (m, 2 H) 2.08 - 2.27 (m, 2 H) 2.98 (s, 6 H) 4.92 - 5.01 (m, 2 H) 5.05 (dq, *J*=17.1, 1.8 Hz, 1 H) 5.31 (dd, *J*=10.9, 1.4 Hz, 1 H) 5.62 (dd, *J*=17.3, 1.7 Hz, 1 H) 5.86 (ddt, *J*=17.1, 10.2, 6.6 Hz, 1 H) 6.60 - 6.87 (m, 2 H) 7.09 (dd, *J*=17.3, 10.9 Hz, 1 H) 7.35 (d, *J*=8*.*8 Hz, 1 H). LC-MS 360.4 [M+H]⁺, RT 2.03 min.

### Step 3: 5-((tert-butyldimethylsilyl)oxy)-N,N-dimethyl-5,6,7,8-tetrahydrobenzo [8] annulen-2-amine

4-(1-((tert-butyldimethylsilyl)oxy)hex-5-en-1-yl)-N,N-dimethyl-3-vinylaniline (1.88 g, 5.23 mmol) was dissolved in toluene (110 mL, 0.05M) under Argon. A second generation Grubbs' catalyst (215 mg, 0.25 mmol, 5 mol%) was added and the mixture was heated at 60 °C for 1.5-2 h until the starting material was completely consumed as indicated by LCMS. After cooling the reaction to room temperature, the toluene was removed under reduced pressure and the residue was purified by column chromatography (EtOAc/hexanes, 0-10% gradient) to afford the product (1.04 g, 60%).
¹H NMR (500 MHz, CHCl₃-*d*) δ ppm -0.05 (s, 3 H) -0.03 (s, 3 H) 0.88 (s, 9 H) 1.34 - 1.53 (m, 2 H) 1.51 - 1.63 (m, 1 H) 1.89 - 1.98 (m, 1 H) 2.11 - 2.25 (m, 1 H) 2.25 - 2.37 (m, 1 H) 2.94 (s, 6 H) 5.06 (dd, *J*=11.0, 4.4 Hz, 1 H) 5.82 (ddd, *J*=12.3, 5.4, 4.4 Hz, 1 H) 6.35 (d, *J*=12.3 Hz, 1 H) 6.45 (br. s., 1 H) 6.73 (d, *J*=8.5 Hz, 1 H) 7.41 (d, *J*=8.5 Hz, 1 H). LC-MS 332.4 [M+H]⁺, RT 1.93 min.

### Step 4: 2-(dimethylamino)-5,6,7,8-tetrahydrobenzo[8]annulen-5-ol

To a solution of 5-((tert-butyldimethylsilyl)oxy)-N,N-dimethyl-5,6,7,8-tetrahydrobenzo[8]annulen-2-amine (0.954 g, 2.88 mmol) in THF (12 mL) was added a solution of TBAF (1M THF, 3.50 mL, 3.50 mmol). The reaction mixture was stirred at room temperature overnight and additional TBAF (1M THF, 1.00 mL, 1.00 mmol) was added. The mixture was stirred for another 2 h at room temperature and the THF was removed. The residue was purified by column chromatography (EtOAc/hexanes, 0-50% gradient) to afford the product 2-(dimethylamino)-5,6,7,8-tetrahydrobenzo[8]annulen-5-ol (0.595 g, 95%).
¹H NMR (500 MHz, CHCl₃-*d*) δ ppm 1.41 - 1.63 (m, 3 H) 1.84 (br. s., 1 H) 1.99 - 2.11 (m, 1 H) 2.16 - 2.30 (m, 2 H) 2.94 (s, 6 H) 5.13 (dd, *J*=10*.*9, 4.3 Hz, 1 H) 5.85 (dt, *J*=12*.*1*,* 5.4 Hz, 1 H) 6.39 (d, *J*=12*.*1 Hz, 1 H) 6.49 (d, *J*=2.5 Hz, 1 H) 6.76 (dd, *J*=8*.*7*,* 2.5 Hz, 1 H) 7.43 (d, *J*=8.7 Hz, 1 H). LC-MS 218.3 [M+H]⁺, RT 0.67 min.

### Step 5: 2-(dimethylamino)-7,8-dihydrobenzo[8]annulen-5(6H)-one

To a solution of 2-(dimethylamino)-5,6,7,8-tetrahydrobenzo[8]annulen-5-ol (0.595 g, 2.74 mmol) in DCM (20 mL) was added DDQ (0.750 g, 3.30 mmol). The mixture was vigorously stirred at room temperature for 20 min, then the solids were filtered off and washed with DCM (10 mL). The mother liquor was concentrated and the residue was purified by column chromatography (EtOAc/hexanes, 0-30% gradient) to afford 2-(dimethylamino)-7,8-dihydrobenzo[8]annulen-5(6H)-one (0.381 g, 65%) as a solid.
¹H NMR (500 MHz, CHCl₃-*d*) δ ppm 2.00 - 2.07 (m, 2 H) 2.12 - 2.19 (m, 2 H) 2.79 - 2.88 (m, 2 H) 3.05 (s, 6 H) 6.02 (dt, *J*=11.7, 8.5 Hz, 1 H) 6.41 (d, *J*=2.7 Hz, 1 H) 6.65 (dd, *J*=9.0*,* 2.7 Hz, 1 H) 6.82 (d, *J*=11*.*7 Hz, 1 H) 8.01 (d, *J*=9.0 Hz, 1 H). LC-MS 216.2 [M+H]⁺, RT 1.24 min.

### Step 6: 2-(dimethylamino)-6,7,8,9-tetrahydro-5H-benzo[7]annulen-5-one

A solution of 2-(dimethylamino)-7,8-dihydrobenzo[8]annulen-5(6H)-one (0.381 g, 1.75 mmol) in MeOH (10 mL) and DCM (1 mL) was hydrogenated under 1 atm of H₂ over Pd/C (10%, 50 mg) for 60 min and monitored by LCMS to avoid reduction of the ketone. After complete consumption of the starting material, the catalyst was filtered off and the filtrate was washed with DCM (10 mL). The mother liquor was concentrated to afford the product (0.358 g, 93%) as a white solid.
¹H NMR (500 MHz, CHCl₃-*d*) δ ppm 1.38 - 1.48 (m, 2 H) 1.75 - 1.92 (m, 4 H) 3.03 (t, *J*=7.4 Hz, 2 H) 3.05 (s, 6 H) 3.18 (t, *J*=6.9 Hz, 2 H) 6.39 (d, *J*=2.8 Hz, 1 H) 6.60 (dd, *J*=9.1*,* 2.8 Hz, 1 H) 8.06 (d, *J*=9.1 Hz, 1 H). LC-MS 218.2 [M+H]⁺, RT 1.27 min.

### Step 7: 5-((2,4-dimethoxybenzyl)imino)-N,N-dimethyl-5,6,7,8,9,10-hexahydrobenzo[8]annulen-2-amine

To a solution of 2-(dimethylamino)-6,7,8,9-tetrahydro-5H-benzo[7]annulen-5-one (0.358 g, 1.64 mmol) in DCM (5 mL) was added 2,4-dimethoxybenzylamine (0.27 mL, 1.87 mmol) and NEt₃ (0.65 mL, 4.66 mmol). The mixture was cooled to 0 °C, then a solution of TiCl₄ (1M DCM, 1.10 mL, 1.10 mmol) was added dropwise via syringe pump over 30 min. The reaction mixture was allowed to warm to room temperature and stirred overnight. The mixture was diluted with DCM (10 mL) and the reaction was quenched with NaHCO₃ (aqueous saturated, 5 mL). After vigorous shaking, the organic phase was separated using a PTFE phase separator, then dried over Na₂SO₄. The solvent was removed to provide the product (0.60 g, quant) as a yellow oil, which was used directly in the next step without further purification.

### Step 8: methyl 1-(2,4-dimethoxybenzyl)-10-(dimethylamino)-2-oxo-1,2,5,6,7,8-hexahydrobenzo[7,8]cycloocta[1,2-b]pyridine-3-carboxylate

The product 5-((2,4-dimethoxybenzyl)imino)-N,N-dimethyl-5,6,7,8,9,10-hexahydrobenzo[8]annulen-2-amine (0.30 g, 0.82 mmol) and dimethyl 2-(methoxymethylene)malonate (0.26 g, 1.49 mmol) were mixed together in Ph₂O (2.0 mL). With stirring, the mixture was placed onto a pre-heated heat block at 200 °C and heated for 10 min after the initial bubbling of MeOH was observed (occurs at ∼160 °C internal reaction temperature). The reaction mixture was cooled to room temperature, then purified by column chromatography (hexanes, followed by EtOAc/hexanes 0-70% gradient) to provide the product as a yellow foam (0.112 g, 29% 2 steps)
¹H NMR (500 MHz, CHCl₃-*d*) δ ppm 1.30 - 1.45 (m, 2 H) 1.83 (dd, *J*=13.9*,* 11.0 Hz, 1 H) 1.87 - 1.99 (m, 3 H) 2.39 (dd, *J*=13.2*,* 7.6 Hz, 1 H) 2.47 (dd, *J*=13.9*,* 7.3 Hz, 1 H) 2.98 (s, 6 H) 3.51 (s, 3 H) 3.75 (s, 3 H) 3.94 (s, 3 H) 5.11 (d, *J*=15.1 Hz, 1 H) 5.28 (d, *J*=15.1 Hz, 1 H) 6.25 (d, *J*=2.2 Hz, 1 H) 6.32 (dd, *J*=8.5*,* 2.2 Hz, 1 H) 6.44 (s, 1 H) 6.48 (dd, *J*=8.5*,* 2.2 Hz, 1 H) 6.73 (d, *J*=8.5 Hz, 1 H) 6.84 (d, *J*=8.5 Hz, 1 H) 8.14 (s, 1 H). LC-MS 477.4 [M+H]⁺, RT 1.45 min.

### Step 9: 1-(2,4-dimethoxybenzyl)-10-(dimethylamino)-2-oxo-1,2,5,6,7,8-hexahydrobenzo[7,8]cycloocta[1,2-b]pyridine-3-carboxylic acid

A mixture of methyl 1-(2,4-dimethoxybenzyl)-10-(dimethylamino)-2-oxo-1,2,5,6,7,8-hexahydrobenzo[7,8]cycloocta[1,2-b]pyridine-3-carboxylate (0.112 g, 0.24 mmol) and LiOH (1M aq, 0.50 mL, 0.50 mmol) in THF (1 mL) was stirred at room temperature for 2 h. The mixture was then acidified with 1M HCl to pH~2 and the product was extracted with DCM (3x5 mL). After drying the organic phase over Na₂SO₄, the solvents were removed to afford 1-(2,4-dimethoxybenzyl)-10-(dimethylamino)-2-oxo-1,2,5,6,7,8-hexahydrobenzo[7,8]cycloocta[1,2-b]pyridine-3-carboxylic acid (0.107 g) as a solid in quantitative yield. LC-MS 463.4 [M+H]⁺, RT 1.48 min.

### Step 10: 10-(dimethylamino)-2-oxo-1,2,5,6,7,8-hexahydrobenzo[7,8]cycloocta[1,2-b]pyridine-3-carboxylic acid

A solution of 1-(2,4-dimethoxybenzyl)-10-(dimethylamino)-2-oxo-1,2,5,6,7,8-hexahydrobenzo[7,8]cycloocta[1,2-b]pyridine-3-carboxylic acid (0.107 g, 0.23 mmol) in DCM (1 mL) was treated with i-Pr₃SiH (0.25 mL) and TFA (0.50 mL). After stirring for 45 min at room temperature, the solvents were removed under reduced pressure and the residue was triturated with Et₂O. The solid was filtered and washed with Et₂O, affording 10-(dimethylamino)-2-oxo-1,2,5,6,7,8-hexahydrobenzo[7,8]cycloocta[1,2-b]pyridine-3-carboxylic acid (0.0478 g, 65% 2 steps) as a bright yellow solid.
¹H NMR (500 MHz, DMSO-*d*₆) δ ppm 1.30 - 1.43 (m, 2 H) 1.76 (dd, *J*=13.6*,* 11.3 Hz, 1 H) 1.96 (t, *J*=7.9 Hz, 1 H) 2.02 (t, *J*=9.1 Hz, 1 H) 2.08 (t, *J*=11.3 Hz, 1 H) 2.69 - 2.80 (m, 1 H) 2.98 (s, 3 H) 6.65 - 6.69 (m, 2 H) 7.19 (d, *J*=8.5 Hz, 1 H) 8.31 (s, 1 H) 13.10 (br. s., 1 H) 15.07 (br. s., 1 H). LC-MS 311.3 [M-H]⁻, 313.2 [M+H]⁺, RT 1.13 min.

### Example 5

### 10-(dimethylamino)-4-hydroxy-2-oxo-1,2,5,6,7,8-hexahydrobenzo[7,8]cycloocta[1,2-b]pyridine-3-carboxylic acid (Cpd 5)

### Step 1: methyl 1-(2,4-dimethoxybenzyl)-10-(dimethylamino)-4-hydroxy-2-oxo-1,2,5,6,7,8-hexahydrobenzo[7,8]cycloocta[1,2-b]pyridine-3-carboxylate

The previously obtained product 5-((2,4-dimethoxybenzyl)imino)-N,N-dimethyl-5,6,7,8,9,10-hexahydrobenzo[8]annulen-2-amine (Example 4, step 7, 0.30 g, 0.82 mmol) and trimethyl methanetricarboxylate (0.28 g, 1.47 mmol) were mixed together in Ph₂O (2.0 mL). With stirring, the mixture was placed onto a pre-heated heat block at 220 °C and heated for 15 min after the initial bubbling of MeOH was observed (occurs at ∼160 °C internal reaction temperature). The reaction mixture was cooled to room temperature, then purified by column chromatography (hexanes, followed by EtOAc/hexanes 0-70% gradient) to provide the product as a yellow foam (0.178 g, 44% 2 steps)
¹H NMR (500 MHz, CHCl₃-*d*) δ ppm 1.29 - 1.44 (m, 2 H) 1.50 (dd, *J*=13.4, 11.2 Hz, 1 H) 1.84 - 2.03 (m, 3 H) 2.41 (dd, *J*=13.1, 7.7 Hz, 1 H) 2.86 (dd, *J*=13.7, 7.7 Hz, 1 H) 2.98 (s, 6 H) 3.51 (s, 3 H) 3.75 (s, 3 H) 4.00 (s, 3 H) 5.01 (d, *J*=15.8 Hz, 1 H) 5.17 (d, *J*=15.8 Hz, 1 H) 6.25 (d, *J*=2*.*4 Hz, 1 H) 6.33 (dd, *J*=8.5*,* 2.4 Hz, 1 H) 6.41 - 6.53 (m, 2 H) 6.74 (d, *J*=8.5 Hz, 1 H) 6.85 (d, *J*=8.5 Hz, 1 H) 13.71 (s, 1 H). LC-MS 491.4 [M-H]⁻, 493.4 [M+H]⁺, RT 1.57 min.

### Step 2: 1-(2,4-dimethoxybenzyl)-10-(dimethylamino)-4-hydroxy-2-oxo-1,2,5,6,7,8-hexahydrobenzo[7,8]cycloocta[1,2-b]pyridine-3-carboxylic acid

To a suspension of methyl 1-(2,4-dimethoxybenzyl)-10-(dimethylamino)-4-hydroxy-2-oxo-1,2,5,6,7,8-hexahydrobenzo[7,8]cycloocta[1,2-b]pyridine-3-carboxylate (0.178 g, 0.36 mmol) in EtOAc (2.0 mL) was added LiI (0.14 g, 1.05 mmol). The reaction mixture was stirred and heated at 60 °C for 1 h until complete consumption of starting material was observed. The mixture was then cooled to room temperature and acidified with HCl (1M aq, 1 mL). The product was extracted with DCM (3x7 mL), then the organic phase was washed with NaCl (aqueous saturated, 5 mL) and dried over Na₂SO₄. The solvent was removed to provide the product as a yellow solid (0.17 g) in nearly quantitative yield. LC-MS 477.4 [M-H]⁻, 479.4 [M+H]⁺, RT 1.64 min.

### Step 3: 10-(dimethylamino)-4-hydroxy-2-oxo-1,2,5,6,7,8-hexahydrobenzo[7,8]cycloocta[1,2-b]pyridine-3-carboxylic acid

A solution of 1-(2,4-dimethoxybenzyl)-10-(dimethylamino)-4-hydroxy-2-oxo-1,2,5,6,7,8-hexahydrobenzo[7,8]cycloocta[1,2-b]pyridine-3-carboxylic acid (0.17 g, 0.36 mmol) in DCM (1.5 mL) was treated with i-Pr₃SiH (0.7 mL) and TFA (0.7 mL). After stirring for 1 h at 45 °C, the solvents were removed under reduced pressure and the residue was triturated with Et₂O. The solid was filtered and washed with Et₂O affording 10-(dimethylamino)-4-hydroxy-2-oxo-1,2,5,6,7,8-hexahydrobenzo[7,8]cycloocta[1,2-b]pyridine-3-carboxylic acid (0.094 g, 80% 2 steps) as bright yellow solid.
¹H NMR (500 MHz, DMSO-d₆) δ ppm 1.27 - 1.44 (m, 2 H) 1.49 (dd, *J*=13.6, 11.3 Hz, 1 H) 1.85 - 1.94 (m, 1 H) 1.97 - 2.07 (m, 1 H) 2.14 (t, *J*=12.3 Hz, 1 H) 2.73 - 2.85 (m, 2 H) 2.98 (s, 6 H) 6.65 - 6.71 (m, 2 H) 7.19 (d, *J*=9.5 Hz, 1 H) 12.63 (br. s., 1 H) 13.87 (br. s., 1 H). LC-MS 327.3 [M-H]⁻, 329.2 [M+H]⁺, RT 1.34 min.

### Example 6

### 9-(3-(dimethylamino)pyrrolidin-1-yl)-4-hydroxy-2-oxo-2,5,6,7-tetrahydro-1H-benzo[6,7]cyclohepta[1,2-b]pyridine-3-carboxylic acid hydrochloride (Cpd 6)

### Step 1: 2-chloro-6,7,8,9-tetrahydro-5H-benzo[7]annulen-5-one

The subject 7-membered ketone was analogously prepared in three steps according to a literature procedure (J. Med. Chem. 2005, 48, 7351-7362). 3-chlorobenzaldehyde (5.62 g, 40.0 mmol) was employed in the Wittig reaction to afford the desired acid (7.3 g, 34.7 mmol, 87%) as a white solid after flash column chromatography purification (0-25% EtOAc in CH₂Cl₂).

To a solution of the Wittig product (5.18 g, 24.6 mmol) in EtOAc (100 mL) was added Pd/C (10% Degussa type, 1.2 g, 1.13 mmol, 0.05 eq) at room temperature. The mixture was evacuated then back filled with H₂ for three cycles. After 1 h, the mixture was filtered through Celite and washed with EtOAc (3x40 mL). The filtrate was concentrated to give a crude product (5.2 g, 24.5 mmol) in quantitative yield which was used in the next step without further purification.

The product obtained above (5.2 g, 24.5 mmol) was employed to give the desired ketone (2.82 g, 14.5 mmol, 59%) as a solid after flash column chromatography purification (0-25% EtOAc in CH₂Cl₂).
¹H NMR (500 MHz, CHCl₃-*d*) δ ppm 1.77 - 1.86 (m, 2 H) 1.86 - 1.94 (m, 2 H) 2.70 - 2.77 (m, 2 H) 2.88 - 2.95 (m, 2 H) 7.19 - 7.24 (m, 1 H) 7.27 - 7.31 (m, 1 H) 7.69 (d, *J*=8.28 Hz, 1 H). LC-MS 195.1/197.1 [M+H]⁺, RT 1.33 min.

### Step 2: N-(2-chloro-6,7,8,9-tetrahydro-5H-benzo[7]annulen-5-ylidene)-1-(2,4-dimethoxyphenyl)methanamine

To a stirred solution of the ketone (2.82 g, 14.5 mmol) in CH₂Cl₂ (15 mL) was added 2,4-dimethoxybenzylamine (2.28 mL, 15.2 mmol, 1.05 eq) and Et₃N (5.30 mL, 38.0 mmol, 2.6 eq) sequentially at 0 °C. Then a solution of TiCl₄ (1.0M in CH₂Cl₂, 9.4 mL, 9.4 mmol, 0.65 eq) was added to the mixture via syringe pump over 30 min at 0°C. The reaction mixture was allowed to warm to room temperature and stirred overnight. The reaction was quenched using a saturated aq. NaHCO₃ solution, then extracted using CH₂Cl₂ (5X30 mL). The combined organic layers were dried over Na₂SO₄ and then concentrated to give the crude product (4.75 g, ca. 13.8 mmol) which was used in the next step without further purification. LC-MS 344.2 [M+H]⁺, RT 1.03 min.

### Step 3: methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate

To a suspension of the crude product obtained above (4.75 g, ca. 13.8 mmol) in Ph₂O (20 mL) was added trimethyl methanetricarboxylate (4.7 g, 24.7 mmol, 1.8 eq). A short-path distillation apparatus was attached to the flask containing the reaction mixture. The reaction mixture was heated to 230 °C for 10 min. The heat was removed after methanol distillation ceased. The mixture was allowed to cool to room temperature, then purified by flash column chromatography (0-25% EtOAc in CH₂Cl₂) to afford the title compound (3.57 g, 7.60 mmol, 52%, two steps).
¹H NMR (500 MHz, CHCl₃-*d*) δ ppm 1.46 (td, *J*=13.69*,* 6.90 Hz, 1 H) 1.83 - 1.96 (m, 1 H) 2.01 (dq, *J*=13.36, 6.71 Hz, 1 H) 2.15 (td, *J*=12.69, 7.88 Hz, 1 H) 2.40 (dd, *J*=13.20, 6.27 Hz, 1 H) 2.99 (dd, *J*=14.19, 5.20 Hz, 1 H) 3.54 (s, 3 H) 3.75 (s, 3 H) 4.01 (s, 3 H) 5.03 (d, *J*=15.68 Hz, 1 H) 5.29 (d, *J*=15.68 Hz, 1 H) 6.26 (d, *J*=2.29 Hz, 1 H) 6.30 - 6.37 (m, 1 H) 6.78 (d, *J*=8.43 Hz, 1 H) 7.06 - 7.17 (m, 2 H) 7.17 - 7.25 (m, 1 H) 13.74 (s, 1 H). LC-MS 468.3/470.3 [M-H]⁻, 470.3 [M+H]⁺, RT 1.57 min.

### Step 4: 9-(3-(dimethylamino)pyrrolidin-1-yl)-4-hydroxy-2-oxo-2,5,6,7-tetrahydro-1H-benzo[6,7]cyclohepta[1,2-b]pyridine-3-carboxylic acid hydrochloride

An oven-dried vial was evacuated then back filled with Argon. The product previously obtained (282 mg, 0.60 mmol), 2-(2'-di-*tert*-butylphosphine)biphenylpalladium(II) acetate (14 mg, 0.030 mmol, 0.05 eq), and *t*-BuONa (288 mg, 3.0 mmol, 5.0 eq) were weighed out then transferred to the vial. The vial was again evacuated then back filled with Argon. To the solid mixture was added toluene (3.0 mL) and 3-dimethylaminopyrrolidine (0.15 mL, 1.2 mmol, 2.0 eq) at room temperature. The mixture was heated to 90 °C for 2 h. The reaction was quenched by the addition of 1N HCl then neutralized by saturated aq. NaHCO₃ to pH = 7. The mixture was extracted with CH₂Cl₂ (5X20 mL). The combined organic layers were concentrated, then the residue was purified by Prep-HPLC (40-90% MeCN/H₂O) to give the desired adduct.

To a suspension of the intermediate obtained above in CH₂Cl₂ (1.5 mL) was added TFA (1.5 mL) at room temperature. The mixture was stirred at room temperature for 3 h. The reaction was monitored by LC-MS. After completion, the solvent was removed under reduced pressure. The residue was dissolved in CH₂Cl₂ (1.5 mL), then HCl (2.0 mL, 2.0M in ether) was added. The mixture was filtered to give the desired product HCl salt (78.0 mg, 0.19 mmol, 31%, 2 steps) as a light brown solid.
¹H NMR (500 MHz, MeOH-*d*₄) δ ppm 2.13 - 2.23 (m, 2 H) 2.27 - 2.35 (m, 1 H) 2.41 (br. s., 2 H) 2.58 - 2.66 (m, 3 H) 2.96 - 3.04 (m, 6 H) 3.42 - 3.50 (m, 1 H) 3.63 (dd, *J*=10.52, 6.27 Hz, 1 H) 3.71 (td, *J*=9.22, 3.78 Hz, 1 H) 3.80 - 3.89 (m, 1 H) 4.05 - 4.14 (m, 1 H) 6.69 (s, 1 H) 6.71 (d, *J*=8.28 Hz, 1 H) 7.41 (d, *J*=8.51 Hz, 1 H). LC-MS 382.1 [M-H]⁻, 384.1 [M+H]⁺, RT 0.96 min.

### Example 7

### 4-hydroxy-2-oxo-9-(pyrrolidin-1-yl)-2,5,6,7-tetrahydro-1H-benzo[6,7]cyclohepta[1,2-b]pyridine-3-carboxylic acid (Cpd 7)

The two step general procedure for Buchwald coupling and deprotection from Example 6, step 4 was followed.

Pyrrolidine (0.10 mL, 1.2 mmol) was employed in the coupling reaction to give the desired adduct (205 mg, 0.42 mmol, 70%) after flash column chromatography purification (0-50% EtOAc in CH₂Cl₂). LC-MS 489.2 [M-H]⁻, 491.1 [M+H]⁺, RT 1.69 min.

The intermediate obtained above (205 mg, 0.42 mmol) was employed in the reaction to give the desired product (67 mg, 0.20 mmol) in 47% after simple filtration.
¹H NMR (500 MHz, DMSO-*d*₆) δ ppm 1.97 (t, *J*=6.46 Hz, 4 H) 2.02 - 2.17 (m, 2 H) 2.28 (br. s., 2 H) 2.49 - 2.55 (m, 2 H) 3.31 (br. s., 4 H) 3.27 - 3.35 (m, 4 H) 6.45 - 6.63 (m, 2 H) 7.34 (d, *J*=9.14 Hz, 1 H) 12.64 (br. s., 1 H) 13.77 (s, 1 H) 16.35 (br. s., 1 H). LC-MS 339.1 [M-H]⁻, 341.1 [M+H]⁺, RT 1.43 min.

### Example 8

### 4-hydroxy-9-(3-(methylamino)pyrrolidin-1-yl)-2-oxo-2,5,6,7-tetrahydro-1H-benzo[6,7]cyclohepta[1,2-b]pyridine-3-carboxylic acid hydrochloride (Cpd 8)

The two step general procedure for Buchwald coupling and deprotection from Example 6, step 4 was employed.

Tert-butyl methyl(pyrrolidin-3-yl)carbamate (240 mg, 1.2 mmol) was employed in the coupling reaction to give the desired adduct (250 mg, 0.40 mmol, 67%) after flash column chromatography purification (0-10% MeOH in CH₂Cl₂).

The intermediate obtained above (250 mg, 0.40 mmol) was deprotected to afford the product (150 mg, 0.37 mmol, 92%) after filtration as an HCl salt.
¹H NMR (500 MHz, MeOH-*d*₄) δ ppm 2.18 (quin, *J*=7.05 Hz, 2 H) 2.25 - 2.34 (m, 1 H) 2.40 (br. s., 2 H) 2.50 - 2.59 (m, 1 H) 2.62 (t, *J*=7.05 Hz, 2 H) 2.82 (s, 3 H) 3.43 - 3.53 (m, 1 H) 3.59 - 3.71 (m, 2 H) 3.74 (dd, *J*=11.23*,* 6.42 Hz, 1 H) 3.97 - 4.04 (m, 1 H) 6.68 (d, *J*=2.36 Hz, 1 H) 6.71 (dd, *J*=8.55*,* 2.48 Hz, 1 H) 7.41 (d, *J*=8.51 Hz, 1 H). LC-MS 368.1 [M-H]⁻, 370.1 [M+H]⁺, RT 0.96 min.

### Example 9

### 9-((cis,cis)-6-amino-3-azabicyclo[3.1.0]hexan-3-yl)-4-hydroxy-2-oxo-2,5,6,7-tetrahydro-1H-benzo[6,7]cyclohepta[1,2-b]pyridine-3-carboxylic acid trifluoroacetate (Cpd 9)

The two step general procedure for Buchwald coupling and deprotection from Example 6, step 4 was employed.

*N*,*N*-dibenzyl-3-azabicyclo[3.1.0]hexan-6-amine (334 mg, 1.2 mmol) was employed in the coupling reaction to give the desired adduct (280 mg, 0.40 mmol, 67%) after flash column chromatography purification (0-20% EtOAc in CH₂Cl₂). LC-MS 696.5 [M-H]⁻, 698.5 [M+H]⁺, RT 2.05 min.

To a solution of the intermediate obtained above (280 mg, 0.40 mmol) in MeOH (3 mL) and CH₂Cl₂ (3 mL) was added Pd(OH)₂ (20% on activated carbon, 50 mg). The vial was evacuated then back filled with H₂ in three cycles. After completion, the reaction mixture was filtered through Celite. The filtrate was concentrated to give a crude product which was purified by Prep-HPLC (20-75% MeCN in H₂O) to afford the desired product (96 mg, 0.15 mmol, 38%) after filtration. LC-MS 516.5 [M-H]⁻, 518.4 [M+H]⁺, RT 1.02 min.

The intermediate obtained above (96 mg, 0.15 mmol) was used in the final deprotection step to afford the product analog as a trifluoroacetate salt (31 mg, 0.064 mmol, 43%) after purification using Prep-HPLC (20-75% MeCN in H₂O).
¹H NMR (500 MHz, MeOH-*d*₄) δ ppm 2.12 - 2.22 (m, 4 H) 2.39 (br. s., 2 H) 2.51 (t, *J*=2.17 Hz, 1 H) 2.59 (t, *J*=7.05 Hz, 2 H) 3.42 (d, *J*=9.46 Hz, 2 H) 3.77 (d, *J*=9.77 Hz, 2 H) 6.61 (d, *J*=2.44 Hz, 1 H) 6.64 (dd, *J*=8.55*,* 2.48 Hz, 1 H) 7.36 (d, *J*=8.59 Hz, 1 H). LC-MS 366.3 [M-H]⁻, 368.3 [M+H]⁺, RT 0.86 min.

### Example 10

### 9-((cis,cis)-6-(benzyl(methyl)amino)-3-azabicyclo[3.1.0]hexan-3-yl)-4-hydroxy-2-oxo-2,5,6,7-tetrahydro-1H-benzo[6,7]cyclohepta[1,2-b]pyridine-3-carboxylic acid hydrochloride (Cpd 10)

The two step general procedure for Buchwald coupling and deprotection from Example 6, step 4 was employed.

*N-*Benzyl-*N*-methyl-3-azabicyclo[3.1.0]hexan-6-amine (243 mg, 1.2 mmol) was employed in the coupling reaction to give 9-((cis,cis)-6-(benzyl(methyl)amino)-3-azabicyclo[3.1.0]hexan-3-yl)-1-(2,4-dimethoxybenzyl)-4-hydroxy-2-oxo-2,5,6,7-tetrahydro-1H-benzo[6,7]cyclohepta[1,2-b]pyridine-3-carboxylic acid (140 mg, 0.23 mmol, 38%) after purification by flash column chromatography (0-50% EtOAc in CH₂Cl₂). LC-MS 620.6 [M-H]⁻, 622.5 [M+H]⁺, RT 1.48 min.

9-((cis,cis)-6-(benzyl(methyl)amino)-3-azabicyclo[3.1.0]hexan-3-yl)-1-(2,4-dimethoxybenzyl)-4-hydroxy-2-oxo-2,5,6,7-tetrahydro-1H-benzo[6,7]cyclohepta[1,2-b]pyridine-3-carboxylic acid (140 mg, 0.23 mmol) was employed in the TFA deprotection step to give the desired product as the hydrochloride salt (101 mg, 0.20 mmol, 86%) after salt exchange.
¹H NMR (500 MHz, DMSO-*d*₆) δ ppm 1.91 (br. s., 1 H) 1.99 - 2.14 (m, 2 H) 2.25 (br. s., 2 H) 2.31 - 2.40 (m, 1 H) 2.48 - 2.54 (m, 2 H) 2.65 - 2.72 (m, 1 H) 2.87 (br. s., 3 H) 3.15 - 3.21 (m, 1 H) 3.27 - 3.34 (m, 1 H) 3.36 - 3.45 (m, 1 H) 3.65 (d, *J*=9.77 Hz, 1 H) 4.35 - 4.42 (m, 1 H) 4.44 - 4.52 (m, 1 H) 6.44 - 6.58 (m, 2 H) 7.34 (d, *J*=9.14 Hz, 1 H) 7.50 (br. s., 3 H) 7.58 (br. s., 2 H) 10.41 (br. s., 1 H) 12.71 (s, 1 H) 13.77 (s, 1 H) 16.33 (br. s., 1 H). LC-MS 470.5 [M-H]⁻, 472.4 [M+H]⁺, RT 1.00 min.

### Example 11

### 4-hydroxy-9-((cis,cis)-6-(methylamino)-3-azabicyclo[3.1.0]hexan-3-yl)-2-oxo-2,5,6,7-tetrahydro-1H-benzo[6,7]cyclohepta[1,2-b]pyridine-3-carboxylic acid hydrochloride (Cpd 11)

The general procedure for hydrogenation (Example 9) was followed, using 9-((cis,cis)-6-(benzyl(methyl)amino)-3-azabicyclo[3.1.0]hexan-3-yl)-4-hydroxy-2-oxo-2,5,6,7-tetrahydro-1H-benzo[6,7]cyclohepta[1,2-b]pyridine-3-carboxylic acid (101 mg, 0.20 mmol) in the reaction to provide the desired analog as the hydrochloride salt (45 mg, 0.11 mmol, 55%) after salt exchange.
¹H NMR (500 MHz, MeOH-*d*₄) δ ppm 2.17 (t, *J*=6*.*94 Hz, 2 H) 2.27 (br. s., 2 H) 2.39 (br. s., 2 H) 2.59 (t, *J*=7.09 Hz, 2 H) 2.67 (br. s., 1 H) 2.84 (s, 3 H) 3.40 - 3.45 (m, 2 H) 3.80 (d, *J*=9.77 Hz, 2 H) 6.62 (s, 1 H) 6.65 (d, *J*=8.51 Hz, 1 H) 7.36 (d, *J*=8*.*51 Hz, 1 H). LC-MS 380.3 [M-H]⁻, 382.3 [M+H]⁺, RT 0.83 min.

### Example 12

### 9-(3-(dimethylamino)pyrrolidin-1-yl)-4-hydroxy-5-methyl-2-oxo-2,5,6,7-tetrahydro-1H-benzo[6,7]cyclohepta[1,2-b]pyridine-3-carboxylic acid hydrochloride (Cpd 12)

### Step 1: 4-methyldihydrofuran-2(3H)-one

To a solution of 4-methylfuran-2(5H)-one (5.0 g, 50.1 mmol) in EtOAc (100 mL) was added Pd/C (wt.10% Degussa type, 3.0 g, 2.8 mmol, 0.056 eq) at room temperature. The reaction flask was evacuated then back filled with H₂ in three cycles. After 12 h, the reaction mixture was filtered through Celite to remove solids, then the filtrate was concentrated to give a crude product (5.0 g, 49.9 mmol) in quantitative yield which was used in the next step without further purification.
¹H NMR (500 MHz, CHCl₃-*d*) δ ppm 1.14 - 1.19 (m, 3 H) 2.10 - 2.20 (m, 1 H) 2.60 - 2.72 (m, 2 H) 3.83 - 3.91 (m, 1 H) 4.37 - 4.46 (m, 1 H).

### Step 2: 4-bromo-3-methylbutanoic acid

To a solution of the above 4-methyldihydrofuran-2(3H)-one (5.0 g, 49.9 mmol) in AcOH (50 mL) was added HBr (33% in HOAc, 15.0 mL, 86.9 mmol, 1.7 eq) at room temperature. The mixture was heated to 90 °C and stirred at 90 °C for 4 h. The reaction mixture was poured onto ice then extracted with CH₂Cl₂ (4X40 mL). The combined organic layers were dried over Na₂SO₄, then concentrated to give a crude product (8.6 g, ca. 47.5 mmol, 95%) which was used in the next step without further purification. ¹H NMR (500 MHz, CHCl₃-*d*) δ ppm 1.08 - 1.16 (m, 3 H) 2.27 - 2.41 (m, 2 H) 2.56 - 2.70 (m, 1 H) 3.39 - 3.46 (m, 1 H) 3.46 - 3.54 (m, 1 H).

### Step 3: (3-carboxy-2-methylpropyl)triphenylphosphonium bromide

To a solution of the above acid (8.6 g, 47.5 mmol) in toluene (50 mL) was added PPh₃ (50 g, 191 mmol, 4.0 eq) at room temperature. The mixture was heated to reflux, then stirred for 48 h. The mixture was cooled, then filtered to give a crude product (14.1 g, 31.8 mmol, 67%). The resulting Wittig salt was used in the next step without further purification. ¹H NMR (500 MHz, DMSO-*d*₆) δ ppm 0.84 (d, *J*=6.07 Hz, 3 H) 2.15 - 2.32 (m, 3 H) 3.52-3.73 (m, 2 H) 7.73 - 7.82 (m, 6 H) 7.82 - 7.96 (m, 9 H).

### Step 4: 5-(3-chlorophenyl)-3-methylpentanoic acid

To a suspension of (3-carboxy-2-methylpropyl)triphenylphosphonium bromide (14.1 g, 31.8 mmol) in DMSO (40 mL) was added *t*-BuOK (7.9 g, 70.4 mmol, 2.2 eq) at 0 °C. The resulting red mixture was stirred for 30 min, then a solution of 3-chlorobenzaldehyde (3.6 mL, 31.6 mmol, 1.0 eq) was added at 0 °C. After reaction overnight, the mixture was poured onto ice (100 g) then extracted with CH₂Cl₂ (3X25 mL). The aqueous phase was acidified with 6N HCl to pH=1, then extracted with CH₂Cl₂ (5X30 mL). The organic layers were concentrated, then the resulting crude product was purified by flash column chromatography (0-25% EtOAc in CH₂Cl₂) to give the desired product (3.25 g, 14.5 mmol, 45%) as an *E*/*Z* isomer mixture. LC-MS 223.6/225.5 [M-H]⁻, RT 1.23 min

To a solution of the above Wittig product (3.25 g, 14.5 mmol) in EtOAc (30 mL) was added Pd/C (10% Degussa type, 1.2 g, 1.13 mmol, 0.08 eq) at room temperature. The mixture was evacuated, then back filled with H₂ for three cycles. After 1 h, the mixture was filtered through Celite and washed with EtOAc (3X40 mL). The filtrate was concentrated to give a crude product (3.1 g, 13.7 mmol, 94%) which was used in the next step without further purification.
¹H NMR (500 MHz, CHCl₃-*d*) δ ppm 1.06 (d, *J*=6.62 Hz, 3 H) 1.48 - 1.60 (m, 1 H) 1.66-1.75 (m, 1 H) 1.97 - 2.07 (m, 1 H) 2.20 - 2.29 (m, 1 H) 2.37 - 2.44 (m, 1 H) 2.55 - 2.62 (m, 1 H) 2.63 - 2.71 (m, 1 H) 7.07 (dt, *J*=7.25, 1.50 Hz, 1 H) 7.13 - 7.25 (m, 3 H). LC-MS 225.5/227.5 [M-H]⁻, RT 1.28 min.

### Step 5: 2-chloro-7-methyl-6,7,8,9-tetrahydro-5H-benzo[7]annulen-5-one

5-(3-chlorophenyl)-3-methylpentanoic acid (3.1 g, 13.7 mmol) and PPA (30 g) were mixed in a flask then heated to 130°C and stirred for 1 h. The mixture was carefully poured onto ice (100 g) then extracted with CH₂Cl₂ (3X40 mL). The combined organic layers were concentrated and the resulting crude product was purified by flash column chromatography (0-25% EtOAc in hexanes) to give the desired ketone (1.89 g, 9.1 mmol, 66%).
¹H NMR (500 MHz, CHCl₃-*d*) δ ppm 1.07 (d, *J*=6.62 Hz, 3 H) 1.48 - 1.55 (m, 1 H) 1.96-2.15 (m, 2 H) 2.59 (dd, *J*=14.82, 9.30 Hz, 1 H) 2.78 (dd, *J*=14.74, 4.02 Hz, 1 H) 2.86 (ddd, *J*=15.00*,* 6.31, 3.47 Hz, 1 H) 2.95 - 3.04 (m, 1 H) 7.20 - 7.25 (m, 1 H) 7.25 - 7.31 (m, 1 H) 7.68 (d, *J*=8*.*28 Hz, 1 H). LC-MS 209.5/211.5 [M+H]⁺, RT 1.41 min

### Step 6: N-(2-chloro-7-methyl-6,7,8,9-tetrahydro-5H-benzo[7]annulen-5-ylidene)-1-(2,4-dimethoxyphenyl)methanamine

To a stirred solution of 2-chloro-7-methyl-6,7,8,9-tetrahydro-5H-benzo[7]annulen-5-one (1.89 g, 9.1 mmol) in CH₂Cl₂ (15 mL) was added 2,4-dimethoxybenzylamine (1.43 mL, 9.5 mmol, 1.05 eq) and Et₃N (3.30 mL, 23.7 mmol, 2.6 eq) sequentially at 0°C. Then a solution of TiCl₄ (1.0M in CH₂Cl₂, 6.0 mL, 6.0 mmol, 0.66 eq) was added to the mixture via syringe pump over 30 min at 0°C. The reaction mixture was allowed to warm to room temperature and stirred overnight. The reaction was quenched by the addition of a saturated aq. NaHCO₃ solution then the solvate was extracted with CH₂Cl₂ (5X30 mL). The combined organic layers were dried over Na₂SO₄ then concentrated to give the a crude product (2.68 g, ca. 7.5 mmol) which was used in the next step without further purification. LC-MS 358.7 [M+H]⁺, RT 1.03 min.

### Step 7: methyl 9-chloro-1-(2,4-dimethoxybenzyl)-4-hydroxy-5-methyl-2-oxo-2,5,6,7-tetrahydro-1H-benzo[6,7]cyclohepta[1,2-b]pyridine-3-carboxylate

To a suspension of the crude product obtained above (2.68 g, ca. 7.5 mmol) in Ph₂O (15 mL) was added trimethyl methanetricarboxylate (3.23 g, 17.0 mmol, 2.3 eq). A short-path distillation apparatus was attached to the flask containing the reaction mixture. The reaction was heated to 230 °C for 10 min. The heat was removed after methanol distillation ceased. The mixture was allowed to cool to room temperature, then purified by flash column chromatography (0-25% EtOAc in CH₂Cl₂) to give the product (2.40 g, 4.96 mmol, 55%, two steps).
¹H NMR (500 MHz, CHCl₃-*d*) δ ppm 0.38 (d, *J*=7.09 Hz, 3 H) 1.62 - 1.75 (m, 1 H) 1.75-2.15 (m, 1 H) 2.18 - 2.27 (m, 1 H) 2.27 - 2.39 (m, 2 H) 3.52 (s, 3 H) 3.75 (s, 3 H) 4.02 (m, 3 H) 4.96 - 5.02 (m, 1 H) 5.39 (d, *J*=15.45 Hz, 1 H) 6.20 - 6.28 (m, 1 H) 6.28 - 6.38 (m, 1 H) 6.77 (d, *J*=8.35 Hz, 1 H) 7.06 - 7.13 (m, 1 H) 7.13 - 7.18 (m, 1 H) 7.18 - 7.25 (m, 1 H) 13.96 (br. s., 1 H). LC-MS 482.9/484.9 [M-H]⁻, 484.8 [M+H]⁺, RT 1.60 min.

### Step 8: 9-(3-(dimethylamino)pyrrolidin-1-yl)-4-hydroxy-5-methyl-2-oxo-2,5,6,7-tetrahydro-1H-benzo[6,7]cyclohepta[1,2-b]pyridine-3-carboxylic acid hydrochloride

An oven-dried vial was evacuated, then back filled with Argon. To the vial was added methyl 9-chloro-1-(2,4-dimethoxybenzyl)-4-hydroxy-5-methyl-2-oxo-2,5,6,7-tetrahydro-1H-benzo[6,7]cyclohepta[1,2-b]pyridine-3-carboxylate (242 mg, 0.50 mmol), *2-*(2'*-*di*-tert-*butylphosphine)biphenylpalladium(II) acetate (10 mg, 0.022 mmol, 0.04 eq), and *t*-BuONa (240 mg, 2.5 mmol, 5.0 eq). The vial was evacuated again, then back filled with Argon. To the solid mixture was added toluene (3.0 mL) and 3-dimethylaminopyrrolidine (0.13 mL, 1.0 mmol, 2.0 eq) at room temperature. The mixture was heated to 90 °C for 2 h. The reaction was quenched with 1N HC1, then neutralized with saturated aq. NaHCO₃ to pH = 7. The mixture was extracted with CH₂Cl₂ (5X20 mL). The combined organic layers were concentrated, then the residue was purified by flash column chromatography (0-5% MeOH in CH₂Cl₂) to give the desired adduct. LC-MS 547.1 [M-H]⁻, 549.1 [M+H]⁺, RT 1.09 min.

To a suspension of the intermediate obtained above in CH₂Cl₂ (1.5 mL) was added TFA (1.5 mL) at room temperature. The mixture was stirred at room temperature for 3 h. The reaction was monitored by LC-MS. After completion, the solvent was removed under reduced pressure. The residue was dissolved in CH₂Cl₂ (1.5 mL), then HCl (2.0 mL, 2.0M in ether) was added. The mixture was filtered to give the desired product (99.0 mg, 0.23 mmol, 46%, two steps) as a light yellow solid HCl salt.
¹H NMR (500 MHz, MeOH-*d*₄) δ ppm 0.75 (br. s., 3 H) 1.85 - 2.03 (m, 2 H) 2.31 (dq, *J*=12.98, 8.34 Hz, 1 H) 2.48 (br. s., 1 H) 2.57 - 2.71 (m, 3 H) 3.01 (d, *J*=4.97 Hz, 6 H) 3.43-3.52 (m, 1 H) 3.58 - 3.67 (m, 1 H) 3.67 - 3.76 (m, 1 H) 3.85 (dd, *J*=10.60, 7.61 Hz, 1 H) 4.09 (quin, *J*=7.21 Hz, 1 H) 6.67 (s, 1 H) 6.71 (dd, *J*=8.47, 2.33 Hz, 1 H) 7.38 (d, *J*=8.43 Hz, 1 H). LC-MS 396.2 [M-H]⁻, 398.2 [M+H]⁺, RT 0.84 min.

### Example 13

### 9-((cis,cis)-6-amino-3-azabicyclo[3.1.0]hexan-3-yl)-4-hydroxy-5-methyl-2-oxo-2,5,6,7-tetrahydro-1H-benzo[6,7]cyclohepta[1,2-b]pyridine-3-carboxylic acid hydrochloride (Cpd 13)

The two step general procedure for Buchwald coupling and deprotection from Example 12, step 8 was employed.

*N*,*N*-dibenzyl-3-azabicyclo[3.1.0]hexan-6-amine (278 mg, 1.0 mmol) was employed in the coupling reaction to give the desired adduct (200 mg, 0.28 mmol, 56%) after flash column chromatography purification (0-20% EtOAc in CH₂Cl₂).

To a solution of the intermediate obtained above (200 mg, 0.28 mmol) in MeOH (3 mL) and CH₂Cl₂ (3 mL) was added Pd(OH)₂ (20 wt% on carbon, 50 mg). The vial was evacuated, then back filled with H₂ in three cycles. After completion, the reaction mixture was filtered through Celite. The filtrate was concentrated to give a crude product which was used in the next step without further purification.

To a suspension of the above intermediate in CH₂Cl₂ (1.5 mL) was added TFA (1.5 mL) at room temperature. The mixture was stirred at room temperature for 3 h. The reaction was monitored by LC-MS. After completion, the solvent was removed under reduced pressure. The residue was dissolved in CH₂Cl₂ (1.5 mL), then HCl (2.0 mL, 2.0M in ether) was added. The mixture was filtered to give the desired product (85.0 mg, 0.20 mmol, 73%, two steps) as a light yellow solid HCl salt.
¹H NMR (500 MHz, MeOH-*d₄*) δ ppm 0.74 (br. s., 3 H) 1.90 - 1.98 (m, 1 H) 2.10 - 2.21 (m, 2 H) 2.47 (br. s., 1 H) 2.50 (t, *J*=2.21 Hz, 1 H) 2.54 - 2.70 (m, 3 H) 3.39 - 3.43 (m, 2 H) 3.77 (dd, *J*=9.77*,* 2.21 Hz, 2 H) 6.59 (d, *J*=2.52 Hz, 1 H) 6.63 (dd, *J*=8.51, 2.52 Hz, 1 H) 7.32 (d, *J*=8.51 Hz, 1 H). LC-MS 380.2 [M-H]⁻, 382.2 [M+H]⁺, RT 0.83 min.

### Example 14

### 9-((cis,cis)-6-amino-3-azabicyclo[3.1.0]hexan-3-yl)-4,7-dihydroxy-2-oxo-2,5,6,7-tetrahydro-1H-benzo[6,7]cyclohepta[1,2-b]pyridine-3-carboxylic acid trifluoroacetate salt (Cpd 14)

### Step 1: 1-(2-bromo-5-chlorophenyl)but-3-en-1-ol

To a solution of 2-bromo-5-chlorobenzaldehyde (4.39 g, 20.0 mmol) in THF (30 mL) was added allylmagnesium bromide (1.0M/ether, 24.0 mL, 24.0 mmol, 1.2 eq) at 0°C. The reaction was allowed to warm to room temperature and stirred for 1 h. After complete consumption of starting material, the reaction was quenched by saturated aq. NH₄Cl then extracted with ether (3X30 mL). The combined organic layers were dried over Na₂SO₄ then concentrated to give a crude product (5.30 g, ca. 20 mmol) which was used in the next step without further purification.
¹H NMR (500 MHz, CHCl₃-*d*) δ ppm 2.26 - 2.36 (m, 1 H) 2.65 (dddt, *J*=12.85, 6.38, 3.63, 1.18, 1.18 Hz, 1 H) 5.05 (dd, *J*=8.51*,* 3.47 Hz, 1 H) 5.19 - 5.27 (m, 2 H) 5.82 - 5.95 (m, 1 H) 7.13 (dd, *J*=8.51, 2.52 Hz, 1 H) 7.45 (d, *J*=8.51 Hz, 1 H) 7.58 (d, *J*=2.52 Hz, 1 H).

### Step 2: 2-(1-(benzyloxy)but-3-enyl)-1-bromo-4-chlorobenzene

To a stirred solution of 1-(2-bromo-5-chlorophenyl)but-3-en-1-ol (2.65 g, ca. 10.0 mmol) in mixed solvent of THF (30 mL) and DMF (7.5 mL) was added NaH (60% in mineral oil, 624 mg, 15.6 mmol, 1.6 eq) at 0°C. The mixture was allowed to warm to room temperature and stirred for 0.5 h, then cooled to 0°C and BnBr (1.43 mL, 12.0 mmol, 1.2 eq) was added. The reaction mixture was allowed to warm to room temperature and stirred overnight. The reaction was quenched by saturated aq. NaHCO₃ solution then extracted with ether (3X30 mL). The combined organic layers were dried over Na₂SO₄ then concentrated to give a crude product which was purified by flash column chromatography (50% CH₂Cl₂ in hexanes) to afford the pure product (3.41 g, 9.7 mmol).
¹H NMR (500 MHz, CHCl₃-*d*) δ ppm 2.42 - 2.52 (m, 2 H) 4.32 (d, *J*=10.25 Hz, 1 H) 4.49 (d, *J*=10.25 Hz, 1 H) 4.80 (dd, *J*=7.25*,* 5.04 Hz, 1 H) 5.04 - 5.12 (m, 2 H) 5.87 (ddt, *J*=17*.*10*,* 10.32, 6.94, 6.94 Hz, 1 H) 7.14 (dd, *J*=8.51, 2.52 Hz, 1 H) 7.29 - 7.42 (m, 5 H) 7.45 - 7.49 (m, 1 H) 7.54 (d, *J*=2.84 Hz, 1 H).

### Step 3: 1-(2-(1-(benzyloxy)but-3-enyl)-4-chlorophenyl)prop-2-en-1-ol

To a solution of the above 2-(1-(benzyloxy)but-3-enyl)-1-bromo-4-chlorobenzene (3.41 g, 9.7 mmol) in THF (25 mL) was added *n*-BuLi (2.5M/hexanes, 4.7 mL, 11.8 mmol, 1.2 eq) dropwise at -78 °C. The mixture was stirred for 20 min at -78 °C, then acrolein (1.0 mL, 15.0 mmol, 1.5 eq) was added. After 1 h at -78 °C, the reaction was quenched by saturated aq. NH₄Cl then extracted with ether (3X30 mL). The combined organic layers were dried over Na₂SO₄ then concentrated to give a crude product which was purified by flash column chromatography (0-15% EtOAc in hexanes) to afford the desired product (2.2 g, 6.7 mmol, 67%, 2 steps).
¹H NMR (500 MHz, CHCl₃-*d*) δ ppm 2.33 - 2.52 (m, 1 H) 2.53 - 2.68 (m, 1 H) 4.28 (t, *J*=11.51 Hz, 1 H) 4.48 (dd, *J*=11.82, 8.35 Hz, 1 H) 4.70 - 4.82 (m, 1 H) 5.03 - 5.12 (m, 2 H) 5.18 - 5.25 (m, 1 H) 5.30 (dq, *J*=17.26, 1.18 Hz, 1 H) 5.37 - 5.44 (m, 1 H) 5.79 - 5.91 (m, 1 H) 5.95 - 6.05 (m, 1 H) 7.27 - 7.39 (m, 6 H) 7.40 - 7.44 (m, 1 H) 7.51 - 7.55 (m, 1 H).

### Step 4: 1-(2-(1-(benzyloxy)but-3-enyl)-4-chlorophenyl)prop-2-en-1-one

To a solution of the above 1-(2-(1-(benzyloxy)but-3-enyl)-4-chlorophenyl)prop-2-en-1-ol (4.8 g, 14.6 mmol) in CH₂Cl₂ (100 mL) was added MnO₂ in four portions (4x6.3 g, 289.6 mmol, 20.0 eq) at room temperature. The mixture was stirred for 2 h, then filtered through Celite to remove the solids. The filtrate was concentrated, then purified by flash column chromatography (50% CH₂Cl₂ in hexanes) to afford the desired ketone (2.0 g, 6.1 mmol, 42%)
¹H NMR (500 MHz, CHCl₃-*d*) δ ppm 2.46 - 2.54 (m, 2 H) 4.31 (d, *J*=10.25 Hz, 1 H) 4.43 (d, *J*=10.25 Hz, 1 H) 4.83 (dd, *J*=6.94*,* 5.67 Hz, 1 H) 5.02 - 5.08 (m, 2 H) 5.86 (ddt, *J*=17.18, 10.25, 6.94, 6.94 Hz, 1 H) 6.02 - 6.05 (m, 1 H) 6.12 - 6.17 (m, 1 H) 6.70 - 6.81 (m, 1 H) 7.27 - 7.36 (m, 6 H) 7.43 (d, *J*=8.20 Hz, 1 H) 7.73 (d, *J*=2.21 Hz, 1 H).

### Step 5:9-(benzyloxy)-2-chloro-8,9-dihydro-5H-benzo[7]annulen-5-one

To a solution of the above 1-(2-(1-(benzyloxy)but-3-enyl)-4-chlorophenyl)prop-2-en-1-one (1.9 g, 5.8 mmol) in toluene (100 mL) was added Grubbs' II catalyst (148 mg, 0.17 mmol, 3mol%) at room temperature. The mixture was heated to 60 °C and stirred for 1.5 h. After complete consumption of the starting material, the solvent was removed under reduced pressure. The residue was purified by flash column chromatography (0-10% EtOAc in hexanes) to afford the product (1.6 g, 5.4 mmol, 92%).
¹H NMR (500 MHz, CHCl₃-*d*) δ ppm 2.78 - 2.86 (m, 1 H) 2.90 - 2.98 (m, 1 H) 4.35 (d, *J*=10.25 Hz, 1 H) 4.49 (d, *J*=10.25 Hz, 1 H) 4.64 (dd, *J*=7.41, 2.36 Hz, 1 H) 6.30 (dt, *J*=10.25, 1.89 Hz, 1 H) 6.52 (dt, *J*=10.25, 4.89 Hz, 1 H) 7.24 - 7.44 (m, 7 H) 7.71 (d, *J=*8*.*20 Hz, 1 H).

### Step 6: 2-chloro-9-hydroxy-6,7,8,9-tetrahydro-5H-benzo[7]annulen-5-one

To a solution of 9-(benzyloxy)-2-chloro-8,9-dihydro-SH-benzo[7]annulen-5-one (1.85 g, 6.2 mmol) in EtOAc (20 mL) was added Pd/C (10wt% Degussa type, 500 mg). The flask was evacuated then back filled with H₂ in three cycles. After completion, the reaction mixture was filtered through Celite. The filtrate was concentrated to give a crude product which was purified by flash column chromatography (0-20% EtOAc in hexanes) to afford the desired product (1.18 g, 5.6 mmol, 90%).
¹H NMR (500 MHz, CHCl₃-*d*) δ ppm 1.72 - 1.90 (m, 2 H) 1.96 (dq, *J*=13.56*,* 6.73 Hz, 1 H) 2.15 - 2.27 (m, 1 H) 2.55 - 2.65 (m, 1 H) 2.85 - 2.95 (m, 1 H) 5.04 (br. s., 1 H) 7.35 (dd, *J*=8.04*,* 2.05 Hz, 1 H) 7.44 - 7.54 (m, 1 H) 7.58 (d, *J*=6.62 Hz, 1 H). LC-MS 211.1 [M+H]⁺, RT 1.00 min.

### Step 7: 9-(tert-butyldimethylsilyloxy)-2-chloro-6,7,8,9-tetrahydro-5H-benzo[7]annulen-5-one

To a solution of 2-chloro-9-hydroxy-6,7,8,9-tetrahydro-5H-benzo[7]annulen-5-one (1.18 g, 5.6 mmol) in CH₂Cl₂ (20 mL) was added imidazole (760 mg, 11.2 mmol, 2.0 eq) followed by TBS-Cl (1.7 g, 11.3 mmol, 2.0 eq) at 0 °C. The mixture was allowed to warm to room temperature and stirred overnight. The reaction was quenched by saturated aq. NaHCO₃ solution then extracted with CH₂Cl₂ (4X30 mL). The solvent was removed and the resulting crude product was purified by flash column chromatography (50% CH₂Cl₂ in hexanes) to afford the desired ketone (1.64 g, 5.0 mmol, 90%).
¹H NMR (500 MHz, CHCl₃-*d*) δ ppm -0.13 (s, 3 H) 0.05 (s, 3 H) 0.87 (s, 9 H) 1.71 - 1.84 (m, 2 H) 1.93 (td, *J*=13.64, 6.15 Hz, 1 H) 2.04 - 2.14 (m, 1 H) 2.57 (ddd, *J*=18.29, 8.04, 3.63 Hz, 1 H) 2.95 (ddd, *J*=18.36, 8.59, 3.63 Hz, 1 H) 4.92 (t, *J*=5.52 Hz, 1 H) 7.33 (dd, *J*=8.04, 2.05 Hz, 1 H) 7.37 (d, *J*=1.89 Hz, 1 H) 7.55 (d, *J*=8.20 Hz, 1 H).

### Step 8:N-(9-(tert-butyldimethylsilyloxy)-2-chloro-6,7,8,9-tetrahydro-5H-benzo[7]annulen-5-ylidene)-1-(2,4-dimethoxyphenyl)methanamine

To a stirred solution of 9-(tert-butyldimethylsilyloxy)-2-chloro-6,7,8,9-tetrahydro-5H-benzo[7]annulen-5-one (1.64 g, 5.0 mmol) in CH₂Cl₂ (6 mL) was added 2,4-dimethoxybenzylamine (0.76 mL, 5.1 mmol, 1.01 eq) followed by Et₃N (1.8 mL, 12.9 mmol, 2.6 eq) at 0°C. Then a solution of TiCl₄ (1.0M/CH₂Cl₂, 3.3 mL, 3.3 mmol, 0.66 eq) was added to the reaction mixture via syringe pump over 30 min at 0°C. The reaction was allowed to warm to room temperature and stirred overnight. The reaction was quenched by saturated aq. NaHCO₃ solution then extracted with CH₂Cl₂ (5X30 mL). The combined organic layers were dried over Na₂SO₄ then concentrated to give a crude product which was used in the next step without further purification. LC-MS 474.3/476.3 [M+H]⁺, RT 1.28 min.

### Step 9: methyl 7-((tert-butyldimethylsilyl)oxy)-9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate

To a suspension of the intermediate obtained above in Ph₂O (10 mL) was added trimethyl methanetricarboxylate (1.6 g, 8.4 mmol, 1.7 eq) at room temperature. A short-path distillation apparatus was attached to the flask and the reaction mixture was heated to 230 °C for 10 min. The heat was removed after methanol distillation ceased. The mixture was allowed to cool to room temperature and then purified by flash column chromatography (0-25% EtOAc in CH₂Cl₂) to give the product (1.13 g, 1.9 mmol, 37%, two steps). ¹H NMR (500 MHz, CHCl₃-*d*) δ ppm -0.13 (s, 3 H) -0.03 (s, 3 H) 0.90 (s, 9 H) 1.51 (td, *J*=13.87, 7.25 Hz, 1 H) 1.66 - 1.77 (m, 1 H) 2.36 (tdd, *J*=13.28, 13.28, 7.49, 6.31 Hz, 1 H) 2.94 (dd, *J*=14.50*,* 5.36 Hz, 1 H) 3.56 (s, 3 H) 3.73 (s, 3 H) 4.01 (s, 3 H) 4.31 - 4.40 (m, 1 H) 5.10 (d, *J*=16.08 Hz, 1 H) 5.25 (d, *J*=16.08 Hz, 1 H) 6.28 (d, *J*=2.21 Hz, 1 H) 6.34 (dd, *J*=8.51, 2.21 Hz, 1 H) 6.73 (d, *J*=8.51 Hz, 1 H) 7.11 (d, *J*=8.51 Hz, 1 H) 7.25 (dd, *J*=8.20, 2.21 Hz, 3 H) 7.64 (d, *J*=2.21 Hz, 1 H) 13.78 (s, 1 H). LC-MS 598.5/600.4 [M-H]⁻, 600.4 [M+H]⁺, RT 1.95 min.

### Step 10: 9-((cis,cis)-6-amino-3-azabicyclo[3.1.0]hexan-3-yl)-4,7-dihydroxy-2-oxo-2,5,6,7-tetrahydro-1H-benzo[6,7]cyclohepta[1,2-b]pyridine-3-carboxylic acid trifluoroacetate salt

An oven-dried vial was evacuated then back filled with Argon. To the vial was added methyl 7-((tert-butyldimethylsilyl)oxy)-9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (300 mg, 0.50 mmol), *N,N-*dibenzyl-3-azabicyclo[3.1.0]hexan-6-amine (278 mg, 1.0 mmol, 2.0 eq), 2-(2'-di-*tert*-butylphosphine)biphenylpalladium(II) acetate (10 mg, 0.022 mmol, 0.04 eq), and *t-*BuONa (240 mg, 2.5 mmol, 5.0 eq). The vial was evacuated again then back filled with Argon. To the solid mixture was added toluene (3 mL) and the resulting mixture was heated to 90 °C for 2 h. The reaction was quenched by 1N HCl then neutralized by saturated aq. NaHCO₃ to pH = 7. The mixture was extracted with CH₂Cl₂ (5X20 mL). The combined organic layers were concentrated, then the residue was purified by flash column chromatography purification (0-20% EtOAc in CH₂Cl₂).

To a solution of the intermediate obtained above in MeOH (3 mL) and CH₂Cl₂ (3 mL) was added Pd(OH)₂/C (20wt%, 50 mg). The vial was evacuated then back filled with H₂ in three cycles. After consumption of the starting material, the reaction mixture was filtered through Celite. The filtrate was concentrated to give a crude product which was used in the next step without further purification. LC-MS 646.5 [M-H]⁻, 648.4 [M+H]⁺, RT 1.23 min.

To a suspension of the above hydrogenation product in CH₂Cl₂ (1.5 mL) was added TFA (1.0 mL) at room temperature. The mixture was stirred at room temperature overnight. The reaction was monitored by LC-MS. After completion, the solvent was removed under reduced pressure. The residue was dissolved in CH₂Cl₂ (1.5 mL), then TFA (2.0 mL) was added. The mixture was filtered then washed with CH₂Cl₂ and ether to give the product trifluoroacetate salt (77 mg, 0.15 mmol, 31%, 3 steps) as a light brown solid. ¹H NMR (500 MHz, MeOH-*d*₄) δ ppm 1.87 - 2.04 (m, 2 H) 2.19 (br. s., 2 H) 2.52 (s, 1 H) 2.54 - 2.63 (m, 1 H) 2.84 - 2.91 (m, 1 H) 3.43 - 3.48 (m, 2 H) 3.78 - 3.83 (m, 2 H) 4.57 - 4.65 (m, 1 H) 6.67 (dd, *J*=8.67*,* 2.36 Hz, 1 H) 6.97 (d, *J*=1*.*89 Hz, 1 H) 7.37 (d, *J*=8.51 Hz, 1 H). LC-MS 382.1 [M-H]⁻, 384.2 [M+H]⁺, RT 0.66 min.

### Example 15

### 9-(3-(dimethylamino)pyrrolidin-1-yl)-4,7-dihydroxy-2-oxo-2,5,6,7-tetrahydro-1H-benzo[6,7]cyclohepta[1,2-b]pyridine-3-carboxylic acid hydrochloride (Cpd 15)

The two step general procedure for Buchwald coupling and deprotection from Example 14, step 10 was employed.

*N*,*N*-dimethylpyrrolidin-3-amine (0.15 mL, 1.0 mmol) was employed in the coupling reaction to give the desired adduct (270 mg, 0.41 mmol, 81%) after flash column chromatography purification (0-5% MeOH in CH₂Cl₂). LC-MS 662.2 [M-H]⁻, 664.2 [M+H]⁺, RT 1.29 min

To a suspension of the intermediate obtained above in CH₂Cl₂ (1.5 mL) was added TFA (1.0 mL) at room temperature. The mixture was stirred at room temperature for 20 h. The reaction was monitored by LC-MS. After completion, the solvent was removed under reduced pressure. The residue was dissolved in CH₂Cl₂ (1.5 mL), then HCl (2.0 mL, 2.0M in ether) was added. The mixture was filtered then washed by CH₂Cl₂ and ether to give the desired product (52 mg, 0.12 mmol, 29%) as an off-white solid HCl salt.
¹H NMR (500 MHz, MeOH-*d*₄) δ ppm 1.88 - 2.03 (m, 2 H) 2.29 - 2.39 (m, 1 H) 2.53 - 2.70 (m, 2 H) 2.84 - 2.92 (m, 1 H) 3.01 (br. s., 6 H) 3.47 - 3.54 (m, 1 H) 3.67 (br. s., 1 H) 3.75 (br. s., 1 H) 3.88 (br. s., 1 H) 4.12 (br. s., 1 H) 4.64 (t, *J*=8.20 Hz, 1 H) 6.74 (br. s., 1 H) 7.03 (br. s., 1 H) 7.42 (d, *J*=6.62 Hz, 1 H). LC-MS 398.1 [M-H]⁻, 400.1 [M+H]⁺, RT 0.68 min.

### Example 16

### 4-hydroxy-9-methyl-2-oxo-1,2,5,9-tetrahydropyrido[3',2':6,7]cyclohepta[1,2-f]indole-3-carboxylic acid (Cpd 16)

### Step 1: 4-amino-2-chloro-5-((trimethylsilyl)ethynyl)benzonitrile

To a suspension of 4-amino-2-chloro-5-iodobenzonitrile (30.30 g, 109 mmol) in CH₃CN (160 mL) was added ethynyltrimethylsilane (18.6 mL, 131 mmol) and NEt₃ (30.5 mL, 219 mmol). The mixture was degassed with Argon, then Pd(PPh₃)₂Cl₂ (0.420 g, 1.1 mmol, 1 mol%) and CuI (0.45 g, 2.4 mmol, 2 mol%) were added. The reaction mixture was heated at 70 °C for 1.5 h until the starting material was completely consumed. After cooling to room temperature, the CH₃CN was removed under reduced pressure. Water (400 mL) was added to the residue and the precipitate was filtered, washed with H₂O (2x200 mL) and dried in an N₂ flow. A crude product was obtained as tan solid and used directly in the next step without further purification.
¹H NMR (500 MHz, Acetone) δ ppm 0.25 (s, 9 H) 6.14 (br. s., 2 H) 6.97 (s, 1 H) 7.63 (s, 1 H). LC-MS 247.1/249.1 [M-H]⁻, RT 1.41 min.

### Step 2: 6-chloro-1-methyl-1H-indole-5-carbonitrile

To a mixture of crude 4-amino-2-chloro-5-((trimethylsilyl)ethynyl)benzonitrile (*ca* 109 mmol) in DMF (270 mL) degassed with Argon was added t-BuOK (29.5 g, 263 mmol). The mixture was heated at 70 °C for 1 h under Argon, then cooled to 0 °C and MeI (20.5 mL, 329 mmol) was added dropwise. After stirring at room temperature for 30 min mixture was carefully poured onto ice (∼500 mL) in the presence of HCl (conc, 20 mL). The precipitate was filtered and washed with H₂O (2x300 mL) and dried in an N₂ flow. The solid was purified by column chromatography using DCM/hexanes (gradient 20-50%), affording the product 6-chloro-1-methyl-1H-indole-5-carbonitrile (18.56 g, 89%).
¹H NMR (500 MHz, CHCl₃-*d*) δ ppm 3.81 (s, 3 H) 6.55 (d, *J*=3.5 Hz, 1 H) 7.17 (d, *J*=3.5 Hz, 1 H) 7.42 (s, 1 H) 7.94 (s, 1 H). LC-MS 191.1 [M+H]⁺, RT 1.17 min.

### Step 3: 6-chloro-1-methyl-1H-indole-5-carbaldehyde

To a solution of 6-chloro-1-methyl-1H-indole-5-carbonitrile (11.88 g, 62.32 mmol) in DCM (250 mL) at -78 °C was added DIBAL-H (1M in DCM, 75.0 mL, 75.0 mmol) dropwise over ∼15 min. The reaction mixture was stirred at -78 °C for 10 min and slowly allowed to warm to -20 °C over ~2 h. After LC/MS indicated complete consumption of the starting material, the mixture was cooled to -40 °C and the reaction was quenched by addition of the Rochelle salt (aqueous saturated, 80 mL). The resulting emulsion was allowed to warm to room temperature and was vigorously stirred for ∼1 h. The organic phase was separated and the aqueous phase was extracted with DCM (100 mL). The combined organics were stirred with 3M HCl (100 mL) for 30 min to accomplish the hydrolysis of the intermediate. The organic phase was subsequently washed with NaHCO₃ (aqueous saturated, 100 mL) and NaCl (aqueous saturated, 100 mL) and then dried over Na₂SO₄. The solvent was removed to provide 6-chloro-1-methyl-1H-indole-5-carbaldehyde as a solid (10.25 g, 85%) which was used directly in the next step without further purification.
¹H NMR (500 MHz, CHCl₃-*d*) δ ppm 3.72 (s, 3 H) 6.53 (d, *J*=3.5 Hz, 1 H) 7.04 (d, *J*=3.5 Hz, 1 H) 7.27 (s, 1 H) 8.18 (s, 1 H) 10.44 (s, 1 H). LC-MS 194.1/196.0 [M+H]⁺, RT 1.16 min.

### Step 4: 1-methyl-6-vinyl-1H-indole-5-carbaldehyde

6-chloro-1-methyl-1H-indole-5-carbaldehyde (8.10 g, 41.83 mmol), potassium vinyltrifluoroborate (8.40 g, 62.71 mmol), Pd(OAc)₂ (280 mg, 1.25 mmol, 3 mol%), S-Phos ligand (1.05 g, 2.56 mmol, 6 mol%) and K₂CO₃ (17.40 g, 125.90 mmol) were mixed together in a 500 mL round bottom flask. The flask was placed under vacuum and back filled with Argon, then dioxane (165 mL) and H₂O (28 mL) were added. The mixture was heated at 85-90 °C for 6 h and monitored by LC/MS. After complete consumption of starting material, the reaction was cooled to room temperature. Water (150 mL) was added to the mixture and a product was extracted with DCM (4x150 mL). The combined organics were washed with NaCl (aqueous saturated, 150 mL) and dried over Na₂SO₄. After concentration of the solvent, the residue was purified by column chromatography (EtOAc/hexanes, 5-15% gradient), affording the product as a white solid (6.02 g, 78%).
¹H NMR (500 MHz, CHCl₃-*d*) δ ppm 3.86 (s, 3 H) 5.42 (dd, *J*=10.4, 1.4 Hz, 1 H) 5.69 (dd, *J*=17.3, 1.4 Hz, 1 H) 6.62 (dd, *J*=3.2, 0.9 Hz, 1 H) 7.15 (d, *J*=3.2 Hz, 1 H) 7.45 (s, 1 H) 7.76 (dd, *J*=17.3, 10.4 Hz, 1 H) 8.12 (s, 1 H) 10.25 (s, 1 H). LC-MS 186.1 [M+H]⁺, RT 1.14 min.

### Step 5: 1-(1-methyl-6-vinyl-1H-indol-5-yl)pent-4-en-1-ol

To a solution of 1-methyl-6-vinyl-1H-indole-5-carbaldehyde (6.02 g, 32.50 mmol) in THF (65 mL) at -78 °C was added 3-butenylmagnesium bromide (0.5M in THF, 80.0 mL, 40.0 mmol) dropwise over ∼10 min. The reaction was stirred at -78 °C for 10 min and slowly allowed to warm to -10 °C. The reaction was quenched by addition of NH₄Cl (aqueous saturated, 100 mL). The product was extracted with EtOAc (4x150 mL). The combined organics were washed with NaCl (aqueous saturated, 100 mL) and dried over Na₂SO₄. The solvent was removed to provide the product as a pale-yellow oil (7.80 g, quant) which was used directly in the next step without further purification.
¹H NMR (500 MHz, Acetone) δ ppm 1.75 - 1.85 (m, 2 H) 2.11 - 2.29 (m, 2 H) 3.83 (s, 3 H) 4.01 (d, *J*=4.1 Hz, 1 H) 4.91 (ddt, *J*=10.2, 1.8, 1.2, 1.2 Hz, 1 H) 5.00 (dq, *J*=17.0, 1.8 Hz, 1 H) 5.06 (dt, *J*=7.4, 4.7 Hz, 1 H) 5.19 (dd, *J*=10.9, 1.9 Hz, 1 H) 5.66 (dd, *J*=17.3, 1.9 Hz, 1 H) 5.87 (ddt, *J*=17.0, 10.2, 6.7, 6.7 Hz, 1 H) 6.38 (dd, *J*=2.8, 0.8 Hz, 1 H) 7.20 (d, *J*=2.8 Hz, 1 H) 7.27 (dd, *J*=17.3, 10.9 Hz, 1 H) 7.51 (s, 1 H) 7.69 (s, 1 H). LC-MS 224.2 [M-H₂O+H]⁺, RT 1.28 min.

### Step 6: 1-methyl-1,5,6,7-tetrahydrocyclohepta[f]indol-5-ol

1-(1-methyl-6-vinyl-1H-indol-5-yl)pent-4-en-1-ol obtained above (*ca* 32.50 mmol) was dissolved in toluene (650 mL, 0.05M) under Argon. A second generation Grubbs' catalyst (800 mg, 0.94 mmol, 3 mol%) was added and the mixture was heated at 60 °C for 2 h until the starting material was completely consumed as indicated by LC/MS. After cooling the reaction mixture to room temperature, the toluene was removed under reduced pressure and the residue was purified by column chromatography (EtOAc/hexanes, 10-30% gradient) to afford a product (5.75 g, 83%) as a pale yellow solid.
¹H NMR (500 MHz, Acetone) δ ppm 2.00 - 2.18 (m, 2 H) 2.35 - 2.44 (m, 1 H) 2.45 - 2.55 (m, 1 H) 3.80 (s, 3 H) 4.05 (d, *J*=4.4 Hz, 1 H) 4.89 (dd, *J*=7.6, 4.4 Hz, 1 H) 5.78 (dt, *J*=12.1, 4.7 Hz, 1 H) 6.38 (dd, *J*=3.0, 0.8 Hz, 1 H) 6.54 (dt, *J*=12.1, 2.1 Hz, 1 H) 7.17 (d, *J*=3.0 Hz, 1 H) 7.19 (s, 1 H) 7.70 (s, 1 H).

### Step 7: 1-methyl-6,7-dihydrocyclohepta[f]indol-5(1H)-one

To activated 4Å molecular sieves (5.6 g, 250 mg/mmol) was added a solution of 1-methyl-1,5,6,7-tetrahydrocyclohepta[f]indol-5-ol (4.83 g, 22.63 mmol) in DCM (115 mL). The mixture was cooled to 0 °C, then NMO (4.00 g, 34.14 mmol) and TPAP (400 mg, 1.14 mmol, 5 mol%) were added. The reaction mixture was stirred at 0 °C and monitored by LC/MS. After complete consumption of starting material (∼1.5 h), the molecular sieves were filtered off and washed with DCM. The mother liquor was concentrated and the residue was purified by column chromatography (EtOAc/hexanes, 0-25% gradient). The product (4.04 g, 84%) was obtained as a white solid.
¹H NMR (500 MHz, CHCl₃-*d*) δ ppm 2.50 - 2.56 (m, 2 H) 2.97 - 3.02 (m, 2 H) 6.08 (dt, *J*=11.7, 5.4 Hz, 1 H) 6.56 (dd, *J*=3.2, 0.9 Hz, 1 H) 6.60 (d, *J*=11.7 Hz, 1 H) 7.10 (d, *J*=3.2 Hz, 1 H) 7.13 (s, 1 H) 8.27 (s, 1 H). LC-MS 212.2 [M+H]⁺, RT 1.17 min.

### Step 8: methyl 1-allyl-4-hydroxy-9-methyl-2-oxo-1,2,5,9-tetrahydropyrido[3',2':6,7]cyclohepta[1,2-f]indole-3-carboxylate

To a solution of 1-methyl-6,7-dihydrocyclohepta[f]indol-5(1H)-one (1.786 g, 8.46 mmol) in DCM (35 mL) was added allylamine (0.80 mL, 10.69 mmol) and NEt₃ (3.2 mL, 22.96 mmol). The mixture was cooled to 0 °C, then a TiCl₄ solution (1M DCM, 5.50 mL, 5.50 mmol) was added dropwise via syringe pump over 30 min. The reaction mixture was allowed to warm to room temperature and stirred overnight. The mixture was diluted with DCM (100 mL) and the reaction was quenched with NaHCO₃ (aqueous saturated, 50 mL). After vigorous shaking, the organic phase was separated using a PTFE phase separator and dried over Na₂SO₄. The solvent was removed to provide a yellow oil, which was used directly in the next step without further purification.

The intermediate N-(1-methyl-6,7-dihydrocyclohepta[f]indol-5(1H)-ylidene)prop-2-en-1-amine obtained above (*ca*. 8.46 mmol) and trimethyl methanetricarboxylate (2.75 g, 14.46 mmol) were mixed together in Ph₂O (17 mL). With stirring, the mixture was placed onto a pre-heated heat block at 230 °C and heated for 10 min after the initial bubbling of MeOH was observed (occurs at ∼160 °C internal reaction temperature). The reaction mixture was cooled to room temperature, then purified by column chromatography (hexanes, followed by EtOAc/hexanes 0-80% gradient) to provide the product as a yellow solid (2.15 g, 56% overall).
¹H NMR (500 MHz, CHCl₃-*d*) δ ppm 2.11 (ddd, *J*=14.1, 5.8, 2.0 Hz, 1 H) 3.51 (dd, *J*=14.1, 8.0 Hz, 1 H) 3.86 (s, 3 H) 4.01 (s, 3 H) 4.38 (dd, *J*=15.1, 5.9 Hz, 1 H) 4.53 (ddt, *J*=115.1, 4.6, 2.1 Hz, 1 H) 4.75 (dd, *J*=17.2, 1.1 Hz, 1 H) 5.05 (dd, *J*=10.4, 1.1 Hz, 1 H) 5.90 (dddd, *J*=17.2, 10.4, 5.9, 4.6 Hz, 1 H) 6.37 (ddd, *J*=9.8, 8.0, 5.8 Hz, 1 H) 6.53 (dd, *J*=3.2, 0.9 Hz, 1 H) 6.76 (dd, *J*=9.8, 2.0 Hz, 1 H) 7.20 (d, *J*=3.2 Hz, 1 H) 7.29 (s, 1 H) 7.85 (s, 1 H) 13.72 (br. s., 1 H). LC-MS 375.3 [M+H]⁺, 377.3 [M+H]⁺, RT 1.40 min.

### Step 9: methyl 4-hydroxy-9-methyl-2-oxo-1,2,5,9-tetrahydropyrido[3',2':6,7]cyclohepta[1,2-f]indole-3-carboxylate

Methyl 1-allyl-4-hydroxy-9-methyl-2-oxo-1,2,5,9-tetrahydropyrido[3',2':6,7]cyclohepta[1,2-f]indole-3-carboxylate (2.04 g, 5.42 mmol), phenylboronic acid (0.99 g, 8.12 mmol), Pd(OAc)₂ (37 mg, 0.16 mmol, 3 mol%), S-Phos ligand (135 g, 0.33 mmol, 6 mol%) and K₂CO₃ (2.25 g, 16.28 mmol) were mixed together in a 100 mL round bottom flask. The flask was placed under vacuum and back filled with Argon, then dioxane (22 mL) and H₂O (3.6 mL) were added. The mixture was heated at 85-90 °C for 2.5 h and monitored by LC/MS. After complete consumption of starting material, the reaction was cooled to room temperature. Water (30 mL) was added to the mixture and the product was extracted with DCM:MeOH mixture (9:1, 4x80 mL). The combined organics were washed with NaCl (aqueous saturated, 50 mL) and dried over Na₂SO₄. After concentration of the solvents, the residue was triturated with Et₂O, affording methyl 4-hydroxy-9-methyl-2-oxo-1,2,5,9-tetrahydropyrido[3',2':6,7]cyclohepta[1,2-f]indole-3-carboxylate (1.29 g, 71%) as green-yellow solid.
¹H NMR (500 MHz, DMSO-*d*₆) δ ppm 2.75 (br. s., 2 H) 3.84 (s, 3 H) 3.85 (s, 3 H) 6.26 (dt, *J*=9.9, 7.1 Hz, 1 H) 6.58 (dd, *J*=3.2, 0.6 Hz, 1 H) 6.81 (d, *J*=9.9 Hz, 1 H) 7.51 (d, *J*=3.2 Hz, 1 H) 7.52 (s, 1 H) 7.99 (s, 1 H) 11.34 (br. s., 1 H) 13.62 (br. s., 1 H). LC-MS 337.2 [M+H]⁺, RT 1.14 min.

### Step 10: 4-hydroxy-9-methyl-2-oxo-1,2,5,9-tetrahydropyrido[3',2':6,7]cyclohepta[1,2-f]indole-3-carboxylic acid

To a suspension of methyl 4-hydroxy-9-methyl-2-oxo-1,2,5,9-tetrahydropyrido[3',2':6,7]cyclohepta[1,2-f]indole-3-carboxylate (60.0 mg, 0.18 mmol) in EtOAc (2 mL) was added LiI (75.0 mg, 0.56 mmol). The reaction mixture was stirred and heated at 60 °C for 1.5 h until complete consumption of starting material was observed. The mixture was cooled to room temperature, then acidified with aqueous HCl (1M, 2 mL) and diluted with Et₂O. The product was collected by filtration, then washed with H₂O and Et₂O. After drying, 4-hydroxy-9-methyl-2-oxo-1,2,5,9-tetrahydropyrido[3',2':6,7]cyclohepta[1,2-f]indole-3-carboxylic acid (33.5 mg, 58%) was obtained as a bright yellow solid.
¹H NMR (500 MHz, DMSO-*d*₆) δ ppm 2.82 (br. s., 2 H) 3.86 (s, 3 H) 6.27 (dt, *J*=9.8, 6.9 Hz, 1 H) 6.62 (dd, *J*=3.2, 0.6 Hz, 1 H) 6.85 (d, *J*=9.8 Hz, 1 H) 7.55 (d, *J*=3.2 Hz, 1 H) 7.58 (s, 1 H) 8.05 (s, 1 H) 12.70 (br. s., 1 H) 13.94 (s, 1 H). LC-MS 321.2 [M-H]⁻, 323.2 [M+H]⁺, RT 1.22 min.

### Example 17

### 4-hydroxy-9-methyl-2-oxo-1,2,5,6,7,9-hexahydropyrido[3',2':6,7]cyclohepta[1,2-f]indole-3-carboxylic acid (Cpd 17)

A solution of 4-hydroxy-9-methyl-2-oxo-1,2,5,9-tetrahydropyrido[3',2':6,7]cyclohepta[1,2-f]indole-3-carboxylic acid (Example 16, Step 10, 15.0 mg, 0.04 mmol) in DCM (10 mL), MeOH (5 mL) and AcOH (1 mL) was hydrogenated over Pd/C (10%, 15 mg) under 1 atm of H₂. After 1.5 h, the catalyst was filtered off and the filtrate was washed with DCM/MeOH. The mother liquor was concentrated and the residue was triturated with Et₂O to afford the product 4-hydroxy-9-methyl-2-oxo-1,2,5,6,7,9-hexahydropyrido[3',2':6,7]cyclohepta[1,2-f]indole-3-carboxylic acid (8.0 mg, 53%) as a bright yellow solid.
¹H NMR (500 MHz, DMSO-*d*₆) δ ppm 2.09 (quin, *J*=6.8 Hz, 2 H) 2.50 - 2.52 (m, 2 H, solvent overlap) 2.69 (br. s., 2 H) 3.83 (s, 3 H) 6.54 (d, *J*=3.2 Hz, 1 H) 7.41 (d, *J*=3.2 Hz, 1 H) 7.47 (s, 1 H) 7.77 (s, 1 H) 12.85 (br. s., 1 H) 13.85 (s, 1 H). LC-MS 323.1 [M-H]⁻, 325.2 [M+H]⁺, RT 1.24 min.

### Example 18

### 8-fluoro-4-hydroxy-9-methyl-2-oxo-1,2,5,6,7,9-hexahydropyrido[3',2':6,7]cyclohepta[1,2-f]indole-3-carboxylic acid (Cpd 18)

### Step 1: 4-bromo-3-chloro-2-fluoro-6-iodoaniline

To a suspension of 4-bromo-3-chloro-2-fluoroaniline (33.4 g, 149 mmol) in HOAc (250 mL) was added NIS (34.2 g, 152 mmol) at room temperature in four portions. The reaction was stirred for 12 h at room temperature then was poured directly onto ice (300 g). The precipitate was filtered, washed with H₂O and dried under N₂ flow overnight. A crude product (54.4 g, quant.) was obtained as a tan solid and was used directly in the next step without further purification.
¹H NMR (500 MHz, CHCl₃-*d*) δ ppm 4.26 (br. s., 2 H) 7.69 (d, *J*=2.21 Hz, 1 H). LC-MS 351.7/353.7 [M+H]⁺, RT 1.54 min.

### Step 2: 4-bromo-3-chloro-2-fluoro-6-((trimethylsilyl)ethynyl)aniline

To a suspension of 4-bromo-3-chloro-2-fluoro-6-iodoaniline (14.0 g, 40 mmol) in CH₃CN (120 mL) was added ethynyltrimethylsilane (6.3 mL, 44 mmol) and NEt₃ (11.2 mL, 80 mmol). The mixture was degassed with Argon, then Pd(PPh₃)₂Cl₂ (0.280 g, 0.4 mmol, 1 mol%) and CuI (0.153 g, 0.8 mmol, 2 mol%) were added. The reaction mixture was heated at 70 °C for 1.5 h until the starting material was completely consumed. After cooling to room temperature, the CH₃CN was removed under reduced pressure. Water (400 mL) was added to the residue and then the mixture was extracted with CH₂Cl₂ (3x100 mL). The combined organic layers were dried, then concentrated to afford the title compound which was used directly in the next step without further purification.
¹H NMR (500 MHz, CHCl₃-*d*) δ ppm 0.27 (s, 9 H) 4.35 (br. s., 2 H) 7.36 (d, *J*=1.89 Hz, 1 H). LC-MS 322.0 [M+H]⁺, RT 1.76 min.

### Step 3: 5-bromo-6-chloro-7-fluoro-1-methyl-1H-indole

A solution of 4-bromo-3-chloro-2-fluoro-6-((trimethylsilyl)ethynyl)aniline (*ca* 40 mmol) in DMF (120 mL) was degassed with Argon, then t-BuOK (9.9 g, 88 mmol) was added. The mixture was heated at 70 °C for 1 h under Argon, then cooled to 0 °C and MeI (5.7 mL, 90 mmol) was added dropwise. After stirring at room temperature for 30 min, mixture was carefully poured onto ice (∼300 mL) in the presence of HCl (conc, 7 mL). The precipitate was filtered, then washed with H₂O (2x100 mL) and dried in an N₂ flow. The resulting crude solid was purified by column chromatography using DCM/hexanes (gradient 20-50%), affording 5-bromo-6-chloro-7-fluoro-1-methyl-1H-indole (7.80 g, 29.7 mmol, 74%, four steps).
¹H NMR (500 MHz, CHCl₃-*d*) δ ppm 3.98 (d, *J*=1.89 Hz, 3 H) 6.37 - 6.43 (m, 1 H) 7.01 (d, *J*=3.15 Hz, 1 H) 7.65 (d, *J*=1.26 Hz, 1 H).

### Step 4: 6-chloro-7-fluoro-1-methyl-1H-indole-5-carbaldehyde

To a solution of 5-bromo-6-chloro-7-fluoro-1-methyl-1H-indole (7.80 g, 29.7 mmol) in THF (100 mL) at -95 °C was added *n*-BuLi (2.5M in hexanes, 13.2 mL, 33.0 mmol) dropwise over ∼15 min. The reaction was stirred at -95 °C for 2 min, then DMF (4 mL, 51 mmol) was added dropwise. After 10 min, the reaction was quenched by saturated aq. NH₄Cl then extracted with ether (3x60 mL). The combined organic layers were dried over Na₂SO₄ then concentrated to give a crude product which was triturated from hexanes to afford the title compound (4.05 g, 19.1 mmol). The filtrate was concentrated, then the residue was purified by column chromatography (50% DCM in hexanes) to afford additional 6-chloro-7-fluoro-1-methyl-1H-indole-5-carbaldehyde (0.849 g, 4.0 mmol, 78%).
¹H NMR (500 MHz, CHCl₃-*d*) δ ppm 4.04 (d, *J*=1.89 Hz, 3 H) 6.62 (dd, *J*=3.15, 2.21 Hz, 1 H) 7.08 (d, *J*=3.15 Hz, 1 H) 8.03 - 8.07 (m, 1 H) 10.45 (s, 1 H). LC-MS 212.0/214.0 [M+H]⁺, RT 1.37 min.

### Step 5: 7-fluoro-1-methyl-6-vinyl-1H-indole-5-carbaldehyde

6-chloro-7-fluoro-1-methyl-1H-indole-5-carbaldehyde (2.7 g, 12.8 mmol), potassium vinyltrifluoroborate (2.6 g, 19.1 mmol), Pd(OAc)₂ (86 mg, 0.38 mmol, 3 mol%), S-Phos ligand (314 mg, 0.77 mmol, 6 mol%) and K₂CO₃ (5.28 g, 38.3 mmol) were mixed together in a 250 mL round bottom flask. The flask was placed under vacuum and back filled with Argon, then dioxane (45 mL) and H₂O (9 mL) were added. The mixture was heated at 85-90 °C for 2 h and monitored by LC/MS. After complete consumption of starting material, the reaction was cooled to room temperature. Water (40 mL) was added to the mixture and the product was extracted with DCM (4x50 mL). The combined organics were washed with NaCl (aqueous saturated, 150 mL) and dried over Na₂SO₄. After concentration of the solvent, the residue was purified by column chromatography (EtOAc/hexanes, 5-15% gradient), affording the product as a light yellow solid (2.10 g, 81%).
¹H NMR (500 MHz, CHCl₃-*d*) δ ppm 4.05 (d, *J*=2.21 Hz, 3 H) 5.66 (dt, *J*=17.65, 1.58 Hz, 1 H) 5.74 (ddd, *J*=11.51, 1.58, 0.79 Hz, 1 H) 6.58 - 6.64 (m, 1 H) 7.09 (d, *J*=3.15 Hz, 1 H) 7.22 - 7.33 (m, 1 H) 7.97 (s, 1 H) 10.19 (d, *J*=0.95 Hz, 1 H). LC-MS 204.1 [M+H]⁺, RT 1.37 min.

### Step 6: 1-(7-fluoro-1-methyl-6-vinyl-1H-indol-5-yl)pent-4-en-1-ol

To a solution of 7-fluoro-1-methyl-6-vinyl-1H-indole-5-carbaldehyde (2.1 g, 10.3 mmol) in THF (10 mL) at -78 °C was added 3-butenylmagnesium bromide (0.5M in THF, 31.0 mL + 12.0 mL, 15.5 mmol + 6.0 mmol) dropwise over ∼10 min at intervals of 1 h. The mixture was stirred at -78 °C for 10 min and slowly allowed to warm to -10 °C. The reaction was quenched by addition of NH₄Cl (aqueous saturated, 20 mL). The product was extracted with EtOAc (4x50 mL). The combined organics were washed with NaCl (aqueous saturated, 100 mL) and dried over Na₂SO₄. The solvent was removed to provide a pale-yellow oil (2.49 g, 9.6 mmol, 93%). The product was used directly in the next step without further purification.
¹H NMR (500 MHz, CHCl₃-*d*) δ ppm 1.82 (d, *J*=3.78 Hz, 1 H) 1.84 - 1.95 (m, 2 H) 2.11 - 2.30 (m, 2 H) 4.00 (d, *J*=2.21 Hz, 3 H) 4.95 - 5.10 (m, 3 H) 5.57 - 5.65 (m, 1 H) 5.69 (dt, *J*=17.73, 2.01 Hz, 1 H) 5.87 (ddt, *J*=17.06, 10.36, 6.62, 6.62 Hz, 1 H) 6.43 (t, *J*=2.68 Hz, 1 H) 6.77 - 6.86 (m, 1 H) 6.97 - 7.01 (m, 1 H) 7.50 (s, 1 H).

### Step 7: 1-(7-fluoro-1-methyl-6-vinyl-1H-indol-5-yl)pent-4-en-1-one

To activated 4Å molecular sieves (2.6 g, 400 mg/mmol) was added a solution of 1-(7-fluoro-1-methyl-6-vinyl-1H-indol-5-yl)pent-4-en-1-ol (1.66 g, 6.4 mmol) in DCM (25 mL). The mixture was cooled to 0 °C, then NMO (1.13 g, 9.6 mmol) and TPAP (112 mg, 0.32 mmol, 5 mol%) were added. The reaction mixture was stirred at 0 °C and monitored by LC/MS. After complete consumption of the starting material (∼1.5 h), the molecular sieves were filtered off and washed with DCM. The mother liquor was concentrated and the residue was purified by column chromatography (EtOAc/hexanes, 0-25% gradient). The product (1.36 g, 83%) was obtained as a white solid.
¹H NMR (500 MHz, CHCl₃-*d*) δ ppm 2.42 - 2.51 (m, 2 H) 2.98 - 3.06 (m, 2 H) 4.02 (d, *J*=1.89 Hz, 3 H) 5.00 (dq, *J*=10.13, 1.46 Hz, 1 H) 5.07 (dq, *J*=17.06, 1.67 Hz, 1 H) 5.50 - 5.64 (m, 2 H) 5.88 (ddt, *J*=16.98, 10.36, 6.50, 6.50 Hz, 1 H) 6.50 - 6.55 (m, 1 H) 6.89 - 6.99 (m, 1 H) 7.05 (d, *J*=3.15 Hz, 1 H) 7.68 (s, 1 H). LC-MS 258.1 [M+H]⁺, RT 1.53 min.

### Step 8: 10-fluoro-1-methyl-6,7-dihydrocyclohepta[f]indol-5(1H)-one

To a solution of 1-(7-fluoro-1-methyl-6-vinyl-1H-indol-5-yl)pent-4-en-1-one (1.36 g, 5.3 mmol) in toluene (100 mL, 0.05M) was added a second generation Grubbs' catalyst (90 + 90 mg, 0.106 + 0.106 mmol, 2 + 2 mol%) under Argon. The reaction mixture was heated at 60 °C for 2 h until the starting material was completely consumed as indicated by LC/MS. After cooling the reaction to room temperature, the toluene was removed under reduced pressure and the residue was purified by column chromatography (EtOAc/hexanes, 10-30% gradient) to afford a product (1.08 g, 89%) as a light yellow solid.
¹H NMR (500 MHz, CHCl₃-*d*) δ ppm 2.50 - 2.58 (m, 2 H) 2.94 - 3.01 (m, 2 H) 4.01 (d, *J*=2.21 Hz, 3 H) 6.14 - 6.23 (m, 1 H) 6.53 (t, *J*=2.84 Hz, 1 H) 6.90 - 6.95 (m, 1 H) 7.03 (d, *J*=3.15 Hz, 1 H) 7.98 (s, 1 H). LC-MS 230.1 [M+H]⁺, RT 1.40 min.

### Step 9: 10-fluoro-1-methyl-6,7,8,9-tetrahydrocyclohepta[f]indol-5(1H)-one

A solution of 10-fluoro-1-methyl-6,7-dihydrocyclohepta[f]indol-5(1H)-one (0.62 g, 2.7 mmol) in ethyl acetate (10 mL) was hydrogenated over Pd/C (10%, 50 mg) under H₂ (1atm). After 1.5 h, the catalyst was filtered off and the filtrate was washed with ethyl acetate (40 mL). The mother liquor was concentrated and the residue was purified by column chromatography (EtOAc/hexanes, 0-15% gradient) to afford a product (0.514 g, 82%) as a light yellow solid.
¹H NMR (500 MHz, CHCl₃-*d*) δ ppm 1.76 - 1.85 (m, 2 H) 1.85 - 1.94 (m, 2 H) 2.71 - 2.79 (m, 2 H) 3.07 (td, *J*=6.46, 1.89 Hz, 2 H) 4.01 (d, *J*=2.21 Hz, 3 H) 6.50 - 6.57 (m, 1 H) 6.99 (d, *J*=3.15 Hz, 1 H) 7.83 (s, 1 H). LC-MS 232.2 [M+H]⁺, RT 0.81 min (1min Method).

### Step 10: N-(10-fluoro-1-methyl-6,7,8,9-tetrahydrocyclohepta[f]indol-5(1H)-ylidene)-2-methylpropan-2-amine

To a solution of 10-fluoro-1-methyl-6,7,8,9-tetrahydrocyclohepta[f]indol-5(1H)-one (514 mg, 2.22 mmol) in DCM (5 mL) was added 2-methylpropan-2-amine (1.17 mL, 11.1 mmol). The reaction mixture was cooled to 0 °C, then a solution of TiCl₄ (1M DCM, 1.78 mL, 1.78 mmol) was added dropwise via syringe pump over 30 min. The mixture was allowed to warm to room temperature and stirred overnight. The mixture was diluted with DCM (20 mL) and the reaction was quenched with NaHCO₃ (aqueous saturated, 20 mL). After vigorous shaking, the organic phase was separated using a PTFE phase separator and dried over Na₂SO₄. The solvent was removed to provide the title compound as a yellow oil, which was used directly in the next step without further purification. LC-MS 287.2 [M+H]⁺, RT 1.11 min.

### Step 11: 8-fluoro-4-hydroxy-9-methyl-2-oxo-1,2,5,6,7,9-hexahydropyrido[3',2':6,7]cyclohepta[1,2-f]indole-3-carboxylic acid

The *N-*(10-fluoro-1-methyl-6,7,8,9-tetrahydrocyclohepta[f]indol-5(1H)-ylidene)-2-methylpropan-2-amine obtained above (*ca*. 2.22 mmol) and trimethyl methanetricarboxylate (0.72 g, 3.79 mmol) were mixed together in Ph₂O (5 mL). With stirring, the mixture was placed onto a pre-heated heat block at 230 °C and heated for 10 min. The reaction mixture was cooled to room temperature. The precipitate was filtered, then washed with ether, to give a crude product (22 mg, 3%) which was used directly in the next step without further purification. LC-MS 357.1 [M+H]⁺, RT 1.38 min.

To a suspension of the ester obtained above (22 mg, 0.062 mmol) in EtOAc (2 mL) was added LiI (50 mg, 0.37 mmol). The reaction mixture was stirred and heated at 60 °C for 1.5 h until complete consumption of the starting material was observed. The mixture was cooled to room temperature, then acidified with aqueous HCl (1M, 1 mL) and diluted with Et₂O. The product was collected by filtration, then washed with H₂O and Et₂O. After drying, the product (12 mg, 57%) was obtained as a bright yellow solid.
¹H NMR (500 MHz, DMSO-*d*₆) δ ppm 2.01 - 2.10 (m, 2 H) 2.15 - 2.40 (m, 2 H) 2.55 - 2.85 (m, 2 H) 4.00 (d, *J*=2.21 Hz, 3 H) 6.58 (br. s., 1 H) 7.42 (d, *J*=3.15 Hz, 1 H) 7.61 (s, 1 H) 12.91 (br. s., 1 H) 14.04 (br. s., 1 H) 16.22 (br. s., 1 H). LC-MS 341.2 [M-H]⁻, 343.2 [M+H]⁺, RT 1.39 min.

### Example 19

### 2-((ethylamino)methyl)-8-hydroxy-1-methyl-6-oxo-1,5,6,9-tetrahydropyrido[3',2':4,5]cyclopenta[1,2-f]indole-7-carboxylic acid hydrochloride (Cpd 19)

### Step 1: 4-amino-2-chloro-5-(3-hydroxyprop-1-ynyl)benzonitrile

To a solution of 4-amino-2-chloro-5-iodobenzonitrile prepared according to J. Med. Chem. 2001, 44, 3856 (51.73 g, 186 mmol) in CH₃CN (270 mL) was added propargyl alcohol (13.3 mL, 223 mmol) and NEt₃ (52 mL, 373 mmol). The mixture was degassed with Argon, then Pd(PPh₃)₂Cl₂ (1.30 g, 1.85 mmol) and CuI (0.70 g, 3.67 mmol) were added. The reaction mixture was heated at 70 °C for 2 h until the starting material was completely consumed. After cooling to room temperature, the CH₃CN was removed under reduced pressure. Water (300 mL) was added to the residue and the resulting precipitate was filtered, then washed with H₂O (2x200 mL) and dried in an N₂ flow. The crude product was obtained as a tan solid (40.00 g) and may be further purified by column chromatography using EtOAc/hexanes (gradient 50-100%) or can be used directly in the next step without further purification.
¹H NMR (500 MHz, DMSO-*d*₆) δ ppm 4.33 (d, *J*=6.0 Hz, 2 H) 5.29 (t, *J*=6.0 Hz, 1 H) 6.67 (br. s., 2 H) 6.86 (s, 1 H) 7.64 (s, 1 H). LC-MS 205.1/207.1 [M-H]⁻, 207.1/209.1 [M+H]⁺, RT 0.92 min.

### Step 2: 6-chloro-2-(hydroxymethyl)-1H-indole-5-carbonitrile

To a solution of the 4-amino-2-chloro-5-(3-hydroxyprop-1-ynyl)benzonitrile (36.70 g, 178 mmol) in DMF (450 mL) was added t-BuOK (44.0 g, 392 mmol). The mixture was heated at 70 °C for 2 h under Argon. After cooling to room temperature, the mixture was carefully poured onto ice (∼800 mL) in the presence of HCl (conc, 50 mL). The resulting precipitate was filtered, then washed with H₂O (2x300 mL) and dried in an N₂ flow. The product was obtained as brownish solid (28.50 g, 77% 2 steps) and was used directly in the next step without further purification.
¹H NMR (500 MHz, DMSO-*d*₆) δ ppm 4.63 (d, *J*=5.7 Hz, 2 H) 5.47 (t, *J*=5.7 Hz, 1 H) 6.44 (s, 1 H) 7.58 (s, 1 H) 8.12 (s, 1 H) 11.79 (br. s., 1 H). LC-MS 205.1/207.1 [M-H]⁻, 207.1/209.1 [M+H]⁺, RT 0.92 min.

### Step 3: 2-((tert-butyldimethylsilyloxy)methyl)-6-chloro-1H-indole-5-carbonitrile

To a solution of the 6-chloro-2-(hydroxymethyl)-1H-indole-5-carbonitrile (32.66 g, 158 mmol) in DMF (450 mL) was added imidazole (14.0 g, 205 mmol). The mixture was stirred at room temperature for 5 min, then TBSCl (29.0 g 192 mmol) was added in one portion. The reaction mixture was stirred at room temperature for 1.5 h and then poured onto ice/H₂O (total final volume ∼1700 mL). A dark brown oil was formed which solidified after addition of pentane (∼100 mL). The resulting solid was filtered, washed with H₂O (2x300 mL) and dried in an N₂ flow overnight. The solid was washed with pentane (2x300 mL) and then suspended in 800 mL of CH₂Cl₂. The resulting mixture was stirred vigorously at room temperature for 1 h, then filtered through Celite. The mother liquor was concentrated to yield the product as a brownish solid (37.42 g, 74%) which was used in the next step without further purification.
¹H NMR (500 MHz, CHCl₃-*d*) δ ppm 0.13 (s, 6 H) 0.94 (s, 9 H) 4.88 (s, 2 H) 6.36 (s, 1 H) 7.49 (s, 1 H) 7.89 (s, 1 H) 8.63 (br. s, 1 H). LC-MS 319.3/321.3 [M-H]⁻, 321.2/323.2 [M+H]⁺, RT 1.62 min.

### Step 4: 2-((tert-butyldimethylsilyloxy)methyl)-6-chloro-1-methyl-1H-indole-5-carbonitrile

To a solution of the 2-((tert-butyldimethylsilyloxy)methyl)-6-chloro-1H-indole-5-carbonitrile (37.50 g, 117 mmol) in DMF (400 mL) at 0 °C was added NaH (60%, 6.7 g, 168 mmol) in portions. After completion of the addition reaction, the mixture was allowed to warm to room temperature with stirring for 10 min. The reaction mixture was then cooled to 0 °C and MeI (10.5 mL, 169 mmol) was added. The reaction mixture was allowed to warm to room temperature and was stirred for 1.5 h, then poured onto ice/H₂O in the presence of 100 mL 1M HCl (total final volume ∼1600 mL). The resulting precipitate was filtered, then washed with H₂O (3x200 mL) and dried in an N₂ flow overnight. The solid was washed with pentane (2x200 mL). The resulting crude product was obtained as a brownish solid (39.00 g), then further purified by column chromatography to remove base-line by-products eluting with CH₂Cl₂/hexanes (gradient 50-100%) to yield a pure product as a pale orange solid (∼30.0 g, 76%). The 4 step process affords a final product having a 38-43% overall yield over the 4 steps.
¹H NMR (500 MHz, CHCl₃-*d*) δ ppm 0.08 (s, 6 H) 0.90 (s, 9 H) 3.79 (s, 3 H) 4.82 (s, 2 H) 6.44 (s, 1 H) 7.41 (s, 1 H) 7.90 (s, 1 H). LC-MS 335.2/337.2 [M+H]⁺, RT 1.72 min.

### Step 5: 2-((tert-butyldimethylsilyloxy)methyl)-6-chloro-1-methyl-1H-indole-5-carbaldehyde

To a solution of 2-((tert-butyldimethylsilyloxy)methyl)-6-chloro-1-methyl-1H-indole-5-carbonitrile (4.15 g, 12.4 mmol) in DCM (50 mL) at -78 °C was added DIBAL-H (1M in DCM, 15.0 mL, 15.0 mmol) dropwise over -10 min. The reaction mixture was stirred at -78 °C for 10 min and slowly allowed to warm to -15 °C over ∼2 h. LC/MS indicated complete consumption of starting material. The mixture was cooled to -40 °C and the reaction was quenched by addition of the Rochelle salt (aqueous saturated, 20 mL). The resulting emulsion was allowed to warm to room temperature and vigorously stirred for ∼1 h, then the organic phase was separated and the aqueous phase was extracted with DCM (50 mL). The combined organics were washed subsequently with 1M HCl (50 mL), NaHCO₃ (aqueous saturated, 50 mL) and NaCl (aqueous saturated, 50 mL), then dried over Na₂SO₄. The solvent was removed and the residue was purified by column chromatography (EtOAc/hexanes, 5-15% gradient), affording the product as an off-white solid (3.80 g, 91 %).
¹H NMR (500 MHz, CHCl₃-*d*) δ ppm 0.08 (s, 6 H) 0.90 (s, 9 H) 3.79 (s, 3 H) 4.82 (s, 2 H) 6.49 (s, 1 H) 7.33 (s, 1 H) 8.21 (s, 1 H) 10.50 (s, 1 H). LC-MS 338.2/340.3 [M+H]⁺, RT 1.76 min.

### Step 6: 2-((tert-butyldimethylsilyloxy)methyl)-1-methyl-6-vinyl-1H-indole-5-carbaldehyde

2-((tert-butyldimethylsilyloxy)methyl)-6-chloro-1-methyl-1 H-indole-5-carbaldehyde (3.80 g, 11.24 mmol), potassium vinyltrifluoroborate (2.30 g, 17.17 mmol), Pd(OAc)₂ (76 mg, 0.34 mmol, 3 mol%), S-Phos ligand (280 mg, 0.68 mmol, 6 mol%) and K₂CO₃ (4.70 g, 34.0 mmol) were mixed together in a 100 mL round bottom flask. The flask was placed under vacuum and back filled with Argon, then dioxane (45 mL) and H₂O (7.5 mL) were added. The mixture was heated at 85-90 °C for 5 h and monitored by LC/MS. After complete consumption of the starting material, the reaction mixture was cooled to room temperature. Water (30 mL) was added to the mixture and the product was extracted with DCM (3x80 mL). The combined organics were washed with NaCl (aqueous saturated, 80 mL) and dried over Na₂SO₄. After concentration of the solvent, the residue was purified by column chromatography (EtOAc/hexanes, 0-10% gradient), affording the product as a white solid (3.32 g, 89 %).
¹H NMR (500 MHz, CHCl₃-*d*) δ ppm 0.09 (s, 6 H) 0.91 (s, 9 H) 3.84 (s, 3 H) 4.85 (s, 3 H) 5.42 (dd, *J*=10.7, 1.7 Hz, 1 H) 5.69 (dd, *J*=17.2, 1.7 Hz, 2 H) 6.51 (s, 1 H) 7.43 (s, 2 H) 7.75 (dd, *J*=17.3, 10.7 Hz, 1 H) 8.07 (s, 1 H) 10.24 (s, 1 H). LC-MS 330.3 [M+H]⁺, RT 1.74 min.

### Step 7: 1-(2-((tert-butyldimethylsilyloxy)methyl)-1-methyl-6-vinyl-1H-indol-5-yl)but-2-en-1-ol

To a solution of 2-((tert-butyldimethylsilyloxy)methyl)-1-methyl-6-vinyl-1H-indole-5-carbaldehyde (4.10 g, 12.4 mmol) in THF (30 mL) at -78 °C was added 1-propenylmagnesium chloride (0.5M in THF, 37.3 mL, 18.7 mmol) dropwise over ∼10 min. The reaction was stirred at -78 °C for 10 min and slowly allowed to warm to 0 °C. The reaction was quenched by addition of NH₄Cl (aqueous saturated, 40 mL). The product was extracted with EtOAc (3x80 mL) and the combined organics were washed with NaCl (aqueous saturated, 50 mL) and dried over Na₂SO₄. The solvent was removed to provide the product as a pale-yellow oil (5.07 g, quant.). The product was used directly in the next step without further purification.
¹H NMR (500 MHz, CHCl₃-*d*) δ ppm 0.00 - 0.12 (m, 6 H) 0.85 - 0.94 (m, 9 H) 1.70 - 1.78 (m, 3 H) 3.78 - 3.80 (m, 3 H) 4.82 (s, 2 H) 5.24 - 5.33 (m, 1 H) 5.52 - 5.69 (m, 2 H) 5.71 - 5.90 (m, 2 H) 6.29 - 6.38 (m, 1 H) 7.24 - 7.33 (m, 1 H) 7.42 (s, 1 H) 7.64 - 7.70 (m, 1 H)

### Step 8: 2-((tert-butyldimethylsilyloxy)methyl)-1-methyl-1,5-dihydrocyclopenta[f]indol-5-ol

The 1-(2-((tert-butyldimethylsilyloxy)methyl)-1-methyl-6-vinyl-1H-indol-5-yl)but-2-en-1-ol obtained above was dissolved in CH₂Cl₂ (250 mL, 0.05M) under Argon. A second generation Grubbs' catalyst (320 mg, 0.38 mmol, 3 mol%) was added and the mixture was stirred at room temperature overnight. After complete consumption of the starting material was indicated by TLC, the solvent was removed under reduced pressure to give a solid crude product. The residue was dissolved in CH₂Cl₂ followed by dropwise addition of hexanes to precipitate the desired product. The solid was filtered and washed by hexanes to afford the product (3.37 g, 10.2 mmol, 82%, 2 steps).
¹H NMR (500 MHz, CHCl₃-*d*) δ ppm 0.06 (s, 6 H) 0.90 (s, 9 H) 3.79 (s, 3 H) 4.82 (s, 2 H) 5.24 (br. s., 1 H) 6.33 - 6.40 (m, 2 H) 6.79 - 6.84 (m, 1 H) 7.14 (s, 1 H) 7.68 (d, *J*=0.95 Hz, 1 H). LC-MS 330.2 [M+H]⁺, RT 1.57 min.

### Step 9: 2-((tert-butyldimethylsilyloxy)methyl)-1-methylcyclopenta[f]indol-5(1H)-one

To activated 4Å molecular sieves (1 g, 400 mg/mmol) was added a solution of 2-((tert-butyldimethylsilyloxy)methyl)-1-methyl-1,5-dihydrocyclopenta[f]indol-5-ol (0.82 g, 2.49 mmol) in DCM (12 mL). The mixture was cooled to 0 °C, then NMO (0.35 g, 2.98 mmol) and TPAP (44 mg, 0.125 mmol, 5 mol%) were added. The reaction mixture was stirred at 0 °C and monitored by LC/MS. After complete consumption of starting material (∼1.5 h), the molecular sieves were filtered off and washed with DCM. The filtrate was concentrated to afford a crude product which was used in the next step without further purification.
¹H NMR (500 MHz, CHCl₃-*d*) δ ppm 0.07 (s, 6 H) 0.90 (s, 9 H) 3.77 (s, 3 H) 4.77 (s, 2 H) 5.85 (d, *J*=5.99 Hz, 1 H) 6.40 (d, *J*=0.63 Hz, 1 H) 6.91 (s, 1 H) 7.51 (dd, *J*=5.99, 0.95 Hz, 1 H) 7.64 (s, 1 H). LC-MS 328.0 [M+H]⁺, RT 1.66 min.

### Step 10: 2-((tert-butyldimethylsilyloxy)methyl)-1-methyl-6,7-dihydrocyclopenta[f]indol-5(1H)-one

A solution of the 2-((tert-butyldimethylsilyloxy)methyl)-1-methylcyclopenta[f]indol-5(1H)-one obtained above (*ca* 2.49 mmol) in EtOAc (15 mL) was hydrogenated over Pd/C (10%, 100 mg) under H₂ (1 atm) until complete consumption of the starting material was indicated by TLC (2x 10%EtOAc/hexanes). After ∼1 h, the catalyst was filtered off and the filtrate was washed with EtOAc. The mother liquor was concentrated and the product was purified by column chromatography (EtOAc/hexanes, 0-25% gradient) to yield the desired product (0.67 g, 2.04 mmol, 82%, 2 steps) as a pale yellow solid.
¹H NMR (500 MHz, CHCl₃-*d*) δ ppm 0.08 (s, 6 H) 0.90 (s, 9 H) 2.72 - 2.78 (m, 2 H) 3.22 - 3.28 (m, 2 H) 3.81 (s, 3 H) 4.83 (s, 2 H) 6.49 (d, *J*=0.63 Hz, 1 H) 7.29 (s, 1 H) 8.02 (s, 1 H). LC-MS 330.1 [M+H]⁺, RT 1.60 min.

### Step 11: N-(2-((tert-butyldimethylsilyloxy)methyl)-1-methyl-6,7-dihydrocyclopenta[f]indol-5(1H)-ylidene)-1-(2,4-dimethoxyphenyl)methanamine

To a solution of 2-((tert-butyldimethylsilyloxy)methyl)-1-methyl-6,7-dihydrocyclopenta[f]indol-5(1H)-one (0.67 g, 2.04 mmol) in DCM (9 mL) was added 2,4-dimethoxybenzylamine (0.32 mL, 2.13 mmol) and NEt₃ (0.74 mL, 5.31 mmol). The reaction mixture was cooled to 0 °C, then a solution of TiCl₄ (1M DCM, 1.33 mL, 1.33 mmol) was added dropwise via syringe pump over 30 min. The mixture was allowed to warm to room temperature and stirred overnight, then diluted with DCM (20 mL) and then the reaction was quenched with NaHCO₃ (aqueous saturated, 10 mL). After vigorous shaking, the organic phase was separated using a PTFE phase separator, and dried over Na₂SO₄. The solvent was removed to afford a crude product (∼1.09 g, quant) as a yellow solid, which was used directly in the next step without further purification. LC-MS 479.1 [M+H]⁺, RT 1.25 min.

### Step 12: methyl 2-(((tert-butyldimethylsilyl)oxy)methyl)-5-(2,4-dimethoxybenzyl)-8-hydroxy-1-methyl-6-oxo-1,5,6,9-tetrahydropyrido[3',2':4,5]cyclopenta[1,2-f]indole-7-carboxylate

The crude product obtained above (1.09 g, ca. 2.04 mmol) and trimethyl methanetricarboxylate (0.68 g, 3.58 mmol) were mixed together in Ph₂O (6.0 mL). With stirring, the mixture was placed onto a pre-heated heat block at 230 °C and heated for 10 min after the initial bubbling of MeOH was observed (occurs ant ∼160 °C internal reaction temperature) under a blanket of Argon. The reaction mixture was cooled to room temperature, then purified by column chromatography (hexanes, followed by EtOAc/hexanes 25-60% gradient) to yield the product as a yellow foam (404 mg, 33% 2 steps). LC-MS 605.3 [M+H]⁺, RT 1.80 min.

### Steps 13-15: 5-(2,4-dimethoxybenzyl)-2-formyl-8-hydroxy-1-methyl-6-oxo-1,5,6,9-tetrahydropyrido[3',2':4,5]cyclopenta[1,2-f]indole-7-carboxylic acid

To a suspension of the methyl 2-(((tert-butyldimethylsilyl)oxy)methyl)-5-(2,4-dimethoxybenzyl)-8-hydroxy-1-methyl-6-oxo-1,5,6,9-tetrahydropyrido[3',2':4,5]cyclopenta[1,2-f]indole-7-carboxylate obtained above (56 mg, 0.093 mmol) in EtOAc (1.5 mL) was added LiI (100 mg, 0.75 mmol). The reaction mixture was stirred and heated at 60 °C for 1.5 h until complete consumption of the starting material was observed. The mixture was cooled to room temperature and acidified with aqueous HCl (1M, 0.5 mL). The product was extracted with EtOAc (3x10 mL), then the organic phase was washed with Na₂S₂O₃ (10% aq, 5 mL), NaCl (aqueous saturated, 10 mL) and dried over Na₂SO₄. The solvent was removed to provide the product as a yellow solid (ca. 60 mg, quant). LC-MS 589.0 [M-H]⁻, 591.0 [M+H]⁺, RT 1.82 min.

To a solution of the 2-(((tert-butyldimethylsilyl)oxy)methyl)-5-(2,4-dimethoxybenzyl)-8-hydroxy-1-methyl-6-oxo-1,5,6,9-tetrahydropyrido[3',2':4,5]cyclopenta[1,2-f]indole-7-carboxylic acid obtained above (ca. 60 mg, ca. 0.1 mmol) in THF (1 mL) was added a TBAF solution (1M THF, 0.5 mL, 0.5 mmol). The reaction mixture was stirred at room temperature for 2 h until the starting material was completely consumed. The THF was removed and the residue was purified by column chromatography (MeOH/DCM, 0-6% gradient). The product was obtained as a yellow foam (ca. 50 mg). LC-MS 475.1 [M-H]⁻, 477.0 [M+H]⁺, RT 1.27 min.

To a solution of 5-(2,4-dimethoxybenzyl)-8-hydroxy-2-(hydroxymethyl)-1-methyl-6-oxo-1,5,6,9-tetrahydropyrido[3',2':4,5]cyclopenta[1,2-f]indole-7-carboxylic acid (ca. 50 mg, ca. 0.1 mmol) in DCM (2 mL) was added activated MnO₂ (90 mg+ 90 mg+ 90 mg, 1.0 mmol + 1.0 mmol + 1.0 mmol) in 3 portions at 30 min intervals. The reaction was monitored by LC/MS. After 2 h, the MnO₂ was filtered and washed with DCM. The mother liquor was concentrated, affording the product as a dark red foam (ca. 40 mg) which was used in the next step without further purification. LC-MS 473.0 [M-H]⁻, RT 1.43 min.

### Steps 16-17: 2-((ethylamino)methyl)-8-hydroxy-1-methyl-6-oxo-1,5,6,9-tetrahydropyrido[3',2':4,5]cyclopenta[1,2-f]indole-7-carboxylic acid hydrochloride

To a solution of the 5-(2,4-dimethoxybenzyl)-2-formyl-8-hydroxy-1-methyl-6-oxo-1,5,6,9-tetrahydropyrido[3',2':4,5]cyclopenta[1,2-f]indole-7-carboxylic acid obtained above (ca. 40 mg, ca. 0.1 mmol) in dichloroethane (1 mL) was added ethylamine hydrochloride (42 mg, 0.5 mmol) followed NEt₃ (70 µL, 0.5 mmol). The mixture was stirred for 5 min at room temperature, then AcOH (30 µL, 0.5 mmol) was added. After stirring at room temperature for 5 min, NaBH(OAc)₃ (106 mg, 0.5 mmol) was added. The reaction mixture was stirred at room temperature for ∼2 h and monitored by LC/MS until the starting aldehyde was completely consumed. The dichloroethane was removed and the residue was dissolved in MeOH (6 mL) and several drops of TFA to generate a homogeneous mixture that was filtered through a PTFE micron filter and purified directly by preparative HPLC to provide the product as a TFA salt (6 mg).

To the product (6 mg) obtained above was added i-Pr₃SiH (0.20 mL), followed by TFA (0.20 mL). The mixture was heated at 60 °C for 2 h and monitored by LC/MS. After complete consumption of starting material, the TFA was removed under reduced pressure. Addition of a HCl solution (2M Et₂O, 1.50 mL) to the oily residue resulted in the formation of a precipitate. The mixture was diluted with Et₂O and the resulting solid was filtered and washed with Et₂O. The product was obtained as a colorless solid HCl salt (2 mg, 5.5% over 5 steps).
¹H NMR (500 MHz, DMSO-*d*₆) δ ppm 1.22 - 1.38 (m, 3 H) 2.54 (s, 2 H) 3.00 - 3.13 (m, 2 H) 3.83 - 3.90 (m, 3 H) 4.42 (br. s., 2 H) 6.89 (br. s., 1 H) 7.84 (s, 1 H) 8.41 (s, 1 H) 9.35 (br. s., 2 H) 13.59 (br. s., 1 H) 13.66 (br. s., 1 H) 16.03 (br. s., 1 H). LC-MS 352.1 [M-H]⁻, 354.1 [M+H]⁺, RT 0.63 min.

### Example 20

### 4-hydroxy-8-methyl-9-((methylamino)methyl)-2-oxo-2,5,6,8-tetrahydro-1H-indolo[6,5-h]quinoline-3-carboxylic acid hydrochloride (Cpd 20)

### Step 1: 1-(2-(((tert-butyldimethylsilyl)oxy)methyl)-1-methyl-6-vinyl-1H-indol-5-yl)but-3-en-1-ol

To a solution of 2-((tert-butyldimethylsilyloxy)methyl)-1-methyl-6-vinyl-1H-indole-5-carbaldehyde (Example 19, step 6, 4.237 g, 12.86 mmol) in THF (50 mL) at -78 °C was added allylmagnesium chloride (2M in THF, 8.0 mL, 16.0 mmol) dropwise over ∼10 min. The reaction mixture was stirred at -78 °C for 10 min and slowly allowed to warm to 0 °C, then the reaction was quenched by addition of NH₄Cl (aqueous saturated, 40 mL). The product was extracted with EtOAc (3x80 mL). The combined organics were washed with NaCl (aqueous saturated, 50 mL) and dried over Na₂SO₄. The solvent was removed to provide the product as a pale-yellow oil (4.30 g, 90%) which solidified after trituration with pentanes. The product was used directly in the next step without further purification.
¹H NMR (500 MHz, Acetone) δ ppm 0.09 (s, 3 H) 0.10 (s, 3 H) 0.91 (s, 9 H) 2.40 - 2.55 (m, 2 H) 3.83 (s, 3 H) 4.01 (d, *J*=4.1 Hz, 1 H) 4.90 (s, 2 H) 4.94 - 5.11 (m, 3 H) 5.21 (dd, *J*=10.9, 1.7 Hz, 1 H) 5.68 (dd, *J*=17.3, 1.6 Hz, 1 H) 5.82 - 5.98 (m, 1 H) 6.37 (s, 1 H) 7.27 (dd, *J*=17.3, 11.0 Hz, 1 H) 7.50 (s, 1 H) 7.66 (s, 1 H).

### Step 2: 2-(((tert-butyldimethylsilyl)oxy)methyl)-1-methyl-5,6,7,8-tetrahydro-1H-benzo[f]indol-5-ol

The 1-(2-(((tert-butyldimethylsilyl)oxy)methyl)-1-methyl-6-vinyl-1H-indol-5-yl)but-3-en-1-ol obtained above was dissolved in toluene (260 mL, 0.05M) under Argon. A second generation Grubbs' catalyst (540 mg, 0.64 mmol, 5 mol%) was added and the mixture was stirred at room temperature overnight until complete consumption of the starting material was indicated by TLC. PtO₂ (200 mg, 0.88 mmol, 7 mol%) was added to the mixture and the reaction was hydrogenated under 1 atm of H₂ until the olefin intermediate was completely consumed. The catalyst was then filtered; the toluene was removed and the residue was purified by column chromatography (EtOAc/hexanes, 0-15% gradient) to provide the product as a solid (3.08 g, 69% 3 steps).
¹H NMR (500 MHz, Acetone) δ ppm 0.07 (s, 3 H) 0.07 (s, 3 H) 0.90 (s, 9 H) 1.67 - 1.78 (m, 1 H) 1.81 - 1.90 (m, 1 H) 1.92 - 2.09 (m, 2 H) 2.81 - 2.88 (m, 1 H) 2.94 (dt, *J*=16.4, 5.8 Hz, 1 H) 3.72 (d, *J*=5.4 Hz, 1 H) 3.75 (s, 3 H) 4.80 (q, *J*=5.4 Hz, 1 H) 4.88 (s, 2 H) 6.31 (s, 1 H) 7.04 (s, 1 H) 7.56 (s, 1 H)

### Step 3: 2-(((tert-butyldimethylsilyl)oxy)methyl)-1-methyl-7,8-dihydro-1H-benzo[f]indol-5(6H)-one

To a solution of 2-(((tert-butyldimethylsilyl)oxy)methyl)-1-methyl-5,6,7,8-tetrahydro-1H-benzo[f]indol-5-ol (4.04 g, 11.69 mmol) in DCM (120 mL) was added activated MnO₂ (11.4 g+11.4 g+5.7 g, 118 mmol +118 mmol +59 mmol) in 3 portions at 20 min intervals. The reaction was monitored by TLC, with KMnO₄ staining due to much higher UV absorption of the product comparing to starting material. After complete consumption of starting material, the MnO₂ was filtered and washed with DCM. The mother liquor was concentrated and the residue was purified by column chromatography (EtOAc/hexanes, 0-15% gradient) to provide the product as an off-white solid (2.580 g, 64%).
¹H NMR (500 MHz, CHCl₃-*d*) δ ppm 0.07 (s, 6 H) 0.90 (s, 9 H) 2.16 (quin, *J*=6.3 Hz, 2 H) 2.68 (t, *J*=6.3 Hz, 2 H) 3.10 (t, *J*=6.3 Hz, 2 H) 3.78 (s, 3 H) 4.82 (s, 2 H) 6.45 (s, 1 H) 7.09 (s, 1 H) 8.37 (s, 1 H). LC-MS 344.5 [M+H]⁺, RT 1.66 min.

### Step 4: N-(2-(((tert-butyldimethylsilyl)oxy)methyl)-1-methyl-7,8-dihydro-1H-benzo[f]indol-5(6H)-ylidene)-1-(2,4-dimethoxyphenyl)methanamine

To a solution of 2-(((tert-butyldimethylsilyl)oxy)methyl)-1-methyl-7,8-dihydro-1H-benzo[f]indol-5(6H)-one (1.01 g, 2.95 mmol) in DCM (9 mL) was added 2,4-dimethoxybenzylamine (0.56 mL, 3.73 mmol) and NEt₃ (1.30 mL, 9.33 mmol). The mixture was cooled to 0 °C, then a solution of TiCl₄ solution (1M DCM, 1.90 mL, 1.90 mmol) was added dropwise via syringe pump over 30 min. The reaction was allowed to warm to room temperature and stirred overnight. The mixture was diluted with DCM (20 mL) and then the reaction was quenched with NaHCO₃ (aqueous saturated, 10 mL). After vigorous shaking, the organic phase was separated using a PTFE phase separator, and dried over Na₂SO₄. The solvent was removed to provide the product (∼1.40 g, quant) as a yellow solid, which was used directly in the next step without further purification.

### Step 5: methyl 9-(((tert-butyldimethylsilyl)oxy)methyl)-1-(2,4-dimethoxybenzyl)-4-hydroxy-8-methyl-2-oxo-2,5,6,8-tetrahydro-1H-indolo[6,5-h]quinoline-3-carboxylate

The *N*-(2-(((tert-butyldimethylsilyl)oxy)methyl)-1-methyl-7,8-dihydro-1H-benzo[f]indol-5(6H)-ylidene)-1-(2,4-dimethoxyphenyl)methanamine (1.40 g, 2.84 mmol) and trimethyl methanetricarboxylate (0.95 g, 5.00 mmol) were mixed together in Ph₂O (7.0 mL). With stirring, the mixture was placed onto a pre-heated heat block at 230 °C and heated for 10 min after the initial bubbling of MeOH was observed (occurs art ∼160 °C internal reaction temperature) under a blanket of Argon. The reaction mixture was cooled to room temperature, then purified by column chromatography (hexanes followed by EtOAc/hexanes 25-60% gradient) to yield the product as a yellow foam (1.158 g, 63% 2 steps).
¹H NMR (500 MHz, CHCl₃-*d*) δ ppm 0.06 (s, 6 H) 0.90 (s, 9 H) 2.58 - 2.72 (m, 2 H) 2.89 (t, *J*=6.6 Hz, 2 H) 3.62 (s, 3 H) 3.78 (s, 3 H) 3.80 (s, 3 H) 3.97 (s, 3 H) 4.77 (s, 2 H) 5.37 (s, 2 H) 6.19 (s, 1 H) 6.42 (d, *J*=2.4 Hz, 1 H) 6.46 (dd, *J*=8.4, 2.4 Hz, 1 H) 7.14 (d, *J*=8.4 Hz, 1 H) 7.18 (s, 1 H) 7.63 (s, 1 H) 13.60 (s, 1 H). LC-MS 619.6 [M+H]⁺, RT 1.76 min.

### Step 6: methyl 1-(2,4-dimethoxybenzyl)-4-hydroxy-9-(hydroxymethyl)-8-methyl-2-oxo-2,5,6,8-tetrahydro-1H-indolo[6,5-h]quinoline-3-carboxylate

To a solution of methyl 9-(((tert-butyldimethylsilyl)oxy)methyl)-1-(2,4-dimethoxybenzyl)-4-hydroxy-8-methyl-2-oxo-2,5,6,8-tetrahydro-1H-indolo[6,5-h]quinoline-3-carboxylate (1.15 g, 1.86 mmol) in THF (7 mL) was added a TBAF solution (1M THF, 5.60 mL, 5.60 mmol). The reaction mixture was stirred at room temperature for 2 h until the starting material was completely consumed. The THF was removed and the residue was purified by column chromatography (MeOH/DCM, 0-6% gradient) to provide the product as a yellow solid (0.920 g, 98 %).
¹H NMR (500 MHz, CHCl₃-*d*) δ ppm 2.59 - 2.69 (m, 2 H) 2.88 (t, *J*=6.6 Hz, 2 H) 3.61 (s, 3 H) 3.78 (s, 3 H) 3.80 (s, 3 H) 3.96 (s, 3 H) 4.76 (s, 2 H) 5.35 (s, 2 H) 6.26 (s, 1 H) 6.40 (d, *J*=2.4 Hz, 1 H) 6.44 (dd, *J*=8.4, 2.4 Hz, 1 H) 7.12 (d, *J*=8.4 Hz, 1 H) 7.19 (s, 1 H) 7.65 (s, 1 H) 13.59 (s, 1 H). LC-MS 503.1 [M-H]⁻, 505.1 [M+H]⁺, RT 1.20 min.

### Step 7: 1-(2,4-dimethoxybenzyl)-4-hydroxy-9-(hydroxymethyl)-8-methyl-2-oxo-2,5,6,8-tetrahydro-1H-indolo[6,5-h]quinoline-3-carboxylic acid

To a suspension of methyl 1-(2,4-dimethoxybenzyl)-4-hydroxy-9-(hydroxymethyl)-8-methyl-2-oxo-2,5,6,8-tetrahydro-1H-indolo[6,5-h]quinoline-3-carboxylate (0.795 g, 1.58 mmol) in EtOAc (6 mL) was added LiI (0.84 g, 6.23 mmol). The reaction mixture was stirred and heated at 60 °C for 1.5 h until complete consumption of starting material was observed. The mixture was then cooled to room temperature and acidified with aqueous HCl (1M, 6 mL). The product was extracted with EtOAc (3x20 mL) and the organic phase was washed with Na₂S₂O₃ (10% aq, 5 mL), NaCl (aqueous saturated, 10 mL) and dried over Na₂SO₄. The solvent was removed to provide the product as a yellow solid (0.70 g, 90%).
¹H NMR (500 MHz, DMSO-*d*₆) δ ppm 2.54 - 2.64 (m, 2 H) 2.87 (t, *J*=6.5 Hz, 2 H) 3.60 (s, 3 H) 3.74 (s, 3 H) 3.75 (s, 3 H) 4.60 (s, 2 H) 5.37 (s, 2 H) 6.22 (s, 1 H) 6.48 (dd, *J*=8.4,2.4 Hz, 1 H) 6.55 (d, *J*=2.4 Hz, 1 H) 7.02 (d, *J*=8.4 Hz, 1 H) 7.49 (s, 1 H) 7.69 (s, 1 H) 13.60 (br. s., 1 H) 15.99 (br. s., 1 H). LC-MS 489.1 [M-H]⁻, 491.0 [M+H]⁺, RT 1.29 min.

### Step 8: 1-(2,4-dimethoxybenzyl)-9-formyl-4-hydroxy-8-methyl-2-oxo-2,5,6,8-tetrahydro-1H-indolo[6,5-h]quinoline-3-carboxylic acid

To a solution of 1-(2,4-dimethoxybenzyl)-4-hydroxy-9-(hydroxymethyl)-8-methyl-2-oxo-2,5,6,8-tetrahydro-1H-indolo[6,5-h]quinoline-3-carboxylic acid (0.70 g, 1.42 mmol) in DCM (15 mL) was added activated MnO₂ (1.4 g+1.4 g+0.70 g, 14.49 mmol + 14.49 mmol + 7.25 mmol) in 3 portions at 30 min intervals. The reaction was monitored by LC/MS. After complete consumption of starting material, the MnO₂ was filtered and washed with DCM. The mother liquor was concentrated, affording a product as a dark red foam (0.48 g, 69%) which was used in the next step without further purification. LC-MS 487.0 [M-H]⁻, RT 1.45 min.

### Steps 9-10: 4-hydroxy-8-methyl-9-((methylamino)methyl)-2-oxo-2,5,6,8-tetrahydro-1H-indolo[6,5-h]quinoline-3-carboxylic acid hydrochloride

To a solution of 1-(2,4-dimethoxybenzyl)-9-formyl-4-hydroxy-8-methyl-2-oxo-2,5,6,8-tetrahydro-1H-indolo[6,5-h]quinoline-3-carboxylic acid (240.0 mg, 0.49 mmol) in dichloroethane (4 mL) was added a MeNH₂ solution (2M THF, 0.50 mL, 1.00 mmol) followed by AcOH (60 µL, 1.05 mmol). After stirring at room temperature for 10 min, NaBH(OAc)₃ (160 mg, 0.75 mmol) was added. The reaction was stirred at room temperature for ∼2 h and monitored by LC/MS until the starting aldehyde was completely consumed. The dichloroethane was removed and the residue was dissolved in MeOH (4 mL), DMSO (4 mL) and TFA (1 mL) to generate a homogeneous mixture that was filtered through a PTFE micron filter and purified directly by preparative HPLC to provide the product as a TFA salt (85.2 mg, 28%).

To the product (85.2 mg, 0.14 mmol) obtained above was added i-Pr₃SiH (0.90 mL), followed by TFA (0.90 mL). The mixture was heated at 60 °C for 2 h and monitored by LC/MS. After complete consumption of the starting material, the TFA was removed under reduced pressure. Addition of a HCl solution (2M Et₂O, 1.50 mL) to the oily residue resulted in precipitate formation. The mixture was diluted with Et₂O and the resulting solid was filtered and washed with Et₂O. The product HCl salt was obtained as a colorless solid (45.3 mg, 84%).
¹H NMR (500 MHz, DMSO-*d*₆) δ ppm 2.63 (br. s, 3 H) 2.68 (d, *J*=7.6 Hz, 2 H) 2.98 (t, *J*=7.6 Hz, 2 H) 3.82 (s, 3 H) 4.40 (br. s., 2 H) 6.79 (s, 1 H) 7.53 (s, 1 H) 8.34 (s, 1 H) 9.26 (br. s., 2 H) 12.70 (br. s., 1 H) 13.66 (br. s., 1 H). LC-MS 352.0 [M-H]⁻, 354.1 [M+H]⁺, RT 0.97 min.

### Example 21

### 9-(azetidin-1-ylmethyl)-4-hydroxy-8-methyl-2-oxo-2,5,6,8-tetrahydro-1H-indolo[6,5-h]quinoline-3-carboxylic acid hydrochloride (Cpd 21)

To a solution of 1-(2,4-dimethoxybenzyl)-9-formyl-4-hydroxy-8-methyl-2-oxo-2,5,6,8-tetrahydro-1H-indolo[6,5-h]quinoline-3-carboxylic acid (Example 20, step 8, 240.0 mg, 0.49 mmol) in dichloroethane (4 mL) was added azetidine hydrochloride (70 mg, 0.75 mmol) followed by NEt₃ (110 µL, 0.79 mmol). The mixture was stirred for 5 min at room temperature, then AcOH (50 µL, 0.87 mmol) was added. After stirring at room temperature for an additional 5 min, NaBH(OAc)₃ (180 mg, 0.85 mmol) was added. The reaction was stirred at room temperature and monitored by LC/MS. After 4 h, a significant amount of the starting material remained. Additional azetidine hydrochloride (70 mg, 0.75 mmol), NEt₃ (110 µL, 0.79 mmol) and NaBH(OAc)₃ (180 mg, 0.85 mmol) were added to the reaction mixture with stirring overnight at room temperature until the starting material was completely consumed. The dichloroethane was then removed and the residue was dissolved in MeOH (4 mL), DMSO (4 mL) and TFA (1 mL) to generate a homogeneous mixture that was filtered through a PTFE micron filter and purified directly by preparative HPLC to provide the product as a TFA salt (82.6 mg, 26%).

To the product (82.6 mg, 0.13 mmol) obtained above was added i-Pr₃SiH (0.90 mL) followed by TFA (0.90 mL). The mixture was heated at 60 °C for 2 h and monitored by LC/MS. After complete consumption of the starting material, the TFA was removed under reduced pressure. Addition of an HCl solution (2M Et₂O, 1.5 mL) to the oily residue resulted in precipitate formation. The mixture was diluted with Et₂O and the resulting solid was filtered and washed with Et₂O. The product HCl salt was obtained as a colorless solid (41.0 mg, 77%).
¹H NMR (500 MHz, DMSO-*d*₆) δ ppm 2.22 - 2.46 (m, 2 H) 2.67 (t, *J*=7.6 Hz, 2 H) 2.98 (t, *J*=7.6 Hz, 2 H) 3.81 (s, 3 H) 3.97 - 4.22 (m, 4 H) 4.64 (br. s., 2 H) 6.82 (s, 1 H) 7.52 (s, 1 H) 8.34 (s, 1 H) 10.75 (br. s., 1 H) 12.70 (br. s., 1 H) 13.66 (br. s., 1 H). LC-MS 378.1 [M-H]⁻, RT 0.99 min.

### Example 22

### 4-hydroxy-9-methyl-10-((methylamino)methyl)-2-oxo-1,2,5,6,7,9-hexahydropyrido[3',2':6,7]cyclohepta[1,2-f]indole-3-carboxylic acid hydrochloride (Cpd 22)

### Step 1: 1-(2-((tert-butyldimethylsilyloxy)methyl)-1-methyl-6-vinyl-1H-indol-5-yl)pent-4-en-1-ol

To a solution of 2-((tert-butyldimethylsilyloxy)methyl)-1-methyl-6-vinyl-1H-indole-5-carbaldehyde (Example 19, step 6, 8.23 g, 24.98 mmol) in THF (50 mL) at -78 °C was added 3-butenylmagnesium bromide (0.5M in THF, 60.0 mL, 30.0 mmol) dropwise over ∼10 min. The reaction was stirred at -78 °C for 10 min and slowly allowed to warm to -10 °C. The reaction was quenched by addition of NH₄Cl (aqueous saturated, 80 mL). The product was extracted with EtOAc (4x150 mL). The combined organics were washed with NaCl (aqueous saturated, 100 mL) and dried over Na₂SO₄. The solvent was removed to provide the product as a pale-yellow oil (9.60 g, quant) which solidified under high-vacuum. The product was used directly in the next step without further purification.
¹H NMR (500 MHz, CHCl₃-*d*) δ ppm 0.06 (s, 3 H) 0.07 (s, 3 H) 0.90 (s, 9 H) 1.86 - 1.99 (m, 2 H) 2.11 - 2.31 (m, 2 H) 3.80 (s, 3 H) 4.82 (s, 2 H) 4.99 (d, *J*=10.4 Hz, 1 H) 5.06 (dd, *J*=17.2,1.7 Hz, 1 H) 5.11 (dd, *J*=7.3, 5.7 Hz, 1 H) 5.29 (dd, *J*=10.4, 1.7 Hz, 1 H) 5.65 (dd, *J*=17.2, 1.7 Hz, 1 H) 5.83 - 5.93 (m, 1 H) 6.34 (s, 1 H) 7.23 (dd, *J*=17.2, 10.9 Hz, 1 H) 7.40 (s, 1 H) 7.66 (s, 1 H). LC-MS 386.3 [M+H]⁺, RT 1.80 min.

### Step 2: 2-((tert-butyldimethylsilyloxy)methyl)-1-methyl-1,5,6,7-tetrahydrocyclohepta[f]indol-5-ol

1-(2-((tert-butyldimethylsilyloxy)methyl)-1-methyl-6-vinyl-1H-indol-5-yl)pent-4-en-1-ol obtained above (*ca* 24.98 mmol) was dissolved in toluene (500 mL, 0.05M) under Argon. A second generation Grubbs' catalyst (640 mg, 0.75 mmol, 3 mol%) was added and the mixture was heated at 60 °C 2-3 h until the starting material was completely consumed as indicated by LC/MS. After cooling the reaction to room temperature, the toluene was removed under reduced pressure and the residue (∼8.9 g) was used directly in the next step. The product may be purified by column chromatography (EtOAc/hexanes, 0-20% gradient) to yield a yellow solid material.
¹H NMR (500 MHz, CHCl₃-*d*) δ ppm 0.06 (s, 6 H) 0.90 (s, 9 H) 1.92 (br. s, 1 H) 2.08 - 2.19 (m, 1 H) 2.23 - 2.34 (m, 1 H) 2.42 - 2.51 (m, 1 H) 2.56 - 2.71 (m, 1 H) 3.77 (s, 3 H) 4.82 (s, 2 H) 5.03 (d, *J*=8.2 Hz, 1 H) 5.84 - 5.91 (m, 1 H) 6.34 (s, 1 H) 6.56 (d, *J*=12.3 Hz, 1 H) 7.14 (s, 1 H) 7.57 (s, 1 H). LC-MS 358.3 [M+H]⁺, RT 1.66 min.

### Step 3: 2-((tert-butyldimethylsilyloxy)methyl)-1-methyl-1,5,6,7,8,9-hexahydrocyclohepta[f]indol-5-ol

A solution of 2-((tert-butyldimethylsilyloxy)methyl)-1-methyl-1,5,6,7-tetrahydrocyclohepta[f]indol-5-ol obtained above (*ca* 24.98 mmol) in EtOAc (105 mL) and DCM (15 mL) was hydrogenated over Pd/C (10%, 880 mg) under H₂ (1 atm) until complete consumption of starting material was indicated by TLC (2x 10%EtOAc/hexanes). After ∼3 h, the catalyst was filtered and washed with EtOAc. The mother liquor was concentrated and the product was obtained as a brown solid, used directly in the next step without further purification. The product may be purified by column chromatography (EtOAc/hexanes, 0-25% gradient) to provide a pale yellow solid material.
¹H NMR (500 MHz, CHCl₃-*d*) δ ppm 0.06 (s, 3 H) 0.07 (s, 3 H) 0.90 (s, 9 H) 1.59 - 1.68 (m, 1 H) 1.70 - 1.85 (m, 2 H) 1.85 - 2.00 (m, 2 H) 2.06 (s, 1 H) 2.83 (dd, *J*=13.7, 10.6 Hz, 1 H) 3.12 (dd, *J*=13.7, 9.3 Hz, 1 H) 3.76 (s,3H) 4.81 (s,2H) 5.03 (d, *J*=9.1 Hz, 1 H) 6.33 (s, 1 H) 7.04 (s, 1 H) 7.58 (s, 1 H). LC-MS 360.3 [M+H]⁺, RT 1.71 min.

### Step 4: 2-((tert-butyldimethylsilyloxy)methyl)-1-methyl-6,7,8,9-tetrahydrocyclohepta[f]indol-5(1H)-one

To activated 4Å molecular sieves (6.2 g, 250 mg/mmol) was added a solution of 2-((tert-butyldimethylsilyloxy)methyl)-1-methyl-1,5,6,7,8,9-hexahydrocyclohepta[f]indol-5-ol obtained above (*ca* 24.98 mmol) in DCM (125 mL). The mixture was cooled to 0 °C, then NMO (4.45 g, 37.99 mmol) and TPAP (445 mg, 1.26 mmol, 5 mol%) were added. The reaction was stirred at 0 °C and monitored by LC/MS. After complete consumption of starting material (∼1.5 h), the molecular sieves were filtered off and washed with DCM. The mother liquor was concentrated and the residue was purified by column chromatography (EtOAc/hexanes, 0-25% gradient). The product was obtained as an off-white solid (7.47 g, 84 % over 4 steps).
¹H NMR (500 MHz, CHCl₃-*d*) δ ppm 0.06 (s, 6 H) 0.90 (s, 9 H) 1.76 - 1.84 (m, 2 H) 1.88 - 1.95 (m, 2 H) 2.74 - 2.77 (m, 2 H) 3.05 (t, *J*=6.6 Hz, 1 H) 3.79 (s, 3 H) 4.82 (s, 2 H) 6.43 (s, 1 H) 7.06 (s, 1 H) 8.04 (s, 1 H). LC-MS 358.3 [M+H]⁺, RT 1.73 min.

### Step 5: N-(2-((tert-butyldimethylsilyloxy)methyl)-1-methyl-6,7,8,9-tetrahydrocyclohepta[f]indol-5(1H)-ylidene)-1-(2,4-dimethoxyphenyl)methanamine

To a solution of 2-((tert-butyldimethylsilyloxy)methyl)-1-methyl-6,7,8,9-tetrahydrocyclohepta[f]indol-5(1H)-one (7.458 g, 20.86 mmol) in DCM (65 mL) was added 2,4-dimethoxybenzylamine (3.30 mL, 21.97 mmol) and NEₜ3 (7.80 mL, 55.96 mmol). The mixture was cooled to 0 °C, then a solution of TiCl₄ (1M DCM, 13.60 mL, 13.60 mmol) was added dropwise via syringe pump over 30 min. The reaction mixture was allowed to warm to room temperature and stirred overnight. The mixture was diluted with DCM (150 mL) and then the reaction was quenched with NaHCO₃ (aqueous saturated, 50 mL). After vigorous shaking, the organic phase was separated using a PTFE phase separator, and dried over Na₂SO₄. The solvent was removed to provide the product (10.6 g, quant) as a yellow oil, which was used directly in the next step without further purification.

### Step 6: methyl 10-(((tert-butyldimethylsilyl)oxy)methyl)-1-(2,4-dimethoxybenzyl)-4-hydroxy-9-methyl-2-oxo-1,2,5,6,7,9-hexahydropyrido[3',2':6,7]cyclohepta[1,2-f]indole-3-carboxylate

Crude *N*-(2-((tert-butyldimethylsilyloxy)methyl)-1-methyl-6,7,8,9-tetrahydrocyclohepta[f]indol-5(1H)-ylidene)-1-(2,4-dimethoxyphenyl)methanamine (10.6 g, 20.91 mmol) and trimethyl methanetricarboxylate (6.80 g, 35.76 mmol) were mixed together in Ph₂O (40 mL). With stirring, the mixture was placed onto a pre-heated heat block at 230 °C and heated for 10 min after the initial bubbling of MeOH was observed (occurs at ∼160 °C internal reaction temperature). The reaction mixture was cooled to room temperature, then purified by column chromatography (hexanes followed by EtOAc/hexanes 0-80% gradient) to yield the product as a yellow foam (7.44 g, 56%, 2 steps).
¹H NMR (500 MHz, CHCl₃-*d*) δ ppm 0.08 (s, 3 H) 0.09 (s, 3 H) 0.90 (s, 9 H) 1.45 - 1.55 (m, 1 H) 1.85 - 1.95 (m, 1 H) 1.96 - 2.05 (m, 1 H) 2.32 - 2.44 (m, 1 H) 2.58 (dd, *J*=13.4, 6.1 Hz, 1 H) 2.96 (dd, *J*=13.4, 5.4 Hz, 1 H) 3.33 (s, 3 H) 3.76 (s, 3 H) 3.78 (s, 3 H) 4.00 (s, 3 H) 4.80 (s, 2 H) 5.13 - 5.37 (m, 2 H) 6.22 (d, *J*=2.2 Hz, 1 H) 6.28 (s, 1 H) 6.34 (dd, *J*=8.4, 2.2 Hz, 1 H) 6.81 (d, *J*=8.4 Hz, 1 H) 7.06 (s, 1 H) 7.32 (s, 1 H) 13.66 (br. s, 1 H). LC-MS 633.5 [M+H]⁺, RT 1.85 min.

### Step 7: methyl 1-(2,4-dimethoxybenzyl)-4-hydroxy-10-(hydroxymethyl)-9-methyl-2-oxo-1,2,5,6,7,9-hexahydropyrido[3',2':6,7]cyclohepta[1,2-f]indole-3-carboxylate

To a solution of methyl 10-(((tert-butyldimethylsilyl)oxy)methyl)-1-(2,4-dimethoxybenzyl)-4-hydroxy-9-methyl-2-oxo-1,2,5,6,7,9-hexahydropyrido[3',2':6,7]cyclohepta[1,2-f]indole-3-carboxylate (7.44 g, 11.76 mmol) in THF (40 mL) was added TBAF solution (1M THF, 30.0 mL, 30.0 mmol). The reaction mixture was stirred at room temperature for 2 h until the starting material was completely consumed. The THF was removed and the residue was purified by column chromatography (EtOAc/DCM, 0-100% gradient). The product was obtained as a yellow solid (5.94 g, 97 %).
¹H NMR (500 MHz, CHCl₃-*d*) δ ppm 1.49 (td, *J*=13.6, 6.9 Hz, 1 H) 1.84 - 1.95 (m, 1 H) 1.96 - 2.05 (m, 1 H) 2.38 (td, *J*=12.8, 7.6 Hz, 1 H) 2.59 (dd, *J*=13.2, 6.3 Hz, 1 H) 2.96 (dd, *J*=13.2, 5.2 Hz, 1 H) 3.33 (s, 3 H) 3.75 (s, 3 H) 3.81 (s, 3 H) 4.00 (s, 3 H) 4.80 (s, 2 H) 5.11 - 5.30 (m, 2 H) 6.22 (d, *J*=2.5 Hz, 1 H) 6.32 - 6.38 (m, 2 H) 6.79 (d, *J*=8.5 Hz, 1 H) 7.07 (s, 1 H) 7.35 (s, 1 H) 13.72 (br. s, 1 H). LC-MS 517.4 [M-H]⁻, 519.3 [M+H]⁺, RT 1.27 min.

### Step 8: 1-(2,4-dimethoxybenzyl)-4-hydroxy-10-(hydroxymethyl)-9-methyl-2-oxo-1,2,5,6,7,9-hexahydropyrido[3',2':6,7]cyclohepta[1,2-f]indole-3-carboxylic acid

To a suspension of methyl 1-(2,4-dimethoxybenzyl)-4-hydroxy-10-(hydroxymethyl)-9-methyl-2-oxo-1,2,5,6,7,9-hexahydropyrido[3',2':6,7]cyclohepta[1,2-f]indole-3-carboxylate (5.94 g, 11.45 mmol) in EtOAc (50 mL) was added LiI (4.60 g, 34.37 mmol). The reaction mixture was stirred and heated at 60 °C for 1.5 h until complete consumption of starting material was observed. The mixture was cooled to room temperature, then acidified with aqueous HCl (1M, 20 mL) and diluted with H₂O. The product was extracted with EtOAc (4x100 mL), then the organic phase was washed with Na₂S₂O₃ (10% aq, 40 mL), NaCl (aqueous saturated, 100 mL) and dried over Na₂SO₄. The solvent was removed to provide the product as a yellow solid (5.24 g, 91%).
¹H NMR (500 MHz, DMSO-*d*₆) δ ppm 1.45 (td, *J*=13.3, 7.4 Hz, 1 H) 1.85 - 1.98 (m, 2 H) 2.29 - 2.40 (m, 1 H) 2.60 - 2.70 (m, 1 H) 2.86 (dd, *J*=113.2, 5.4 Hz, 1 H) 3.43 (s, 3 H) 3.69 (s, 3 H) 3.74 (s, 3 H) 4.62 (s, 2 H) 5.13 - 5.33 (m, 2 H) 6.31 (s, 1 H) 6.36 (dd, *J*=8.4, 2.2 Hz, 1 H) 6.39 (d, *J*=2.2 Hz, 1 H) 6.59 (d, *J*=8.4 Hz, 1 H) 7.35 (s, 1 H) 7.52 (s, 1 H) 13.74 (s, 1 H). LC-MS 503.1 [M-H]⁻, 505.2 [M+H]⁺, RT 1.34 min.

### Step 9: 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid

To a solution of 1-(2,4-dimethoxybenzyl)-4-hydroxy-10-(hydroxymethyl)-9-methyl-2-oxo-1,2,5,6,7,9-hexahydropyrido[3',2':6,7]cyclohepta[1,2-f]indole-3-carboxylic acid (5.24 g, 10.39 mmol) in DCM (100 mL) was added activated MnO₂ (10.0 g+10.0 g+5.0 g, 103 mmol +103 mmol +52 mmol) in 3 portions at 30 min intervals. The reaction was monitored by LC/MS. After complete consumption of starting material MnO₂ was filtered and washed with DCM. The mother liquor was concentrated affording product as dark red foam (4.30 g, 73% 3 steps) which was used in the next steps without further purification.
¹H NMR (500 MHz, CHCl₃-*d*) δ ppm 1.55 (td, *J*=13.6, 6.6 Hz, 1 H) 1.90 - 2.10 (m, 2 H) 2.20 - 2.33 (m, 1 H) 2.64 (dd, *J*=13.1, 5.8 Hz, 1 H) 3.03 (dd, *J*=14.0, 5.5 Hz, 1 H) 3.35 (s, 3 H) 3.77 (s, 3 H) 4.13 (s, 3 H) 5.19 (d, *J*=15.4 Hz, 1 H) 5.39 (d, *J*=15.4 Hz, 1 H) 6.26 (s, 1 H) 6.33 (d, *J*=8.2 Hz, 1 H) 6.66 (d, *J*=8.2 Hz, 1 H) 7.20 (s, 2 H) 7.56 (s, 1 H) 9.90 (s, 1 H) 13.97 (s, 1 H) 15.93 (s, 1 H). LC-MS 501.1 [M-H]⁻ RT 1.47 min.

### Step 10-11: 4-hydroxy-9-methyl-10-((methylamino)methyl)-2-oxo-1,2,5,6,7,9-hexahydropyrido[3',2':6,7]cyclohepta[1,2-f]indole-3-carboxylic acid hydrochloride

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (350 mg, 0.70 mmol) in dichloroethane (6 mL) was added MeNH₂ solution (2M THF, 0.70 mL, 1.40 mmol) followed by AcOH (85 µL, 1.44 mmol). After stirring at room temperature for 10 min, NaBH(OAc)₃ (220 mg, 1.03 mmol) was added. The reaction was stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt (228.3 mg, 52%).

To the product (228.3 mg, 0.36 mmol) obtained above was added i-Pr₃SiH (2.0 mL) followed by TFA (2.0 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a pale pink solid (125.2 mg, 86%).
¹H NMR (500 MHz, DMSO-*d*₆) δ ppm 2.03 - 2.22 (m, 2 H) 2.60 - 2.67 (m, 3 H) 2.68 - 2.78 (m, 2 H) 3.27 - 3.44 (m, 2 H, solvent overlap) 3.85 (s, 3 H) 4.43 (br. s., 2 H) 6.80 (s, 1 H) 7.55 (s, 1 H) 7.82 (s, 1 H) 9.22 (br. s., 2 H) 12.89 (s, 1 H) 13.85 (br. s., 1 H). LC-MS 366.8 [M-H]⁻, 368.8 [M+H]⁺, RT 0.81 min.

### Example 23

### 10-((cyclobutylamino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 23)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 90 mg, 0.19 mmol) in dichloroethane (1.5 mL) was added cyclobutylamine (25 µL, 0.29 mmol) followed by AcOH (20 µL, 0.33 mmol). After stirring at room temperature for 10 min, NaBH(OAc)₃ (70 mg, 0.33 mmol) was added. The reaction was stirred at room temperature 1.5 h and monitored by LC/MS until the starting aldehyde was completely consumed. The dichloroethane was then removed and the residue was dissolved in MeOH (4 mL) and several drops of TFA to generate a homogeneous mixture that was filtered through a PTFE micron filter and purified directly by preparative HPLC to provide the product as a TFA salt (86.3 mg, 69%).

To the product (86.3 mg, 0.13 mmol) obtained above was added i-Pr₃SiH (0.9 mL) followed by TFA (0.9 mL). The mixture was heated at 60 °C for 2 h and monitored by LC/MS. After complete consumption of starting material, the TFA was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a pale pink solid (42.1 mg, 74%) as an.
¹H NMR (500 MHz, DMSO-*d*₆) δ ppm 1.70 - 1.89 (m, 2 H) 2.09 (quin, *J*=6.6 Hz, 2 H) 2.15 - 2.32 (m, 4 H) 2.64 - 2.77 (m, 2 H) 3.27 - 3.44 (m, 2 H, solvent overlap) 3.75 - 3.82 (m, 1 H) 3.84 (s, 3 H) 4.31 (br. s., 2 H) 6.79 (s, 1 H) 7.54 (s, 1 H) 7.81 (s, 1 H) 9.53 (br. s, 2 H) 12.91 (br. s., 1 H) 13.84 (s, 1 H). LC-MS 406.3 [M-H]⁻, 408.3 [M+H]⁺, RT 0.85 min.

### Example 24

### 10-(azetidin-1-ylmethyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 24)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 350 mg, 0.70 mmol) in dichloroethane (6 mL)was added azetidine hydrochloride (100 mg, 1.06 mmol) followed NEt₃ (160 µL, 1.15 mmol). The mixture was stirred for 5 min at room temperature, then AcOH (70 µL, 1.17 mmol) was added. After stirring at room temperature for 5 min, NaBH(OAc)₃ (260 mg, 1.23 mmol) was added. The reaction was stirred at room temperature and monitored by LC/MS. After 4 h, a significant amount of starting material remained. Additional azetidine hydrochloride (100 mg, 1.06 mmol), NEt₃ (160 µL, 1.15 mmol) and NaBH(OAc)₃ (260 mg, 1.23 mmol) were added to the reaction mixture with stirring overnight at room temperature. After the starting material was completely consumed, the dichloroethane was removed and the residue was dissolved in MeOH (10 mL) and several drops of TFA to generate a homogeneous mixture that was filtered through a PTFE micron filter and purified directly by preparative HPLC to provide the product as a TFA salt (249.4 mg, 54%).

To the product (249.4 mg, 0.38 mmol) obtained above was added i-Pr₃SiH (2.0 mL) followed by TFA (2.0 mL). The mixture was heated at 60 °C for 2 h and monitored by LC/MS. After complete consumption of starting material, the TFA was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a pale pink solid (140.0 mg, 86%).
¹H NMR (500 MHz, DMSO-*d*₆) δ ppm 2.09 (quin, *J*=6.6 Hz, 2 H) 2.28 - 2.47 (m, 4 H) 2.64 - 2.76 (m, 2 H) 3.27 - 3.44 (m, 2 H, solvent overlap) 3.84 (s, 3 H) 3.99 - 4.08 (m, 2 H) 4.09 - 4.19 (m, 2 H) 4.66 (d, *J*=5.0 Hz, 2 H) 6.83 (s, 1 H) 7.53 (s, 1 H) 7.80 (s, 1 H) 10.86 (br. s., 1 H) 12.88 (br. s., 1 H) 13.84 (s, 1 H). LC-MS 392.1 [M-H]⁻, 394.4 [M+H]⁺, RT 0.85 min.

### Example 25

### 10-((ethylamino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 25)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 126.0 mg, 0.25 mmol) in dichloroethane (2.0 mL) was added ethylamine hydrochloride (31 mg, 0.38 mmol) followed NEt₃ (55 µL, 0.39 mmol). The mixture was stirred for 5 min at room temperature, then AcOH (25 µL, 0.42 mmol) was added followed by an EtNH₂ solution (2M THF, 0.20 mL, 0.40 mmol). After stirring at room temperature for 5 min, NaBH(OAc)₃ (90 mg, 0.42 mmol) was added. The reaction was stirred at room temperature for ∼2 h and monitored by LC/MS until the starting aldehyde was completely consumed. The dichloroethane was then removed and the residue was dissolved in MeOH (6 mL) and several drops of TFA to generate a homogeneous mixture that was filtered through a PTFE micron filter and purified directly by preparative HPLC to provide the product as a TFA salt (110.0 mg, 68%).

To the product (110.0 mg, 0.17 mmol) obtained above was added i-Pr₃SiH (1.10 mL) followed by TFA (1.10 mL). The mixture was heated at 60 °C for 2 h and monitored by LC/MS. After complete consumption of starting material, the TFA was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a pale pink solid (59.4 mg, 83%).
¹H NMR (500 MHz, DMSO-*d*₆) δ ppm 1.28 (t, *J*=7.3 Hz, 3 H) 2.09 (quin, *J*=6.3 Hz, 2 H) 2.64 - 2.77 (m, 2 H) 3.06 (q, *J*=7.3 Hz, 2 H) 3.27 - 3.44 (m, 2 H, solvent overlap) 3.85 (s, 3 H) 4.39 - 4.44 (m, 2 H) 6.81 (s, 1 H) 7.54 (s, 1 H) 7.81 (s, 1 H) 9.25 (br. s., 2 H) 12.89 (br. s., 1 H) 13.84 (br. s., 1 H). LC-MS 380.2 [M-H]⁻, 382.1 [M+H]⁺, RT 0.82 min.

### Example 26

### 4-hydroxy-10-((isopropylamino)methyl)-9-methyl-2-oxo-1,2,5,6,7,9-hexahydropyrido[3',2':6,7]cyclohepta[1,2-f]indole-3-carboxylic acid hydrochloride (Cpd 26)

To a solution of a 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 170 mg, 0.34 mmol) in dichloroethane (3.0 mL) was added isopropylamine (60 µL, 0.70 mmol) followed by AcOH (41 µL, 0.68 mmol). After stirring at room temperature for 10 min, NaBH(OAc)₃ (110 mg, 0.52 mmol) was added. The reaction mixture was stirred at room temperature overnight. LC/MS indicated complete consumption of starting aldehyde. The dichloroethane was then removed and the residue was dissolved in MeOH (6 mL) and several drops of TFA to generate a homogeneous mixture that was filtered through a PTFE micron filter and purified directly by preparative HPLC to provide the product as a TFA salt (123.0 mg, 55%).

To the product (123.0 mg, 0.19 mmol) obtained above was added i-Pr₃SiH (1.10 mL) followed by TFA (1.10 mL). The mixture was heated at 60 °C for 2 h and monitored by LC/MS. After complete consumption of starting material, the TFA was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a colorless solid (63.7 mg, 79%).
¹H NMR (500 MHz, DMSO-*d*₆) δ ppm 1.35 (d, *J*=6.3 Hz, 6 H) 2.09 (quin, *J*=6.5 Hz, 2 H) 2.66 - 2.76 (m, 2 H) 3.27 - 3.44 (m, 2 H, solvent overlap) 3.41-3.49 (m, 1 H) 3.85 (s, 3 H) 4.43 (t, *J*=5.5 Hz, 2 H) 6.81 (s, 1 H) 7.54 (s, 1 H) 7.81 (s, 1 H) 9.12 (br. s., 2 H) 12.90 (s, 1 H) 13.84 (br. s., 1 H). LC-MS 394.2 [M-H]⁻, RT 0.85 min.

### Example 27

### 10-((tert-butylamino)methyl)-4-hydroxy-9-methyl-2-oxo-1,2,5,6,7,9-hexahydropyrido[3',2':6,7]cyclohepta[1,2-f]indole-3-carboxylic acid hydrochloride

### (Cpd 27)

To a solution of a 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 170 mg, 0.34 mmol) in dichloroethane (3.0 mL) was added tert-butylamine (72 µL, 0.68 mmol) followed by AcOH (41 µL, 0.68 mmol). After stirring at room temperature for 10 min, NaBH(OAc)₃ (110 mg, 0.52 mmol) was added. The reaction mixture was stirred at room temperature overnight. LC/MS indicated that some starting material remained. Additional tert-butylamine (36 µL, 0.34 mmol), AcOH (20 µL, 0.33 mmol) and NaBH(OAc)₃ (50 mg, 0.24 mmol) were added to the reaction mixture, with stirring overnight at room temperature. After complete consumption of the starting material was observed, the dichloroethane was removed and the residue was dissolved in MeOH (7 mL) and several drops of TFA to generate a homogeneous mixture that was filtered through a PTFE micron filter and purified directly by preparative HPLC to provide the product as a TFA salt (102.5 mg, 45%).

To the product (102.5 mg, 0.15 mmol) obtained above was added i-Pr₃SiH (1.0 mL) followed by TFA (1.0 mL). The mixture was heated at 60 °C for 2 h and monitored by LC/MS. After complete consumption of starting material, the TFA was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a colorless solid (60.7 mg, 89%).
¹H NMR (500 MHz, DMSO-*d*₆) δ ppm 1.42 (s, 9 H) 2.09 (quin, *J*=6.9 Hz, 2 H) 2.65 - 2.77 (m, 2 H) 3.27 - 3.44 (m, 2 H, solvent overlap) 3.85 (s, 3 H) 4.40 (t, *J*=6.0 Hz, 1 H) 6.81 (s, 1 H) 7.55 (s, 1 H) 7.82 (s, 1 H) 9.11 (br. s, 2 H) 12.91 (s, 1 H) 13.84 (br. s., 1 H). LC-MS 408.2 [M-H]⁻, RT 0.86 min.

### Example 28

### 4-hydroxy-10-((4-hydroxypiperidin-1-yl)methyl)-9-methyl-2-oxo-1,2,5,6,7,9-hexahydropyrido[3',2':6,7]cyclohepta[1,2-f]indole-3-carboxylic acid hydrochloride (Cpd 28)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (3 mL) was added piperidin-4-ol (50 mg, 0.5 mmol) followed by AcOH (20 µL, 0.35 mmol). After stirring at room temperature for 10 min, NaBH(OAc)₃ (80 mg, 0.36 mmol) was added. The reaction was stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, TFA was removed under reduced pressure. Addition of an HCl solution (2M Et₂O, 2.0 mL) to the oily residue resulted in precipitate formation. The mixture was diluted with Et₂O and the resulting solid was filtered and washed with Et₂O. The product HCl salt was obtained as a pale pink solid (22 mg, 21% over 2 steps).
¹H NMR (500 MHz, DMSO-*d*₆) δ ppm 1.72 - 1.81 (m, 2 H) 1.93 - 2.00 (m, 2 H) 2.06 - 2.15 (m, 3 H) 2.67 - 2.77 (m, 3 H) 3.02 - 3.11 (m, 1 H) 3.21 - 3.31 (m, 2 H) 3.40 - 3.47 (m, 1 H) 3.89 (d, *J*=7.96 Hz, 3 H) 3.93 - 3.97 (m, 1 H) 4.53 - 4.64 (m, 2 H) 6.89 - 6.96 (m, 2 H) 7.54 - 7.59 (s, 1 H) 7.82 - 7.86 (s, 1 H) 12.86 - 12.92 (s, 1 H) 13.84 - 13.88 (s, 1 H).. LC-MS: 438.2 [M+H]⁺, RT 0.84 min.

### Example 29

### 10-((4-aminopiperidin-1-yl)methyl)-4-hydroxy-9-methyl-2-oxo-1,2,5,6,7,9-hexahydropyrido[3',2':6,7]cyclohepta[1,2-f]indole-3-carboxylic acid dihydrochloride (Cpd 29)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (3 mL) was added tert-butyl piperidin-4-ylcarbamate (100 mg, 0.5 mmol) followed by AcOH (20 µL, 0.35 mmol). After stirring at room temperature for 10 min, NaBH(OAc)₃ (80 mg, 0.36 mmol) was added. The reaction was stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, TFA was removed under reduced pressure. Addition of an HCl solution (2M Et₂O, 2.0 mL) to the oily residue resulted in precipitate formation. The mixture was diluted with Et₂O and the resulting solid was filtered and washed with Et₂O. The product was obtained as a pale pink solid (31 mg, 30% over 2 steps) as dihydrochloride salt.
¹H NMR (500 MHz, DMSO-*d*₆) δ ppm 1.97 - 2.17 (m, 5 H) 2.67 - 2.78 (m, 2 H) 3.09 - 3.22 (m, 1 H) 3.42 - 3.49 (m, 5 H) 3.49 - 3.57 (m, 1 H) 3.91 (s, 4 H) 4.51 - 4.61 (m, 2 H) 6.91 - 6.99 (s, 1 H) 7.53 - 7.61 (s, 1 H) 7.82 - 7.87 (s, 1 H) 12.87 - 12.94 (s, 1 H) 13.82 - 13.89 (m, 1 H).. LC-MS: 437.4 [M+H]⁺, RT 0.79 min.

### Example 30

### 10-((4-(dimethylamino)piperidin-1-yl)methyl)-4-hydroxy-9-methyl-2-oxo-1,2,5,6,7,9-hexahydropyrido[3',2':6,7]cyclohepta[1,2-f]indole-3-carboxylic acid dihydrochloride (Cpd 30)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (3 mL) was added *N,N-*dimethylpiperidin-4-amine (60 µL, 0.5 mmol) followed by AcOH (20 µL, 0.35 mmol). After stirring at room temperature for 10 min, NaBH(OAc)₃ (80 mg, 0.36 mmol) was added. The reaction was then stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, TFA was removed under reduced pressure. Addition of an HCl solution (2M Et₂O, 2.0 mL) to the oily residue resulted in precipitate formation. The mixture was diluted with Et₂O and the resulting solid was filtered and washed with Et₂O. The product was obtained as a pale pink solid (88 mg, 80% over 2 steps) as dihydrochloride salt.
¹H NMR (500 MHz, DMSO-*d*₆) δ ppm 2.06 - 2.15 (m, 2 H) 2.16 - 2.30 (m, 5 H) 2.71 (br. s, 6 H) 2.78 - 2.84 (m, 1 H) 2.84 - 2.95 (m, 2 H) 3.35 - 3.46 (m, 4 H) 3.91 (s, 4 H) 4.56 - 4.66 (m, 2 H) 6.91 - 6.97 (s, 1 H) 7.54 - 7.60 (s, 1 H) 7.82 - 7.87 (s, 1 H). LC-MS: 465.5 [M+H]⁺, RT 0.89 min.

### Example 31

### 10-((3-aminopiperidin-1-yl)methyl)-4-hydroxy-9-methyl-2-oxo-1,2,5,6,7,9-hexahydropyrido[3',2':6,7]cyclohepta[1,2-f]indole-3-carboxylic acid dihydrochloride (Cpd 31)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (3 mL) was added tert-butyl piperidin-3-ylcarbamate (100 mg, 0.5 mmol) followed by AcOH (20 µL, 0.35 mmol). After stirring at room temperature for 10 min, NaBH(OAc)₃ (80 mg, 0.36 mmol) was added. The reaction was then stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and then monitored by LC/MS. After complete consumption of starting material, TFA was removed under reduced pressure. Addition of an HCl solution (2M Et₂O, 2.0 mL) to the oily residue resulted in precipitate formation. The mixture was diluted with Et₂O and the resulting solid was filtered and washed with Et₂O. The product was obtained as a pale pink solid (40 mg, 38% over 2 steps) as dihydrochloride salt.
¹H NMR (500 MHz, DMSO-*d*₆) δ ppm 2.04 - 2.15 (m, 2 H) 2.67 - 2.77 (m, 2 H) 2.95 - 3.10 (m, 2 H) 3.45 - 3.62 (m, 4 H) 3.64 - 3.77 (m, 2 H) 3.94 (s, 3 H) 3.99 - 4.22 (m, 3 H) 4.65 - 4.80 (m, 2 H) 6.91 - 7.01 (s, 1 H) 7.55 - 7.61 (s, 1 H) 7.81 - 7.87 (s, 1 H) 8.40 - 8.48 (s, 1 H).. LC-MS: 437.4 [M+H]⁺, RT 0.74 min.

### Example 32

### 10-(((cyclopropylmethyl)amino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 32)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (3 mL) was added cyclopropylmethanamine (50 µL, 0.5 mmol) followed by AcOH (20 µL, 0.35 mmol). After stirring at room temperature for 10 min, NaBH(OAc)₃ (80 mg, 0.36 mmol) was added. The reaction was stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, TFA was removed under reduced pressure. Addition of an HCl solution (2M Et₂O, 2.0 mL) to the oily residue resulted in precipitate formation. The mixture was diluted with Et₂O and the resulting solid was filtered and washed with Et₂O. The product HCl salt was obtained as a beige solid (38 mg, 40% over 2 steps).
¹H NMR (500 MHz, DMSO-*d*₆) δ ppm 0.42 (d, *J*=5.60 Hz, 2 H) 0.58 - 0.66 (m, 2 H) 1.13 - 1.23 (m, 1 H) 2.04 - 2.16 (m, 2 H) 2.68 - 2.76 (m, 1 H) 2.89 - 2.99 (m, 2 H) 3.44 - 3.57 (m, 3 H) 3.86 (s, 3 H) 4.40 - 4.48 (m, 2 H) 6.84 (s, 1 H) 7.55 (s, 1 H) 7.82 (s, 1 H) 9.34 - 9.43 (s, 1 H) 12.86 - 12.97 (s, 1 H) 13.80 - 13.89 (s, 1 H). LC-MS: 406.2 [M-H]⁻, RT 0.89 min.

### Example 33

### 4-hydroxy-9-methyl-10-(((1-methylcyclopropyl)amino)methyl)-2-oxo-1,2,5,6,7,9-hexahydropyrido[3',2':6,7]cyclohepta[1,2-f]indole-3-carboxylic acid hydrochloride (Cpd 33)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (3 mL) was added 1-methylcyclopropanamine hydrochloride (60 mg, 0.5 mmol) followed by AcOH (20 µL, 0.35 mmol). After stirring at room temperature for 10 min, NaBH(OAc)₃ (80 mg, 0.36 mmol) was added. The reaction was stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, TFA was removed under reduced pressure. Addition of an HCl solution (2M Et₂O, 2.0 mL) to the oily residue resulted in precipitate formation. The mixture was diluted with Et₂O and the resulting solid was filtered and washed with Et₂O. The product HCl salt was obtained as a beige solid (32 mg, 33% over 2 steps).
¹H NMR (500 MHz, DMSO-*d*₆) δ ppm 0.69 - 0.78 (m, 2 H) 1.18 (s, 2 H) 1.53 (s, 3 H) 2.04 - 2.16 (m, 2 H) 2.52 - 2.54 (m, 4 H) 3.86 (s, 3 H) 4.49 - 4.58 (m, 2 H) 6.82 (s, 1 H) 7.56 (s, 1 H) 7.82 (s, 1 H) 9.47 - 9.57 (s, 1 H) 12.87 - 12.95 (s, 1 H). LC-MS: 406.2 [M-H]⁻, RT 0.89 min.

### Example 34

### 10-((benzylamino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 34)

To a solution of an a 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 212 mg, 0.42 mmol) in dichloroethane (4.5 mL) was added benzylamine (60 gel, 0.55 mmol) followed by AcOH (40 µL, 0.67 mmol). After stirring at room temperature for 10 min, NaBH(OAc)₃ (150 mg, 0.71 mmol) was added. The reaction was stirred at room temperature overnight. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt (130.0 mg, 44%).

To the product (130.0 mg, 0.18 mmol) obtained above was added i-Pr₃SiH (1.3 mL) followed by TFA (1.3 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a pale yellow solid (80.3 mg, 91%).
¹H NMR (500 MHz, DMSO-*d*₆) δ ppm 2.09 (quin, *J*=6.6 Hz, 2 H) 2.70 (br. s., 2 H) 3.50 (br. s., 2 H) 3.80 (s, 3 H) 4.27 (br. s., 2 H) 4.38 - 4.48 (m, 2 H) 6.85 (s, 1 H) 7.40 - 7.49 (m, 3 H) 7.53 (s, 1 H) 7.60 (d, *J*=6.3 Hz, 2 H) 7.81 (s, 1 H) 9.79 (br. s., 2 H) 12.89 (br. s., 1 H) 13.84 (br. s., 1 H). LC-MS 442.1 [M-H]⁻, 444.2 [M+H]⁺, RT 1.09 min.

### Example 35

### 10-(aminomethyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 35)

A solution of 10-((benzylamino)methyl)-4-hydroxy-9-methyl-2-oxo-1,2,5,6,7,9-hexahydropyrido[3',2':6,7]cyclohepta[1,2-f]indole-3-carboxylic acid hydrochloride (51.0 mg, 0.11 mmol) in MeOH (5.0 mL) was hydrogenated over Pd(OH)₂/C (20%, 20 mg) at 1 atm of H₂ for 2.5 h. After complete consumption of the starting material, the catalyst was filtered and washed with MeOH (5 mL) and TFA (2 mL). The mother liquor was concentrated and the residue was treated with HCl solution (2M Et₂O, 2.0 mL). The mixture was diluted with Et₂O and the resulting solid was filtered and washed with Et₂O. The product HCl salt was obtained as a pale yellow solid (28.8 mg, 70%).
¹H NMR (500 MHz, DMSO-*d*₆) δ ppm 2.09 (quin, *J*=6.0 Hz, 2 H) 2.63 - 2.77 (m, 2 H) 3.44 - 3.65 (m, 2 H) 3.81 (s, 3 H) 4.32 (br. s., 2 H) 6.70 (s, 1 H) 7.52 (s, 1 H) 7.79 (s, 1 H) 8.50 (br. s., 3 H) 12.89 (br. s., 1 H) 13.84 (s, 1 H). LC-MS 352.1 [M-H]⁻, 354.2 [M+H]⁺, RT 0.69 min.

### Example 36

### 10-((cyclopropylamino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 36)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120.0 mg, 0.24 mmol) in dichloroethane (2.5 mL) was added benzylamine (35 µL, 0.55 mmol) followed by AcOH (30 µL, 0.52 mmol). After stirring at room temperature for 10 min, NaBH(OAc)₃ (100.0 mg, 0.47 mmol) was added. The reaction was stirred at room temperature overnight. The dichloroethane was removed and the residue was dissolved in MeOH (7 mL) and several drops of TFA to generate a homogeneous mixture that was filtered through a PTFE micron filter and purified directly by preparative HPLC to provide the product as a TFA salt (91.4 mg, 58%).

To the product (91.4 mg, 0.14 mmol) obtained above was added i-Pr₃SiH (0.9 mL) followed by TFA (0.9 mL). The mixture was heated at 60 °C for 2 h and monitored by LC/MS. After complete consumption of starting material, the TFA was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a pale pink solid (48.6 mg, 81%).
¹H NMR (500 MHz, DMSO-*d*₆) δ ppm 0.78 (q, *J*=6.5 Hz, 2 H) 0.94 - 1.00 (m, 2 H) 2.09 (quin, *J*=6.3 Hz, 2 H) 2.70 (br. s., 2 H) 2.74 - 2.81 (m, 1 H) 3.45 - 3.64 (m, 2 H) 3.87 (s, 3 H) 4.51 (br. s., 2 H) 6.82 (s, 1 H) 7.54 (s, 1 H) 7.80 (s, 1 H) 9.65 (br. s., 2 H) 12.89 (br. s., 1 H) 13.84 (br. s., 1 H). LC-MS 392.1 [M-H]⁻, 394.1 [M+H]⁺, RT 1.02 min.

### Example 37

### 10-(((cyclobutylmethyl)amino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 37)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2.5 mL)was added cyclobutylmethanamine hydrochloride (45.0 mg, 0.37 mmol) followed NEt₃ (55.0 µL, 0.39 mmol). The mixture was stirred for 5 min at room temperature, then AcOH (25 µL, 0.43 mmol) was added. After stirring at room temperature for 5 min, NaBH(OAc)₃ (90.0 mg, 0.42 mmol) was added. The reaction was stirred at room temperature overnight. The dichloroethane was then removed and the residue was dissolved in MeOH (7 mL) and several drops of TFA to generate a homogeneous mixture that was filtered through a PTFE micron filter and purified directly by preparative HPLC to provide the product as a TFA salt (79.3 mg, 48%).

To the product (79.3 mg, 0.12 mmol) obtained above was added i-Pr₃SiH (0.8 mL) followed by TFA (0.8 mL). The mixture was heated at 60 °C for 2 h and monitored by LC/MS. After complete consumption of starting material, the TFA was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a pale yellow solid (45.0 mg, 85%).
¹H NMR (500 MHz, DMSO-*d*₆) δ ppm 1.73 - 1.93 (m, 4 H) 2.08 (s, 4 H) 2.65 - 2.80 (m, 3 H) 3.06 (q, *J*=6.3 Hz, 2 H) 3.63 (br. s., 2 H) 3.84 (s, 3 H) 4.38 (br. s., 2 H) 6.83 (s, 1 H) 7.54 (s, 1 H) 7.81 (s, 1 H) 9.27 (br. s., 2 H) 12.89 (br. s., 1 H) 13.84 (s, 1 H). LC-MS 420.1 [M-H]⁻, 422.2 [M+H]⁺, RT 1.06 min.

### Example 38

### 4-hydroxy-9-methyl-10-((((2-methylcyclopropyl)methyl)amino)methyl)-2-oxo-1,2,5,6,7,9-hexahydropyrido[3',2':6,7]cyclohepta[1,2-f]indole-3-carboxylic acid hydrochloride (Cpd 38)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2.5 mL) was added (2-methylcyclopropyl)methanamine hydrochloride (45.0 mg, 0.37 mmol) followed NEt₃ (55.0 µL, 0.39 mmol). The mixture was stirred for 5 min at room temperature, then AcOH (25 µL, 0.43 mmol) was added. After stirring at room temperature for 5 min, NaBH(OAc)₃ (90.0 mg, 0.42 mmol) was added. The reaction was stirred at room temperature overnight. The dichloroethane was then removed and the residue was dissolved in MeOH (7 mL) and several drops of TFA to generate a homogeneous mixture that was filtered through a PTFE micron filter and purified directly by preparative HPLC to provide the product as a TFA salt (83.0 mg, 51%).

To the product (83.0 mg, 0.12 mmol) obtained above was added i-Pr₃SiH (0.8 mL) followed by TFA (0.8 mL). The mixture was heated at 60 °C for 2 h and monitored by LC/MS. After complete consumption of starting material, the TFA was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a pale yellow solid (45.0 mg, 81%).
¹H NMR (500 MHz, DMSO-*d*₆) δ ppm 0.34 - 0.42 (m, 1 H) 0.56 (dt, *J*=8.6, 4.4 Hz, 1 H) 0.73 - 0.92 (m, 2 H) 1.04 (d, *J*=5.7 Hz, 3 H) 2.09 (quin, *J*=6.6 Hz, 2 H) 2.64 - 2.78 (m, 2 H) 2.83 - 3.01 (m, 2 H) 3.58 (br. s., 2 H) 3.85 (s, 3 H) 4.33 - 4.48 (m, 2 H) 6.83 (s, 1 H) 7.54 (s, 1 H) 7.81 (s, 1 H) 9.39 (br. s., 2 H) 12.89 (s, 1 H) 13.84 (s, 1 H). LC-MS 420.1 [M-H]⁻, 422.2 [M+H]⁺, RT 1.08 min.

### Example 39

### 4-hydroxy-9-methyl-2-oxo-10-(((pyridin-4-ylmethyl)amino)methyl)-1,2,5,6,7,9-hexahydropyrido[3',2':6,7]cyclohepta[1,2-f]indole-3-carboxylic acid hydrochloride (Cpd 39)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120.0 mg, 0.24 mmol) in dichloroethane (2.5 mL) was added pyridin-4-ylmethanamine (30 µL, 0.30 mmol) followed by AcOH (20 µL, 0.35 mmol). After stirring at room temperature for 10 min, NaBH(OAc)₃ (85.0 mg, 0.40 mmol) was added. The reaction was stirred at room temperature overnight. The dichloroethane was then removed and the residue was dissolved in MeOH (7 mL) and several drops of TFA to generate a homogeneous mixture that was filtered through a PTFE micron filter and purified directly by preparative HPLC to provide the product as a TFA salt (26.0 mg, 15%).

To the product (26.0 mg, 0.04 mmol) obtained above was added i-Pr₃SiH (0.3 mL) followed by TFA (0.3 mL). The mixture was stirred at room temperature and monitored by LC/MS. After complete consumption of starting material, the TFA was removed under reduced pressure. Addition of HCl solution (2M Et₂O, 1.0 mL) to the oily residue resulted in precipitate formation. The mixture was diluted with Et₂O and the resulting solid was filtered and washed with Et₂O. The product HCl salt was obtained as a pale pink solid (11.0 mg, 62%).
¹H NMR (500 MHz, DMSO-*d*₆) δ ppm 2.09 (quin, *J*=6.6 Hz, 2 H) 2.63 - 2.76 (m, 2 H) 3.80 - 3.84 (m, 2 H) 3.85 (s, 3 H) 4.42 - 4.47 (m, 2 H) 4.48 - 4.53 (m, 2 H) 6.88 (s, 1 H) 7.53 (s, 1 H) 7.81 (s, 1 H) 7.93 (d, *J*=5.4 Hz, 2 H) 8.80 (d, *J*=5.4 Hz, 2 H) 10.27 (br. s., 2 H) 12.90 (br. s., 1 H) 13.84 (br. s., 1 H). LC-MS 443.2 [M-H]⁻, 445.2 [M+H]⁺, RT 0.99 min.

### Example 40

### 4-hydroxy-9-methyl-2-oxo-10-(piperidin-1-ylmethyl)-1,2,5,6,7,9-hexahydropyrido [3',2':6,7]cyclohepta[1,2-f]indole-3-carboxylic acid hydrochloride (Cpd 40)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2.5 mL) was added piperidine (47 µL, 0.48 mmol) followed by AcOH (34 µL, 0.48 mmol). After stirring at room temperature for 10 min, NaBH(OAc)₃ (105 mg, 0.50 mmol) was added. The reaction mixture was stirred at room temperature for 1.5 h and monitored by LC/MS until the starting material was completely consumed. The dichloroethane was then removed and the residue was dissolved in MeOH (5.0 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a pale pink solid (22.0 mg, 20%).
¹H NMR (500 MHz, DMSO-*d*₆) δ ppm 1.62 - 1.91 (m, 6 H) 2.09 (m, 2 H) 2.70 (m, 2 H) 2.98 (m, 2 H) 3.43 (m, 4 H, solvent overlap) 3.90 (s, 3 H) 4.54 (m, 2 H) 6.94 (br. s., 1 H) 7.55 (br. s., 1 H) 7.82 (s, 1 H) 10.68 (br. s., 1 H) 12.90 (br. s., 1 H) 13.84 (br. s., 1 H).. LC-MS 420.2 [M-H]⁻, 422.2 [M+H]⁺, RT 0.82 min.

### Example 41

### 4-hydroxy-9-methyl-10-(morpholinomethyl)-2-oxo-1,2,5,6,7,9-hexahydropyrido[3',2':6,7]cyclohepta[1,2-f]indole-3-carboxylic acid hydrochloride (Cpd 41)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2.5 mL) was added morpholine (41 µL, 0.48 mmol) followed by AcOH (34 µL, 0.48 mmol). After stirring at room temperature for 10 min, NaBH(OAc)₃ (105 mg, 0.50 mmol) was added. The reaction mixture was stirred at room temperature for 1.5 h and monitored by LC/MS until the starting material was completely consumed. The dichloroethane was then removed and the residue was dissolved in MeOH (5.0 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a pale pink solid (40.0 mg, 39%).
¹H NMR (500 MHz, DMSO-*d*₆) δ ppm 1.96 - 2.15 (m, 2 H) 2.59 - 2.79 (m, 2 H) 3.15 - 3.25 (m, 2 H) 3.28 - 3.62 (m, 4 H) 3.76 - 4.04 (m, 7 H) 4.62 (br. s., 2 H) 6.95 (br. s., 1 H) 7.56 (br. s., 1 H) 7.82 (s, 1 H) 11.54 (br. s., 1 H) 12.90 (br. s., 1 H) 13.84 (br. s., 1 H). LC-MS 422.2 [M-H]⁻, 424.2 [M+H]⁺, RT 0.80 min.

### Example 42

### 4-hydroxy-9-methyl-10-((4-methylpiperazin-1-yl)methyl)-2-oxo-1,2,5,6,7,9-hexahydropyrido[3',2':6,7]cyclohepta[1,2-f]indole-3-carboxylic acid dihydrochloride (Cpd 42)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2.5 mL) was added 1-methyl piperazine (47 µL, 0.48 mmol) followed by AcOH (34 µL, 0.48 mmol). After stirring at room temperature for 10 min, NaBH(OAc)₃ (105 mg, 0.50 mmol) was added. The reaction mixture was stirred at room temperature for 1.5 h and monitored by LC/MS until the starting material was completely consumed. The dichloroethane was then removed and the residue was dissolved in MeOH (5.0 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product was obtained as a pale pink solid (75.2 mg, 70%) as dihydrochloride salt.
¹H NMR (500 MHz, DMSO-*d*₆) δ ppm 2.02 - 2.15 (m, 3 H) 2.61 - 2.73 (m, 2 H) 2.74 - 2.86 (m, 2 H) 3.22 - 3.69 (m, 9 H, solvent overlap) 3.87 (br. s., 3 H) 3.96 - 4.01 (m, 2 H) 6.75 - 6.79 (m, 1 H) 7.52 (s, 1 H) 7.78 (br. s., 1 H) 9.47 - 9.83 (m, 2 H) 12.92 (s, 1 H) 13.84 (br. s., 1 H). LC-MS 435.2 [M-H]⁻, 437.3 [M+H]⁺, RT 0.83 min.

### Example 43

### 10-((diethylamino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 43)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2.5 mL) was added diethyl amine (50 µL, 0.48 mmol) followed by AcOH (34 µL, 0.48 mmol). After stirring at room temperature for 10 min, NaBH(OAc)₃ (105 mg, 0.50 mmol) was added. The reaction mixture was stirred at room temperature for 1.5 h and monitored by LC/MS until the starting material was completely consumed. The dichloroethane was then removed and the residue was dissolved in MeOH (5.0 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a pale pink solid (8.5 mg, 7%).
¹H NMR (500 MHz, DMSO-*d*₆) δ ppm 1.31 (t, *J*=6.78 Hz, 6 H) 1.96 - 2.23 (m, 2 H) 2.51 (s, 2 H) 2.57 - 2.86 (m, 2 H) 3.06 - 3.25 (m, 4 H) 3.90 (d, *J*=3.15 Hz, 3 H) 4.59 (br. s., 2 H) 6.82 - 7.02 (m, 1 H) 7.58 (s, 1 H) 7.84 (s, 1 H) 10.11 (s, 1 H) 12.71 - 13.04 (m, 1 H) 13.85 (s, 1 H) . LC-MS 408.2 [M-H]⁻, 410.2 [M+H]⁺, RT 0.80 min.

### Example 44

### 4-hydroxy-9-methyl-2-oxo-10-((propylamino)methyl)-1,2,5,6,7,9-hexahydropyrido[3',2':6,7]cyclohepta[1,2-f]indole-3-carboxylic acid hydrochloride (Cpd 44)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2.5 mL) was added propyl amine (40 µL, 0.48 mmol) followed by AcOH (34 µL, 0.48 mmol). After stirring at room temperature for 10 min, NaBH(OAc)₃ (105 mg, 0.50 mmol) was added. The reaction mixture was stirred at room temperature for 1.5 h and monitored by LC/MS until the starting material was completely consumed. The dichloroethane was then removed and the residue was dissolved in MeOH (5.0 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of the starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a pale pink solid (24.6 mg, 24%).
¹H NMR (500 MHz, DMSO-*d*₆) δ ppm 0.94 (t, *J*=7.41 Hz, 3 H) 1.53 - 1.85 (m, 2 H) 1.92 - 2.21 (m, 2 H) 2.56 - 2.83 (m, 2 H) 2.81 - 3.11 (m, 2 H) 3.86 (s, 3 H) 4.43 (br. s., 2 H) 6.84 (s, 1 H) 7.55 (s, 1 H) 7.82 (s, 1 H) 9.35 (br. s., 2 H) 12.92 (s, 1 H) 13.85 (s, 1 H) .. LC-MS 394.2 [M-H]⁻, 396.3 [M+H]⁺, RT 0.82 min.

### Example 45

### 4-hydroxy-9-methyl-2-oxo-10-((prop-2-yn-1-ylamino)methyl)-1,2,5,6,7,9-hexahydropyrido[3',2':6,7]cyclohepta[1,2-f]indole-3-carboxylic acid hydrochloride (Cpd 45)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2.5 mL) was added propargyl amine (30 µL, 0.48 mmol) followed by AcOH (34 µL, 0.48 mmol). After stirring at room temperature for 10 min, NaBH(OAc)₃ (105 mg, 0.50 mmol) was added. The reaction mixture was stirred at room temperature for 1.5 h and monitored by LC/MS until the starting material was completely consumed. The dichloroethane was then removed and the residue was dissolved in MeOH (5.0 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a pale pink solid (29.0 mg, 31%).
¹H NMR (500 MHz, DMSO-*d*₆) δ ppm 2.01 - 2.18 (m, 2 H) 2.62 - 2.80 (m, 2 H) 3.43 (m, 2 H, solvent overlap) 3.77 - 3.89 (m, 4 H) 4.00 (br. s., 2 H) 4.48 (br. s., 2 H) 6.82 (s, 1 H) 7.55 (s, 1 H) 7.82 (s, 1 H) 9.59 - 10.20 (m, 2 H) 12.77 - 13.03 (m, 1 H) 13.62 - 14.00 (m, 1 H) LC-MS 390.1 [M-H]⁻, 392.2 [M+H]⁺, RT 0.80 min.

### Example 46

### (R)-10-((3-fluoropyrrolidin-1-yl)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 46)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2 mL) was added (R)-3-fluoropyrrolidine hydrochloride (63 mg, 0.50 mmol) followed NEt₃ (70 µL, 0.50 mmol). The mixture was stirred for 5 min at room temperature, then AcOH (30 µL, 0.50 mmol) was added. After stirring at room temperature for 10 min, NaBH(OAc)₃ (106 mg, 0.50 mmol) was added. The reaction mixture was stirred at room temperature for 1.5 h and monitored by LC/MS until the starting material was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a pale white solid (51 mg, 46% overall yield).
¹H NMR (500 MHz, DMSO-*d*₆) δ ppm 1.98 - 2.15 (m, 2 H) 2.15 - 2.41 (m, 2 H) 2.45 - 2.60 (m, 2 H) 2.70 (br. s., 2 H) 3.45 - 3.70 (m, 3 H) 3.70 - 3.85 (m, 1 H) 3.85 - 3.95 (m, 3 H) 4.70 (br. s., 2 H) 5.38 - 5.58 (m, 1 H) 6.90 - 7.00 (m, 1 H) 7.55 (s, 1 H) 7.82 (s, 1 H) 11.19 (br. s., 1 H) 11.57 (br. s., 1 H) 12.88 (br. s., 1 H) 13.85 (s, 1 H). LC-MS 424.1 [M-H]⁻, 426.2 [M+H]⁺, RT 0.99 min.

### Example 47

### 10-((3-(dimethylamino)pyrrolidin-1-yl)methyl)-4-hydroxy-9-methyl-2-oxo-1,2,5,6,7,9-hexahydropyrido[3',2':6,7]cyclohepta[1,2-f]indole-3-carboxylic acid dihydrochloride (Cpd 47)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2 mL) was added *N, N-*dimethylaminopyrrolidine (70 µL, 0.55 mmol) followed by AcOH (30 µL, 0.50 mmol). After stirring at room temperature for 10 min, NaBH(OAc)₃ (106 mg, 0.50 mmol) was added. The reaction mixture was stirred at room temperature for 1.5 h and monitored by LC/MS until the starting material was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product was obtained as a pale yellow solid (67 mg, 53% overall yield) as dihydrochloride salt.
¹H NMR (500 MHz, MeOH-*d*₄) δ ppm 2.10 - 2.27 (m, 2 H) 2.45 (br. s., 2 H) 2.65 (br. s., 2 H) 2.79 (br. s., 2 H) 2.97 (br. s., 6 H) 3.64 - 4.05 (m, 3 H) 3.95 (s, 3 H) 4.21 (br. s., 2 H) 4.75 (br. s., 2 H) 6.95 (br. s., 1 H) 7.50 (s, 1 H) 7.82 (br. s., 1 H). LC-MS 449.2 [M-H]⁻, 451.2 [M+H]⁺, RT 0.97 min.

### Example 48

### 10-((dimethylamino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 48)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2 mL) was added Me₂NH solution (2M THF, 0.30 mL, 0.60 mmol) followed by AcOH (70 µL, 0.50 mmol). After stirring at room temperature for 10 min, NaBH(OAc)₃ (106 mg, 0.50 mmol) was added. The reaction mixture was stirred at room temperature for 1.5 h and monitored by LC/MS until the starting material was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a yellow solid (53 mg, 53% overall yield).
¹H NMR (500 MHz, DMSO-*d*₆) δ ppm 2.09 (t, *J*=6.62 Hz, 2 H) 2.45 - 2.57 (m, 2 H) 2.71 (br. s., 2 H) 2.79 (s, 6 H) 3.88 (s, 3 H) 4.58 (s, 2 H) 6.89 (s, 1 H) 7.56 (s, 1 H) 7.84 (s, 1 H) 10.53 (br. s., 1 H) 12.89 (br. s., 1 H) 13.85 (s, 1 H). LC-MS 380.1 [M-H]⁻, 382.1 [M+H]⁺, RT 0.97 min.

### Example 49

### (S)-10-((3-aminopyrrolidin-1-yl)methyl)-4-hydroxy-9-methyl-2-oxo-1,2,5,6,7,9-hexahydropyrido[3',2':6,7]cyclohepta[1,2-f]indole-3-carboxylic acid dihydrochloride (Cpd 49)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2 mL) was added (S)-tert-butyl pyrrolidin-3-ylcarbamate (95 mg, 0.51 mmol) followed NEt₃ (70 µL, 0.50 mmol). The mixture was stirred for 5 min at room temperature, then AcOH (30 µL, 0.50 mmol) was added. After stirring at room temperature for 10 min, NaBH(OAc)₃ (106 mg, 0.50 mmol) was added. The reaction mixture was stirred at room temperature for 1.5 h and monitored by LC/MS until the starting material was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product was obtained as a pale white solid (63 mg, 53% overall yield) as dihydrochloride salt. ¹H NMR (500 MHz, DMSO-*d*₆) δ ppm 1.98 - 2.14 (m, 2 H) 2.14 - 2.43 (m, 2 H) 2.45 - 2.60 (m, 2 H) 2.71 (br. s., 2 H) 3.45 - 3.85 (m, 4 H) 3.92 (s, 3 H) 4.11 (br. s., 1 H) 4.70 - 4.91 (m, 2 H) 6.92 (br. s., 1 H) 7.56 (s, 1 H) 7.82 (s, 1 H) 8.48 (br. s., 2 H) 8.63 (br. s., 1 H) 11.41 (br. s., 1 H) 11.78 (br. s., 1 H) 12.89 (br. s., 1 H) 13.85 (s, 1 H).. LC-MS 421.1 [M-H]⁻, 423.2 [M+H]⁺, RT 0.88 min.

### Example 50

### 4-hydroxy-9-methyl-2-oxo-10-(pyrrolidin-1-ylmethyl)-1,2,5,6,7,9-hexahydropyrido[3',2':6,7]cyclohepta[1,2-f]indole-3-carboxylic acid hydrochloride (Cpd 50)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2 mL) was added pyrrolidine (50 µL, 0.60 mmol) followed by AcOH (30 µL, 0.50 mmol). After stirring at room temperature for 10 min, NaBH(OAc)₃ (106 mg, 0.50 mmol) was added. The reaction mixture was stirred at room temperature for 1.5 h and monitored by LC/MS until the starting material was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a pale white solid (54 mg, 51 % overall yield).
¹H NMR (500 MHz, DMSO-*d*₆) δ ppm 1.85 - 2.00 (m, 2 H) 2.00 - 2.15 (m, 4 H) 2.48 - 2.55 (m, 2 H) 2.65 - 2.77 (m, 2 H) 3.14 - 3.23 (m, 2 H) 3.43 - 3.54 (m, 2 H) 3.90 (s, 3 H) 4.67 (br. s., 2 H) 6.91 (br. s., 1 H) 7.56 (s, 1 H) 7.82 (s, 1 H) 10.70 (br. s., 1 H) 12.89 (br. s., 1 H) 13.85 (s, 1 H). LC-MS 406.2 [M-H]⁻, 408.2 [M+H]⁺, RT 0.99 min.

### Example 51

### 4-hydroxy-10-((3-hydroxypyrrolidin-1-yl)methyl)-9-methyl-2-oxo-1,2,5,6,7,9-hexahydropyrido[3',2':6,7]cyclohepta[1,2-f]indole-3-carboxylic acid hydrochloride (Cpd 51)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2 mL) was added pyrrolidin-3-ol (40 µL, 0.49 mmol) followed by AcOH (30 µL, 0.50 mmol). After stirring at room temperature for 10 min, NaBH(OAc)₃ (106 mg, 0.50 mmol) was added. The reaction mixture was stirred at room temperature for 1.5 h and monitored by LC/MS until the starting material was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was stirred at room temperature for 12 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a pale white solid (50 mg, 45% overall yield).
¹H NMR (500 MHz, DMSO-*d*₆) δ ppm 1.80 - 1.91 (m, 1 H) 1.91 - 2.01 (m, 1 H) 2.01 - 2.16 (m, 3 H) 2.25 - 2.36 (m, 1 H) 2.71 (br. s., 2 H) 3.11 (d, *J*=11.66 Hz, 1 H) 3.27 - 3.35 (m, 1 H) 3.47 - 3.63 (m, 2 H) 3.84 - 3.92 (m, 3 H) 4.40 - 4.51 (m, 1 H) 4.60 - 4.75 (m, 2 H) 5.40 - 5.65 (m, 1 H) 6.89 - 6.95 (m, 1 H) 7.55 (s, 1 H) 7.82 (s, 1 H) 10.71 (br. s., 1 H) 11.03 (br. s., 1 H) 12.88 (br. s., 1 H) 13.85 (s, 1 H). LC-MS 422.2 [M-H]⁻, 424.2 [M+H]⁺, RT 0.77 min.

### Example 52

### 4-hydroxy-9-methyl-2-oxo-10-(((pyridin-3-ylmethyl)amino)methyl)-1,2,5,6,7,9-hexahydropyrido[3',2':6,7]cyclohepta[1,2-f]indole-3-carboxylic acid hydrochloride (Cpd 52)

To a solution of 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 120 mg, 0.24 mmol) in dichloroethane (2 mL) was added pyridin-3-ylmethanamine (50 µL, 0.49 mmol) followed by AcOH (30 µL, 0.50 mmol). After stirring at room temperature for 10 min, NaBH(OAc)₃ (106 mg, 0.50 mmol) was added. The reaction mixture was stirred at room temperature for 1.5 h and monitored by LC/MS until the starting material was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt.

To the product obtained above was added i-Pr₃SiH (1.0 mL) followed by TFA (1.0 mL). The mixture was stirred at room temperature for 12 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a pale pink solid (51 mg, 44% overall yield). ¹H NMR (500 MHz, DMSO-*d*₆) δ ppm 2.09 (t, *J*=6.62 Hz, 2 H) 2.49 - 2.53 (m, 2 H) 2.70 (br. s., 2 H) 3.84 (s, 3 H) 4.39 (br. s., 2 H) 4.50 (br. s., 2 H) 6.85 (s, 1 H) 7.54 (s, 1 H) 7.61 - 7.71 (m, 1 H) 7.81 (s, 1 H) 8.24 (d, *J*=7.25 Hz, 1 H) 8.71 (dd, *J*=5.04, 1.26 Hz, 1 H) 8.86 (s, 1 H) 9.90 (br. s., 2 H) 12.90 (br. s., 1 H) 13.83 (br. s., 1 H). LC-MS 443.2 [M-H]⁻, 445.3 [M+H]⁺, RT 0.96 min.

### Example 53

### 9-methyl-10-((methylamino)methyl)-2-oxo-1,2,5,6,7,9-hexahydropyrido[3',2':6,7]cyclohepta[1,2-f]indole-3-carboxylic acid hydrochloride (Cpd 53)

### Step 1: methyl 10-(((tert-butyldimethylsilyl)oxy)methyl)-1-(2,4-dimethoxybenzyl)-9-methyl-2-oxo-1,2,5,6,7,9-hexahydropyrido [3',2': 6,7] cyclohepta[1,2-f] indole-3-carboxylate

Crude *N*-(2-((tert-butyldimethylsilyloxy)methyl)-1-methyl-6,7,8,9-tetrahydrocyclohepta[f]indol-5(1H)-ylidene)-1-(2,4-dimethoxyphenyl)methanamine (Example 22, step 5, 2.24 g, 4.42 mmol) and dimethyl 2-(methoxymethylene)malonate (1.20 g, 7.46 mmol) were mixed together in Ph₂O (10 mL). With stirring, the mixture was placed onto a pre-heated heat block at 200 °C and heated for 15 min after the initial bubbling of MeOH was observed (occurs ant ∼ 160 °C internal reaction temperature). The reaction mixture was cooled to room temperature, then purified by column chromatography (hexanes followed by EtOAc/hexanes 0-80% gradient) to yield the product as a yellow foam (1.007 g, 37% 2 steps) LC-MS 617.6 [M+H]⁺, RT 1.73 min.

### Step 2: methyl 1-(2,4-dimethoxybenzyl)-10-(hydroxymethyl)-9-methyl-2-oxo-1,2,5,6,7,9-hexahydropyrido[3',2':6,7]cyclohepta[1,2-f]indole-3-carboxylate

To a solution of methyl 10-(((tert-butyldimethylsilyl)oxy)methyl)-1-(2,4-dimethoxybenzyl)-9-methyl-2-oxo-1,2,5,6,7,9-hexahydropyrido[3',2':6,7]cyclohepta[1,2-f]indole-3-carboxylate (1.007 g, 1.63 mmol) in THF (6 mL) was added TBAF solution (1M THF, 2.50 mL, 2.50 mmol). The reaction mixture was stirred at room temperature for 2 h until the starting material was completely consumed. The THF was removed and the residue was purified by column chromatography (MeOH/DCM, 0-5% gradient). The product was obtained as a yellow solid in nearly quantitative yield (0.80 g).
¹H NMR (500 MHz, CHCl₃-*d*) δ ppm 1.86 - 2.10 (m, 3 H) 2.30 - 2.42 (m, 2 H) 2.60 (dd, *J*=13.1, 5.8 Hz, 1 H) 3.35 (s, 3 H) 3.75 (s, 3 H) 3.82 (s, 3 H) 3.93 (s, 3 H) 4.80 (s, 2 H) 5.28 - 5.37 (m, 2 H) 6.24 (d, *J*=2.2 Hz, 1 H) 6.33 (dd, *J*=8.5, 2.2 Hz, 1 H) 6.36 (s, 1 H) 6.81 (d, *J*=8.5 Hz, 1 H) 7.09 (s, 1 H) 7.35 (s, 1 H) 8.10 (s, 1 H). LC-MS 503.5 [M+H]⁺, RT 1.18 min.

### Step 3: 1-(2,4-dimethoxybenzyl)-10-(hydroxymethyl)-9-methyl-2-oxo-1,2,5,6,7,9-hexahydropyrido[3',2':6,7]cyclohepta[1,2-f]indole-3-carboxylic acid

To a suspension of methyl 1-(2,4-dimethoxybenzyl)-10-(hydroxymethyl)-9-methyl-2-oxo-1,2,5,6,7,9-hexahydropyrido[3',2':6,7]cyclohepta[1,2-f]indole-3-carboxylate (0.80 g, 1.60 mmol) in THF (6 mL) was added LiOH solution (1M H₂O, 3.0 mL, 3.0 mmol). The reaction mixture was stirred at room temperature for 1 h, then acidified with 1M HCl to pH∼2. The product was extracted with DCM (2x50 mL). The combined organics were dried over Na₂SO₄ and DCM was removed to afford a product as a yellow solid in nearly quantitative yield (0.75 g) LC-MS 489.5 [M+H]⁺, RT 1.20 min.

### Step 4: 1-(2,4-dimethoxybenzyl)-10-formyl-9-methyl-2-oxo-1,2,5,6,7,9-hexahydropyrido[3',2':6,7]cyclohepta[1,2-f]indole-3-carboxylic acid

To a solution of 1-(2,4-dimethoxybenzyl)-10-(hydroxymethyl)-9-methyl-2-oxo-1,2,5,6,7,9-hexahydropyrido[3',2':6,7]cyclohepta[1,2-f]indole-3-carboxylic acid (0.75 g, 1.54 mmol) in DCM (15 mL) was added activated MnO₂ (1.5 g+1.5 g+0.75 g, 15.5 mmol +15.5 mmol +7.75 mmol) in 3 portions at 30 min intervals. The reaction was monitored by LC/MS. After complete consumption of starting material MnO₂ was filtered and washed with DCM. The mother liquor was concentrated affording product as dark red foam (0.638 g, 80% 3 steps) which was used in the next step without further purification. LC-MS 487.0 [M+H]⁺, RT 1.31 min.

### Steps 5-6: 9-methyl-10-((methylamino)methyl)-2-oxo-1,2,5,6,7,9-hexahydropyrido[3',2':6,7]cyclohepta[1,2-f]indole-3-carboxylic acid hydrochloride

To a solution of 1-(2,4-dimethoxybenzyl)-10-formyl-9-methyl-2-oxo-1,2,5,6,7,9-hexahydropyrido[3',2':6,7]cyclohepta[1,2-f]indole-3-carboxylic acid (128 mg, 0.26 mmol) in dichloroethane (2 mL) was added MeNH₂ solution (2M THF, 0.50 mL, 1.0 mmol) followed by AcOH (30 µL, 0.50 mmol). After stirring at room temperature for 10 min, NaBH(OAc)₃ (95 mg, 0.45 mmol) was added. The reaction was stirred at room temperature 4 h and monitored by LC/MS. Starting material remained in the reaction mixture after 4 h. Additional MeNH₂ solution (2M THF, 0.50 mL, 1.0 mmol) and NaBH(OAc)₃ (95 mg, 0.45 mmol) were added and the mixture was stirred overnight at room temperature. The dichloroethane was removed and the residue was dissolved in MeOH (7 mL). The solution was filtered through a PTFE micron filter, then purified by preparative HPLC to provide the product as a TFA salt (77.8 mg, 48%).

To a solution of the product (77.8 mg, 0.13 mmol) obtained above in DCM (1 mL) was added i-Pr₃SiH (0.50 mL) followed by TFA (0.50 mL). The mixture was stirred at room temperature for 1 h and monitored by LC/MS. After complete consumption of starting material, the DCM and TFA were removed under reduced pressure. Addition of HCl solution (2M Et₂O, 1.5 mL) to the oily residue resulted in precipitate formation. The product HCl salt was obtained as a yellow solid (38.6 mg, 79%).
¹H NMR (500 MHz, DMSO-*d*₆) δ ppm 2.13 (br. t, *J*= 6.8 Hz, 2 H) 2.23 - 2.41 (m, 2 H) 2.61 - 2.65 (m, 3 H) 2.66 - 2.72 (m, 2 H) 3.84 (s, 3 H) 4.42 (br. s., 2 H) 6.79 (s, 1 H) 7.54 (s, 1 H) 7.83 (s, 1 H) 8.34 (s, 1 H) 9.17 (br. s., 2 H) 13.39 (br. s., 1 H) 15.05 (br. s., 1 H). LC-MS 350.5 [M-H]⁻, 352.5 [M+H]⁺, RT 0.46 min.

### Example 54

### 10-((ethylamino)methyl)-9-methyl-2-oxo-1,2,5,6,7,9-hexahydropyrido[3',2':6,7]cyclohepta[1,2-f]indole-3-carboxylic acid hydrochloride (Cpd 54)

To a solution of 1-(2,4-dimethoxybenzyl)-10-formyl-9-methyl-2-oxo-1,2,5,6,7,9-hexahydropyrido[3',2':6,7]cyclohepta[1,2-f]indole-3-carboxylic acid (Example 53, step 4, 128.0 mg, 0.26 mmol) in dichloroethane (2.0 mL) was added ethylamine hydrochloride (31 mg, 0.38 mmol) followed NEt₃ (55 µL, 0.39 mmol). The mixture was stirred for 5 min at room temperature, then AcOH (30 µL, 0.50 mmol) was added, followed by an EtNH₂ solution (2M THF, 0.20 mL, 0.40 mmol). After stirring at room temperature for 5 min, NaBH(OAc)₃ (95 mg, 0.45 mmol) was added. The reaction was stirred at room temperature for 4 h and monitored by LC/MS. Starting material remained in the reaction mixture after 4 h. Additional EtNH₂ solution (2M THF, 0.20 mL, 0.40 mmol) and NaBH(OAc)₃ (95 mg, 0.45 mmol) were added and the mixture was stirred overnight at room temperature. The dichloroethane was removed, the residue was dissolved in MeOH (7 mL) and several drops of TFA were added. The resulting homogeneous mixture was filtered through a PTFE micron filter and purified directly by preparative HPLC to provide the product as a TFA salt (74.6 mg, 45%).

To a solution of the product (74.6 mg, 0.12 mmol) obtained above in DCM (1 mL) was added i-Pr₃SiH (0.50 mL) followed by TFA (0.50 mL). The mixture was stirred at room temperature for 1 h and monitored by LC/MS. After complete consumption of starting material, the DCM and TFA were removed under reduced pressure. Addition of an HCl solution (2M Et₂O, 1.5 mL) to the oily residue resulted in formation of a precipitate. The product HCl salt was obtained as a yellow solid (38.5 mg, 81%).
¹H NMR (500 MHz, DMSO-*d*₆) δ ppm 1.27 (t, *J*=7.3 Hz, 2 H) 2.13 (br. t, *J*=6.8 Hz, 2 H) 2.24 - 2.38 (m, 2 H) 2.65 - 2.72 (m, 2 H) 3.01 - 3.14 (m, 2 H) 3.84 (s, 3 H) 4.44 (br. s., 1 H) 6.79 (s, 1 H) 7.54 (s, 1 H) 7.83 (s, 1 H) 8.34 (s, 1 H) 9.10 (br. s., 2 H) 13.40 (br. s., 1 H) 15.06 (br. s., 1 H). LC-MS 364.6 [M-H]⁻, 366.5 [M+H]⁺, RT 0.47 min.

### Example 55

### 10-((isopropylamino)methyl)-9-methyl-2-oxo-1,2,5,6,7,9-hexahydropyrido[3',2':6,7]cyclohepta[1,2-f]indole-3-carboxylic acid hydrochloride (Cpd 55)

To a solution of 1-(2,4-dimethoxybenzyl)-10-formyl-9-methyl-2-oxo-1,2,5,6,7,9-hexahydropyrido[3',2':6,7]cyclohepta[1,2-f]indole-3-carboxylic acid (Example 53, step 4, 128 mg, 0.26 mmol) in dichloroethane (2 mL) was added isopropylamine (45 µL, 0.53 mmol) followed by AcOH (30 µL, 0.50 mmol). After stirring at room temperature for 10 min, NaBH(OAc)₃ (95 mg, 0.45 mmol) was added. The reaction was stirred at room temperature 4 h and monitored by LC/MS. Starting material remained in the reaction mixture after 4 h. Additional isopropylamine (45 µL, 0.53 mmol) and NaBH(OAc)₃ (95 mg, 0.45 mmol) were added and the mixture was stirred overnight at room temperature. The dichloroethane was removed, the residue was dissolved in MeOH (7 mL) and the solution was filtered through a PTFE micron filter, then purified by preparative HPLC to provide the product as a TFA salt (84.2 mg, 50%).

To a solution of the product (84.2 mg, 0.13 mmol) obtained above in DCM (1 mL) was added i-Pr₃SiH (0.50 mL) followed by TFA (0.50 mL). The mixture was stirred at room temperature for 1 h and monitored by LC/MS. After complete consumption of starting material, the DCM and TFA were removed under reduced pressure. Addition of HCl solution (2M Et₂O, 1.5 mL) to the oily residue resulted in precipitate formation. The product HCl salt was obtained as a yellow solid (42.9 mg, 79%).
¹H NMR (500 MHz, DMSO-*d*₆) δ ppm 1.35 (d, *J*=6.3 Hz, 6 H) 2.13 (br. t, *J*=6.8 Hz, 2 H) 2.22 - 2.42 (m, 2 H) 2.68 (m, 2 H) 3.41 - 3.51 (m, 1 H) 3.85 (s, 3 H) 4.42 (t, *J*=5.7 Hz, 2 H) 6.81 (s, 1 H) 7.54 (s, 1 H) 7.82 (s, 1 H) 8.34 (s, 1 H) 9.17 (br. s., 2 H) 13.41 (br. s., 1 H) 15.06 (br. s., 1 H). LC-MS 378.6 [M-H]⁻, 380.5 [M+H]⁺, RT 0.48 min.

### Example 56

### 10-((tert-butylamino)methyl)-9-methyl-2-oxo-1,2,5,6,7,9-hexahydropyrido[3',2':6,7]cyclohepta[1,2-f]indole-3-carboxylic acid hydrochloride (Cpd 56)

To a solution of 1-(2,4-dimethoxybenzyl)-10-formyl-9-methyl-2-oxo-1,2,5,6,7,9-hexahydropyrido[3',2':6,7]cyclohepta[1,2-f]indole-3-carboxylic acid (Example 53, step 4, 128 mg, 0.26 mmol) in dichloroethane (2 mL) was added tert-butylamine (55 µL, 0.52 mmol) followed by AcOH (30 µL, 0.50 mmol). After stirring at room temperature for 10 min, NaBH(OAc)₃ (95 mg, 0.45 mmol) was added. The reaction was stirred at room temperature 4 h and monitored by LC/MS. Starting material remained in the reaction mixture after 4 h. Additional tert-butylamine (55 µL, 0.52 mmol) and NaBH(OAc)₃ (95 mg, 0.45 mmol) were added and the mixture was stirred overnight at room temperature. The dichloroethane was removed, the residue was dissolved in MeOH (7 mL), and the solution was filtered through a PTFE micron filter, then purified by preparative HPLC to provide the product as a TFA salt (81.2 mg, 47%).

To a solution of the product (81.2 mg, 0.12 mmol) obtained above in DCM (1 mL) was added i-Pr₃SiH (0.50 mL) followed by TFA (0.50 mL). The mixture was stirred at room temperature for 1 h and monitored by LC/MS. After complete consumption of starting material, the DCM and TFA were removed under reduced pressure. Addition of HCl solution (2M Et₂O, 1.5 mL) to the oily residue resulted in precipitate formation. The product HCl salt was obtained as a yellow solid (43.9 mg, 83%).
¹H NMR (500 MHz, DMSO-*d*₆) δ ppm 1.42 (s, 9 H) 2.13 (br. t, *J*=6.6 Hz, 2 H) 2.21 - 2.39 (m, 2 H) 2.64 - 2.72 (m, 2 H) 3.84 (s, 3 H) 4.40 (t, *J*=6.3 Hz, 2 H) 6.81 (s, 1 H) 7.55 (s, 1 H) 7.83 (s, 1 H) 8.34 (s, 1 H) 9.10 (br. s., 2 H) 13.42 (br. s., 1 H) 15.06 (br. s., 1 H). LC-MS 392.6 [M-H]⁻, 394.5 [M+H]⁺, RT 0.49 min.

### Example 57

### 10-(azetidin-1-ylmethyl)-9-methyl-2-oxo-1,2,5,6,7,9-hexahydropyrido[3',2':6,7]cyclohepta[1,2-f]indole-3-carboxylic acid hydrochloride (Cpd 57)

To a solution of 1-(2,4-dimethoxybenzyl)-10-formyl-9-methyl-2-oxo-1,2,5,6,7,9-hexahydropyrido[3',2':6,7]cyclohepta[1,2-f]indole-3-carboxylic acid (Example 53, step 4, 128.0 mg, 0.26 mmol) in dichloroethane (2.0 mL) was added azetidine hydrochloride (36 mg, 0.38 mmol) followed NEt₃ (55 µL, 0.39 mmol). The mixture was stirred for 10 min at room temperature, then AcOH (25 µL, 0.42 mmol) followed by NaBH(OAc)₃ (95 mg, 0.45 mmol) were added. The reaction was stirred at room temperature 4 h and monitored by LC/MS. Starting material remained in the reaction mixture after 4 h. Additional azetidine hydrochloride (36 mg, 0.38 mmol), NEt₃ (55 µL, 0.39 mmol) and NaBH(OAc)₃ (95 mg, 0.45 mmol) were added and the mixture was stirred overnight at room temperature. The dichloroethane was then removed, the residue was dissolved in MeOH (7 mL) and the solution was filtered through a PTFE micron filter, then purified by preparative HPLC to provide the product as a TFA salt (87.7 mg, 52%).

To a solution of the product (87.7 mg, 0.14 mmol) obtained above in DCM (1 mL) was added i-Pr₃SiH (0.50 mL) followed by TFA (0.50 mL). The mixture was stirred at room temperature for 1 h and monitored by LC/MS. After complete consumption of starting material, the DCM and TFA were removed under reduced pressure. Addition of HCl solution (2M Et₂O, 1.5 mL) to the oily residue resulted in precipitate formation. The product HCl salt was obtained as a yellow solid (44.3 mg, 78%).
¹H NMR (500 MHz, DMSO-*d*₆) δ ppm 2.13 (br. t, *J*=6.8 Hz, 2 H) 2.22 - 2.46 (m, 4 H) 2.63 - 2.72 (m, 2 H) 3.83 (s, 3 H) 4.00 - 4.09 (m, 2 H) 4.09 - 4.20 (m, 2 H) 4.65 (br. s., 1 H) 6.83 (s, 1 H) 7.53 (s, 1 H) 7.82 (s, 1 H) 8.34 (s, 1 H) 10.76 (br. s., 1 H) 13.37 (br. s., 1 H) 14.91 - 15.22 (m, 1 H). LC-MS 376.6 [M-H]⁻, 378.5 [M+H]⁺, RT 0.47 min.

### Example 58

### 4-hydroxy-10-methyl-11-((methylamino)methyl)-2-oxo-2,5,6,7,8,10-hexahydro-1H-pyrido[3',2':7,8]cycloocta[1,2-f]indole-3-carboxylic acid hydrochloride (Cpd 58)

### Step 1: 1-(2-((tert-butyldimethylsilyloxy)methyl)-1-methyl-6-vinyl-1H-indol-5-yl)hex-5-en-1-ol

To a solution of 2-((tert-butyldimethylsilyloxy)methyl)-1-methyl-6-vinyl-1H-indole-5-carbaldehyde (Example 19, step 6, 5.20 g, 15.77 mmol) in THF (35 mL) at -78 °C was added 4-pentenylmagnesium bromide (0.5M in 2-Me-THF, 45.0 mL, 22.5 mmol) dropwise over ∼15 min. The reaction was stirred at -78 °C for 10 min and slowly allowed to warm up to room temperature over 2 h. LC/MS showed complete consumption of starting material. The reaction mixture was cooled to 0 °C and then the reaction was quenched by addition of NH₄Cl (aqueous saturated, 30 mL). The product was extracted with EtOAc (3x80 mL). The combined organics were washed with NaCl (aqueous saturated, 50 mL) and dried over Na₂SO₄. The solvent was removed to provide the product as a pale-yellow oil (6.25 g, quant) in nearly quantitative yield. The product was used directly in the next step without further purification.
¹H NMR (500 MHz, CHCl₃-*d*) δ ppm 0.07 (s, 3 H) 0.08 (s, 3 H) 0.91 (s, 9 H) 1.41 - 1.51 (m, 1 H) 1.55 - 1.73 (m, 1 H) 1.78 - 1.88 (m, 2 H) 2.07 - 2.13 (m, 2 H) 3.80 (s, 3 H) 4.83 (s, 2 H) 4.94 (ddt, *J*=10.3, 2.1, 1.2, 1.2 Hz, 1 H) 5.01 (dq, *J*=17.0, 1.8 Hz, 1 H) 5.09 (t, *J*=6.5 Hz, 1 H) 5.29(dd, *J*=10.7, 1.6 Hz, 1 H) 5.65 (dd, *J*=17.2, 1.7 Hz, 1 H) 5.81 (ddt, *J*=17.0, 10.2,6.7,6.7 Hz, 1 H) 6.35 (s, 1 H) 7.24 (dd, *J*=17.7, 11.0 Hz, 1 H) 7.40 (s, 1 H) 7.66 (s, 1 H). LC-MS 400.4 [M+H]⁺, RT 1.80 min.

### Step 2: 5-(1-(tert-butyldimethylsilyloxy)hex-5-enyl)-2-((tert-butyldimethylsilyloxy)methyl)-1-methyl-6-vinyl-1H-indole

To a solution of 1-(2-((tert-butyldimethylsilyloxy)methyl)-1-methyl-6-vinyl-1H-indol-5-yl)hex-5-en-1-ol (6.25 g, 15.64 mmol) in DCM (65 mL) at room temperature was added imidazole (1.90 g, 27.91 mmol) followed TBSCl (3.60 g, 23.88 mmol). The reaction mixture was stirred overnight under inert atmosphere. Additional imidazole (0.70 g, 10.30 mmol) and TBSCl (1.30 g, 8.63 mmol) were added to the mixture, with stirring for 6 h to push reaction to completion. The reaction was washed with water (60 mL) then NaCl (aqueous saturated, 60 mL) and dried over Na₂SO₄. The solvent was removed and the residue was purified by column chromatography on deactivated NEt₃ silica gel, eluting with hexanes (containing 2% NEt₃) and EtOAc using 0-10% gradient. The product was obtained as a yellowish oil (6.13 g, 76 % 2 steps).
¹H NMR (500 MHz, CHCl₃-*d*) δ ppm -0.18 (s, 3 H) 0.02 (s, 3 H) 0.08 (s, 3 H) 0.09 (s, 3 H) 0.89 (s, 9 H) 0.91 (s, 9 H) 1.35 - 1.46 (m, 1 H) 1.49 - 1.60 (m, 1 H) 1.61 - 1.78 (m, 2 H) 2.01 - 2.07 (m, 2 H) 3.78 (s, 3 H) 4.81 (s, 2 H) 4.92 (br. d, *J*=10.4 Hz, 1 H) 4.94 - 5.02 (m, 2 H) 5.23 (dd, *J*=10.7, 1.6 Hz, 1 H) 5.62 (dd, *J*=17.0, 1.6 Hz, 1 H) 5.73 - 5.83 (m, 1 H) 6.32 (s, 1 H) 7.27 (dd, *J*=17.0, 11.0 Hz, 1 H) 7.36 (s, 1 H) 7.59 (s, 1 H). LC-MS 514.5 [M+H]⁺, RT 1.98 min.

### Step 3: Mixture of 5-(tert-butyldimethylsilyloxy)-2-((tert-butyldimethylsilyloxy)methyl)-1-methyl-5,6,7,8-tetrahydro-1H-cycloocta[f]indole and 2-((tert-butyldimethylsilyloxy)methyl)-1-methyl-1,5,6,7-tetrahydrocyclohepta[f]indol-5-ol

To a solution of 5-(1-(tert-butyldimethylsilyloxy)hex-5-enyl)-2-((tert-butyldimethylsilyloxy)methyl)-1-methyl-6-vinyl-1H-indole (6.127 g, 11.92 mmol) in toluene (240 mL, 0.05M) under Argon was added second generation Grubbs' catalyst (300 mg, 0.35 mmol, 3 mol%). The mixture was heated at 60 °C for 2 h until the starting material was completely consumed as indicated by TLC. After cooling the reaction to room temperature, the toluene was removed under reduced pressure and the residue was purified by column chromatography using a silica gel column (EtOAc/hexanes, 0-10% gradient) to provide two inseparable products. The obtained oily material (5.058 g) was used directly in the next step.

### Step 4: Mixture of 2-(hydroxymethyl)-1-methyl-5,6,7,8-tetrahydro-1H-cycloocta[f]indol-5-ol and 2-(hydroxymethyl)-1-methyl-1,5,6,7-tetrahydrocyclohepta[f]indol-5-ol

To a solution of the mixture (5.058 g) obtained above in THF (30 mL) was added TBAF (1M THF, 32.0 mL, 32.0 mmol). The reaction mixture was stirred over 96 h at room temperature and monitored by TLC and LC/MS. After complete consumption of the starting material, the THF was removed and the residue was partitioned between H₂O (80 mL) and EtOAc (mL). The organic layer was separated and the aqueous phase was extracted with EtOAc (3x80 mL). The combined organics were washed with NaCl (aqueous saturated, 80 mL) and dried over Na₂SO₄. The solvent was removed and the two resulting products were separated by column chromatography (EtOAc/hexanes, 10-60% gradient) yielding 2-(hydroxymethyl)-1-methyl-5,6,7,8-tetrahydro-1H-cycloocta[f]indol-5-ol (0.6234 g, 20%, 2 steps) and 2-(hydroxymethyl)-1-methyl-1,5,6,7-tetrahydrocyclohepta[f]indol-5-ol (0.3949 g, 14%, 2 steps) as colorless solids.
*2-(hydroxymethyl)-1-methyl-5,6,7,8-tetrahydro-1H-cycloocta[f]indol-5-ol:* ¹H NMR (500 MHz, CHCl₃-*d*) δ ppm 1.35 - 1.48 (m, 1 H) 1.48 - 1.74 (br. m, 4 H) 2.13 (s, 1 H) 2.20 - 2.40 (m, 2 H) 3.79 (s, 3 H) 4.80 (s, 2 H) 5.32 (dd, *J*=10.9, 4.3 Hz, 1 H) 5.87 (dt, *J*=12.3, 5.0 Hz, 1 H) 6.44 (s, 1 H) 6.58 (d, *J*=12.3 Hz, 1 H) 7.06 (s, 1 H) 7.78 (s, 1 H). LC-MS 258.2 [M+H]⁺, RT 1.00 min.

### Step 5: 2-((tert-butyldimethylsilyloxy)methyl)-1-methyl-5,6,7,8-tetrahydro-1H-cycloocta[f]indol-5-ol

To a suspension of 2-(hydroxymethyl)-1-methyl-5,6,7,8-tetrahydro-1H-cycloocta[f]indol-5-ol (0.6234 g, 2.42 mmol) in DCM (15 mL) was added imidazole (0.20 g, 2.94 mmol). The reaction was cooled to 0 °C, then a solution of TBSC1 (0.40 g, 2.65 mmol) in DCM (5 mL) was added dropwise. The mixture was stirred at 0 °C and slowly allowed to warm to room temperature over 1.5 h.

LC/MS showed ∼10% of the starting material remained, including the formation of ∼10% of a di-TBS protected by-product. The reaction mixture was diluted with DCM (50 mL) and washed with H₂O (40 mL). The organic phase was dried over Na₂SO₄. The solvent was removed and all three products were separated by column chromatography (EtOAc/hexanes, 0-80% gradient), affording the desired product (0.6987 g, 77%), a di-TBS by-product (0.0775 g, 7%) and recovered starting material (0.0566 g, 9%). The di-TBS by-product was deprotected using TBAF and recycled. The desired product 2-((tert-butyldimethylsilyloxy)methyl)-1-methyl-5,6,7,8-tetrahydro-1H-cycloocta[f]indol-5-ol was obtained (0.7935 g) in 88% overall yield. LC-MS 372.3 [M+H]⁺, RT 1.64 min.

### Step 6: 2-((tert-butyldimethylsilyloxy)methyl)-1-methyl-5,6,7,8,9,10-hexahydro-1H-cycloocta[f]indol-5-ol

A solution of 2-((tert-butyldimethylsilyloxy)methyl)-1-methyl-5,6,7,8-tetrahydro-1H-cycloocta[f]indol-5-ol (0.7935 g, 2.14 mmol) in EtOAc (10 mL) and DCM (5 mL) was hydrogenated over 10% Pd/C (Degussa type, 80 mg) under H₂ (1 atm) until complete consumption of starting material as indicated by LC/MS. After 1 h, the catalyst was filtered off and the filtrate was washed with DCM. The mother liquor was concentrated affording product as a yellowish solid which was used directly in the next step.
¹H NMR (500 MHz, CHCl₃-*d*) δ ppm 0.05 (s, 3 H) 0.09 (s, 3 H) 0.89 - 0.91 (m, 9 H) 1.33 - 1.64 (m, 4 H) 1.83 (br. s, 1 H) 1.96 - 2.04 (m, 1 H) 2.11 - 2.21 (m, 1 H) 2.86 - 2.92 (m, 2 H) 3.77 (s, 3 H) 4.82 (s, 2 H) 5.25 (dd, *J*=10.4, 4.4 Hz, 1 H) 6.34 (s, 1 H) 7.03 (s, 1 H) 7.74 (s, 1 H). LC-MS 374.3 [M+H]⁺, RT 1.80 min.

### Step 7: 2-((tert-butyldimethylsilyloxy)methyl)-1-methyl-7,8,9,10-tetrahydro-1H-cycloocta[f]indol-5(6H)-one

To a solution of 2-((tert-butyldimethylsilyloxy)methyl)-1-methyl-5,6,7,8,9,10-hexahydro-1H-cycloocta[f]indol-5-ol (2.14 mmol) in DCM (50 mL) at 0 °C was added solid NaHCO₃ (1.8 g, 21.4 mmol) followed by Dess-Martin periodinane (1.09 g, 2.57 mmol). The reaction was stirred at 0 °C 15 min. LC/MC and TLC indicated complete consumption of starting material. The reaction mixture was diluted with DCM, washed with NaHCO₃ (aqueous saturated, 50 mL), Na₂S₂O₃ (10% aq, 50 mL) and NaCl (aqueous saturated, 80 mL), then the organic phase was dried over Na₂SO₄. The solvent was removed and the residue was purified by column chromatography (EtOAc/hexanes, 0-15% gradient), affording the desired product (0.4216 g, 53% 2 steps).
¹H NMR (500 MHz, CHCl₃-*d*) δ ppm 0.07 (s, 6 H) 0.90 (s, 9 H) 1.45 - 1.52 (m, 2 H) 1.87 (m, 4 H) 3.07 (t, *J*=7.1 Hz, 2 H) 3.27 (t, *J*=6.8 Hz, 2 H) 3.78 (s, 3 H) 4.82 (s, 2 H) 6.43 (s, 1 H) 7.06 (s, 1 H) 8.22 (s, 1 H). LC-MS 372.3 [M+H]⁺, RT 1.84 min.

### Step 8: N-(2-((tert-butyldimethylsilyloxy)methyl)-1-methyl-7,8,9,10-tetrahydro-1H-cycloocta[f]indol-5(6H)-ylidene)-1-(2,4-dimethoxyphenyl)methanamine

To a solution of 2-((tert-butyldimethylsilyloxy)methyl)-1-methyl-7,8,9,10-tetrahydro-1H-cycloocta[f]indol-5(6H)-one (0.4216 g, 1.13 mmol) in DCM (3 mL) was added 2,4-dimethoxybenzylamine (0.20 mL, 1.33 mmol) and NEt₃ (0.45 mL, 3.23 mmol). The mixture was cooled to 0 °C, then a solution of TiCl₄ (1M DCM, 0.75 mL, 0.75 mmol) was added dropwise via syringe pump over 30 min. The reaction was allowed to warm to room temperature and stirred overnight. The mixture was diluted with DCM (10 mL) and then the reaction was quenched with NaHCO₃ (aqueous saturated, 5 mL). After vigorous shaking, the organic phase was separated using a PTFE phase separator, and dried over Na₂SO₄. The solvent was removed to provide the product (∼0.60 g, quant) as a yellow oil, which was used directly in the next step without further purification.

### Step 9: methyl 11-(((tert-butyldimethylsilyl)oxy)methyl)-1-(2,4-dimethoxybenzyl)-4-hydroxy-10-methyl-2-oxo-2,5,6,7,8,10-hexahydro-1H-pyrido [3',2':7,8] cycloocta[1,2-f]indole-3-carboxylate

Crude N-(2-((tert-butyldimethylsilyloxy)methyl)-1-methyl-7,8,9,10-tetrahydro-1H-cycloocta[f]indol-5(6H)-ylidene)-1-(2,4-dimethoxyphenyl)methanamine (∼0.60 g, 1.17 mmol) and trimethyl methanetricarboxylate (0.37 g, 1.95 mmol) were mixed together in Ph₂O (2.5 mL). With stirring, the mixture was placed onto a pre-heated heat block at 230 °C and heated for 10 min after the initial bubbling of MeOH was observed (occurs art ∼160 °C internal reaction temperature). The reaction mixture was cooled to room temperature, then purified by column chromatography (hexanes, followed by EtOAc/hexanes 0-60% gradient) to yield the product as a yellow foam (0.3750 g, 51%, 2 steps).
¹H NMR (500 MHz, CHCl₃-*d*) δ ppm 0.10 (s, 3 H) 0.11 (s, 3 H) 0.91 (s, 9 H) 1.33 - 1.51 (m, 3 H) 1.80 - 1.90 (m, 1 H) 2.02 (t, *J*=7.7 Hz, 1 H) 2.13 (t, *J*=12.0 Hz, 1 H) 2.55 (dd, *J*=13.6, 7.3 Hz, 1 H) 2.82 (dd, *J*=13.2, 7.6 Hz, 1 H) 3.10 (s, 3 H) 3.76 (s, 3 H) 3.77 (s, 3 H) 4.03 (s, 3 H) 4.80 (d, *J*=13.2 Hz, 1 H) 4.82 (d, *J*=13.2 Hz, 1 H) 4.96 - 5.26 (m, 2 H) 6.12 (d, *J*=2.5 Hz, 1 H) 6.24 (s, 1 H) 6.32 (dd, *J*=8.5, 2.2 Hz, 1 H) 6.81 (d, *J*=8.5 Hz, 1 H) 7.04 (s, 1 H) 7.11 (s, 1 H) 13.73 (s, 1 H). LC-MS 647.5 [M+H]⁺, RT 1.90 min.

### Step 10: methyl 1-(2,4-dimethoxybenzyl)-4-hydroxy-11-(hydroxymethyl)-10-methyl-2-oxo-2,5,6,7,8,10-hexahydro-1H-pyrido[3',2':7,8]cycloocta[1,2-f]indole-3-carboxylate

To a solution of methyl 11-(((tert-butyldimethylsilyl)oxy)methyl)-1-(2,4-dimethoxybenzyl)-4-hydroxy-10-methyl-2-oxo-2,5,6,7,8,10-hexahydro-1H-pyrido[3',2':7,8]cycloocta[1,2-f]indole-3-carboxylate (0.3750 g, 0.51 mmol) in THF (2 mL) was added TBAF solution (1M THF, 1.30 mL, 1.30 mmol). The reaction mixture was stirred at room temperature for 2 h until the starting material was completely consumed. The THF was removed and the residue was purified by column chromatography (EtOAc/DCM, 0-80% gradient). The product was obtained as a yellow solid (0.2587 g, 84 %).
¹H NMR (500 MHz, CHCl₃-*d*) δ ppm 1.30 - 1.53 (m, 3 H) 1.78 - 1.88 (m, 1 H) 2.03 (t, *J*=8.2 Hz, 1 H) 2.12 (t, *J*=12.3 Hz, 1 H) 2.55 (dd, *J*=13.6, 7.3 Hz, 1 H) 2.82 (dd, *J*=12.9, 7.3 Hz, 1 H) 3.12 (s, 3 H) 3.76 (s, 3 H) 3.81 (s, 3 H) 4.03 (s, 3 H) 4.81 (s, 2 H) 5.01 (d, *J*=15.4 Hz, 1 H) 5.18 (br. d, *J*=15.4 Hz, 1 H) 6.13 (d, *J*=2.4 Hz, 1 H) 6.32 (s, 1 H) 6.33 (dd, *J*=8.5, 2.4 Hz, 1 H) 6.80 (d, *J*=8.5 Hz, 1 H) 7.06 (s, 1 H) 7.14 (s, 1 H) 13.73 (br. s., 1 H). LC-MS 531.2 [M-H]⁻, 533.2 [M+H]⁺, RT 1.31 min.

### Step 11: 1-(2,4-dimethoxybenzyl)-4-hydroxy-11-(hydroxymethyl)-10-methyl-2-oxo-2,5,6,7,8,10-hexahydro-1H-pyrido[3',2':7,8]cycloocta[1,2-f]indole-3-carboxylic acid

To a suspension of methyl 1-(2,4-dimethoxybenzyl)-4-hydroxy-11-(hydroxymethyl)-10-methyl-2-oxo-2,5,6,7,8,10-hexahydro-1H-pyrido[3',2':7,8]cycloocta[1,2-f]indole-3-carboxylate (0.2587 g, 0.49 mmol) in EtOAc (2 mL) was added LiI (0.20 g, 1.49 mmol). The reaction mixture was stirred and heated at 60 °C for 1.5 h until complete consumption of starting material was observed. The mixture was then cooled to room temperature and acidified with aqueous HCl (1M, 3 mL). The product was extracted with DCM (3x10 mL), then the organic phase was washed with NaCl (aqueous saturated, 10 mL) and dried over Na₂SO₄. The solvent was removed to provide the product as a yellow solid (0.2517 g, ∼quant).
¹H NMR (500 MHz, DMSO-*d*₆) δ ppm 1.26 - 1.46 (m, 3 H) 1.84 (t, *J*=7.1 Hz, 1 H) 1.94 - 2.02 (m, 1 H) 2.08 (t, *J*=12.0 Hz, 1 H) 2.57 (dd, *J*=13.7, 7.4 Hz, 1 H) 2.77 (dd, *J*=12.9, 7.6 Hz, 1 H) 3.28 (s, 3 H) 3.69 (s, 3 H) 3.74 (s, 3 H) 4.62 (s, 2 H) 4.97 (d, *J*=15.8 Hz, 1 H) 5.11 (br. d, *J*=15.8 Hz, 1 H) 5.24 (br. s., 1 H) 6.28 (s, 1 H) 6.32 (d, *J*=2.5 Hz, 1 H) 6.35 (dd, *J*=8.4, 2.5 Hz, 1 H) 6.55 (d, *J*=8.4 Hz, 1 H) 7.30 (s, 1 H) 7.34 (s, 1 H) 13.90 (s, 1 H) 16.27 (s, 1 H). LC-MS 517.2 [M-H]⁻, 519.2 [M+H]⁺, RT 1.39 min.

### Step 12: 1-(2,4-dimethoxybenzyl)-11-formyl-4-hydroxy-10-methyl-2-oxo-2,5,6,7,8,10-hexahydro-1H-pyrido[3',2':7,8]cycloocta[1,2-f]indole-3-carboxylic acid

To a solution of 1-(2,4-dimethoxybenzyl)-4-hydroxy-11-(hydroxymethyl)-10-methyl-2-oxo-2,5,6,7,8,10-hexahydro-1H-pyrido[3',2':7,8]cycloocta[1,2-f]indole-3-carboxylic acid (0.2517 g, 0.49 mmol) in DCM (8 mL) was added activated MnO₂ (0.47 g+0.47 g+0.15 g, 4.87 mmol +4.87 mmol +1.55 mmol) in 3 portions at 30 min intervals. The reaction was monitored by LC/MS. After complete consumption of starting material MnO₂ was filtered and washed with DCM. The mother liquor was concentrated affording product as dark red foam (0.1831 g, 73%) which was used in the next step without further purification. LC-MS 515.2 [M-H]⁻, 517.2 [M+H]⁺, RT 1.53 min.

### Steps 13-14: 4-hydroxy-10-methyl-11-((methylamino)methyl)-2-oxo-2,5,6,7,8,10-hexahydro-1H-pyrido[3',2':7,8]cycloocta[1,2-f]indole-3-carboxylic acid hydrochloride

To a solution of 1-(2,4-dimethoxybenzyl)-11-formyl-4-hydroxy-10-methyl-2-oxo-2,5,6,7,8,10-hexahydro-1H-pyrido[3',2':7,8]cycloocta[1,2-f]indole-3-carboxylic acid (92.0 mg, 0.18 mmol) in dichloroethane (1.5 mL) was added MeNH₂ solution (2M THF, 0.27 mL, 0.54 mmol) followed by AcOH (20 µL, 0.33 mmol). After stirring at room temperature for 10 min, NaBH(OAc)₃ (70 mg, 0.33 mmol) was added. The reaction was stirred at room temperature ∼2 h and monitored by LC/MS until the starting aldehyde was completely consumed. The dichloroethane was then removed and the residue was dissolved in MeOH (5 mL) and several drops of TFA to generate a homogeneous mixture that was filtered through a PTFE micron filter and purified directly by preparative HPLC to provide the product as a TFA salt (56.0 mg, 49%).

To the product (56.0 mg, 0.09 mmol) obtained above was added i-Pr₃SiH (0.60 mL) followed by TFA (0.60 mL). The mixture was heated at 60 °C for 2 h and monitored by LC/MS. After complete consumption of starting material, the TFA was removed under reduced pressure. Addition of HCl solution (2M Et₂O, 1.0 mL) to the oily residue resulted in precipitate formation. The mixture was diluted with Et₂O and the resulting solid was filtered and washed with Et₂O. The product HCl salt was obtained as a colorless solid (27.6 mg, 70%).
¹H NMR (500 MHz, DMSO-*d*₆) δ ppm 1.28 - 1.65 (m, 3 H) 1.90 (br. s., 1 H) 2.12 (br. s., 1 H) 2.33 (t, *J*=12.5 Hz, 1 H) 2.62 (br. s., 3 H) 2.80 (dd, *J*=13.2, 7.9 Hz, 1 H) 2.97 (dd, *J*=13.2, 7.7 Hz, 1 H) 3.84 (s, 3 H) 4.41 (br. s., 2 H) 6.75 (s, 1 H) 7.54 (s, 1 H) 7.64 (s, 1 H) 9.31 (br. s., 2 H) 12.79 (s, 1 H) 13.91 (s, 1 H). LC-MS 380.1 [M-H]⁻, 382.2 [M+H]⁺, RT 0.85 min.

### Example 59

### 11-((ethylamino)methyl)-4-hydroxy-10-methyl-2-oxo-2,5,6,7,8,10-hexahydro-1H-pyrido[3',2':7,8]cycloocta[1,2-f]indole-3-carboxylic acid hydrochloride (Cpd 59)

To a solution of 1-(2,4-dimethoxybenzyl)-11-formyl-4-hydroxy-10-methyl-2-oxo-2,5,6,7,8,10-hexahydro-1H-pyrido[3',2':7,8]cycloocta[1,2-f]indole-3-carboxylic acid (Example 58, step 12, 88.6 mg, 0.17 mmol) in dichloroethane (1.5 mL) was added ethylamine hydrochloride (21 mg, 0.26 mmol) followed NEt₃ (36 µL, 0.26 mmol). The mixture was stirred for 5 min at room temperature, then AcOH (20 µL, 0.33 mmol) was added followed by an EtNH₂ solution (2M THF, 0.20 mL, 0.40 mmol). After stirring at room temperature for 5 min, NaBH(OAc)₃ (70 mg, 0.33 mmol) was added. The reaction was stirred at room temperature for ∼2 h and monitored by LC/MS until the starting aldehyde was completely consumed. The dichloroethane was then removed and the residue was dissolved in MeOH (6 mL) and several drops of TFA to generate a homogeneous mixture that was filtered through a PTFE micron filter and purified directly by preparative HPLC to provide the product as a TFA salt (84.7 mg, 75%).

To the product (84.7 mg, 0.13 mmol) obtained above was added i-Pr₃SiH (0.90 mL) followed by TFA (0.90 mL). The mixture was heated at 60 °C for 2 h and monitored by LC/MS. After complete consumption of starting material, the TFA was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a colorless solid (38.7 mg, 70%).
¹H NMR (500 MHz, DMSO-*d*₆) δ ppm 1.27 (t, *J*=7.3 Hz, 3 H) 1.33 - 1.53 (m, 3 H) 1.90 (br. s., 1 H) 2.13 (br. s., 1 H) 2.34 (t, *J*=12.9 Hz, 1 H) 2.80 (dd, *J*=13.2, 7.6 Hz, 1 H) 2.97 (dd, *J*=13.1, 7.7 Hz, 1 H) 3.08 (tq, *J*=7.6, 6.0 Hz, 2 H) 3.84 (s, 3 H) 4.42 (t, *J*=5.4 Hz, 2 H) 6.75 (s, 1 H) 7.54 (s, 1 H) 7.65 (s, 1 H) 9.14 (br. s., 2 H) 12.79 (s, 1 H) 13.91 (s, 1 H). LC-MS 394.8 [M-H]⁻, RT 0.88 min.

### Example 60

### 7-hydroxy-1-methyl-2-((methylamino)methyl)-9-oxo-1,4,5,6,9,10-hexahydropyrido[2',3':3,4]cyclohepta[1,2-e]indole-8-carboxylic acid hydrochloride (Cpd 60)

### Step 1: Mixture of 4-bromo-5-chloro-2-iodoaniline and 4-bromo-3-chloro-2-iodoaniline

To a solution on 4-bromo-3-chloroaniline (10.30 g, 49.89 mmol) in DCM (100 mL) was added AcOH (9 mL, 150 mmol) followed NIS (12.50 g, 55.60 mmol). The reaction was stirred overnight and then washed with NaHCO₃ (aqueous saturated, ∼100 mL) until the AcOH was neutralized. The organic layer was washed with NaCl (aqueous saturated, 50 mL) and dried over Na₂SO₄. The solvent was removed and the two resulting products (∼ 3 : 2 ratio by LC/MS) were separated by column chromatography (EtOAc/hexanes, 0-10% gradient). The 4-bromo-5-chloro-2-iodoaniline product (9.17 g, 55%) and the 4-bromo-3-chloro-2-iodoaniline product (6.25 g, 38%) were obtained as tan solids. The 4-bromo-3-chloro-2-iodoaniline was taken forward to the next step.
*4-bromo-3-chloro-2-iodoaniline: ¹*H NMR (500 MHz, CHCl₃-*d*) δ ppm 4.35 (br. s., 2 H) 6.53 (d, *J*=8.5 Hz, 1 H) 7.37 (d, *J*=8.5 Hz, 1 H). LC-MS 374.9/377.0 [M+H]⁺, RT 1.36 min.

### Step 2: 3-(6-amino-3-bromo-2-chlorophenyl)prop-2-yn-1-ol

To a solution of 4-bromo-3-chloro-2-iodoaniline (15.74 g, 47.36 mmol) in CH₃CN (50 mL) was added propargyl alcohol (3.40 mL, 57.01 mmol) and NEt₃ (13.2 mL, 94.71 mmol). The mixture was degassed with Argon, then Pd(PPh₃)₂Cl₂ (0.65 g, 0.93 mmol, 2 mol%) and CuI (0.36 g, 1.89 mmol, 2 mol%) were added. The reaction was heated at 70 °C for 2 h. LC/MS indicated ∼50% conversion. After cooling the mixture to room temperature, the CH₃CN was removed under reduced pressure. EtOAc (200 mL) was added to the residue and the resulting solid was filtered off and washed with EtOAc. After concentration of the mother liquor, the product (6.189 g, 50%) and starting material (6.131 g, 39%) were separated on the column (EtOAc/hexanes, 0-60% gradient). The recovered starting material was recycled two times using the described procedure to yield the desired product (9.48 g, 77% overall).
¹H NMR (500 MHz, DMSO-*d*₆) δ ppm 4.39 (d, *J*=6.0 Hz, 2 H) 5.34 (t, *J*=6.0 Hz, 1 H) 5.88 (br. s, 2 H) 6.61 (d, *J*=8.8 Hz, 1 H) 7.33 (d, *J*=8.8 Hz, 1 H). LC-MS 260.1/262.1 [M+H]⁺, RT 1.07 min.

### Step 3: (5-bromo-4-chloro-1H-indol-2-yl)methanol

To a solution of crude 3-(6-amino-3-bromo-2-chlorophenyl)prop-2-yn-1-ol (9.48 g, 36.39 mmol) in DMF (75 mL) was added t-BuOK (9.0 g, 80.21 mmol). The mixture was heated at 50 °C for 1 h under Argon. After cooling to 0 °C, the reaction was quenched by addition of NH₄Cl (aqueous saturated, 50 mL) and H₂O (150 mL). The product was extracted with EtOAc (3x100 mL) and the combined organics were washed with NaCl (aqueous saturated, 100 mL) and dried over Na₂SO₄. The solvent was removed and the residue was purified by column chromatography (EtOAc/hexanes, 0-50% gradient). The obtained product (10.20 g) contained some DMF and was used directly in the next step.
¹H NMR (500 MHz, DMSO-*d*₆) δ ppm 4.62 (d, *J*=5.7 Hz, 2 H) 5.41 (t, *J*=5.7 Hz, 1 H) 6.34 (s, 1 H) 7.27 (d, *J*=8.5 Hz, 1 H) 7.32 (d, *J*=8.5 Hz, 1 H) 11.58 (br. s., 3 H). LC-MS 260.1/262.0 [M+H]⁺, RT 1.14 min.

### Step 4: 5-bromo-2-((tert-butyldimethylsilyloxy)methyl)-4-chloro-1H-indole

To a solution of (5-bromo-4-chloro-1H-indol-2-yl)methanol obtained above (∼36.39 mmol) in DCM (125 mL) was added imidazole (2.6 g, 38.19 mmol). The mixture was cooled to 0 °C, then TBSCl (5.50 g 36.49 mmol) was added in portions. The reaction was stirred at room temperature for 30 and LC/MS indicated complete consumption of starting material. The mixture was washed with H₂O (100 mL), then the organic phase was separated, washed with NaCl (aqueous saturated, 50 mL) and dried over Na₂SO₄. The solvent was removed and the residue was purified by column chromatography (EtOAc/hexanes, 0-15% gradient) to afford the product (7.127 g, 52% 2 steps) as a solid.
¹H NMR (500 MHz, CHCl₃-*d*) δ ppm 0.13 (s, 6 H) 0.95 (s, 9 H) 4.89 (d, *J*=0.9 Hz, 2 H) 6.40 (dd, *J*=2.2, 0.9 Hz, 1 H) 7.16 (dd, *J*=8.5, 0.9 Hz, 1 H) 7.34 (d, *J*=8.5 Hz, 1 H) 8.48 (br. s., 1 H). LC-MS 374.2/376.2 [M+H]⁺, RT 1.78 min.

### Step 5: 5-bromo-2-((tert-butyldimethylsilyloxy)methyl)-4-chloro-1-methyl-1H-indole

To a solution of 5-bromo-2-((tert-butyldimethylsilyloxy)methyl)-4-chloro-1H-indole (7.10 g, 18.94 mmol) in DMF (55 mL) at 0 °C was added NaH (60%, 0.91 g, 22.75 mmol) in portions. After completion of the addition, the reaction mixture was allowed to warm to room temperature, stirred for 10 min then cooled to 0 °C and MeI (1.8 mL, 28.91 mmol) was added. The reaction mixture was allowed to warm to room temperature, stirred for 30 min then cooled to 0 °C and the reaction was quenched by addition of NH₄Cl (aqueous saturated). The precipitate that formed was filtered, then washed with H₂O, dried in an N₂ flow, and further purified by column chromatography (EtOAc/hexanes, 0-10% gradient) to afford the product (6.25 g, 85%) as a solid.
¹H NMR (500 MHz, CHCl₃-*d*) δ ppm 0.08 (s, 6 H) 0.91 (s, 9 H) 3.77 (s, 3 H) 4.82 (s, 2 H) 6.47 (s, 1 H) 7.09 (d, *J*=8.8 Hz, 1 H) 7.38 (d, *J*=8.8 Hz, 1 H). LC-MS 378.2/380.2 [M+H]⁺, RT 1.88 min.

### Step 6: 2-((tert-butyldimethylsilyloxy)methyl)-4-chloro-1-methyl-1H-indole-5-carbaldehyde

To a solution of 5-bromo-2-((tert-butyldimethylsilyloxy)methyl)-4-chloro-1-methyl-1H-indole (0.4933 g, 1.27 mmol) in THF (5 mL) at -78 °C was added n-BuLi solution (2.5M hexanes, 0.60 mL, 1.50 mmol) dropwise over ∼5 min. The reaction was stirred at -78 °C for 10 min, then DMF (0.25 mL, 3.21 mmol) was added. The mixture was stirred at -78 °C for 15 min and then the reaction was quenched by NH₄Cl (aqueous saturated, 5 mL). After warming the reaction to room temperature, the product was extracted with EtOAc (3x30 mL), then the organic phase was washed with NaCl (aqueous saturated, 30 mL) and dried over Na₂SO₄. The solvents were removed and the residue was purified by column chromatography (EtOAc/hexanes, 0-20% gradient) to afford the product (0.2744 g, 64%) as a solid.
¹H NMR (500 MHz, CHCl₃-*d*) δ ppm 0.10 (s, 6 H) 0.92 (s, 9 H) 3.83 (s, 3 H) 4.85 (s, 2 H) 6.66 (s, 1 H) 7.27 (d, *J*=8.5 Hz, 1 H) 7.81 (d, *J*=8.5 Hz, 1 H) 10.58 (s, 1 H). LC-MS 338.2/340.2 [M+H]⁺, RT 1.76 min.

### Step 7: 2-((tert-butyldimethylsilyloxy)methyl)-1-methyl-4-vinyl-1H-indole-5-carbaldehyde

2-((tert-butyldimethylsilyloxy)methyl)-4-chloro-1-methyl-1 H-indole-5-carbaldehyde (0.2744 g, 0.81 mmol), potassium vinyltrifluoroborate (0.22 g, 1.64 mmol), Pd(OAc)₂ (5.5 mg, 0.02 mmol, 3 mol%), S-Phos ligand (20 mg, 0.05 mmol, 6 mol%) and K₂CO₃ (0.34 g, 2.46 mmol) were mixed together in a vial. The vial was placed under vacuum and back filled with Argon, then dioxane (3.2 mL) and H₂O (0.5 mL) were added. The mixture was heated at 85 °C for 2 h and then cooled to room temperature. Water (5 mL) was added to the reaction mixture and the product was extracted with DCM (2x15 mL). The combined organics were washed with NaCl (aqueous saturated, 10 mL) and dried over Na₂SO₄. After concentration of the solvent, the residue was purified by column chromatography (EtOAc/hexanes, 0-10% gradient), affording the product as a white solid (0.170 g, 65 %).
¹H NMR (500 MHz, CHCl₃-*d*) δ ppm 0.09 (s, 6 H) 0.91 (s, 9 H) 3.83 (s, 3 H) 5.69 (dd, *J*=17.7, 1.6 Hz, 1 H) 5.79 (dd, *J*=11.2, 1.6 Hz, 1 H) 7.31 (d, *J*=8.5Hz, 1 H) 7.47 (dd, *J*=17.7, 11.2 Hz, 1 H) 7.81 (d, *J*=8.5 Hz, 1 H) 10.36 (s, 1 H). LC-MS 330.3 [M+H]⁺, RT 1.72 min.

### Step 8: 1-(2-((tert-butyldimethylsilyloxy)methyl)-4-vinyl-1H-indol-5-yl)pent-4-en-1-ol

To a solution of 2-((tert-butyldimethylsilyloxy)methyl)-1-methyl-4-vinyl-1H-indole-5-carbaldehyde (0.17 g, 0.52 mmol) in THF (1 mL) at -78 °C was added 3-butenylmagnesium bromide (0.5M in THF, 1.25 mL, 0.63 mmol) dropwise over ∼10 min. The reaction was stirred at -78 °C for 10 min, slowly allowed to warm to 0 °C, then the reaction was quenched by addition of NH₄Cl (aqueous saturated, 2 mL). The product was extracted with EtOAc (3x10 mL) and the combined organics were washed with NaCl (aqueous saturated, 50 mL) and dried over Na₂SO₄. The solvent was removed and the resulting product was purified by column chromatography (EtOAc/hexanes, 0-15% gradient), affording the product (0.1522 g, 77%) as an oil.
¹H NMR (500 MHz, CHCl₃-*d*) δ ppm 0.07 (s, 3 H) 0.08 (s, 3 H) 0.91 (s, 9 H) 1.81 - 1.91 (m, 1 H) 1.93 - 2.01 (m, 1 H) 2.07 - 2.17 (m, 1 H) 2.18 - 2.28 (m, 1 H) 3.79 (s, 3 H) 4.83 (s, 2 H) 4.97 (ddt, *J*=10.2, 2.1, 1.1 Hz, 1 H) 5.05 (dq, *J*=17.0, 1.7 Hz, 1 H) 5.16 (dd, *J*=7.9, 5.4 Hz, 1 H) 5.60 (dd, *J*=11.3, 1.9 Hz, 1 H) 5.68 (dd, *J*=17.7, 1.9 Hz, 1 H) 5.86 (ddt, *J*=17.0, 10.2,6.6 Hz, 1 H) 6.55 (s, 1 H) 7.13 (dd, *J*=17.7, 11.3 Hz, 1 H) 7.26 (d, *J*=8.5 Hz, 1 H) 7.39 (d, *J*=8.5 Hz, 1 H). LC-MS 386.3 [M+H]⁺, RT 1.78 min.

### Step 9: 2-((tert-butyldimethylsilyloxy)methyl)-3-methyl-3,6,7,8-tetrahydrocyclohepta[e]indol-6-ol

To a solution of 1-(2-((tert-butyldimethylsilyloxy)methyl)-4-vinyl-1H-indol-5-yl)pent-4-en-1-ol (1.668 g, 4.32 mmol) in toluene (170 mL, 0.025M) under Argon was added a second generation Grubbs' catalyst (186 mg, 0.22 mmol, 5 mol%). The reaction mixture was heated at 75 °C for 40 min until the starting material was completely consumed as indicated by LC/MS. After cooling the reaction to room temperature, the toluene was removed under reduced pressure and the residue was purified by column chromatography (EtOAc/hexanes, 0-20% gradient). The product (0.964 g, 62%) was obtained as a solid.
¹H NMR (500 MHz, CHCl₃-*d*) δ ppm 0.08 (s, 6 H) 0.91 (s, 9 H) 1.91 (br. s., 1 H) 2.11 - 2.20 (m, 1 H) 2.25 - 2.36 (m, 1 H) 2.45 - 2.54 (m, 1 H) 2.60 - 2.73 (m, 1 H) 3.78 (s, 3 H) 4.84 (s, 2 H) 5.05 (br. d, *J*=7.6 Hz, 1 H) 6.12 (ddd, *J*=12.0, 5.4, 3.8 Hz, 1 H) 6.50 (s, 1 H) 6.90 (dt, *J*=12.0, 1.8 Hz, 1 H) 7.17 (d, *J*=8.2 Hz, 1 H) 7.29 (d, *J*=8.2 Hz, 1 H). LC-MS 358.3 [M+H]⁺, RT 1.68 min.

### Step 10: 2-((tert-butyldimethylsilyloxy)methyl)-3-methyl-3,6,7,8,9,10-hexahydrocyclohepta[e]indol-6-ol

A solution of 2-((tert-butyldimethylsilyloxy)methyl)-3-methyl-3,6,7,8-tetrahydrocyclohepta[e]indol-6-ol (0.964 g, 2.70 mmol) in EtOAc (10 mL) was hydrogenated over 10% Pd/C (Degussa type, 100 mg) under H₂ (1 atm) until complete consumption of starting material as indicated by TLC (2x 10%EtOAc/hexanes). After ∼1 h, the catalyst was filtered off and the filtrate was washed with EtOAc. The mother liquor was concentrated and the residue was purified by column chromatography (EtOAc/hexanes, 0-20% gradient). The product (0.808 g, 83 %) was obtained as a solid.
¹H NMR (500 MHz, CHCl₃-*d*) δ ppm 0.08 (s, 3 H) 0.09 (s, 3 H) 0.91 (s, 9 H) 1.60 - 1.68 (m, 1 H) 1.70 - 1.98 (m, 4 H) 2.09 - 2.20 (m, 1 H) 2.93 (ddd, *J*=14.4, 9.5, 1.6 Hz, 1 H) 3.26 (ddd, *J*=14.4, 9.1, 1.9 Hz, 1 H) 3.76 (s, 3 H) 4.83 (s, 2 H) 5.07 (dd, *J*=6.8, 3.0 Hz, 1 H) 6.42 (s, 1 H) 7.12 (d, *J*=8.2 Hz, 1 H) 7.30 (d, *J*=8.2 Hz, 1 H). LC-MS 360.3 [M+H]⁺, RT 1.72 min.

### Step 11: 2-((tert-butyldimethylsilyloxy)methyl)-3-methyl-7,8,9,10-tetrahydrocyclohepta[e]indol-6(3H)-one

To a solution of 2-((tert-butyldimethylsilyloxy)methyl)-3-methyl-3,6,7,8,9,10-hexahydrocyclohepta[e]indol-6-ol (0.7911 g, 2.20 mmol) in DCM (50 mL) at 0 °C was added solid NaHCO₃ (1.90 g, 22.61 mmol) followed by Dess-Martin periodinane (1.20 g, 2.83 mmol). The reaction was stirred at 0 °C and monitored by TLC and LC/MS. After complete conversion of starting material, the reaction mixture was diluted with DCM, washed with NaHCO₃ (aqueous saturated, 10 mL), Na₂S₂O₃ (10% aq, 10 mL) and NaCl (aqueous saturated, 10 mL). The organic phase was dried over Na₂SO₄. The solvent was removed and the resulting residue was purified by column chromatography (EtOAc/hexanes, 0-15% gradient), affording the desired product (0.5687 g, 72%) as a colorless solid.
¹H NMR (500 MHz, CHCl₃-*d*) δ ppm 0.10 (s, 6 H) 0.92 (s, 9 H) 1.77 - 1.89 (m, 2 H) 1.91 - 1.99 (m, 2 H) 2.80 (t, *J*=6.0 Hz, 0 H) 3.21 (t, *J*=6.6 Hz, 2 H) 3.80 (s, 3 H) 4.84 (s, 2 H) 6.55 (s, 1 H) 7.21 (d, *J*=8.2 Hz, 1 H) 7.74 (d, *J*=8.2 Hz, 1 H). LC-MS 358.3 [M+H]⁺, RT 1.75 min.

### Step 12: N-(2-((tert-butyldimethylsilyloxy)methyl)-3-methyl-7,8,9,10-tetrahydrocyclohepta[e]indol-6(3H)-ylidene)-1-(2,4-dimethoxyphenyl)methanamine

To a solution of 2-((tert-butyldimethylsilyloxy)methyl)-3-methyl-7,8,9,10-tetrahydrocyclohepta[e]indol-6(3H)-one (0.6187 g, 1.73 mmol) in DCM (5 mL) was added 2,4-dimethoxybenzylamine (0.30 mL, 2.00 mmol) and NEt₃ (0.70 mL, 5.02 mmol). The mixture was cooled to 0 °C, then a solution of TiCl₄ (1M DCM, 1.15 mL, 1.15 mmol) was added dropwise via syringe pump over 30 min. The reaction was allowed to warm to room temperature and stirred overnight. The mixture was diluted with DCM (10 mL) and then the reaction was quenched with NaHCO₃ (aqueous saturated, 5 mL). After vigorous shaking, the organic phase was separated using a PTFE phase separator, and dried over Na₂SO₄. The solvent was removed to provide the resulting product (0.858 g, -quant) as a yellow oil, which was used directly in the next step without further purification.

### Step 13: methyl 2-(((tert-butyldimethylsilyl)oxy)methyl)-10-(2,4-dimethoxybenzyl)-7-hydroxy-1-methyl-9-oxo-1,4,5,6,9,10-hexahydropyrido[2',3':3,4]cyclohepta[1,2-e]indole-8-carboxylate

Crude *N-*(2-((tert-butyldimethylsilyloxy)methyl)-3-methyl-7,8,9,10-tetrahydrocyclohepta[e]indol-6(3H)-ylidene)-1-(2,4-dimethoxyphenyl)methanamine (0.858 g, 1.69 mmol) and trimethyl methanetricarboxylate (0.55 g, 2.89 mmol) were mixed together in Ph₂O (4 mL). With stirring, the mixture was placed onto a pre-heated heat block at 230 °C and heated for 10 min after the initial bubbling of MeOH was observed (occurs art ∼160 °C internal reaction temperature). The reaction mixture was cooled to room temperature, then purified by column chromatography (hexanes followed by EtOAc/hexanes 0-70% gradient) to yield the product as a yellow foam (0.5425 g, 50%, 2 steps).
¹H NMR (500 MHz, CHCl₃-*d*) δ ppm 0.09 (s, 3 H) 0.12 (s, 3 H) 0.92 (s, 9 H) 1.49 (td, *J*=13.6, 7.3 Hz, 1 H) 1.97 - 2.17 (m, 2 H) 2.41 (td, *J*=12.9, 7.9 Hz, 1 H) 2.99 (dd, *J*=13.9, 6.0 Hz, 2 H) 3.48 (s, 3 H) 3.75 (s, 3 H) 3.78 (s, 3 H) 3.99 (s, 3 H) 4.84 (d, *J*=13.2 Hz, 1 H) 4.84 (d, *J*=13.2 Hz, 1 H) 5.18 (br. s., 2 H) 6.27 (d, *J*=2.4 Hz, 1 H) 6.35 (dd, *J*=8.2, 2.4 Hz, 1 H) 6.45 (s, 1 H) 6.82 (d, *J*=8.2 Hz, 1 H) 7.00 (d, *J*=8.8 Hz, 1 H) 7.11 (d, *J*=8.8 Hz, 1 H) 13.68 (s, 1 H). LC-MS 633.5 [M+H]⁺, RT 1.85 min.

### Step 14: methyl lo-(2,4-dimethoxybenzyl)-7-hydroxy-2-(hydroxymethyl)-1-methyl-9-oxo-1,4,5,6,9,10-hexahydropyrido [2',3':3,4] cyclohepta[1,2-e] indole-8-carboxylate

To a solution of methyl 2-(((tert-butyldimethylsilyl)oxy)methyl)-10-(2,4-dimethoxybenzyl)-7-hydroxy-1-methyl-9-oxo-1,4,5,6,9,10-hexahydropyrido[2',3':3,4]cyclohepta[1,2-e]indole-8-carboxylate (0.5424 g, 0.86 mmol) in THF (3 mL) was added TBAF solution (1M THF, 2.40 mL, 2.40 mmol). The reaction mixture was stirred at room temperature for 1 h until the starting material was completely consumed. The THF was removed and the residue was purified by column chromatography (EtOAc/DCM, 0-80% gradient). The product was obtained as a yellow solid (0.4048 g, 91 %).
¹H NMR (500 MHz, CHCl₃-*d*) δ ppm 1.49 (dt, *J*=13.6, 6.8 Hz, 1 H) 1.96 - 2.18 (m, 2 H) 2.42 (td, *J*=12.8, 7.7 Hz, 1 H) 2.99 (dt, *J*=13.6, 4.5 Hz, 2 H) 3.49 (s, 3 H) 3.75 (s, 3 H) 3.81 (s, 3 H) 3.99 (s, 3 H) 4.83 (s, 2 H) 5.17 (br. s, 2 H) 6.28 (d, *J*=2.4 Hz, 1 H) 6.36 (dd, *J*=8.4,2.4 Hz, 1 H) 6.54 (s, 1 H) 6.82 (d, *J*=8.4 Hz, 1 H) 7.02 (d, *J*=8.8 Hz, 1 H) 7.13 (d, *J*=8.8 Hz, 1 H) 13.68 (br. s., 1 H). LC-MS 517.3 [M-H]⁻, 519.2 [M+H]⁺, RT 1.22 min.

### Step 15: 10-(2,4-dimethoxybenzyl)-7-hydroxy-2-(hydroxymethyl)-1-methyl-9-oxo-1,4,5,6,9,10-hexahydropyrido[2',3':3,4]cyclohepta[1,2-e]indole-8-carboxylic acid

To a suspension of methyl 10-(2,4-dimethoxybenzyl)-7-hydroxy-2-(hydroxymethyl)-1-methyl-9-oxo-1,4,5,6,9,10-hexahydropyrido[2',3':3,4]cyclohepta[1,2-e]indole-8-carboxylate (0.4048 g, 0.78 mmol) in EtOAc (2.5 mL) was added LiI (0.31 g, 2.32 mmol). The reaction mixture was stirred and heated at 60 °C for 1.5 h until complete consumption of starting material was observed. The mixture was then cooled to room temperature and acidified with aqueous HCl (1M, 3 mL). The product was extracted with DCM (3x10 mL), then the organic phase was washed with NaCl (aqueous saturated, 10 mL) and dried over Na₂SO₄. The solvent was removed and the resulting product was obtained as a yellow solid (0.3814 g, 97%).
¹H NMR (500 MHz, DMSO-*d*₆) δ ppm 1.41 (td, *J*=13.5, 7.1 Hz, 1 H) 1.86 - 2.15 (m, 2 H) 2.38 (td, *J*=12.8, 8.0 Hz, 1 H) 2.88 (dd, *J*=113.6, 5.7 Hz, 1 H) 3.01 (dd, *J*=113.6, 6.0 Hz, 1 H) 3.33 (br. s., 1 H) 3.53 (s, 3 H) 3.69 (s, 3 H) 3.75 (s, 3 H) 4.64 (s, 2 H) 5.12 - 5.32 (m, 2 H) 6.37 (dd, *J*=8.4, 2.4 Hz, 1 H) 6.43 (d, *J*=2.4 Hz, 1 H) 6.57 (s, 1 H) 6.64 (d, *J*=8.4 Hz, 1 H) 7.10 (d, *J*=8.5 Hz, 1 H) 7.36 (d, *J*=8.5 Hz, 1 H) 13.82 (s, 1 H) 16.17 (s, 1 H). LC-MS 503.1 [M-H]⁻, 505.2 [M+H]⁺, RT 1.33 min.

### Step 16: 10-(2,4-dimethoxybenzyl)-2-formyl-7-hydroxy-1-methyl-9-oxo-1,4,5,6,9,10-hexahydropyrido[2',3':3,4]cyclohepta[1,2-e]indole-8-carboxylic acid

To a solution of 10-(2,4-dimethoxybenzyl)-7-hydroxy-2-(hydroxymethyl)-1-methyl-9-oxo-1,4,5,6,9,10-hexahydropyrido[2',3':3,4]cyclohepta[1,2-e]indole-8-carboxylic acid (0.3814 g, 0.76 mmol) in DCM (10 mL) was added activated MnO₂ (0.74 g+0.74 g+0.37 g, 7.66 mmol +7.66 mmol +3.83 mmol) in 3 portions at 30 min intervals. The reaction was monitored by LC/MS. After complete consumption of starting material MnO₂ was filtered and washed with DCM. The mother liquor was concentrated affording product as dark red foam (0.2742 g, 72%) which was used in the next step without further purification. LC-MS 501.1 [M-H]⁻, RT 1.47 min.

### Steps 17-18: 7-hydroxy-1-methyl-2-((methylamino)methyl)-9-oxo-1,4,5,6,9,10-hexahydropyrido[2',3':3,4]cyclohepta[1,2-e]indole-8-carboxylic acid hydrochloride

To a solution of 10-(2,4-dimethoxybenzyl)-2-formyl-7-hydroxy-1-methyl-9-oxo-1,4,5,6,9,10-hexahydropyrido[2',3':3,4]cyclohepta[1,2-e]indole-8-carboxylic acid (139.1 mg, 0.28 mmol) in dichloroethane (2.2 mL) was added MeNH₂ solution (2M THF, 0.42 mL, 0.84 mmol) followed by AcOH (30 µL, 0.50 mmol). After stirring at room temperature for 10 min, NaBH(OAc)₃ (105 mg, 0.50 mmol) was added. The reaction was stirred at room temperature ∼2 h and monitored by LC/MS until the starting aldehyde was completely consumed. The dichloroethane was then removed and the residue was dissolved in MeOH (6 mL) and several drops of TFA to generate a homogeneous mixture that was filtered through a PTFE micron filter and purified directly by preparative HPLC to provide the product as a TFA salt (94.2 mg, 54%).

To the product (94.2 mg, 0.15 mmol) obtained above was added i-Pr₃SiH (0.90 mL) followed by TFA (0.90 mL). The mixture was heated at 60 °C for 2 h and monitored by LC/MS. After complete consumption of starting material, the TFA was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a colorless solid (45.2 mg, 75%).
¹H NMR (500 MHz, DMSO-*d*₆) δ ppm 2.08 - 2.35 (m, 4 H) 2.64 (br. s., 3 H) 2.84 (br. s., 2 H) 3.86 (s, 3 H) 4.43 (br. s., 2 H) 6.92 (s, 1 H) 7.38 (d, *J*=8.5 Hz, 1 H) 7.56 (d, *J*=8.5 Hz, 1 H) 9.25 (br. s., 2 H) 12.90 (br. s., 1 H) 13.87 (s, 1 H). LC-MS 366.1 [M-H]⁻, 368.2 [M+H]⁺, RT 0.76 min.

### Example 61

### 2-((ethylamino)methyl)-7-hydroxy-1-methyl-9-oxo-1,4,5,6,9,10-hexahydropyrido[2',3':3,4]cyclohepta[1,2-e]indole-8-carboxylic acid hydrochloride (Cpd 61)

To a solution of 10-(2,4-dimethoxybenzyl)-2-formyl-7-hydroxy-1-methyl-9-oxo-1,4,5,6,9,10-hexahydropyrido[2',3':3,4]cyclohepta[1,2-e]indole-8-carboxylic acid (from Example 60, Step 16: 132.0 mg, 0.28 mmol) in dichloroethane (2.2 mL) was added ethylamine hydrochloride (32 mg, 0.39 mmol) followed NEt₃ (55 µL, 0.39 mmol). The mixture was stirred for 5 min at room temperature, then AcOH (30 µL, 0.50 mmol) was added followed by an EtNH₂ solution (2M THF, 0.25 mL, 0.50 mmol). After stirring at room temperature for 5 min, NaBH(OAc)₃ (100 mg, 0.47 mmol) was added. The reaction was stirred at room temperature for ∼2 h and monitored by LC/MS until the starting aldehyde was completely consumed. The dichloroethane was then removed and the residue was dissolved in MeOH (6 mL) and several drops of TFA to generate a homogeneous mixture that was filtered through a PTFE micron filter and purified directly by preparative HPLC to provide the product as a TFA salt (124.3 mg, 73%).

To the product (124.3 mg, 0.19 mmol) obtained above was added i-Pr₃SiH (1.20 mL) followed by TFA (1.20 mL). The mixture was heated at 60 °C for 2 h and monitored by LC/MS. After complete consumption of starting material, the TFA was removed under reduced pressure. Addition of an HCl solution (2M Et₂O, 1.5 mL) to the oily residue resulted in formation of a precipitate. The mixture was diluted with Et₂O and the resulting solid was filtered and washed with Et₂O. The product HCl salt was obtained as a colorless solid (59.9 mg, 74%).
¹H NMR (500 MHz, DMSO-*d*₆) δ ppm 1.28 (t, *J*=7.3 Hz, 3 H) 2.12 - 2.35 (m, 4 H) 2.84 (br. s., 2 H) 3.01 - 3.16 (m, 2 H) 3.86 (s, 3 H) 4.43 (t, *J*=5.0 Hz, 2 H) 6.93 (s, 1 H) 7.38 (d, *J*=8.5 Hz, 1 H) 7.56 (d, *J*=8.5 Hz, 1 H) 9.21 (br. s., 2 H) 12.89 (s, 1 H) 13.87 (s, 1 H). LC-MS 380.1 [M-H]⁻, 382.2 [M+H]⁺, RT 0.78 min.

### Example 62

### 4-hydroxy-7,9-dimethyl-10-((methylamino)methyl)-2-oxo-1,2,5,6,7,9-hexahydropyrido[3',2':6,7]cyclohepta[1,2-f]indole-3-carboxylic acid hydrochloride (Cpd 62)

### Step 1: 2-(((tert-butyldimethylsilyl)oxy)methyl)-1-methyl-6-(prop-1-en-2-yl)-1H-indole-5-carbaldehyde

2-((tert-butyldimethylsilyloxy)methyl)-6-chloro-1-methyl-1H-indole-5-carbaldehyde (Example 19, step 5, 4.94 g, 14.61 mmol), potassium triftuoro(prop-1-en-2-yl)borate (3.25 g, 21.96 mmol), Pd(OAc)₂ (100 mg, 0.45 mmol, 3 mol%), S-Phos ligand (360 mg, 0.88 mmol, 6 mol%) and K₂CO₃ (6.10 g, 44.14 mmol) were mixed together in a 250 mL round bottom flask. The flask was placed under vacuum and back filled with Argon, then dioxane (60 mL) and H₂O (10 mL) were added. The mixture was heated at 85-90 °C for 3 h, then additional Pd(OAc)₂ (100 mg, 0.45 mmol, 3 mol%) was added. The reaction was heated at 85-90 °C and monitored by LC/MS for complete consumption of starting material (∼3 h). The reaction was cooled to room temperature, then water (50 mL) was added and the product was extracted with DCM (4x90 mL). The combined organics were washed with NaCl (aqueous saturated, 80 mL) and dried over Na₂SO₄. After concentration of the solvent, the residue was purified by column chromatography (EtOAc/hexanes, 0-15% gradient), affording the product as a white solid (4.72 g, 89 %).
¹H NMR (500 MHz, CHCl₃-*d*) δ ppm 0.08 (s, 6 H) 0.91 (s, 9 H) 2.23 (dd, *J*=1.6, 0.9 Hz, 3 H) 3.81 (s, 3 H) 4.83 (s, 2 H) 4.97 (dd, *J*=1.6, 0.9 Hz, 1 H) 5.41 (quin, *J*=1.6 Hz, 1 H) 6.50 (s, 1 H) 7.16 (s, 1 H) 8.22 (s, 1 H) 10.21 (s, 1 H). LC-MS 344.2 [M+H]⁺, RT 1.78 min.

### Step 2: 1-(2-(((tert-butyldimethylsilyl)oxy)methyl)-1-methyl-6-(prop-1-en-2-yl)-1H-indol-5-yl)pent-4-en-1-ol

To a solution of 2-(((tert-butyldimethylsilyl)oxy)methyl)-1-methyl-6-(prop-1-en-2-yl)-1H-indole-5-carbaldehyde (4.70 g, 13.68 mmol) in THF (30 mL) at -78 °C was added 3-butenylmagnesium bromide (0.5M in THF, 33.0 mL, 16.5 mmol) dropwise over ∼10 min. The reaction mixture was stirred at -78 °C for 10 min and slowly allowed to warm to -10 °C, then the reaction was quenched by addition of NH₄Cl (aqueous saturated, 60 mL). The product was extracted with EtOAc (4x100 mL) and the combined organics were washed with NaCl (aqueous saturated, 80 mL) and dried over Na₂SO₄. The solvent was removed and the resulting 1-(2-(((tert-butyldimethylsilyl)oxy)methyl)-1-methyl-6-(prop-1-en-2-yl)-1H-indol-5-yl)pent-4-en-1-ol was obtained as pale-yellow solid (5.50 g) in quantitative yield. The product was used directly in the next step without further purification.
¹H NMR (500 MHz, Acetone) δ ppm 0.09 (s, 3 H) 0.10 (s, 3 H) 0.91 (s, 9 H) 1.57 - 1.88 (m, 3 H) 2.11 (t, *J*=1.3 Hz, 3 H) 2.15 - 2.30 (m, 1 H) 3.79 (s, 3 H) 4.85 (dd, *J*=2.4, 0.8 Hz, 1 H) 4.87 - 4.89 (m, 1 H) 4.90 (s, 2 H) 4.92 - 5.01 (m, 2 H) 5.22 (dq, *J*=2.4, 1.3 Hz, 1 H) 5.85 (ddt, *J*=17.1, 10.4, 6.6 Hz, 1 H) 6.37 (s, 1 H) 7.06 (s, 1 H) 7.70 (s, 1 H).

### Step 3: 1-(2-(((tert-butyldimethylsilyl)oxy)methyl)-1-methyl-6-(prop-1-en-2-yl)-1H-indol-5-yl)pent-4-en-1-one

To activated 4Å molecular sieves (3.1 g, 250 mg/mmol) was added solution of 1-(2-(((tert-butyldimethylsilyl)oxy)methyl)-1-methyl-6-(prop-1-en-2-yl)-1H-indo 1-5-yl)pent-4-en-1-ol (4.93 g, 12.34 mmol) in DCM (60 mL). The mixture was cooled to 0 °C, then NMO (2.20 g, 18.77 mmol) and TPAP (220 mg, 0.63 mmol, 5 mol%) were added. The reaction was stirred at 0 °C and monitored by LC/MS. After complete consumption of starting material (∼1.5 h), the molecular sieves were filtered off and washed with DCM. The mother liquor was concentrated and the residue was purified by column chromatography (EtOAc/hexanes, 0-10% gradient). The product was obtained as a white crystalline solid (4.33 g, 88%).
¹H NMR (500 MHz, Acetone) δ ppm 0.11 (s, 6 H) 0.91 (s, 9 H) 2.14 (dd, *J*=1.4, 0.8 Hz, 3 H) 2.32 - 2.43 (m, 2 H) 2.96 (t, *J*=6.9 Hz, 2 H) 3.86 (s, 3 H) 4.80 (dd, *J*=2.2, 0.8 Hz, 1 H) 4.91 - 4.96 (m, 1 H) 4.93 (s, 2 H) 5.04 (dq, *J*=17.2, 1.7 Hz, 3 H) 5.09 (dq, *J*=2.2, 1.4 Hz, 3 H) 5.89 (ddt, *J*=17.2, 10.4, 6.6 Hz, 3 H) 6.51 (s, 1 H) 7.30 (s, 1 H) 7.82 (s, 1 H). LC-MS 398.3 [M+H]⁺, RT 1.75 min.

### Step 4: 2-(((tert-butyldimethylsilyl)oxy)methyl)-1,9-dimethyl-6,7-dihydrocyclohepta[f]indol-5(1H)-one

To the solution of 1-(2-(((tert-butyldimethylsilyl)oxy)methyl)-1-methyl-6-(prop-1-en-2-yl)-1H-indol-5-yl)pent-4-en-1-one (3.80 g, 9.57 mmol) degassed with Argon in toluene (190 mL, 0.05M) was added a second generation Grubbs' catalyst (325 mg, 0.38 mmol, 4 mol%). The reaction mixture was heated at 60 °C for 1.5 h and the second portion of the Grubbs-II catalyst (160 mg, 0.19 mmol, 2 mol%) was added, then heating was continued for another 2 h. The toluene was removed and the residue was purified by column chromatography (EtOAc/hexanes, 0-15% gradient) to separate the starting material (0.80 g) and the product 2-(((tert-butyldimethylsilyl)oxy)methyl)-1,9-dimethyl-6,7-dihydrocyclohepta[f]indol-5(1H)-one (1.52 g, 55% BORSM). The recovered starting material was subsequently reacted to obtain additional product (0.50 g).
¹H NMR (500 MHz, CHCl₃-*d*) δ ppm 0.07 (s, 6 H) 0.90 (s, 9 H) 2.21 (s, 3 H) 2.27 (q, *J*=6.4 Hz, 2 H) 2.88 (t, *J*=6.4 Hz, 2 H) 3.81 (s, 3 H) 4.83 (s, 2 H) 6.12 (td, *J*=7.2, 1.4 Hz, 1 H) 6.44 (s, 1 H) 7.23 (s, 1 H) 7.92 (s, 1 H). LC-MS 370.3 [M+H]⁺, RT 1.66 min.

### Step 5: 2-(((tert-butyldimethylsilyl)oxy)methyl)-1,9-dimethyl-6,7,8,9-tetrahydrocyclohepta[f]indol-5(1H)-one

A solution of 2-(((tert-butyldimethylsilyl)oxy)methyl)-1,9-dimethyl-6,7-dihydrocyclohepta[f]indol-5(1H)-one (2.14 g, 5.80 mmol) in EtOAc (60 mL) and DCM (5 mL) was hydrogenated over Pd/C (10%, 310 mg) under H₂ (1 atm) until complete consumption of starting material was indicated by TLC. After ∼5 h, the catalyst was filtered off and the filtrate was washed with EtOAc. The mother liquor was concentrated and the product was purified by column chromatography (EtOAc/hexanes, 0-15% gradient) to yield 2-(((tert-butyldimethylsilyl)oxy)methyl)-1,9-dimethyl-6,7,8,9-tetrahydrocyclohepta[f]indol-5(1H)-one (1.35 g, 63%) as a white solid.
¹H NMR (500 MHz, CHCl₃-*d*) δ ppm 0.06 (s, 3 H) 0.08 (s, 3 H) 0.90 (s, 9 H) 1.49 (d, *J*=6.6 Hz, 3 H) 1.50 - 1.66 (m, 2 H) 1.80 - 1.92 (m, 1 H) 1.92 - 2.08 (m, 1 H) 2.64 (ddd, *J*=18.6, 12.6, 2.2 Hz, 1 H) 2.75 (ddd, *J*=18.6, 5.5, 2.0 Hz, 1 H) 3.15 - 3.31 (m, 1 H) 3.81 (s, 3 H) 4.82 (s, 2 H) 6.42 (s, 1 H) 7.12 (s, 1 H) 7.86 (s, 1 H). LC-MS 372.3 [M+H]⁺, RT 1.78 min.

### Step 6: N-(2-(((tert-butyldimethylsilyl)oxy)methyl)-1,9-dimethyl-6,7,8,9-tetrahydrocyclohepta[f]indol-5(1H)-ylidene)-1-(2,4-dimethoxyphenyl)methanamine

To a solution of 2-(((tert-butyldimethylsilyl)oxy)methyl)-1,9-dimethyl-6,7,8,9-tetrahydrocyclohepta[f]indol-5(1H)-one (1.35 g, 3.63 mmol) in DCM (11 mL) was added 2,4-dimethoxybenzylamine (0.60 mL, 4.00 mmol) and NEt₃ (1.50 mL, 10.76 mmol). The mixture was cooled to 0 °C, then a solution of TiCl₄ (1M DCM, 2.40 mL, 2.40 mmol) was added dropwise via syringe pump over 30 min. The reaction was allowed to warm to room temperature and stirred overnight. The mixture was diluted with DCM (50 mL) and then the reaction was quenched with NaHCO₃ (aqueous saturated, 20 mL). After vigorous shaking, the organic phase was separated using a PTFE phase separator and dried over Na₂SO₄. The solvent was removed to afford the desired product (1.9 g, quant) as a yellow oil, which was used directly in the next step without further purification.

### Step 7: methyl 10-(((tert-butyldimethylsilyl)oxy)methyl)-1-(2,4-dimethoxybenzyl)-4-hydroxy-7,9-dimethyl-2-oxo-1,2,5,6,7,9-hexahydropyrido[3',2':6,7]cyclohepta[1,2-f]indole-3-carboxylate

Crude N-(2-(((tert-butyldimethylsilyl)oxy)methyl)-1,9-dimethyl-6,7,8,9-tetrahydrocyclohepta[f]indol-5(1H)-ylidene)-1-(2,4-dimethoxyphenyl)methanamine (*ca* 3.63 mmol) and trimethyl methanetricarboxylate (1.20 g, 6.31 mmol) were mixed together in Ph₂O (7 mL). With stirring, the mixture was placed onto a pre-heated heat block at 230 °C and heated for 10 min after the initial bubbling of MeOH was observed (occurs art ∼160 °C internal reaction temperature). The reaction mixture was cooled to room temperature, then purified by column chromatography (hexanes followed by EtOAc/hexanes 0-80% gradient) to yield the product as a yellow foam (1.41 g, 60% 2 steps).
¹H NMR (500 MHz, CHCl₃-*d*) δ ppm 0.09 (s, 3 H) 0.10 (s, 3 H) 0.91 (s, 9 H) 1.23 (d, *J*=6.9 Hz, 3 H) 1.37 - 1.52 (m, 2 H) 2.00 - 2.12 (m, 1 H) 2.33 - 2.47 (m, 1 H) 2.89 (dd, *J*=13.4, 5.5 Hz, 1 H) 3.21 (s, 3 H) 3.74 (s, 3 H) 3.80 (s, 3 H) 4.02 (s, 3 H) 4.82 (s, 2 H) 5.09 (d, *J*=15.4 Hz, 1 H) 5.39 - 5.58 (m, 1 H) 6.14 (d, *J*=1.9 Hz, 1 H) 6.27 (dd, *J*=8.4, 1.9 Hz, 1 H) 6.31 (s, 1 H) 6.69 (d, *J*=8.4 Hz, 1 H) 7.04 (s, 1 H) 7.35 (s, 1 H) 13.64 (s, 1 H) LC-MS 647.4 [M+H]⁺, RT 1.92 min

### Step 8: methyl 1-(2,4-dimethoxybenzyl)-4-hydroxy-10-(hydroxymethyl)-7,9-dimethyl-2-oxo-1,2,5,6,7,9-hexahydropyrido [3',2': 6,7] cyclohepta[1,2-f]indole-3-carboxylate

To a solution of methyl 10-(((tert-butyldimethylsilyl)oxy)methyl)-1-(2,4-dimethoxybenzyl)-4-hydroxy-7,9-dimethyl-2-oxo-1,2,5,6,7,9-hexahydropyrido[3',2':6,7]cyclohepta[1,2-f]indole-3-carboxylate (1.40 g, 2.16 mmol) in THF (9 mL) was added TBAF solution (1M THF, 5.4 mL, 5.4 mmol). The reaction mixture was stirred at room temperature for 2 h until the starting material was completely consumed. The THF was removed and the residue was purified by column chromatography (EtOAc/DCM, 0-100% gradient). The product was obtained as a yellow solid (1.11 g, 95 %).
¹H NMR (500 MHz, CHCl₃-*d*) δ ppm 1.24 (d, *J*=6.9 Hz, 3 H) 1.36 - 1.48 (m, 2 H) 2.05 - 2.11 (m, 1 H) 2.34 - 2.46 (m, 1 H) 2.90 (dd, *J*=13.9, 6.6 Hz, 1 H) 3.23 (s, 3 H) 3.74 (s, 3 H) 3.84 (s, 3 H) 4.01 (s, 3 H) 4.82 (s, 2 H) 5.08 (d, *J*=15.4 Hz, 1 H) 5.46 (d, *J*=15.4 Hz, 1 H) 6.15 (d, *J*=2.5 Hz, 1 H) 6.28 (dd, *J*=8.4, 2.5 Hz, 1 H) 6.39 (s, 1 H) 6.68 (d, *J*=8.4 Hz, 1 H) 7.07 (s, 1 H) 7.38 (s, 1 H) 13.65 (s, 1 H). LC-MS 531.2 [M-H]⁻, 533.3 [M+H]⁺, RT 0.92 min (1 minute Method).

### Step 9: 1-(2,4-dimethoxybenzyl)-4-hydroxy-10-(hydroxymethyl)-7,9-dimethyl-2-oxo-1,2,5,6,7,9-hexahydropyrido[3',2':6,7]cyclohepta[1,2-f]indole-3-carboxylic acid

To a suspension of methyl 1-(2,4-dimethoxybenzyl)-4-hydroxy-10-(hydroxymethyl)-7,9-dimethyl-2-oxo-1,2,5,6,7,9-hexahydropyrido[3',2':6,7]cyclohepta[1,2-f]indole-3-carboxylate (1.10 g, 2.07 mmol) in EtOAc (9 mL) was added LiI (0.83 g, 6.21 mmol). The reaction mixture was stirred and heated at 60 °C for 1.5 h until complete consumption of starting material was observed. The mixture was cooled to room temperature, then acidified with aqueous HCl (1M, 20 mL) and diluted with H₂O. The product was extracted with EtOAc (4x40 mL) and the organic phase was washed with Na₂S₂O₃ (10% aq, 20 mL), NaCl (aqueous saturated, 50 mL) and dried over Na₂SO₄. The solvent was removed and the resulting product was obtained as a yellow solid (1.03 g, 96%).
¹H NMR (500 MHz, DMSO-*d*₆) δ ppm 1.20 (d, *J*=6.6 Hz, 3 H) 1.26 - 1.46 (m, 2 H) 1.94 - 2.05 (m, 1 H) 2.28 - 2.37 (m, 1 H) 2.79 (dd, *J*=13.2, 6.0 Hz, 1 H) 3.31 (s, 3 H) 3.68 (s, 3 H) 3.78 (s, 3 H) 4.64 (d, *J*=5.4 Hz, 2 H) 5.10 (d, *J*=15.8 Hz, 1 H) 5.25 (t, *J*=5.4 Hz, 1 H) 5.38 (d, *J*=15.8 Hz, 1 H) 6.27 (dd, *J*=8.5, 1.9 Hz, 1 H) 6.33 (d, *J*=1.9 Hz, 1 H) 6.34 (br. s., 1 H) 6.42 (d, *J*=8.5 Hz, 1 H) 7.23 (s, 1 H) 7.54 (s, 1 H) 13.83 (br. s., 1 H). LC-MS 517.4 [M-H]⁻, 519.3 [M+H]⁺, RT 1.38 min.

### Step 10: 1-(2,4-dimethoxybenzyl)-10-formyl-4-hydroxy-7,9-dimethyl-2-oxo-1,2,5,6,7,9-hexahydropyrido[3',2':6,7]cyclohepta[1,2-f]indole-3-carboxylic acid

To a solution of 1-(2,4-dimethoxybenzyl)-4-hydroxy-10-(hydroxymethyl)-7,9-dimethyl-2-oxo-1,2,5,6,7,9-hexahydropyrido[3',2':6,7]cyclohepta[1,2-f]indole-3-carboxylic acid (1.03 g, 1.99 mmol) in DCM (20 mL) was added activated MnO₂ (1.9 g+1.9 g+0.9 g, 19.67 mmol +19.67 mmol +9.32 mmol) in 3 portions at 30 min intervals. The reaction was monitored by LC/MS. After complete consumption of starting material MnO₂ was filtered and washed with DCM. The mother liquor was concentrated affording product as dark red foam (0.766 g, 68% 3 steps) which was used in the next steps without further purification.
¹H NMR (500 MHz, CHCl₃-*d*) δ ppm 1.22 (d, *J*=6.6 Hz, 3 H) 1.43 - 1.56 (m, 2 H) 2.06 - 2.13 (m, 1 H) 2.20 - 2.30 (m, 1 H) 2.95 (dd, *J*=13.6, 6.6 Hz, 1 H) 3.27 (s, 3 H) 3.75 (s, 3 H) 4.15 (s, 3 H) 5.08 (d, *J*=15.1 Hz, 1 H) 5.57 (d, *J*=15.1 Hz, 1 H) 6.18 (d, *J*=1.9 Hz, 1 H) 6.25 (dd, *J*=8.4, 1.9 Hz, 1 H) 6.48 (d, *J*=8.4 Hz, 1 H) 7.17 (s, 1 H) 7.24 (s, 1 H) 7.58 (s, 1 H) 9.92 (s, 1 H) 13.97 (s, 1 H) 15.99 (s, 1 H). LC-MS 515.0 [M-H]⁻ RT 1.60 min.

### Steps 11-12: 4-hydroxy-7,9-dimethyl-10-((methylamino)methyl)-2-oxo-1,2,5,6,7,9-hexahydropyrido[3',2':6,7]cyclohepta[1,2-f]indole-3-carboxylic acid hydrochloride

To a solution of 1-(2,4-dimethoxybenzyl)-10-formyl-4-hydroxy-7,9-dimethyl-2-oxo-1,2,5,6,7,9-hexahydropyrido[3',2':6,7]cyclohepta[1,2-f]indole-3-carboxylic acid (145 mg, 0.28 mmol) in dichloroethane (3 mL) was added MeNH₂ solution (2M THF, 0.30 mL, 0.60 mmol) followed by AcOH (35 µL, 0.61 mmol). After stirring at room temperature for 10 min, NaBH(OAc)₃ (90 mg, 0.43 mmol) was added. The reaction was stirred at room temperature 2 h and monitored by LC/MS until the starting aldehyde was completely consumed. The dichloroethane was then removed and the residue was dissolved in MeOH (5 mL) and several drops of TFA to generate a homogeneous mixture that was filtered through a PTFE micron filter and purified directly by preparative HPLC to provide the product as a TFA salt (94.8 mg, 52%).

To the product (94.8 mg, 0.15 mmol) obtained above was added i-Pr₃SiH (0.9 mL) followed by TFA (0.9 mL). The mixture was heated at 60 °C for 2 h and monitored by LC/MS. After complete consumption of starting material, the TFA was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a pale pink solid (47.8 mg, 78%).
¹H NMR (500 MHz, DMSO-*d*₆) δ ppm 1.39 (d, *J*=6.0 Hz, 3 H) 1.61 - 1.75 (m, 2 H) 2.12 - 2.33 (m, 1 H) 2.63 (br. s., 3 H) 2.75 - 2.88 (m, 2 H) 3.88 (s, 3 H) 4.42 (br. s., 2 H) 6.80 (s, 1 H) 7.48 (s, 1 H) 7.78 (s, 1 H) 9.29 (br. s., 2 H) 12.91 (s, 1 H) 13.82 (s, 1 H). LC-MS 380.1 [M-H]⁻, 382.2 [M+H]⁺, RT 0.83 min.

### Example 63

### 10-((ethylamino)methyl)-4-hydroxy-7,9-dimethyl-2-oxo-1,2,5,6,7,9-hexahydropyrido[3',2':6,7]cyclohepta[1,2-f]indole-3-carboxylic acid hydrochloride (Cpd 63)

To a solution of 1-(2,4-dimethoxybenzyl)-10-formyl-4-hydroxy-7,9-dimethyl-2-oxo-1,2,5,6,7,9-hexahydropyrido[3',2':6,7]cyclohepta[1,2-f]indole-3-carboxylic acid (Example 62, step 10, 145 mg, 0.28 mmol) in dichloroethane (3 mL) was added EtNH₂ solution (2M THF, 0.30 mL, 0.60 mmol) followed by AcOH (35 µL, 0.61 mmol). After stirring at room temperature for 10 min, NaBH(OAc)₃ (90 mg, 0.43 mmol) was added. The reaction was stirred at room temperature 2 h and monitored by LC/MS until the starting aldehyde was completely consumed. The dichloroethane was then removed and the residue was dissolved in MeOH (5 mL) and several drops of TFA to generate a homogeneous mixture that was filtered through a PTFE micron filter and purified directly by preparative HPLC to provide the product as a TFA salt (117.3 mg, 63%).

To the product (117.3 mg, 0.18 mmol) obtained above was added i-Pr₃SiH (1.20 mL) followed by TFA (1.20 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a pale pink solid (59.5 mg, 77%).
¹H NMR (500 MHz, DMSO-*d*₆) δ ppm 1.28 (t, *J*=7.3 Hz, 3 H) 1.39 (d, *J*=6.0 Hz, 3 H) 1.56 - 1.77 (m, 2 H) 2.13 - 2.27 (m, 1 H) 2.81 (br. s., 2 H) 3.00 - 3.13 (m, 2 H) 3.89 (s, 3 H) 4.42 (br. s., 2 H) 6.81 (s, 1 H) 7.48 (s, 1 H) 7.78 (s, 1 H) 9.34 (br. s., 2 H) 12.92 (s, 1 H) 13.82 (s, 1 H). LC-MS 394.2 [M-H]⁻, 396.2 [M+H]⁺, RT 0.84 min.

### Example 64

### 4-hydroxy-10-((isopropylamino)methyl)-7,9-dimethyl-2-oxo-1,2,5,6,7,9-hexahydropyrido[3',2':6,7]cyclohepta[1,2-f]indole-3-carboxylic acid hydrochloride (Cpd 64)

To a solution of 1-(2,4-dimethoxybenzyl)-10-formyl-4-hydroxy-7,9-dimethyl-2-oxo-1,2,5,6,7,9-hexahydropyrido[3',2':6,7]cyclohepta[1,2-f]indole-3-carboxylic acid (Example 62, step 10, 145 mg, 0.28 mmol) in dichloroethane (3 mL) was added isopropylamine (50 µL, 0.59 mmol) followed by AcOH (35 µL, 0.61 mmol). After stirring at room temperature for 10 min, NaBH(OAc)₃ (90 mg, 0.43 mmol) was added. The reaction mixture was stirred at room temperature 2 h, then additional isopropylamine (25 µL, 0.30 mmol) was added. The mixture was stirred overnight. LC/MS indicated complete consumption of starting aldehyde. The dichloroethane was then removed and the residue was dissolved in MeOH (5 mL) and several drops of TFA to generate a homogeneous mixture that was filtered through a PTFE micron filter and purified directly by preparative HPLC to provide the product as a TFA salt (125.4 mg, 66%).

To the product (125.4 mg, 0.19 mmol) obtained above was added i-Pr₃SiH (1.20 mL) followed by TFA (1.10 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a colorless solid (67.8 mg, 82%).
¹H NMR (500 MHz, DMSO-*d*₆) δ ppm 1.36 (d, *J*=5.7 Hz, 6 H) 1.40 (d, *J*=6.3 Hz, 3 H) 1.56 - 1.77 (m, 2 H) 2.14 - 2.28 (m, 1 H) 2.82 (br. s., 2 H) 3.41 - 3.49 (m, 1 H) 3.89 (s, 3 H) 4.42 (t, *J*=5.4 Hz, 2 H) 6.82 (s, 1 H) 7.48 (s, 1 H) 7.78 (s, 1 H) 9.29 (br. s, 2 H) 12.93 (s, 1 H) 13.82 (s, 1 H). LC-MS 408.2 [M-H]⁻, RT 0.88 min.

### Example 65

### 10-((tert-butylamino)methyl)-4-hydroxy-7,9-dimethyl-2-oxo-1,2,5,6,7,9-hexahydropyrido[3',2':6,7]cyclohepta[1,2-f]indole-3-carboxylic acid hydrochloride (Cpd 65)

To a solution of 1-(2,4-dimethoxybenzyl)-10-formyl-4-hydroxy-7,9-dimethyl-2-oxo-1,2,5,6,7,9-hexahydropyrido[3',2':6,7]cyclohepta[1,2-f]indole-3-carboxylic acid (Example 62, step 10, 145 mg, 0.28 mmol) in dichloroethane (3 mL) was added tert-butylamine (60 µL, 0.57 mmol) followed by AcOH (35 µL, 0.61 mmol). After stirring at room temperature for 10 min, NaBH(OAc)₃ (90 mg, 0.43 mmol) was added. The reaction mixture was stirred at room temperature for 2 h, then additional tert-butylamine (30 µL, 0.28 mmol) was added. The mixture was stirred overnight. LC/MS indicated complete consumption of starting aldehyde. The dichloroethane was then removed and the residue was dissolved in MeOH (5 mL) and several drops of TFA to generate a homogeneous mixture that was filtered through a PTFE micron filter and purified directly by preparative HPLC to provide the product as a TFA salt (81.8 mg, 42%).

To the product (81.8 mg, 0.12 mmol) obtained above was added i-Pr₃SiH (0.8 mL) followed by TFA (0.8 mL). The mixture was heated at 60 °C for 2 h and monitored by LC/MS. After complete consumption of starting material, the TFA was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a colorless solid (45.8 mg, 84%).
¹H NMR (500 MHz, DMSO-*d*₆) δ ppm 1.40 (d, *J*=6.3 Hz, 3 H) 1.43 (s, 9 H) 1.58 - 1.75 (m, 2 H) 2.14 - 2.29 (m, 1 H) 2.82 (br. s., 2 H) 3.89 (s, 3 H) 4.40 (t, *J*=5.4 Hz, 2 H) 6.82 (s, 1 H) 7.49 (s, 1 H) 7.79 (s, 1 H) 9.17 (br. s., 2 H) 12.94 (s, 1 H) 13.82 (s, 1 H). LC-MS 422.2 [M-H]⁻, RT 0.89 min.

### Example 66

### 4-hydroxy-7,9-dimethyl-10-(((1-methylcyclopropyl)amino)methyl)-2-oxo-1,2,5,6,7,9-hexahydropyrido[3',2':6,7]cyclohepta[1,2-f]indole-3-carboxylic acid hydrochloride (Cpd 66)

To a solution of 1-(2,4-dimethoxybenzyl)-10-formyl-4-hydroxy-7,9-dimethyl-2-oxo-1,2,5,6,7,9-hexahydropyrido[3',2':6,7]cyclohepta[1,2-f]indole-3-carboxylic acid (Example 62, step 10, 145 mg, 0.28 mmol) in dichloroethane (3 mL) was added 1-methylcyclopropanamine hydrochloride (45.0 mg, 0.42 mmol) followed NEt₃ (60 µL, 0.43 mmol). The mixture was stirred for 5 min at room temperature, then AcOH (30 µL, 0.52 mmol) was added. After stirring at room temperature for 5 min, NaBH(OAc)₃ (90 mg, 0.43 mmol) was added. The reaction was stirred overnight at room temperature. The dichloroethane was then removed and the residue was dissolved in MeOH (5 mL) and several drops of TFA to generate a homogeneous mixture that was filtered through a PTFE micron filter and purified directly by preparative HPLC to provide the product as a TFA salt (124.4 mg, 65%).

To the product (124.4 mg, 0.18 mmol) obtained above was added i-Pr₃SiH (1.2 mL) followed by TFA (1.2 mL). The mixture was heated at 60 °C for 2 h and monitored by LC/MS. After complete consumption of starting material, the TFA was removed under reduced pressure. Addition of HCl solution (2M Et₂O, 2.0 mL) to the oily residue resulted in precipitate formation. The mixture was diluted with Et₂O and the resulting solid was filtered and washed with Et₂O. The product HCl salt was obtained as a colorless solid (65.9 mg, 79%).
¹H NMR (500 MHz, DMSO-*d*₆) δ ppm 0.71 (t, *J*=6.0 Hz, 2 H) 1.20 (t, *J*=6.0 Hz, 2 H) 1.40 (d, *J*=6.0 Hz, 3 H) 1.53 (s, 3 H) 1.60 - 1.75 (m, 2 H) 2.13 - 2.28 (m, 1 H) 2.81 (br. s., 2 H) 3.90 (s, 3 H) 4.50 (br. s., 2 H) 6.83 (s, 1 H) 7.48 (s, 1 H) 7.78 (s, 1 H) 9.70 (br. s, 2 H) 12.92 (br. s., 1 H) 13.82 (br. s., 1 H). LC-MS 420.2 [M-H]⁻, 422.2 [M+H]⁺, RT 0.88 min.

### Example 67

### 10-((ethylamino)methyl)-4-hydroxy-5,9-dimethyl-2-oxo-1,2,5,6,7,9-hexahydropyrido[3',2':6,7]cyclohepta[1,2-f]indole-3-carboxylic acid hydrochloride (Cpd 67)

### Step 1: 4-iodo-3-methylbut-1-ene

The titled iodide was prepared according to literature procedure (J. Org. Chem. 1999, 64, 8263-8266).
¹H NMR (500 MHz, CHCl₃-*d*) δ ppm 1.14 (d, *J*=6.62 Hz, 3 H) 2.38 (dt, *J*=12.85, 6.66 Hz, 1 H) 3.14 (dd, *J*=9.62, 6.46 Hz, 1 H) 3.20 (dd, *J*=9.62, 5.83 Hz, 1 H) 5.07 (d, *J*=1.26 Hz, 1 H) 5.08 - 5.11 (m, 1 H) 5.66 - 5.78 (m, 1 H).

### Step 2: 1-(2-((tert-butyldimethylsilyloxy)methyl)-1-methyl-6-vinyl-1H-indol-5-yl)-3-methylpent-4-en-1-ol

To a solution of 4-iodo-3-methylbut-1-ene (0.5 g, 2.55 mmol) in anhydrous ether (5 mL) was added t-BuLi (1.7M/pentanes, 3.1 mL, 5.3 mL, 2.1 eq) dropwise at -78 °C. The mixture was stirred at -78 °C for 1 h, then a solution of 2-((tert-butyldimethylsilyloxy)methyl)-1-methyl-6-vinyl-1H-indole-5-carbaldehyde (Example 19, step 6, 820 mg, 2.49 mmol) in THF (3 mL) was added at -78 °C. After stirring 15 min at -78 °C, the reaction was quenched by NH₄Cl (aqueous saturated, 3 mL) then extracted with EtOAc (4x30 mL). The solvent was removed to give crude product (1 g, ca. 2.5 mmol) as pale yellow oil which was used in the next step without further purification.
¹H NMR (500 MHz, CHCl₃-*d*) δ ppm 0.05 - 0.07 (m, 3 H) 0.07 - 0.08 (m, 3 H) 0.88 - 0.93 (m, 9 H) 1.03 - 1.10 (m, 3 H) 1.64 - 1.94 (m, 3 H) 2.33 - 2.56 (m, 1 H) 3.78 - 3.80 (m, 3 H) 4.82 (s, 2 H) 4.94 - 5.17 (m, 3 H) 5.25 - 5.30 (m, 1 H) 5.65 (ddd, *J*=17.10, 1.66, 0.79 Hz, 1 H) 5.72 - 5.88 (m, 1 H) 6.32 - 6.36 (m, 1 H) 7.12 - 7.26 (m, 1 H) 7.39 (d, *J*=5.04 Hz, 1 H) 7.65 - 7.69 (m, 1 H). LC-MS 400.1 [M+H]⁺, RT 1.80 min.

### Step 3: 2-((tert-butyldimethylsilyloxy)methyl)-1,7-dimethyl-1,5,6,7-tetrahydrocyclohepta[f]indol-5-ol

To a solution of crude 1-(2-((tert-butyldimethylsilyloxy)methyl)-1-methyl-6-vinyl-1H-indol-5-yl)-3-methylpent-4-en-1-ol (1 g, ca. 2.5 mmol) obtained above in toluene (50 mL) was added second generation Grubbs' catalyst (64 mg, 0.075 mmol, 3 mol%) at room temperature. The reaction mixture was heated to 60 °C, then stirred overnight. The solvent was removed under reduced pressure and the residue was purified by column chromatography (EtOAc/hexanes, 0-20% gradient) to afford the desired product (422 mg, 1.14 mmol, 45%, two steps) as 1:1 diastereomers.
¹H NMR (500 MHz, CHCl₃-*d*) δ ppm 0.04 - 0.07 (m, 6 H) 0.87 - 0.91 (m, 9 H) 1.11 (d, *J*=6.94 Hz, 1.5 H) 1.18 (d, *J*=7.25 Hz, 1.5 H) 1.92 - 2.02 (m, 1 H) 2.23 - 2.33 (m, 1 H) 2.77 - 2.89 (m, 1 H) 3.75 - 3.79 (m, 3 H) 4.79 - 4.85 (m, 2 H) 4.97 (d, *J*=8.83 Hz, 1 H) 5.66 - 5.78 (m, 1 H) 6.30 - 6.38 (m, 1 H) 6.49 (dd, *J*=4.73, 2.52 Hz, 0.5 H) 6.51 (dd, *J*=4.57, 2.36 Hz, 0.5 H) 7.13 (s, 0.5 H) 7.09 (s, 0.5 H) 7.57 (s, 0.5 H) 7.67 (s, 0.5 H). LC-MS 372.1 [M+H]⁺, RT 1.72 min.

### Step 4: 2-((tert-butyldimethylsilyloxy)methyl)-1,7-dimethyl-1,5,6,7,8,9-hexahydrocyclohepta[f]indol-5-ol

To a solution of 2-((tert-butyldimethylsilyloxy)methyl)-1,7-dimethyl-1,5,6,7-tetrahydrocyclohepta[f]indol-5-ol (422 mg, 1.14 mmol) in EtOAc (6 mL) and CH₂Cl₂ (2 mL) was added Pd/C (10wt%, Degussa type, 50 mg). The vial was evacuated then back filled with H₂ in three cycles. After completion, the reaction mixture was filtered through Celite and washed by EtOAc. The filtrate was concentrated to give crude product (412 mg, 1.10 mmol) which was used in the next step without further purification.
¹H NMR (500 MHz, CHCl₃-*d*) δ ppm 0.04 - 0.06 (m, 3 H) 0.06 - 0.08 (m, 3 H) 0.88 - 0.93 (m, 9 H) 0.94 - 0.99 (m, 3 H) 0.93 - 1.05 (m, 1 H) 1.33 - 1.52 (m, 1 H) 1.69 - 1.89 (m, 1 H) 1.93 - 2.14 (m, 2 H) 2.17 - 2.35 (m, 1 H) 2.74 - 2.92 (m, 2 H) 3.38 (t, *J*=12.45 Hz, 1 H) 3.72 - 3.78 (m, 3 H) 4.78 - 4.83 (m, 2 H) 5.00 - 5.08 (m, 1 H) 6.28 - 6.39 (m, 1 H) 7.02 - 7.08 (m, 1 H) 7.40 (s, 0.5 H) 7.73 (s, 0.5 H). LC-MS 374.1 [M+H]⁺, RT 1.77 min.

### Step 5: 2-((tert-butyldimethylsilyloxy)methyl)-1,7-dimethyl-6,7,8,9-tetrahydrocyclohepta[f]indol-5(1H)-one

To a solution of 2-((tert-butyldimethylsilyloxy)methyl)-1,7-dimethyl-1,5,6,7,8,9-hexahydrocyclohepta[f]indol-5-ol (412 mg, 1.10 mmol) in DCM (6 mL) at 0 °C was added solid NaHCO₃ (0.84 g, 10.0 mmol) followed by Dess-Martin periodinane (563 mg, 1.3 mmol, 1.2 eq). The reaction was stirred at 0 °C for 45 min, until LC/MC and TLC indicated complete consumption of starting material. The reaction mixture was diluted with DCM, washed with NaHCO₃ (aqueous saturated, 20 mL), Na₂S₂O₃ (10% aq, 20 mL) and NaCl (aqueous saturated, 20 mL), then the organic phase was dried over Na₂SO₄. The solvent was removed and the resulting residue was purified by column chromatography (EtOAc/hexanes, 0-15% gradient), affording the desired product (120 mg, 0.32 mmol, 28%, two steps). LC-MS 372.2 [M+H]⁺, RT 1.78 min.

### Step 6: N-(2-((tert-butyldimethylsilyloxy)methyl)-1,7-dimethyl-6,7,8,9-tetrahydrocyclohepta[f]indol-5(1H)-ylidene)-1-(2,4-dimethoxyphenyl)methanamine

To a solution of 2-((tert-butyldimethylsilyloxy)methyl)-1,7-dimethyl-6,7,8,9-tetrahydrocyclohepta[f]indol-5(1H)-one (326 mg, 0.88 mmol) in DCM (3 mL) was added 2,4-dimethoxybenzylamine (0.14 mL, 0.96 mmol) and NEt₃ (0.49 mL, 3.52 mmol). The mixture was cooled to 0 °C, then a solution of TiCl₄ (1M DCM, 0.88 mL, 0.88 mmol) was added dropwise via syringe pump over 30 min. The reaction was allowed to warm to room temperature and stirred overnight. The mixture was diluted with DCM (10 mL) and then the reaction was quenched with NaHCO₃ (aqueous saturated, 5 mL). After vigorous shaking, the organic phase was separated using a PTFE phase separator, and dried over Na₂SO₄. The solvent was removed and the resulting crude product (450 mg, quant) was obtained as a yellow oil, which was used directly in the next step without further purification. LC-MS 521.3 [M+H]⁺, RT 1.44 min.

### Step 7: methyl 10-(((tert-butyldimethylsilyl)oxy)methyl)-1-(2,4-dimethoxybenzyl)-4-hydroxy-5,9-dimethyl-2-oxo-1,2,5,6,7,9-hexahydropyrido [3',2':6,7] cyclohepta[1,2-f] indole-3-carboxylate

The crude product obtained above (450 mg, ca. 0.88 mmol) and trimethyl methanetricarboxylate (380 mg, 2.0 mmol) were mixed together in Ph₂O (2 mL). With stirring, the mixture was placed onto a pre-heated heat block at 230 °C and heated for 10 min after the initial bubbling of MeOH was observed (occurs ant ∼160 °C internal reaction temperature). The reaction mixture was cooled to room temperature, then purified by column chromatography (hexanes followed by EtOAc/hexanes 0-80% gradient) to yield the product as a yellow foam (210 mg, 37% 2 steps) LC-MS 645.3 [M-H]⁻, 647.3 [M+H]⁺, RT 1.87 min.

### Steps 8-10: 1-(2,4-dimethoxybenzyl)-10-formyl-4-hydroxy-5,9-dimethyl-2-oxo-1,2,5,6,7,9-hexahydropyrido[3',2':6,7]cyclohepta[1,2-f]indole-3-carboxylic acid

To a solution of methyl 10-(((tert-butyldimethylsilyl)oxy)methyl)-1-(2,4-dimethoxybenzyl)-4-hydroxy-5,9-dimethyl-2-oxo-1,2,5,6,7,9-hexahydropyrido[3',2':6,7]cyclohepta[1,2-f]indole-3-carboxylate (210 mg, 0.32 mmol) in THF (2 mL) was added TBAF solution (1M THF, 1.0 mL, 1.0 mmol). The reaction mixture was stirred at room temperature for 2 h until the starting material was completely consumed. The THF was removed and the residue was purified by column chromatography (EtOAc/DCM, 0-100% gradient). The product was obtained as a yellow solid (141 mg, 83 %). LC-MS 531.2 [M-H]⁻, 533.1 [M+H]⁺, RT 1.30 min.

To a suspension of the above methyl ester (141 mg, 0.26 mmol) in EtOAc (3 mL) was added LiI (134 mg, 1.00 mmol). The reaction mixture was stirred and heated at 60 °C for 1.5 h until complete consumption of starting material was observed. The mixture was cooled to room temperature, then acidified with aqueous HCl (1M, 1 mL) and diluted with H₂O. The product was extracted with EtOAc (4x10 mL) and the organic phase was washed with Na₂S₂O₃ (10% aq, 4 mL), NaCl (aqueous saturated, 10 mL) and dried over Na₂SO₄. The solvent was removed and the resulting product was obtained as a yellow solid (123 mg, 91%). LC-MS 517.1 [M-H]⁻, 519.0 [M+H]⁺, RT 1.36 min.

To a solution of the obtained crude acid (123 mg, 0.24 mmol) in DCM (3 mL) was added activated MnO₂ (180 mg+180 mg+90 mg, 2.1 mmol +2.1 mmol +1.0 mmol) in 3 portions at 30 min intervals. The reaction was monitored by LC/MS. After complete consumption of starting material MnO₂ was filtered and washed with DCM. The mother liquor was concentrated affording product as dark red foam (89 mg, 72%) which was used in the next steps without further purification. LC-MS 515.6 [M-H]⁻, 517.5 [M+H]⁺, RT 0.96 min (1min Method).

### Step 11-12: 10-((ethylamino)methyl)-4-hydroxy-5,9-dimethyl-2-oxo-1,2,5,6,7,9-hexahydropyrido[3',2':6,7]cyclohepta[1,2-f]indole-3-carboxylic acid hydrochloride

To a solution of 1-(2,4-dimethoxybenzyl)-10-formyl-4-hydroxy-5,9-dimethyl-2-oxo-1,2,5,6,7,9-hexahydropyrido[3',2':6,7]cyclohepta[1,2-f]indole-3-carboxylic acid (89 mg, 0.17 mmol) in dichloroethane (2.0 mL) was added ethylamine hydrochloride (40 mg, 0.49 mmol) followed by NEt₃ (70 µL, 0.50 mmol). The mixture was stirred for 5 min at room temperature, then AcOH (30 µL, 0.52 mmol) was added. After stirring at room temperature for 5 min, NaBH(OAc)₃ (106 mg, 0.50 mmol) was added. The reaction was stirred at room temperature (∼2 h) and monitored by LC/MS until the starting aldehyde was completely consumed. The dichloroethane was then removed and the residue was dissolved in MeOH (6 mL) and several drops of TFA to generate a homogeneous mixture that was filtered through a PTFE micron filter and purified directly by preparative HPLC to provide the product as a TFA salt. LC-MS 544.1 [M-H]⁻, RT 0.70 min (1min Method).

To the product obtained above was added i-Pr₃SiH (1.0 mL) followed by TFA (1.0 mL). The mixture was heated at 60 °C for 2 h and monitored by LC/MS. After complete consumption of starting material, the TFA was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a pale pink solid (18 mg, 25% 2 steps).
¹H NMR (500 MHz, DMSO-*d*₆) δ ppm 0.57 (br. s., 3 H) 1.28 (t, *J*= 6.78 Hz, 3 H) 1.80 - 1.94 (m, 1 H) 2.32 - 2.42 (m, 1 H) 2.49 - 2.53 (m, 1 H) 2.68 - 2.80 (m, 2 H) 3.07 (q, *J*= 6.78 Hz, 2 H) 3.85 (s, 3 H) 4.41 (t, *J*=5.36 Hz, 2 H) 6.81 (s, 1 H) 7.53 (s, 1 H) 7.79 (s, 1 H) 9.25 (br. s., 2 H) 12.80 (s, 1 H) 14.12 (s, 1 H) 16.46 (br. s., 1 H). LC-MS 394.1 [M-H]⁻, 396.1 [M+H]⁺, RT 0.56 min (1min Method).

### Example 68

### 4-hydroxy-9-methyl-10-((methylamino)methyl)-2-oxo-1,2,5,9-tetrahydropyrido[3',2':6,7]cyclohepta[1,2-f]indole-3-carboxylic acid hydrochloride (Cpd 68)

### Step 1: 2-(((tert-butyldimethylsilyl)oxy)methyl)-1-methyl-6,7-dihydrocyclohepta[f]indol-5(1H)-one

To activated 4Å molecular sieves (5.8 g, 250 mg/mmol) was added solution 2-(((tertbutyldimethylsilyl)oxy)methyl)-1-methyl-1,5,6,7-tetrahydrocyclohepta[f]indol-5-ol (Example 22, step 2, 8.30 g, 23.21 mmol) in DCM (120 mL). The mixture was cooled to 0 °C, then NMO (4.10 g, 35.00 mmol) and TPAP (410 mg, 1.17 mmol, 5 mol%) were added subsequently. The reaction was stirred at 0 °C and monitored by LC/MS. After complete consumption of starting material (∼1.5 h), the molecular sieves were filtered off and washed with DCM. The mother liquor was concentrated and the residue was purified by column chromatography (EtOAc/hexanes, 0-15% gradient). The product (5.80 g, 70%) was obtained as a pale yellow solid.
¹H NMR (500 MHz, CHCl₃-*d*) δ ppm 0.06 (s, 6 H) 0.90 (s, 9 H) 2.50 - 2.55 (m, 2 H) 2.95 - 3.03 (m, 2 H) 3.79 (s, 3 H) 4.82 (s, 2 H) 6.07 (dt, *J*=11.7, 5.8 Hz, 1 H) 6.45 (s, 1 H) 6.60 (d, *J*=11.7 Hz, 1 H) 7.10 (s, 1 H) 8.22 (s, 1 H). LC-MS 356.3 [M+H]⁺, RT 1.71 min.

### Steps 2-3: methyl 10-(((tert-butyldimethylsilyl)oxy)methyl)-1-(2,4-dimethoxybenzyl)-4-hydroxy-9-methyl-2-oxo-1,2,5,9-tetrahydropyrido [3',2':6,7] cyclohepta[1,2-f]indole-3-carboxylate

To a solution of 2-(((tert-butyldimethylsilyl)oxy)methyl)-1-methyl-6,7-dihydrocyclohepta[f]indol-5(1H)-one (5.80 g, 16.31 mmol) in DCM (70 mL) was added 2,4-dimethoxybenzylamine (2.60 mL, 17.30 mmol) and NEt₃ (6.20 mL, 44.48 mmol). The mixture was cooled to 0 °C, then a solution of TiCl₄ (1M DCM, 10.60 mL, 10.60 mmol) was added dropwise via syringe pump over 30 min. The reaction was allowed to warm to room temperature and stirred overnight. The mixture was diluted with DCM (200 mL) and then the reaction was quenched with NaHCO₃ (aqueous saturated, 80 mL). After vigorous shaking, organic phase was separated using a PTFE phase separator and dried over Na₂SO₄. The solvent was removed and the product was obtained as a yellow oil, which was used directly in the next step without further purification.

The crude product obtained above (*ca.* 16.31 mmol) and trimethyl methanetricarboxylate (5.20 g, 27.35 mmol) were mixed together in Ph₂O (32 mL). With stirring, the mixture was placed onto a pre-heated heat block at 230 °C and heated for 10 min after the initial bubbling of MeOH was observed (occurs at ∼160 °C internal reaction temperature). The reaction mixture was cooled to room temperature, then purified by column chromatography (hexanes followed by EtOAc/hexanes 0-70% gradient) to yield the product as a yellow foam (5.40 g, 52% overall).
¹H NMR (500 MHz, CHCl₃-*d*) δ ppm 0.10 (s, 3 H) 0.10 (s, 3 H) 0.91 (s, 9 H) 2.09 (ddd, *J*=14.1, 5.9, 2.0 Hz, 1 H) 3.35 (s, 3 H) 3.50 (dd, *J*=14.1, 7.9 Hz, 1 H) 3.74 (s, 3 H) 3.79 (s, 3 H) 3.99 (s, 3 H) 4.82 (s, 2 H) 4.91 (d, *J*=15.4 Hz, 1 H) 5.20 (br. s., 1 H) 6.19 (d, *J*=2.2 Hz, 1 H) 6.24 - 6.36 (m, 3 H) 6.60 - 6.68 (m, 2 H) 7.14 (s, 1 H) 7.57 (s, 1 H) 13.72 (br. s., 1 H). LC-MS 629.3 [M+H]⁺, 631.3 [M+H]⁺, RT 1.84 min.

### Step 4: methyl 1-(2,4-dimethoxybenzyl)-4-hydroxy-10-(hydroxymethyl)-9-methyl-2-oxo-1,2,5,9-tetrahydropyrido [3',2':6,7] cyclohepta[1,2-f]indole-3-carboxylate

To a solution of methyl 10-(((tert-butyldimethylsilyl)oxy)methyl)-1-(2,4-dimethoxybenzyl)-4-hydroxy-9-methyl-2-oxo-1,2,5,9-tetrahydropyrido[3',2':6,7]cyclohepta[1,2-f]indole-3-carboxylate (0.400 g, 0.63 mmol) in THF (2.5 mL) was added TBAF solution (1M THF, 1.60 mL, 1.60 mmol). The reaction mixture was stirred at room temperature for 1.5 h until the starting material was completely consumed. The THF was removed and the residue was purified by column chromatography (EtOAc/DCM, 0-100% gradient). The product was obtained as a yellow solid (0.316 g, 96 %).
¹H NMR (500 MHz, DMSO-*d*₆) δ ppm 1.96 (ddd, *J*=14.0, 6.0, 2.2 Hz, 1 H) 3.39 (dd, *J*=14.0, 8.2 Hz, 1 H) 3.42 (s, 3 H) 3.67 (s, 3 H) 3.75 (s, 3 H) 3.83 (s, 3 H) 4.65 (d, *J*=5.5 Hz, 2 H) 4.74 (d, *J*=15.4 Hz, 1 H) 5.07 (br. s., 1 H) 5.30 (d, *J*=5.5 Hz, 1 H) 6.27 - 6.38 (m, 4 H) 6.42 (br. s., 1 H) 6.74 (d, *J*=10.1 Hz, 1 H) 7.42 (s, 1 H) 7.67 (br. s., 1 H) 13.22 (br. s., 1 H). LC-MS 515.3 [M-H]⁻, 517.3 [M+H]⁺, RT 1.25 min.

### Step 5: 1-(2,4-dimethoxybenzyl)-4-hydroxy-10-(hydroxymethyl)-9-methyl-2-oxo-1,2,5,9-tetrahydropyrido[3',2':6,7]cyclohepta[1,2-f]indole-3-carboxylic acid

To a suspension of methyl 1-(2,4-dimethoxybenzyl)-4-hydroxy-10-(hydroxymethyl)-9-methyl-2-oxo-1,2,5,9-tetrahydropyrido[3',2':6,7]cyclohepta[1,2-f]indole-3-carboxylate (0.316 g, 0.61 mmol) in EtOAc (3 mL) was added LiI (0.25 g, 1.87 mmol). The reaction mixture was stirred and heated at 60 °C for 2 h until complete consumption of starting material was observed. The mixture was cooled to room temperature, then acidified with aqueous HCl (1M, 2 mL) and diluted with H₂O. The product was extracted with EtOAc (4x10 mL) and the organic phase was washed with Na₂S₂O₃ (10% aq, 10 mL), NaCl (aqueous saturated, 10 mL) and dried over Na₂SO₄. The solvent was removed and the resulting product was obtained as a yellow solid (0.290 g, 94%).
¹H NMR (500 MHz, DMSO-*d*₆) δ ppm 2.03 (ddd, *J*=14.5, 5.7, 1.9 Hz, 1 H) 3.38 - 3.44 (m, 4 H) 3.67 (s, 3 H) 3.78 (s, 3 H) 4.66 (d, *J*=5.4 Hz, 2 H) 4.94 (d, *J*=15.4 Hz, 1 H) 5.21 (br. s., 1 H) 5.33 (t, *J*=5.4 Hz, 1 H) 6.21 - 6.49 (m, 5 H) 6.74 (d, *J*=9.8 Hz, 1 H) 7.47 (s, 1 H) 7.81 (br. s., 1 H) 13.86 (br. s., 1 H) 16.16 (br. s., 1 H). LC-MS 501.1 [M-H]⁻, 503.1 [M+H]⁺, RT 0.83 min (1-minute method).

### Step 6: 1-(2,4-dimethoxybenzyl)-10-formyl-4-hydroxy-9-methyl-2-oxo-1,2,5,9-tetrahydropyrido[3',2':6,7]cyclohepta[1,2-f]indole-3-carboxylic acid

To a solution of 1-(2,4-dimethoxybenzyl)-4-hydroxy-10-(hydroxymethyl)-9-methyl-2-oxo-1,2,5,9-tetrahydropyrido[3',2':6,7]cyclohepta[1,2-f]indole-3-carboxylic acid (0.290 g, 0.58 mmol) in DCM (6 mL) was added activated MnO₂ (0.56 g+0.56 g+0.28 g, 5.80 mmol +5.80 mmol +2.90 mmol) in 3 portions at 30 min intervals. The reaction was monitored by LC/MS. After complete consumption of starting material MnO₂ was filtered and washed with DCM. The mother liquor was concentrated affording product as dark red foam (0.206 g, 65% 3 steps) which was used in the next steps without further purification.
¹H NMR (500 MHz, CHCl₃-*d*) δ ppm 2.16 (dd, *J*=13.9, 4.1 Hz, 1 H) 3.36 (s, 3 H) 3.55 (dd, *J*=13.9, 8.2 Hz, 1 H) 3.77 (s, 3 H) 4.14 (s, 3 H) 4.97 (d, *J*=15.1 Hz, 1 H) 5.23 (d, *J*=15.1 Hz, 1 H) 6.23 (br. s., 1 H) 6.29 (d, *J*=7.9 Hz, 1 H) 6.37 - 6.45 (m, 1 H) 6.57 (d, *J*=7.9 Hz, 1 H) 6.64 (d, *J*=9.5 Hz, 1 H) 7.20 (br. s., 1 H) 7.29 (br. s., 1 H) 7.80 (s, 1 H) 9.96 (br. s., 1 H) 14.03 (br. s., 1 H) 15.87 (br. s., 1 H). LC-MS 499.1 [M-H]⁻, 501.1 [M+H]⁺ RT 0.90 min (1-minute method).

### Steps 7-8: 4-hydroxy-9-methyl-10-((methylamino)methyl)-2-oxo-1,2,5,9-tetrahydropyrido[3',2':6,7]cyclohepta[1,2-f]indole-3-carboxylic acid hydrochloride

To a solution of 1-(2,4-dimethoxybenzyl)-10-formyl-4-hydroxy-9-methyl-2-oxo-1,2,5,9-tetrahydropyrido[3',2':6,7]cyclohepta[1,2-f]indole-3-carboxylic acid (100 mg, 0.20 mmol) in dichloroethane (2 mL) was added MeNH₂ solution (2M THF, 0.20 mL, 0.40 mmol) followed by AcOH (25 µL, 0.44 mmol). After stirring at room temperature for 10 min, NaBH(OAc)₃ (65 mg, 0.31 mmol) was added. The reaction was stirred at room temperature 1.5 h and monitored by LC/MS until the starting aldehyde was completely consumed. The dichloroethane was then removed and the residue was dissolved in MeOH (6 mL) and several drops of TFA to generate a homogeneous mixture that was filtered through a PTFE micron filter and purified directly by preparative HPLC to provide the product as a TFA salt (57.7 mg, 46%).

To a solution of the product (57.7 mg, 0.09 mmol) obtained above in DCM (1.0 mL) was added i-Pr₃SiH (0.3 mL) followed by TFA (0.6 mL). The mixture was stirred at room temperature and monitored by LC/MS. After complete consumption of starting material (∼2 h), solvent was removed under reduced pressure. Addition of HCl solution (2M Et₂O, 1.0 mL) to the oily residue resulted in precipitate formation. The mixture was diluted with Et₂O and the resulting solid was filtered and washed with Et₂O. The product HCl salt was obtained as a pale pink solid (26.7 mg, 72%).
¹H NMR (500 MHz, DMSO-*d*₆) δ ppm 2.65 (br. s., 3 H) 2.86 (br. s, 2 H) 3.87 (s, 3 H) 4.46 (br. s., 2 H) 6.30 (dt, *J*=9.8, 6.9 Hz, 1 H) 6.70 - 6.99 (m, 2 H) 7.65 (s, 1 H) 8.10 (s, 1 H) 9.25 (br. s, 2 H) 12.74 (br. s., 1 H) 13.93 (br. s., 1 H). LC-MS 364.0 [M-H]⁻, 366.1 [M+H]⁺, RT 0.98 min.

### Example 69

### 10-((ethylamino)methyl)-4-hydroxy-9-methyl-2-oxo-1,2,5,9-tetrahydropyrido[3',2':6,7]cyclohepta[1,2-f]indole-3-carboxylic acid hydrochloride (Cpd 69)

To a solution of 1-(2,4-dimethoxybenzyl)-10-formyl-4-hydroxy-9-methyl-2-oxo-1,2,5,9-tetrahydropyrido[3',2':6,7]cyclohepta[1,2-f]indole-3-carboxylic acid (Example 68, step 6, 100 mg, 0.20 mmol) in dichloroethane (2 mL) was added EtNH₂ solution (2M THF, 0.20 mL, 0.40 mmol) followed by AcOH (25 µL, 0.44 mmol). After stirring at room temperature for 10 min, NaBH(OAc)₃ (65 mg, 0.31 mmol) was added. The reaction was stirred at room temperature 1.5 h and monitored by LC/MS until the starting aldehyde was completely consumed. The dichloroethane was then removed and the residue was dissolved in MeOH (6 mL) and several drops of TFA to generate a homogeneous mixture that was filtered through a PTFE micron filter and purified directly by preparative HPLC to provide the product as a TFA salt (81.3 mg, 63%).

To a solution of the product (81.3 mg, 0.13 mmol) obtained above in DCM (1.5 mL) was added i-Pr₃SiH (0.4 mL) followed by TFA (0.7 mL). The mixture was stirred at room temperature and monitored by LC/MS. After complete consumption of starting material (∼2 h), the solvent was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a colorless solid (42.1 mg, 80%).
¹H NMR (500 MHz, DMSO-*d*₆) δ ppm 1.29 (t, *J*=7.3 Hz, 3 H) 2.84 (br. s, 2 H) 2.99 - 3.15 (m, 2 H) 3.88 (s, 3 H) 4.46 (br. s., 2 H) 6.30 (dt, *J*=9.9, 6.9 Hz, 4 H) 6.86 (d, *J*=9.9 Hz, 1 H) 6.89 (s, 1 H) 7.65 (s, 1 H) 8.10 (s, 1 H) 9.36 (br. s., 2 H) 12.75 (br. s., 1 H) 13.93 (br. s., 1 H). LC-MS 378.1 [M-H]⁻, 380.1 [M+H]⁺, RT 1.00 min.

### Example 70

### 10-((ethylamino)methyl)-4-hydroxy-7,9-dimethyl-2-oxo-1,2,5,9-tetrahydropyrido[3',2':6,7]cyclohepta[1,2-f]indole-3-carboxylic acid hydrochloride (Cpd 70)

### Steps 1-2: methyl 10-(((tert-butyldimethylsilyl)oxy)methyl)-1-(2,4-dimethoxybenzyl)-4-hydroxy-7,9-dimethyl-2-oxo-1,2,5,9-tetrahydropyrido[3',2':6,7]cyclohepta[1,2-f]indole-3-carboxylate

To a solution of 2-(((tert-butyldimethylsilyl)oxy)methyl)-1,9-dimethyl-6,7-dihydrocyclohepta[f]indol-5(1H)-one (Example 62, step 4, 0.544 g, 1.47 mmol) in DCM (6 mL) was added 2,4-dimethoxybenzylamine (0.23 mL, 1.53 mmol) and NEt₃ (0.60 mL, 4.30 mmol). The mixture was cooled to 0 °C, then a solution of TiCl₄ solution (1M DCM, 1.0 mL, 1.0 mmol) was added dropwise via syringe pump over 30 min. The reaction was allowed to warm to room temperature and stirred overnight. The mixture was diluted with DCM (15 mL) and then the reaction was quenched with NaHCO₃ (aqueous saturated, 8 mL). After vigorous shaking, the organic phase was separated using a PTFE phase separator and dried over Na₂SO₄. The solvent was removed and the resulting product was obtained as a yellow oil, which was used directly in the next step without further purification. The crude product obtained above (*ca.* 1.47 mmol) and trimethyl methanetricarboxylate (0.50 g, 2.63 mmol) were mixed together in Ph₂O (3 mL). With stirring, the mixture was placed onto a pre-heated heat block at 230 °C and heated for 10 min after the initial bubbling of MeOH was observed (occurs at ∼160 °C internal reaction temperature). The reaction mixture was cooled to room temperature, then purified by column chromatography (hexanes followed by EtOAc/hexanes 0-70% gradient) to yield the product as a yellow foam (0.322 g, 34% overall). LC-MS 645.6 [M+H]⁺, RT 2.04 min.

### Step 3: methyl 1-(2,4-dimethoxybenzyl)-4-hydroxy-10-(hydroxymethyl)-7,9-dimethyl-2-oxo-1,2,5,9-tetrahydropyrido[3',2':6,7] cyclohepta[1,2-f]indole-3-carboxylate

To a solution of methyl 10-(((tert-butyldimethylsilyl)oxy)methyl)-1-(2,4-dimethoxybenzyl)-4-hydroxy-7,9-dimethyl-2-oxo-1,2,5,9-tetrahydropyrido[3',2':6,7]cyclohepta[1,2-f]indole-3-carboxylate (0.322 g, 0.50 mmol) in THF (2.0 mL) was added TBAF solution (1M THF, 1.30 mL, 1.30 mmol). The reaction mixture was stirred at room temperature for 1 h until the starting material was completely consumed. The THF was removed and the residue was purified by column chromatography (EtOAc/DCM, 0-100% gradient). The product was obtained as a yellow solid (0.234 g, 88 %).
¹H NMR (500 MHz, DMSO-*d*₆) δ ppm 1.85 (ddd, *J*=14.0, 6.6, 1.3 Hz, 1 H) 1.99 (s, 3 H) 3.19 (dd, *J*=14.0, 7.9 Hz, 1 H) 3.36 (s, 3 H) 3.65 (s, 3 H) 3.77 (s, 3 H) 3.84 (s, 3 H) 4.65 (d, *J*=5.4 Hz, 2 H) 4.78 (d, *J*=15.8 Hz, 1 H) 5.30 (t, *J*=5.4 Hz, 1 H) 5.37 (br. s., 1 H) 6.09 (ddd, *J*=7.9, 6.6, 1.3 Hz, 1 H) 6.12 - 6.44 (m, 4 H) 7.52 (s, 1 H) 7.70 (br. s., 1 H) 13.33 (br. s., 1 H). LC-MS 529.4 [M-H]⁻, 531.4 [M+H]⁺, RT 1.45 min.

### Step 4: 1-(2,4-dimethoxybenzyl)-4-hydroxy-10-(hydroxymethyl)-7,9-dimethyl-2-oxo-1,2,5,9-tetrahydropyrido[3',2':6,7]cyclohepta[1,2-f]indole-3-carboxylic acid

To a suspension of methyl 1-(2,4-dimethoxybenzyl)-4-hydroxy-10-(hydroxymethyl)-7,9-dimethyl-2-oxo-1,2,5,9-tetrahydropyrido[3',2':6,7]cyclohepta[1,2-f]indole-3-carboxylate (0.233 g, 0.44 mmol) in EtOAc (2 mL) was added LiI (0.18 g, 1.34 mmol). The reaction mixture was stirred and heated at 60 °C for 2 h until complete consumption of starting material was observed. The mixture was cooled to room temperature, then acidified with aqueous HCl (1M, 2 mL) and diluted with H₂O. The product was extracted with EtOAc (4x10 mL) and the organic phase was washed with Na₂S₂O₃ (10% aq, 10 mL), NaCl (aqueous saturated, 10 mL) and dried over Na₂SO₄. The solvent was removed and the resulting product was obtained as a yellow solid (0.216 g, 95%). LC-MS 515.4 [M-H]⁻, 517.4 [M+H]⁺, RT 1.54 min.

### Step 5: 1-(2,4-dimethoxybenzyl)-10-formyl-4-hydroxy-7,9-dimethyl-2-oxo-1,2,5,9-tetrahydropyrido[3',2':6,7]cyclohepta[1,2-f]indole-3-carboxylic acid

To a solution of 1-(2,4-dimethoxybenzyl)-4-hydroxy-10-(hydroxymethyl)-7,9-dimethyl-2-oxo-1,2,5,9-tetrahydropyrido[3',2':6,7]cyclohepta[1,2-f]indole-3-carboxylic acid (0.216 g, 0.48 mmol) in DCM (5 mL) was added activated Mn0₂ (0.40 g+0.40 g, 4.14 mmol +4.14 mmol) in 2 portions at 30 min intervals. The reaction was monitored by LC/MS. After complete consumption of starting material MnO₂ was filtered and washed with DCM. The mother liquor was concentrated affording product as dark red foam (0.152 g, 59% 3 steps) which was used in the next steps without further purification. LC-MS 513.4 [M-H]⁻, 515.4 [M+H]⁺ RT 1.66 min.

### Steps 6-7: 10-((ethylamino)methyl)-4-hydroxy-9-methyl-2-oxo-1,2,5,9-tetrahydropyrido[3',2':6,7]cyclohepta[1,2-f]indole-3-carboxylic acid hydrochloride

To a solution of 11-(2,4-dimethoxybenzyl)-10-formyl-4-hydroxy-7,9-dimethyl-2-oxo-1,2,5,9-tetrahydropyrido[3',2':6,7]cyclohepta[1,2-f]indole-3-carboxylic acid (151.6 mg, 0.29 mmol) in dichloroethane (2 mL) was added EtNH₂ solution (2M THF, 0.30 mL, 0.60 mmol) followed by AcOH (35 µL, 0.61 mmol). After stirring at room temperature for 10 min, NaBH(OAc)₃ (105 mg, 0.50 mmol) was added. The reaction was stirred at room temperature 2 h and monitored by LC/MS until the starting aldehyde was completely consumed. The dichloroethane was then removed and the residue was dissolved in MeOH (7 mL) and several drops of TFA to generate a homogeneous mixture that was filtered through a PTFE micron filter and purified directly by preparative HPLC to provide the product as a TFA salt (97.3 mg, 51 %). LC-MS 542.5 [M-H]⁻, RT 1.14 min

To a solution of the product (97.3 mg, 0.15 mmol) obtained above in DCM (1.0 mL) was added i-Pr₃SiH (0.5 mL) followed by TFA (1.0 mL). The mixture was stirred at room temperature and monitored by LC/MS. After complete consumption of starting material (∼2 h), the solvent was removed under reduced pressure. Addition of HCl solution (2M Et₂O, 1.5 mL) to the oily residue resulted in precipitate formation. The mixture was diluted with Et₂O and the resulting solid was filtered and washed with Et₂O. The product HCl salt was obtained as a colorless solid (47.3 mg, 74%).
¹H NMR (500 MHz, DMSO-*d*₆) δ ppm 1.29 (q, *J*=6.9 Hz, 3 H) 2.03 (dd, *J*=14.2, 6.3 Hz, 1 H) 2.21 (s, 3 H) 3.02 - 3.13 (m, 2 H) 3.30 (dd, *J*=14.2, 8.2 Hz, 1 H) 3.92 (s, 3 H) 4.47 (t, *J*=5.7 Hz, 2 H) 6.14 (dd, *J*=8.2, 6.3 Hz, 1 H) 6.88 (s, 1 H) 7.82 (s, 1 H) 8.05 (s, 1 H) 9.33 (br. s., 2 H) 12.85 (br. s., 1 H) 13.88 (s, 1 H). LC-MS 392.4 [M-H]⁻, RT 0.98 min.

### Example 71

### (cis)-10-((ethylamino)methyl)-4,6,7-trihydroxy-9-methyl-2-oxo-1,2,5,6,7,9-hexahydropyrido[3',2':6,7]cyclohepta[1,2-f]indole-3-carboxylic acid hydrochloride (Cpd 71)

### Step 1: methyl 4-(benzyloxy)-10-(((tert-butyldimethylsilyl)oxy)methyl)-1-(2,4-dimethoxybenzyl)-9-methyl-2-oxo-1,2,5,9-tetrahydropyrido [3',2':6,7]cyclohepta[1,2-f]indole-3-carboxylate

To a solution of methyl 10-(((tert-butyldimethylsilyl)oxy)methyl)-1-(2,4-dimethoxybenzyl)-4-hydroxy-9-methyl-2-oxo-1,2,5,9-tetrahydropyrido[3',2':6,7]cyclohepta[1,2-f]indole-3-carboxylate (Example 68, Step 3, 0.530 g 0.84 mmol), PPh₃ (0.330 g, 1.26 mmol) and BnOH (0.13 mL, 1.26 mmol)in THF (4 mL) at 0 °C was added DIAD (0.25 mL, 1.26 mmol) dropwise. The reaction was stirred at 0 °C 10 min and allowed to warm to room temperature and stirred 30 min. The THF was removed and the residue was purified by column chromatography (EtOAc/hexanes, 0-50% gradient). The product (0.546 g, 90%) was obtained as a yellow solid.
¹H NMR (500 MHz, CHCl₃-*d*) δ ppm 0.10 (s, 3 H) 0.10 (s, 3 H) 0.91 (s, 9 H) 2.05 (ddd, *J=*14.2, 6.0, 2.2 Hz, 1 H) 3.30 (dd, *J*=14.2, 7.7 Hz, 1 H) 3.34 (s, 3 H) 3.75 (s, 3 H) 3.79 (s, 3 H) 3.94 (s, 3 H) 4.83 (s, 2 H) 4.95 (d, *J*=15.1 Hz, 1 H) 5.11 - 5.18 (m, 2 H) 5.22 (br. s., 1 H) 6.14 (ddd, *J*=10.1, 7.7, 6.0 Hz, 1 H) 6.19 (d, *J*=2.2 Hz, 1 H) 6.24 - 6.30 (m, 2 H) 6.56 (d, *J*=10.1 Hz, 1 H) 6.59 - 6.65 (m, 1 H) 7.11 (s, 1 H) 7.35 - 7.47 (m, 5 H) 7.54 (s, 1 H). LC-MS 721.5 [M+H]⁺, RT 1.95 min.

### Step 2: methyl 4-(benzyloxy)-1-(2,4-dimethoxybenzyl)-10-(hydroxymethyl)-9-methyl-2-oxo-1,2,5,9-tetrahydropyrido[3',2':6,7]cyclohepta[1,2-f]indole-3-carboxylate

To a solution of methyl 4-(benzyloxy)-10-(((tert-butyldimethylsilyl)oxy)methyl)-1-(2,4-dimethoxybenzyl)-9-methyl-2-oxo-1,2,5,9-tetrahydropyrido[3',2':6,7]cyclohepta[1,2-f]indole-3-carboxylate (0.546 g, 0.63 mmol) in THF (1.5 mL) was added TBAF solution (1M THF, 1.50 mL, 1.50 mmol). The reaction mixture was stirred at room temperature for 30 min until the starting material was completely consumed. The THF was removed and the residue was purified by column chromatography (EtOAc/DCM, 0-100% gradient). The product was obtained as a yellow solid (0.385 g, 84 %).
¹H NMR (500 MHz, CHCl₃-*d*) δ ppm 2.02 (ddd, *J*=14.2, 6.0, 2.0 Hz, 1 H) 3.28 (dd, *J*=14.2, 7.9 Hz, 1 H) 3.33 (s, 3 H) 3.74 (s, 3 H) 3.80 (s, 3 H) 3.94 (s, 3 H) 4.81 (s, 2 H) 4.91 (d, *J*=15.4 Hz, 1 H) 5.09 - 5.23 (m, 3 H) 6.13 (ddd, *J*=10.4, 7.9, 6.0 Hz, 1 H) 6.18 (d, *J*=2.2 Hz, 1 H) 6.25 (dd, *J*=8.2, 2.2 Hz, 1 H) 6.35 (s, 1 H) 6.54 (d, *J*=10.4 Hz, 1 H) 6.57 (d, *J*=8.2 Hz, 1 H) 7.10 (s, 1 H) 7.35 - 7.46 (m, 5 H) 7.55 (s, 1 H). LC-MS 607.6 [M+H]⁺, RT 1.30 min.

### Step 3: methyl 4-(benzyloxy)-1-(2,4-dimethoxybenzyl)-10-formyl-9-methyl-2-oxo-1,2,5,9-tetrahydropyrido[3',2':6,7]cyclohepta[1,2-f]indole-3-carboxylate

To a solution of methyl 4-(benzyloxy)-1-(2,4-dimethoxybenzyl)-10-(hydroxymethyl)-9-methyl-2-oxo-1,2,5,9-tetrahydropyrido[3',2':6,7]cyclohepta[1,2-f]indole-3-carboxylate (0.385 g, 0.63 mmol) in DCM (6.5 mL) was added activated MnO₂ (0.6 g+0.6 g, 6.2 mmol +6.2 mmol) in 2 portions at 30 min intervals. The reaction was monitored by LC/MS. After complete consumption of starting material, the MnO₂ was filtered and washed with DCM. The mother liquor was concentrated and the product was purified by column chromatography (EtOAc/hexanes, 40-80% gradient). The product was obtained as a yellow solid (0.285 g, 75 %).
¹H NMR (500 MHz, CHCl₃-*d*) δ ppm 2.06 (ddd, *J*=14.2, 6.0, 2.2 Hz, 1 H) 3.29 (s, 3 H) 3.32 (dd, *J*=14.2, 8.0 Hz, 1 H) 3.76 (s, 3 H) 3.96 (s, 3 H) 4.10 (s, 3 H) 4.92 (d, *J*=15.4 Hz, 1 H) 5.13 - 5.25 (m, 3 H) 6.17 (d, *J*=2.2 Hz, 1 H) 6.22 (ddd, *J*=10.2, 8.0, 6.0 Hz, 1 H) 6.29 (dd, *J*=8.5, 2.2 Hz, 1 H) 6.55 (d, *J*=10.1 Hz, 1 H) 6.65 (d, *J*=8.5 Hz, 1 H) 7.17 (s, 1 H) 7.20 (s, 1 H) 7.33 - 7.48 (m, 5 H) 7.72 (s, 1 H) 9.92 (s, 1 H). LC-MS 605.4 [M+H]⁺ RT 1.61 min.

### Steps 4-5: methyl 4-(benzyloxy)-10-(((tert-butoxycarbonyl)(ethyl)amino)methyl)-1-(2,4-dimethoxybenzyl)-9-methyl-2-oxo-1,2,5,9-tetrahydropyrido [3',2':6,7]cyclohepta[1,2-f]indole-3-carboxylate

To a solution of methyl 4-(benzyloxy)-1-(2,4-dimethoxybenzyl)-10-formyl-9-methyl-2-oxo-1,2,5,9-tetrahydropyrido[3',2':6,7]cyclohepta[1,2-f]indole-3-carboxylate (1.05 g, 1.74 mmol) in dichloroethane (10 mL) was added ethylamine hydrochloride (284 mg, 3.5 mmol) followed by NEt₃ (0.49 mL, 3.5 mmol). The mixture was stirred for 5 min at room temperature, then AcOH (0.20 mL, 3.5 mmol) was added. After stirring at room temperature for 5 min, NaBH(OAc)₃ (740 mg, 3.5 mmol) was added. The reaction was stirred at room temperature for ~2 h and monitored by LC/MS until the starting aldehyde was completely consumed. The mixture was diluted with DCM (10 mL) and then the reaction was quenched with NaHCO₃ (aqueous saturated, 5 mL). The organic layer was separated using a PTFE phase separator, and dried over Na₂SO₄. The solvent was removed and the resulting crude product was obtained (1.45 g, quant) as a yellow oil, which was used directly in the next step without further purification. LC-MS 634.5 [M+H]⁺, RT 1.23 min.

To a solution of the obtained product (1.45 g) in CH₂Cl₂ (10 mL) was added (Boc)₂O (760 mg, 3.5 mmol) at room temperature. After complete consumption of starting material, the solvent was removed under reduced pressure, then the residue was purified by flash column chromatography (0-40% EtOAc in hexanes) to afford the desired product (0.94 g, 74% over two steps) as a yellow solid.
¹H NMR (500 MHz, Acetone) δ ppm 1.01 (t, *J*=6.9 Hz, 3 H) 1.50 (s, 9 H) 2.05 (ddd, *J*=14.3, 6.0, 2.2 Hz, 1 H) 3.25 (q, *J*=6.9 Hz, 2 H) 3.31 (dd, *J*=14.3, 7.7 Hz, 1 H) 3.41 (s, 3 H) 3.73 (s, 3 H) 3.79 (s, 3 H) 3.84 (s, 3 H) 4.71 (s, 2 H) 4.93 (d, *J*=15.4 Hz, 1 H) 5.12 - 5.25 (m, 3 H) 6.24 (ddd, *J*=10.1, 7.7, 6.0 Hz, 1 H) 6.28 - 6.33 (m, 2 H) 6.39 (br. s., 1 H) 6.54 (d, *J*=6.9 Hz, 1 H) 6.66 (d, *J*=10.1 Hz, 1 H) 7.31 (s, 1 H) 7.36 - 7.41 (m, 1 H) 7.43 - 7.47 (m, 2 H) 7.51 - 7.57 (m, 3 H). LC-MS 734.5 [M+H]⁺ RT 1.65 min.

### Step 6: (cis)-methyl 4-(benzyloxy)-10-(((tert-butoxycarbonyl)(ethyl)amino)methyl)-1-(2,4-dimethoxybenzyl)-6,7-dihydroxy-9-methyl-2-oxo-1,2,5,6,7,9-hexahydropyrido [3',2':6,7]cyclohepta[1,2-f]indole-3-carboxylate

To a solution of methyl 4-(benzyloxy)-10-(((tert-butoxycarbonyl)(ethyl)amino)methyl)-1-(2,4-dimethoxybenzyl)-9-methyl-2-oxo-1,2,5,9-tetrahydropyrido[3',2':6,7]cyclohepta[1,2-f]indole-3-carboxylate (120 mg, 0.16 mmol) in acetone (1.0 mL), t-BuOH (1.0 mL) and H₂O (0.5 mL) was added NMO (30 mg, 0.26 mmol) followed by K₂OsO₄*2H₂O (3 mg, 0.008 mmol, 5 mol%). The reaction mixture was stirred at room temperature overnight and then the reaction was quenched with Na₂S₂O₃ (10% aq, 5 mL). The product was extracted with DCM (3x7 mL) and the organic phase was dried over Na₂SO₄. The solvents were removed and the residue was purified by column chromatography (EtOAc/DCM, 0-100% gradient). The product was obtained as a yellow solid (64.8 mg, 52 %).
¹H NMR (500 MHz, Acetone) δ ppm 0.98 (t, *J*=6.9 Hz, 3 H) 1.49 (s, 9 H) 1.95 (dd, *J*=14.8, 4.4 Hz, 1 H) 3.00 (dd, *J*=14.8, 0.6 Hz, 1 H) 3.20 (q, *J*=6.9 Hz, 2 H) 3.47 (s, 3 H) 3.79 (s, 3 H) 3.80 (s, 3 H) 3.83 (s, 3 H) 4.20 - 4.29 (m, 2 H) 4.43 (d, *J*=6.9 Hz, 1 H) 4.66 (s, 2 H) 4.85 (d, *J*=15.4 Hz, 1 H) 4.97 (dd, *J*=5.5, 2.7 Hz, 1 H) 5.09 (d, *J*=10.7 Hz, 1 H) 5.21 (dd, *J*=15.4, 0.8 Hz, 1 H) 5.40 (d, *J*=10.7 Hz, 1 H) 6.19 (br. s., 1 H) 6.46 (d, *J*=2.2 Hz, 1 H) 6.53 (dd, *J*=8.5, 2.2 Hz, 1 H) 7.10 (d, *J*=8.5 Hz, 1 H) 7.14 (s, 1 H) 7.32 - 7.37 (m, 1 H) 7.38 - 7.43 (m, 3 H) 7.52 - 7.56 (m, 2 H). LC-MS 768.8 [M+H]⁺ RT 1.57 min.

### Step 7: (cis)-methyl 10-(((tert-butoxycarbonyl)(ethyl)amino)methyl)-1-(2,4-dimethoxybenzyl)-4,6,7-trihydroxy-9-methyl-2-oxo-1,2,5,6,7,9-hexahydropyrido [3',2':6,7]cyclohepta[1,2-f]indole-3-carboxylate

A solution of (cis)-methyl 4-(benzyloxy)-10-(((tert-butoxycarbonyl)(ethyl)amino)methyl)-1-(2,4-dimethoxybenzyl)-6,7-dihydroxy-9-methyl-2-oxo-1,2,5,6,7,9-hexahydropyrido[3',2':6,7]cyclohepta[1,2-f]indole-3-carboxylate (86.0 mg, 0.11 mmol) in EtOAc was hydrogenated over Pd/C (10%, 20 mg) at 1 atm H₂ for 1 h until the starting material was consumed. The catalyst was then filtered off and the filtrate was washed with DCM. The mother liquor was concentrated and purified by column chromatography (EtOAc). The product (57.9 mg, 76%) was obtained as a yellowish solid. ¹H NMR (500 MHz, CHCl₃-*d*) δ ppm 1.00 (t, *J*=6.9 Hz, 3 H) 1.51 (s, 9 H) 1.93 (dd, *J*=15.1, 4.1 Hz, 1 H) 3.12 (d, *J*=15.1 Hz, 1 H) 3.15 - 3.24 (m, 2 H) 3.33 (s, 3 H) 3.74 (s, 3 H) 3.77 (s, 3 H) 4.01 (s, 3 H) 4.23 - 4.29 (m, 1 H) 4.58 - 4.68 (m, 2 H) 4.86 (d, *J*=6.0 Hz, 1 H) 5.20 - 5.33 (m, 2 H) 6.27 (d, *J*=2.2 Hz, 1 H) 6.31 (s, 1 H) 6.38 (dd, *J*=8.4, 2.2 Hz, 1 H) 6.97 (d, *J*=8.4 Hz, 1 H) 7.13 (s, 1 H) 7.37 (s, 1 H) . LC-MS 676.4 [M-H]⁻, 678.5 [M+H]⁺ RT 1.47 min.

### Steps 8-9: (cis)-10-((Ethylamino)methyl)-4,6,7-trihydroxy-9-methyl-2-oxo-1,2,5,6,7,9-hexahydropyrido[3',2':6,7]cyclohepta[1,2-f]indole-3-carboxylic acid hydrochloride

To a solution of (cis)-methyl 10-(((tert-butoxycarbonyl)(ethyl)amino)methyl)-1-(2,4-dimethoxybenzyl)-4,6,7-trihydroxy-9-methyl-2-oxo-1,2,5,6,7,9-hexahydropyrido[3',2':6,7]cyclohepta[1,2-f]indole-3-carboxylate (57.0 mg, 0.06 mmol) in EtOAc (1 mL) was added LiI (24.0 mg, 0.18 mmol). The mixture was heated at 60 °C for 2 h and monitored by LC/MS. After >90% conversion reaction was cooled to room temperature and acidified with 1M HCl (1 mL). The product was extracted with EtOAc (3x7 mL), then the organic phase was dried over Na₂SO₄ and the solvent was removed. The residue was purified by preparative HPLC affording (cis)-10-(((tert-butoxycarbonyl)(ethyl)amino)methyl)-4,6,7-trihydroxy-9-methyl-2-oxo-1,2,5,6,7,9-hexahydropyrido[3',2':6,7]cyclohepta[1,2-f]indole-3-carboxylic acid (16.4 mg, 29%). LC-MS 662.5 [M-H]⁻, 664.5 [M+H]⁺ RT 1.58 min.

To a solution of (cis)-10-(((tert-butoxycarbonyl)(ethyl)amino)methyl)-4,6,7-trihydroxy-9-methyl-2-oxo-1,2,5,6,7,9-hexahydropyrido[3',2':6,7]cyclohepta[1,2-f]indole-3-carboxylic acid (16.4 mg, 0.025 mmol) obtained above in DCM (0.5 mL) was added i-Pr₃SiH (0.1 mL) followed by TFA (0.2 mL). The mixture was stirred at room temperature for 5 h and monitored by LC/MS. Once complete removal of both protecting groups was observed, the solvent was removed under reduced pressure. Addition of HCl solution (2M Et₂O, 0.50 mL) to the oily residue resulted in precipitate formation. The mixture was diluted with Et₂O and the resulting solid was filtered and washed with Et₂O. The product HCl salt was obtained as a pale yellow solid (6.0 mg, 16% over 2 steps).
¹H NMR (500 MHz, DMSO-*d*₆) δ ppm 1.27 (t, *J*=7.3 Hz, 3 H) 1.40 - 1.52 (m, 1 H) 3.02 - 3.12 (m, 4 H) 3.86 (s, 3 H) 4.27 (ddd, *J*=10.4, 6.3, 4.4 Hz, 1 H) 4.44 (br. s., 2 H) 4.51 (d, *J*=4.4 Hz, 1 H) 4.94 (br. s., 1 H) 5.29 (br. s., 1 H) 6.81 (s, 1 H) 7.74 (s, 1 H) 7.82 (s, 1 H) 9.17 (br. s., 2 H) 12.92 (br. s., 1 H) 13.85 (br. s., 1 H). LC-MS 412.2 [M+H]⁺, 414.3 [M+H]⁺ RT 0.59 min.

### Example 72

### 1-benzyl-8-chloro-5-methyl-2-oxo-2,5-dihydro-1H-chromeno[4,3-b]pyridine-3-carboxylic acid (Cpd 72)

### Step 1: N-benzyl-2-(but-3-yn-2-yloxy)-4-chlorobenzamide

To a solution of N-benzyl-4-chloro-2-hydroxybenzamide (2.55 g, 9.74 mmol), but-3-yn-2-ol (1.00 mL, 12.70 mmol) and PPh₃ (3.30 g, 12.58 mmol) in THF (40 mL) at 0 °C was added DIAD (2.50 mL, 12.61 mmol) dropwise. The reaction was slowly allowed to warm to room temperature and stirred overnight. The THF was removed under reduced pressure and the residue was purified by column chromatography using EtOAc/hexanes (gradient 0-40%). The product N-benzyl-2-(but-3-yn-2-yloxy)-4-chlorobenzamide (2.80 g, 92%) was obtained as a colorless solid.
¹H NMR (500 MHz, CHCl₃-*d*) δ ppm 1.55 (d, *J*=6.5 Hz, 3 H) 2.56 (d, *J*=2.2 Hz, 1 H) 4.61 (dd, *J*=14.8, 5.4 Hz, 1 H) 4.70 (dd, *J*=14.8, 5.4 Hz, 1 H) 4.93 (qd, *J*=6.5, 2.2 Hz, 1 H) 7.12 (dd, *J*=8.5, 1.9 Hz, 2 H) 7.15 (d, *J*=1.9 Hz, 1 H) 7.28 - 7.32 (m, 1 H) 7.33 - 7.41 (m, 4 H) 7.94 (br. s., 1 H) 8.18 (d, *J*=8.5 Hz, 1 H). LC-MS 314.1/316.2 [M+H]⁺, RT 1.36 min.

### Step 2: N-benzyl-2-(but-3-yn-2-yloxy)-4-chlorobenzothioamide

To N-benzyl-2-(but-3-yn-2-yloxy)-4-chlorobenzamide (2.72 g, 8.67 mmol) was added THF (50 mL) followed by Lawesson's reagent (2.10 g, 5.19 mmol). The reaction was heated at 60 °C for 1 h. The THF was removed and the residue was purified by column chromatography using EtOAc/hexanes (gradient 0-20%). The product N-benzyl-2-(but-3-yn-2-yloxy)-4-chlorobenzothioamide (2.82 g, 99%) was obtained as a yellow solid.
¹H NMR (500 MHz, CHCl₃-*d*) δ ppm 1.40 (d, *J*=6.6 Hz, 3 H) 2.52 (d, *J*=1.9 Hz, 1 H) 4.87 (qd, *J*=6.6, 1.9 Hz, 1 H) 4.95 (dd, *J*=15.1, 4.7 Hz, 1 H) 5.04 (dd, *J*=15.1, 4.7 Hz, 1 H) 7.08 (dd, *J*=8.5, 1.9 Hz, 1 H) 7.10 (d, *J*=1.9 Hz, 1 H) 7.31 - 7.46 (m, 5 H) 8.46 (d, *J*=8.5 Hz, 1 H) 9.19 (br. s., 1 H). LC-MS 330.0/332.0 [M+H]⁺, RT 1.42 min.

### Step 3: ethyl 1-benzyl-8-chloro-5-methyl-2-oxo-2,5-dihydro-1H-chromeno[4,3-b]pyridine-3-carboxylate

To a solution of *N*-benzyl-2-(but-3-yn-2-yloxy)-4-chlorobenzothioamide (2.17 g, 6.58 mmol) and NEt₃ (3.0 mL, 21.52 mmol) in toluene (90 mL) was added ethyl 2-bromo-3-chloro-3-oxopropanoate (2.40 g, 10.46 mmol) dropwise over 10 min. The reaction stirred at room temperature for 2.5 h until complete consumption of starting material was observed. Then reaction mixture was heated to reflux for 3 h to complete cycloaddition. After cooling to room metperature, the mixture was diluted with EtOAc (100 mL) and the resulting solid was filtered off. The mother liquor was concentrated and the residue was purified by column chromatography using EtOAc/hexanes (gradient 0-60%). The product ethyl 1-benzyl-8-chloro-5-methyl-2-oxo-2,5-dihydro-1H-chromeno[4,3-b]pyridine-3-carboxylate (1.77 g, 66%) was obtained.
¹H NMR (500 MHz, CHCl₃-*d*) δ ppm 1.40 (t, *J*=7.1 Hz, 3 H) 1.57 (br. s, 3 H) 4.40 (qd, *J*=7.1, 2.2 Hz, 2 H) 4.94 - 5.12 (m, 1 H) 5.31 (br. s, 2 H) 6.86 (dd, *J*=8.5, 2.2 Hz, 1 H) 7.10 (d, *J*=2.2 Hz, 1 H) 7.19 (d, *J*=7.3 Hz, 2 H) 7.29 - 7.41 (m, 4 H) 8.12 (s, 1 H). LC-MS 410.1/412.0 [M+H]⁺, RT 1.46 min.

### Step 4: 1-benzyl-8-chloro-5-methyl-2-oxo-2,5-dihydro-1H-chromeno[4,3-b]pyridine-3-carboxylic acid

To a suspension of ethyl 1-benzyl-8-chloro-5-methyl-2-oxo-2,5-dihydro-1H-chromeno[4,3-b]pyridine-3-carboxylate (1.77 g, 4.31 mmol) in THF (15 mL) was added LiOH solution (1M aq, 6.0 mL, 6.0 mmol). The reaction was heated at 50 °C for 30 min. After cooling to room temperature reaction was acidified with 1 M HCl to pH∼2. The solid was filtered and washed with H₂O and Et₂O. After drying, the desired product 1-benzyl-8-chloro-5-methyl-2-oxo-2,5-dihydro-1H-chromeno[4,3-b]pyridine-3-carboxylic acid (1.54 g, 93%) was obtained.
¹H NMR (500 MHz, CHCl₃-*d*) δ ppm 1.58 (s, 3 H) 5.03 - 5.17 (m, 1 H) 5.34 - 5.71 (m, 2 H) 6.94 (dd, *J*=8.7, 2.0 Hz, 1 H) 7.18 (d, *J*=2.0 Hz, 1 H) 7.21 (d, *J*=7.3 Hz, 2 H) 7.37 (d, *J*=8.7 Hz, 1 H) 7.39 - 7.43 (m, 1 H) 7.45 - 7.50 (m, 2 H) 8.48 (s, 1 H) 14.12 (s, 1 H). LC-MS 382.0/384.0 [M+H]⁺, RT 1.41 min.

### Example 73

### 5-methyl-2-oxo-2,5-dihydro-1H-chromeno[4,3-b]pyridine-3-carboxylic acid (Cpd 73)

A suspension of 1-benzyl-8-chloro-5-methyl-2-oxo-2,5-dihydro-1H-chromeno[4,3-b]pyridine-3-carboxylic acid (Example 72, 62.0 mg, 0.16 mmol) in MeOH (3 mL) and AcOH (1 mL) was hydrogenated over Pd(OH)₂/C (20%, 30 mg) at 60 psi for 4 h. The catalyst was filtered off and the filtrate was washed with MeOH. After concentration of mother liquor, the residue was triturated with Et₂O and solid was filtered and washed with Et₂O. The product (13.4 mg, 32%) was obtained as a white solid.
¹H NMR (500 MHz, DMSO-*d*₆) δ ppm 1.52 (d, *J*=6.0 Hz, 3 H) 5.45 (q, *J*=6.0 Hz, 1 H) 7.05 (d, *J*=7.4 Hz, 1 H) 7.13 (t, *J*=7.4 Hz, 1 H) 7.48 (t, *J*=7.4 Hz, 1 H) 8.16 (d, *J*=7.4 Hz, 1 H) 8.27 (s, 1 H). LC-MS 256.1 [M-H]⁻, 258.0 [M+H]⁺, RT 0.95 min.

### Example 74

### 5-methyl-2-oxo-8-(pyrrolidin-1-yl)-2,5-dihydro-1H-chromeno[4,3-b]pyridine-3-carboxylic acid (Cpd 74)

1-benzyl-8-chloro-5-methyl-2-oxo-2,5-dihydro-1H-chromeno[4,3-b]pyridine-3-carboxylic acid (Example 72, 0.206 g, 0.54 mmol), 2-(2'-di-*tert-*butylphosphine)biphenylpalladium(II) acetate (12.5 mg, 5 mol%) and t-BuONa (0.12 g, 1.25 mmol) were mixed under an Argon in a heat-gun dried vial. Anhydrous toluene (2.5 mL) was added to the mixture followed by pyrrolidine (60 µL, 0.73 mmol). The reaction was heated under Argon at 80 °C for 1 h, then additional Pd catalyst (7.0 mg) was added. After heating for another 2 h at 80 °C, complete consumption of the starting material was observed. The reaction was cooled to room temperature and then quenched with HCl (1M aq, 10 mL). The product was extracted with DCM (3x10 mL). After drying the organic phase over Na₂SO₄, the solvents were removed. The residue was triturated with Et₂O and the resulting solid was filtered and dried. The obtained material (0.170 g) was washed with MeOH (∼2 mL) to afford 1-benzyl-5-methyl-2-oxo-8-(pyrrolidin-1-yl)-2,5-dihydro-1H-chromeno[4,3-b]pyridine-3-carboxylic acid (0.110 g, 49%).
¹H NMR (500 MHz, DMSO-*d*₆) δ ppm 1.55 (br. s., 3 H) 1.92 (t, *J*=6.3 Hz, 4 H) 3.28 (t, *J*=6.3 Hz, 4 H) 5.15 (br. s., 1 H) 5.47 (br. s., 2 H) 6.13 - 6.24 (m, 2 H) 7.12 - 7.25 (m, 3 H) 7.33 (t, *J*=7.3 Hz, 1 H) 7.43 (t, *J*=7.3 Hz, 2 H) 8.25 (s, 1 H) 14.39 (br. s., 1 H). LC-MS 417.1 [M+H]⁺, RT 1.46 min.

A solution of 1-benzyl-5-methyl-2-oxo-8-(pyrrolidin-1-yl)-2,5-dihydro-1H-chromeno[4,3-b]pyridine-3-carboxylic acid (0.100 g, 0.24 mmol) in MeOH (5 mL) and AcOH (5 mL) was hydrogenated over Pd(OH)₂/C (20%, 50 mg) at 60 psi of H₂ overnight. The product precipitated out from the mixture. The solvents were carefully decanted and the product was dissolved in DCM. The catalyst was filtered off through Celite and the filtrate was washed with DCM. The mother liquor was concentrated, the residue was triturated with Et₂O and the solid was filtered to provide 5-methyl-2-oxo-8-(pyrrolidin-1-yl)-2,5-dihydro-1H-chromeno[4,3-b]pyridine-3-carboxylic acid (0.050 g, 64%).
¹H NMR (500 MHz, CHCl₃-*d*) δ ppm 1.67 (d, *J*=6.6 Hz, 3 H) 2.04 - 2.09 (m, 4 H) 3.35 - 3.44 (m, 4 H) 5.19 (d, *J*=6.6 Hz, 1 H) 6.11 (d, *J*=2.2 Hz, 1 H) 6.41 (dd, *J*=8.8, 2.2 Hz, 1 H) 7.74 (d, *J*=8.8 Hz, 1 H) 8.25 (s, 1 H) 12.37 (br. s., 1 H) 13.88 (br. s., 1 H). LC-MS 325.1 [M-H]⁻, 327.1 [M+H]⁺, RT 1.15 min.

### Example 75

### 8-(3-(dimethylamino)pyrrolidin-1-yl)-5-methyl-2-oxo-2,5-dihydro-1H-chromeno[4,3-b]pyridine-3-carboxylic acid hydrochloride (Cpd 75)

Title compound was prepared according to the two step procedure described for Example 74 from 1-benzyl-8-chloro-5-methyl-2-oxo-2,5-dihydro-1H-chromeno[4,3-b]pyridine-3-carboxylic acid (Example 72) to provide the title compound (26%).
¹H NMR (500 MHz, DMSO-*d*₆) δ ppm 1.50 (d, *J*=6.3 Hz, 3 H) 2.22 - 2.37 (m, 1 H) 2.35 - 2.47 (m, 1 H) 2.81 (s, 6 H) 3.33 - 3.43 (m, 1 H) 3.52 - 3.67 (m, 2 H) 3.74 - 3.83 (m, 1 H) 3.89 - 4.04 (m, 1 H) 5.33 (q, *J*=6.3 Hz, 1 H) 6.21 (s, 1 H) 6.43 (d, *J*=8.5 Hz, 1 H) 8.02 (d, *J*=8.5 Hz, 1 H) 8.12 (s, 1 H) 11.14 (br. s., 1 H) 12.93 (br. s., 1 H) 14.64 (br. s., 1 H) LC-MS 368.2 [M-H]⁻, 370.3 [M+H]⁺, RT 1.03 min (Polar Method).

### Example 76

### 9-(dimethylamino)-4-hydroxy-2-oxo-1,2,5,6-tetrahydrobenzo[2,3]oxepino[4,5-b]pyridine-3-carboxylic acid (Cpd 76)

### Steps 1-2: 4-(3-chlorophenoxy)butanoic acid

To a suspension of K₂CO₃ (7.6 g, 55 mmol) in acetone (100 mL) was added 3-chlorophenol (5.4 mL, 50mmol). After heating at 60 °C for 16 hrs, the reaction mixture was cooled to room temperature and filtered. The solid was rinsed with Et₂O and the combined filtrates were concentrated. The crude residue was dissolved in Et₂O (200 mL) and washed with 10% NaOH (100 mL). The organic layer was separated, dried with MgSO₄, then filtered, and concentrated to afford the product as a clear oil. Without further purication, obtained material was dissolved in EtOH (20 mL) and 10% NaOH was added. The cloudy solution was then heated to 70 °C for 30 min. The resulting clear solution was cooled to 0 °C and neutralized with concentrated HCl. The resulting oil was then extracted with CH₂Cl₂, dried with Na₂SO₄, then filtered, and concentrated to give the product as a clear oil (7.3 g, 70%).
¹H NMR (500 MHz, CHCl₃-*d*) δ ppm 2.09 - 2.20 (m, 2 H) 2.57 - 2.64 (m, 2 H) 4.04 (t, J=6.07 Hz, 2 H) 6.80 (ddd, *J*=8.35, 2.44, 0.87 Hz, 1 H) 6.91 (t, *J*=2.17 Hz, 1 H) 6.95 (ddd, *J*=7.92, 1.93, 0.87 Hz, 1 H) 7.21 (t, *J*=8.16 Hz, 1 H). LC-MS: 213.1 [M-H]⁻, RT 1.12 min.

### Step 3: 8-chloro-3,4-dihydrobenzo[b]oxepin-5(2H)-one

4-(3-chlorophenoxy)butanoic acid (6.5 g, 30.2 mmol) in PPA (40 g) was heated at 90 °C for 1 hr. The resulting orange solution was poured over ice. The resulting suspension was extracted with EtOAc (2 X 200 mL). The combined organic extracts were washed with 10% NaOH and brine, then dried over Na₂SO₄, filtered, and concentrated. The crude residue was purified on silica gel (9:1 Hexane/EA) to afford a product as a light yellow solid (2.5 g, 43%).
¹H NMR (500 MHz, CHCl₃-*d*) δ ppm 2.19 - 2.30 (m, 2 H) 2.88 - 2.95 (m, 2 H) 4.24 - 4.31 (m, 2 H) 7.09 - 7.13 (m, 2 H) 7.74 (dd, *J*=8.28, 0.47 Hz, 1 H). LC-MS: 198.1 [M+H]⁺, RT 1.22 min.

### Step 4: N-(8-chloro-3,4-dihydrobenzo[b]oxepin-5(2H)-ylidene)-1-(2,4-dimethoxyphenyl)methanamine

To a solution of 8-chloro-3,4-dihydrobenzo[b]oxepin-5(2H)-one(2.5 g, 12.8 mmol), dimethoxylbenzylamine (2.1 mL, 14 mmol), and Et₃N (5.3 mL, 39 mmol) in CH₂Cl₂ (25 mL) was added a 1M solution of TiCl₄ (7.7 mL, 7.7 mmol) dropwise at 0 °C over 30 min. After completion, the reaction mixture was stirred at room temperature overnight, then quenched with saturated NaHCO₃. The product was extracted with DCM and the combined organic phases were dried over Na₂SO₄, then filtered and concentrated to give a crude product that was used immediately in the next step without further purification. LC-MS: 348.3 [M+H]⁺, RT 0.87 min.

### Step 5: methyl 9-chloro-1-(2,4-dimethoxybenzyl)-4-hydroxy-2-oxo-1,2,5,6-tetrahydrobenzo[2,3]oxepino[4,5-b]pyridine-3-carboxylate

A solution of *N*-(8-chloro-3,4-dihydrobenzo[b]oxepin-5(2H)-ylidene)-1-(2,4-dimethoxyphenyl)methanamine (4.4 g crude) and trimethyl methanetricarboxylate (4.9 g, 25.6 mmol) in Ph₂O (13 mL) was heated at 230 °C for 15 min. The reaction mixture was cooled to room temperature, and purified on silica gel using EtOAc/Hex (0-60% gradient) to afford the product (2.6 g, 43%) as a yellow foam.
¹H NMR (500 MHz, CHCl₃-*d*) δ ppm 2.06 - 2.17 (m, 1 H) 3.18 (dd, *J*=15.09, 3.90 Hz, 1 H) 3.63 - 3.68 (m, 3 H) 3.75 - 3.79 (m, 3 H) 3.99 (s, 3 H) 4.37 (dd, *J*=9.81, 6.34 Hz, 1 H) 4.41 - 4.50 (m, 1 H) 4.93 - 5.04 (m, 1 H) 5.15 (d, *J*=15.76 Hz, 1 H) 6.32 - 6.44 (m, 2 H) 6.94 (d, *J*=8.35 Hz, 1 H) 7.06 - 7.12 (m, 2 H) 7.17 (s, 1 H) 13.80 (s, 1 H). LC-MS: 470.1 [M-H]⁻, RT 1.45 min.

### Steps 6-7 9-(dimethylamino)-4-hydroxy-2-oxo-1,2,5,6-tetrahydrobenzo[2,3]oxepino[4,5-b]pyridine-3-carboxylic acid

Methyl 9-chloro-1-(2,4-dimethoxybenzyl)-4-hydroxy-2-oxo-1,2,5,6-tetrahydrobenzo[2,3]oxepino[4,5-b]pyridine-3-carboxylate (0.3 g, 0.6 mmol), NaOtBu (173 mg, 0.18 mmol), and 2-(2'-di-*tert*-butylphosphine)biphenylpalladium(II) acetate (3 mg) were weighed into a vial and degassed with Argon. Toluene (6 mL) was added followed by Me₂NH (1.2 mL, 2M solution) and the vial was sealed under Argon and heated to 90 °C for 2 hrs. The reaction mixture was then cooled to room temperature, poured into 1M HCl and extracted with DCM. The combined organic phase was dried over Na₂SO₄, filtered, and concentrated. The crude residue was dissolved in DCM and triturated with Et₂O. The solid was then filtered to afford a semipure product which was dissolved in TFA (1 mL) and stirred at room temperature for 1 hr at which point MeOH (3 mL) was added and the precipitate filtered to afford pure material (42 mg, 23%) as yellow solid.
¹H NMR (500 MHz, DMSO-*d*₆) δ ppm 2.65 (t, *J*=6.11 Hz, 2 H) 3.01 (s, 6 H) 4.46 (t, *J*=6.15 Hz, 2 H) 6.47 (d, *J*=2.52 Hz, 1 H) 6.64 (dd, *J*=8.83, 2.52 Hz, 1 H) 7.40 (d, *J*=8.83 Hz, 1 H) 12.68 - 12.79 (m, 1 H) 13.79 (br. s., 1 H). LC-MS: 317.1 [M+H]⁺, RT 1.25 min.

### Example 77

### 4-hydroxy-2-oxo-9-(pyrrolidin-1-yl)-1,2,5,6-tetrahydrobenzo[2,3]oxepino[4,5-b]pyridine-3-carboxylic acid (Cpd 77)

The title compound was prepared from methyl 9-chloro-1-(2,4-dimethoxybenzyl)-4-hydroxy-2-oxo-1,2,5,6-tetrahydrobenzo[2,3]oxepino[4,5-b]pyridine-3-carboxylate (Example 76, step 5) and pyrrolidine according to the two step procedure described for Example 76 (steps 6-7)
¹H NMR (500 MHz, DMSO-*d*₆) δ ppm 1.92 - 2.04 (m, 4 H) 2.65 (t, *J*=1.00 Hz, 2 H) 3.27 - 3.33 (m, 4 H) 4.45 (t, *J*=6.23 Hz, 2 H) 6.31 (d, *J*=2.36 Hz, 1 H) 6.44 - 6.52 (m, 1 H) 7.39 (d, *J*=8.67 Hz, 1 H) 12.67 - 12.79 (m, 1 H) 13.73 - 13.85 (m, 1 H). LC-MS: 343.2 [M+H]⁺, RT 1.29 min.

### Example 78

### 9-(3-(dimethylamino)pyrrolidin-1-yl)-4-hydroxy-2-oxo-1,2,5,6-tetrahydrobenzo[2,3]oxepino[4,5-b]pyridine-3-carboxylic acid hydrochloride (Cpd 78)

The title compound was prepared from methyl 9-chloro-1-(2,4-dimethoxybenzyl)-4-hydroxy-2-oxo-1,2,5,6-tetrahydrobenzo[2,3]oxepino[4,5-b]pyridine-3-carboxylate (Example 76, step 5) and 3-dimethylaminopyrrolidine according to the two step procedure described for Example 76 (steps 6-7). The final product was obtained as the hydrochloride salt after treatment with HCl/Et₂O.
¹H NMR (500 MHz, DMSO-*d*₆) δ ppm 2.29 - 2.40 (m, 1 H) 2.40 - 2.49 (m, 1 H) 2.65 (t, *J*=6.15 Hz, 2 H) 2.82 (dd, *J*=7.05, 4.93 Hz, 6 H) 3.30 - 3.39 (m, 2 H) 3.55 - 3.66 (m, 2 H) 3.71 - 3.80 (m, 1 H) 4.47 (t, *J*=6.23 Hz, 2 H) 6.42 (d, *J*=2.29 Hz, 1 H) 6.56 (dd, *J*=8.83, 2.29 Hz, 1 H) 7.44 (d, *J*=8.75 Hz, 1 H) 11.44 - 11.58 (m, 1 H) 12.73 - 12.86 (m, 1 H) 13.74 - 13.89 (m, 1 H). LC-MS 386.2 [M+H]⁺, RT 0.76 min.

### Example 79

### 9-((cis,cis)-6-(dibenzylamino)-3-azabicyclo[3.1.0]hexan-3-yl)-4-hydroxy-2-oxo-1,2,5,6-tetrahydrobenzo[2,3]oxepino[4,5-b]pyridine-3-carboxylic acid trifluoroacetate (Cpd 79)

The title compound was prepared from methyl 9-chloro-1-(2,4-dimethoxybenzyl)-4-hydroxy-2-oxo-1,2,5,6-tetrahydrobenzo[2,3]oxepino[4,5-b]pyridine-3-carboxylate (Example 76, step 5) and (cis,cis)-*N*,*N*-dibenzyl-3-azabicyclo[3.1.0]hexan-6-amine according to the two step procedure described for Example 76 (steps 6-7) to provide the final product as the trifluoracetate salt.
¹H NMR (500 MHz, DMSO-*d*₆) δ ppm 1.72 - 1.86 (m, 2 H) 2.62 (s, 2 H) 3.10 - 3.19 (m, 2 H) 3.34 (br. d, *J*=1.00 Hz, 2 H) 3.75 (br. s., 3 H) 4.45 (s, 2 H) 6.27 (d, *J*=2.36 Hz, 1 H) 6.39 - 6.46 (m, 1 H) 7.38 (d, *J*=8.67 Hz, 1 H) 7.50 (br. s., 5 H) 7.58 - 7.71 (m, 4 H) 12.74 - 12.82 (m, 1 H) 13.74 - 13.83 (m, 1 H). LC-MS: 550.2 [M+H]⁺, RT 0.88 min.

### Example 80

### 9-((cis,cis)-6-amino-3-azabicyclo[3.1.0]hexan-3-yl)-4-hydroxy-2-oxo-1,2,5,6-tetrahydrobenzo[2,3]oxepino[4,5-b]pyridine-3-carboxylic acid hydrochloride (Cpd 80)

To a solution of 9-((cis,cis)-6-(dibenzylamino)-3-azabicyclo[3.1.0]hexan-3-yl)-4-hydroxy-2-oxo-1,2,5,6-tetrahydrobenzo[2,3]oxepino[4,5-b]pyridine-3-carboxylic acid (Example 79, 60 mg, .1 mmol) in MeOH (5 mL) was added 20% Pd(OH)₂/C (100 mg). The mixture was stirred under H₂ (1 atm) for 3 hrs. The suspension was then filtered through Celite and concentrated. The residue was treated with HCl/Et₂O, then the solid was filtered and washed with Et₂O to afford the product HCl salt (44 mg, 99%) as a bright yellow solid.
¹H NMR (500 MHz, DMSO-*d*₆) δ ppm 2.17 (br. s., 2 H) 2.40 - 2.46 (m, 1 H) 2.63 (t, *J*=6.27 Hz, 2 H) 3.35 - 3.43 (m, 3 H) 3.62 (d, *J*=10.09 Hz, 2 H) 4.45 (t, *J*=6.19 Hz, 2 H) 6.37 (d, *J*=2.36 Hz, 1 H) 6.48 - 6.54 (m, 1 H) 7.39 (d, *J*=8.75 Hz, 1 H) 8.46 (br. s., 2 H) 12.71 - 12.83 (m, 1 H) 13.73 - 13.87 (m, 1 H). LC-MS: 370.3 [M+H]⁺, RT 0.71 min.

### Example 81

### 2-oxo-9-(pyrrolidin-1-yl)-1,2,5,6-tetrahydrobenzo[2,3]oxepino[4,5-b]pyridine-3-carboxylic acid (Cpd 81)

### Step 1: methyl 9-chloro-1-(2,4-dimethoxybenzyl)-2-oxo-1,2,5,6-tetrahydrobenzo[2,3]oxepino[4,5-b]pyridine-3-carboxylate

A solution of *N*-(8-chloro-3,4-dihydrobenzo[b]oxepin-5(2H)-ylidene)-1-(2,4-dimethoxyphenyl)methanamine (Example 76, step 4, 1.5 g crude, 4.4 mmol) and dimethyl 2-(methoxymethylene)malonate (1.5 g, 8.6 mmol) in Ph₂O (5 mL) was heated at 230 °C for 15 min. The reaction mixture was then cooled to room temperature and purified on silica gel using EtOAc/Hex (0-60% gradient) to afford the product (1.1 g, 56%) as a yellow foam.
¹H NMR (500 MHz, CHCl₃-*d*) δ ppm 2.45 - 2.57 (m, 2 H) 3.65 (s, 3 H) 3.77 (s, 3 H) 3.92 (s, 3 H) 4.34 - 4.43 (m, 1 H) 4.43 - 4.53 (m, 1 H) 5.10 (d, *J*=15.29 Hz, 1 H) 5.28 (d, *J*=15.37 Hz, 1 H) 6.33 - 6.43 (m, 2 H) 6.95 (d, *J*=8.35 Hz, 1 H) 7.08 - 7.15 (m, 2 H) 7.20 (dd, *J*=1.77, 0.59 Hz, 1 H) 8.15 (s, 1 H).

### Step 2: 9-chloro-1-(2,4-dimethoxybenzyl)-2-oxo-1,2,5,6-tetrahydrobenzo[2,3]oxepino[4,5-b]pyridine-3-carboxylic acid

To a solution of methyl 9-chloro-1-(2,4-dimethoxybenzyl)-2-oxo-1,2,5,6-tetrahydrobenzo[2,3]oxepino[4,5-b]pyridine-3-carboxylate (1.1 g, 2.4 mmol) in THF was added aqueous LiOH (3.0 mL, 1M). The solution was heated to 60 °C for 1 hr and then cooled to room temperature. The reaction mixture was poured into 1H HCl (10 mL) and extracted with DCM (20 mL). The organic phase was dried with Na₂SO₄, filtered, and concentrated to afford a product (0.75 g, 71 %) as a yellow solid.
¹H NMR (500 MHz, CHCl₃-*d*) δ ppm 2.55 - 2.67 (m, 2 H) 3.68 (s, 3 H) 3.81 (s, 3 H) 4.40 - 4.47 (m, 1 H) 4.47 - 4.56 (m, 1 H) 5.19 (d, *J*=15.37 Hz, 1 H) 5.37 (d, *J*=15.45 Hz, 1 H) 6.37 - 6.46 (m, 2 H) 6.88 (d, *J*=8.35 Hz, 1 H) 7.12 - 7.23 (m, 2 H) 7.26 (d, *J*=1.89 Hz, 1 H) 8.48 (s, 1 H). LC-MS: 442.3 [M+H]⁺, RT 1.39 min.

### Steps 3-4: 2-oxo-9-(pyrrolidin-1-yl)-1,2,5,6-tetrahydrobenzo[2,3]oxepino[4,5-b]pyridine-3-carboxylic acid

9-chloro-1-(2,4-dimethoxybenzyl)-2-oxo-1,2,5,6-tetrahydrobenzo[2,3]oxepino[4,5-b]pyridine-3-carboxylic acid (150 mg, 0.34 mmol), NaOtBu (100 mg, 1.02 mmol), and 2-(2'-di-*tert*-butylphosphine)biphenylpalladium(II) acetate (3 mg) were weighed into a vial and degassed with Argon. Toluene (3 mL) was added followed by Pyrrolidine (60 µL, 0.68 mmol) and the vial was sealed under Argon and heated to 90 °C for 2 hrs. The reaction mixture was then cooled to room temperature, poured into 1M HCl and extracted with DCM. The combined organic phase was dried over Na₂SO₄, filtered, and concentrated. The crude residue was dissolved in DCM and triturated with Et₂O. The solid was then filtered to afford a semipure solid which was dissolved in TFA (1 mL) and stirred at room temperature for 1 hr at which point MeOH (3 mL) was added and the precipitate was filtered to afford a product (42 mg, 23%) as a yellow solid.
¹H NMR (500 MHz, DMSO-*d*₆) δ ppm 1.94 - 2.01 (m, 4 H) 2.70 (t, *J*=6.07 Hz, 2 H) 3.31 (t, *J*=6.42 Hz, 4 H) 4.47 (t, *J*=6.19 Hz, 2 H) 6.31 (d, *J*=2.29 Hz, 1 H) 6.48 (dd, *J*=8.75, 2.29 Hz, 1 H) 7.41 (d, *J*=8.75 Hz, 1 H) 8.32 (s, 1 H) 13.18 - 13.25 (m, 1 H). LC-MS: 327.1 [M+H]⁺, RT 0.70 min.

### Example 82

### 9-(3-(dimethylamino)pyrrolidin-1-yl)-2-oxo-1,2,5,6-tetrahydrobenzo[2,3]oxepino[4,5-b]pyridine-3-carboxylic acid hydrochloride (Cpd 82)

9-chloro-1-(2,4-dimethoxybenzyl)-2-oxo-1,2,5,6-tetrahydrobenzo[2,3]oxepino[4,5-b]pyridine-3-carboxylic acid (Example 81, step 2, 150 mg, 0.34 mmol), NaOtBu (100 mg, 1.02 mmol), and 2-(2'-di-*tert*-butylphosphine)biphenylpalladium(II) acetate (3 mg) were weighed into a vial and degassed with Argon. Toluene (3 mL) was added followed by N,N-dimethylpyrrolidin-3-amine (100 µL, 0.68 mmol) and the vial was sealed under Argon and heated to 90 °C for 2 hrs. The reaction mixture was then cooled to room temperature, poured into 1M HCl and extracted with DCM. The combined organic phase was dried over Na₂SO₄, filtered, and concentrated. The crude residue was dissolved in DCM and triturated with Et₂O. The solid was then filtered to afford a semipure solid which was dissolved in TFA (1 mL) and stirred at room temperature for 1 hr at which point, excess TFA was removed from the reaction mixture. A solution of 2M HCl in Et₂O was added. The orange solid which precipitated was filtered off and rinsed with Et₂O to afford a product (80 mg, 62%).
¹H NMR (500 MHz, DMSO-*d*₆) δ ppm 2.24 - 2.35 (m, 1 H) 2.41 - 2.49 (m, 1 H) 2.71 (s, 2 H) 2.84 (t, *J*=5.52 Hz, 6 H) 3.28 - 3.40 (m, 1 H) 3.54 - 3.65 (m, 2 H) 3.70 - 3.80 (m, 1 H) 3.94 - 4.06 (m, 1 H) 4.49 (s, 2 H) 6.42 (d, *J*=2.36 Hz, 1 H) 6.51 - 6.63 (m, 1 H) 7.46 (d, *J*=8.67 Hz, 1 H) 8.35 (s, 1 H) 11.00 - 11.10 (m, 1 H).. LC-MS: 370.1 [M+H]⁺, RT 0.39 min.

### Example 83

### 4-hydroxy-5-methyl-2-oxo-9-(pyrrolidin-1-yl)-1,2,5,6-tetrahydrobenzo[2,3]oxepino[4,5-b]pyridine-3-carboxylic acid (Cpd 83)

### Step 1: 4-bromo-2-(2-methylallyloxy)benzaldehyde

To a solution of 4-bromo-2-hydroxybenzaldehyde (10.0 g, 49.8 mmol) in DMF (25 mL), was added K₂CO₃ (7.6 g, 54.7 mmol) and 3-bromo-2-methylprop-1-ene (5.5 mL, 54.7 mmol). The reaction mixture was stirred at room temperature for 16 hrs and then poured into H₂O (100 mL). The aqueous phase was extracted with Et₂O (3 x 100 mL) and the combined organic phases were dried over MgSO₄, filtered, and concentrated to afford the title compound as a clear oil (11.8 g, 93%) which was used without further purification.
¹H NMR (500 MHz, CHCl₃-*d*) δ ppm 1.79 (d, *J*=0.55 Hz, 3 H) 4.47 (s, 2 H) 4.95 - 5.09 (m, 2 H) 7.04 - 7.14 (m, 2 H) 7.63 (d, *J*=8.28 Hz, 1 H) 10.39 (d, *J*=0.79 Hz, 1 H).

### Step 2: 1-(4-bromo-2-(2-methylallyloxy)phenyl)prop-2-en-1-ol

To a solution of 4-bromo-2-(2-methylallyloxy)benzaldehyde (1.05 g, 4.1 mmol) in THF (10 mL), cooled to 0 °C, was added vinylmagnesium bromide (4.5 mL, 4.5 mmol, 1M/THF). The solution was stirred at 0 °C for 30 min, warmed to room temperature, and then allowed to stir for an additional 30 min at which point the reaction was complete by TLC. The reaction was quenched by the addition of saturated NH₄Cl solution (5 mL) and then poured into H₂O (50 mL). The aqueous phase was extracted with Et₂O (3 x 50 mL) and the combined organic phases were dried over MgSO₄, filtered, and concentrated. The crude residue was purified on silica gel (3:2 Hexane/EA) to afford the title compound as a clear oil (84%).
¹H NMR (500 MHz, CHCl₃-*d*) δ ppm 1.86 (d, *J*=0.47 Hz, 3 H) 4.48 (s, 2 H) 5.05 (tt, *J*=1.61, 0.88 Hz, 1 H) 5.12 (t, *J*=1.18 Hz, 1 H) 5.20 (dt, *J*=10.40, 1.46 Hz, 1 H) 5.34 (dt, *J*=17.20, 1.53 Hz, 1 H) 5.43 - 5.49 (m, 1 H) 6.10 (ddd, *J*=17.18, 10.40,5.52 Hz, 1 H) 7.03 (d, *J*=1.81 Hz, 1 H) 7.13 (dd, *J*=8.12, 1.81 Hz, 1 H) 7.24 (dd, *J*=8.08, 0.51 Hz, 1 H). LC-MS: 283.4 [M-H]⁻, RT 1.39 min.

### Step 3: 8-bromo-3-methyl-2,5-dihydrobenzo[b]oxepin-5-ol

To a solution of 1-(4-bromo-2-(2-methylallyloxy)phenyl)prop-2-en-1-ol (5.0 g, 17.7 mmol) in CH₂Cl₂ (200 mL) was added Grubbs' catalyst second generation (400 mg, 3 mol%). The reaction mixture was stirred for 16 hrs at room temperature and then concentrated. The crude reaction mixture was purified on silica gel (1:1 Hexane/EA) to afford the title compound as an off-white solid.
¹H NMR (500 MHz, CHCl₃-*d*) δ ppm 1.58 - 1.63 (m, 3 H) 4.37 - 4.50 (m, 2 H) 5.37 (br. s., 1 H) 5.78 (dq, *J*=4.70, 1.66 Hz, 1 H) 7.20 - 7.23 (m, 1 H) 7.26 - 7.30 (m, 2 H).

### Step 4: 8-bromo-3-methylbenzo[b]oxepin-5(2H)-one

To a solution of 8-bromo-3-methyl-2,5-dihydrobenzo[b]oxepin-5-ol (3.5 g, 13.8 mmol) in CH₂Cl₂ (30 mL) was added MnO₂ (6.0 g, 69 mmol). The reaction mixture was stirred at room temperature for 16 hrs at which point the reaction was complete according to TLC. The reaction mixture was then filtered through Celite and concentrated to afford the title compound as a light brown solid (2.8 g, 80%).
¹H NMR (500 MHz, CHCl₃-*d*) δ ppm 2.05 - 2.10 (m, 3 H) 4.64 - 4.69 (m, 2 H) 6.32 - 6.36 (m, 1 H) 7.27 - 7.30 (m, 1 H) 7.33 (dd, *J*=8.51, 1.97 Hz, 1 H) 7.87 (dd, *J*=8.51, 0.24 Hz, 1 H). LC-MS: 253.1 [M+H]⁺, RT 0.92 min.

### Step 5: 8-bromo-3-methyl-3,4-dihydrobenzo[b]oxepin-5(2H)-one

To a solution of 8-bromo-3-methylbenzo[b]oxepin-5(2H)-one (2.5 g, 10 mmol) in EtOH/THF (5:1) (12 mL) was added PtO₂ (60 mg). The flask was evacuated and then back filled with H₂. The reaction was stirred at room temperature for 3 hrs at which point the reaction was complete by TLC. The reaction mixture was then filtered through Celite and concentrated to afford the title compound as a light brown oil (2.3 g, 91 %).
¹H NMR (500 MHz, DMSO-*d*₆) δ ppm 1.03 (d, *J*=7.80 Hz, 3 H) 2.53 - 2.58 (m, 2 H) 2.88 - 2.95 (m, 1 H) 3.87 (dd, *J*=12.30, 6.86 Hz, 1 H) 4.29 (dd, *J*=12.30, 5.44 Hz, 1 H) 7.33 (dd, *J*=8.35, 1.81 Hz, 1 H) 7.38 (d, *J*=1.81 Hz, 1 H) 7.54 (d, *J*=8.35 Hz, 1 H). LC-MS: 257.0 [M+H]⁺, RT 0.88 min.

### Step 6: N-(8-bromo-3-methyl-3,4-dihydrobenzo[b]oxepin-5(2H)-ylidene)-1-(2,4-dimethoxyphenyl)methanamine

To a solution of 8-bromo-3-methyl-3,4-dihydrobenzo[b]oxepin-5(2H)-one (2.5 g, 10.0 mmol), dimethoxylbenzylamine (1.6 mL, 11 mmol), and Et₃N (4.2 mL, 30 mmol) in CH₂Cl₂ (30 mL) was added a 1M solution of TiCl₄ (6.0 mL, 6.0 mmol) dropwise at 0 °C over 30 min. After completion of addition, the reaction mixture was stirred at room temperature overnight, then the reaction was quenched with saturated NaHCO₃. The product was extracted with DCM and the combined organic phases were dried over Na₂SO₄, then filtered and concentrated to give a crude *N*-(8-bromo-3-methyl-3,4-dihydrobenzo[b]oxepin-5(2H)-ylidene)-1-(2,4-dimethoxyphenyl)methanamine that was used immediately in the next step without further purification. LC-MS: 406.2 [M+H]⁺, RT 0.65 min.

### Step 7: methyl 9-bromo-1-(2,4-dimethoxybenzyl)-4-hydroxy-5-methyl-2-oxo-1,2,5,6-tetrahydrobenzo[2,3]oxepino[4,5-b]pyridine-3-carboxylate

A solution of *N*-(8-bromo-3-methyl-3,4-dihydrobenzo[b]oxepin-5(2H)-ylidene)-1-(2,4-dimethoxyphenyl)methanamine (3.7 g, crude) and trimethyl methanetricarboxylate (3.4 g, 18 mmol) in Ph₂O (10 mL) was heated at 230 °C for 15 min. The reaction mixture was cooled to room temperature and purified on silica gel using EtOAc/Hex (0-60% gradient) to afford the product methyl 9-bromo-1-(2,4-dimethoxybenzyl)-4-hydroxy-5-methyl-2-oxo-1,2,5,6-tetrahydrobenzo[2,3]oxepino[4,5-b]pyridine-3-carboxylate (2.6 g, 43%) as a brown foam.
¹H NMR (500 MHz, DMSO-*d*₆) δ ppm 0.56 (d, *J*=7.72 Hz, 3 H) 3.57 - 3.66 (m, 4 H) 3.68 - 3.72 (m, 3 H) 3.85 (s, 3 H) 4.14 (d, *J*=10.96 Hz, 1 H) 4.60 (dd, *J*=11.07, 6.66 Hz, 1 H) 4.90 (d, *J*=15.68 Hz, 1 H) 5.13 (br. s., 1 H) 6.35 - 6.46 (m, 2 H) 6.74 (d, *J*=8.43 Hz, 1 H) 7.31 - 7.51 (m, 3 H) 13.51 (br. s, 1 H).LC-MS: 530.2 [M-H]⁻, RT 0.92 min.

### Step 8-9: 4-hydroxy-5-methyl-2-oxo-9-(pyrrolidin-1-yl)-1,2,5,6-tetrahydrobenzo[2,3]oxepino[4,5-b]pyridine-3-carboxylic acid

Methyl 9-bromo-1-(2,4-dimethoxybenzyl)-4-hydroxy-5-methyl-2-oxo-1,2,5,6-tetrahydrobenzo[2,3]oxepino[4,5-b]pyridine-3-carboxylate (0.3 g, 0.6 mmol), NaOtBu (220 mg, 2.2 mmol), and 2-(2'-di-*tert*-butylphosphine)biphenylpalladium(II) acetate (12 mg) were weighed into a vial and degassed with Argon. Toluene (6 mL) was added followed by pyrrolidine (0.1 mL, 1.14 mmol) and the vial was sealed under Argon and heated to 90 °C for 2 hrs. The reaction mixture was then cooled to room temperature, poured into 1M HCl and extracted with DCM. The combined organic phase was dried over Na₂SO₄, filtered, and concentrated. The crude residue was dissolved in DCM and triturated with Et₂O. The solid was then filtered to afford a semipure solid which was dissolved in TFA (1 mL) and stirred at room temperature for 1 hr at which point MeOH (3 mL) was added and the precipitate was filtered to afford 4-hydroxy-5-methyl-2-oxo-9-(pyrrolidin-1-yl)-1,2,5,6-tetrahydrobenzo[2,3]oxepino[4,5-b]pyridine-3-carboxylic acid (170 mg, 70%) as a yellow solid.
¹H NMR (500 MHz, DMSO-*d*₆) δ ppm 0.97 (d, *J*=7.33 Hz, 2 H) 1.91 - 2.02 (m, 4 H) 3.31 (t, *J*=6.42 Hz, 4 H) 3.51 (s, 1 H) 4.30 (dd, *J*=11.39, 3.51 Hz, 1 H) 4.38 - 4.46 (m, 1 H) 6.22 (d, *J*=2.36 Hz, 1 H) 6.41 (dd, *J*=8.91, 2.36 Hz, 1 H) 7.46 (d, *J*=8.83 Hz, 1 H) 13.89 (br. s., 1 H). LC-MS: 357.3 [M+H]⁺, RT 1.36 min.

### Example 84

### 9-(3-(dimethylamino)pyrrolidin-1-yl)-4-hydroxy-5-methyl-2-oxo-1,2,5,6-tetrahydrobenzo[2,3]oxepino[4,5-b]pyridine-3-carboxylic acid hydrochloride (Cpd 84)

The title compound was prepared from methyl 9-bromo-1-(2,4-dimethoxybenzyl)-4-hydroxy-5-methyl-2-oxo-1,2,5,6-tetrahydrobenzo[2,3]oxepino[4,5-b]pyridine-3-carboxylate (Example 83, step 7) and 3-dimethylaminopyrrollidine according to the two step procedure described for Example 83 (steps 8-9). The final product was obtained as the hydrochloride salt after treatment with HCl/Et₂O.
¹H NMR (500 MHz, DMSO-*d*₆) δ ppm 0.97 (d, *J*=7.33 Hz, 3 H) 2.24 - 2.35 (m, 1 H) 2.41 - 2.49 (m, 1 H) 2.84 (br. s., 6 H) 3.29 - 3.41 (m, 2 H) 3.51 - 3.64 (m, 3 H) 3.69 - 3.79 (m, 1 H) 3.95 - 4.04 (m, 1 H) 4.28 - 4.35 (m, 1 H) 4.42 - 4.49 (m, 1 H) 6.34 (d, *J*=2.44 Hz, 1 H) 6.46 - 6.53 (m, 1 H) 7.51 (d, *J*=8.91 Hz, 1 H) 13.87 - 13.94 (m, 1 H). LC-MS: 400.3 [M+H]⁺, RT 0.76 min.

### Example 85

### 9-((cis,cis)-6-(dibenzylamino)-3-azabicyclo[3.1.0]hexan-3-yl)-4-hydroxy-5-methyl-2-oxo-1,2,5,6-tetrahydrobenzo[2,3]oxepino[4,5-b]pyridine-3-carboxylicacid hydrochloride (Cpd 85)

The title compound was prepared from methyl 9-bromo-1-(2,4-dimethoxybenzyl)-4-hydroxy-5-methyl-2-oxo-1,2,5,6-tetrahydrobenzo[2,3]oxepino[4,5-b]pyridine-3-carboxylate (Example 83, step 7) and (cis,cis)-*N*,*N*-dibenzyl-3-azabicyclo[3.1.0]hexan-6-amine according to the two step procedure described for Example 83 (steps 8-9). The final product was obtained as the hydrochloride salt after treatment with HCl/Et₂O.
¹H NMR (500 MHz, DMSO-*d*₆) δ ppm 0.95 (d, *J*=7.41 Hz, 3 H) 1.64 - 1.72 (m, 1 H) 3.13 - 3.20 (m, 2 H) 3.38 (d, *J*=10.09 Hz, 2 H) 3.48 - 3.63 (m, 4 H) 4.30 (s, 1 H) 4.38 - 4.46 (m, 1 H) 6.18 (d, *J*=2.05 Hz, 1 H) 6.33 - 6.39 (m, 1 H) 7.41 - 7.53 (m, 11 H) 13.86 - 13.93 (m, 1 H).LC-MS: 564.4 [M+H]⁺, RT 0.91 min.

### Example 86

### 9-((cis,cis)-6-amino-3-azabicyclo[3.1.0]hexan-3-yl)-4-hydroxy-5-methyl-2-oxo-1,2,5,6-tetrahydrobenzo[2,3]oxepino[4,5-b]pyridine-3-carboxylic acid hydrochloride (Cpd 86)

The title compound was prepared from 9-((cis,cis)-6-(dibenzylamino)-3-azabicyclo[3.1.0]hexan-3-yl)-4-hydroxy-5-methyl-2-oxo-1,2,5,6-tetrahydrobenzo[2,3]oxepino[4,5-b]pyridine-3-carboxylic acid (Example 85) according to the hydrogenation protocol described for Example 80. The final product was obtained as the hydrochloride salt after treatment with HCl/Et₂O.
¹H NMR (500 MHz, DMSO-*d*₆) δ ppm 0.96 (d, *J*=7.33 Hz, 3 H) 2.16 (br. s., 2 H) 2.42 - 2.48 (m, 1 H) 3.49 - 3.56 (m, 1 H) 3.62 (d, *J*=9.62 Hz, 2 H) 4.27 - 4.33 (m, 2 H) 4.39 - 4.46 (m, 2 H) 6.27 (d, *J*=2.36 Hz, 1 H) 6.41 - 6.48 (m, 1 H) 7.46 (d, *J*=8.91 Hz, 1 H) 8.38 - 8.44 (m, 2 H) 12.44 - 12.50 (m, 1 H) 13.86 - 13.94 (m, 1 H). LC-MS: 384.3 [M+H]⁺, RT 0.77 min.

### Example 87

### 4-hydroxy-9-methyl-2-oxo-2,5,6,9-tetrahydro-1H-pyrido[2',3':4,5]oxepino[3,2-f]indole-3-carboxylic acid (Cpd 87)

### Step 1: tert-butyl 5-oxo-2,3,4,5-tetrahydrobenzo[b]oxepin-8-ylcarbamate

8-bromo-3,4-dihydrobenzo[b]oxepin-5(2H)-one (2.8 g, 11.7 mmol), tert-butyl carbamate (2.05 g, 17.5 mmol), Cs₂CO₃ (5.7 g, 17.5 mmol), Pd(OAc)₂ (80 mg, 3 mol%), and S-Phos (240 mg, 5 mol%) were weighed out in a flask. The flask was degassed and back filled with Argon. Dioxane (12 mL) was added and the flask was sealed and heated at 100 °C for 24 hrs. The flask was then cooled to room temperature and the crude reaction mixture was filtered through a plug of Celite and concentrated. The crude residue was purified on silica gel (9:1 Hexane/EA) to afford the title compound as a light orange solid (2.0 g, 63%).
¹H NMR (500 MHz, DMSO-*d*₆) δ ppm 1.49 (s, 9 H) 2.05 - 2.12 (m, 2 H) 2.70 - 2.76 (m, 2 H) 4.18 (t, *J*=6.62 Hz, 2 H) 7.20 (dd, *J*=8.67, 2.05 Hz, 1 H) 7.28 (d, *J*=2.05 Hz, 1 H) 7.57 (d, *J*=8.59 Hz, 1 H) 9.74 (s, 1 H). LC-MS: 278.1 [M+H]⁺, RT 1.33 min.

### Step 2: tert-butyl methyl(5-oxo-2,3,4,5-tetrahydrobenzo[b]oxepin-8-yl)carbamate

To a solution of tert-butyl 5-oxo-2,3,4,5-tetrahydrobenzo[b]oxepin-8-ylcarbamate (2.0 g, 7.25 mmol), in DMF (10 mL) cooled to 0 °C was added NaH (60%) (320 mg, 8.0 mmol). Gas evolution was observed and the mixture was allowed to stir for 30 min at which point iodomethane (0.5 mL, 8.0 mmol) was added. After stirring for an additional 30 min at 0 °C, the reaction mixture was allowed to warm to room temperature. After stirring at room temperature for 1 hr, the reaction was quenched with saturated NH₄Cl (10 mL), poured into H₂O (100 mL), and extracted with Et₂O (3 x 100 mL). The combined organic phases were dried over MgSO₄, filtered, and concentrated. The crude residue was purified on silica gel (3:2 Hexane/EA) to afford the title compound as a light brown oil (1.82 g, 87%). ¹H NMR (500 MHz, DMSO-*d*₆) δ ppm 1.44 (s, 9 H) 2.08 - 2.16 (m, 2 H) 2.74 - 2.82 (m, 2 H) 3.22 (s, 3 H) 4.21 (t, *J*=6.54 Hz, 2 H) 7.07 (d, *J*=2.13 Hz, 1 H) 7.13 (dd, *J*=8.59, 2.21 Hz, 1 H) 7.60 (d, *J*=8.51 Hz, 1 H). LC-MS: 292.3 [M+H]⁺, RT 0.82.

### Steps 3-4: 7,9-diiodo-8-(methylamino)-3,4-dihydrobenzo[b]oxepin-5(2H)-one

To a solution of tert-butyl methyl(5-oxo-2,3,4,5-tetrahydrobenzo[b]oxepin-8-yl)carbamate (1.82 g, 6.3 mmol) in DCM (20 mL) was added TFA (4.0 mL). The reaction mixture was allowed to stir at room temperature for 24 hrs at which point the starting material was completely consumed according to LC-MS. The reaction mixture was then concentrated *in vacuo* and the crude residue was dissolved in DCM (20 mL). NIS (2.84 g, 12.6 mmol) was then added and the reaction mixture was allowed to stir at room temperature for 1 hr. The reaction mixture was then concentrated and the crude residue was purified on silica gel (4:1 Hexane/EA) to afford the title compound as a light red oil (2.45 g, 88%).
¹H NMR (500 MHz, CHCL₃-*d*) δ ppm 2.25 (quin, *J*=6.76 Hz, 2 H) 2.86 - 2.92 (m, 2 H) 3.11 (s, 3 H) 4.29 - 4.36 (m, 2 H) 8.24 (s, 1 H).LC-MS: 443.9 [M+H]⁺, RT 1.49 min.

### Step 5: 9-iodo-8-(methylamino)-7-((trimethylsilyl)ethynyl)-3,4-dihydrobenzo[b]oxepin-5(2H)-one

To a flask was added 7,9-diiodo-8-(methylamino)-3,4-dihydrobenzo[b]oxepin-5(2H)-one (2.0 g, 4.5 mmol), PdCl₂(PPh₃)₂ (64 mg, 2 mol%), and CuI (32 mg, 4 mol%). The flask was degassed and back filled with Argon. To the flask was then added CH₃CN (9 mL), triethylamine (1.3 mL, 9.0 mmol), and ethynyltrimethylsilane (0.77 mL, 5.4 mmol). The flask was then sealed under Argon and heated to 70 °C for 16 hrs. The reaction was then cooled to room temperature and concentrated. The crude residue was purified on silica gel (9:1 Hexane/EA) to afford the title compound as a light yellow solid (750 mg, 42%).
¹H NMR (500 MHz, DMSO-*d*₆) δ ppm -0.03 - 0.02 (m, 9 H) 1.87 (s, 2 H) 2.46 - 2.53 (m, 2 H) 3.09 (d, *J*=5.28 Hz, 3 H) 4.01 (t, *J*=6.66 Hz, 2 H) 5.49 - 5.59 (m, 1 H) 7.35 (s, 1 H). LC-MS: 414.1 [M+H]⁺, RT 1.68 min.

### Step 6: 10-iodo-9-methyl-3,4-dihydro-2H-oxepino[3,2-f]indol-5(9H)-one

To a flask was added 9-iodo-8-(methylamino)-7-((trimethylsilyl)ethynyl)-3,4-dihydrobenzo[b]oxepin-5(2H)-one (1.5 g, 3.6 mmol) and CuI (760 mg, 4.0 mmol). The flask was degassed and back filled with Argon. DMF (10 mL) was then added and the reaction mixture was heated 100 °C for 2 hrs. The reaction mixture was then cooled to room temperature, poured into H₂O (100 mL), and extracted with Et₂O (3 x 100 mL). The combined organic extracts were dried over MSO₄, filtered, and concentrated. The crude residue was purified on silica gel (4:1 Hexane/EA) to afford the title compound as a clear oil (0.8 g, 65 %).
¹H NMR (500 MHz, CHCl₃-*d*) δ ppm 1.92 - 2.00 (m, 2 H) 2.72 - 2.77 (m, 2 H) 4.03 - 4.07 (m, 3 H) 4.08 - 4.14 (m, 2 H) 6.33 (d, *J*=3.23 Hz, 1 H) 6.84 (d, *J*=3.31 Hz, 1 H) 7.86 (s, 1 H). LC-MS: 342.1 [M+H]⁺, RT 0.81 min.

### Step 7: 9-methyl-3,4-dihydro-2H-oxepino[3,2-f]indol-5(9H)-one

To a solution of 10-iodo-9-methyl-3,4-dihydro-2H-oxepino[3,2-f]indol-5(9H)-one (0.8 g, 2.3 mmol) in AcOH (5 mL) was added zinc dust (1 g, 15 mmol). The reaction mixture was stirred at room temperature for 1 hr and then filtered through Celite, eluting with EtOAc. The filtrate was then poured into H₂O (100 mL) and extracted with Et₂O (3 x 100 mL). The combined organic phases were dried over MgSO₄, filtered, and concentrated to afford the title compound as a light brown solid (500 mg, 99%).
¹H NMR (500 MHz, CHCl₃-*d*) δ ppm 2.14 - 2.20 (m, 2 H) 2.91 - 2.97 (m, 2 H) 3.76 (s, 3 H) 4.26 (t, *J*=6.78 Hz, 2 H) 6.55 (dd, *J*=3.19, 0.83 Hz, 1 H) 7.00 (s, 1 H) 7.06 (d, *J*=3.23 Hz, 1 H) 8.15 (s, 1 H). LC-MS: 216.2 [M+H]⁺, RT 0.71 min.

### Steps 8-9: methyl 4-hydroxy-9-methyl-2-oxo-2,5,6,9-tetrahydro-1H-pyrido[2',3':4,5]oxepino[3,2-f]indole-3-carboxylate

To a solution of 9-methyl-3,4-dihydro-2H-oxepino[3,2-f]indol-5(9H)-one (800 mg, 3.72 mmol) in DCM (10 mL) was added tert-butylamine (1.2 mL, 11.2 mmol). The reaction mixture was cooled to 0 °C and TiCl₄ (2.3 mL, 2.3 mmol) was added dropwise. Addition was complete after 20 min and the reaction mixture was allowed to warm to room temperature and stirred for an additional 16 hrs. The reaction was then quenched with saturated NaHCO₃ (10 mL) and poured into H₂O (50 mL). The aqueous phase was extracted with DCM (3 x 100 mL) and the combined organic phases were dried over Na₂SO₄, filtered, and concentrated to give a crude product that was used immediately without further purification.

A solution of the crude product obtained above and trimethyl methanetricarboxylate (1.0 g, 5.6 mmol) in Ph₂O (5 mL) was heated at 230 °C for 15 min. The reaction mixture was then cooled to room temperature at which point the product precipitates. The solid was filtered and rinsed with Et₂O (20 mL) to afford the title compound as a brown solid (480 mg, 38%).
¹H NMR (500 MHz, DMSO-*d*₆) δ ppm 2.56 (t, *J*=6.46 Hz, 2 H) 3.79 (s, 3 H) 4.38 (t, *J*=6.34 Hz, 2 H) 6.52 (dd, *J*=3.11, 0.75 Hz, 1 H) 7.29 (s, 1 H) 7.37 - 7.39 (m, 1 H) 7.74 (s, 1 H) 11.72 (br. s., 1 H) 13.59 (br. s., 1 H). LC-MS: 341.3 [M+H]⁺, 0.68 min.

### Step 10: 4-hydroxy-9-methyl-2-oxo-2,5,6,9-tetrahydro-1H-pyrido[2',3':4,5]oxepino[3,2-f]indole-3-carboxylic acid

To a suspension of methyl 4-hydroxy-9-methyl-2-oxo-2,5,6,9-tetrahydro-1H-pyrido[2',3':4,5]oxepino[3,2-f]indole-3-carboxylate (0.2 g, 0.6 mmol) in EtOAc (3 mL) was added LiI (0.24 g, 1.8 mmol) and the mixture was heated to 60 °C for 2 hrs. The reaction mixture was then cooled to room temperature and poured into 1M HCl (10 mL) and extracted with DCM (2 x 20 mL). The combined organic phases were dried over Na₂SO₄, filtered, and concentrated to afford the title compound as a brown solid (0.12 g, 61 %).
¹H NMR (500 MHz, DMSO-*d*₆) δ ppm 2.62 (t, *J*=6.38 Hz, 2 H) 3.81 (s, 3 H) 4.43 (t, *J*=6.38 Hz, 2 H) 6.56 (dd, *J*=3.11, 0.75 Hz, 1 H) 7.35 (s, 1 H) 7.43 (d, *J*=3.15 Hz, 1 H) 7.80 (s, 1 H) 13.86 (br. s, 1 H). LC-MS 327.2 [M+H]⁺, RT 0.73 min.

### Example 88

### 4-hydroxy-9-methyl-10-((methylamino)methyl)-2-oxo-2,5,6,9-tetrahydro-1H-pyrido[2',3':4,5]oxepino[3,2-f]indole-3-carboxylic acid hydrochloride (Cpd 88)

### Step 1: 4-(allyloxy)-3-bromobenzaldehyde

To a solution of 3-bromo-4-hydroxybenzaldehyde (10.0 g, 50 mmol) in DMF (50 mL) was added K₂CO₃ (7.6 g, 55 mmol) and allylbromide (4.5 mL, 52.5 mmol). The reaction mixture was stirred at room temperature for 16 hrs at which point TLC showed complete consumption of starting material. The reaction mixture was then poured into H₂O (100 mL) and extracted with Et₂O (2 X 100 mL). The combined organic extracts were washed with brine, dried over MgSO₄, then filtered, and concentrated to afford the product (11.2 g, 93%) as a clear oil.
¹H NMR (500 MHz, CHCl₃-*d*) δ ppm 4.62 (s, 2 H) 5.32 (dq, *J*=10.60, 1.62 Hz, 1 H) 5.52 (dq, *J*=17.26, 1.79 Hz, 1 H) 6.10 (ddt, *J*=17.25, 10.61, 4.83, 4.83 Hz, 1 H) 7.01 (d, *J*=8.51 Hz, 1 H) 7.81 (dd, *J*=8.43, 2.05 Hz, 1 H) 8.04 - 8.15 (m, 1 H) 9.87 (s, 1 H). LC-MS: 243.1 [M+H]⁺ 1.46 min.

### Step 2: ethyl 3-(4-(allyloxy)-3-bromophenyl)-2-azidoacrylate

To a solution of 4-(allyloxy)-3-bromobenzaldehyde(11.2 g, 46.5 mmol) and ethyl 2-azidoacetate (19.0 g, 140 mmol) in EtOH (100 mL), cooled to -10 °C, was added NaOEt (50 mL, 2.76 M) dropwise over 20 minutes. The reaction mixture was then warmed to 5 °C and stirred for 16 hrs at which point the reaction mixture was cooled to 0 °C and H₂O was added. The precipitate was then filtered and washed with H₂O to afford a product (10.1 g, 62%) as a beige powder.
¹H NMR (500 MHz, DMSO-*d*₆) δ ppm 1.29 - 1.35 (m, 3 H) 1.80 (s, 3 H) 4.31 (q, *J*=7.09 Hz, 2 H) 4.62 (s, 2 H) 5.32 (dq, *J*=10.60, 1.62 Hz, 1 H) 5.52 (dq, *J*=17.26, 1.79 Hz, 1 H) 6.10 (ddt, *J*=17.25, 10.61, 4.83, 4.83 Hz, 1 H) 7.15 (d, *J*=8.75 Hz, 1 H) 7.78 - 7.90 (m, 1 H) 8.15 - 8.22 (m, 1 H).

### Step 3: ethyl 6-(allyloxy)-5-bromo-1H-indole-2-carboxylate

A solution of ethyl 3-(4-(allyloxy)-3-bromophenyl)-2-azidoacrylate (6.5 g, 19 mmol) in xylenes (40 mL) was heated to 140 °C for 1 hr. The solution was then cooled to room temperature and concentrated. The crude residue was purified on silica gel (EtOAc/Hex 1:1) to afford the title compound (3.0 g, 47 %) as light yellow solid.
¹H NMR (500 MHz, DMSO-*d*₆) δ ppm 1.33 (t, *J*=7.09 Hz, 3 H) 4.33 (q, *J*=7.15 Hz, 2 H) 4.66 (dt, *J*=4.79, 1.59 Hz, 2 H) 5.32 (dq, *J*=10.60, 1.62 Hz, 1 H) 5.52 (dq, *J*=17.26, 1.79 Hz, 1 H) 6.10 (ddt, *J*=17.25, 10.61, 4.83, 4.83 Hz, 1 H) 7.01 (s, 1 H) 7.07 (d, *J*=1.02 Hz, 1 H) 7.91 (s, 1 H) 11.88 (s, 1 H). LC-MS: 326.1 [M+H]⁺, RT 0.90 min.

### Step 4: ethyl 6-(allyloxy)-5-bromo-1-methyl-1H-indole-2-carboxylate

To a solution of ethyl 6-(allyloxy)-5-bromo-1H-indole-2-carboxylate (5.5 g, 16.9 mmol) in DMF (17 mL), cooled to 0 °C, was added NaH (60%) (0.75 g, 18.6 mmol). Gas evolution was observed and the mixture was stirred for 30 min at which point MeI (1.2 mL, 18.6 mmol) was added and the solution was allowed to warm to room temperature. After stirring at room temperature for 1 hr, saturated NH₄Cl was added and the mixture was poured into H₂O and extracted with Et₂O. The combined organic extracts were washed with brine, dried over MgSO₄, then filtered, and concentrated to give the product (5.24 g, 92%) as a white solid.
¹H NMR (500 MHz, CHCl₃-*d*) δ ppm 1.31 (t, *J*=7.13 Hz, 3 H) 3.86 - 3.91 (m, 3 H) 4.26 (q, *J*=7.17 Hz, 2 H) 4.55 (dt, *J*=4.95, 1.59 Hz, 2 H) 5.25 (dq, *J*=10.63, 1.47 Hz, 1 H) 5.46 (dq, *J*=17.26, 1.66 Hz, 1 H) 5.97 - 6.08 (m, 1 H) 6.63 (s, 1 H) 7.05 (d, *J*=0.79 Hz, 1 H) 7.71 (s, 1 H). LC-MS: 340.1 [M+H]⁺, RT 1.00 min.

### Step 5: (6-(allyloxy)-5-bromo-1-methyl-1H-indol-2-yl)methanol

To a solution of ethyl 6-(allyloxy)-5-bromo-1-methyl-1H-indole-2-carboxylate (5.24 g, 15.4 mmol) in CH₂Cl₂ (40 mL), cooled to -78 °C, was added DIBAL-H (32.4 mL, 1M). After stirring at -78 °C for 30 min, TLC showed complete consumption of starting material. The reaction was then warmed to 0 °C and a saturated solution of Rochelle's Salts (30 mL) was added followed by CH₂Cl₂ (100 mL). The solution was then warmed to room temperature and stirred for 1 hr. The organic layer was then separated and washed with brine, dried over Na₂SO₄, then filtered, and concentrated to afford the product (4.0 g, 87%) as a white solid.
¹H NMR (500 MHz, CHCl₃-*d*) δ ppm 3.66 (s, 3 H) 4.60 (dt, *J*=5.04, 1.62 Hz, 2 H) 4.73 (s, 2 H) 5.27 (dd, *J*=10.56, 1.50 Hz, 1 H) 5.49 (dd, *J*=17.22, 1.62 Hz, 1 H) 6.04 - 6.14 (m, 1 H) 6.20 (d, *J*=0.63 Hz, 1 H) 6.74 (s, 1 H) 7.66 (s, 1 H). LC-MS: 298.0 [M+H]⁺, RT 0.94 min.

### Step 6: 6-(allyloxy)-5-bromo-2-((tert-butyldimethylsilyloxy)methyl)-1-methyl-1H-indole

To a solution of (6-(allyloxy)-5-bromo-1-methyl-1H-indol-2-yl)methanol (4.8 g, 16 mmol) was added imidazole (1.2 g, 17 mmol) and TBSCl (2.6 g, 17 mmol). After stirring for 3 hrs, the solution was washed with H₂O, brine, dried with Na₂SO₄, then filtered, and concentrated. The residue was purified on silica gel (4:1 Hexane/EA) to afford a product (5.58 g, 85%) as a white solid. LC-MS: 410.2 [M+H]⁺, RT 1.14 min.

### Step 7: 6-(allyloxy)-2-((tert-butyldimethylsilyloxy)methyl)-1-methyl-1H-indole-5-carbaldehyde

To a solution of 6-(allyloxy)-5-bromo-2-((tert-butyldimethylsilyloxy)methyl)-1-methyl-1H-indole (0.2 g, 0.5 mmol) in THF (5 mL), cooled to -78 °C) was added nBuLi (300 µL, 2.5M solution). After stirring at -78 °C for 30 min, DMF (100 µL) was added and the solution was warmed to room temperature. After stirring at room temperature for 30 min, the reaction was quenched with saturated NH₄Cl (2 mL) and poured into H₂O. The aqueous layer was extracted with Et₂O (2 x 20 mL) and the combined organics were washed with brine, dried with MgSO₄, then filtered and concentrated to afford the product (165 mg, 92%) as a white solid.
¹H NMR (500 MHz, CHCl₃-*d*) δ ppm -0.03 - 0.02 (m, 6 H) 0.80 - 0.85 (m, 9 H) 3.67 (s, 3 H) 4.63 (dt, *J*=5.10, 1.55 Hz, 2 H) 4.71 (d, *J*=0.32 Hz, 2 H) 5.28 (dd, *J*=10.56, 1.42 Hz, 1 H) 5.40 - 5.47 (m, 1 H) 6.00 - 6.15 (m, 1 H) 6.33 (d, *J*=0.55 Hz, 1 H) 6.64 (s, 1 H) 8.03 (s, 1 H) 10.45 (s, 1 H). LC-MS: 360.2 [M+H]⁺, RT 1.16 min.

### Step 8: 1-(6-(allyloxy)-2-((tert-butyldimethylsilyloxy)methyl)-1-methyl-1H-indol-5-yl)prop-2-en-1-ol

To a solution of 6-(allyloxy)-2-((tert-butyldimethylsilyloxy)methyl)-1-methyl-1H-indole-5-carbaldehyde (5.2 g, 14.6 mmol) in THF (60 mL) cooled to 0 °C was added vinylmagnesium bromide (16.0 mL, 1M). After stirring at 0 °C for 30 min, the solution was warmed to room temperature and stirred for an additional 30 min at which point saturated NH₄Cl (10 mL) was added. The crude reaction mixture was poured into H₂O and extracted with Et₂O (2 x 100 mL). The combined organics were washed with brine, dried with MgSO₄, then filtered, and concentrated to afford the product (5.5 g, 98%) as a yellow oil which was used immediately in the subsequent step without further purification.
¹H NMR (500 MHz, DMSO-*d*₆) δ ppm -0.02 - 0.03 (m, 6 H) 0.80 - 0.86 (m, 9 H) 3.63 - 3.68 (m, 3 H) 4.59 (dt, *J*=4.93, 1.60 Hz, 2 H) 4.76 (s, 2 H) 4.88 - 4.94 (m, 1 H) 5.12 (d, *J*=4.81 Hz, 1 H) 5.13 - 5.19 (m, 1 H) 5.22 - 5.27 (m, 1 H) 5.40 - 5.48 (m, 1 H) 5.91 - 6.01 (m, 1 H) 6.04 - 6.14 (m, 1 H) 6.26 (s, 1 H) 6.94 (s, 1 H) 7.43 (s, 1 H).

### Step 9: 8-((tert-butyldimethylsilyloxy)methyl)-9-methyl-5,9-dihydro-2H-oxepino[3,2-f]indol-5-ol

To a solution of 1-(6-(allyloxy)-2-((tert-butyldimethylsilyloxy)methyl)-1-methyl-1H-indol-5-yl)prop-2-en-1-ol (5.5 g, 14.3 mmol) in toluene (200 mL) was added Grubbs' catalyst second generation (350 mg) and the reaction mixture was heated at 60 °C for 3 hrs. The reaction mixture was then cooled to room temperature, concentrated, and the residue was purified on silica gel (4:1 Hexane/EA) to afford a product as a light green solid (2.7 g, 51 %).
¹H NMR (500 MHz, DMSO-*d*₆) δ ppm -0.03 - 0.03 (m, 6 H) 0.79 - 0.86 (m, 9 H) 3.64 (s, 3 H) 4.17 - 4.28 (m, 1 H) 4.65 - 4.74 (m, 1 H) 4.76 (s, 2 H) 5.23 - 5.34 (m, 1 H) 5.47 (d, *J*=5.60 Hz, 1 H) 5.69 - 5.80 (m, 2 H) 6.30 (d, *J*=0.55 Hz, 1 H) 7.08 (s, 1 H) 7.41 (d, *J*=0.63 Hz, 1 H). LC-MS: 342.3 [M-H₂O]⁺, RT 1.00 min.

### Step 10: 8-((tert-butyldimethylsilyloxy)methyl)-9-methyl-2H-oxepino[3,2-f]indol-5(9H)-one

To a solution of 8-((tert-butyldimethylsilyloxy)methyl)-9-methyl-5,9-dihydro-2H-oxepino[3,2-f]indol-5-ol (2.7 g, 7.5 mmol) in CH₂Cl₂ (40 mL) was added MnO₂ in three batches over 2 hrs (1.5 g, 1.0 g, and 1.0 g). The reaction mixture was then filtered through Celite and concentrated to afford a product as an orange solid (2.0 g, 75%).
¹H NMR (500 MHz, DMSO-*d*₆) δ ppm -0.02 - 0.02 (m, 6 H) 0.79 - 0.84 (m, 9 H) 3.66 (s, 3 H) 4.70 (dd, *J*=4.26, 1.66 Hz, 2 H) 4.76 (s, 2 H) 6.21 (d, *J*=11.82 Hz, 1 H) 6.45 (d, *J*=0.63 Hz, 1 H) 6.84 (d, *J*=11.82 Hz, 1 H) 7.11 (s, 1 H) 7.89 (s, 1 H). LC-MS: 358.8 [M+H]⁺, RT 1.02 min.

### Step 11: 8-((tert-butyldimethylsilyloxy)methyl)-9-methyl-3,4-dihydro-2H-oxepino[3,2-f]indol-5(9H)-one

To a solution of 8-((tert-butyldimethylsilyloxy)methyl)-9-methyl-2H-oxepino[3,2-f]indol-5(9H)-one (2.0 g, 5.6 mmol) in EtOH (30 mL) was added PtO₂ (20 mg). The flask was evacuated under vacuum and then back filled with H₂. After stirring at room temperature for 3 hrs, the reaction mixture was filtered through Celite and concentrated to afford the title compound (1.9 g, 94%) as a tan solid.
¹H NMR (500 MHz, DMSO-*d*₆) δ ppm 0.00 (s, 6 H) 0.81 (s, 9 H) 1.92 - 2.01 (m, 2 H) 2.65 - 2.72 (m, 2 H) 3.65 (s, 3 H) 4.11 (t, *J*=6.70 Hz, 2 H) 4.76 (s, 2 H) 6.43 (d, *J*=0.63 Hz, 1 H) 7.10 (s, 1 H) 7.81 (s, 1 H). LC-MS: 360.8 [M+H]⁺, RT 1.03 min.

### Step 12: N-(8-((tert-butyldimethylsilyloxy)methyl)-9-methyl-3,4-dihydro-2H-oxepino[3,2-f]indol-5(9H)-ylidene)-1-(2,4-dimethoxyphenyl)methanamine

To a solution of 8-((tert-butyldimethylsilyloxy)methyl)-9-methyl-3,4-dihydro-2H-oxepino[3,2-f]indol-5(9H)-one (2.7 g, 7.5 mmol), dimethoxylbenzylamine (1.25 mL, 8.3 mmol), and Et₃N (3.1 mL, 22.5 mmol) in CH₂Cl₂ (40 mL) was added a 1M solution of TiCl₄ (4.5 mL, 4.5 mmol) dropwise at 0 °C over 30 min. After completion of addition, the reaction mixture was stirred at room temperature for 16 hrs then the reaction was quenched with saturated NaHCO₃. The product was extracted with DCM and the combined organic phases were dried over Na₂SO₄, filtered, and concentrated to give a product that was used immediately without further purification. LC-MS: 509.3 [M+H]⁺, RT 1.27 min.

### Step 13: methyl 10-(((tert-butyldimethylsilyl)oxy)methyl)-1-(2,4-dimethoxybenzyl)-4-hydroxy-9-methyl-2-oxo-2,5,6,9-tetrahydro-1H-pyrido[2',3':4,5]oxepino[3,2-f]indole-3-carboxylate

A solution of *N*-(8-((tert-butyldimethylsilyloxy)methyl)-9-methyl-3,4-dihydro-2H-oxepino[3,2-f]indol-5(9H)-ylidene)-1-(2,4-dimethoxyphenyl)methanamine (3.81 g crude) and trimethyl methanetricarboxylate (2.9 g, 15.0 mmol) in Ph₂O (15 mL) was heated at 230 °C for 15 min. The reaction mixture was cooled to room temperature and purified on silica gel using EtOAc/Hex (0-60% gradient) to afford the product (2.0 g, 43%) as a yellow foam.
¹H NMR (500 MHz, DMSO-*d*₆) δ ppm 0.00 (d, *J*=0.95 Hz, 6 H) 0.77 - 0.83 (m, 9 H) 1.87 - 1.96 (m, 2 H) 2.88 - 2.96 (m, 1 H) 3.48 (s, 3 H) 3.64 (d, *J*=1.89 Hz, 6 H) 3.77 (s, 3 H) 4.21 (br. s., 2 H) 4.74 (s, 2 H) 4.98 - 5.07 (m, 1 H) 6.24 (s, 1 H) 6.37 (s, 2 H) 6.61 - 6.68 (m, 1 H) 7.21 (s, 1 H) 7.31 - 7.40 (m, 1 H) 13.28 - 13.36 (m, 1 H). LC-MS: 633.2 [M-H]⁻, RT 1.80 min.

### Step 14: methyl 1-(2,4-dimethoxybenzyl)-4-hydroxy-10-(hydroxymethyl)-9-methyl-2-oxo-2,5,6,9-tetrahydro-1H-pyrido[2',3':4,5]oxepino[3,2-f]indole-3-carboxylate

To a solution of methyl 10-(((tert-butyldimethylsilyl)oxy)methyl)-1-(2,4-dimethoxybenzyl)-4-hydroxy-9-methyl-2-oxo-2,5,6,9-tetrahydro-1H-pyrido[2',3':4,5]oxepino[3,2-f]indole-3-carboxylate (2.0 g, 3.15 mmol) in THF (40 mL) was added TBAF (6.6 mL, 1M solution in THF). After stirring at room temperature for 1.5 hrs, the reaction mixture was concentrated under reduced pressure and the crude residue was purified on silica gel (50-100% Hexane/EA) to afford the product (1.3 g, 80%) as a yellow solid.
¹H NMR (500 MHz, DMSO-*d*₆) δ ppm 1.96 - 2.07 (m, 1 H) 3.03 (dd, *J*=14.50, 3.86 Hz, 1 H) 3.60 (s, 3 H) 3.75 (d, *J*=1.89 Hz, 6 H) 3.87 (s, 3 H) 4.26 - 4.37 (m, 2 H) 4.63 (d, *J*=3.39 Hz, 2 H) 5.13 (br. s., 1 H) 5.27 (br. s., 1 H) 6.30 (s, 1 H) 6.41 - 6.52 (m, 2 H) 6.75 (d, *J*=8.35 Hz, 1 H) 7.31 (s, 1 H) 7.45 (br. s., 1 H). LC-MS: 521.5 [M+H]⁺, RT 0.75 min.

### Step 15: 1-(2,4-dimethoxybenzyl)-4-hydroxy-10-(hydroxymethyl)-9-methyl-2-oxo-2,5,6,9-tetrahydro-1H-pyrido[2',3':4,5]oxepino[3,2-f]indole-3-carboxylic acid

To a suspension of methyl 1-(2,4-dimethoxybenzyl)-4-hydroxy-10-(hydroxymethyl)-9-methyl-2-oxo-2,5,6,9-tetrahydro-1H-pyrido[2',3':4,5]oxepino[3,2-f]indole-3-carboxylate (1.3 g, 2.5 mmol) in EtOAc (25 mL) was added LiI (1.0 g, 7.5 mmol). The resulting solution was heated at 60 °C for 3 hrs and then cooled to room temperature, poured into 1M HCl, and extracted with CH₂Cl₂ (2 x 50 mL). The combined organic extracts were dried over Na₂SO₄, filtered, and concentrated to afford a product (1.25 g, 98%) as an off-white solid.
¹H NMR (500 MHz, DMSO-*d*₆) δ ppm 2.05 - 2.15 (m, 1 H) 3.06 (dd, *J*=14.50, 3.86 Hz, 1 H) 3.05 - 3.05 (m, 1 H) 3.56 - 3.62 (m, 3 H) 3.75 (s, 3 H) 3.77 (s, 3 H) 4.31 - 4.42 (m, 2 H) 4.65 (s, 2 H) 5.22 - 5.37 (m, 2 H) 6.36 (s, 1 H) 6.44 (dd, *J*=8.43, 2.29 Hz, 1 H) 6.49 (d, *J*=2.21 Hz, 1 H) 6.79 (d, *J*=8.43 Hz, 1 H) 7.36 (s, 1 H) 7.58 (br. s., 1 H) 13.85 (s, 1 H) 16.00 - 16.08 (m, 1 H). LC-MS: 505.1 [M-H]⁻, RT 1.27 min.

### Step 16: 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid

To a solution of 1-(2,4-dimethoxybenzyl)-4-hydroxy-10-(hydroxymethyl)-9-methyl-2-oxo-2,5,6,9-tetrahydro-1H-pyrido[2',3':4,5]oxepino[3,2-f]indole-3-carboxylic acid (1.25 g, 2.5 mmol) in CH₂Cl₂ (25 mL) was added MnO₂ in three batches over 2 hrs (1.5 g, 1.0 g, and 1.0 g). The reaction mixture was then filtered through Celite and concentrated to afford the product as a brown foam (0.81 g, 64%) which was used in the subsequent reactions without further purification.

### Steps 17-18: 4-hydroxy-9-methyl-10-((methylamino)methyl)-2-oxo-2,5,6,9-tetrahydro-1H-pyrido[2',3':4,5]oxepino[3,2-f]indole-3-carboxylic acid hydrochloride

To a solution of the crude aldehyde (0.2 g, 0.4 mmol) in DCE (4 mL) was added MeNH₂ (500 µl, 2M/THF) followed by AcOH (50 µl). The reaction mixture was stirred for 1 hr at room temperature, then NaBH(OAc)₃ (0.18 g, 0.83 mmol) was added. After competition, the reaction mixture was concentrated under reduced pressure and H₂O was added. The resulting precipitate was collected by filtration and purified by preparative HPLC (40-90% MeCN in H₂O).

To the product obtained above was added TIPS-H (1 mL) and TFA (1 mL) and the resulting biphasic solution was heated to 60 °C for 1 hr. The reaction mixture was then cooled to room temperature and concentrated under reduced pressure. To the crude residue was added HCl (2.0 mL, 2M/Et₂O) and the resulting white precipitate was filtered, then washed with Et₂O and dried under an N₂ stream to afford the final product (75 mg, 50% over 3 steps) as the HCl salt.
¹H NMR (500 MHz, DMSO-*d*₆) δ ppm 2.63 (t, *J*=5.20 Hz, 5 H) 3.83 (s, 3 H) 4.35 - 4.51 (m, 4 H) 6.83 (s, 1 H) 7.44 (s, 1 H) 7.86 (s, 1 H) 9.33 (d, *J*=4.10 Hz, 2 H) 13.04 (br. s., 1 H) 13.87 (s, 1 H). LC-MS: 370.1 [M+H]⁺, RT 0.48 min.

### Example 89

### 10-((ethylamino)methyl)-4-hydroxy-9-methyl-2-oxo-2,5,6,9-tetrahydro-1H-pyrido[2',3':4,5]oxepino[3,2-f]indole-3-carboxylic acid hydrochloride (Cpd 89)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 1.29 (t, *J*=7.17 Hz, 3 H) 2.63 (t, *J*=6.03 Hz, 2 H) 3.06 (d, *J*=5.44 Hz, 2 H) 3.84 (s, 3 H) 4.35 - 4.50 (m, 4 H) 6.84 (s, 1 H) 7.44 (s, 1 H) 7.85 (s, 1 H) 9.33 (br. s., 2 H) 13.05 (br. s., 1 H) 13.87 (s, 1 H). LC-MS: 382.2 [M-H]⁻, RT 0.50 min.

### Example 90

### 4-hydroxy-10-((isopropylamino)methyl)-9-methyl-2-oxo-2,5,6,9-tetrahydro-1H-pyrido[2',3':4,5]oxepino[3,2-f]indole-3-carboxylic acid hydrochloride (Cpd 90)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 1.36 (d, *J*=6.54 Hz, 6 H) 2.63 (s, 2 H) 3.42 - 3.51 (m, 1 H) 3.83 (s, 3 H) 4.38 - 4.49 (m, 4 H) 6.83 (s, 1 H) 7.44 (s, 1 H) 7.86 (s, 1 H) 9.12 - 9.23 (m, 2 H) 13.01 - 13.08 (s, 1 H) 13.83 - 13.90 (s, 1 H). LC-MS: 396.5 [M-H]⁻, RT 0.49 min.

### Example 91

### 10-(azetidin-1-ylmethyl)-4-hydroxy-9-methyl-2-oxo-2,5,6,9-tetrahydro-1H-pyrido[2',3':4,5]oxepino[3,2-f]indole-3-carboxylic acid hydrochloride (Cpd 91)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 2.28 - 2.47 (m, 2 H) 2.63 (t, *J*=6.27 Hz, 2 H) 3.84 (s, 3 H) 4.05 (d, *J*=3.86 Hz, 2 H) 4.09 - 4.19 (m, 2 H) 4.45 (t, *J*=6.27 Hz, 2 H) 4.65 (d, *J*=5.36 Hz, 2 H) 6.87 (s, 1 H) 7.43 (s, 1 H) 7.85 (s, 1 H) 11.13 - 11.24 (s, 1 H) 13.02 (br. s., 1 H) 13.87 (br. s., 1 H). LC-MS: 394.2 [M-H]⁻, RT 0.50 min.

### Example 92

### 10-((ethylamino)methyl)-4-hydroxy-5,9-dimethyl-2-oxo-2,5,6,9-tetrahydro-1H-pyrido[2',3':4,5]oxepino[3,2-f]indole-3-carboxylic acid hydrochloride (Cpd 92)

### Step 1: 3-bromo-4-(2-methylallyloxy)benzaldehyde

To a solution of 3-bromo-4-hydroxybenzaldehyde (25.0 g, 124.4 mmol) in DMF (100 mL) was added K₂CO₃ (19.0 g, 137 mmol) and 3-bromo-2-methylprop-1-ene (12.6 mL, 126 mmol). The reaction mixture was stirred at room temperature for 16 hrs at which point TLC showed complete consumption of starting material. The reaction mixture was then poured into H₂O (300 mL) and extracted with Et₂O (3 X 300 mL). The combined organic extracts were washed with brine, dried over MgSO₄, then filtered, and concentrated to afford the title compound (32.8 g, 99%) as a clear oil
¹H NMR (500 MHz, CHCl₃-*d*) δ ppm 1.88 - 1.93 (m, 3 H) 4.62 (s, 2 H) 5.09 (dt, *J*=2.70, 1.21 Hz, 1 H) 5.20 (dd, *J*=1.42, 0.87 Hz, 1 H) 7.01 (d, *J*=8.51 Hz, 1 H) 7.81 (dd, *J*=8.43, 2.05 Hz, 1 H) 8.12 (d, *J*=2.05 Hz, 1 H) 9.87 (s, 1 H)

### Step 2: ethyl 2-azido-3-(3-bromo-4-(2-methylallyloxy)phenyl)acrylate

To a solution of 3-bromo-4-(2-methylallyloxy)benzaldehyde (32.0 g, 124.4 mmol) and ethyl 2-azidoacetate (38.0 g, 295 mmol) in EtOH (200 mL), cooled to -10 °C, was added NaOEt (100 mL, 2.76 M) dropwise over 20 minutes. The reaction mixture was then warmed to 5 °C and stirred for 16 hrs at which point the reaction mixture was cooled to 0 °C and H₂O was added. The precipitate was then filtered and washed with H₂O to afford the title compound (38.0 g, 86%) as a beige powder.
¹H NMR (500 MHz, CHCl₃-*d*) δ ppm 1.42 (t, *J*=7.13 Hz, 3 H) 1.88 (d, *J*=0.55 Hz, 3 H) 4.38 (q, *J*=7.12 Hz, 2 H) 4.56 (s, 2 H) 5.06 (dt, *J*=2.74, 1.19 Hz, 1 H) 5.19 (dd, *J*=1.50, 0.87 Hz, 1 H) 6.81 (s, 1 H) 6.89 (d, *J*=8.67 Hz, 1 H) 7.70 - 7.76 (m, 1 H) 8.12 (dd, *J*=2.17, 0.35 Hz, 1 H).

### Step 3: ethyl 5-bromo-6-(2-methylallyloxy)-1H-indole-2-carboxylate

A solution of ethyl 2-azido-3-(3-bromo-4-(2-methylallyloxy)phenyl)acrylate (10.5 g, 30 mmol) in toluene (30 mL) was heated to 100 °C for 1 hr. The solution was then cooled to room temperature and concentrated. The crude residue was purified on silica gel (EtOAc/Hex 1:1) to afford the title compound (4.8 g, 47 %) as light yellow solid.
¹H NMR (500 MHz, CHCl₃-*d*) δ ppm 1.44 (t, *J*=7.13 Hz, 3 H) 1.90 (s, 3 H) 4.43 (q, *J*=7.12 Hz, 2 H) 4.53 (s, 2 H) 5.06 (s, 1 H) 5.23 (s, 1 H) 6.89 (s, 1 H) 7.12 (d, *J*=1.34 Hz, 1 H) 7.87 (s, 1 H) 9.13 (br. s., 1 H). LC-MS: 338.5 [M-H]⁻, RT 0.97 min.

### Step 4: ethyl 5-bromo-1-methyl-6-(2-methylallyloxy)-1H-indole-2-carboxylate

To a solution of ethyl 5-bromo-6-(2-methylallyloxy)-1H-indole-2-carboxylate (6.6 g, 19.5 mmol) in DMF (20 mL), cooled to 0 °C, was added NaH (60%) (0.9 g, 21.5 mmol). Gas evolution was observed and the mixture was stirred for 30 min at which point MeI (1.4 mL, 21.5 mmol) was added and the solution was allowed to warm to room temperature. After stirring at room temperature for 1 hr, saturated NH₄Cl was added and the mixture was poured into H₂O and extracted with Et₂O. The combined organic extracts were washed with brine, dried over MgSO₄, then filtered, and concentrated to give the title compound (6.2 g, 90%) as a white solid.
¹H NMR (500 MHz, CHCl₃-*d*) δ ppm 1.33 (t, *J*=7.13 Hz, 3 H) 1.83 (d, *J*=0.47 Hz, 3 H) 3.92 - 3.96 (m, 3 H) 4.28 (q, *J*=7.09 Hz, 2 H) 4.49 (s, 2 H) 4.96 - 5.02 (m, 1 H) 5.17 (dd, *J*=1.42, 0.87 Hz, 1 H) 6.69 (s, 1 H) 7.10 (d, *J*=0.71 Hz, 1 H) 7.75 (s, 1 H).

### Step 5: (5-bromo-1-methyl-6-(2-methylallyloxy)-1H-indol-2-yl)methanol

To a solution of ethyl 5-bromo-1-methyl-6-(2-methylallyloxy)-1H-indole-2-carboxylate (6.2 g, 17.5 mmol) in CH₂Cl₂ (60 mL), cooled to -78 °C, was added DIBAL-H (38.5 mL, 1M). After stirring at -78 °C for 30 min, TLC showed complete consumption of starting material. The reaction was then warmed to 0 °C and a saturated solution of Rochelle's Salts (30 mL) was added followed by CH₂Cl₂ (100 mL). The solution was then warmed to room temperature and stirred for 1 hr. The organic layer was then separated and washed with brine, dried over Na₂SO₄, then filtered, and concentrated to afford the title compound (5.5 g, 98%) as a light brown solid.
¹H NMR (500 MHz, CHCl₃-*d*) δ ppm 1.82 (br. s., 3 H) 3.65 (br. s, 3 H) 4.45 (br. s., 2 H) 4.66 (br. s., 2 H) 4.96 (br. s., 1 H) 5.16 (br. s., 1 H) 6.23 (br. s., 1 H) 6.67 (br. s., 1 H) 7.64 (br. s., 1 H). LC-MS: 311.9 [M+H]⁺, RT 0.82 min.

### Step 6: 5-bromo-2-((tert-butyldimethylsilyloxy)methyl)-1-methyl-6-(2-methylallyloxy)-1H-indole

To a solution of (5-bromo-1-methyl-6-(2-methylallyloxy)-1H-indol-2-yl)methanol (5.5 g, 17.7 mmol) was added imidazole (1.3 g, 19.5 mmol) and TBSCl (2.9 g, 19.5 mmol). After stirring for 3 hrs, the solution was washed with H₂O, brine, dried with Na₂SO₄, then filtered, and concentrated. The crude residue was purified on silica gel (4:1 Hexane/EA) to afford the title compound (6.8 g, 90%) as a white solid.
¹H NMR (500 MHz, CHCl₃-*d*) δ ppm 0.00 (s, 6 H) 0.85 (s, 9 H) 1.86 (d, *J*=0.55 Hz, 3 H) 3.66 (s, 3 H) 4.49 (s, 2 H) 4.73 (s, 2 H) 4.99 (d, *J*=1.58 Hz, 1 H) 5.19 (dd, *J*=1.58, 0.87 Hz, 1 H) 6.19 (d, *J*=0.55 Hz, 1 H) 6.73 (s, 1 H) 7.66 (s, 1 H). LC-MS: 426.1 [M+H]⁺, RT 1.88 min.

### Step 7: 2-((tert-butyldimethylsilyloxy)methyl)-1-methyl-6-(2-methylallyloxy)-1H-indole-5-carbaldehyde

To a solution of 5-bromo-2-((tert-butyldimethylsilyloxy)methyl)-1-methyl-6-(2-methylallyloxy)-1H-indole (6.8 g, 16 mmol) in THF (60 mL), cooled to -78 °C was added nBuLi (7.1 mL, 2.5M solution). After stirring at -78 °C for 30 min, DMF (2.5 mL) was added and the solution was warmed to room temperature. After stirring at room temperature for 30 min, the reaction was quenched with saturated NH₄Cl (10 mL) and poured into H₂O (100 mL). The aqueous layer was extracted with Et₂O (2 x 100 mL) and the combined organics were washed with brine, dried with MgSO₄, then filtered and concentrated to afford the title compound (5.6 g, 93%) as a light yellow solid.
¹H NMR (500 MHz, DMSO-*d*₆) δ ppm 0.00 (s, 6 H) 0.81 (s, 9 H) 1.79 (d, *J*=0.39 Hz, 3 H) 3.67 (s, 3 H) 4.60 (s, 2 H) 4.75 (s, 2 H) 4.96 (d, *J*=0.39 Hz, 1 H) 5.13 (dd, *J*=1.93, 0.91 Hz, 1 H) 6.42 (d, *J*=0.47 Hz, 1 H) 7.09 (s, 1 H) 7.86 (s, 1 H) 10.33 (s, 1 H). LC-MS: 374.2 [M+H]⁺, RT 1.76 min.

### Step 8: 1-(2-((tert-butyldimethylsilyloxy)methyl)-1-methyl-6-(2-methylallyloxy)-1H-indol-5-yl)prop-2-en-1-ol

To a solution of 2-((tert-butyldimethylsilyloxy)methyl)-1-methyl-6-(2-methylallyloxy)-1H-indole-5-carbaldehyde (5.6 g, 15.0 mmol) in THF (50 mL) cooled to 0 °C was added vinylmagnesium bromide (16.5 mL, 1M). After stirring at 0 °C for 30 min, the solution was warmed to room temperature and stirred for an additional 30 min at which point saturated NH₄Cl (10 mL) was added. The crude reaction mixture was poured into H₂O and extracted with Et₂O (2 x 100 mL). The combined organics were washed with brine, dried with MgSO₄, then filtered, and concentrated to afford the product (5.47 g, 91 %) as a yellow oil which was used immediately in the subsequent step without further purification.
¹H NMR (500 MHz, DMSO-*d*₆) δ ppm -0.04 - 0.02 (m, 6 H) 0.80 - 0.86 (m, 9 H) 1.80 (s, 3 H) 3.65 (s, 3 H) 4.48 (s, 2 H) 4.75 (s, 2 H) 4.87 - 4.98 (m, 2 H) 5.09 - 5.19 (m, 2 H) 5.41 - 5.48 (m, 1 H) 5.90 - 6.02 (m, 1 H) 6.25 (s, 1 H) 6.93 (s, 1 H) 7.42 (s, 1 H). LC-MS: 384.2 [M-H₂O]⁺, RT 1.74 min.

### Step 9: 1-(2-((tert-butyldimethylsilyloxy)methyl)-1-methyl-6-(2-methylallyloxy)-1H-indol-5-yl)prop-2-en-1-one

To a solution of 1-(2-((tert-butyldimethylsilyloxy)methyl)-1-methyl-6-(2-methylallyloxy)-1H-indol-5-yl)prop-2-en-1-ol (5.47 g, 13.7 mmol) in CH₂Cl₂ (40 mL) was added MnO₂ in three batches over 2 hrs (1.5 g, 1.0 g, and 1.0 g). The reaction mixture was then filtered through Celite and concentrated to afford the title compound as a light brown oil (5.1 g, 92%) which was used immediately in the subsequent step.
¹H NMR (500 MHz, DMSO-*d*₆) δ ppm -0.01 - 0.00 (m, 6 H) 0.81 - 0.82 (m, 9 H) 1.76 (s, 3 H) 3.68 (s, 3 H) 4.55 (s, 2 H) 4.76 (s, 2 H) 4.93 (s, 1 H) 5.07 - 5.10 (m, 1 H) 6.09 (dd, *J*=17.22, 1.93 Hz, 1 H) 6.38 (s, 1 H) 7.02 - 7.12 (m, 3 H) 7.87 (s, 1 H).

### Step 10: 8-((tert-butyldimethylsilyloxy)methyl)-3,9-dimethyl-2H-oxepino[3,2-f]indol-5(9H)-one

To a solution of 1-(2-((tert-butyldimethylsilyloxy)methyl)-1-methyl-6-(2-methylallyloxy)-1H-indol-5-yl)prop-2-en-1-one 3.0 g, 7.5 mmol) in toluene (40 mL) was added Grubbs' catalyst second generation (120 mg) and the reaction mixture was heated at 60 °C for 16 hrs. The reaction mixture was then cooled to room temperature, concentrated, and the crude residue was purified on silica gel (4:1 Hexane/EA) to afford the title compound as a white solid (2.0 g, 72%).
¹H NMR (500 MHz, CHCl₃-*d*) δ ppm -0.01 - 0.02 (m, 6 H) 0.80 - 0.84 (m, 9 H) 1.84 (d, *J*=0.47 Hz, 3 H) 3.68 (s, 3 H) 4.53 (s, 2 H) 4.99 (dd, *J*=1.69, 0.75 Hz, 1 H) 5.13 (dd, *J*=1.50, 0.95 Hz, 1 H) 6.33 (d, *J*=0.55 Hz, 1 H) 6.65 (s, 1 H) 8.04 (s, 1 H) 10.44 - 10.51 (m, 1 H)

### Step 11: 8-((tert-butyldimethylsilyloxy)methyl)-3,9-dimethyl-3,4-dihydro-2H-oxepino[3,2-f]indol-5(9H)-one

To a solution of 8-((tert-butyldimethylsilyloxy)methyl)-3,9-dimethyl-2H-oxepino[3,2-f]indol-5(9H)-one (2.0 g, 5.4 mmol) in EtOH (20 mL) was added PtO₂ (50 mg). The flask was evacuated under vacuum and then back filled with H₂. After stirring at room temperature for 3 hrs, the reaction mixture was filtered through Celite and concentrated to afford the titled compound (1.95 g, 96%) as a brown oil.
¹H NMR (500 MHz, CHCl₃-*d*) δ ppm -0.02 - 0.01 (m, 6 H) 0.82 - 0.84 (m, 9 H) 1.06 (d, J=7.70 Hz, 3 H) 2.41 - 2.52 (m, 1 H) 2.69 (s, 1 H) 2.88 - 2.96 (m, 1 H) 3.67 (s, 3 H) 3.81 (d, *J*=7.49 Hz, 1 H) 4.23 (dd, *J*=11.90, 6.23 Hz, 1 H) 4.73 (s, 2 H) 6.34 (s, 1 H) 6.87 (s, 1 H) 8.00 (s, 1 H).

### Steps 12-18 10-((ethylamino)methyl)-4-hydroxy-5,9-dimethyl-2-oxo-2,5,6,9-tetrahydro-1H-pyrido[2',3':4,5]oxepino[3,2-f]indole-3-carboxylic acid

The title compound was prepared from the 8-((tert-butyldimethylsilyloxy)methyl)-3,9-dimethyl-3,4-dihydro-2H-oxepino[3,2-f]indol-5(9H)-one following 6 steps protocol described for Example 88 (steps 12-18).
¹H NMR (500 MHz, DMSO-*d*₆) δ ppm 0.82 - 0.90 (m, 3 H) 1.23 - 1.29 (m, 3 H) 3.51 - 3.58 (m, 2 H) 3.78 - 3.84 (m, 3 H) 4.11 - 4.16 (m, 2 H) 4.19 - 4.26 (m, 1 H) 4.39 - 4.47 (m, 2 H) 6.77 - 6.81 (s, 1 H) 7.40 - 7.46 (s, 1 H) 7.66 - 7.76 (s, 1 H). LC-MS: 396.1 [M-H]⁻, RT 0.51 min.

### Example 93

### 4-hydroxy-7-methyl-2-oxo-9-(pyrrolidin-1-yl)-2,5,6,7-tetrahydro-1H-benzo[b]pyrido[2,3-d]azepine-3-carboxylic acid (Cpd 93)

### Step 1: 8-chloro-1-methyl-3,4-dihydro-1H-benzo[b]azepin-5(2H)-one

To a solution of 8-chloro-3,4-dihydro-1H-benzo[b]azepin-5(2H)-one (2.62 g, 13.4 mmol) in DMF (30 mL), under Argon, was added NaH (776 mg, 40.3 mmol) at room temperature. The suspension was stirred for 10 minutes, then MeI (1.7 mL, 26.8 mmol) was added. After 30 minutes, the reaction was completed, as shown by LCMS, and the reaction mixture was poured into H₂O (300 mL) and extracted with EtOAc (300 mL). The organic layer was dried over Na₂SO₄, then filtered and concentrated. The product was purified by column chromatography (0 to 35% gradient of EtOAc in hexanes to provide the desired product (1.17 g, 42%) as a light yellow solid.
¹H NMR (500 MHz, CHCl₃-*d*) δ ppm 2.24 (quin, *J*=6.9 Hz, 2 H) 2.76 (t, *J*=7.1 Hz, 2 H) 3.11 (s, 3 H) 3.21 - 3.28 (m, 2 H) 6.76 - 6.80 (m, 1 H) 6.85 (d, *J*=1.6 Hz, 1 H) 7.68 (d, *J*=8.5 Hz, 1 H). LC-MS 208.2 [M-H]⁻, 210.2 [M+H]⁺, RT 1.25 min.

### Step 2: N-(8-chloro-1-methyl-3,4-dihydro-1H-benzo[b]azepin-5(2H)-ylidene)-1-(2,4-dimethoxyphenyl)methanamine

To a solution of 8-chloro-1-methyl-3,4-dihydro-1H-benzo[b]azepin-5(2H)-one (0.50 g, 2.40 mmol) in CH₂Cl₂ (8 mL) was added 2,4-dimethoxybenzylamine (0.44 g, 2.63 mmol) and NEt₃ (1.0 mL, 7.20 mmol). The mixture was cooled to 0 °C, then a solution of TiCl₄ (1.0M CH₂Cl₂, 1.56 mL, 1.56 mmol) was added dropwise via syringe pump over 30 minutes. The reaction mixture was allowed to warm to room temperature and stirred overnight. The mixture was diluted with CH₂Cl₂ (20 mL) and then the reaction was quenched with NaHCO₃ (sat. aq., 10 mL). Following vigorous shaking, the organic phase was separated using a PTFE phase separator, then dried over Na₂SO₄ and filtered. The solvent was removed to afford the desired product (∼0.7 g, ∼90 %) as a yellow solid, which was used directly in the next step without further purification. LC-MS 359.2 [M+H]⁺, RT 0.97 min.

### Step 3: methyl 9-chloro-1-(2,4-dimethoxybenzyl)-4-hydroxy-7-methyl-2-oxo-2,5,6,7-tetrahydro-1H-benzo[b]pyrido[2,3-d]azepine-3-carboxylate

Crude *N*-(8-chloro-1-methyl-3,4-dihydro-1H-benzo[b]azepin-5(2H)-ylidene)-1-(2,4-dimethoxyphenyl)methanamine (690 mg, 1.95 mmol) and trimethyl methanetricarboxylate (631 mg, 3.32 mmol) were mixed together in Ph₂O (4.0 mL). With stirring, the mixture was placed into a pre-heated heat block at 210 °C and heated for 10 minutes under a blanket of Argon. The reaction mixture was cooled to room temperature and was filtered directly on a silica cartridge. The product was purified by column chromatography (100% hexanes followed by EtOAc in hexanes 0 to 85% gradient) to provide the desired product (289 mg, 31 %, two steps) as an off-white foam which was used directly in the next step. LC-MS 483.9 [M-H]⁻, 485.8 [M+H]⁺, RT 1.53 min.

### Step 4: 1-(2,4-dimethoxybenzyl)-4-hydroxy-7-methyl-2-oxo-9-(pyrrolidin-1-yl)-2,5,6,7-tetrahydro-1H-benzo[b]pyrido[2,3-d]azepine-3-carboxylic acid

To a vial was added methyl 9-chloro-1-(2,4-dimethoxybenzyl)-4-hydroxy-7-methyl-2-oxo-2,5,6,7-tetrahydro-1H-benzo[b]pyrido[2,3-d]azepine-3-carboxylate (140 mg, 0.29 mmol), NaOt-Bu (110 mg, 1.15 mmol), and 2-(2'-di-tert-butylphosphine)biphenyl palladium(II) acetate (4 mg, 8.7 µmol). The vial was purged with Argon for 5 minutes, then toluene (3.0 mL) and pyrrolidine (50 µL, 0.57 mmol) were each added via syringe. The resultant solution was stirred at 90 °C for 40 minutes. The reaction mixture was then cooled, diluted with CH₂Cl₂ (10 mL) and washed with HCl (15.0 mL, 1.0 M aqueous solution). The organic layer was then concentrated onto silica gel and purified by column chromatography (0 to 40% gradient of EtOAc in hexanes) to give 34 mg (23%) of the desired product which was used directly in the next step. LC-MS 506.9 [M+H]⁺, RT 1.67 min.

### Step 5: 4-hydroxy-7-methyl-2-oxo-9-(pyrrolidin-1-yl)-2,5,6,7-tetrahydro-1H-benzo[b]pyrido[2,3-d]azepine-3-carboxylic acid

To a vial was added 1-(2,4-dimethoxybenzyl)-4-hydroxy-7-methyl-2-oxo-9-(pyrrolidin-1-yl)-2,5,6,7-tetrahydro-1H-benzo[b]pyrido[2,3-d]azepine-3-carboxylic acid (34 mg, 6.7 µmol), trifluroacetic acid (0.5 mL) and CH₂Cl₂ (0.5 mL). The resultant solution was stirred at room temperature for 90 minutes. The solution was concentrated under reduced pressure, and 23 mg (95%) of product was collected via filtration after trituration from Et₂O (10 mL). ¹H NMR (500 MHz, CHCl₃-*d*) δ ppm 1.96 - 2.14 (m, 4 H) 2.66 - 2.79 (m, 2 H) 3.30 - 3.43 (m, 4 H) 3.44 - 3.55 (m, 2 H) 3.79 (br. s., 3 H) 6.12 (br. s., 1 H) 6.17 - 6.33 (m, 1 H) 6.33 - 6.46 (m, 1 H) 10.12 (br. s., 1 H) 13.66 (br. s., 1 H) 14.90 (br. s., 1 H) LC-MS 354.1 [M-H]⁻, 356.2 [M+H]⁺, RT 1.35 min.

### Example 94

### 4-hydroxy-2-oxo-9-(pyrrolidin-1-yl)-2,5,6,7-tetrahydro-1H-benzo[b]pyrido[2,3-d]azepine-3-carboxylic acid (Cpd 94)

### Step 1: benzyl 8-chloro-5-oxo-2,3,4,5-tetrahydro-1H-benzo[b]azepine-1-carboxylate

To a solution of 8-chloro-3,4-dihydro-1H-benzo[b]azepin-5(2H)-one (1.46 g, 7.48 mmol) in CH₂Cl₂ (25 mL) and pyridine (1.2 mL, 14.96 mmol), under Argon, was added benzyl chloroformate (1.8 mL, 12.72 mmol) dropwise at room temperature. The solution was stirred overnight. After the starting material was consumed, the reaction mixture was poured into H₂O (100 mL) and extracted with EtOAc (300 mL). The organic layer was dried over Na₂SO₄, then filtered and concentrated. The product was purified by column chromatography (0 to 40% gradient of EtOAc in hexanes) to provide the desired product (1.95 g, 80%) as a white solid. LC-MS 330.1 [M+H]⁺, RT 1.48 min.

### Step 2: benzyl 8-chloro-5-((2,4-dimethoxybenzyl)imino)-2,3,4,5-tetrahydro-1H-benzo[b]azepine-1-carboxylate

To a solution of benzyl 8-chloro-5-oxo-2,3,4,5-tetrahydro-1H-benzo[b]azepine-1-carboxylate (1.95 g, 5.91 mmol) in CH₂Cl₂ (20 mL) was added 2,4-dimethoxybenzylamine (980 µL, 6.50 mmol) and NEt₃ (2.46 mL, 17.71 mmol). The mixture was cooled to 0 °C, then a solution of TiCl₄ (1.0M CH₂Cl₂, 3.84 mL, 3.84 mmol) was added dropwise via syringe pump over 30 minutes. The reaction mixture was allowed to warm to room temperature and stirred overnight. The mixture was diluted with CH₂Cl₂ (50 mL) and then the reaction was quenched with NaHCO₃ (sat. aq., 30 mL). Following vigorous shaking, the organic phase was separated using a PTFE phase separator, then dried over Na₂SO₄ and filtered. The solvent was removed and the resulting crude product was obtained (2.75 g, -95 %) as a yellow solid, which was used directly in the next step without further purification. LC-MS 479.2 [M+H]⁺, RT 1.27 min.

### Step 3: 7-benzyl 3-methyl 9-chloro-1-(2,4-dimethoxybenzyl)-4-hydroxy-2-oxo-5,6-dihydro-1H-benzo[b]pyrido[2,3-d]azepine-3,7(2H)-dicarboxylate

The crude benzyl 8-chloro-5-((2,4-dimethoxybenzyl)imino)-2,3,4,5-tetrahydro-1H-benzo[b]azepine-1-carboxylate (2.75 g, 5.75 mmol) and trimethyl methanetricarboxylate (1.85 g, 9.78 mmol) were mixed together in Ph₂O (12.0 mL). With stirring, the mixture was placed into a pre-heated heat block at 210 °C and heated for 10 minutes under a blanket of Argon. The reaction mixture was cooled to room temperature and filtered directly on a silica cartridge. The product was purified by column chromatography (100% hexanes followed by EtOAc in hexanes 0 to 85% gradient) to give the desired product (1.13 g, 32%, two steps) as an off-white foam which was used directly in the next step.. LC-MS 603.4 [M-H]⁻, 605.4 [M+H]⁺, RT 0.94 min.

### Steps 4-6: 4-hydroxy-2-oxo-9-(pyrrolidin-1-yl)-2,5,6,7-tetrahydro-1H-benzo[b]pyrido[2,3-d]azepine-3-carboxylic acid

To a vial was added 7-benzyl 3-methyl 9-chloro-1-(2,4-dimethoxybenzyl)-4-hydroxy-2-oxo-5,6-dihydro-1H-benzo[b]pyrido[2,3-d]azepine-3,7(2H)-dicarboxylate (189 mg, 0.31 mmol), NaOt-Bu (120 mg, 1.24 mmol), and 2-(2'-Di-tert-butylphosphine)biphenylpalladium(II) acetate (4 mg, 8.7 µmol). The vial was purged with Argon for 5 minutes, then toluene (3.0 mL) and pyrrolidine (52 µL, 0.62 mmol) were each added via syringe. The resultant solution was stirred at 90 °C for 60 minutes. The reaction was then cooled, diluted with CH₂Cl₂ (10 mL) and washed with HCl (15.0 mL, 1.0 M aqueous solution). The organic layer was concentrated to yield a crude mixture (∼1:1) of desired product:deprotected product which was used directly in the next step. LC-MS (desired product) 624.5 [M-H]⁻, 626.4 [M+H]⁺, RT 1.66 min. LC-MS (deprotected product) 490.2 [M-H]⁻, 492.3 [M+H]⁺, RT 1.57 min.

The crude mixture from above (∼0.3 mmol) was suspended in MeOH (3.0 mL) and THF (2.0 mL). To the solution was added Pd(OH)₂ (100 mg) and the resultant suspension was placed under H₂ (1 atm). After 1 hour of vigorous stirring, the starting material had been consumed and the reaction was diluted with EtOAc and filtered through a pad of Celite. The solution was concentrated and purified directly by preparative HPLC. The product was obtained as a TFA salt (32 mg, 21% from aryl chloride) before deprotection. LC-MS (deprotected product) 490.2 [M-H]⁻, 492.3 [M+H]⁺, RT 1.57 min.

To a vial was added 1-(2,4-dimethoxybenzyl)-4-hydroxy-2-oxo-9-(pyrrolidin-1-yl)-2,5,6,7-tetrahydro-1H-benzo[b]pyrido[2,3-d]azepine-3-carboxylic acid (32 mg, 6.45 µmol), trifluroacetic acid (1.0 mL) and CH₂Cl₂ (1.0 mL). The resultant solution was stirred at room temperature for 2 hours. The solution was concentrated under reduced pressure, and the product (17 mg, 77%) was collected via filtration after trituration from Et₂O (10 mL).
¹H NMR (500 MHz, DMSO-*d*₆) δ ppm 1.87 - 2.00 (m, 4 H) 2.64 - 2.73 (m, 2 H) 3.25 (t, *J*=6.3 Hz, 4 H) 3.44 - 3.53 (m, 2 H) 5.92 (s, 1 H) 6.07 - 6.15 (m, 1 H) 7.15 - 7.24 (m, 1 H) 12.20 (br. s, 1 H) 13.67 (s, 1 H) 16.23 (br. s, 1 H). LC-MS 340.2 [M-H]⁻, 342.1 [M+H]⁺, RT 1.25 min.

### Example 95

### 9-(3-(dimethylamino)pyrrolidin-1-yl)-4-hydroxy-2-oxo-2,5,6,7-tetrahydro-1H-benzo[b]pyrido[2,3-d]azepine-3-carboxylic acid hydrochloride (Cpd 95)

To a vial was added 7-benzyl 3-methyl 9-chloro-1-(2,4-dimethoxybenzyl)-4-hydroxy-2-oxo-5,6-dihydro-1H-benzo[b]pyrido[2,3-d]azepine-3,7(2H)-dicarboxylate (Example 94, step 3, 189 mg, 0.31 mmol), NaOt-Bu (120 mg, 1.24 mmol), and 2-(2'-Di-tert-butylphosphine)biphenylpalla-dium(II) acetate (4 mg, 8.7 µmol). The vial was purged with Argon for 5 minutes, then toluene (3.0 mL) and *N*,*N*-dimethylpyrrolidin-3-amine (79 µL, 0.62 mmol) were each added via syringe. The resultant solution was stirred at 90 °C for 60 minutes. The reaction was then cooled, diluted with CH₂Cl₂ (10 mL) and washed with HCl (15.0 mL, 1.0 M aqueous solution). The organic layer was then concentrated to yield a crude mixture (∼1:1) of desired product to deprotected product. The crude mixture was used directly in the next step. LC-MS (desired product) 667.5 [M-H]⁻, 669.5 [M+H]⁺, RT 1.20 min. LC-MS (deprotected product) 533.3 [M-H]⁻, RT 1.09 min.

The crude mixture from above (∼0.3 mmol) was suspended in MeOH (3.0 mL) and THF (2.0 mL) and Pd(OH)₂ (100 mg) was added. The resultant suspension was placed under H₂ (1 atm). After 1 hour of vigorous stirring, the starting material was consumed and the reaction mixture was diluted with EtOAc and filtered through a pad of Celite. The solution was concentrated and purified directly by preparative HPLC. The product was obtained as a TFA salt which and was subsequently deprotected. LC-MS (deprotected product) 533.3 [M-H]⁻, RT 1.09 min.

To a vial was added 1-(2,4-dimethoxybenzyl)-9-(3-(dimethylamino)pyrrolidin-1-yl)-4-hydroxy-2-oxo-2,5,6,7-tetrahydro-1H-benzo[b]pyrido[2,3-d]azepine-3-carboxylic acid trifluroacetic acid (1.0 mL) and CH₂Cl₂ (1.0 mL). The resultant solution was stirred at room temperature for 2 hours. The solution was concentrated under reduced pressure, the product was obtained following addition of an HCl solution (2M Et₂O, 1.5 mL) to the oily residue resulted in precipitate formation. The mixture was diluted with Et₂O and the resulting solid was filtered and washed with Et₂O. The product HCl salt was obtained as a pale pink solid (27 mg, 22% over two steps).
¹H NMR (500 MHz, DMSO-*d*₆) δ ppm 2.56 - 2.68 (m, 2 H) 2.80 (m, *J*=5.2, 5.2 Hz, 6 H) 3.27 - 3.39 (m, 1 H) 3.49 - 3.81 (m, 6 H) 3.92 - 4.09 (m, 2 H) 6.48 - 6.76 (m, 2 H) 7.39 - 7.49 (m, 1 H) 11.40 (s, 1 H) 12.73 (s, 1 H) 13.78 (s, 1 H). LC-MS 383.2 [M-H]⁻, 385.3 [M+H]⁺, RT 0.85 min.

### Example 96

### 4-hydroxy-9-(4-methylpiperazin-1-yl)-2-oxo-2,5,6,7-tetrahydro-1H-benzo[b]pyrido[2,3-d]azepine-3-carboxylic acid hydrochloride (Cpd 96)

To a vial was added 7-benzyl 3-methyl 9-chloro-1-(2,4-dimethoxybenzyl)-4-hydroxy-2-oxo-5,6-dihydro-1H-benzo[b]pyrido[2,3-d]azepine-3,7(2H)-dicarboxylate (Example 94, step 3, 189 mg, 0.31 mmol), NaOt-Bu (120 mg, 1.24 mmol), and 2-(2'-Di-tert-butylphosphine)biphenylpalla-dium(II) acetate (4 mg, 8.7 µmol). The vial was purged with Argon for 5 minutes, then toluene (3.0 mL) and 1-methylpiperazine (69 µL, 0.62 mmol) were each added via syringe. The resultant solution was stirred at 90 °C for 60 minutes. The reaction was then cooled, diluted with CH₂Cl₂ (10 mL) and washed with HCl (15.0 mL, 1.0 M aqueous solution). The organic layer was then concentrated to yield a crude mixture (∼1:1) of desired product to deprotected product. The crude mixture was used directly in the next step. LC-MS (desired product) 653.5 [M-H]⁻, 655.5 [M+H]⁺, RT 1.16 min. LC-MS (deprotected product) 519.2 [M-H]⁻, RT 1.08 min.

The crude mixture from above (∼0.3 mmol) was suspended in MeOH (3.0 mL) and THF (2.0 mL) and Pd(OH)₂ (100 mg) was added. The resultant suspension was placed under H₂ (1 atm). After 1 hour of vigorous stirring, the starting material had been consumed and the reaction was diluted with EtOAc and filtered through a pad of Celite. The solution was concentrated and purified directly by preparative HPLC. The product was obtained as a TFA salt before deprotection. LC-MS (deprotected product) 519.2 [M-H]⁻, RT 1.08 min.

To a vial was added 1-(2,4-dimethoxybenzyl)-4-hydroxy-9-(4-methylpiperazin-1-yl)-2-oxo-2,5,6,7-tetrahydro-1H-benzo[b]pyrido[2,3-d]azepine-3-carboxylic acid, trifluroacetic acid (1.0 mL) and CH₂Cl₂ (1.0 mL). The resultant solution was stirred at room temperature for 2 hours. The solution was concentrated under reduced pressure, the product was obtained following addition of an HCl solution (2M Et₂O, 1.5 mL) to the oily residue resulted in precipitate formation. The mixture was diluted with Et₂O and the resulting solid was filtered and washed with Et₂O. The product HCl salt was obtained as a pale pink solid (42 mg, 36% over two steps).
¹H NMR (500 MHz, DMSO-*d*₆) δ ppm 2.60 - 2.69 (m, 2 H) 2.81 (d, *J*=3.2 Hz, 3 H) 3.05 - 3.28 (m, 4 H) 3.43 - 3.56 (m, 2 H) 3.58 - 3.65 (m, 2 H) 3.80 - 3.96 (m, 2 H) 6.57 - 7.02 (m, 2 H) 7.36 - 7.44 (m, 1 H) 11.08 (br. s, 1 H) 12.67 (br. s, 1 H) 13.79 (s, 1 H). LC-MS 369.2 [M-H]⁻, 371.2 [M+H]⁺, RT 0.86 min.

### Example 97

### 9-((cis,cis)-6-amino-3-azabicyclo[3.1.0]hexan-3-yl)-4-hydroxy-2-oxo-2,5,6,7-tetrahydro-1H-benzo[b]pyrido[2,3-d]azepine-3-carboxylic acid hydrochloride (Cpd 97)

To a vial was added 7-benzyl 3-methyl 9-chloro-1-(2,4-dimethoxybenzyl)-4-hydroxy-2-oxo-5,6-dihydro-1H-benzo[b]pyrido[2,3-d]azepine-3,7(2H)-dicarboxylate (Example 94, step 3, 189 mg, 0.31 mmol), NaOt-Bu (120 mg, 1.24 mmol), and 2-(2'-Di-tert-butylphosphine)biphenylpalla-dium(II) acetate (4 mg, 8.7 µmol). The vial was purged with Argon for 5 minutes, then toluene (3.0 mL) and (cis,cis)-N,N-dibenzyl-3-azabicyclo[3.1.0]hexan-6-amine (173 mg, 0.62 mmol) were each added via syringe. The resultant solution was stirred at 90 °C for 60 minutes. The reaction was then cooled, diluted with CH₂Cl₂ (10 mL) and washed with HCl (15.0 mL, 1.0 M aqueous solution). The organic layer was then concentrated to yield the crude deprotected product (loss of Cbz group). The crude product was used directly in the next step. LC-MS (deprotected product) 699.7 [M+H]⁺, RT 1.78 min.

The crude mixture from above (∼0.3 mmol) was suspended in MeOH (3.0 mL) and THF (2.0 mL) and Pd(OH)₂ (100 mg) was added. The resultant suspension was placed under H₂ (1 atm). After 1 hour of vigorous stirring, the starting material had been consumed and the reaction was diluted with EtOAc and filtered through a pad of Celite. The solution was concentrated and purified directly by preparative HPLC. The product was obtained as a TFA salt before deprotection.LC-MS (deprotected product - both Cbz and Bn group removal) 517.3 [M-H]⁻, 519.3 [M+H]⁺, RT 1.14 min.

To a vial was added 9-((cis,cis)-6-amino-3-azabicyclo[3.1.0]hexan-3-yl)-1-(2,4-dimethoxybenzyl)-4-hydroxy-2-oxo-2,5,6,7-tetrahydro-1H-benzo[b]pyrido[2,3-d]azepine-3-carboxylic acid, trifluroacetic acid (1.0 mL) and CH₂Cl₂ (1.0 mL). The resultant solution was stirred at room temperature for 2 hours. The solution was concentrated under reduced pressure, the product was obtained following addition of an HCl solution (2M Et₂O, 1.5 mL) to the oily residue resulted in precipitate formation. The mixture was diluted with Et₂O and the resulting solid was filtered and washed with Et₂O. The product HCl salt was obtained as a pale pink solid (2.1 mg, 1.8% over two steps).
¹H NMR (500 MHz, DMSO-*d*₆) δ ppm 2.09 - 2.22 (m, 2 H) 2.31 - 2.39 (m, 1 H) 2.61 - 2.67 (m, 1 H) 3.27 - 3.44 (m, 2 H) 3.51 - 3.76 (m, 5 H) 6.21 - 6.51 (m, 1 H) 7.27 - 7.34 (m, 1 H) 8.30 - 8.49 (m, 1 H) 12.57 (br. s, 1 H) 13.74 (s, 1 H). LC-MS 367.2 [M-H]⁻, 369.2 [M+H]⁺, RT 0.88 min.

### Example 98

### 9-(hexahydro-1H-pyrrolo[3,4-b]pyridin-6(2H)-yl)-4-hydroxy-2-oxo-2,5,6,7-tetrahydro-1H-benzo[b]pyrido[2,3-d]azepine-3-carboxylic acid hydrochloride (Cpd 98)

To a vial was added 7-benzyl 3-methyl 9-chloro-1-(2,4-dimethoxybenzyl)-4-hydroxy-2-oxo-5,6-dihydro-1H-benzo[b]pyrido[2,3-d]azepine-3,7(2H)-dicarboxylate (Example 94, step 3, 189 mg, 0.31 mmol), NaOt-Bu (120 mg, 1.24 mmol), and 2-(2'-Di-tert-butylphosphine)biphenylpalla-dium(II) acetate (4 mg, 8.7 µmol). The vial was purged with Argon for 5 minutes, then toluene (3.0 mL) and tert-butyl octahydro-1H-pyrrolo[3,4-b]pyridine-1-carboxylate (140 mg, 0.62 mmol) were each added via syringe. The resultant solution was stirred at 90 °C for 60 minutes. The reaction was then cooled, diluted with CH₂Cl₂ (10 mL) and washed with HCl (15.0 mL, 1.0 M aqueous solution). The organic layer was then concentrated to yield a crude mixture (∼1:1) of desired product to deprotected product. The crude mixture was used directly in the next step. LC-MS (desired product) 779.5 [M-H]⁻, 781.6 [M+H]⁺, RT 1.73 min. LC-MS (deprotected product - Cbz removal) 645.4 [M-H]⁻, 647.5 [M+H]⁺, RT 1.65 min.

The crude mixture from above (∼0.3 mmol) was suspended in MeOH (3.0 mL) and THF (2.0 mL) and Pd(OH)₂ (100 mg) was added. The resultant suspension was placed under H₂ (1 atm). After 1 hour of vigorous stirring, the starting material had been consumed and the reaction was diluted with EtOAc and filtered through a pad of Celite. The solution was concentrated and purified directly by preparative HPLC. The product was obtained as a TFA salt before deprotection. LC-MS (deprotected product - Cbz removal) 645.4 [M-H]⁻, 647.5 [M+H]⁺, RT 1.65 min.

To a vial was added 9-(1-(tert-butoxycarbonyl)hexahydro-1H-pyrrolo[3,4-b]pyridin-6(2H)-yl)-1-(2,4-dimethoxybenzyl)-4-hydroxy-2-oxo-2,5,6,7-tetrahydro-1H-benzo[b]pyrido[2,3-d]azepine-3-carboxylic acid, trifluroacetic acid (1.0 mL) and CH₂Cl₂ (1.0 mL). The solution was concentrated under reduced pressure, the product was obtained following addition of an HCl solution (2M Et₂O, 1.5 mL) to the oily residue resulted in precipitate formation. The mixture was diluted with Et₂O and the resulting solid was filtered and washed with Et₂O. The product HCl salt was obtained as a pale pink solid (15 mg, 12% over two steps).
¹H NMR (500 MHz, DMSO-*d*₆) δ ppm 1.57 - 1.89 (m, 4 H) 2.53 - 2.65 (m, 2 H) 2.75 (br. s., 1 H) 2.89 (d, *J*=9.8 Hz, 1 H) 3.15 (d, *J*=12.0 Hz, 1 H) 3.31 - 3.57 (m, 3 H) 3.60 - 3.78 (m, 4 H) 3.89 (d, *J*=3.8 Hz, 1 H) 6.63 (d, *J*=8.2 Hz, 1 H) 6.86 (br. s., 1 H) 7.47 (d, *J*=8.5 Hz, 1 H) 8.81 (d, *J*=9.5 Hz, 1 H) 10.06 (d, *J*=9.1 Hz, 1 H) 12.88 (br. s, 1 H) 13.78 (br. s, 1 H). LC-MS 395.3 [M-H]⁻, 397.2 [M+H]⁺, RT 0.89 min.

### Example 99

### 4-hydroxy-9-methyl-10-((methylamino)methyl)-2-oxo-1,2,5,6,7,9-hexahydropyrido[2',3':4,5]azepino[3,2-f]indole-3-carboxylic acid hydrochloride (Cpd 99)

### Step 1: tert-butyl (2-(((tert-butyldimethylsilyl)oxy)methyl)-5-formyl-1-methyl-1H-indol-6-yl)carbamate

A round bottomed flask was charged with 2-(((tert-butyldimethylsilyl)oxy)methyl)-6-chloro-1-methyl-1H-indole-5-carbaldehyde (Example 19, step 5, 3.0 g, 8.87 mmol), tert-butyl carbamate (1.56 g, 13.31 mmol), palladium acetate (60 mg, 0.27 mmol), S-Phos (182 mg, 0.44 mmol) and Cs₂CO₃ (4.33 g, 13.31 mmol). The flask was purged with Argon for 10 minutes and dioxane (30 mL) was added via cannula. The mixture was then heated at 100 °C for 4 hours. After cooling the reaction to room temperature, the mixture was filtered through a plug of silica gel, washed with EtOAc (250 mL) and concentrated under reduced pressure. The residue was purified by column chromatography (0 to 15% gradient of EtOAc in hexanes) to give the desired product (3.55 g, 94%) as an off-white solid.
¹H NMR (500 MHz, CHCl₃-*d*) δ ppm -0.13 - 0.21 (m, 6 H) 0.72 - 1.04 (m, 9 H) 1.57 (s, 9 H) 3.79 (s, 3 H) 4.81 (s, 2 H) 6.43 (d, *J*=0.6 Hz, 1 H) 7.81 (s, 1 H) 8.36 (s, 1 H) 9.87 (s, 1 H) 10.63 (s, 1 H). LC-MS RT 1.73 min.

### Step 2: tert-butyl allyl(2-(((tert-butyldimethylsilyl)oxy)methyl)-5-formyl-1-methyl-1H-indol-6-yl)carbamate

A round bottomed flask was charged with tert-butyl (2-(((tertbutyldimethylsilyl)oxy)methyl)-5-formyl-1-methyl-1H-indol-6-yl)carbamate (4.95 g, 11.82 mmol). The flask was purged with Argon for 10 minutes and DMF (60 mL) was added via cannula. The solid NaH (710 mg, 17.74 mmol, 60 wt%) was then added in one portion and the suspension was stirred at room temperature. After 10 minutes, allyl iodide (2.2 mL, 23.64 mmol) was added via syringe. The solution was stirred under Argon for 15 minutes then poured into H₂O (250 mL). The product was then extracted with EtOAc (2 x 150 mL), dried over Na₂SO₄, then filtered and concentrated under reduced pressure. The product was directly purified by column chromatography (0 to 20% gradient of EtOAc in hexanes) to give the desired product (4.87 g, 90%) as a yellow solid.
¹H NMR (500 MHz, CHCl₃-*d*) δ ppm 0.02 - 0.16 (m, 6 H) 0.77 - 1.04 (m, 9 H) 1.26 - 1.62 (m, 9 H) 3.79 (s, 3 H) 4.20 - 4.45 (m, 2 H) 4.83 (s, 2 H) 5.04 - 5.19 (m, 2 H) 5.89 - 6.07 (m, 1 H) 6.51 (s, 1 H) 7.03 - 7.17 (m, 1 H) 8.15 (s, 1 H) 10.07 (s, 1 H). LC-MS (loss of Boc group) 359.1 [M+H]⁺, RT 1.75 min.

### Steps 3-4: tert-butyl 2-(((tert-butyldimethylsilyl)oxy)methyl)-5-hydroxy-1-methyl-5,8-dihydroazepino[3,2-f]indole-9(1H)-carboxylate

A round bottomed flask was charged with tert-butyl allyl(2-(((tert-butyldimethylsilyl)oxy)methyl)-5-formyl-1-methyl-1H-indol-6-yl)carbamate (4.86 g, 10.59 mmol). The flask was purged with Argon for 10 minutes and THF (50 mL) was added via cannula. The solution was cooled to -78 °C using a dry ice/acetone bath. At -78 °C, prop-1-en-1-ylmagnesium bromide (30.0 mL, 14.83 mmol, 0.5M in THF) was added dropwise. After completion of the addition, the cooling bath was removed and the reaction mixture was allowed to warm to room temperature over 1 hour. The reaction was then quenched by the addition of saturated aqueous NH₄Cl (100 mL). The product was extracted with EtOAc (2 x 150 mL), dried over Na₂SO₄, then filtered and concentrated under reduced pressure. The desired product was obtained as a glassy yellow solid (4.60 g, 87%) which was used directly in the next step.

To a round bottomed flask was added crude tert-butyl allyl(2-(((tertbutyldimethylsilyl)oxy)methyl)-5-(1-hydroxybut-2-en-1-yl)-1-methyl-1H-indol-6-yl)carbamate (2.7 g, 5.40 mmol). The flask was purged with Argon for 10 minutes and CH₂Cl₂ (100 mL) was added via cannula. Under Argon, solid (1,3-bis(2,4,6-trimethylphenyl)-2-imidazolidinylidene)dichloro(phenylmethylene)(tricyclohexylphosphine)ruthenium (230 mg, 0.27 mmol) was added and the mixture was stirred at 40 °C for 3 hours. After consumption of the starting material, the reaction mixture was concentrated using silica gel and purified by column chromatography (0 to 35% gradient of EtOAc in hexanes) to give the desired product (1.63 g, 66%) as a tan solid
¹H NMR (500 MHz, DMSO-*d*₆) δ ppm 0.05 (d, *J*=4.1 Hz, 6 H) 0.77 - 0.95 (m, 9 H) 1.21 - 1.56 (m, 9 H) 3.36 - 3.49 (m, 1 H) 3.71 (s, 3 H) 4.56 - 4.75 (m, 1 H) 4.84 (s, 2 H) 5.21 - 5.41 (m, 1 H) 5.46 - 5.77 (m, 3 H) 6.39 (s, 1 H) 7.23 (br. s., 1 H) 7.52 (s, 1 H). LC-MS RT 1.71 min.

### Step 5: tert-butyl 2-(((tert-butyldimethylsilyl)oxy)methyl)-1-methyl-5-oxo-5,8-dihydroazepino[3,2-f]indole-9(1H)-carboxylate

Tert-butyl 2-(((tert-butyldimethylsilyl)oxy)methyl)-5-hydroxy-1-methyl-5,8-dihydroazepino[3,2-f]indole-9(1H)-carboxylate (2.0 g, 4.40 mmol) was added to a round bottomed flask. The flask was purged with Argon for 10 minutes and CH₂Cl₂ (25 mL) was added via cannula. Under Argon, solid MnO₂ (230 mg, 0.27 mmol) was added and the mixture was stirred at room temperature for 2 hours. After consumption of the starting material, the reaction was filtered through a plug of silica gel (eluting with EtOAc) and concentrated under reduced pressure. The desired product (2.0 g, quant) was isolated as yellow crystals which required no additional purification.
¹H NMR (500 MHz, DMSO-*d*₆) δ ppm 0.06 (s, 6 H) 0.76 - 0.97 (m, 9 H) 1.18 - 1.50 (m, 9 H) 3.77 (s, 3 H) 4.87 (s, 2 H) 6.10 - 6.32 (m, 1 H) 6.56 (s, 1 H) 6.87 - 7.04 (m, 1 H) 7.37 (s, 1 H) 7.88 (br. s., 1 H). LC-MS 357.2 [M+H]⁺, RT 1.75 min.

### Step 6: tert-butyl 2-(((tert-butyldimethylsilyl)oxy)methyl)-1-methyl-5-oxo-5,6,7,8-tetrahydroazepino[3,2-f]indole-9(1H)-carboxylate

A round-bottomed flask was charged with tert-Butyl 2-(((tert-butyldimethylsilyl)oxy)-methyl)-1-methyl-5-oxo-5,8-dihydroazepino[3,2-f]indole-9(1H)-carboxylate (2.0 g, 4.4 mmol), EtOAc (20 mL) was then added via syringe. To the stirring solution was added solid Pd/C (200 mg) and the reaction was then placed under H₂ (1 atm). The suspension was stirred vigorously at room temperature for 3 hours. After the reaction was completed, as shown by LCMS, the mixture was filtered through a short plug of silica gel, eluting with EtOAc. The eluted solution was concentrated under reduced pressure purified by column chromatography (0 to 40% gradient of EtOAc in hexanes) to give the desired product (1.81 g, 90%) as a light yellow foam.
¹H NMR (500 MHz, DMSO-*d*₆) δ ppm 0.06 (s, 6 H) 0.68 - 1.01 (m, 9 H) 1.20 - 1.61 (m, 9 H) 1.79 - 2.11 (m, 2 H) 2.51 - 2.64 (m, 2 H) 3.54 - 3.84 (m, 5 H) 4.86 (s, 2 H) 6.55 (s, 1 H) 7.39 (s, 1 H) 7.81 - 8.05 (m, 1 H). LC-MS RT 1.70 min.

### Step 7: tert-butyl 2-(((tert-butyldimethylsilyl)oxy)methyl)-5-((2,4-dimethoxybenzyl)imino)-1-methyl-5,6,7,8-tetrahydroazepino[3,2-f]indole-9(1H)-carboxylate

To a solution of tert-butyl 2-(((tert-butyldimethylsilyl)oxy)methyl)-1-methyl-5-oxo-5,6,7,8-tetrahydroazepino[3,2-f]indole-9(1H)-carboxylate (1.76 g, 3.84 mmol) in CH₂Cl₂ (13 mL) was added 2,4-dimethoxybenzylamine (640 µL, 4.22 mmol) and NEt₃ (1.6 mL, 11.52 mmol). The mixture was cooled to 0 °C, then a solution of TiCl₄ (1.0M CH₂Cl₂, 2.5 mL, 2.49 mmol) was added dropwise via syringe pump over 30 minutes. The reaction mixture was allowed to warm to room temperature and stirred overnight. The mixture was diluted with CH₂Cl₂ (20 mL) and then the reaction was quenched with NaHCO₃ (sat. aq., 10 mL). Following vigorous shaking, the organic phase was separated using a PTFE phase separator, then dried over Na₂SO₄ and filtered. The solvent was removed and the resulting crude product was obtained (2.11 g, ∼91 %) as a yellow solid, which was used directly in the next step without further purification. LC-MS 608.3 [M+H]⁺, RT 1.41 min.

### Step 8: 7-tert-butyl 3-methyl 10-(((tert-butyldimethylsilyl)oxy)methyl)-1-(2,4-dimethoxy-benzyl)-4-hydroxy-9-methyl-2-oxo-1,2,5,6-tetrahydropyrido[2',3':4,5]azepino[3,2-f]indole-3,7(9H)-dicarboxylate

Crude tert-butyl 2-(((tert-butyldimethylsilyl)oxy)methyl)-5-((2,4-dimethoxybenzyl)imino)-1-methyl-5,6,7,8-tetrahydroazepino[3,2-f]indole-9(1H)-carboxylate (2.11 g, 3.46 mmol) and trimethyl methanetricarboxylate (1.12 g, 5.89 mmol) were mixed together in Ph₂O (7.0 mL). With stirring, the mixture was placed into a pre-heated heat block at 210 °C and heated for 10 minutes under a blanket of Argon. The reaction mixture was cooled to room temperature and filtered directly on a silica cartridge. The product was purified by column chromatography (100% hexanesfollowed by EtOAc in hexanes 0 to 85% gradient) to give the desired product (1.75 g, 69%) as burnt orange crystals which were used directly in the next step. LC-MS 734.2 [M+H]⁺, RT 1.86 min.

### Steps 9-11: 7-(tert-butoxycarbonyl)-1-(2,4-dimethoxybenzyl)-10-formyl-4-hydroxy-9-methyl-2-oxo-1,2,5,6,7,9-hexahydropyrido[2',3':4,5]azepino[3,2-f]indole-3-carboxylic acid

To a solution of 7-tert-butyl 3-methyl 10-(((tert-butyldimethylsilyl)oxy)methyl)-1-(2,4-dimethoxy-benzyl)-4-hydroxy-9-methyl-2-oxo-1,2,5,6-tetrahydropyrido-[2',3':4,5]azepino[3,2-f]indole-3,7(9H)-dicarboxylate (1.75 g, 2.38 mmol) in THF (10 mL) was added TBAF (1M in THF, 6.0 mL, 6.0 mmol). The reaction mixture was stirred at room temperature for 2 h until the starting material was completely consumed. The THF was removed and the residue was purified by column chromatography (EtOAc/DCM, 0-100% gradient). The product (1.16 g, 80 %) was obtained as yellow crystals which were used directly in the next step. LC-MS 618.3 [M-H]⁻, 620.3 [M+H]⁺, RT 1.37 min.

To a suspension of 7-tert-butyl 3-methyl 1-(2,4-dimethoxybenzyl)-4-hydroxy-10-(hydroxymethyl)-9-methyl-2-oxo-1,2,5,6-tetrahydropyrido[2',3':4,5]azepino[3,2-f]indole-3,7(9H)-dicarboxylate (1.16 g, 1.87 mmol) in EtOAc (6.0 mL) was added LiI (750 mg, 5.62 mmol). The reaction mixture was stirred and heated at 60 °C until complete consumption of starting material was observed (∼2 hours). The mixture was then cooled to room temperature and the reaction was quenched with aqueous HCl (1.0 M, 10 mL) and diluted with H₂O. The product was extracted with EtOAc (2x100 mL) and the organic phase was washed with Na₂S₂O₃ (10% aq., 40 mL), NaCl (sat. aq., 100 mL) and dried over Na₂SO₄. The solvent was removed and the resulting product (1.04 g, 92%) was obtained as yellow solid that was used directly in the next step. LC-MS 604.6 [M-H]⁻, 606.6 [M+H]⁺, RT 1.37 min.

To a solution of 7-(tert-butoxycarbonyl)-1-(2,4-dimethoxybenzyl)-4-hydroxy-10-(hydroxymethyl)-9-methyl-2-oxo-1,2,5,6,7,9-hexahydropyrido[2',3':4,5]azepino[3,2-f]indole-3-carboxylic acid (1.04 g, 1.73 mmol) in DCM (9.0 mL) was added activated MnO₂ (3.7 g, 43.1 mmol). The reaction was monitored by LC/MS. After complete consumption of the starting material, MnO₂ was filtered and washed with DCM. The mother liquor was concentrated to afford the product (833 mg, 80%) as an off-white foam that required no further purification.
¹H NMR (500 MHz, DMSO-*d*₆) δ ppm 1.00 - 1.40 (m, 9 H) 3.32 (br. s., 5 H) 3.43 - 3.64 (m, 2 H) 3.78 (s, 3 H) 4.05 (s, 3 H) 4.72 - 5.29 (m, 2 H) 6.57 (m, *J*=1.9 Hz, 1 H) 6.73 - 7.01 (m, 1 H) 7.24 - 7.35 (m, 1 H) 7.35 - 7.48 (m, 1 H) 7.47 - 7.58 (m, 1 H) 7.48 - 7.48 (m, 1 H) 7.74 (s, 1 H) 9.91 (s, 1 H) 13.54 - 14.03 (m, 1 H) 15.75 (s, 1 H). LC-MS 603.5 [M-H]⁻, 604.5 [M+H]⁺, RT 1.50 min.

### Steps 12-13: 4-hydroxy-9-methyl-10-((methylamino)methyl)-2-oxo-1,2,5,6,7,9-hexahydropyrido[2',3':4,5]azepino[3,2-f]indole-3-carboxylic acid hydrochloride.

To a solution of 7-(tert-butoxycarbonyl)-1-(2,4-dimethoxybenzyl)-10-formyl-4-hydroxy-9-methyl-2-oxo-1,2,5,6,7,9-hexahydropyrido[2',3':4,5]azepino[3,2-f]indole-3-carboxylic acid (200 mg, 0.33 mmol) in dichloroethane (3.5 mL) was added a solution of methyl amine (2.0 M THF, 330 µL, 0.66 mmol) followed by AcOH (47 µL, 0.66 mmol). After stirring at room temperature for 10 min, NaBH(OAc)₃ (147 mg, 0.70 mmol) was added. The reaction was stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt. LC-MS 617.4 [M-H]⁻, 619.5 [M+H]⁺, RT 1.06 min.

To the product obtained above was added i-Pr₃SiH (1.0 mL) followed by TFA (1.5 mL). The resultant mixture was heated at 60 °C for 2 h and monitored by LC/MS. After complete consumption of the starting material, the TFA was removed under reduced pressure. Addition of an HCl solution (2.0 M Et₂O, 2.0 mL) to the oily residue led to precipitate formation. The mixture was diluted with Et₂O, the solid was filtered and then washed with Et₂O. The product HCl salt was obtained as a pale pink solid (41 mg, 29%).
¹H NMR (500 MHz, DMSO-*d*₆) δ ppm 2.64 (t, *J*=5.0 Hz, 2 H) 3.69 - 3.78 (m, 2 H) 3.86 (s, 5 H) 4.46 (t, *J*=5.0 Hz, 2 H) 6.93 (s, 1 H) 7.80 - 7.93 (m, 1 H) 8.00 (s, 1 H) 9.26 - 9.61 (m, 2 H) 13.15 (s, 1 H) 13.89 (s, 1 H) 16.07 (s, 1 H). LC-MS 367.3 [M-H]⁻, RT 0.67 min.

### Example 100

### 10-((ethylamino)methyl)-4-hydroxy-9-methyl-2-oxo-1,2,5,6,7,9-hexahydropyrido[2',3':4,5]azepino[3,2-f]indole-3-carboxylic acid hydrochloride (Cpd 100)

To a solution of 7-(tert-butoxycarbonyl)-1-(2,4-dimethoxybenzyl)-10-formyl-4-hydroxy-9-methyl-2-oxo-1,2,5,6,7,9-hexahydropyrido[2',3':4,5]azepino[3,2-f]indole-3-carboxylic acid (Example 99, steps 9-11, 200 mg, 0.33 mmol) in dichloroethane (3.5 mL) was added a solution of ethyl amine (2.0 M THF, 330 µL, 0.66 mmol) followed by AcOH (47 µL, 0.66 mmol). After stirring at room temperature for 10 min, NaBH(OAc)₃ (147 mg, 0.70 mmol) was added. The reaction was stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt. LC-MS 631.5 [M-H]⁻, 633.5 [M+H]⁺, RT 1.08 min.

To the product obtained above was added i-Pr₃SiH (1.0 mL) followed by TFA (1.5 mL). The resultant mixture was heated at 60 °C for 2 h and monitored by LC/MS. After complete consumption of starting material, the TFA was removed under reduced pressure. Addition of an HCl solution (2.0 M Et₂O, 2.0 mL) to the oily residue led to precipitate formation. The mixture was diluted with Et₂O, the solid was filtered and then washed with Et₂O. The product HCl salt was obtained as a pale pink solid (43 mg, 30%).
¹H NMR (500 MHz, DMSO-*d*₆) δ ppm 1.29 (t, *J*=7.3 Hz, 3 H) 2.98 - 3.16 (m, 2 H) 3.64-3.81 (m, 4 H) 3.86 (s, 3 H) 4.46 (t, *J*=5.5 Hz, 2 H) 6.93 (s, 1 H) 7.78 - 7.91 (m, 1 H) 8.00 (s, 1 H) 9.21 - 9.55 (m, 2 H) 13.02 - 13.40 (m, 1 H) 13.69 - 14.10 (m, 1 H). LC-MS 381.4 [M-H]⁻, RT 0.69 min.

### Example 101

### 4-hydroxy-10-((isopropylamino)methyl)-9-methyl-2-oxo-1,2,5,6,7,9-hexahydropyrido[2',3':4,5]azepino[3,2-f]indole-3-carboxylic acid hydrochloride (Cpd 101)

To a solution of 7-(tert-butoxycarbonyl)-1-(2,4-dimethoxybenzyl)-10-formyl-4-hydroxy-9-methyl-2-oxo-1,2,5,6,7,9-hexahydropyrido[2',3':4,5]azepino[3,2-f]indole-3-carboxylic acid (Example 99, steps 9-11, 200 mg, 0.33 mmol) in dichloroethane (3.5 mL) was added a solution of isopropyl amine (60 µL, 0.66 mmol) followed by AcOH (47 µL, 0.66 mmol). After stirring at room temperature for 10 min, NaBH(OAc)₃ (147 mg, 0.70 mmol) was added. The reaction was stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt. LC-MS 645.6 [M-H]⁻, 647.6 [M+H]⁺, RT 1.10 min.

To the product obtained above was added i-Pr₃SiH (1.0 mL) followed by TFA (1.5 mL). The resultant mixture was heated at 60 °C for 2 h and monitored by LC/MS. After complete consumption of starting material, the TFA was removed under reduced pressure. Addition of an HCl solution (2.0 M Et₂O, 2.0 mL) to the oily residue led to precipitate formation. The mixture was diluted with Et₂O, the solid was filtered and then washed with Et₂O. The product HCl salt was obtained as a pale pink solid (43 mg, 30%).
¹H NMR (500 MHz, DMSO-*d*₆) δ ppm 1.37 (d, *J=*6.6 Hz, 6 H) 3.36 - 3.55 (m, 1 H) 3.71 - 3.82 (m, 2 H) 3.87 (s, 2 H) 4.36 - 4.54 (m, 4 H) 6.96 (s, 1 H) 7.90 (s, 1 H) 8.01 (s, 1 H) 9.43 (br. s., 2 H) 13.21 (s, 1 H) 13.92 (s, 1 H). LC-MS 395.3 [M-H]⁻, RT 0.70 min.

### Example 102

### 10-((tert-butylamino)methyl)-4-hydroxy-9-methyl-2-oxo-1,2,5,6,7,9-hexahydropyrido[2',3':4,5]azepino[3,2-f]indole-3-carboxylic acid hydrochloride (Cpd 102)

To a solution of 7-(tert-butoxycarbonyl)-1-(2,4-dimethoxybenzyl)-10-formyl-4-hydroxy-9-methyl-2-oxo-1,2,5,6,7,9-hexahydropyrido[2',3':4,5]azepino[3,2-f]indole-3-carboxylic acid (Example 99, steps 9-11, 200 mg, 0.33 mmol) in dichloroethane (3.5 mL) was added a solution of tert-butyl amine (2.0 M THF, 70 µL, 0.66 mmol) followed by AcOH (47 µL, 0.66 mmol). After stirring at room temperature for 10 min, NaBH(OAc)₃ (147 mg, 0.70 mmol) was added. The reaction was stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt. LC-MS 659.6 [M-H]⁻, 661.6 [M+H]⁺, RT 1.13 min.

To the product obtained above was added i-Pr₃SiH (1.0 mL) followed by TFA (1.5 mL). The resultant mixture was heated at 60 °C for 2 h and monitored by LC/MS. After complete consumption of starting material, the TFA was removed under reduced pressure. Addition of an HCl solution (2.0 M Et₂O, 2.0 mL) to the oily residue led to precipitate formation. The mixture was diluted with Et₂O, the solid was filtered and then washed with Et₂O. The product HCl salt was obtained as a pale pink solid (9 mg, 6%).
¹H NMR (500 MHz, DMSO-*d*₆) δ ppm 1.44 (s, 9 H) 3.56 - 3.80 (m, 4 H) 3.86 (s, 3 H) 4.34-4.50 (m, 2 H) 6.93 (s, 1 H) 7.85 (s, 1 H) 8.00 (s, 1 H) 9.36 (br. s, 2 H) 13.20 (s, 1 H) 13.90 (s, 1 H). LC-MS 409.3 [M-H]⁻, RT 0.71 min.

### Example 103

### 4-hydroxy-10-methyl-11-((methylamino)methyl)-2-oxo-2,5,6,7,8,10-hexahydro-1H-pyrido[2',3':4,5]azocino[3,2-f]indole-3-carboxylic acid hydrochloride (Cpd 103)

### Step 1: tert-butyl but-3-en-1-yl(2-(((tert-butyldimethylsilyl)oxy)methyl)-5-formyl-1-methyl-1H-indol-6-yl)carbamate

A round bottomed flask was charged with tert-butyl (2-(((tert-butyldimethylsilyl)oxy)methyl)-5-formyl-1-methyl-1H-indol-6-yl)carbamate (Example 99, step 1, 9.3 g, 22.2 mmol). The flask was purged with Argon for 10 minutes and DMF (100 mL) was added via cannula. The solid NaH (1.3 g, 33.3 mmol, 60 wt%) was then added in one portion and the suspension was stirred at room temperature. After 10 minutes, 4-bromobut-1-ene (8.9 g, 66.6 mmol) was added via syringe. The solution was heated at 80 °C for 3 hours and then poured into H₂O (250 mL). The product was extracted with EtOAc (2 x 150 mL), dried over Na₂SO₄, then filtered and concentrated under reduced pressure. The concentrate was purified by column chromatography (0 to 20% gradient of EtOAc in hexanes) to give the desired product (3.71 g, 36%) as a yellow solid.
¹H NMR (500 MHz, DMSO-*d*₆) δ ppm 0.09 (s, 6 H) 0.89 (s, 9 H) 1.20 (br. s., 4 H) 1.36 - 1.56 (m, 2 H) 2.14 - 2.39 (m, 2 H) 3.62 - 3.91 (m, 6 H) 4.87 (s, 3 H) 4.95 - 5.10 (m, 2 H) 5.53 - 5.92 (m, 2 H) 6.62 (s, 1 H) 7.48 (s, 1 H) 8.05 (s, 1 H) 9.91 (br. s, 1 H). LC-MS (loss of Boc group) 373.5 [M+H]⁺, RT 1.74 min.

### Steps 2-4: tert-butyl 2-(((tert-butyldimethylsilyl)oxy)methyl)-1-methyl-5-oxo-8,9-dihydro-1H-azocino [3,2-f]indole-10(5H)-carboxylate

Tert-butyl but-3-en-1-yl(2-(((tert-butyldimethylsilyl)oxy)methyl)-5-formyl-1-methyl-1H-indol-6-yl)carbamate (3.71 g, 7.86 mmol) was added to a round bottomed flask. The flask was purged with Argon for 10 minutes and THF (40 mL) was added via cannula. The solution was cooled to -78 °C using dry ice/acetone bath. At -78 °C, prop-1-en-1-ylmagnesium bromide (22.0 mL, 11.0 mmol, 0.5M in THF) was added dropwise. After completion of the addition, the cooling bath was removed and the reaction mixture was allowed to warm to room temperature over 1 hour. The reaction was then quenched by the addition of saturated aqueous NH₄Cl (100 mL). The product was extracted with EtOAc (2 x 150 mL), dried over Na₂SO₄, then filtered and concentrated under reduced pressure. The desired crude product was obtained as a yellow glassy solid (4.51 g, >95%) which was used directly in the next step.

To a round-bottomed flask was added crude tert-butyl but-3-en-1-yl(2-(((tert-butyldimethylsilyl)oxy)methyl)-5-(1-hydroxybut-2-en-1-yl)-1-methyl-1H-indo 1-6-yl)carbamate (-7.8 mmol). The flask was purged with Argon for 10 minutes and CH₂Cl₂ (150 mL) was added via cannula. Under Argon, solid (1,3-bis(2,4,6-trimethylphenyl)-2-imidazolidinylidene)dichloro(phenylmethylene)(tricyclohexyl-phosphine)ruthenium (330 mg, 0.39 mmol) was added and the mixture was stirred at 40 °C for 1 hour. After consumption of the starting material, the reaction was filtered through a plug of silica gel, eluting with 1:1 hexanes:EtOAc. A crude product was collected after removal of the solvent under reduced pressure and was used directly in the next step without additional purification.

To a round-bottomed flask was added tert-butyl 2-(((tert-butyldimethylsilyl)oxy)-methyl)-5-hydroxy-1-methyl-8,9-dihydro-1H-azocino[3,2-f]indole-10(5H)-carboxylate (∼7.8 mmol). The flask was purged with Argon for 10 minutes and CH₂Cl₂ (40 mL) was added via cannula. Under Argon, solid MnO₂ (16 g, 183 mmol) was added and the mixture was stirred at room temperature for 2 hours. After consumption of the starting material, the reaction was filtered through a plug of silica gel (eluting with EtOAc) and concentrated under reduced pressure. The desired product (3.34 g, 91 % over three steps) was isolated as a light colored solid which required no additional purification.
¹H NMR (500 MHz, DMSO-*d*₆) δ ppm 0.00 - 0.14 (m, 6 H) 0.77 - 0.95 (m, 9 H) 1.08 - 1.19 (m, 9 H) 2.04 - 2.19 (m, 2 H) 3.32 - 3.42 (m, 1 H) 3.77 (s, 3 H) 4.08 - 4.23 (m, 1 H) 4.73 - 5.03 (m, 2 H) 6.22 (d, *J*=12.0 Hz, 1 H) 6.48 (d, *J*=12.0 Hz, 1 H) 6.56 (s, 1 H) 7.44 (s, 1 H) 7.86 (s, 1 H). LC-MS RT 1.05 min.

### Step 5: tert-butyl 2-(((tert-butyldimethylsilyl)oxy)methyl)-1-methyl-5-oxo-6,7,8,9-tetrahydro-1H-azocino[3,2-f]indole-10(5H)-carboxylate

A round-bottomed flask was charged with tert-butyl 2-(((tertbutyldimethylsilyl)oxy)methyl)-1-methyl-5-oxo-8,9-dihydro-1H-azocino[3,2-f]indole-10(5H)-carboxylate (3.34 g, 7.1 mmol), EtOAc (40 mL) was then added via syringe. To the stirring solution was added solid Pd/C (400 mg) and the reaction was then placed under H₂ (1 atm). The suspension was stirred vigorously at room temperature for 2 hours. After the reaction was completed, as shown by LCMS, the mixture was filtered through a short plug of silica gel, eluting with EtOAc. Thesolution was concentrated under reduced pressure, then the concentrate was purified by column chromatography (0 to 20% gradient of EtOAc in hexanes) to give the desired product (2.24 g, 68%) as a white solid. LC-MS 1.71 min.

### Step 6: tert-butyl2-(((tert-butyldimethylsilyl)oxy)methyl)-5-((2,4-dimethoxybenzyl)imino)-1-methyl-6,7,8,9-tetrahydro-1H-azocino[3,2-f]indole-10(5H)-carboxylate

To a solution of tert-butyl 2-(((tert-butyldimethylsilyl)oxy)methyl)-1-methyl-5-oxo-6,7,8,9-tetrahydro-1H-azocino[3,2-f]indole-10(5H)-carboxylate (1.80 g, 3.78 mmol) in CH₂Cl₂ (13 mL) was added 2,4-dimethoxybenzylamine (630 µL, 4.18 mmol) and NEt₃ (1.6 mL, 11.5 mmol). The mixture was cooled to 0 °C, then a solution of TiCl₄ (1.0M CH₂Cl₂, 2.5 mL, 2.47 mmol) was added dropwise via syringe pump over 30 minutes. The reaction mixture was allowed to warm to room temperature and stirred overnight. The mixture was diluted with CH₂Cl₂ (20 mL) and then the reaction was quenched with NaHCO₃ (sat. aq., 10 mL). Following vigorous shaking, the organic phase was separated using a PTFE phase separator, then dried over Na₂SO₄ and filtered. The solvent was removed and the resulting crude product was obtained (2.27 g, -97 %) as a light yellow foam, which was used directly into the next step without purification. LC-MS 622.5 [M+H]⁺, RT 1.36 min.

### Step 7: 8-tert-butyl 3-methyl 11-(((tert-butyldimethylsilyl)oxy)methyl)-1-(2,4-dimethoxybenzyl)-4-hydroxy-10-methyl-2-oxo-5,6,7,10-tetrahydro-1H-pyrido [2',3' :4,5] azocino [3,2-f]indole-3,8(2H)-dicarboxylate

Crude tert-butyl 2-(((tert-butyldimethylsilyl)oxy)methyl)-5-((2,4-dimethoxybenzyl)imino)-1-methyl-6,7,8,9-tetrahydro-1H-azocino[3,2-f]indole-10(5H)-carboxylate (2.27 g, 3.65 mmol) and trimethyl methanetricarboxylate (1.17 g, 5.89 mmol) were mixed together in Ph₂O (7.0 mL). With stirring, the mixture was placed into a pre-heated heat block at 210 °C and heated for 10 minutes under a blanket of Argon. The reaction mixture was cooled to room temperature and filtered directly on a silica cartridge. The filtrate was purified by column chromatography (100% hexanes followed by EtOAc in hexanes 0 to 85% gradient) to give the desired product (1.31 g, 48%) as an off-white solid which was used directly in the next step. LC-MS 748.5 [M+H]⁺, RT 1.77 min.

### Step 8-10: 8-(tert-butoxycarbonyl)-1-(2,4-dimethoxybenzyl)-11-formyl-4-hydroxy-10-methyl-2-oxo-2,5,6,7,8,10-hexahydro-1H-pyrido[2',3':4,5]azocino[3,2-f]indole-3-carboxylic acid

To a solution of 8-tert-butyl 3-methyl 11-(((tert-butyldimethylsilyl)oxy)methyl)-1-(2,4-dimethoxybenzyl)-4-hydroxy-10-methyl-2-oxo-5,6,7,10-tetrahydro-1H-pyrido[2',3':4,5]azocino[3,2-f]indole-3,8(2H)-dicarboxylate (1.31 g, 1.75 mmol) in THF (10 mL) was added TBAF (1M in THF, 4.4 mL, 4.37 mmol). The reaction mixture was stirred at room temperature for 2 h until the starting material was completely consumed. The THF was removed and the residue was purified by column chromatography (EtOAc/DCM, 0-100% gradient). The product (0.94 g, 85 %) was obtained as an off-white solid used directly in the next step. LC-MS 634.3 [M+H]⁺, RT 1.35 min.

To a suspension of 8-tert-butyl 3-methyl 1-(2,4-dimethoxybenzyl)-4-hydroxy-11-(hydroxymethyl)-10-methyl-2-oxo-5,6,7,10-tetrahydro-1H-pyrido[2',3':4,5]azocino[3,2-f]indole-3,8(2H)-dicarboxylate (0.94 g, 1.48 mmol) in EtOAc (5.0 mL) was added LiI (600 mg, 4.45 mmol). The reaction mixture was stirred and heated at 60 °C until complete consumption of the starting material was observed (∼ 2 hours). The mixture was then cooled to room temperature and the reaction was quenched with aqueous HCl (1.0 M, 10 mL) and diluted with H₂O. The product was extracted with EtOAc (2x100 mL) and the organic phase was washed with Na₂S₂O₃ (10% aq., 40 mL), NaCl (sat. aq., 100 mL) and dried over Na₂SO₄. The solvent was removed and the resulting the product (0.90 g, 97%) was obtained as a yellow solid that was used directly in the next step. LC-MS 604.6 [M-H]⁻, 606.6 [M+H]⁺, RT 1.37 min.

To a solution of 8-(tert-butoxycarbonyl)-1-(2,4-dimethoxybenzyl)-4-hydroxy-11-(hydroxymethyl)-10-methyl-2-oxo-2,5,6,7,8,10-hexahydro-1H-pyrido[2',3':4,5]azocino[3,2-f]indole-3-carboxylic acid (0.90 g, 1.45 mmol) in DCM (10.0 mL) was added activated MnO₂ (3.2 g, 36.3 mmol). The reaction was monitored by LC/MS. After complete consumption of the starting material, the MnO₂ was filtered out and the mixture was washed with DCM. The mother liquor was concentrated to afford the product (560 mg, 63%) as an orange crystalline solid that required no further purification and was used directly in the next step. LC-MS 616.3 [M-H]⁻, RT 0.95 min.

### Steps 11-12: 4-hydroxy-10-methyl-11-((methylamino)methyl)-2-oxo-2,5,6,7,8,10-hexahydro-1H-pyrido[2',3':4,5]azocino[3,2-f]indole-3-carboxylic acid hydrochloride.

To a solution of 8-(tert-butoxycarbonyl)-1-(2,4-dimethoxybenzyl)-11-formyl-4-hydroxy-10-methyl-2-oxo-2,5,6,7,8,10-hexahydro-1H-pyrido[2',3':4,5]azocino[3,2-f]indole-3-carboxylic acid (185 mg, 0.30 mmol) in dichloroethane (3.0 mL) was added a solution of methyl amine (2.0 M THF, 300 µL, 0.60 mmol) followed by AcOH (42 µL, 0.60 mmol). After stirring at room temperature for 10 min, NaBH(OAc)₃ (133 mg, 0.63 mmol) was added. The reaction was stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt. LC-MS 631.3 [M-H]⁻, 633.3 [M+H]⁺, RT 1.04 min.

To the product obtained above was added i-Pr₃SiH (1.0 mL) followed by TFA (1.5 mL). The resultant mixture was heated at 60 °C for 2 h and monitored by LC/MS. After complete consumption of starting material, the TFA was removed under reduced pressure. Addition of an HCl solution (2.0 M Et₂O, 2.0 mL) to the oily residue led to precipitate formation. The mixture was diluted with Et₂O, the solid was filtered and then washed with Et₂O. The product HCl salt was obtained as a pale pink solid (26 mg, 21%, two steps).
¹H NMR (500 MHz, DMSO-*d*₆) δ ppm 1.64 - 1.87 (m, 1 H) 1.89 - 2.11 (m, 1 H) 2.59 - 2.69 (m, 3 H) 2.90 - 3.03 (m, 1 H) 3.05 - 3.19 (m, 1 H) 3.56 - 4.01 (m, 4 H, under solvent peak) 4.44 (s, 3 H) 6.84 (s, 1 H) 7.83 (s, 1 H) 9.34 (s, 1 H) 12.97 (br. s, 1 H) 13.92 (br. s, 1 H). LC-MS 381.1 [M-H]⁻, RT 0.39 min.

### Example 104

### 11-((ethylamino)methyl)-4-hydroxy-10-methyl-2-oxo-2,5,6,7,8,10-hexahydro-1H-pyrido[2',3':4,5]azocino[3,2-f]indole-3-carboxylic acid hydrochloride (Cpd 104)

To a solution of 8-(tert-butoxycarbonyl)-1-(2,4-dimethoxybenzyl)-11-formyl-4-hydroxy-10-methyl-2-oxo-2,5,6,7,8,10-hexahydro-1H-pyrido[2',3':4,5]azocino[3,2-f]indole-3-carboxylic acid (Example 103, steps 8-10, 185 mg, 0.30 mmol) in dichloroethane (3.0 mL) was added a solution of ethyl amine (2.0 M THF, 300 µL, 0.60 mmol) followed by AcOH (42 µL, 0.60 mmol). After stirring at room temperature for 10 min, NaBH(OAc)₃ (133 mg, 0.63 mmol) was added. The reaction was stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt. LC-MS 645.4 [M-H]⁻, 647.4 [M+H]⁺, RT 1.04 min.

To the product obtained above was added i-Pr₃SiH (1.0 mL) followed by TFA (1.5 mL). The resultant mixture was heated at 60 °C for 2 h and monitored by LC/MS. After complete consumption of starting material, the TFA was removed under reduced pressure. Addition of an HCl solution (2.0 M Et₂O, 2.0 mL) to the oily residue led to precipitate formation. The mixture was diluted with Et₂O, the solid was filtered and then washed with Et₂O. The product HCl salt was obtained as a pale pink solid (37 mg, 29%, two steps).
¹H NMR (500 MHz, DMSO-*d*₆) δ ppm 1.29 (t, *J*=7.3 Hz, 3 H) 1.64 - 1.83 (m, 2 H) 1.96 - 2.11 (m, 1 H) 3.07 (d, *J*=6.6 Hz, 3 H) 3.69 - 3.95 (m, 5 H) 4.45 (br. s., 2 H) 6.87 (s, 1 H) 7.82 (s, 1 H) 9.45 (s, 1 H) 12.97 (br. s, 1 H) 13.93 (br. s, 1 H). LC-MS 395.1 [M-H]⁻, RT 0.40 min.

### Example 105

### 4-hydroxy-11-((isopropylamino)methyl)-10-methyl-2-oxo-2,5,6,7,8,10-hexahydro-1H-pyrido[2',3':4,5]azocino[3,2-f]indole-3-carboxylic acid hydrochloride (Cpd 105)

To a solution of 8-(tert-butoxycarbonyl)-1-(2,4-dimethoxybenzyl)-11-formyl-4-hydroxy-10-methyl-2-oxo-2,5,6,7,8,10-hexahydro-1H-pyrido[2',3':4,5]azocino[3,2-f]indole-3-carboxylic acid (Example 103, steps 8-10, 185 mg, 0.30 mmol) in dichloroethane (3.0 mL) was added a solution of isopropyl amine (51 µL, 0.60 mmol) followed by AcOH (42 µL, 0.60 mmol). After stirring at room temperature for 10 min, NaBH(OAc)₃ (133 mg, 0.63 mmol) was added. The reaction was stirred at room temperature 1.5 h and monitored by LC/MS until the starting aldehyde was completely consumed. The dichloroethane was then removed and the residue was dissolved in MeOH (10 mL) and several drops of TFA to generate a homogeneous mixture that was filtered through a PTFE micron filter and purified directly by preparative HPLC to provide the product as a TFA salt. LC-MS 659.4 [M-H]⁻, 661.4 [M+H]⁺, RT 1.10 min.

To the product obtained above was added i-Pr₃SiH (1.0 mL) followed by TFA (1.5 mL). The resultant mixture was heated at 60 °C for 2 h and monitored by LC/MS. After complete consumption of starting material, the TFA was removed under reduced pressure. Addition of an HCl solution (2.0 M Et₂O, 2.0 mL) to the oily residue led to precipitate formation. The mixture was diluted with Et₂O, the solid was filtered and then washed with Et₂O. The product HCl salt was obtained as a pale pink solid (17 mg, 13%, two steps).
¹H NMR (500 MHz, DMSO-*d*₆) δ ppm 1.25 - 1.42 (m, 6 H) 1.56 - 1.81 (m, 1 H) 1.92 - 2.11 (m, 1 H) 2.87 - 3.04 (m, 1 H) 3.07 - 3.22 (m, 1 H) 3.39 - 3.97 (m, 5 H) 4.41 (s, 3 H) 6.84 (s, 1 H) 7.86 (s, 1 H) 9.23 (s, 1 H) 13.00 (s, 1 H) 13.91 (s, 1 H). LC-MS 409.1 [M-H]⁻, RT 0.41 min.

### Example 106

### 4-hydroxy-5-methyl-2-oxo-1,2,5,6-tetrahydro- [1,3] dioxolo [4',5':4,5]benzo [1,2-h]quinoline-3-carboxylic acid (Cpd 106)

### Step 1: 2-(benzo[d] [1,3]dioxol-5-yl)-N-methoxy-N-methylacetamide

To a solution of 2-(benzo[1,3]dioxol-5-yl)acetic acid (10.0 g, 55.5 mmol) in DCM (100 mL) was added CDI (9.9 g, 61 mmol). The resulting solution was stirred at room temperature for 1 hr until gas evolution ceased at which point N,O-dimethylhydroxylamine hydrochloride (5.6 g, 61 mmol) was added. After stirring for an additional 2 hrs the reaction mixture was washed with H₂O (100 mL), dried over Na₂SO₄, then filtered and concentrated to afford the desired product as a clear oil (10.9 g, 94%). The product was used in the next step without further purification.
¹H NMR (500 MHz, CHCl₃-*d*) δ ppm 3.19 (s, 3 H) 3.64 (s, 3 H) 3.68 (s, 2 H) 5.93 (s, 2 H) 6.71 - 6.77 (m, 2 H) 6.81 (d, *J*=1.26 Hz, 1 H).

### Step 2: 1-(benzo[d] [1,3]dioxol-5-yl)propan-2-one

To a solution of 2-(benzo[1,3]dioxol-5-yl)-N-methoxy-N-methylacetamide (10.9 g, 49 mmol) in THF (100 mL) cooled to 0 °C, was added methylmagnesium bromide (3M THF, 18 mL, 54 mmol). The resulting solution was stirred at 0 °C for 30 min and then allowed to warm to room temperature. After stirring at room temperature for an additional 1 hr, the reaction was quenched with saturated NH₄Cl (100 mL). The residue was extracted with Et₂O (2 x 100 mL), dried over MgSO₄, then filtered, and concentrated to afford the desired product (8.3 g, 95%).
¹H NMR (500 MHz, CHCl₃-*d*) δ ppm 2.16 (s, 3 H) 3.62 (s, 2 H) 5.96 (s, 2 H) 6.63 - 6.73 (m, 2 H) 6.78 (d, *J*=7.88 Hz, 1 H).

### Step 3: ethyl 4-(benzo[d][1,3]dioxol-5-yl)-3-methylbutanoate

To a solution of 1-(benzo[d][1,3]dioxol-5-yl)propan-2-one (8.0 g, 45 mmol) and ethyl 2-(diethoxyphosphoryl)acetate (13.5 mL, 68 mmol) in THF (100 mL), cooled to 0 °C, was added NaH (60%, 2.7 g, 68 mmol). The resulting solution was stirred at 0 °C for 1 hr and then allowed to warm to room temperature. After stirring at room temperature for 12 hrs, the reaction was quenched with saturated NH₄Cl (100 mL) and extracted with Et₂O (2 x 100 mL). The combined organic layers were dried over MgSO₄, filtered, and concentrated. The crude residue was dissolved in EtOH (80 mL) and hydrogenated under 1 atm of H₂ over PtO₂ (200 mg) for 4 hrs. The reaction mixture was then filtered through a pad of Celite and concentrated. The crude residue was purified on silica gel (9:1 Hex/EtOAc) to afford the desired product as a clear oil (9.3 g, 83%).
¹H NMR (500 MHz, CHCl₃-*d*) δ ppm 0.93 (d, *J*=6.62 Hz, 3 H) 1.22 - 1.28 (m, 3 H) 2.07 - 2.14 (m, 1 H) 2.16 - 2.25 (m, 1 H) 2.27 - 2.34 (m, 1 H) 2.38 - 2.45 (m, 1 H) 2.50 - 2.57 (m, 1 H) 4.12 (q, *J*=7.25 Hz, 2 H) 5.92 (s, 2 H) 6.60 (dd, *J*=7.88, 1.58 Hz, 1 H) 6.66 (d, *J*=1.58 Hz, 1 H) 6.72 (d, *J*=7.57 Hz, 4 H).

### Step 4: 4-(benzo[d][1,3]dioxol-5-yl)-3-methylbutanoic acid

To a solution of ethyl 4-(benzo[d][1,3]dioxol-5-yl)-3-methylbutanoate (9.3 g, 37.2 mmol) in EtOH (20 mL), NaOH (10%, 100 mL) was added. The resulting cloudy solution was heated to 70 °C for 1 hr. The resulting yellow solution was cooled to 0°C and acidified with conc. HCl (20 mL). The reaction mixture was extracted with DCM (2 x 100 mL) and the combined organic layers were dried over Na₂SO₄, filtered, and concentrated to afford the desired product (8.2 g, 99%).
¹H NMR (500 MHz, CHCl₃-*d*) δ ppm 0.97 (d, *J*=6.31 Hz, 3 H) 2.10 - 2.26 (m, 2 H) 2.32-2.40 (m, 1 H) 2.41 - 2.48 (m, 1 H) 2.51 - 2.59 (m, 1 H) 5.92 (s, 2 H) 6.60 (dd, *J*=7.88, 1.58 Hz, 1 H) 6.66 (d, *J*=1.58 Hz, 1 H) 6.72 (d, *J*=7.88 Hz, 1 H).

### Step 5: 7-methyl-7,8-dihydronaphtho[2,3-d][1,3]dioxol-5(6H)-one

To a solution of 4-(benzo[d][1,3]dioxol-5-yl)-3-methylbutanoic acid (1.1 g, 5.0 mmol) in CH₃CN (17 mL) was added K₂CO₃ (1.4 g, 10 mmol) and POCl₃ (2.3 mL, 25 mmol). The reaction mixture was heated to 55 °C for 30 min and then allowed to cool to room temperature. The crude reaction mixture was poured over ice and basified with 10% NaOH to pH 10. The aqueous phase was extracted with DCM (2 x 100 mL). The combined organic layers were dried over MgSO₄, filtered, and concentrated to afford the desired product (720 mg, 70%) as a beige solid.
¹H NMR (500 MHz, CHCl₃-*d*) δ ppm 1.12 (d, *J*=6.31 Hz, 3 H) 2.20 - 2.33 (m, 2 H) 2.56-2.63 (m, 1 H) 2.64 - 2.72 (m, 1 H) 2.83 - 2.91 (m, 1 H) 6.00 (s, 2 H) 6.65 (s, 1 H) 7.45 (s, 1 H).

### Step 6: methyl 1-(2,4-dimethoxybenzyl)-4-hydroxy-5-methyl-2-oxo-1,2,5,6-tetrahydro-[1,3]dioxolo[4',5':4,5]benzo[1,2-h]quinoline-3-carboxylate

To a solution of 7-methyl-7,8-dihydronaphtho[2,3-d][1,3]dioxol-5(6H)-one (3.0 g, 14.7 mmol) in DCM (30 mL) was added 2,4-dimethoxybenzylamine (2.4 mL, 16.2 mmol) and NEt₃ (6.1 mL, 44.1 mmol). The mixture was cooled to 0 °C and a solution of TiCl₄ (1M DCM, 8.8 mL, 8.8 mmol) was added dropwise over 30 min. The reaction mixture was allowed to warm to room temperature and stirred overnight. The mixture was then diluted with DCM (20 mL) and the reaction was quenched with saturated NaHCO₃ (20 mL). After vigorous shaking, the organic phase was separated using a PTFE phase separator, dried over Na₂SO₄, then filtered, and concentrated to afford the desired product (4.0 g, 77%) as an orange solid, which was used directly into the next step without further purification.

The crude 1-(2,4-dimethoxyphenyl)-N-(7-methyl-7,8-dihydronaphtho[2,3-d][1,3]dioxol-5(6H)-ylidene)methanamine (1.0 g, 2.8 mmol) and trimethyl methanetricarboxylate (0.8 g, 4.2 mmol) were mixed together in Ph₂O (3.0 mL). The mixture was placed onto pre-heated heat block at 220 °C and heated for 15 minutes. The heating block was then removed and the reaction mixture was allowed to cool to room temperature. The crude reaction mixture was purified directly on a silica gel column (hexanes followed by EtOAc/hexanes gradient 40-90%) to provide the product as a yellow foam (0.8 g, 61%).
¹H NMR (500 MHz, CHCl₃-*d*) δ ppm 0.85 (d, *J*=6.94 Hz, 3 H) 2.54 (dd, *J*=14.66, 1.73 Hz, 1 H) 2.96 (dd, *J*=14.19, 5.67 Hz, 1 H) 3.30 - 3.39 (m, 1 H) 3.71 (s, 3 H) 3.79 (s, 3 H) 3.97 (s, 3 H) 5.18 (br. s., 1 H) 5.34 (d, *J*=15.76 Hz, 1 H) 5.97 (dd, *J*=11.03, 1.26 Hz, 2 H) 6.40 - 6.48 (m, 2 H) 6.77 (s, 1 H) 6.95 (s, 1 H) 7.09 (d, *J*=8.51 Hz, 1 H) 13.60 (s, 1 H).

### Step 7: 4-hydroxy-5-methyl-2-oxo-1,2,5,6-tetrahydro-[1,3]dioxolo[4',5':4,5]benzo[1,2-h]quinoline-3-carboxylic acid

To a solution of methyl 1-(2,4-dimethoxybenzyl)-4-hydroxy-5-methyl-2-oxo-1,2,5,6-tetrahydro-[1,3]dioxolo[4',5':4,5]benzo[1,2-h]quinoline-3-carboxylate (313 mg, 0.7 mmol) in EtOAc (7 mL) was added lithium iodide (0.26 g, 2.0 mmol). The solution was heated at 60 °C for 1.5 hrs then cooled to room temperature and diluted with EtOAc (20 mL). The reaction mixture was washed with 1M HCl (20 mL), 10% Na₂S₂O₃ (20 mL), and brine (20 mL). The organic layer was then dried over Na₂SO₄, filtered, and concentrated. The crude residue was then dissolved in DCM (5 mL) and TFA (1 mL) was added. After stirring at room temperature for 1 hr, MeOH (5 mL) was added. The precipitate was filtered to afford the desired product (100 mg) as a bright yellow solid in 46% over two steps. ¹H NMR (500 MHz, DMSO-*d*₆) δ ppm 0.91 (d, *J*=6.94 Hz, 3 H) 2.68 - 2.76 (m, 1 H) 2.93 - 3.06 (m, 1 H) 3.17 - 3.28 (m, 1 H) 6.13 (s, 2 H) 7.04 (s, 1 H) 7.69 (s, 1 H) 12.54 (br. s, 1 H) 13.64 (br. s, 1 H) 16.04 (br. s, 1 H). LC-MS: 316.1 [M+H]⁺, RT 0.77 min.

### Example 107

### 4-hydroxy-5-methyl-2-oxo-8-(pyrrolidin-1-yl)-1,2,5,6-tetrahydrobenzo [h] quinoline-3-carboxylic acid (Cpd 107)

### Step 1: 2-(3-chlorophenyl)-N-methoxy-N-methylacetamide

Following the procedure in Example 106, Step 1, 2-(3-chlorophenyl)acetic acid (4.2 g, 24.6 mmol), CDI (4.4 g, 27 mmol), and N,O-dimethylhydroxylamine hydrochloride (2.9 g, 27 mmol) in DCM (50 mL) gave the title compound as a clear oil (4.2 g, 80%).
¹H NMR (500 MHz, CHCl₃-*d*) δ ppm 3.20 (s, 3 H) 3.64 (s, 3 H) 3.74 (s, 2 H) 7.15 - 7.32 (m, 4 H).

### Step 2: 1-(3-chlorophenyl)propan-2-one

Following the procedure in Example 106, Step 2, 2-(3-chlorophenyl)-N-methoxy-N-methylacetamide (4.2 g, 20 mmol) and methylmagnesium bromide (3M THF, 7.3 mL, 22 mmol) in THF (40 mL) gave the title compound (3.0 g, 89%).
¹H NMR (500 MHz, CHCl₃-*d*) δ ppm 2.09 (s, 3 H) 3.59 (s, 11 H) 6.99 (dt, *J*=5.99, 1.89 Hz, 1 H) 7.03 - 7.22 (m, 3 H).

### Step 3: ethyl 4-(3-chlorophenyl)-3-methylbutanoate

Following the procedure in Example 106, Step 3, 1-(3-chlorophenyl)propan-2-one (3.0 g, 17.8 mmol), ethyl 2-(diethoxyphosphoryl)acetate (5.4 mL, 26.7 mmol), and NaH (60%, 1.1 g, 26.7 mmol) in THF (20 mL) gave the desired intermediate that was then hydrogenated under 1 atm of H₂ over PtO₂ (100 mg) in EtOH (50 mL) to afford the title compound (3.1 g, 74%).
¹H NMR (500 MHz, CHCl₃-*d*) δ ppm 0.97 (d, *J*=6.62 Hz, 3 H) 1.27 - 1.30 (m, 3 H) 2.12 - 2.19 (m, 1 H) 2.22 - 2.33 (m, 2 H) 2.47 (d, *J*=7.57 Hz, 1 H) 2.61 (d, *J*=6.31 Hz, 1 H) 4.09 - 4.15 (m, 3 H) 7.03 - 7.07 (m, 1 H) 7.14 - 7.23 (m, 3 H).

### Step 4: 4-(3-chlorophenyl)-3-methylbutanoic acid

Following the procedure in Example 106, Step 4, ethyl 4-(3-chlorophenyl)-3-methylbutanoate (4.0 g, 16.6 mmol) and 10% NaOH (50 mL) in EtOH (10 mL) afforded the title compound (3.0 g, 85%).
¹H NMR (500 MHz, DMSO-*d*₆) δ ppm 0.86 (d, *J*=6.31 Hz, 3 H) 2.07 (s, 1 H) 2.08 - 2.16 (m, 1 H) 2.17 - 2.24 (m, 1 H) 2.42 - 2.48 (m, 1 H) 2.59 - 2.66 (m, 1 H) 7.13 - 7.22 (m, 1 H) 7.24-7.36 (m, 2 H).

### Step 5: 6-chloro-3-methyl-3,4-dihydronaphthalen-1(2H)-one

To a solution of 4-(3-chlorophenyl)-3-methylbutanoic acid (0.9 g, 4.2 mmol) in DCM (10 mL) was added oxalyl chloride (0.4 mL, 4.6 mmol) and DMF (2 drops). The resulting solution was stirred at room temperature for 1 hr until all gas evolution had ceased. The reaction mixture was then concentrated and the crude residue was dissolved in DCE (40 mL). To the resulting solution was added AlCl₃ (0.56 g, 4.6 mmol). The reaction mixture was stirred for 1 hr at room temperature and then poured over ice and extracted with DCM (2 x 50 mL). The combined organic phases were dried over Na₂SO₄, filtered, and concentrated to afford the title compound (0.62 g, 75%) as a 4:1 mixture of regioisomers.
¹H NMR (500 MHz, CHCl₃-*d*) δ ppm 1.15 (d, *J*=6.30 Hz, 3 H) 2.24 - 2.38 (m, 2 H) 2.61 - 2.77 (m, 2 H) 2.91 - 3.02 (m, 1 H) 7.20 - 7.29 (m, 2 H) 7.96 (d, *J*=8.20 Hz, 1 H).

### Step 6: methyl 8-chloro-1-(2,4-dimethoxybenzyl)-4-hydroxy-5-methyl-2-oxo-1,2,5,6-tetrahydrobenzo [h] quinoline-3-carboxylate

Following the procedure in Example 106, Step 6, 6-chloro-3-methyl-3,4-dihydronaphthalen-1(2H)-one (2.5 g, 12.8 mmol), 2,4-dimethoxybenzylamine (2.2 mL, 14.1 mmol), NEt₃ (5.5 mL, 38 mmol), and a solution of TiCl₄ (1M DCM, 7.7 mL, 7.7 mmol) in DCM (40 mL) were reacted to give the desired intermediate that was then reacted with trimethyl methanetricarboxylate (2.4 g, 12.8 mmol) in Ph₂O (10.0 mL) according to the procedure described in Example 106, Step 6 to afford the title compound (2.4 g, 40%).
¹H NMR (500 MHz, CHCl₃-*d*) δ ppm 0.83 (d, *J*=6.94 Hz, 3 H) 2.56 - 2.65 (m, 1 H) 2.94-3.05 (m, 1 H) 3.35 - 3.44 (m, 1 H) 3.67 (s, 3 H) 3.78 (s, 3 H) 3.96 (s, 3 H) 5.04 - 5.15 (m, 1 H) 5.27 - 5.34 (m, 1 H) 6.38 - 6.41 (m, 1 H) 6.42 - 6.47 (m, 1 H) 7.04 - 7.09 (m, 1 H) 7.10 - 7.16 (m, 1 H) 7.27 - 7.29 (m, 1 H) 7.33 - 7.40 (m, 1 H).

### Step 7: 4-hydroxy-5-methyl-2-oxo-8-(pyrrolidin-1-yl)-1,2,5,6-tetrahydrobenzo [h] quinoline-3-carboxylic acid

An oven-dried vial was evacuated then back filled with Argon. To the vial was added methyl 8-chloro-1-(2,4-dimethoxybenzyl)-4-hydroxy-5-methyl-2-oxo-1,2,5,6-tetrahydrobenzo[h]quinoline-3-carboxylate (106 mg, 0.22 mmol), 2-(2'-di-*tert-*butylphosphine)biphenylpalladium(II) acetate (10 mg, 0.022 mmol), and *t*-BuONa (63 mg, 0.66 mmol,). The vial was evacuated then back filled with Argon. To the solid mixture was added toluene (2.0 mL) and pyrrolidine (0.06 mL, 0.7 mmol,) at room temperature. The mixture was heated to 90 °C for 2 h. The reaction was quenched by 1N HCl then neutralized by saturated aq. NaHCO₃ to pH = 7. The mixture was extracted with CH₂Cl₂ (5X20 mL). The combined organic layers were concentrated and the residue was dissolved in DCM (10 mL). TFA (1 mL) was added and the mixture was stirred at room temperature for 1 hr. MeOH (5 mL) was then added to the solution and the resulting solid was filtered to afford the title compound (30 mg, 40%).
¹H NMR (500 MHz, DMSO-*d*₆) δ ppm 0.90 (d, *J*=6.94 Hz, 3 H) 1.98 (m, 4 H) 2.62 - 2.71 (m, 1 H) 2.97 - 3.06 (m, 1 H) 3.19 - 3.28 (m, 1 H) 3.35 - 3.41 (m, 4 H) 6.49 - 6.54 (m, 2 H) 7.89 - 7.96 (m, 1 H) 12.39 (br. s, 1 H) 13.56 (br. s, 1 H) 16.08 (br. s, 1 H). LC-MS: 316.1 [M+H]⁺, RT 0.77 min.

### Example 108

### 8-(dimethylamino)-4-hydroxy-5-methyl-2-oxo-1,2,5,6-tetrahydrobenzo [h] quinoline-3-carboxylic acid (Cpd 108)

The title compound was prepared from methyl 8-chloro-1-(2,4-dimethoxybenzyl)-4-hydroxy-5-methyl-2-oxo-1,2,5,6-tetrahydrobenzo[h]quinoline-3-carboxylate (Example 107, step 6) and dimethylamine solution according to the two step procedure described for Example 107 (step 7)
¹H NMR (500 MHz, DMSO-*d*₆) δ ppm 0.90 (d, *J*=6.9 Hz, 3 H) 2.67 (d, *J*=15.1 Hz, 1 H) 2.96 - 3.02 (m, 1 H) 3.03 (s, 6 H) 3.19 - 3.27 (m, 1 H) 6.57 - 6.70 (m, 2 H) 7.92 (d, *J*=9.5 Hz, 1 H) 12.42 (br. s., 1 H) 13.55 (s, 1 H). LC-MS 313.3 [M-H]⁻, 315.3 [M+H]⁺, RT 1.27 min

### Example 109

### 8-(3-(dimethylamino)pyrrolidin-1-yl)-4-hydroxy-5-methyl-2-oxo-1,2,5,6-tetrahydrobenzo[h]quinoline-3-carboxylic acid hydrochloride (Cpd 109)

The title compound was prepared from methyl 8-chloro-1-(2,4-dimethoxybenzyl)-4-hydroxy-5-methyl-2-oxo-1,2,5,6-tetrahydrobenzo[h]quinoline-3-carboxylate (Example 107, step 6) and 3-dimethylaminopyrrolidine according to the two step procedure described for Example 107 (step 7). The final product was obtained as the hydrochloride salt after treatment with HCl/Et₂O.
¹H NMR (500 MHz, MeOH-*d*₄) δ ppm 0.81 (d, *J*=7.30 Hz, 3 H) 2.16 - 2.30 (m, 2 H) 2.44-2.55 (m, 2 H) 3.25 (s, 6 H) 3.32 - 3.43 (m, 4 H) 3.92 - 4.08 (m, 2 H) 6.47 - 6.62 (m, 2 H) 7.53 - 7.68 (m, 1 H). LC-MS: 384.4 [M+H]⁺, RT 0.58 min.

### Example 110

### 8-((cis,cis)-6-amino-3-azabicyclo[3.1.0]hexan-3-yl)-4-hydroxy-5-methyl-2-oxo-1,2,5,6-tetrahydrobenzo[h]quinoline-3-carboxylic acid trifluoroacetate (Cpd 110)

The title compound was prepared from methyl 8-chloro-1-(2,4-dimethoxybenzyl)-4-hydroxy-5-methyl-2-oxo-1,2,5,6-tetrahydrobenzo[h]quinoline-3-carboxylate (Example 107, step 6) and (cis,cis)-*N*,*N-*dibenzyl-3-azabicyclo[3.1.0]hexan-6-amine according to the two step procedure described for Example 107 (step 7) with subsequent hydrogenation under 1 atm of H₂ over Pd(OH)₂/C to provide the final product as the TFA salt.
¹H NMR (500 MHz, DMSO-*d*₆) δ ppm 0.89 (d, *J*=7.25 Hz, 3 H) 2.23 - 2.30 (m, 1 H) 2.79-2.86 (m, 1 H) 3.03 - 3.12 (m, 1 H) 3.25 - 3.31 (m, 2 H) 3.32 - 3.35 (m, 4 H) 3.57 - 3.64 (m, 1 H) 6.47 - 6.54 (m, 2 H) 8.05 - 8.10 (m, 1 H) 9.61 - 9.69 (m, 1 H) 9.74 - 9.82 (m, 1 H). LC-MS: 368.2 [M+H]⁺, RT 0.57 min.

### Example 111

### 9-(1,4-diazepan-1-yl)-4-hydroxy-2-oxo-2,5,6,7-tetrahydro-1H-benzo[6,7]cyclohepta[1,2-b]pyridine-3-carboxylic acid hydrochloride (Cpd 111)

The title compound was prepared according to the two step general procedure for Buchwald coupling and deprotection from Example 6, step 4 starting from methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (Example 6, step 3) and tert-butyl 1,4-diazepane-1-carboxylate.
¹H NMR (500 MHz, *DMSO-d*₆*)* δ ppm 2.03 - 2.15 (m, 4 H), 2.22 - 2.32 (m, 2 H), 2.53 - 2.57 (m, 2 H), 3.09 - 3.18 (m, 2 H), 3.20 - 3.28 (m, 2 H), 3.54 - 3.64 (m, 2 H), 3.75 - 3.89 (m, 2 H), 6.75 - 6.87 (m, 2 H), 7.38 (d, *J=* 9.2 Hz, 1 H), 8.98 - 9.13 (br s, 2 H), 12.65 - 12.77 (br s, 1 H), 13.74 - 13.84 (br s, 1 H), 16.13 - 16.46 (br s, 1 H). LC-MS 370.2 [M+H]⁺, 368.2 [M-H]⁻, RT 0.89 min.

### Example 112

### 4-hydroxy-9-(4-methyl-1,4-diazepan-1-yl)-2-oxo-2,5,6,7-tetrahydro-1H-benzo[6,7]cyclohepta[1,2-b]pyridine-3-carboxylic acid hydrochloride (Cpd 112)

The title compound was prepared according to the two step general procedure for Buchwald coupling and deprotection from Example 6, step 4 starting from methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (Example 6, step 3) and 1-methyl-1,4-diazepane.
¹H NMR (500 MHz, *DMSO-d*₆) δ ppm 2.03 - 2.38 (m, 6 H), 2.52 - 2.58 (m, 2 H), 2.81 (d, J=4.4 Hz, 3 H), 3.09 - 3.22 (m, 2 H), 3.40 - 3.60 (m, 4H), 3.70 - 3.79 (m, 1 H), 3.86 - 3.96 (m, 1 H), 6.79 (m, 2 H), 7.39 (d, *J=* 9.2 Hz, 1 H), 10.48 - 10.61 (br s, 1 H), 12.65 - 12.76 (br s, 1 H), 13.74 - 13.82 (br s, 1 H), 16.23 - 16.44 (br s, 1 H). LC-MS 384.2 [M+H]⁺, 382.2 [M-H]⁻, RT 0.98 min.

### Example 113

### 9-((2-(dimethylamino)ethyl)amino)-4-hydroxy-2-oxo-2,5,6,7-tetrahydro-1H-benzo[6,7]cyclohepta[1,2-b]pyridine-3-carboxylic acid hydrochloride (Cpd 113)

The title compound was prepared according to the two step general procedure for Buchwald coupling and deprotection from Example 6, step 4 starting from methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (Example 6, step 3) and N,N-dimethylethane-1,2-diamine.
¹H NMR (500 MHz, DMSO-*d*₆/MeOH-*d*₄) δ ppm 2.02 - 2.11 (m, 2 H), 2.23 - 2.33 (m, 2 H), 2.75 - 2.90 (m, 8 H), 3.24 - 3.29 (m, 2 H), 3.46 - 3.51 (m, 2 H), 6.62 (s, 1 H), 6.64 *(d, J=* 8.5 Hz, 1H), 7.30 (d, *J=* 8.5 Hz, 1 H). LC-MS 358.2 [M+H]⁺, 356.2 [M-H]⁻, RT 0.94 min.

### Example 114

### 9-((4aR,7aR)-hexahydro-1H-pyrrolo[3,4-b]pyridin-6(2H)-yl)-4-hydroxy-2-oxo-2,5,6,7-tetrahydro-1H-benzo[6,7]cyclohepta[1,2-b]pyridine-3-carboxylic acid hydrochloride (Cpd 114)

The title compound was prepared according to the two step general procedure for Buchwald coupling and deprotection from Example 6, step 4 starting from methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (Example 6, step 3) and (4aR,7aR)-tert-butyl octahydro-1H-pyrrolo[3,4-b]pyridine-1-carboxylate.
¹H NMR (500 MHz, DMSO-*d*₆/MeOH-*d*₄) δ ppm 1.98 - 2.13 (m, 2 H), 2.19 - 2.33 (m, 2 H), 2.72 - 2.84 (m, 2 H), 2.88 - 2.99 (m, 2 H), 3.14 - 3.22 (m, 2 H), 3.31 - 3.42 (m, 2 H), 3.43 - 3.53 (m, 2 H), 3.59 - 3.70 (m, 2 H), 3.84 - 3.93 (m, 2 H), 6.49 - 6.63 (m, 2 H), 7.38 *(d, J=* 9.1 Hz, 1 H). LC-MS 396.4 [M+H]⁺, 394.3 [M-H]⁻, RT 0.95 min.

### Example 115

### 4-hydroxy-9-((4aR,7aR)-1-methylhexahydro-1H-pyrrolo[3,4-b]pyridin-6(2H)-yl)-2-oxo-2,5,6,7-tetrahydro-1H-benzo[6,7]cyclohepta[1,2-b]pyridine-3-carboxylic acid hydrochloride (Cpd 115)

The title compound was prepared according to the two step general procedure for Buchwald coupling and deprotection from Example 6, step 4 starting from methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (Example 6, step 3) and (4aR,7aR)-1-methyloctahydro-1H-pyrrolo[3,4-b]pyridine.
¹H NMR (500 MHz, MeOH-*d*₄) δ ppm 1.97 - 2.07 (m, 2 H), 2.51 - 2.72 (m, 2 H), 2.83 - 2.95 (m, 2 H), 3.02 - 3.14 (m, 2 H), 3.58 - 3.74 (m, 5 H), 3.77 - 3.87 (m, 2 H), 4.06 - 4.21 (m, 2 H), 4.22 - 4.35 (m, 2 H), 4.36 - 4.49 (m, 2 H), 7.36 - 7.45 (m, 2 H), 8.16 - 8.22 (m, 1 H). LC-MS 410.2 [M+H]⁺, 408.2 [M-H]⁻, RT 1.00 min.

### Example 116

### 9-(4-(dimethylamino)piperidin-1-yl)-4-hydroxy-2-oxo-2,5,6,7-tetrahydro-1H-benzo[6,7]cyclohepta[1,2-b]pyridine-3-carboxylic acid hydrochloride (Cpd 116)

The title compound was prepared according to the two step general procedure for Buchwald coupling and deprotection from Example 6, step 4 starting from methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (Example 6, step 3) and N,N-dimethylpiperidin-4-amine.
¹H NMR (500 MHz, *DMSO-d*₆*)* δ ppm 1.69 (br. s., 2 H), 2.01 - 2.19 (m, 4 H), 2.26 (br. s., 2 H), 2.64 - 2.90 (m, 10 H), 3.32 (br. s., 1 H), 4.06 (br. s., 2 H), 6.99 (br. s., 2 H), 7.38 (br. s., 1 H), 10.85 - 11.03 (br s, 1 H), 12.74 (br. s., 1 H), 13.79 (br. s., 1 H), 16.10 - 16.46 (br s, 1 H). LC-MS 398.3 [M+H]⁺, 396.3 [M-H]⁻, RT 0.90 min.

### Example 117

### 9-(3-(dimethylamino)piperidin-1-yl)-4-hydroxy-2-oxo-2,5,6,7-tetrahydro-1H-benzo[6,7]cyclohepta[1,2-b]pyridine-3-carboxylic acid hydrochloride (Cpd 117)

The title compound was prepared according to the two step general procedure for Buchwald coupling and deprotection from Example 6, step 4 starting from methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (Example 6, step 3) and N,N-dimethylpiperidin-3-amine.
¹H NMR (500 MHz, *DMSO-d*₆) δ ppm 1.51 - 1.63 (m, 1 H), 1.64 - 1.75 (m, 1 H), 1.82 - 1.91 (m, 1 H), 2.04 - 2.17 (m, 4 H), 2.20 - 2.34 (m, 2 H), 2.53 - 2.60 (m, 2H), 2.74 - 2.87 (m, 6 H), 3.02 - 3.12 (m, 1 H), 3.23 - 3.31 (m, 1 H), 3.80 - 3.89 (m, 1 H), 4.15 - 4.25 (m, 1 H), 6.97 - 7.08 (m, 2 H), 7.40 (d, *J=* 8.5 Hz, 1 H), 10.51 - 10.69 (br s, 1 H), 12.69 - 12.80 (br s, 1 H), 13.73 - 13.85 (br s, 1 H), 16.17 - 16.42 (br s, 1 H). LC-MS 398.3 [M+H]⁺, 396.3 [M-H]⁻, RT 0.99 min.

### Example 118

### 4-hydroxy-2-oxo-9-(piperidin-4-ylamino)-2,5,6,7-tetrahydro-1H-benzo[6,7]cyclohepta[1,2-b]pyridine-3-carboxylic acid hydrochloride (Cpd 118)

The title compound was prepared according to the two step general procedure for Buchwald coupling and deprotection from Example 6, step 4 starting from methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (Example 6, step 3) and tert-butyl 4-aminopiperidine-1-carboxylate.
¹H NMR ¹H NMR (500 MHz, *DMSO-d*₆) δ ppm 1.65 (br. s., 2 H), 2.05 (br. s., 4 H), 2.27 (br. s., 2 H), 2.98 (br. s., 3 H), 3.29 (br. s., 4 H), 6.61 (br. s., 2 H), 7.11 - 7.40 (m, 1 H), 8.32 - 8.66 (m, 1 H), 9.12 (br. s., 2 H), 12.62 (br. s., 1 H), 13.75 (br. s., 1 H), 16.03 - 16.60 (br s, 1 H). LC-MS 370.2 [M+H]⁺, 368.2 [M-H]⁻, RT 0.96 min.

### Example 119

### 4-hydroxy-2-oxo-9-(piperazin-1-yl)-2,5,6,7-tetrahydro-1H-benzo[6,7]cyclohepta[1,2-b]pyridine-3-carboxylic acid hydrochloride (Cpd 119)

The title compound was prepared according to the two step general procedure for Buchwald coupling and deprotection from Example 6, step 4 starting from methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (Example 6, step 3) and tert-butyl piperazine-1-carboxylate.
¹H NMR (500 MHz, *DMSO-d*₆) δ ppm 2.03 - 2.15 (m, 2 H), 2.19 - 2.31 (m, 2 H), 2.53 - 2.60 (m, 2 H), 3.17 - 3.26 (m, 4 H), 3.45 - 3.60 (m, 4 H), 6.96 - 7.05 (m, 2 H), 7.42 (d, *J= 9.1* Hz, 1 H), 9.14 - 9.28 (br s, 2 H), 12.72 - 12.82 (br s, 1 H), 13.76 - 13.86 (br s, 1 H), 16.20 - 16.43 (br s, 1 H). LC-MS 356.2 [M+H]⁺, 354.3 [M-H]⁻, RT 0.49 min.

### Example 120

### 4-hydroxy-9-(4-methylpiperazin-1-yl)-2-oxo-2,5,6,7-tetrahydro-1H-benzo[6,7]cyclohepta[1,2-b]pyridine-3-carboxylic acid hydrochloride (Cpd 120)

The title compound was prepared according to the two step general procedure for Buchwald coupling and deprotection from Example 6, step 4 starting from methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (Example 6, step 3) and 1-methylpiperazine.
¹H NMR (500 MHz, *DMSO-d*₆) δ ppm 2.03 - 2.14 (m, 2 H), 2.20 - 2.31 (m, 2 H), 2.53 -2.59 (m, 2 H), 2.82 (s, 3 H), 3.08 - 3.25 (m, 4 H), 3.44 - 3.56 (m, 2 H), 3.98 - 4.08 (m, 2 H), 6.95 - 7.07 (m, 2 H), 7.43 (d, *J=* 8.8 Hz, 1 H), 10.66 - 10.83 (br s, 1 H), 12.74 - 12.84 (br s, 1 H), 13.75 - 13.85 (br s, 1 H), 16.13 - 16.46 (br s, 1 H). LC-MS 370.3 [M+H]⁺, 368.3 [M-H]⁻, RT 0.49 min.

### Example 121

### 4-hydroxy-2-oxo-9-(2,6-diazaspiro[3.4]octan-6-yl)-2,5,6,7-tetrahydro-1H-benzo[6,7]cyclohepta[1,2-b]pyridine-3-carboxylic acid hydrochloride (Cpd 121)

The title compound was prepared according to the two step general procedure for Buchwald coupling and deprotection from Example 6, step 4 starting from methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (Example 6, step 3) and tert-butyl 2,6-diazaspiro[3.4]octane-2-carboxylate.
¹H NMR (500 MHz, *DMSO-d*₆) δ ppm 2.03 - 2.12 (m, 2 H), 2.21 - 2.34 (m, 4 H), 3.33 - 3.39 (m, 2 H), 3.57 (br s, 2 H), 3.88 - 3.96 (m, 2 H), 3.97 - 4.06 (m, 2 H), 6.48 - 6.54 (m, 2 H), 7.36 (J= 9.2 Hz, 1 H), 9.01 - 9.14 (br s, 1 H), 9.22 - 9.36 (br s, 1 H), 12.65 - 12.72 (br s, 1 H), 13.71 - 13.82 (br s, 1 H), 16.13 - 16.46 (br s, 1 H). LC-MS 382.3 [M+H]⁺, 380.2 [M-H]⁻, RT 0.88 min.

### Example 122

### 4-hydroxy-2-oxo-9-(2,7-diazaspiro[4.4]nonan-2-yl)-2,5,6,7-tetrahydro-1H-benzo[6,7]cyclohepta[1,2-b]pyridine-3-carboxylic acid hydrochloride (Cpd 122)

The title compound was prepared according to the two step general procedure for Buchwald coupling and deprotection from Example 6, step 4 starting from methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (Example 6, step 3) and tert-butyl 2,7-diazaspiro[4.4]nonane-2-carboxylate.
¹H NMR (500 MHz, *DMSO-d*₆) δ ppm 1.93 - 2.12 (m, 7 H), 2.22 - 2.32 (m, 2 H), 3.14 - 3.19 (m, 2 H), 3.27 - 3.35 (m, 3 H), 3.40 - 3.44 (m, 4 H), 6.49 - 6.53 (m, 2 H), 7.36 (d, *J= 9.1* Hz, 1 H), 9.00 - 9.13 (br s, 2 H), 12.67 - 12.72 (br s, 1 H), 13.75 - 13.79 (br s, 1 H), 16.24 - 16.41 (br s, 1 H). LC-MS 396.4 [M+H]⁺, 394.3 [M-H]⁻, RT 0.89 min.

### Example 123

### 4-hydroxy-9-(7-methyl-2,7-diazaspiro[4.4]nonan-2-yl)-2-oxo-2,5,6,7-tetrahydro-1H-benzo[6,7]cyclohepta[1,2-b]pyridine-3-carboxylic acid hydrochloride (Cpd 123)

The title compound was prepared according to the two step general procedure for Buchwald coupling and deprotection from Example 6, step 4 starting from methyl 9-chloro-1-(2,4-dimethoxybenzyl)-4-hydroxy-2-oxo-2,5,6,7-tetrahydro-1H-benzo[6,7]cyclohepta[1,2-b]pyridine-3-carboxylate (Example 6, step 3) and 2-methyl-2,7-diazaspiro[4.4]nonane.
¹H NMR (500 MHz, MeOH-*d*₄) δ ppm 2.10 - 2.33 (m, 6 H), 2.37 - 2.45 (m, 2 H), 2.56 - 2.63 (m, 2 H), 3.00 (s, 3 H), 3.16 - 3.25 (m, 1 H), 3.38 - 3.42 (m, 1 H), 3.48 - 3.55 (m, 3 H), 3.69-3.74 (m, 1 H), 3.77 - 3.84 (m, 1 H), 6.57 *(d, J=* 2.5 Hz, 1 H), 6.61 (dd, *J* = 8.5, 2.5 Hz, 1 H), 7.36 (d, *J =* 8.5 Hz, 1 H), 7.84 (s, 1 H). LC-MS 410.4 [M+H]⁺, 408.3 [M-H]⁻, RT 0.90 min.

### Example 124

### 9-((cis,cis)-6-amino-3-azabicyclo[3.1.0]hexan-3-yl)-10,11-difluoro-4-hydroxy-2-oxo-2,5,6,7-tetrahydro-1H-benzo[6,7]cyclohepta[1,2-b]pyridine-3-carboxylic acid hydrochloride (Cpd 124)

### Step 1: 2,3,4-trifluoro-6,7,8,9-tetrahydro-5H-benzo[7]annulen-5-one

The title compound was prepared from 6-bromo-2,3,4-trifluorobenzaldehyde according to Example 2, Steps 1-5.
¹H NMR (500 MHz, CHCl₃-*d*) 1.83 - 1.91 (m, 4 H) 2.72 (m, 2 H) 2.81 (t, *J*=6.5 Hz, 2 H) 6.82 (m, 1 H), RT 1.20 min.

### Step 2: 2-((cis,cis)-6-(dibenzylamino)-3-azabicyclo[3.1.0]hexan-3-yl)-3,4-difluoro-6,7,8,9-tetrahydro-5H-benzo [7] annulen-5-one

The material from Step 1 (214 mg, 1 mmol) was dissolved in DMSO (1 mL) with N,N-dibenzyl-3-azabicyclo[3.1.0]hexan-6-amine (1.1 mmol) and K₂CO₃ (276 mg, 2 mmol). The mixture was stirred at 100 °C for 2 h, then partitioned in water and EtOAc. The organic layer was concentrated and chromatographed on silica gel (0-20% EtOAc in hexanes) to yield the title compound (130 mg, 27%).
¹H NMR (500 MHz, DMSO-*d*₆) δ ppm 1.67 - 1.78 (m, 4 H) 2.58 (m, 2 H) 2.65 (m, 1 H) 2.77 (m, 2 H) 2.94 (m, 1 H) 3.21 (br. s., 4 H) 3.31 (m, 1 H) 3.35 (m, 2 H) 3.53 (m, 2 H) 6.29 (d, *J*=8.0 Hz, 1 H), 7.29 - 7.52 (m, 10 H). LC-MS 473.5 [M+H]⁺, RT 1.41 min.

### Step 3: methyl 9-((cis,cis)-6-(dibenzylamino)-3-azabicyclo[3.1.0]hexan-3-yl)-1-(2,4-dimethoxybenzyl)-10,11-difluoro-4-hydroxy-2-oxo-2,5,6,7-tetrahydro-1H-benzo[6,7]cyclohepta[1,2-b]pyridine-3-carboxylate

The title compound was prepared from the intermediate of Step 2 according to Example 6, Steps 2-3. LC-MS 748.4 [M+H]⁺, RT 1.84 min.

### Step 4: 9-((cis,cis)-6-amino-3-azabicyclo[3.1.0]hexan-3-yl)-10,11-difluoro-4-hydroxy-2-oxo-2,5,6,7-tetrahydro-1H-benzo[6,7]cyclohepta[1,2-b]pyridine-3-carboxylic acid hydrochloride

The title compound was prepared from the intermediate of Step 3 according to the procedure of Example 9.
¹H NMR (500 MHz, DMSO-*d*₆) δ ppm 1.74 (m, 1 H) 2.03 (m, 2 H) 2.16 (m, 2 H) 2.35 (m, 1 H) 2.64 (m, 2 H) 2.85 (m, 1 H) 3.72 - 3.85 (m, 4 H) 6.60 (d, *J*=8.0 Hz, 1 H) 8.43 - 8.50 (m, 3 H) 12.88 (br. s., 1 H) 13.82 (br. s., 1 H) 16.19 (br. s., 1 H). LC-MS 402.1 [M-H]⁻, 404.0 [M+H]⁺, RT 0.88 min.

### Example 125

### 9-((cis,cis)-6-amino-3-azabicyclo[3.1.0]hexan-3-yl)-11-fluoro-4-hydroxy-2-oxo-2,5,6,7-tetrahydro-1H-benzo[6,7]cyclohepta[1,2-b]pyridine-3-carboxylic acid hydrochloride (Cpd 125)

### Step 1: methyl 9-bromo-1-(2,4-dimethoxybenzyl)-11-fluoro-4-hydroxy-2-oxo-2,5,6,7-tetrahydro-1H-benzo[6,7]cyclohepta[1,2-b]pyridine-3-carboxylate

The title compound was prepared from 3-bromo-5-fluorobenzaldehyde according to Example 6, Steps 1-3.
¹H NMR (500 MHz, CHCl₃-*d*) δ ppm 1.49 (td, *J*=13.7, 7.0 Hz, 1 H) 1.81 - 2.02 (m, 3 H) 2.28 (dd, *J*=13.2, 6.4 Hz, 1 H) 2.99 (dd, *J*=14.1, 5.6 Hz, 1 H) 3.46 (s, 3 H) 3.74 (s, 3 H) 4.04 (s, 3 H) 5.33 (s, 2H) 6.16 (d, *J*=2.4 Hz, 1 H) 6.28 (dd, *J*=8.4, 2.4 Hz, 1 H) 6.84 (d, *J*=8.5 Hz, 1 H) 7.07 (d, *J*=1.8 Hz, 1 H) 7.29 (dd, *J*=9.1, 1.8 Hz, 1 H). LC-MS 530.0, 532.0 [M-H]⁻, 532.0, 534.0 [M+H]⁺, RT 1.60 min.

### Step 2: 9-((cis,cis)-6-amino-3-azabicyclo[3.1.0]hexan-3-yl)-11-fluoro-4-hydroxy-2-oxo-2,5,6,7-tetrahydro-1H-benzo[6,7]cyclohepta[1,2-b]pyridine-3-carboxylic acid hydrochloride

The title compound was prepared from the intermediate of Step 1 according to Example 6, step 4 and the procedure of Example 9.
¹H NMR (500 MHz, DMSO-*d*₆) δ ppm 1.72 (m, 1 H) 2.03 (m, 2 H) 2.17 (m, 2 H) 2.38 (m, 1 H) 2.66 (m, 2 H) 2.86 (m, 1 H) 3.38 (dt, *J*=10.3, 2.2 Hz, 2 H) 3.63 (d, *J*=10.3 Hz, 2 H) 6.41 - 6.46 (m, 2 H) 8.41 (br. s., 3 H) 12.78 (br. s., 1 H) 13.84 (br. s., 1 H) 16.28 (br. s., 1 H). LC-MS 384.3 [M-H]⁻, 386.3 [M+H]⁺, RT 0.79 min.

### Example 126

### 4-hydroxy-2-oxo-10-(pyrrolidin-1-yl)-1,2,5,6,7,8-hexahydrobenzo [7,8] cycloocta[1,2-b]pyridine-3-carboxylic acid (Cpd 126)

### Step 1. methyl 10-chloro-1-(2,4-dimethoxybenzyl)-4-hydroxy-2-oxo-1,2,5,6,7,8-hexahydrobenzo[7,8]cycloocta[1,2-b]pyridine-3-carboxylate

To a stirred solution of 2-bromo-7,8,9,10-tetrahydrobenzo[8]annulen-5(6H)-one (0.37 g, 1.5 mmol) (prepared by the procedure disclosed in US Patent US20090202478) in CH₂Cl₂ (6 mL) was added 2,4-dimethoxybenzylamine (0.28 g, 1.6 mmol, 1.1 eq) and Et₃N (0.5 mL, 3.9 mmol, 2.6 eq) sequentially at 0°C. Then a solution of TiCl₄ (1.0M in CH₂Cl₂, 1.0 mL, 1.0 mmol, 0.67 eq) was added to the mixture via syringe pump over 30 min at 0°C. The reaction mixture was allowed to warm to room temperature and stirred overnight. The reaction was quenched by saturated aq. NaHCO₃ solution then extracted with Et₂O. The combined organic layers were dried over Na₂SO₄ then concentrated to give a yellow oil (∼ 0.6 g) which was applied to next step without further purification.

To melted trimethyl methanetricarboxylate (0.51 g, 2.7 mmol, 1.8 eq) at 190 °C was added dropwise a solution of the crude product obtained above (-0.6 g, ca. 1.5 mmol) in Ph₂O (3 mL) was added trimethyl methanetricarboxylate (4.7 g, 24.7 mmol, 1.8 eq). The reaction mixture was continuously heated at 190 °C for 1 hr during which time a short-path distillation apparatus was attached to remove the solvent. The heat was removed after methanol distillation ceased. The mixture was allowed to cool to room temperature then purified by flash column chromatography (0-100% EtOAc in hexane) to give the title compound (0.62 g, 1.2 mmol, 78%, 2 steps).
¹H NMR (500 MHz, *MeOD*) δ ppm 1.31-1.45 (m, 3 H), 1.84-1.96 (m, 3 H), 2.23-2.34 (m, 1 H), 2.88-2.93 (m, 1 H), 3.52 (s, 3 H), 3.75 (s, 3 H), 4.00 (s, 3 H), 4.85 (s, 2 H), 6.31 (d, *J=* 3 Hz, 1 H), 6.35 (dd, *J=* 9, 3 Hz, 1 H), 6.63 (d, *J=* 9 Hz, 1 H), 7.10 (d, *J=* 8 Hz, 1 H), 7.39-7.42 (m, 2 H). LC-MS 582.2/530.2 [M+H]⁺, RT 0.99 min.

### Step 2. 1-(2,4-dimethoxybenzyl)-4-hydroxy-2-oxo-10-(pyrrolidin-1-yl)-1,2,5,6,7,8-hexahydrobenzo[7,8]cycloocta[1,2-b]pyridine-3-carboxylic acid

The intermediate from step 1 (0.10 g, 0.19 mmol) was mixed with pyrrolidine (0.027 g, 0.38 mmol, 2.0 eq), 2-(2'-di-*tert*-butylphosphine)biphenylpalladium(II) acetate (4 mg, 5 mol%), and *t*-BuONa (0.11 g, 1.14 mmol, 6.0 eq) in toluene (1 mL). The mixture was degassed by purging the reaction flask with vacuum and then back filling with Argon (3x). The reaction mixture was heated at 80 °C with vigorous magnetic stirring for 2 hr. After cooling to room temperature, the mixture was filtered and the resulting solid was washed with EtOAc. The filtrate was concentrated and purified by chromatography on silica gel (0-100% EtOAc in dichloromethane) to afford the product as a yellow solid (0.09 g, 94%). LC-MS 505.4 [M+H]⁺, RT 1.05 min.

### Step 3. 4-hydroxy-2-oxo-10-(pyrrolidin-1-yl)-1,2,5,6,7,8-hexahydrobenzo[7,8]cycloocta[1,2-b]pyridine-3-carboxylic acid

To a suspension of the above intermediate (0.09 g, 0.18 mmol) in CH₂Cl₂ (1.0 mL) was added TFA (1.0 mL) at room temperature. The mixture was stirred at room temperature for 3 h. The reaction was monitored by LC-MS. After completion, the solvent was removed under reduced pressure. The residue was dissolved in CH₂Cl₂ (1.5 mL), then HCl (2.0 mL, 2.0M in ether) was added. The mixture was filtered and washed with diethyl ether to give the desired product (0.055 g, 0.14 mmol, 79%) as a light yellow solid.
¹H NMR (500 MHz, DMSO-*d*₆) δ ppm 1.31-1.41 (m, 2 H), 1.48-1.53 (m, 1 H), 1.91-2.16 (m, 6 H), 2.36-2.37 (m, 1 H), 2.64-2.85 (m, 2 H), 3.28-3.31, (m, 4 H). 6.50-6.51 (m, 2 H), 7.18 (d, *J*= 8 Hz, 1 H), 12.65 (s, 1 H), 13.86 (s, 1 H). LC-MS 355.3 [M+H]⁺, RT 0.90 min.

### Example 127

### 10-((cis,cis)-6-amino-3-azabicyclo[3.1.0]hexan-3-yl)-4-hydroxy-2-oxo-1,2,5,6,7,8-hexahydrobenzo[7,8]cycloocta[1,2-b]pyridine-3-carboxylic acid hydrochloride (Cpd 127)

The title compound was prepared starting from methyl 10-chloro-1-(2,4-dimethoxybenzyl)-4-hydroxy-2-oxo-1,2,5,6,7,8-hexahydrobenzo[7,8]cycloocta[1,2-b]pyridine-3-carboxylate (Example 126, step 1) and (cis,cis)-N,N-dibenzyl-3-azabicyclo[3.1.0]hexan-6-amine according to the two step procedure described in Example 126, steps 2-3 and the deprotection route of Example 9.
¹H NMR (500 MHz, *MeOD*) δ ppm 1.46-1.48 (m, 2 H), 1.59-1.64 (m, 1 H), 1.96-2.00 (m, 1 H), 2.13-2.19 (m, 2 H), 2.23 (s, 1H), 2.26-2.28 (m, 1 H), 2.55 (s, 1H), 2.82-2.87 (m, 1 H), 2.95-2.99 (m, 1 H), 3.39-3.41 (m, 2 H), 3.79, (t, *J=* 9 Hz, 2 H). 6.61-6.64 (m, 2 H), 7.22 (d, *J* = 8 Hz, 1 H). LC-MS 382.4 [M+H]⁺, RT 0.54 min.

### Example 128

### 4-hydroxy-2-oxo-10-(propylamino)-1,2,5,6,7,8-hexahydrobenzo [7,8] cycloocta[1,2-b]pyridine-3-carboxylic acid (Cpd 128)

The title compound was prepared starting from methyl 10-chloro-1-(2,4-dimethoxybenzyl)-4-hydroxy-2-oxo-1,2,5,6,7,8-hexahydrobenzo[7,8]cycloocta[1,2-b]pyridine-3-carboxylate (Example 126, step 1) and propylamine and according to the two step procedure described in Example 126, steps 2-3.
¹H NMR (500 MHz, *MeOD*) δ ppm 1.09 (t, *J=* 7 Hz, 3 H), 1.47-1.53 (m, 2 H), 1.56-1.61 (m, 1 H), 1.78-1.83 (m, 2 H), 1.99-2.02 (m, 1 H), 2.18-2.22 (m, 1 H), 2.39-2.44 (m, 1 H), 2.96-3.04 (m, 2 H), 3.39-3.42 (m, 2 H), 7.38 (d, *J=* 8 Hz, 1 H), 7.43 (s, 1 H), 7.58 (d, *J=* 8 Hz, 1 H). LC-MS 343.3 [M+H]⁺, RT 1.33 min.

### Example 129

### 10-(ethylamino)-4-hydroxy-2-oxo-1,2,5,6,7,8-hexahydrobenzo [7,8] cycloocta[1,2-b]pyridine-3-carboxylic acid (Cpd 129)

The title compound was prepared using methyl 10-chloro-1-(2,4-dimethoxybenzyl)-4-hydroxy-2-oxo-1,2,5,6,7,8-hexahydrobenzo[7,8]cycloocta[1,2-b]pyridine-3-carboxylate (Example 126, step 1) and ethylamine, according to the two step procedure described in Example 126, steps 2-3.
¹H NMR (500 MHz, *MeOD*) δ ppm 1.50 (t, *J=* 7 Hz, 3 H), 1.55-1.61 (m, 3 H), 1.98-2.06 (m, 1 H), 2.19-2.22 (m, 1 H), 2.40-2.45 (m, 1 H), 2.96-3.04 (m, 2 H), 3.49-3.56 (m, 2 H), 7.40 (d, *J=* 8 Hz, 1 H), 7.46 (s, 1 H), 7.60 (d, *J=* 8 Hz, 1 H). LC-MS 329.3 [M+H]⁺, RT 0.79 min.

### Example 130

### 1,9-dimethyl-2-oxo-1,2,5,9-tetrahydropyrido [3',2':6,7] cyclohepta[1,2-f]indole-3-carboxylic acid (Cpd 130)

### Step 1: N-(1-methyl-6,7-dihydrocyclohepta[f]indol-5(1H)-ylidene)methanamine

To a solution of 1-methyl-6,7-dihydrocyclohepta[f]indol-5(1H)-one (Example 16, step 7, 0.218 g, 1.03 mmol) in DCM (3 mL) was added MeNH₂ solution (2M THF, 2.00 mL, 4.00 mmol). The mixture was cooled to 0 °C, then a solution of TiCl₄ (1M DCM, 0.70 mL, 0.70 mmol) was added dropwise via syringe pump over 30 min. The reaction mixture was allowed to warm to room temperature and stirred overnight. The mixture was diluted with DCM (10 mL) and then the reaction was quenched with NaHCO₃ (aqueous saturated 5 mL). After vigorous shaking, the organic phase was separated using a PTFE phase separator, and dried over Na₂SO₄. The solvent was removed and the resulting crude product was obtained (-0.23 g, quant) as a yellow oil, which was used directly in the next step without further purification.

### Step 2: Methyl 1,9-dimethyl-2-oxo-1,2,5,9-tetrahydropyrido[3',2':6,7]cyclohepta[1,2-f]indole-3-carboxylate

The product N-(1-methyl-6,7-dihydrocyclohepta[f]indol-5(1H)-ylidene)methanamine (*ca.* 0.52 mmol) obtained above and dimethyl 2-(methoxymethylene)malonate (0.16 g, 0.92 mmol) were mixed together in Ph₂O (1.0 mL). With stirring, the mixture was placed onto a pre-heated heat block at 200 °C and heated for 10 min after the initial bubbling of MeOH was observed (occurs at ∼160 °C internal reaction temperature). The reaction mixture was cooled to room temperature and was purified directly on a silica gel column (hexanes followed by EtOAc/hexanes gradient 40-100%) to provide the product as a yellow foam (0.105 g, 60% 2 steps).
¹H NMR (500 MHz, CHCl₃-*d*) δ ppm 2.59 (ddd, *J*=14.1, 5.6, 1.9 Hz, 1 H) 2.86 (dd, *J*=14.1, 8.0 Hz, 1 H) 3.45 (s, 3 H) 3.87 (s, 3 H) 3.95 (s, 3 H) 6.35 (ddd, *J*=10.1, 8.0, 5.6 Hz, 1 H) 6.58 (dd, *J*=3.2, 0.6 Hz, 1 H) 6.75 (dd, *J*=10.1*,* 1.9 Hz, 1 H) 7.22 (d, *J*=3.2 Hz, 1 H) 7.32 (s, 1 H) 7.72 (s, 1 H) 8.11 (s, 1 H). LC-MS 335.2 [M+H]⁺, RT 1.19 min.

### Step 3: 1,9-dimethyl-2-oxo-1,2,5,9-tetrahydropyrido[3',2':6,7]cyclohepta[1,2-f]indole-3-carboxylic acid

To a suspension of methyl 1,9-dimethyl-2-oxo-1,2,5,9-tetrahydropyrido[3',2':6,7]cyclohepta[1,2-f]indole-3-carboxylate (48.5 mg, 0.15 mmol) in THF (0.6 mL) was added LiOH solution (1M H₂O, 0.3 mL, 0.3 mmol). The reaction mixture was stirred at room temperature for 1.5 h and was acidified with the addition of 1M HCl to pH∼2. The product was extracted with DCM (2x5 mL), the combined organics were dried over Na₂SO₄ and the DCM was removed to afford the product (40.7 mg, 88%) as a yellow solid.
¹H NMR (500 MHz, CHCl₃-*d*) δ ppm 2.64 (ddd, *J*=14.0*,* 5.7, 2.0 Hz, 1 H) 2.97 (dd, *J*=14.0, 7.9 Hz, 1 H) 3.56 (s, 3 H) 3.89 (s, 3 H) 6.35 (ddd, *J*=9.8, 7.9, 5.7 Hz, 1 H) 6.60 (dd, *J*=3.2, 0.6 Hz, 1 H) 6.77 (dd, *J*=9.8, 2.0 Hz, 1 H) 7.25 (d, *J*=3.2 Hz, 1 H) 7.36 (s, 1 H) 7.74 (s, 1 H) 8.40 (s, 1 H) 14.81 (br. s., 1 H). LC-MS 321.3 [M+H]⁺, RT 1.17 min.

### Example 131

### 4-hydroxy-1,9-dimethyl-2-oxo-1,2,5,9-tetrahydropyrido[3',2':6,7] cyclohepta[1,2-f]indole-3-carboxylic acid (Cpd 131)

### Step 1: methyl 4-hydroxy-1,9-dimethyl-2-oxo-1,2,5,9-tetrahydropyrido [3',2':6,7] cyclohepta[1,2-f] indole-3-carboxylate

Crude N-(1-methyl-6,7-dihydrocyclohepta[f]indol-5(1H)-ylidene)methanamine (Example 130, step 1, *ca.* 0.52 mmol) and trimethyl methanetricarboxylate (0.17 g, 0.89 mmol) were mixed together in Ph₂O (1.0 mL). With stirring, the mixture was placed onto a pre-heated heat block at 230 °C and heated for 10 min after the initial bubbling of MeOH was observed (occurs at ∼160 °C internal reaction temperature). The reaction mixture was cooled to room temperature and was purified directly on a silica gel column (hexanes followed by EtOAc/hexanes gradient 40-100%) to provide the product as yellow foam (0.120 g, 66% 2 steps).
¹H NMR (500 MHz, CHCl₃-*d*) δ ppm 2.10 (ddd, *J*=14.5, 5.7, 1.9 Hz, 1 H) 3.29 (s, 3 H) 3.53 (ddd, *J*=14.5, 7.9, 0.9 Hz, 1 H) 3.87 (s, 3 H) 4.02 (s, 3 H) 6.37 (ddd, *J*=9.9, 7.9, 5.7 Hz, 1 H) 6.57 (dd, *J*=3.2, 0.9 Hz, 1 H) 6.78 (dd, *J*=9.9, 1.9 Hz, 1 H) 7.21 (d, *J*=3.2 Hz, 1 H) 7.32 (s, 1 H) 7.71 (s, 1 H). LC-MS 349.3 [M-H]⁻, 351.3 [M+H]⁺, RT 1.32 min.

### Step 2: 4-hydroxy-1,9-dimethyl-2-oxo-1,2,5,9-tetrahydropyrido[3',2':6,7]cyclohepta[1,2-f]indole-3-carboxylic acid

To a suspension of methyl 4-hydroxy-1,9-dimethyl-2-oxo-1,2,5,9-tetrahydropyrido[3',2':6,7]cyclohepta[1,2-f]indole-3-carboxylate (58.0 mg, 0.17 mmol) in EtOAc (1.0 mL) was added LiI (60 mg, 0.45 mmol). The reaction mixture was stirred and heated at 60 °C for 1 h until complete consumption of starting material was observed. The mixture was cooled to room temperature, then acidified with aqueous HCl (1M, 1.0 mL) and diluted with H₂O. The product was extracted with EtOAc (3x5 mL). The organic phase was washed with Na₂S₂O₃ (10% aq, 4 mL), NaCl (aqueous saturated, 5 mL) and dried over Na₂SO₄. The solvent was removed and the resulting product was obtained as a yellow solid (55.6 g, quant).
¹H NMR (500 MHz, CHCl₃-*d*) δ ppm 2.15 (ddd, *J*=14.2, 5.7, 1.9 Hz, 1 H) 3.40 (s, 3 H) 3.55 (ddd, *J*=14.2, 8.0, 0.8 Hz, 1 H) 3.88 (s, 3 H) 6.36 (ddd, *J*=9.9, 8.0, 5.7 Hz, 1 H) 6.59 (dd, *J*=3.2, 0.8 Hz, 1 H) 6.80 (dd, *J*=9.9, 1.9 Hz, 1 H) 7.24 (d, *J*=3.2 Hz, 1 H) 7.35 (s, 1 H) 7.73 (s, 1 H) 13.85 (s, 1 H) 15.89 (s, 1 H). LC-MS 337.3 [M+H]⁺, RT 1.34 min.

### Example 132

### 1-ethyl-4-hydroxy-9-methyl-2-oxo-1,2,5,9-tetrahydropyrido [3',2': 6,7] cyclohepta[1,2-f]indole-3-carboxylic acid (Cpd 132)

### Steps 1-2: methyl 1-ethyl-4-hydroxy-9-methyl-2-oxo-1,2,5,9-tetrahydropyrido [3',2':6,7] cyclohepta[1,2-f] indole-3-carboxylate

To a solution of 1-methyl-6,7-dihydrocyclohepta[f]indol-5(1H)-one (Example 16, step 7, 0.218 g, 1.03 mmol) in DCM (3 mL) was added EtNH₂ solution (2M THF, 2.00 mL, 4.00 mmol). The mixture was cooled to 0 °C, then a solution of TiCl₄ (1M DCM, 0.70 mL, 0.70 mmol) was added dropwise via syringe pump over 30 min. The reaction was allowed to warm to room temperature and stirred overnight. The mixture was diluted with DCM (10 mL) and then the reaction was quenched with NaHCO₃ (aqueous saturated, 5 mL). After vigorous shaking the organic phase was separated using a PTFE phase separator and dried over Na₂SO₄. The solvent was removed and the resulting crude product was obtained (-0.25 g, quant) as a yellow oil, which was used directly in the next step without further purification.

Crude N-(1-methyl-6,7-dihydrocyclohepta[f]indol-5(1H)-ylidene)ethanamine (*ca*. 0.52 mmol) obtained above and trimethyl methanetricarboxylate (0.17 g, 0.89 mmol) were mixed together in Ph₂O (1.0 mL). With stirring, the mixture was placed onto a pre-heated heat block at 230 °C and heated for 10 min after the initial bubbling of MeOH was observed (occurs at ∼160 °C internal reaction temperature). The reaction mixture was cooled to room temperature and was purified directly on a silica gel column (hexanes followed by EtOAc/hexanes 40-100% gradient) to give the product as a yellow foam (0.127 g, 67% 2 steps).
¹H NMR (500 MHz, CHCl₃-*d*) δ ppm 1.01 (t, *J*=6.9 Hz, 3 H) 2.10 (ddd, *J*=14.2, 5.7, 2.2 Hz, 1 H) 3.50 (ddd, J=14.2, 8.0, 0.8 Hz, 1 H) 3.83 - 3.89 (m, 1 H) 3.85 (s, 3 H) 4.02 (s, 3 H) 4.06 - 4.16 (m, 1 H) 6.39 (ddd, *J*=10.1, 8.0, 5.7 Hz, 1 H) 6.57 (dd, *J*=3.2, 0.8 Hz, 1 H) 6.77 (dd, *J*=10.1, 2.2 Hz, 1 H) 7.21 (d, *J*=3.2 Hz, 1 H) 7.31 (s, 1 H) 7.76 (s, 1 H) 13.64 (br. s., 1 H). LC-MS 363.3 [M-H]⁻, 365.3 [M+H]⁺, RT 1.38 min.

### Step 3: 1-ethyl-4-hydroxy-9-methyl-2-oxo-1,2,5,9-tetrahydropyrido[3',2':6,7]cyclohepta[1,2-f]indole-3-carboxylic acid

To a suspension of methyl 1-ethyl-4-hydroxy-9-methyl-2-oxo-1,2,5,9-tetrahydropyrido[3',2':6,7]cyclohepta[1,2-f]indole-3-carboxylate (0.126 g, 0.35 mmol) in EtOAc (2.0 mL) was added LiI (0.14 mg, 1.05 mmol). The reaction mixture was stirred and heated at 60 °C for 1 h until complete consumption of starting material was observed. The mixture was cooled to room temperature, then acidified with aqueous HCl (1M, 2.0 mL) and diluted with H₂O. The product was extracted with EtOAc (3x8 mL). The organic phase was washed with Na₂S₂O₃ (10% aq, 4 mL), NaCl (aqueous saturated, 5 mL) and dried over Na₂SO₄. The solvent was removed and the resulting product was obtained as a yellow solid (0.105 g, 87%).
¹H NMR (500 MHz, CHCl₃-*d*) δ ppm 1.02 (t, *J*=7.1 Hz, 3 H) 2.15 (ddd, *J*=14.2, 5.7, 2.2 Hz, 1 H) 3.53 (ddd, *J*=14.2, 8.0, 0.9 Hz, 1 H) 3.88 (s, 3 H) 3.96 - 4.05 (m, 1 H) 4.16 - 4.26 (m, 1 H) 6.38 (ddd, *J*=9.9, 8.0, 5.7 Hz, 1 H) 6.59 (dd, *J*=3.2, 0.9 Hz, 1 H) 6.80 (dd, *J*=9.9, 2.2 Hz, 1 H) 7.24 (d, *J*=3.2 Hz, 1 H) 7.35 (s, 1 H) 7.77 (s, 1 H) 13.94 (s, 1 H) 16.11 (s, 1 H). LC-MS 351.2 [M+H]⁺, RT 1.51 min.

### Example 133

### 1,9-dimethyl-2-oxo-1,2,5,6,7,9-hexahydropyrido [3',2': 6,7] cyclohepta[1,2-f]indole-3-carboxylic acid (Cpd 133)

A solution of 1,9-dimethyl-2-oxo-1,2,5,9-tetrahydropyrido[3',2':6,7]cyclohepta[1,2-f]indole-3-carboxylic acid (Example 130, step 3, 28.0 mg, 0.09 mmol) in EtOAc (1 mL) and DCM (2 mL) was hydrogenated under H₂ (1 atm) over Pd/C (10%, 10 mg) until the starting material was completely consumed (5-6 h). The catalyst was then filtered off and the filtrate was washed with DCM. The mother liquor was dried over Na₂SO₄ and concentrated to afford a product (26.0 mg, 93%) as a yellow solid.
¹H NMR (500 MHz, CHCl₃-*d*) δ ppm 1.96 - 2.20 (m, 3 H) 2.49 (dd, *J*=13.2, 5.5 Hz, 1 H) 2.58 (td, *J*=12.8, 7.6 Hz, 1 H) 2.81 (ddd, *J*=13.2, 5.8, 1.1 Hz, 1 H) 3.71 (s, 3 H) 3.87 (s, 3 H) 6.56 (dd, *J*=3.2, 1.1 Hz, 1 H) 7.15 (d, *J*=3.2 Hz, 1 H) 7.28 (s, 1 H) 7.55 (s, 1 H) 8.40 (s, 1 H) 14.82 (br. s., 1 H). LC-MS 323.2 [M+H]⁺, RT 1.28 min.

### Example 134

### 4-hydroxy-1,9-dimethyl-2-oxo-1,2,5,6,7,9-hexahydropyrido[3',2':6,7]cyclohepta[1,2-f]indole-3-carboxylic acid (Cpd 134)

A solution of methyl 4-hydroxy-1,9-dimethyl-2-oxo-1,2,5,9-tetrahydropyrido[3',2':6,7]cyclohepta[1,2-f]indole-3-carboxylate (Example 131, step 1, 61.8 mg, 0.18 mmol) in EtOAc (3 mL) and DCM (4 mL) was hydrogenated under H₂ (1 atm) over Pd/C (10%, 25 mg) until the starting material was completely consumed (2-3 h). The catalyst was then filtered off and the filtrate was washed with DCM. The mother liquor was dried over Na₂SO₄ and concentrated. The residue was suspended in EtOAc (1.0 mL) and LiI (70.0 mg, 1.05 mmol) was added. The reaction mixture was stirred and heated at 60 °C for 1 h until complete consumption of the starting material was observed. The mixture was then cooled to room temperature, then acidified with aqueous HCl (1M, 2.0 mL) and diluted with H₂O. The product was extracted with EtOAc (3x5 mL) and the organic phase was washed with Na₂S₂O₃ (10% aq, 4 mL), NaCl (aqueous saturated, 5 mL) and dried over Na₂SO₄. The solvent was removed and the resulting product (51.3 mg, 86% overall) was obtained as a yellow solid.
¹H NMR (500 MHz, CHCl₃-*d*) δ ppm 1.59 (td, *J*=13.2, 7.1 Hz, 1 H) 1.93 - 2.23 (m, 2 H) 2.62 (td, *J*=13.2, 7.6 Hz, 1 H) 2.80 (dd, *J*=13.9, 6.3 Hz, 1 H) 3.03 (ddd, *J*=13.9, 5.8, 1.1 Hz, 1 H) 3.56 (s, 3 H) 3.86 (s, 3 H) 6.55 (dd, *J*=3.2, 1.1 Hz, 1 H) 7.14 (d, *J*=3.2 Hz, 1 H) 7.27 (s, 1 H) 7.52 (s, 1 H) 13.79 (s, 1 H) 15.90 (s, 1 H). LC-MS 337.2 [M-H]⁻, 339.2 [M+H]⁺, RT 1.46 min.

### Example 135

### 4-hydroxy-1,9-dimethyl-2-oxo-1,2,5,6,7,9,10,11-octahydropyrido[3',2':6,7]cyclohepta[1,2-f]indole-3-carboxylic acid (Cpd 135)

A solution of 4-hydroxy-1,9-dimethyl-2-oxo-1,2,5,9-tetrahydropyrido[3',2':6,7]cyclohepta[1,2-f]indole-3-carboxylic acid (Example 131, step 2, 38.0 mg, 0.11 mmol) in EtOAc (2 mL) and DCM (3 mL) was hydrogenated under H₂ (1 atm) over PtO₂ (13.0 mg) until complete conversion to indoline was observed. The catalyst was then filtered off and the filtrate was washed with DCM. The mother liquor was dried over Na₂SO₄ and concentrated. The residue was triturated with Et₂O and the resulting solid was filtered and washed with Et₂O. The product (10.0 mg, 26%) was obtained as a bright yellow solid.
¹H NMR (500 MHz, CHCl₃-*d*) δ ppm 1.64 (td, *J*=13.3, 7.6 Hz, 1 H) 1.98 - 2.14 (m, 2 H) 2.42 (td, *J*=12.7, 8.0 Hz, 1 H) 2.58 (ddd, *J*=13.3, 5.9, 1.3 Hz, 1 H) 2.89 (s, 3 H) 2.97 - 3.11 (m, 3 H) 3.47 - 3.53 (m, 2 H) 3.52 (s, 3 H) 6.39 (s, 1 H) 6.92 (s, 1 H) 13.74 (s, 1 H) 15.87 (s, 1 H). LC-MS 339.3 [M-H]⁻, 341.3 [M+H]⁺, RT 1.52 min.

### Example 136

### 10-((butylamino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 136)

The title compound (25.0 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol) to provide the product HCl salt (47%).
¹H NMR (500 MHz, DMSO-*d*₆) δ ppm 0.91 (t, *J*=7.4 Hz, 3 H) 1.35 (sxt, *J*=7.4 Hz, 2 H) 1.68 (quin, *J*=7.4 Hz, 2 H) 2.09 (quin, *J*=6.6 Hz, 2 H) 2.70 (br. s., 2 H) 2.93 - 3.09 (m, 2 H) 3.27 - 3.44 (m, 2 H, solvent overlap) 3.85 (s, 3 H) 4.42 (br. s., 2 H) 6.82 (s, 1 H) 7.54 (s, 1 H) 7.81 (s, 1 H) 9.27 (br. s., 2 H) 12.89 (s, 1 H) 13.84 (s, 1 H). LC-MS 408.3 [M-H]⁻, RT 1.08 min.

### Example 137

### 4-hydroxy-9-methyl-2-oxo-10-((pentylamino)methyl)-1,2,5,6,7,9-hexahydropyrido[3',2':6,7]cyclohepta[1,2-f]indole-3-carboxylic acid hydrochloride (Cpd 137)

The title compound (31.4 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol) to provide the product HCl salt (57%).
¹H NMR (500 MHz, DMSO-*d*₆) δ ppm 0.89 (t, *J*=6.3 Hz, 3 H) 1.31 (br. s., 4 H) 1.60 - 1.76 (m, 2 H) 2.01 - 2.15 (m, 2 H) 2.65 - 2.76 (m, 2 H) 2.92 - 3.06 (m, 2 H) 3.31 - 3.40 (m, 2 H, solvent overlap) 3.84 (s, 3 H) 4.43 (br. s., 2 H) 6.81 (s, 1 H) 7.54 (s, 1 H) 7.81 (s, 1 H) 9.15 (br. s., 2 H) 12.89 (br. s., 1 H) 13.84 (s, 1 H). LC-MS 422.3 [M-H]⁻, RT 1.11 min.

### Example 138

### 10-((hexylamino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 138)

The title compound (22.8 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol) to provide the product HCl salt (40%).
¹H NMR (500 MHz, DMSO-*d*₆) δ ppm 0.88 (t, *J*=6.6 Hz, 3 H) 1.19 - 1.38 (m, 6 H) 1.61 - 1.73 (m, 2 H) 2.01 - 2.16 (m, 2 H) 2.64 - 2.77 (m, 2 H) 2.92 - 3.05 (m, 2 H) 3.35 - 3.40 (m, 2 H, solvent overlap) 3.84 (s, 3 H) 4.43 (br. s., 2 H) 6.80 (s, 1 H) 7.54 (s, 1 H) 7.81 (s, 1 H) 9.13 (br. s., 2 H) 12.89 (br. s., 1 H) 13.84 (s, 1 H). LC-MS 436.3 [M-H]⁻, RT 1.17 min.

### Example 139

### 10-((heptylamino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 139)

The title compound (19.7 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol) to provide the product HCl salt (34%).
¹H NMR (500 MHz, DMSO-*d*₆) δ ppm 0.87 (t, *J*=7.0 Hz, 3 H) 1.20 - 1.37 (m, 8 H) 1.64 - 1.74 (m, 2 H) 2.03 - 2.15 (m, 2 H) 2.61 - 2.77 (m, 2 H) 2.91 - 3.07 (m, 2 H) 3.37 - 3.44 (m, 2 H, solvent overlap) 3.84 (s, 3 H) 4.42 (br. s., 2 H) 6.81 (s, 1 H) 7.54 (s, 1 H) 7.81 (s, 1 H) 9.24 (br. s., 2 H) 12.89 (s, 1 H) 13.84 (s, 1 H). LC-MS 450.3 [M-H]⁻, RT 1.21 min.

### Example 140

### 4-hydroxy-9-methyl-10-((octylamino)methyl)-2-oxo-1,2,5,6,7,9-hexahydropyrido[3',2':6,7]cyclohepta[1,2-f]indole-3-carboxylicacidhydrochloride (Cpd 140)

The title compound (15.7 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol) to provide the product HCl salt (26%).
¹H NMR (500 MHz, DMSO-*d*₆) δ ppm 0.86 (t, *J*=6.6 Hz, 5 H) 1.18 - 1.37 (m, 10 H) 1.62 - 1.75 (m, 2 H) 2.02 - 2.16 (m, 2 H) 2.63 - 2.77 (m, 2 H) 2.92 - 3.04 (m, 2 H) 3.38 - 3.42 (m, 2 H, solvent overlap) 3.84 (s, 3 H) 4.42 (br. s., 2 H) 6.81 (s, 1 H) 7.54 (s, 1 H) 7.81 (s, 1 H) 9.24 (br. s., 2 H) 12.89 (s, 1 H) 13.84 (s, 1 H). LC-MS 464.3 [M-H]⁻, RT 1.25 min.

### Example 141

### 4-hydroxy-9-methyl-10-((nonylamino)methyl)-2-oxo-1,2,5,6,7,9-hexahydropyrido[3',2':6,7]cyclohepta[1,2-f]indole-3-carboxylic acid hydrochloride (Cpd 141)

The title compound (19.6 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol) to provide the product HCl salt (32%).
¹H NMR (500 MHz, DMSO-*d*₆) δ ppm 0.86 (t, *J*=6.9 Hz, 3 H) 1.18 - 1.36 (m, 12 H) 1.60 - 1.74 (m, 2 H) 2.02 - 2.15 (m, 2 H) 2.62 - 2.77 (m, 2 H) 2.93 - 3.04 (m, 2 H) 3.37 - 3.44 (m, 2 H, solvent overlap) 3.84 (s, 3 H) 4.42 (br. s., 2 H) 6.81 (s, 1 H) 7.54 (s, 1 H) 7.81 (s, 1 H) 9.18 (br. s., 2 H) 12.89 (s, 1 H) 13.84 (s, 1 H). LC-MS 478.4 [M-H]⁻, RT 1.30 min.

### Example 142

### 10-(((2-(dimethylamino)ethyl)amino)methyl)-4-hydroxy-9-methyl-2-oxo-1,2,5,6,7,9-hexahydropyrido[3',2':6,7]cyclohepta[1,2-f]indole-3-carboxylic acid dihydrochloride (Cpd 142)

The title compound (16.8 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol, 28%) as a dihydrochloride salt.
¹H NMR (500 MHz, DMSO-*d*₆) δ ppm 2.02 - 2.15 (m, 2 H) 2.64 - 2.77 (m, 2 H) 2.85 (s, 6 H) 3.28 - 3.34 (m, 2 H) 3.52 (s, 4 H) 3.88 (s, 3 H) 4.50 (s, 2 H) 6.87 (s, 1 H) 7.55 (s, 1 H) 7.81 (s, 1 H) 9.86 (br. s., 1 H) 10.87 (br. s., 1 H) 12.90 (br. s., 1 H) 13.85 (br. s., 1 H). LC-MS 423.3 [M-H]⁻, 425.3 [M+H]⁺, RT 0.91 min.

### Example 143

### 4-hydroxy-9-methyl-10-(((2-(methylamino)ethyl)amino)methyl)-2-oxo-1,2,5,6,7,9-hexahydropyrido[3',2':6,7]cyclohepta[1,2-f]indole-3-carboxylic acid dihydrochloride (Cpd 143)

The title compound (23.8 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol, 41%) as a dihydrochloride salt.
¹H NMR (500 MHz, DMSO-*d*₆) δ ppm 2.09 (quin, *J*=6.6 Hz, 2 H) 2.61 (s, 3 H) 2.70 (m, 2 H) 3.35 - 3.49 (m, 6 H, solvent overlap) 3.88 (s, 3 H) 4.51 (s, 2 H) 6.86 (s, 1 H) 7.55 (s, 1 H) 7.81 (s, 1 H) 9.29 (br. s., 2 H) 9.88 (br. s., 2 H) 12.90 (br. s., 1 H) 13.85 (br. s., 1 H). LC-MS 409.3 [M-H]⁻, RT 0.88 min.

### Example 144

### 10-(((2-aminoethyl)amino)methyl)-4-hydroxy-9-methyl-2-oxo-1,2,5,6,7,9-hexahydropyrido[3',2':6,7]cyclohepta[1,2-f]indole-3-carboxylic acid dihydrochloride (Cpd 144)

The title compound (19.0 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol, 34%) as a dihydrochloride salt.
¹H NMR (500 MHz, DMSO-*d*₆) δ ppm 2.09 (quin, *J*=6.6 Hz, 2 H) 2.65 - 2.77 (m, 2 H) 3.25 - 3.41 (m, 6 H, solvent overlap) 3.88 (s, 3 H) 4.51 (s, 2 H) 6.86 (s, 1 H) 7.55 (s, 1 H) 7.81 (s, 1 H) 8.35 (br. s., 3 H) 9.94 (br. s., 2 H) 12.91 (br. s., 1 H) 13.84 (br. s., 1 H). LC-MS 395.2 [M-H]⁻, RT 0.88 min.

### Example 145

### 10-(((2-(dimethylamino)ethyl)(methyl)amino)methyl)-4-hydroxy-9-methyl-2-oxo-1,2,5,6,7,9-hexahydropyrido[3',2':6,7]cyclohepta[1,2-f]indole-3-carboxylic acid dihydrochloride (Cpd 145)

The title compound (29.6 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol, 48%) as a dihydrochloride salt.
¹H NMR (500 MHz, DMSO-*d*₆) δ ppm 2.09 (quin, *J=6.6* Hz, 2 H) 2.64 - 2.75 (m, 2 H) 2.83 (br. s., 9 H) 3.50 - 3.72 (m, 6 H, solvent overlap) 3.90 (s, 3 H) 4.47 - 4.93 (m, 2 H) 7.00 (s, 1 H) 7.55 (s, 1 H) 7.82 (s, 1 H) 10.77 (br. s., 1 H) 11.23 (br. s., 1 H) 12.88 (br. s., 1 H) 13.85 (br. s., 1 H). LC-MS 437.3 [M-H]⁻, 439.4 [M+H]⁺, RT 1.01 min.

### Example 146

### 10-((sec-butylamino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 146)

The title compound (17.7 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol) to provide the product HCl salt (33%).
¹H NMR (500 MHz, DMSO-*d*₆) δ ppm 0.94 (t, *J*=7.4 Hz, 3 H) 1.34 (d, *J*=6.3 Hz, 3 H) 1.51 - 1.63 (m, 1 H) 1.87 - 1.99 (m, 1 H) 2.09 (quin, *J*=6.4 Hz, 2 H) 2.63 - 2.76 (m, 2 H) 3.19 - 3.29 (m, 1 H) 3.31 - 3.36 (m, 2 H, solvent overlap) 3.85 (s, 3 H) 4.44 (br. s., 2 H) 6.83 (s, 1 H) 7.54 (s, 1 H) 7.81 (s, 1 H) 9.17 (br. s., 1 H) 9.26 (br. s., 1 H) 12.90 (br. s., 1 H) 13.84 (br. s., 1 H). LC-MS 408.3 [M-H]⁻, RT 1.04 min.

### Example 147

### 4-hydroxy-10-(((2-hydroxyethyl)amino)methyl)-9-methyl-2-oxo-1,2,5,6,7,9-hexahydropyrido[3',2':6,7]cyclohepta[1,2-f]indole-3-carboxylic acid hydrochloride (Cpd 147)

The title compound (20.1 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol) to provide the product (39%). After removal of excess TFA, the residue was stirred with MeOH (2 mL) until the product was deprotected. MeOH was then removed under reduced pressure and the residue was treated with HCl solution (2M Et₂O) to yield the title compound as an HCl salt.
¹H NMR (500 MHz, DMSO-*d*₆) δ ppm 2.09 (quin, *J*=6.6 Hz, 2 H) 2.64 - 2.79 (m, 2 H) 3.03 - 3.14 (m, 2 H) 3.28 - 3.37 (m, 2 H, solvent overlap) 3.70 - 3.76 (m, 2 H) 3.84 (s, 3 H) 4.46 (br. s., 2 H) 5.30 (br. s., 1 H) 6.82 (s, 1 H) 7.54 (s, 1 H) 7.81 (s, 1 H) 9.17 (br. s., 2 H) 12.88 (br. s., 1 H) 13.84 (br. s., 1 H). LC-MS 396.3 [M-H]⁻, 398.4 [M+H]⁺, RT 0.98 min.

### Example 148

### 4-hydroxy-10-(((2-methoxyethyl)amino)methyl)-9-methyl-2-oxo-1,2,5,6,7,9-hexahydropyrido[3',2':6,7]cyclohepta[1,2-f]indole-3-carboxylic acid hydrochloride (Cpd 148)

The title compound (21.7 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol) to provide the product HCl salt (41 %).
¹H NMR (500 MHz, DMSO-*d*₆) δ ppm 2.09 (quin, *J*=6.6 Hz, 2 H) 2.64 - 2.77 (m, 2 H) 3.18 - 3.22 (m, 2 H) 3.31 - 3.40 (m, 2 H, solvent overlap) 3.33 (s, 3 H) 3.68 (t, *J*=5.2 Hz, 2 H) 3.84 (s, 3 H) 4.44 (br. s, 2 H) 6.83 (s, 1 H) 7.54 (s, 1 H) 7.81 (s, 1 H) 9.42 (br. s., 2 H) 12.89 (br. s., 1 H) 13.84 (br. s., 1 H). LC-MS 410.3 [M-H]⁻, RT 1.01 min.

### Example 149

### 4-hydroxy-10-(((2-hydroxyethyl)(methyl)amino)methyl)-9-methyl-2-oxo-1,2,5,6,7,9-hexahydropyrido[3',2':6,7]cyclohepta[1,2-f]indole-3-carboxylic acid hydrochloride (Cpd 149)

The title compound (22.1 mg) was prepared according to the 2 step procedure described for Example 147 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol) to provide the product HCl salt (40%).
¹H NMR (500 MHz, DMSO-*d*₆) δ ppm 2.09 (quin, *J*=6.3 Hz, 2 H) 2.66 - 2.78 (m, 2 H) 2.82 (br. s., 3 H) 3.18 - 3.33 (m, 2 H) 3.37 - 3.44 (m, 2 H, solvent overlap) 3.81 (t, *J*=4.7 Hz, 2 H) 3.87 (s, 3 H) 4.55 (d, *J*=14.2 Hz, 1 H) 4.73 (d, *J*=14.2 Hz, 1 H) 5.42 (br. s., 1 H) 6.91 (s, 1 H) 7.56 (s, 1 H) 7.84 (s, 1 H) 9.94 (br. s., 1 H) 12.88 (br. s., 1 H) 13.85 (s, 1 H). LC-MS 410.2 [M-H]⁻, RT 0.98.

### Example 150

### 4-hydroxy-10-(((1-methoxypropan-2-yl)amino)methyl)-9-methyl-2-oxo-1,2,5,6,7,9-hexahydropyrido[3',2':6,7]cyclohepta[1,2-f]indole-3-carboxylic acid hydrochloride (Cpd 150)

The title compound (29.8 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol) to provide the product HCl salt (54%).
¹H NMR (500 MHz, DMSO-*d*₆) δ ppm 1.33 (d, *J*=6.9 Hz, 3 H) 2.09 (quin, *J*=6.5 Hz, 2 H) 2.63 - 2.79 (m, 2 H) 3.33 - 3.35 (m, 2 H, solvent overlap) 3.36 (s, 3 H) 3.49 - 3.57 (m, 1 H) 3.58 - 3.68 (m, 2 H) 3.84 (s, 3 H) 4.45 (br. s., 2 H) 6.83 (s, 1 H) 7.54 (s, 1 H) 7.81 (s, 1 H) 9.20 (br. s., 1 H) 9.36 (br. s., 1 H) 12.90 (br. s., 1 H) 13.84 (br. s., 1 H). LC-MS 424.3 [M-H]⁻, 426.2 [M+H]⁺, RT 1.03 min.

### Example 151

### 4-hydroxy-10-(((1-hydroxypropan-2-yl)amino)methyl)-9-methyl-2-oxo-1,2,5,6,7,9-hexahydropyrido[3',2':6,7]cyclohepta[1,2-f]indole-3-carboxylic acid hydrochloride (Cpd 151)

The title compound (21.6 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol) to provide the product HCl salt (40%).
¹H NMR (500 MHz, DMSO-*d*₆) δ ppm 1.30 (d, *J*=6.6 Hz, 3 H) 2.00 - 2.17 (m, 2 H) 2.64 - 2.80 (m, 2 H) 3.35 - 3.47 (m, 3 H, solvent overlap) 3.61 (dd, *J=*11.5, 5.2 Hz, 1 H) 3.68 - 3.77 (m, 1 H) 3.84 (s, 3 H) 4.47 (br. s., 2 H) 5.46 (br. s., 1 H) 6.82 (s, 1 H) 7.54 (s, 1 H) 7.81 (s, 1 H) 8.97 (br. s., 1 H) 9.15 (br. s., 1 H) 12.90 (br. s., 1 H) 13.84 (br. s., 1 H). LC-MS 410.2 [M-H]⁻, RT 0.99.

### Example 152

### 4-hydroxy-9-methyl-2-oxo-10-(((2-(pyrrolidin-1-yl)ethyl)amino)methyl)-1,2,5,6,7,9-hexahydropyrido[3',2':6,7]cyclohepta[1,2-f]indole-3-carboxylic acid dihydrochloride (Cpd 152)

The title compound (21.4 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 60.0 mg, 0.12 mmol, 34%) as a dihydrochloride salt.
¹H NMR (500 MHz, DMSO-*d*₆) δ ppm 1.84 - 2.05 (m, 4 H) 2.09 (quin, *J*=6.3 Hz, 2 H) 2.65 - 2.76 (m, 2 H) 2.97 - 3.16 (m, 2 H) 3.30 - 3.34 (m, 2 H, solvent overlap) 3.43-3.71 (m, 6 H) 3.88 (s, 3 H) 4.51 (br. s., 2 H) 6.86 (s, 1 H) 7.55 (s, 1 H) 7.81 (s, 1 H) 9.79 (br. s., 2 H) 11.01 (br. s., 1 H) 12.90 (br. s., 1 H) 13.84 (br. s., 1 H). LC-MS 449.3 [M-H]⁻, 451.3 [M+H]⁺ RT 0.92.

### Example 153

### 10-((allylamino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 153)

The title compound (19.6 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 50.0 mg, 0.10 mmol) to provide the product HCl salt (46%).
¹H NMR (500 MHz, DMSO-*d*₆) δ ppm 2.09 (quin, *J*=6.8 Hz, 2 H) 2.65 - 2.78 (m, 2 H) 3.29 - 3.42 (m, 2 H) 3.70 (d, *J*=6.6 Hz, 2 H) 3.84 (s, 3 H) 4.40 (br. s., 2 H) 5.45 (dd, *J*=10.4, 1.3 Hz, 1 H) 5.52 (dd, *J*=17.3, 1.3 Hz, 1 H) 6.00 (ddt, *J*=17.3, 10.4, 6.6 Hz, 1 H) 6.82 (s, 1 H) 7.54 (s, 1 H) 7.81 (s, 1 H) 9.52 (br. s., 2 H) 12.89 (br. s., 1 H) 13.84 (br. s., 1 H). LC-MS 392.3 [M-H]⁻, 394.2 [M+H]⁺, RT 0.54 min.

### Example 154

### 4-hydroxy-10-((isobutylamino)methyl)-9-methyl-2-oxo-1,2,5,6,7,9-hexahydropyrido[3',2':6,7]cyclohepta[1,2-f]indole-3-carboxylic acid hydrochloride (Cpd 154)

The title compound (21.7 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 50.0 mg, 0.10 mmol) to provide the product HCl salt (49%).
¹H NMR (500 MHz, DMSO-*d*₆) δ ppm 0.97 (d, *J*=6.6 Hz, 6 H) 2.00 - 2.14 (m, 3 H) 2.65 - 2.77 (m, 2 H) 2.81 - 2.90 (m, 2 H) 3.34 - 3.48 (m, 2 H) 3.85 (s, 3 H) 4.43 (br. s., 2 H) 6.86 (s, 1 H) 7.54 (s, 1 H) 7.81 (s, 1 H) 9.18 (br. s., 2 H) 12.89 (br. s., 1 H) 13.84 (s, 1 H). LC-MS 408.3 [M-H]⁻, 410.3 [M+H]⁺, RT 0.56 min.

### Example 155

### 4-hydroxy-9-methyl-10-((neopentylamino)methyl)-2-oxo-1,2,5,6,7,9-hexahydropyrido[3',2':6,7]cyclohepta[1,2-f]indole-3-carboxylic acid hydrochloride (Cpd 155)

The title compound (20.4 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 50.0 mg, 0.10 mmol) to provide the product HCl salt (45%).
¹H NMR (500 MHz, DMSO-*d*₆) δ ppm 1.00 (s, 9 H) 2.09 (quin, *J*=6.5 Hz, 2 H) 2.65 - 2.76 (m, 2 H) 2.80 - 2.88 (m, 2 H) 3.32 - 3.44 (m, 2 H) 3.85 (s, 3 H) 4.46 (br. s., 2 H) 6.90 (s, 1 H) 7.55 (s, 1 H) 7.82 (s, 1 H) 9.00 (br. s., 2 H) 12.89 (br. s., 1 H) 13.84 (br. s., 1 H). LC-MS 422.4 [M-H]⁻, 424.4 [M+H]⁺, RT 0.59 min.

### Example 156

### 4-hydroxy-9-methyl-10-((4-methyl-1,4-diazepan-1-yl)methyl)-2-oxo-1,2,5,6,7,9-hexahydropyrido[3',2':6,7]cyclohepta[1,2-f]indole-3-carboxylic acid dihydrochloride (Cpd 156)

The title compound (31.4 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 50.0 mg, 0.10 mmol, 60%) as a dihydrochloride salt.
¹H NMR (500 MHz, DMSO-*d*₆) δ ppm 2.09 (quin, *J*=6.6 Hz, 2 H) 2.15 - 2.34 (m, 2 H) 2.64 - 2.74 (m, 2 H) 2.78 (s, 3 H) 3.09 - 3.87 (m, 10 H) 3.90 (s, 3 H) 4.67 (br. s., 2 H) 6.97 (s, 1 H) 7.56 (s, 1 H) 7.82 (s, 1 H) 10.84 - 11.46 (m, 1 H) 11.50 - 12.01 (m, 1 H) 12.88 (br. s., 1 H) 13.85 (s, 1 H). LC-MS 449.3 [M-H]⁻, 451.3 [M+H]⁺, RT 0.53 min.

### Example 157

### 4-hydroxy-9-methyl-10-(((1-methylpiperidin-4-yl)amino)methyl)-2-oxo-1,2,5,6,7,9-hexahydropyrido[3',2':6,7]cyclohepta[1,2-f]indole-3-carboxylic acid dihydrochloride (Cpd 157)

The title compound (30.7 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 50.0 mg, 0.10 mmol, 59%) as a dihydrochloride salt.
¹H NMR (500 MHz, DMSO-*d*₆) δ ppm 1.99 - 2.16 (m, 4 H) 2.34 - 2.45 (m, 2 H) 2.67 - 2.72 (m, 2 H) 2.73 (s, 3 H) 2.91 - 3.07 (m, 2 H) 3.33 - 3.43 (m, 3 H, solvent overlap) 3.53 (br. d, *J*=12.0 Hz, 2 H) 3.86 (s, 3 H) 4.48 (br. s., 2 H) 6.85 (s, 1 H) 7.55 (s, 1 H) 7.81 (s, 1 H) 9.76 (br. s., 2 H) 10.53 (br. s., 1 H) 12.90 (br. s., 1 H) 13.84 (br. s., 1 H). LC-MS 449.3 [M-H]⁻, 451.3 [M+H]⁺, RT 0.46 min.

### Example 158

### 4-hydroxy-10-((3-methoxypyrrolidin-1-yl)methyl)-9-methyl-2-oxo-1,2,5,6,7,9-hexahydropyrido[3',2':6,7]cyclohepta[1,2-f]indole-3-carboxylic acid hydrochloride (Cpd 158)

The title compound (27.3 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 50.0 mg, 0.10 mmol) to provide the product HCl salt (45%).
¹H NMR (500 MHz, DMSO-*d*₆) δ ppm 1.91 - 2.24 (m, 4 H) 2.65 - 2.79 (m, 2 H) 3.21 - 3.33 (m, 4 H) 3.38 (s, 3 H) 3.47 - 3.75 (m, 2 H) 3.86 (s, 3 H) 4.07 - 4.25 (m, 1 H) 4.66 (br. s., 2 H) 6.91 (s, 1 H) 7.55 (s, 1 H) 7.82 (s, 1 H) 10.52 - 11.14 (m, 1 H) 12.88 (br. s., 1 H) 13.85 (s, 1 H). LC-MS 436.4 [M-H]⁻, 438.3 [M+H]⁺, RT 0.55 min.

### Example 159

### 10-((3-acetamidopyrrolidin-1-yl)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 159)

The title compound (30.8 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 50.0 mg, 0.10 mmol) to provide the product HCl salt (62%).
¹H NMR (500 MHz, DMSO-*d*₆) δ ppm 1.79 - 1.85 (m, 3 H) 1.85 - 2.28 (m, 4 H) 2.65 - 2.81 (m, 2 H) 2.97 - 3.79 (m, 6 H) 3.87 (s, 3 H) 4.21 - 4.42 (m, 1 H) 4.60 - 4.82 (m, 2 H) 6.85 - 6.94 (m, 1 H) 7.55 (s, 1 H) 7.83 (s, 1 H) 8.20 - 8.41 (m, 1 H) 10.50 - 10.82 (m, 1 H) 12.87 (br. s., 1 H) 13.85 (s, 1 H). LC-MS 463.3 [M-H]⁻, 465.3 [M+H]⁺, RT 0.52 min.

### Example 160

### 10-(((1-(dimethylamino)propan-2-yl)amino)methyl)-4-hydroxy-9-methyl-2-oxo-1,2,5,6,7,9-hexahydropyrido[3',2':6,7]cyclohepta[1,2-f]indole-3-carboxylic acid dihydrochloride (Cpd 160)

The title compound (30.7 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 50.0 mg, 0.10 mmol, 60%) as a dihydrochloride salt.
¹H NMR (500 MHz, DMSO-*d*₆) δ ppm 1.51 (d, *J*=4.4 Hz, 3 H) 2.04 - 2.15 (m, 2 H) 2.66 - 2.76 (m, 2 H) 2.80 - 2.83 (m, 1 H) 2.87 (s, 3 H) 2.92 (s, 3 H) 3.30 - 3.43 (m, 2 H, solvent overlap) 3.58 - 3.68 (m, 1 H) 3.87 (s, 3 H) 3.93 - 4.05 (m, 1 H) 4.34 - 4.71 (m, 2 H) 6.89 (s, 1 H) 7.55 (s, 1 H) 7.82 (s, 1 H) 9.75 (br. s., 2 H) 10.64 (br. s., 1 H) 12.92 (br. s., 1 H) 13.83 (br. s., 1 H). LC-MS 437.3 [M-H]⁻, 439.3 [M+H]⁺, RT 0.57 min.

### Example 161

### 4-hydroxy-9-methyl-2-oxo-10-((((tetrahydrofuran-2-yl)methyl)amino)methyl)-1,2,5,6,7,9-hexahydropyrido[3',2':6,7]cyclohepta[1,2-f]indole-3-carboxylic acid hydrochloride (Cpd 161)

The title compound (21.7 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 50.0 mg, 0.10 mmol) to provide the product HCl salt (46%).
¹H NMR (500 MHz, DMSO-*d*₆) δ ppm 1.51 - 1.65 (m, 1 H) 1.77 - 1.94 (m, 2 H) 1.97 - 2.06 (m, 1 H) 2.09 (quin, *J*=6.6 Hz, 2 H) 2.63 - 2.76 (m, 2 H) 2.94 - 3.05 (m, 1 H) 3.10 - 3.22 (m, 1 H) 3.36 - 3.42 (m, 2 H, solvent overlap) 3.74 (dd, *J*=13.9, 6.3 Hz, 2 H) 3.79 - 3.86 (m, 1 H) 3.83 (s, 3 H) 4.15 - 4.28 (m, 1 H) 4.32 - 4.56 (m, 2 H) 6.83 (s, 1 H) 7.54 (s, 1 H) 7.81 (s, 1 H) 9.26 (br. s., 1 H) 9.38 (br. s., 1 H) 12.90 (br. s., 1 H) 13.84 (s, 1 H). LC-MS 436.2 [M-H]⁻, 438.1 [M+H]⁺, RT 0.73 min.

### Example 162

### 4-hydroxy-9-methyl-10-((1-methylhexahydropyrrolo[3,4-b]pyrrol-5(1H)-yl)methyl)-2-oxo-1,2,5,6,7,9-hexahydropyrido[3',2':6,7]cyclohepta[1,2-f]indole-3-carboxylic acid dihydrochloride (Cpd 162)

The title compound (33.3 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 50.0 mg, 0.10 mmol, 63%) as a dihydrochloride salt.
¹H NMR (500 MHz, MeOH-*d*₄) δ ppm 2.14 - 2.24 (m, 3 H) 2.41 - 2.58 (m, 1 H) 2.70 - 2.84 (m, 2 H) 2.97 (s, 3 H) 3.17 - 3.81 (m, 8 H) 3.96 (s, 3 H) 4.17 - 4.81 (m, 4 H) 6.91 (br. s, 1 H) 7.49 (s, 1 H) 7.81 (s, 1 H). LC-MS 461.4 [M-H]⁻, 463.3 [M+H]⁺, RT 0.58 min.

### Example 163

### 10-(((2-(dimethylamino)-2-oxoethyl)amino)methyl)-4-hydroxy-9-methyl-2-oxo-1,2,5,6,7,9-hexahydropyrido[3',2':6,7]cyclohepta[1,2-f]indole-3-carboxylic acid hydrochloride (Cpd 163)

The title compound (20.4 mg) was prepared according to the 2 step procedure described for Example 44 starting from 1-(2,4-dimethoxybenzyl)-10-formyl-4-hydroxy-9-methyl-2-oxo-1,2,5,6,7,9-hexahydropyrido[3',2':6,7]cyclohepta[1,2-f]indole-3-carboxylic acid (Example 22, step 9, 50.0 mg, 0.10 mmol) to provide the product HCl salt (43%).
¹H NMR (500 MHz, DMSO-*d*₆) δ ppm 2.09 (quin, *J*=6.9 Hz, 2 H) 2.64 - 2.76 (m, 2 H) 2.88 (s, 3 H) 2.95 (s, 3 H) 3.38 - 3.41 (m, 2 H, solvent overlap) 3.85 (s, 3 H) 4.13 (br. s., 2 H) 4.41 (br. s., 2 H) 6.84 (s, 1 H) 7.53 (s, 1 H) 7.82 (s, 1 H) 9.43 (br. s., 2 H) 12.91 (br. s., 1 H) 13.84 (s, 1 H). LC-MS 437.3 [M-H]⁻, 439.3 [M+H]⁺, RT 0.54 min.

### Example 164

### 10-(2-(ethylamino)ethyl)-4-hydroxy-9-methyl-2-oxo-1,2,5,9-tetrahydropyrido[3',2':6,7]cyclohepta[1,2-f]indole-3-carboxylic acid trifluoroacetate (Cpd 164)

### Steps 1-2: 6-chloro-2-formyl-1-methyl-1H-indole-5-carbonitrile

To a solution of 2-((tert-butyldimethylsilyloxy)methyl)-6-chloro-1H-indole-5-carbonitrile (Example 19, step 4, 15.50 g, 46.28 mmol) in THF (120 mL) was added TBAF solution (1M THF, 60.0 mL, 60.0 mmol). The reaction mixture was stirred at room temperature for 30 min until the starting material was completely consumed. The THF was removed and water was added to the residue. The solid was collected by filtration, washed with H₂O and dried with air flow and then suspended in DCM (400 mL) and treated with MnO₂ (40 g, 460 mmol). The reaction mixture was stirred at room temperature for 1.5 h. MnO₂ was filtered off and washed with DCM (2x400 mL). The mother liquor was concentrated and the residue was triturated with Et₂O to afford 6-chloro-2-formyl-1-methyl-1H-indole-5-carbonitrile (5.22 g, 53% overall) as a colorless solid.
¹H NMR (500 MHz, DMSO-*d*₆) δ ppm 4.04 (s, 3 H) 7.58 (s, 1 H) 8.09 (s, 1 H) 8.49 (s, 1 H) 9.98 (s, 1 H). LC-MS RT 1.39 min.

### Step 3: 6-chloro-2-(2-methoxyvinyl)-1-methyl-1H-indole-5-carbonitrile

To a suspension of (methoxymethyl)triphenylphosphonium chloride (12.3 g, 35.88 mmol) in THF (90 mL) at -78 °C was added a solution of LHMDS (1M THF, 36.0 mL, 36.0 mmol) dropwise. The mixture was stirred at 0 °C for 10 min, then a solution of 6-chloro-2-formyl-1-methyl-1H-indole-5-carbonitrile (5.22 g, 23.92 mmol) in THF (170 mL) was added. The reaction mixture was allowed to warm to room temperature, with stirring for 1 h, then the reaction was quenched with NH₄Cl. The product was extracted with EtOAc (3x100 mL) and the combined organics were washed with NaCl (aqueous saturated, 100 mL) then dried over Na₂SO₄. The solvent was removed and the resulting residue was purified by column chromatography (EtOAc/10%DCM-hexanes, 0-50% gradient), affording the product (4.86 g, 82 %) as 1:1 mixture of isomers.
¹H NMR (500 MHz, DMSO-*d*₆) δ ppm 3.73 (s, 3 H) 3.73 (s, 3 H) 3.74 (s, 3 H) 3.87 (s, 3 H) 5.55 (d, *J*=6.9 Hz, 1 H) 5.97 (d, *J*=12.6 Hz, 1 H) 6.58 (s, 1 H) 6.64 (d, *J*=6.9 Hz, 1 H) 6.85 (s, 1 H) 7.38 (d, *J*=12.6 Hz, 1 H) 7.81 (s, 1 H) 7.82 (s, 1 H) 8.02 (s, 1 H) 8.08 (s, 1 H). LC-MS 247.2/249.2 [M+H]⁺, RT 1.57 min.

### Step 4-6: tert-butyl(2-(6-chloro-5-cyano-1-methyl-1H-indol-2-yl)ethyl)(ethyl)carbamate

To a solution of 6-chloro-2-(2-methoxyvinyl)-1-methyl-1H-indole-5-carbonitrile (4.80 g, 19.46 mmol) in dioxane (200 mL) and H₂O (5 mL) was added a solution of HCl (conc., 2.0 mL). The mixture was stirred at room temperature and closely monitored by TLC. After complete consumption of starting material the reaction mixture was diluted with H₂O (300 mL) and the product was extracted with DCM (3x200 mL). The combined organics were dried over Na₂SO₄ and then concentrated. The residue was suspended in DCE (150 mL) and EtNH₂ solution (2M THF, 20.4 mL, 40.8 mmol), AcOH (3.1 mL, 54.56 mmol) and NaBH(OAc)₃ (10.2 g, 48.13 mmol) were added subsequently. The reaction mixture was stirred at room temperature overnight and then the reaction was quenched with NaHCO₃ (aqueous saturated, 50 mL). The product was extracted with DCM (3x200 mL). The combined organics were dried over Na₂SO₄ and then concentrated. The residue was suspended in DCM (150 mL) and treated with BoC₂O (5.6 g, 25.66 mmol) and DMAP (∼20 mg, cat.). After stirring at room temperature for 2 h, the reaction was quenched with NaHCO₃ (aqueous saturated, 50 mL). The product was extracted with DCM (3x200 mL) and the combined organics were dried over Na₂SO₄ and concentrated. The residue was purified by column chromatography (EtOAc/hexanes, 0-50% gradient), affording the product *test*-butyl (2-(6-chloro-5-cyano-1-methyl-1H-indol-2-yl)ethyl)(ethyl)carbamate (1.56 g, 22%, over 3 steps).
¹H NMR (500 MHz, Acetone) δ ppm 1.08 (t, *J*=6.9 Hz, 3 H) 1.28 - 1.45 (m, 9 H) 3.09 (t, *J*=7.3 Hz, 2 H) 3.26 (br. s., 2 H) 3.55 (t, *J*=7.3 Hz, 2 H) 3.87 (s, 3 H) 6.47 (s, 1 H) 7.69 (br. s., 1 H) 7.98 (s, 1 H). LC-MS 362.4/364.4 [M+H]⁺, RT 1.45 min.

### Step 7: tert-butyl (2-(6-chloro-5-formyl-1-methyl-1H-indol-2-yl)ethyl)(ethyl)carbamate

To a solution of tert-butyl (2-(6-chloro-5-cyano-1-methyl-1H-indol-2-yl)ethyl)(ethyl)carbamate (1.55 g, 4.28 mmol) in DCM (17 mL) at -78 °C was added DIBAL-H (1M in DCM, 5.1 mL, 5.1 mmol) dropwise over∼10 min. The reaction was stirred at -78 °C for 60 min, then another portion of DIBAL-H (1M in DCM, 2.5 mL, 2.5 mmol) was added. The reaction mixture was allowed to slowly warm to -40 °C and the reaction was carefully quenched by addition of Rochelle salt (aqueous saturated, 10 mL). The resulting emulsion was allowed to warm to room temperature and was vigorously stirred for ∼2 h. The organic phase was separated and the aqueous layer was extracted with DCM (50 mL). The combined organics were stirred vigorously with 1M HCl (50 mL) for 15 min, then washed with NaHCO₃ (aqueous saturated, 50 mL) and NaCl (aqueous saturated, 50 mL). After drying over Na₂SO₄, the solvent was removed and the residue was purified by column chromatography (EtOAc/hexanes, 0-40% gradient), affording the product as a colorless solid (1.12 g, 72 %).
¹H NMR (500 MHz, CHCl₃-*d*) δ ppm 1.10 (t, *J*=6.9 Hz, 3 H) 1.43 (br. s., 9 H) 2.99 (t, *J*=6.9 Hz, 2 H) 3.22 (br. s., 2 H) 3.48 (dd, *J*=8.4, 6.9 Hz, 2 H) 3.73 (s, 3 H) 6.38 (d, *J*=0.6 Hz, 1 H) 7.29 (s, 1 H) 8.15 (s, 1 H) 10.49 (s, 1 H).

### Step 8: tert-butyl ethyl(2-(5-formyl-1-methyl-6-vinyl-1H-indol-2-yl)ethyl)carbamate

tert-butyl (2-(6-chloro-5-formyl-1-methyl-1H-indol-2-yl)ethyl)(ethyl)carbamate (1.12 g, 3.07 mmol), potassium vinyltrifluoroborate (0.62 g, 4.63 mmol), Pd(OAc)₂ (21 mg, 0.09 mmol, 3 mol%), S-Phos ligand (76 mg, 0.18 mmol, 6 mol%) and K₂CO₃ (1.30 g, 9.41 mmol) were mixed together in a 50 mL round bottom flask. The flask was placed under vacuum and back filled with Argon, then dioxane (12 mL) and H₂O (2 mL) were added. The mixture was heated at 85-90 °C for 3 h, then another portion of Pd(OAc)₂ (21 mg, 0.09 mmol, 3 mol%) was added. After heating for another 3 h at 90 °C, complete consumption of the starting material was observed. The reaction was cooled to room temperature and water (10 mL) was added. The product was extracted with DCM (3x50 mL) and the combined organics were washed with NaCl (aqueous saturated, 40 mL) then dried over Na₂SO₄. After concentration of the solvent, the residue was purified by column chromatography (EtOAc/hexanes, 0-40% gradient), affording the product as a white solid (0.896 g, 82 %).
¹H NMR (500 MHz, Acetone) δ ppm 1.08 (t, *J*=6.9 Hz, 3 H) 1.28 - 1.50 (m, 9 H) 3.08 (t, *J*=7.4 Hz, 2 H) 3.27 (br. s., 2 H) 3.56 (t, *J*=7.4 Hz, 2 H) 3.88 (s, 3 H) 5.31 (dd, *J*=11.0, 1.6 Hz, 1 H) 5.77 (dd, *J*=17.3, 1.6 Hz, 1 H) 6.47 (s, 1 H) 7.67 (s, 1 H) 7.85 (dd, *J*=17.3, 11.0 Hz, 1 H) 8.02 (s, 1 H) 10.21 (s, 1 H). LC-MS 357.3 [M+H]⁺, RT 1.82 min.

### Step 9-11: tert-butyl ethyl(2-(1-methyl-5-oxo-1,5,6,7-tetrahydrocyclohepta[f]indol-2-yl)ethyl)carbamate

To a solution of tert-butyl ethyl(2-(5-formyl-1-methyl-6-vinyl-1H-indol-2-yl)ethyl)carbamate (0.85 g, 2.38 mmol) in THF (5 mL) at -78 °C was added 3-butenylmagnesium bromide (0.5M in THF, 6.0 mL, 3.0 mmol) dropwise over ∼10 min. The reaction mixture was stirred for 10 min and slowly allowed to warm to 0 °C. After 2 h, the reaction was quenched by addition of NH₄Cl (aqueous saturated, 8 mL). The product was extracted with EtOAc (4x15 mL) and the combined organics were washed with NaCl (aqueous saturated, 100 mL) and dried over Na₂SO₄. The solvent was removed and the resulting residue was dissolved in toluene (48 mL, 0.05M) under Argon. A second generation Grubbs catalyst (61 mg, 0.07 mmol, 3 mol%) was added and the mixture was heated at 60 °C for 3 h until the starting material was completely consumed as indicated by LC/MS. After cooling the reaction mixture to room temperature, the toluene was removed under reduced pressure and the residue was dissolved in DCM (12 mL) and added to activated 4Å molecular sieves (0.60 g, 250 mg/mmol). The mixture was cooled to 0 °C, then NMO (0.41 g, 3.50 mmol) and TPAP (41 mg, 0.12 mmol, 5 mol%) were added. The reaction was stirred at 0 °C for 10 min and slowly allowed to warm to room temperature. Once LC/MS and TLC indicated complete consumption of the starting material, the molecular sieves were filtered off and washed with DCM. The mother liquor was concentrated and the residue was purified by column chromatography (EtOAc/hexanes, 0-30% gradient). The product was obtained as a colorless solid (0.55 g, 60%).
¹H NMR (500 MHz, Acetone) δ ppm 1.07 (t, *J*=7.1 Hz, 3 H) 1.32 - 1.48 (m, 9 H) 2.42 - 2.49 (m, 2 H) 2.85 - 2.89 (m, 2 H) 3.05 (t, *J*=7.4 Hz, 2 H) 3.25 (br. s., 2 H) 3.53 (t, *J*=7.4 Hz, 2 H) 3.81 (s, 3 H) 6.05 (dt, *J*=11.3, 5.4 Hz, 1 H) 6.40 (s, 1 H) 6.64 (d, *J*=11. Hz, 1 H) 7.30 (s, 1 H) 8.05 (s, 1 H).

### Step 12-13: methyl 10-(2-((tert-butoxycarbonyl)(ethyl)amino)ethyl)-1-(2,4-dimethoxybenzyl)-4-hydroxy-9-methyl-2-oxo-1,2,5,9-tetrahydropyrido[3',2':6,7]cyclohepta[1,2-f]indole-3-carboxylate

To a solution of tert-butyl ethyl(2-(1-methyl-5-oxo-1,5,6,7-tetrahydrocyclohepta[f]indol-2-yl)ethyl)carbamate (0.55 g, 1.43 mmol) in DCM (6.0 mL) was added 2,4-dimethoxybenzylamine (0.23 mL, 1.53 mmol) and NEt₃ (0.60 mL, 4.30 mmol). The mixture was cooled to 0 °C, then a solution of TiCl₄ (1M DCM, 1.0 mL, 1.0 mmol) was added dropwise via syringe pump over 30 min. The reaction mixture was allowed to warm to room temperature and stirred overnight. The mixture was diluted with DCM (15 mL) and then the reaction was quenched with NaHCO₃ (aqueous saturated, 5 mL). After vigorous shaking the organic phase was separated using a PTFE phase separator and dried over Na₂SO₄. The solvent was removed and the resulting residue was mixed with Ph₂O (3 mL). With stirring, the mixture was placed onto a pre-heated heat block at 230 °C and heated for 10 min after the initial bubbling of MeOH was observed (occurs ant ∼160 °C internal reaction temperature). The reaction mixture was cooled to room temperature, then purified directly on a silica gel column (hexanes followed by EtOAc/hexanes 10-70% gradient) to provide the product as a yellow foam (0.382 g, 40% overall)
¹H NMR (500 MHz, CHCl₃-*d*) δ ppm 1.12 (t, *J*=6.6 Hz, 3 H) 1.45 (br. s., 9 H) 2.08 (ddd, *J*=14.5, 6.0, 2.2 Hz, 1 H) 3.00 (br. s., 2 H) 3.24 (br. s., 2 H) 3.39 (s, 3 H) 3.43 - 3.54 (m, 3 H) 3.73 (br. s, 6 H) 3.98 (s, 3 H) 4.91 (d, *J*=15.1 Hz, 1 H) 5.22 (br. s., 1 H) 6.20 (br. s., 2 H) 6.24 - 6.37 (m, 2 H) 6.57 - 6.74 (m, 2 H) 7.13 (s, 1 H) 7.54 (s, 1 H) LC-MS 658.8 [M+H]⁺, RT 1.58 min.

### Step 14: 10-(2-((tert-butoxycarbonyl)(ethyl)amino)ethyl)-1-(2,4-dimethoxybenzyl)-4-hydroxy-9-methyl-2-oxo-1,2,5,9-tetrahydropyrido[3',2':6,7]cyclohepta[1,2-f]indole-3-carboxylic acid

To a solution of methyl 10-(2-((tert-butoxycarbonyl)(ethyl)amino)ethyl)-1-(2,4-dimethoxybenzyl)-4-hydroxy-9-methyl-2-oxo-1,2,5,9-tetrahydropyrido[3',2':6,7]cyclohepta[1,2-f]indole-3-carboxylate (0.175 g, 0.27 mmol) in EtOAc (1.5 mL) was added LiI (0.11 g, 0.82 mmol). The reaction mixture was stirred and heated at 60 °C for 1 h until complete consumption of starting material was observed. The mixture was cooled to room temperature, then acidified with aqueous HCl (1M, 0.5 mL). The product was extracted with EtOAc (3x10 mL). The organic phase was washed with Na₂S₂O₃ (10% aq, 5 mL), NaCl (aqueous saturated, 10 mL) and dried over Na₂SO₄. The solvent was removed and the resulting product was obtained as a solid (0.165 g, 96%).
¹H NMR (500 MHz, CHCl₃-*d*) δ ppm 1.12 (t, *J*=5.7 Hz, 3 H) 1.33 - 1.52 (m, 9 H) 2.11 (ddd, J=14.2, 6.0, 2.2 Hz, 1 H) 3.03 (br. s., 2 H) 3.15 - 3.34 (m, 2 H) 3.43 (s, 3 H) 3.46 - 3.57 (m, 3 H) 3.76 (s, 3 H) 3.78 (br. s., 3 H) 5.00 (d, *J*=15.4 Hz, 1 H) 5.24 (d, *J*=15.4 Hz, 1 H) 6.16 - 6.36 (m, 4 H) 6.50 - 6.61 (m, 1 H) 6.66 (d, *J*=9.1 Hz, 1 H) 7.18 (s, 1 H) 7.57 (s, 1 H) 13.95 (s, 1 H) 16.00 (br. s., 1 H). LC-MS 642.8 [M-H]⁻, 644.7 [M+H]⁺, RT 1.63 min.

### Step 15: 10-(2-(ethylamino)ethyl)-4-hydroxy-9-methyl-2-oxo-1,2,5,9-tetrahydropyrido[3',2':6,7]cyclohepta[1,2-f]indole-3-carboxylic acid trifluoroacetate

To a solution of 10-(2-((tert-butoxycarbonyl)(ethyl)amino)ethyl)-1-(2,4-dimethoxybenzyl)-4-hydroxy-9-methyl-2-oxo-1,2,5,9-tetrahydropyrido[3',2':6,7]cyclohepta[1,2-f]indole-3-carboxylic acid (55 mg, 0.09 mmol) in DCM (1.0 mL) was added i-Pr₃SiH (0.50 mL) followed by TFA (0.50 mL). The mixture was stirred at room temperature and monitored by LC/MS. After complete consumption of starting material (∼8 h), the solvent was removed under reduced pressure. Addition of an HCl solution (2M Et₂O, 1.0 mL) to the oily residue resulted in precipitate formation. The mixture was diluted with Et₂O, the solid was filtered and washed with additional Et₂O. The product HCl salt was obtained as a colorless solid (29.3 mg, 92%) by LC/MS and was further purified by preparative HPLC, affording the title compound as a TFA salt.
¹H NMR (500 MHz, DMSO-*d*₆) δ ppm 1.22 (t, *J*=7.4 Hz, 3 H) 2.51 - 2.52 (m, 1 H) 3.03 - 3.10 (m, 2 H) 3.17 (t, *J*=7.4 Hz, 2 H) 3.31 (br. s., 2 H) 3.41 - 3.62 (m, 1 H) 3.78 (s, 3 H) 6.27 (dt, *J*=9.9, 7.0 Hz, 1 H) 6.54 (s, 1 H) 6.85 (d, *J*=9.9 Hz, 1 H) 7.59 (s, 1 H) 7.99 (s, 1 H) 8.56 (br. s., 2 H) 12.72 (br. s., 1 H) 13.93 (br. s., 1 H). LC-MS 392.3 [M-H]⁻, 394.4 [M+H]⁺, RT 0.84 min.

### Example 165

### 10-(2-(ethylamino)ethyl)-4-hydroxy-9-methyl-2-oxo-1,2,5,6,7,9-hexahydropyrido[3',2':6,7]cyclohepta[1,2-f]indole-3-carboxylic acid trifluoroacetate (Cpd 165)

### Step 1: methyl 10-(2-((tert-butoxycarbonyl)(ethyl)amino)ethyl)-1-(2,4-dimethoxybenzyl)-4-hydroxy-9-methyl-2-oxo-1,2,5,6,7,9-hexahydropyrido[3',2':6,7]cyclohepta[1,2-f]indole-3-carboxylate

A solution of methyl 10-(2-((tert-butoxycarbonyl)(ethyl)amino)ethyl)-1-(2,4-dimethoxybenzyl)-4-hydroxy-9-methyl-2-oxo-1,2,5,9-tetrahydropyrido[3',2':6,7]cyclohepta[1,2-f]indole-3-carboxylate (Example 164, step 12-13, 0.175 g, 0.27 mmol) in EtOAc (2.0 mL) was hydrogenated under H₂ (1 atm) over Pd/C (10%, 20 mg) until complete conversion was observed (∼5-6 h). The catalyst was filtered off and the filtrate was washed with EtOAc. The mother liquor was concentrated affording the product (0.155 g, 89%) as a yellowish solid.
¹H NMR (500 MHz, CHCl₃-*d*) δ ppm 1.12 (t, *J*=6.9 Hz, 3 H) 1.32 - 1.56 (m, 11 H) 1.84 - 1.95 (m, 1 H) 1.96 - 2.05 (m, 1 H) 2.32 - 2.48 (m, 1 H) 2.54 - 2.64 (m, 1 H) 2.92 - 3.01 (m, 2 H) 3.25 (br. s., 2 H) 3.37 (s, 3 H) 3.46 (dd, *J*=8.7, 6.1 Hz, 2 H) 3.73 (s, 3 H) 3.76 (s, 3 H) 4.00 (s, 3 H) 5.19 (d, *J*=15.4 Hz, 1 H) 5.24 - 5.30 (m, 1 H) 6.18 (s, 1 H) 6.24 (d, *J*=2.2 Hz, 1 H) 6.34 (dd, *J*=8.2, 2.2 Hz, 1 H) 6.81 (d, *J*=8.2 Hz, 1 H) 7.04 (s, 1 H) 7.30 (s, 1 H). LC-MS 660.8 [M+H]⁺, RT 1.61 min.

### Step 2: 10-(2-((tert-butoxycarbonyl)(ethyl)amino)ethyl)-1-(2,4-dimethoxybenzyl)-4-hydroxy-9-methyl-2-oxo-1,2,5,6,7,9-hexahydropyrido[3',2':6,7]cyclohepta[1,2-f]indole-3-carboxylic acid

To a solution of methyl 10-(2-((tert-butoxycarbonyl)(ethyl)amino)ethyl)-1-(2,4-dimethoxybenzyl)-4-hydroxy-9-methyl-2-oxo-1,2,5,6,7,9-hexahydropyrido[3',2':6,7]cyclohepta[1,2-f]indole-3-carboxylate (0.155 g, 0.24 mmol) in EtOAc (1.5 mL) was added LiI (0.100 g, 0.75 mmol). The reaction mixture was stirred and heated at 60 °C for 1 h until complete consumption of starting material was observed. The mixture was then cooled to room temperature and acidified with aqueous HCl (1M, 0.5 mL). The product was extracted with EtOAc (3x10 mL). The organic phase was washed with Na₂S₂O₃ (10% aq, 5 mL), NaCl (aqueous saturated, 10 mL) and dried over Na₂SO₄. The solvent was removed and the resulting product was obtained as a solid (0.150 g, 98%).
¹H NMR (500 MHz, CHCl₃-*d*) δ ppm 1.12 (t, *J*=7.1 Hz, 3 H) 1.43 (br. s., 9 H) 1.51 - 1.59 (m, 2 H) 1.86 - 2.09 (m, 2 H) 2.24 - 2.40 (m, 1 H) 2.61 (dd, *J*=13.4, 6.1 Hz, 1 H) 2.92 - 3.05 (m, 2 H) 3.25 (br. s., 2 H) 3.44 (s, 3 H) 3.48 (dd, *J*=8.5, 6.3 Hz, 2 H) 3.75 (br. s, 3 H) 3.77 (s, 3 H) 5.23 (d, *J*=15.1 Hz, 1 H) 5.36 (d, *J*=15.1 Hz, 1 H) 6.21 (s, 1 H) 6.29 (d, *J*=2.2 Hz, 1 H) 6.32 (dd, *J*=8.4, 2.2 Hz, 1 H) 6.64 (d, *J*=8.4 Hz, 1 H) 7.08 (s, 1 H) 7.34 (s, 1 H) 13.89 (br. s., 1 H) 16.04 (br. s., 1 H). LC-MS 644.5 [M-H]⁻, 646.6 [M+H]⁺, RT 1.74 min.

### Step 3: 10-(2-(ethylamino)ethyl)-4-hydroxy-9-methyl-2-oxo-1,2,5,6,7,9-hexahydropyrido[3',2':6,7]cyclohepta[1,2-f]indole-3-carboxylic acid trifluoroacetate

To a solution of 10-(2-((tert-butoxycarbonyl)(ethyl)amino)ethyl)-1-(2,4-dimethoxybenzyl)-4-hydroxy-9-methyl-2-oxo-1,2,5,6,7,9-hexahydropyrido[3',2':6,7]cyclohepta[1,2-f]indole-3-carboxylic acid (55 mg, 0.085 mmol) in DCM (1.0 mL) was added i-Pr₃SiH (0.50 mL) followed by TFA (0.50 mL). The mixture was stirred at room temperature and monitored by LC/MS. After complete consumption of starting material (∼8 h), the solvent was removed under reduced pressure. Addition of HCl solution (2M Et₂O, 1.0 mL) to the oily residue resulted in precipitate formation. The mixture was diluted with Et₂O and the resulting solid was filtered and washed with Et₂O. The product HCl salt was obtained as a colorless solid (35.5 mg, 90%). Further purification by preparative HPLC afforded the product 10-(2-(ethylamino)ethyl)-4-hydroxy-9-methyl-2-oxo-1,2,5,6,7,9-hexahydropyrido[3',2':6,7]cyclohepta[1,2-f]indole-3-carboxylic acid (12.1 mg, 29%) as a TFA salt.
¹H NMR (500 MHz, DMSO-*d*₆) δ ppm 1.21 (t, *J*=7.3 Hz, 3 H) 2.02 - 2.16 (m, 2 H) 2.51 - 2.53 (m, 2 H) 2.68 (br. s., 2 H) 3.05 (br. s., 2 H) 3.13 (t, *J*=7.6 Hz, 2 H) 3.29 (br. s., 2 H) 3.75 (s, 3 H) 6.46 (s, 1 H) 7.47 (s, 1 H) 7.70 (s, 1 H) 8.51 (br. s., 2 H) 12.86 (br. s., 1 H) 13.85 (br. s., 1 H). LC-MS 394.3 [M-H]⁻, 396.3 [M+H]⁺, RT 0.88 min.

### Example 166

### 4-hydroxy-9-(((cis)-octahydrocyclopenta[c]pyrrol-4-yl)-(cis)-amino)-2-oxo-1,2,5,6-tetrahydrobenzo[2,3]oxepino[4,5-b]pyridine-3-carboxylic acid hydrochloride (Cpd 166)

The title compound was prepared from methyl 9-chloro-1-(2,4-dimethoxybenzyl)-4-hydroxy-2-oxo-1,2,5,6-tetrahydrobenzo[2,3]oxepino[4,5-b]pyridine-3-carboxylate (Example 76, step 5) and (cis,cis)-tert-butyl 4-aminohexahydrocyclopenta[c]pyrrole-2(1H)-carboxylate according to the two step procedure described for Example 76 (steps 6-7) and subsequent HCl salt exchange with HCl/Et₂O.
¹H NMR (500 MHz, DMSO-*d*₆) δ ppm 1.47 - 1.57 (m, 2 H) 1.96 - 2.11 (m, 2 H) 2.60 - 2.68 (m, 3 H) 2.83 - 2.95 (m, 2 H) 3.02 (m, 1 H) 3.30 (m, 1 H) 3.38 (m, 1 H) 3.77 (m, 1 H) 4.43 (t, *J*=6.5 Hz, 2 H) 6.35 (d, *J*=2.3 Hz, 1 H) 6.51 (dd, *J*=8.7, 2.3 Hz, 1 H) 7.32 (d, *J*=8.7 Hz, 1 H) 9.16 (br. s., 2 H) 12.70 (s, 1 H) 13.79 (s, 1 H) 16.17 (br. s., 1 H). LC-MS 396.0 [M-H]⁻, 398.1 [M+H]⁺, RT 0.86 min.

### Example 167

### 4-hydroxy-9-methyl-10-(((1-methylcyclopropyl)amino)methyl)-2-oxo-2,5,6,9-tetrahydro-1H-pyrido[2',3':4,5]oxepino[3,2-f]indole-3-carboxylic acid hydrochloride (Cpd 167)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 0.70 - 0.77 (m, 2 H) 1.14 - 1.21 (m, 2 H) 1.53 (s, 3 H) 2.59 - 2.66 (m, 2 H) 3.84 (s, 3 H) 4.42 - 4.48 (m, 2 H) 4.49 - 4.55 (m, 2 H) 6.84 (s, 1 H) 7.44 (s, 1 H) 7.86 (s, 1 H) 9.51 (br. s, 1 H) 13.04 (br. s, 1 H) 13.87 (br. s, 1 H). LC-MS: 408.5 [M-H]⁻, RT 0.51 min.

### Example 168

### 10-((sec-butylamino)methyl)-4-hydroxy-9-methyl-2-oxo-2,5,6,9-tetrahydro-1H-pyrido[2',3':4,5]oxepino[3,2-f]indole-3-carboxylic acid hydrochloride (Cpd 168)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 0.95 (t, *J*=7.41 Hz, 3 H) 1.35 (d, *J*=6.62 Hz, 3 H) 1.54 - 1.64 (m, 1 H) 1.89 - 2.01 (m, 1 H) 2.62 (t, *J*=6.30 Hz, 2 H) 3.16 - 3.30 (m, 1 H) 3.84 (s, 3 H) 4.36 - 4.51 (m, 4 H) 6.87 (s, 1 H) 7.44 (s, 1 H) 7.86 (s, 1 H) 9.27 (br. s, 1 H) 9.38 (br. s, 2 H) 13.05 (br. s, 1 H) 13.88 (br. s, 1 H). LC-MS: 410.2 [M-H]⁻, RT 0.94 min.

### Example 169

### 4-hydroxy-9-methyl-2-oxo-10-((propylamino)methyl)-2,5,6,9-tetrahydro-1H-pyrido[2',3':4,5]oxepino[3,2-f]indole-3-carboxylic acid hydrochloride (Cpd 169)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 0.94 (t, *J*=7.40 Hz, 3 H) 1.64 - 1.76 (m, 2 H) 2.60 - 2.66 (m, 2 H) 2.93 - 3.02 (m, 2 H) 3.82 (s, 3 H) 4.40 - 4.48 (m, 4 H) 6.84 (s, 1 H) 7.43 (s, 1 H) 7.86 (s, 1 H) 9.10 (br. s, 1 H) 13.03 (br. s, 1 H) 13.88 (br. s, 1 H). LC-MS: 396.3 [M-H]⁻, RT 0.54 min.

### Example 170

### 10-(((2,4-dimethoxybenzyl)amino)methyl)-4-hydroxy-9-methyl-2-oxo-2,5,6,9-tetrahydro-1H-pyrido[2',3':4,5]oxepino[3,2-f]indole-3-carboxylic acid hydrochloride (Cpd 170)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 2.60 - 2.66 (m, 2 H) 3.77 (s, 3 H) 3.80 (s, 3 H) 3.83 (s, 3 H) 4.10 - 4.17 (m, 2 H) 4.38 - 4.50 (m, 4 H) 6.57 - 6.62 (m, 1 H) 6.63 - 6.67 (m, 1 H) 6.85 (s, 1 H) 7.35 - 7.40 (m, 1 H) 7.44 (s, 1 H) 7.87 (s, 1 H) 9.24 (br. s, 1 H) 13.03 (br. s, 1 H) 13.87 (br. s, 1 H). LC-MS: 506.5 [M+H]⁺, RT 0.56 min.

### Example 171

### 10-((cyclopropylamino)methyl)-4-hydroxy-9-methyl-2-oxo-2,5,6,9-tetrahydro-1H-pyrido[2',3':4,5]oxepino[3,2-f]indole-3-carboxylic acid hydrochloride (Cpd 171)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 0.75 - 0.84 (m, 2 H) 0.90 - 1.00 (m, 3 H) 2.60 - 2.68 (m, 2 H) 3.85 (s, 3 H) 4.41 - 4.48 (m, 2 H) 4.49 - 4.56 (m, 2 H) 6.83 (s, 1 H) 7.44 (s, 1 H) 7.86 (s, 1 H) 9.50 - 9.61 (m, 1 H) 12.96 - 13.11 (m, 1 H) 13.79 - 13.93 (m, 1 H). LC-MS: 394.4 [M-H]⁻, RT 0.50 min.

### Example 172

### 10-((cyclobutylamino)methyl)-4-hydroxy-9-methyl-2-oxo-2,5,6,9-tetrahydro-1H-pyrido[2',3':4,5]oxepino[3,2-f]indole-3-carboxylic acid hydrochloride (Cpd 172)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 1.75 - 1.89 (m, 2 H) 2.16 - 2.30 (m, 4 H) 2.60 - 2.67 (m, 2 H) 3.76 - 3.85 (m, 4 H) 4.28 - 4.36 (m, 2 H) 4.41 - 4.50 (m, 2 H) 6.81 (s, 1 H) 7.44 (s, 1 H) 7.85 (s, 1 H) 9.45 (br. s, 1 H) 13.04 (br. s, 1 H) 13.86 (br. s, 1 H). LC-MS: 408.4 [M-H]⁻, RT 0.51 min.

### Example 173

### 10-((dimethylamino)methyl)-4-hydroxy-9-methyl-2-oxo-2,5,6,9-tetrahydro-1H-pyrido[2',3':4,5]oxepino[3,2-f]indole-3-carboxylic acid hydrochloride (Cpd 173)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 2.61 - 2.66 (m, 2 H) 2.81 (s, 6 H) 3.86 (s, 3 H) 4.40 - 4.51 (m, 2 H) 4.51 - 4.63 (m, 2 H) 6.80 - 7.02 (m, 1 H) 7.43 - 7.52 (m, 1 H) 7.89 (s, 1 H) 10.30 - 10.59 (m, 1 H) 12.85 - 13.21 (m, 1 H) 13.68 - 14.11 (m, 1 H). LC-MS: 382.2 [M-H]⁻, RT 0.92 min.

### Example 174

### 4-hydroxy-9-methyl-2-oxo-10-(pyrrolidin-1-ylmethyl)-2,5,6,9-tetrahydro-1H-pyrido[2',3':4,5]oxepino[3,2-f]indole-3-carboxylic acid hydrochloride (Cpd 174)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 1.85 - 2.14 (m, 4 H) 2.60 - 2.69 (m, 4 H) 3.87 (s, 3 H) 4.37 - 4.55 (m, 4 H) 4.58 - 4.73 (m, 2 H) 6.90 - 6.95 (s, 1 H) 7.42 - 7.49 (s, 1 H) 7.83 - 7.91 (s, 1 H) 10.50 - 10.73 (bs, 1 H) 12.92 - 13.16 (bs, 1 H) 13.81 - 13.99 (bs, 1 H). LC-MS: 408.2 [M-H]⁻, RT 0.95 min.

### Example 175

### 10-((tert-butylamino)methyl)-4-hydroxy-9-methyl-2-oxo-2,5,6,9-tetrahydro-1H-pyrido[2',3':4,5]oxepino[3,2-f]indole-3-carboxylic acid hydrochloride (Cpd 175)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 1.42 (s, 9 H) 2.60 - 2.67 (m, 2 H) 3.82 (s, 3 H) 4.37 - 4.50 (m, 4 H) 6.80 (s, 1 H) 7.46 (s, 1 H) 7.88 (s, 1 H) 8.95 (br. s, 1 H) 13.06 (br. s, 1 H) 13.88 (br. s, 1 H). LC-MS: 410.2 [M-H]⁻, RT 0.96 min.

### Example 176

### 4-hydroxy-9-methyl-10-((4-methylpiperazin-1-yl)methyl)-2-oxo-2,5,6,9-tetrahydro-1H-pyrido[2',3':4,5]oxepino[3,2-f]indole-3-carboxylic acid dihydrochloride (Cpd 176)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 2.60 - 2.66 (m, 2 H) 2.74 - 2.81 (m, 4 H) 3.02 - 3.30 (m, 4 H) 3.79 - 3.87 (m, 6 H) 4.41 - 4.48 (m, 4 H) 6.69 (s, 1 H) 7.40 (s, 1 H) 7.80 (s, 1 H) 12.95 - 13.06 (m, 1 H) 13.81 - 13.94 (m, 1 H). LC-MS: 439.3 [M+H]⁺, RT 0.98 min.

### Example 177

### 4-hydroxy-10-(((2-hydroxyethyl)amino)methyl)-9-methyl-2-oxo-2,5,6,9-tetrahydro-1H-pyrido[2',3':4,5]oxepino[3,2-f]indole-3-carboxylic acid hydrochloride (Cpd 177)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 2.61 - 2.67 (m, 2 H) 3.07 - 3.15 (m, 2 H) 3.71 - 3.75 (m, 2 H) 3.82 (s, 3 H) 4.42 - 4.49 (m, 4 H) 6.85 (s, 1 H) 7.44 (s, 1 H) 7.86 (s, 1 H) 9.16 (br. s, 1 H) 9.55 (br. s, 1 H) 13.02 (br. s, 1 H) 13.87 (br. s, 1 H). LC-MS: 400.2 [M+H]⁺, RT 0.50 min.

### Example 178

### 4-hydroxy-10-(((1-hydroxypropan-2-yl)amino)methyl)-9-methyl-2-oxo-2,5,6,9-tetrahydro-1H-pyrido[2',3':4,5]oxepino[3,2-f]indole-3-carboxylic acid hydrochloride (Cpd 178)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 1.31 (d, *J*=6.94 Hz, 3 H) 2.63 (s, 2 H) 3.57 - 3.67 (m, 1 H) 3.71 - 3.78 (m, 1 H) 3.84 (s, 3 H) 4.39 - 4.51 (m, 4 H) 6.86 (s, 1 H) 7.44 (s, 1 H) 7.86 (s, 1 H) 9.05 (br. s, 1 H) 9.23 (br. s, 1 H) 13.03 (br. s, 1 H) 13.87 (br. s, 1 H). LC-MS: 414.2 [M+H]⁺, RT 0.50 min.

### Example 179

### 4-hydroxy-9-methyl-2-oxo-10-(((2-(pyrrolidin-1-yl)ethyl)amino)methyl)-2,5,6,9-tetrahydro-1H-pyrido[2',3':4,5]oxepino[3,2-f]indole-3-carboxylic acid dihydrochloride (Cpd 179)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 1.86 - 1.96 (m, 2 H) 1.97 - 2.09 (m, 2 H) 2.55 - 2.60 (m, 2 H) 2.96 - 3.13 (m, 4 H) 3.54 - 3.70 (m, 4 H) 3.85 (s, 3 H) 4.42 - 4.49 (m, 2 H) 4.50 - 4.57 (m, 2 H) 6.88 (s, 1 H) 7.44 (s, 1 H) 7.87 (s, 1 H) 10.89 (br. s, 1 H) 13.05 (br. s, 1 H) 13.87 (br. s, 1 H). LC-MS: 453.2 [M+H]⁺, RT 0.48 min.

### Example 180

### 10-(((2-aminoethyl)amino)methyl)-4-hydroxy-9-methyl-2-oxo-2,5,6,9-tetrahydro-1H-pyrido[2',3':4,5]oxepino[3,2-f]indole-3-carboxylic acid dihydrochloride (Cpd 180)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 2.62 (t, *J*=5.99 Hz, 2 H) 3.21 - 3.33 (m, 4 H) 3.86 (s, 3 H) 4.44 (t, *J*=5.99 Hz, 2 H) 4.50 (br. s., 2 H) 6.87 (s, 1 H) 7.44 (s, 1 H) 7.85 (s, 1 H) 8.33 (br. s., 3 H) 9.93 (br. s., 2 H) 13.03 (br. s., 1 H) 13.86 (br. s., 1 H). LC-MS 397.3 [M-H]⁻, 399.3 [M+H]⁺, RT 0.41 min.

### Example 181

### 4-hydroxy-9-methyl-10-(((2-(methylamino)ethyl)amino)methyl)-2-oxo-2,5,6,9-tetrahydro-1H-pyrido[2',3':4,5]oxepino[3,2-f]indole-3-carboxylic acid dihydrochloride (Cpd 181)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 2.61 (s, 3 H) 2.62 (t, *J*=6.31 Hz, 2 H) 3.35 - 3.45 (m, 4 H) 3.86 (s, 3 H) 4.44 (t, *J*=6.31 Hz, 2 H) 4.50 (br. s., 2 H) 6.89 (s, 1 H) 7.44 (s, 1 H) 7.85 (s, 1 H) 9.35 (br. s., 2 H) 9.96 (br. s., 2 H) 13.04 (br. s., 1 H) 13.86 (br. s., 1 H). LC-MS 411.3 [M-H]⁻, 413.3 [M+H]⁺, RT 0.41 min.

### Example 182

### 10-(((2-(dimethylamino)ethyl)amino)methyl)-4-hydroxy-9-methyl-2-oxo-2,5,6,9-tetrahydro-1H-pyrido[2',3':4,5]oxepino[3,2-f]indole-3-carboxylic acid dihydrochloride (Cpd 182)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 2.62 (t, *J*=6.3 Hz, 2 H) 2.85 (s, 6 H) 3.37 - 3.55 (m, 4 H) 3.85 (s, 3 H) 4.45 (t, *J*=6.3 Hz, 2 H) 4.49 (br. s., 2 H) 6.87 (s, 1 H) 7.44 (s, 1 H) 7.86 (s, 1 H) 9.75 (br. s., 2 H) 10.68 (br. s., 1 H) 13.02 (br. s., 1 H) 13.87 (br. s., 1 H). LC-MS 425.2 [M-H]⁻, 427.3 [M+H]⁺, RT 0.42 min.

### Example 183

### 4-hydroxy-10-(((2-methoxyethyl)amino)methyl)-9-methyl-2-oxo-2,5,6,9-tetrahydro-1H-pyrido[2',3':4,5]oxepino[3,2-f]indole-3-carboxylic acid hydrochloride (Cpd 183)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 2.62 (t, *J*=6.31 Hz, 2 H) 3.13 - 3.24 (m, 2 H) 3.33 (s, 3 H) 3.60 - 3.72 (m, 2 H) 3.82 (s, 3 H) 4.43 (br. s., 2 H) 4.44 (t, *J*=6.31 Hz, 2 H) 6.85 (s, 1 H) 7.42 (s, 1 H) 7.85 (s, 1 H) 9.39 (br. s., 2 H) 13.03 (br. s., 1 H) 13.87 (br. s., 1 H). LC-MS 412.3 [M-H]⁻, 414.3 [M+H]⁺, RT 0.49 min. (1min Method).

### Example 184

### 4-hydroxy-10-(((1-methoxypropan-2-yl)amino)methyl)-9-methyl-2-oxo-2,5,6,9-tetrahydro-1H-pyrido[2',3':4,5]oxepino[3,2-f]indole-3-carboxylic acid hydrochloride (Cpd 184)

The title compound was prepared from 1-(2,4-dimethoxybenzyl)-10-formyl-4-hydroxy-9-methyl-2-oxo-2,5,6,9-tetrahydro-1H-pyrido[2',3':4,5]oxepino[3,2-f]indole-3-carboxylic acid (Example 88, step 16) according to the two step procedure described for Example 88 (steps 17-18).
¹H NMR (500 MHz, DMSO-*d*₆) δ ppm 1.32 (d, *J*=6.62 Hz, 3 H) 2.58 - 2.67 (m, 2 H) 3.36 (s, 3 H) 3.49 - 3.62 (m, 2 H) 3.62 - 3.69 (m, 1 H) 3.81 (s, 3 H) 4.44 (br. s., 2 H) 4.44 (t, *J*=6.15 Hz, 2 H) 6.84 (s, 1 H) 7.43 (s, 1 H) 7.85 (s, 1 H) 9.11 (br. s., 1 H) 9.25 (br. s., 1 H) 13.03 (br. s., 1 H) 13.86 (br. s., 1 H). LC-MS 426.3 [M-H]⁻, 428.3 [M+H]⁺, RT 0.51 min.

### Example 185

### 9-(dimethylamino)-4-hydroxy-2-oxo-1,2,5,6-tetrahydrobenzo[2,3]thiepino[4,5-b]pyridine-3-carboxylic acid) (Cpd 185)

### Step 1: ethyl 4-((3-bromophenyl)thio)butanoate

To a solution of 3-bromothiophenol (50 g, 265 mmol) in dry DMF (500 mL), at 0 °C was added 60% NaH in mineral oil (12.7 g, 318 mmol). The mixture was stirred for 10 min, then ethyl 4-bromobutanoate (45.5 mL, 318 mmol) was added. The cooling bath was then removed and the mixture was stirred overnight. The mixture was then diluted with water (1.5 L) and extracted with ethyl ether (3 x 300 mL). The extracts were combined and washed with water (2 x 100 mL) and brine (100 mL) and dried over anhydrous Na₂SO₄. The solvent was removed in vacuo to furnish ethyl 4-((3-bromophenyl)thio)butanoate as a light brown oil, used directly in the next step.
¹H NMR (500 MHz, CHCl₃-*d*) δ ppm 7.46 (1 H, t, *J*=1.89 Hz), 7.29 - 7.33 (1 H, m), 7.24 - 7.27 (1 H, m), 7.13 - 7.17 (1 H, m), 4.15 (1 H, q, *J*=7.25 Hz), 2.98 (2 H, t, *J*=7.25 Hz), 2.47 (2 H, t, *J*=7.25 Hz), 1.97 (2 H, quin, *J*=7.17 Hz), 1.27 (3 H, tt, *J*=6.94, 1.00 Hz).

### Step 2: 8-bromo-3,4-dihydrobenzo[b]thiepin-5(2H)-one

To stirred PPA, pre-heated to 120 °C (600 g) was added the intermediate obtained in Step 1. The mixture was mixed at 120 °C for 2 h and then poured onto ice (1000 mL) and stirred for 0.5 h. The organic phase was extracted with DCM (3 x 300 mL) and the DCM extracts were combined, then washed with water (2 x 200 mL) and aqueous saturated NaHCO₃ (300 mL) and dried over Na₂SO₄. The solvent was removed on a rotovap and the residue was chromatographed (silica gel, ethyl acetate in hexanes, 0-30% gradient) to provide a benzothiepinone intermediate as a light brown oil (39.9 g, 59%, 2 steps).
¹H NMR (500 MHz, CHCl₃-*d*) δ ppm 7.71 (1 H, d, *J*=8.51 Hz), 7.66 (1 H, d, *J*=1.89 Hz), 7.38 (1 H, dd, *J*=8.51, 1.89 Hz), 3.04 (1 H, t, *J*=13.20 Hz), 2.99 (2 H, t, *J*=6.78 Hz), 2.28 (2 H, quin, *J*=6.78 Hz).

### Step 3: methyl 9-bromo-1-(2,4-dimethoxybenzyl)-4-hydroxy-2-oxo-1,2,5,6-tetrahydrobenzo [2,3] thiepino [4,5-b] pyridine-3-carboxylate

To a solution of benzothiepinone obtained in Step 2 (16.1 g, 62.6 mmol) in DCM (120 mL), 2,4-dimethoxybenzylamine (11.5 g, 68.9 mmol) and triethylamine (19.0 g, 187.8 mmol) was added, at 0 °C, a solution of TiCl₄ (37.6 mL, 1.0 M in DCM). The mixture was stirred 10 min. at 0 °C and overnight at room temperature, and the reaction was quenched with saturated NaHCO₃ (20 mL). The mixture was stirred for an additional 0.5 h, then filtered using a Phase Separator cartridge. The organic layer was evaporated to dryness on a rotovap. 2,4-dimethoxybenzylamine (23.8 g, 125.2 mmol) and diphenyl ether (100 mL) were added to the residue, which was heated to 230 °C with stirring for 10 min. and then cooled under nitrogen. Column chromatography of the reaction mixture (silica gel, ethyl acetate in hexanes 0-100 %) provided an intermediate as a brown solid (22.2 g, 67%).
¹H NMR (500 MHz, CHCl₃-*d*) δ ppm 7.72 (1 H, d, *J*=1.89 Hz), 7.44 - 7.47 (1 H, m), 7.04 (1 H, d, *J*=8.51 Hz), 6.82 (1 H, d, *J*=8.51 Hz), 6.34 (1 H, dd, *J*=8.35, 2.36 Hz), 6.26 (1 H, d, *J*=2.21 Hz), 5.22 (1 H, d, *J*=15.76 Hz), 4.93 (1 H, d, *J*=16.08 Hz), 4.02 (3 H, s), 3.76 (3 H, s), 3.57 (3 H, s), 3.37 - 3.42 (1 H, m), 3.30 - 3.36 (1 H, m), 3.01 (1 H, ddd, *J*=13.56,11.51, 5.20 Hz), 1.70 (2 H, td, *J*=14.03, 5.67 Hz). LC-MS 530.1 and 533.1 [M-H]⁻, RT 1.49 min.

### Step 4: 9-(dimethylamino)-4-hydroxy-2-oxo-1,2,5,6-tetrahydrobenzo[2,3]thiepino[4,5-b]pyridine-3-carboxylic acid

An oven-dried vial was charged with the intermediate obtained above (319 mg, 0.6 mmol), 2-(2'-di-*tert*-butylphosphine)biphenylpalladium(II) acetate (3 mg, 0.006 mmol), and *t*-BuONa (173 mg, 0.18 mmol). The vial was evacuated then back filled with Argon, followed by the addition of toluene (3.0 mL) and N,N-dimethyl amine (1.2 mL, 2.0 M in THF). The mixture was heated to 80 °C overnight. The reaction mixture was then cooled to room temperature, poured into 1M HCl and extracted with DCM. The combined organic phase was dried over Na₂SO₄, then filtered and concentrated. The residue was dissolved in DCM and triturated with Et₂O. The solid was filtered to afford a semi-pure product which was dissolved in TFA (1 mL) and stirred at room temperature for 1 hr, then MeOH (3 mL) was added and the resulting precipitate was filtered to afford the title compound, 9-(dimethylamino)-4-hydroxy-2-oxo-1,2,5,6-tetrahydrobenzo[2,3]thiepino[4,5-b]pyridine-3-carboxylic acid.
¹H NMR (500 MHz, DMSO-*d*₆) δ ppm 16.18 (1 H, br. s), 13.79 (1 H, br. s), 12.78 (1 H, br. s), 7.42 (1 H, d, *J*=8.83 Hz), 6.96 (1 H, d, *J*=2.84 Hz), 6.84 (1 H, dd, *J*=8.83, 2.84 Hz), 3.34 (4 H, br. s), 3.01 (6 H, s). LC-MS: 333.2 [M+H]⁺, RT 1.25 min.

### Example 186

### 4-hydroxy-2-oxo-9-(pyrrolidin-1-yl)-1,2,5,6-tetrahydrobenzo[2,3]thiepino[4,5-b]pyridine-3-carboxylic acid (Cpd 186)

The title compound was prepared starting from methyl 9-bromo-1-(2,4-dimethoxybenzyl)-4-hydroxy-2-oxo-1,2,5,6-tetrahydrobenzo[2,3]thiepino[4,5-b]pyridine-3-carboxylate (Example 185, step 3) and pyrrolidine by the two step procedure described for Example 185, step 4.
¹H NMR (500 MHz, DMSO-*d*₆) δ ppm 16.18 (1 H, br. s), 13.77 (1 H, s), 12.77 (1 H, br. s), 7.40 (1 H, d, *J*=8.51 Hz), 6.81 (1 H, d, *J*=2.52 Hz), 6.66 (1 H, dd, *J*=8.51, 2.52 Hz), 3.36 (4 H, br. s.), 3.31 (4 H, m), 1.97 (4 H, m). LC-MS: 359.2 [M+H]⁺, RT 1.35 min.

### Biological Examples

The following biological examples demonstrate the usefulness of the compounds of the present description for treating bacterial infections.

### Example 1

The antibacterial activity from a microbroth dilution method may be presented as the minimum inhibitory concentration (MIC in µg/mL). The MIC value is the lowest concentration of drug which prevents macroscopically visible growth under test conditions.

In the following tables, an MIC value between > 12.5 µg/mL and ≤ 150 µg/mL is indicated by a single star (*), an MIC value between > 3.5 µg/mL and ≤ 12.5 µg/mL is indicated by two stars (**), an MIC value between > 1.0 µg/mL and ≤ 3.5 µg/mL is indicated by three stars (***) and an MIC value of ≤ 1.0 µg/mL is indicated by four stars (****). The term ND indicates that the MIC value was Not Determined.

Antibacterial activity of test compounds against the super sensitive Gram-negative *Escherichia coli* (*E. coli*) BAS849 bacterium, the control Gram-negative *E. coli* 25922 strain and the Gram-positive *Staphylococcus aureus* (*S. aureus*) 29213 bacterium are shown in Table 1.

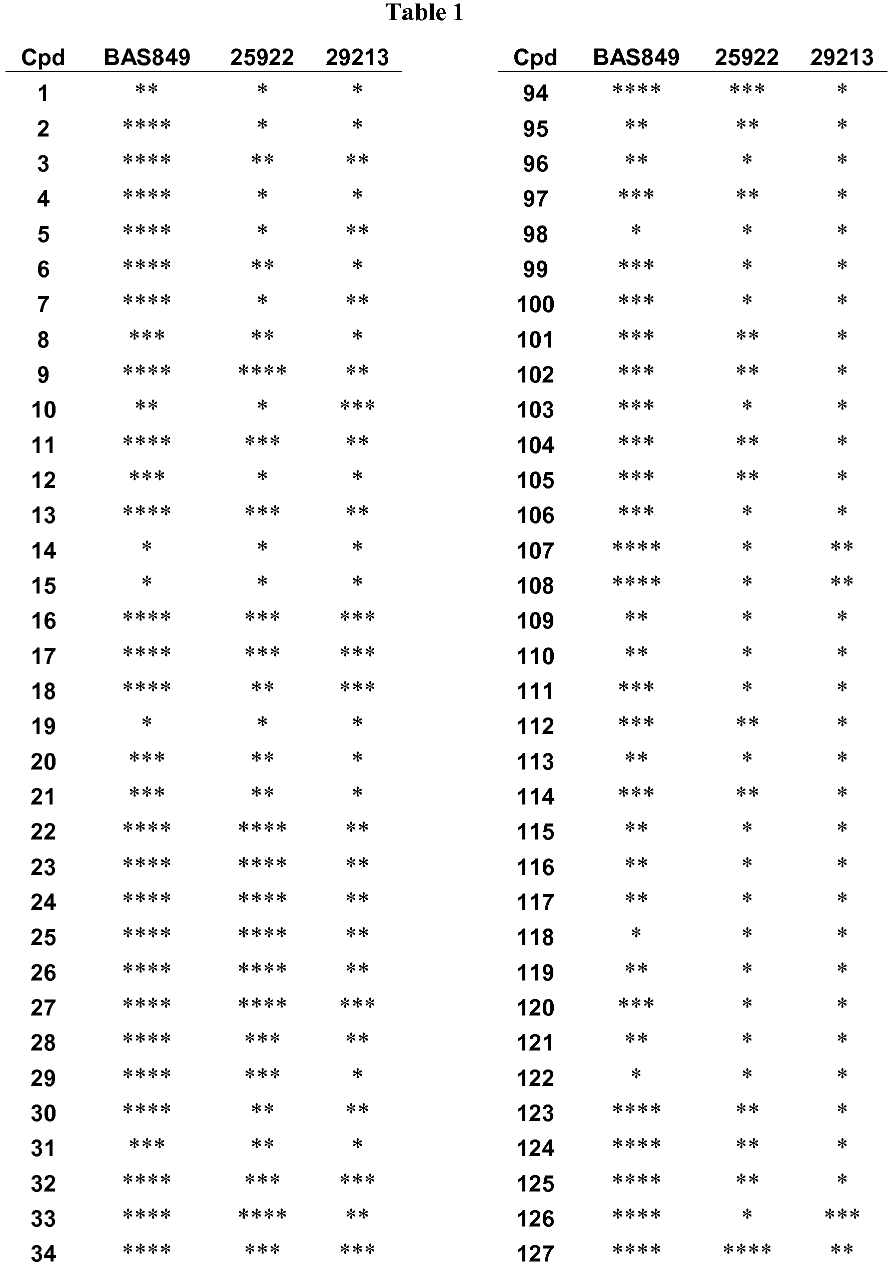

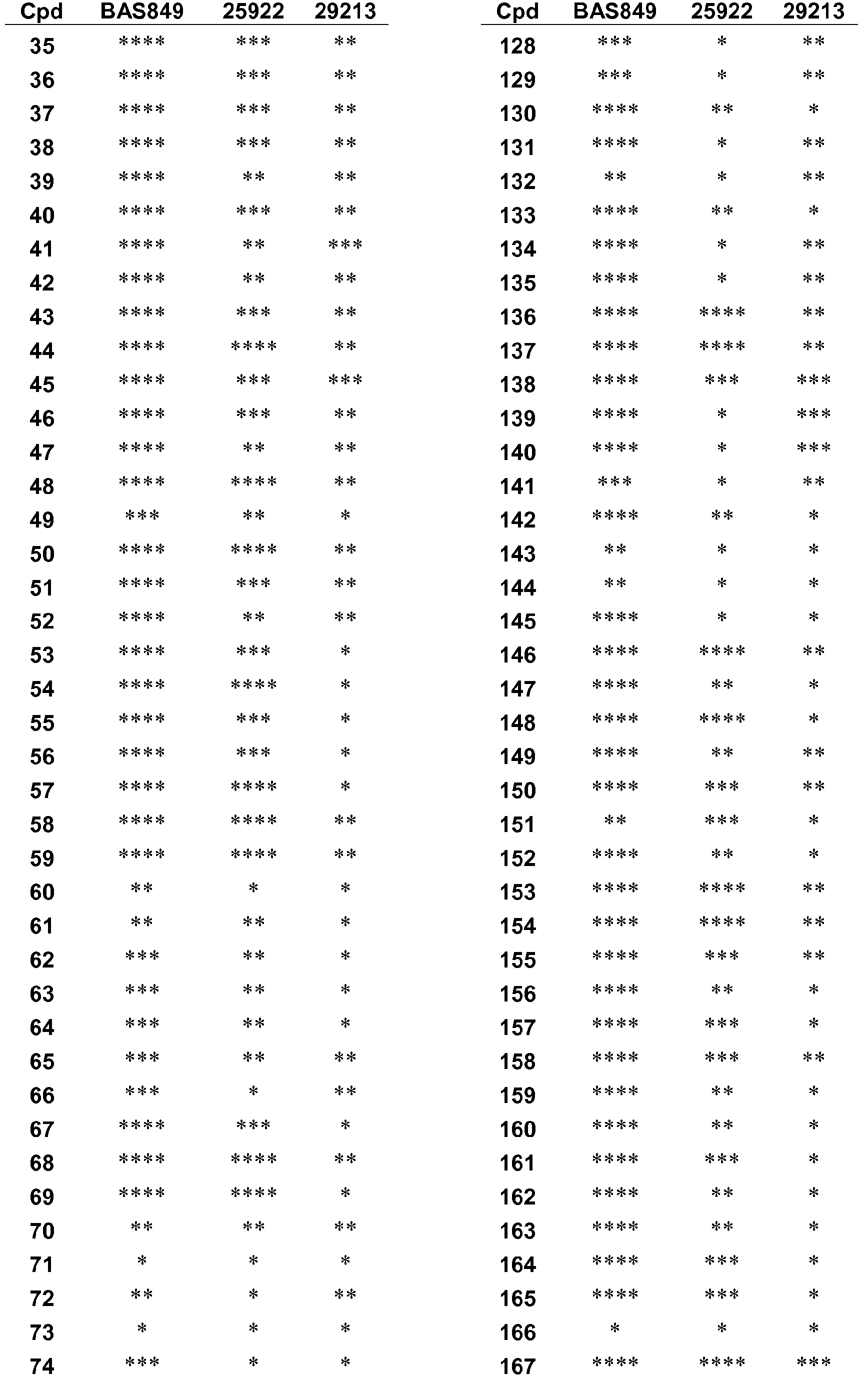

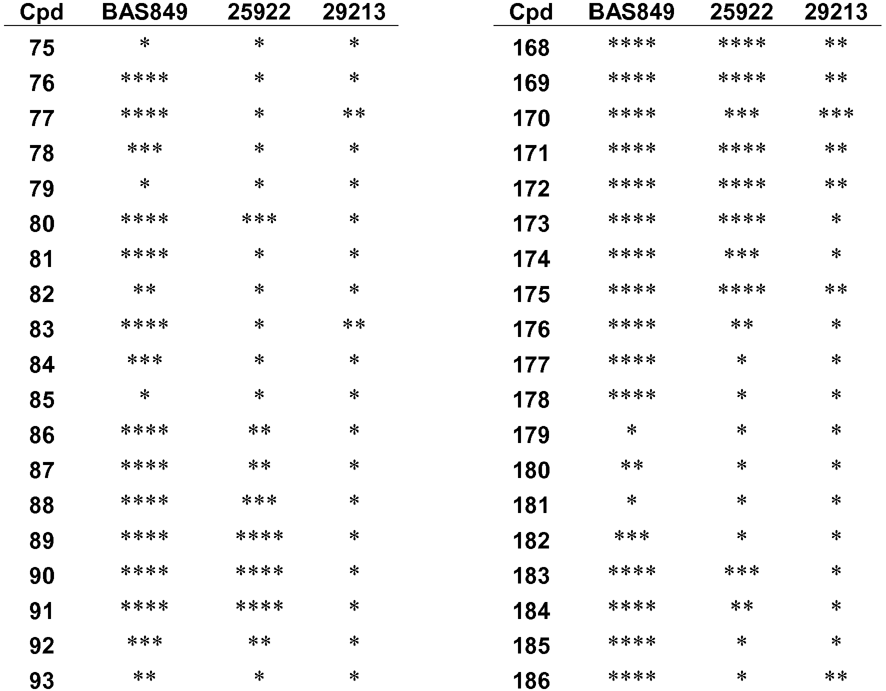

### Example 2

Antibacterial activity of test compounds against the quinolone resistant *E. coli* LZ3111 bacterium is shown in Table 2. The *E. coli* LZ3111 strain is genetically engineered and possesses double mutations in both the gyrase A and par C regions of the topoisomerase subunits. Mutations in these regions are known to confer a high level resistance to the quinolone class of antibiotics.

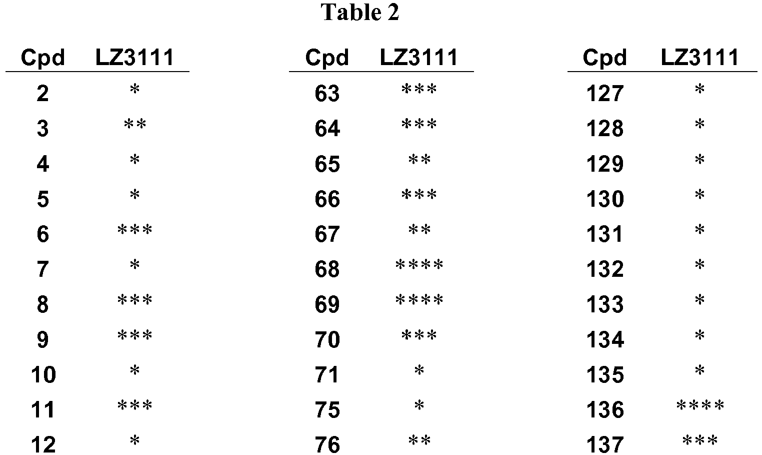

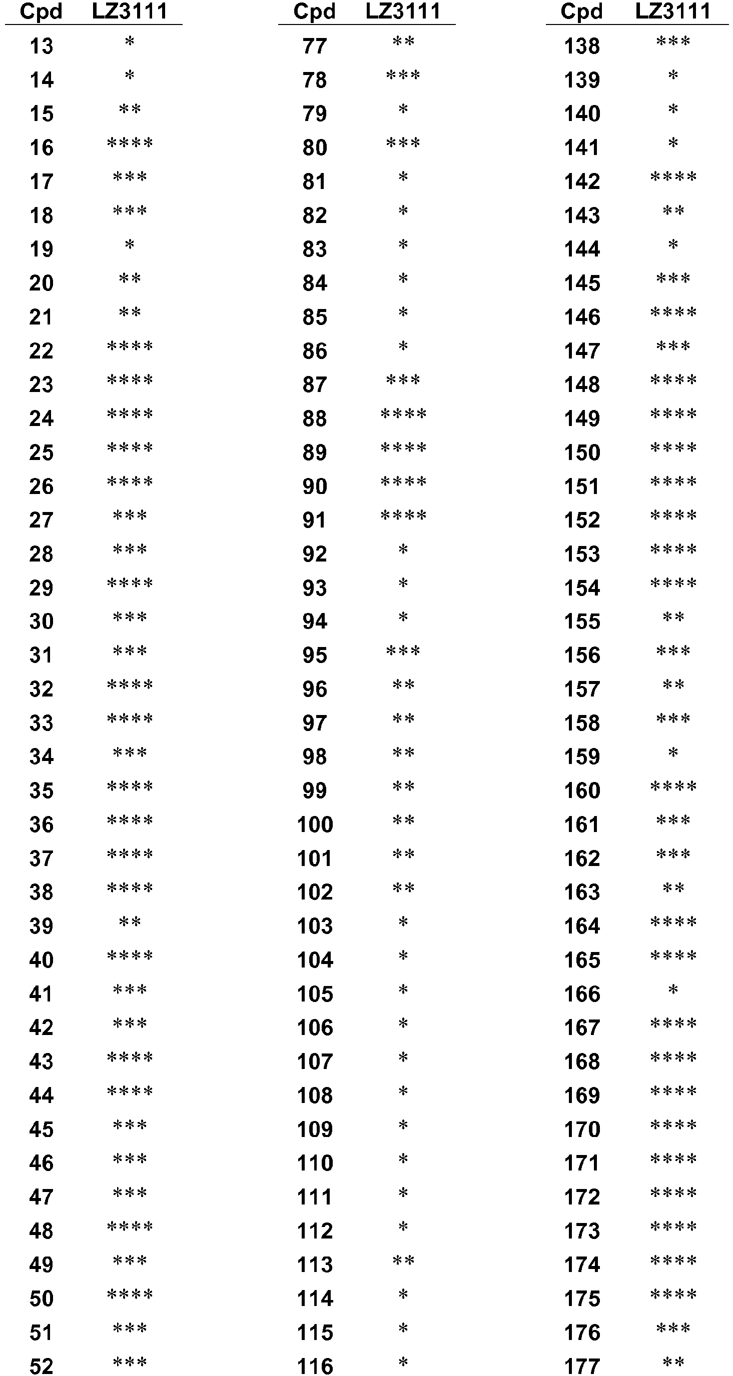

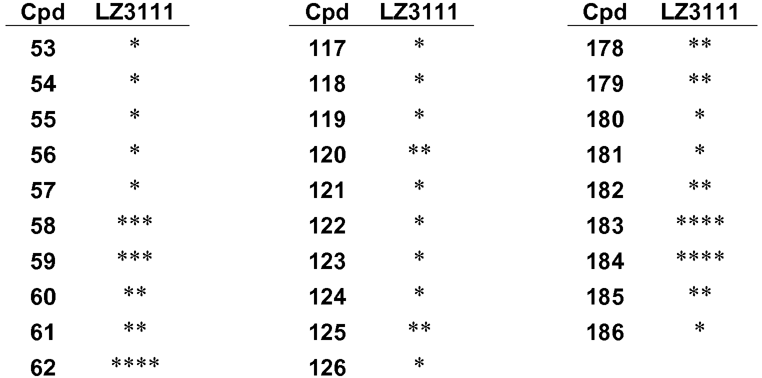

### Example 3

Antibacterial activity of test compounds against *E. coli* multi-drug resistant clinical isolates are provided in Table 3 and Table 4. The activity for the *E. coli* ELZ4251 strain is provided in Table 3 and the *E. coli* ELZ4000 strain is provided in Table 4.

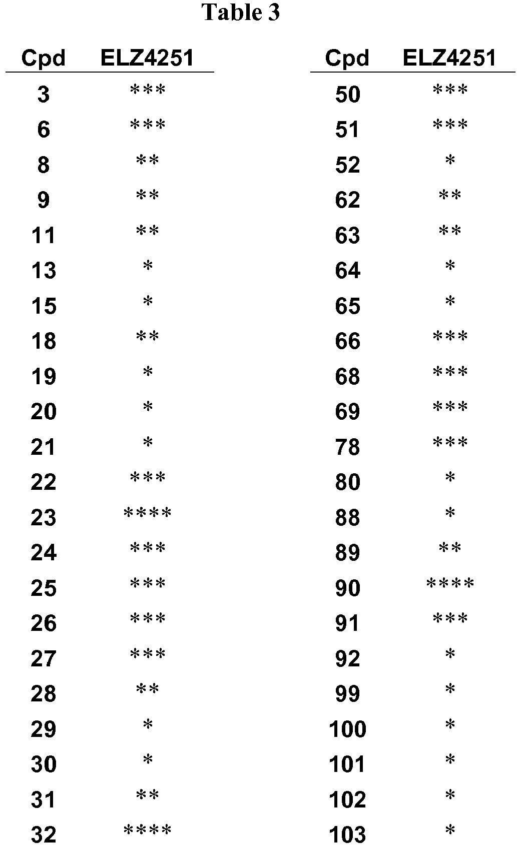

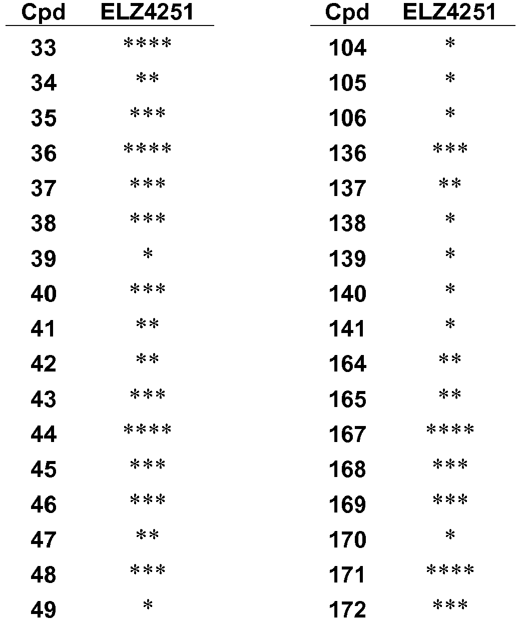

**Table 4**

| **Cpd** | **ELZ4000** |
|---|---|
| **3** | ** |
| **13** | ** |
| **15** | * |
| **22** | *** |
| **23** | *** |
| **24** | *** |
| **25** | *** |
| **26** | *** |
| **27** | *** |
| **78** | ** |
| **80** | * |
| **88** | ** |
| **89** | ** |
| **91** | *** |

### Example 4

Antibacterial activity of test compounds against the Gram-negative *Acinetobacter baumannii* (*A. baumannii*) BAA747 and *Klebsiella pneumoniae* (*K. pneumoniae*) 35657 bacterium and the resistant *A. baumannii* MXX2240 and *K. pneumoniae* MXX1232 strains are shown in Table 5. The MMX strains are multi-drug resistant clinical isolates.

**Table 5**

| **Cpd** | **BAA747** | **MMX2240** | **35657** | **MMX1232** |
|---|---|---|---|---|
| **3** | ** | *** | ND | * |
| **6** | ** | *** | ** | * |
| **8** | * | ** | *** | * |
| **9** | ND | ** | ND | * |
| **10** | ND | * | ND | * |
| **11** | ND | * | ND | * |
| **13** | * | * | *** | * |
| **14** | * | * | * | * |
| **15** | * | * | * | * |
| **16** | *** | *** | *** | * |
| **17** | *** | *** | *** | * |
| **18** | ** | *** | * | * |
| **22** | *** | *** | **** | ** |
| **23** | **** | *** | **** | * |
| **24** | **** | *** | *** | * |
| **25** | **** | *** | **** | * |
| **26** | **** | **** | **** | ** |
| **27** | **** | **** | **** | * |
| **28** | *** | ** | *** | * |
| **29** | * | * | *** | * |
| **30** | ** | * | *** | * |
| **31** | * | * | *** | * |
| **32** | **** | **** | **** | ** |
| **33** | *** | **** | *** | * |
| **34** | *** | **** | *** | * |
| **35** | *** | *** | **** | ** |
| **36** | *** | **** | *** | * |
| **37** | *** | **** | **** | * |
| **38** | *** | *** | **** | * |
| **39** | ** | * | ** | * |
| **40** | *** | **** | **** | * |
| **41** | ** | *** | ** | * |
| **42** | ** | ** | *** | * |
| **43** | *** | *** | *** | * |
| **44** | *** | **** | **** | * |
| **45** | *** | *** | *** | * |
| **46** | ** | ** | *** | * |
| **47** | ** | * | *** | * |
| **48** | *** | *** | **** | * |
| **49** | * | * | *** | * |
| **50** | **** | *** | **** | * |
| **51** | *** | *** | **** | * |
| **52** | ** | ** | *** | * |
| **58** | **** | *** | **** | * |
| **59** | **** | ** | *** | * |
| **60** | * | * | * | * |
| **61** | ** | * | * | * |
| **62** | ** | *** | *** | * |
| **63** | ** | ** | *** | * |
| **64** | ** | *** | ** | * |
| **65** | *** | ** | ** | * |
| **66** | ** | ** | ** | * |
| **68** | *** | *** | **** | * |
| **69** | *** | *** | **** | * |
| **71** | * | * | * | * |
| **78** | ND | ND | ** | ND |
| **80** | * | * | **** | * |
| **81** | ND | * | ND | * |
| **82** | ND | * | ND | * |
| **83** | * | * | * | * |
| **84** | * | * | * | * |
| **85** | * | * | * | * |
| **86** | * | * | ** | * |
| **87** | ** | *** | *** | * |
| **88** | ** | * | *** | * |
| **89** | ** | ** | ** | * |
| **90** | ** | ** | *** | * |
| **91** | *** | *** | ** | * |
| **94** | *** | * | ** | * |
| **95** | * | * | ** | * |
| **96** | * | * | * | * |
| **97** | * | * | ** | * |
| **98** | * | * | * | * |
| **99** | * | * | ** | * |
| **100** | * | * | ** | * |
| **101** | * | * | ** | * |
| **102** | * | * | *** | * |
| **103** | * | * | ** | * |
| **104** | * | * | ** | * |
| **105** | * | * | *** | * |
| **106** | * | * | * | * |
| **107** | * | * | * | * |
| **108** | * | * | * | * |
| **109** | * | * | * | * |
| **110** | * | * | * | * |
| **111** | * | * | ** | * |
| **112** | * | * | * | * |
| **113** | * | * | ** | * |
| **114** | * | * | ** | * |
| **115** | * | * | * | * |
| **116** | * | * | * | * |
| **117** | * | * | * | * |
| **118** | * | * | * | * |
| **119** | * | * | * | * |
| **120** | * | * | * | * |
| **121** | * | * | * | * |
| **122** | * | * | * | * |
| **123** | * | * | ** | * |
| **124** | * | * | ** | * |
| **125** | ** | * | * | * |
| **126** | * | * | * | * |
| **127** | **** | * | **** | * |
| **128** | * | * | * | * |
| **129** | * | * | * | * |
| **130** | * | ** | ** | * |
| **131** | * | * | * | * |
| **132** | * | * | * | * |
| **133** | * | * | ** | * |
| **134** | * | * | * | * |
| **135** | * | * | * | * |
| **136** | **** | **** | **** | * |
| **137** | **** | **** | *** | * |
| **138** | ** | ** | *** | * |
| **139** | * | * | * | * |
| **140** | * | * | * | * |
| **141** | * | * | * | * |
| **142** | * | * | *** | * |
| **143** | * | * | * | * |
| **144** | * | * | * | * |
| **145** | * | * | ** | * |
| **146** | **** | **** | **** | * |
| **147** | * | * | *** | * |
| **148** | *** | **** | **** | * |
| **149** | ** | ** | **** | * |
| **150** | ** | ** | *** | * |
| **151** | ** | * | *** | * |
| **152** | * | * | *** | * |
| **153** | **** | **** | **** | * |
| **154** | *** | **** | **** | * |
| **155** | ** | ** | *** | * |
| **156** | ** | * | ** | * |
| **157** | * | * | ** | * |
| **158** | *** | *** | *** | * |
| **159** | * | * | ** | * |
| **160** | * | * | ** | * |
| **161** | *** | * | ** | * |
| **162** | ** | * | ** | * |
| **163** | ** | * | ** | * |
| **164** | * | * | ** | * |
| **165** | * | * | ** | * |
| **166** | * | * | * | * |
| **167** | **** | **** | ** | * |
| **168** | *** | **** | **** | * |
| **169** | *** | *** | **** | * |
| **170** | *** | *** | ** | * |
| **171** | *** | **** | *** | * |
| **172** | *** | *** | *** | * |
| **173** | *** | ** | **** | * |
| **174** | ** | ** | *** | * |
| **175** | *** | *** | **** | * |
| **176** | * | * | ** | * |
| **177** | * | * | * | * |
| **178** | * | * | ** | * |
| **179** | * | * | * | * |
| **180** | * | * | * | * |
| **181** | * | * | * | * |
| **182** | * | * | * | * |
| **183** | ** | ** | *** | * |
| **184** | ** | ** | **** | * |
| **185** | ** | ** | * | * |
| **186** | * | * | * | * |

### Example 5

Antibacterial activity of test compounds against the Gram-negative bacterium *Pseudomonas aeruginosa* (*P. aeruginosa*) 27853 is shown in Table 6.

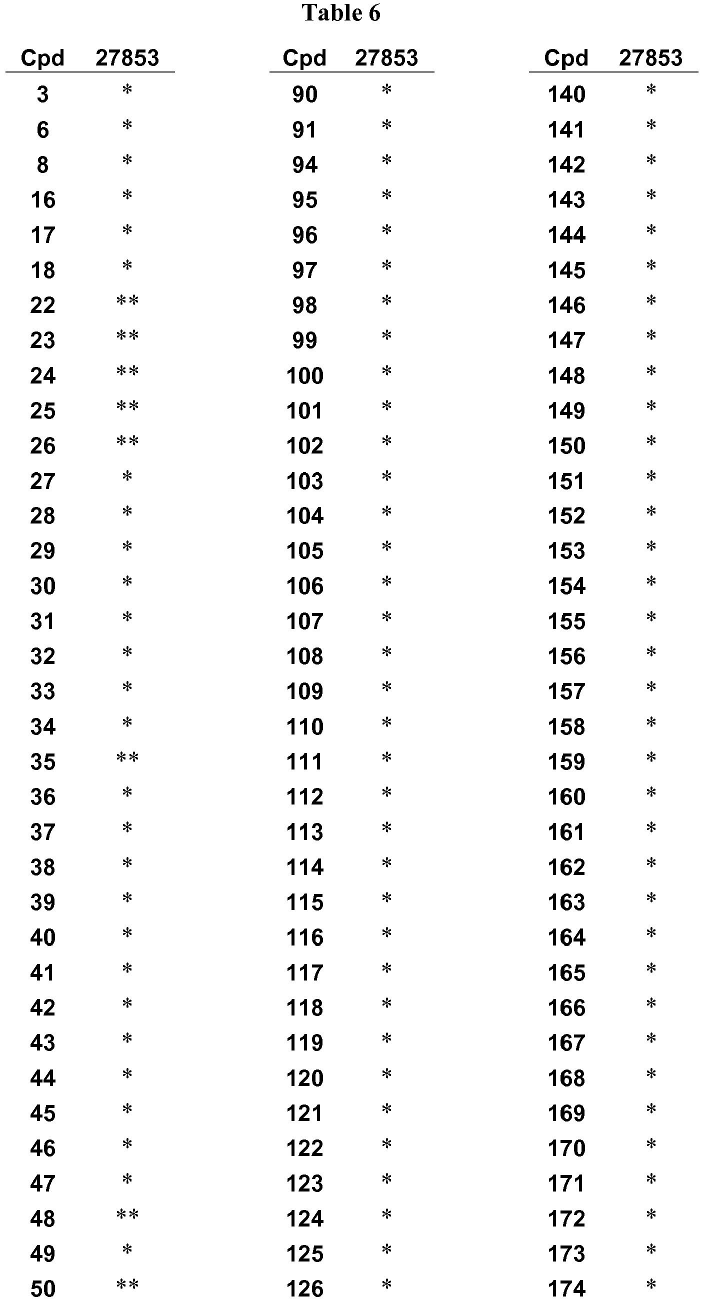

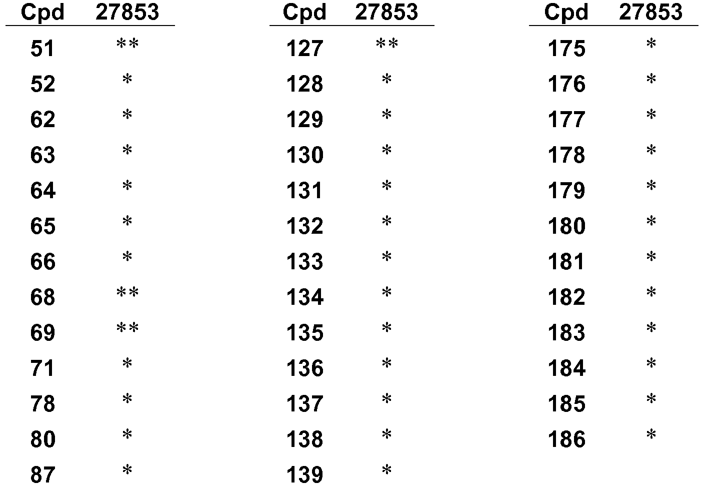

### Example 6

Antibacterial activity of test compounds against the Gram-negative bacteria *Haemophilus influenzae* (*H. influenzae*) 49247 is shown in Table 7, *Moraxella catarrhalis* (*M. catarrhalis*) 25238 is shown in Table 8 and *Neisseria meningitidis* (*N. meningitidis*) 13090 is shown in Table 9.

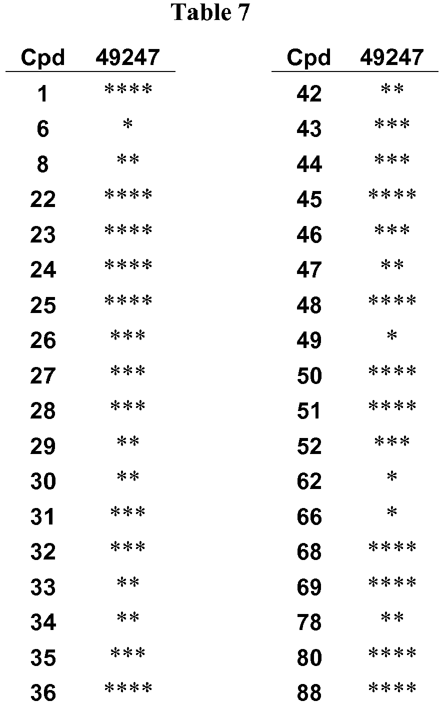

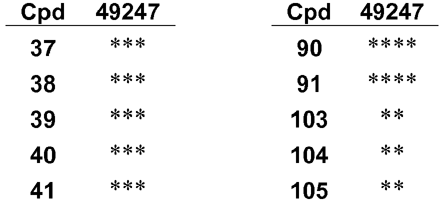

**Table 8**

| **Cpd** | **25238** |
|---|---|
| **1** | * |
| **8** | *** |
| **22** | **** |
| **23** | **** |
| **24** | **** |
| **25** | **** |
| **26** | **** |
| **28** | **** |
| **29** | ** |
| **30** | *** |
| **31** | ** |
| **32** | **** |
| **33** | **** |
| **34** | **** |
| **35** | **** |
| **36** | **** |
| **37** | **** |
| **38** | **** |
| **39** | **** |
| **40** | **** |
| **41** | **** |
| **42** | **** |
| **43** | **** |
| **44** | **** |
| **45** | **** |
| **46** | **** |
| **47** | *** |
| **48** | **** |
| **49** | ** |
| **50** | **** |
| **51** | **** |
| **52** | **** |
| **68** | **** |
| **69** | **** |
| **78** | * |
| **80** | ** |
| **103** | * |
| **104** | ** |
| **105** | ** |

**Table 9**

| **Cpd** | **13090** |
|---|---|
| **1** | **** |
| **22** | **** |
| **23** | **** |
| **24** | **** |
| **78** | *** |
| **80** | **** |

### Example 7

### Combinations with Antibacterial Agents

The *in vitro* effects of compounds described herein in combination with ciprofloxacin are investigated in various organisms using the microdilution checkerboard method for the measurement of antibiotic synergy. Assays can be performed in a 96-well checkerboard titration format, with serial dilutions of each compound to identify the lowest MIC value (µg/mL) for each drug where the bacterial culture is completely inhibited. The ability of compounds to either act synergistically, additively, indifferently or antagonistically can be determined. Synergy is defined such that when the elements A and B are combined, the result is greater than the expected arithmetic sum A+B. The calculated fractional inhibitory concentration (FIC) is a quantitative measure of drug interactions: where values ≤ 0.5 = synergy, values between >0.5 and < 2 = additive, values between ≥2 and ≤4 = indifference, and values > 4 = antagonism. The fractional inhibition indices are calculated using the checkerboard method in a 96-well microtiter plate. Combinations that demonstrated no difference (Indiff) in the resulting activity and those that demonstrated synergistic (Syn) or additive (Add) activity are indicated.

Development of a combination therapy is an option to treat certain infections caused by organisms regardless of whether they are resistant or responsive to ciprofloxacin.

Combination therapy can be applied with any quinolone antibiotic including, without limitation, one or more of Ciprofloxacin, Enoxacin, Gatifloxacin, Levofloxacin, Lomefloxacin, Moxifloxacin, Nalidixic acid, Norfloxacin or Ofloxacin.

In addition, combination therapy can be applied with any non-quinolone antibiotic including, without limitation, one or more of Amikacin, Amoxicillin, Ampicillin, Arsphenamine, Azithromycin, Azlocillin, Aztreonam, Bacitracin, Capreomycin, Carbenicillin, Cefaclor, Cefadroxil, Cefalexin, Cefalotin (Cefalothin), Cefamandole, Cefazolin, Cefdinir, Cefditoren, Cefixime, Cefoperazone, Cefotaxime, Cefoxitin, Cefpodoxime, Cefprozil, Ceftazidime, Ceftibuten, Ceftizoxime, Ceftriaxone, Cefuroxime, Chloramphenicol, Cilastatin, Clarithromycin, Clavulanate, Clindamycin, Clofazimine, Cloxacillin, Colistin, Cycloserine, Dalfopristin, Dapsone, Daptomycin, Dicloxacillin, Dirithromycin, Doripenem, Doxycycline, Erythromycin, Ethambutol, Ethionamide, Flucloxacillin, Fosfomycin, Furazolidone, Fusidic acid, Gentamicin, Imipenem, Isoniazid, Kanamycin, Lincomycin, Linezolid, Loracarbef, Mafenide, Meropenem, Methicillin, Metronidazole, Mezlocillin, Minocycline, Mupirocin, Nafcillin, Neomycin, Netilmicin, Nitrofurantoin, Oxacillin, Oxytetracycline, Paromomycin, Penicillin G, Penicillin V, Piperacillin, Platensimycin, Polymyxin B, Pyrazinamide, Quinupristin, Rapamycin, Rifabutin, Rifampicin, Rifampin, Rifapentine, Rifaximin, Roxithromycin, Silver sulfadiazine, Spectinomycin, Streptomycin, Sulbactam, Sulfacetamide, Sulfadiazine, Sulfamethizole, Sulfamethoxazole, Sulfanilimide, Sulfasalazine, Sulfisoxazole, Tazobactam, Teicoplanin, Telavancin, Telithromycin, Temocillin, Tetracycline, Thiamphenicol, Ticarcillin, Tigecycline, Tinidazole, Tobramycin, Trimethoprim, Troleandomycin or Vancomycin.

Several examples of preferred embodiments of the present description are listed below.

In a preferred embodiment, the description relates to compounds for use in a method of treating or ameliorating a bacterial infection in a subject in need thereof comprising administering an effective amount of a compound of Formula (I), Formula (II) or Formula (III) or a form thereof to the subject.

In another preferred embodiment, the description relates to compounds for use in a method of treating or ameliorating a bacterial infection in a subject in need thereof comprising administering an effective amount of a compound of Formula (I), Formula (II) or Formula (III) or a form thereof to the subject, wherein the bacterial infection results from a bacteria that is a Gram-negative or Gram-positive type.

In another preferred embodiment, the description relates to compounds for use in a method of treating or ameliorating a bacterial infection in a subject in need thereof comprising administering an effective amount of a compound of Formula (I), Formula (II) or Formula (III) or a form thereof to the subject, wherein the bacterial infection results from a bacteria that is a multi-drug resistant Gram-negative or Gram-positive type.

In another preferred embodiment, the description relates to compounds for use in a method of treating or ameliorating a bacterial infection in a subject in need thereof comprising administering an effective amount of a compound of Formula (I), Formula (II) or Formula (III) or a form thereof to the subject, wherein the bacterial infection results from a bacteria of the phyla selected from Acidobacteria; Actinobacteria; Aquificae; Bacteroidetes; Caldiserica; Chlamydiae; Chlorobi; Chloroflexi; Chrysiogenetes; Cyanobacteria; Deferribacteres; Deinococcus-Thermus; Dictyoglomi; Elusimicrobia; Fibrobacteres; Firmicutes; Fusobacteria; Gemmatimonadetes; Lentisphaerae; Nitrospira; Planctomycetes; Proteobacteria; Spirochaetes; Synergistetes; Tenericutes; Firmicutes; Thermodesulfobacteria; Thermomicrobia; Thermotogae; or Verrucomicrobia.

In another preferred embodiment, the description relates to compounds for use in a method of treating or ameliorating a bacterial infection in a subject in need thereof comprising administering an effective amount of a compound of Formula (I), Formula (II) or Formula (III) or a form thereof to the subject, wherein the bacterial infection results from a bacteria of the phyla selected from Proteobacteria, Spirochaetes, Bacteriodetes, Chlamydiae, Firmicutes or Actinobacteria.

In another preferred embodiment, the description relates to compounds for use in a method of treating or ameliorating a bacterial infection in a subject in need thereof comprising administering an effective amount of a compound of Formula (I), Formula (II) or Formula (III) or a form thereof to the subject, wherein the bacterial infection results from a bacterial species selected from *Acinetobacter baumannii, Bacillus anthracis, Bacillus subtilis, Enterobacter* spp., *Enterococcus faecalis, Enterococcus faecalis, Enterococcus faecium, Escherichia coli, Francisella tularensis, Haemophilus influenzae, Klebsiella pneumoniae, Moraxella catarrhalis, Mycobacterium tuberculosis, Neisseria* spp., *Pseudomonas aeruginosa, Shigella* spp., *Staphylococcus aureus, Streptococcus pyogenes*, *Streptococcus pneumoniae* and *Yersinia pestis.*

In another preferred embodiment, the description relates to compounds for use in a method of treating or ameliorating a bacterial infection in a subject in need thereof comprising administering an effective amount of a compound of Formula (I), Formula (II) or Formula (III) or a form thereof to the subject, wherein the effective amount of the compound of Formula (I), Formula (II) or Formula (III) or a form thereof is in a range of from 0.001 mg/Kg/day to 500 mg/Kg/day.

In another preferred embodiment, the description relates to a combination therapy comprising an effective amount of a compound of Formula (I), Formula (II) or Formula (III) or a form thereof and an effective amount of an antibiotic or antibacterial agent.

In another preferred embodiment, the description relates to a combination therapy comprising an effective amount of a compound of Formula (I), Formula (II) or Formula (III) or a form thereof and an effective amount of an antibiotic or antibacterial agent, wherein the agent is selected from one or more of Ciprofloxacin, Enoxacin, Gatifloxacin, Levofloxacin, Lomefloxacin, Moxifloxacin, Nalidixic acid, Norfloxacin or Ofloxacin.

In another preferred embodiment, the description relates to a combination therapy comprising an effective amount of a compound of Formula (I), Formula (II) or Formula (III) or a form thereof and an effective amount of an antibiotic or antibacterial agent, wherein the agent is selected from one or more of Amikacin, Amoxicillin, Ampicillin, Arsphenamine, Azithromycin, Azlocillin, Aztreonam, Bacitracin, Capreomycin, Carbenicillin, Cefaclor, Cefadroxil, Cefalexin, Cefalotin, Cefamandole, Cefazolin, Cefdinir, Cefditoren, Cefixime, Cefoperazone, Cefotaxime, Cefoxitin, Cefpodoxime, Cefprozil, Ceftazidime, Ceftibuten, Ceftizoxime, Ceftriaxone, Cefuroxime, Chloramphenicol, Cilastatin, Clarithromycin, Clavulanate, Clindamycin, Clofazimine, Cloxacillin, Colistin, Cycloserine, Dalfopristin, Dapsone, Daptomycin, Dicloxacillin, Dirithromycin, Doripenem, Doxycycline, Erythromycin, Ethambutol, Ethionamide, Flucloxacillin, Fosfomycin, Furazolidone, Fusidic acid, Gentamicin, Imipenem, Isoniazid, Kanamycin, Lincomycin, Linezolid, Loracarbef, Mafenide, Meropenem, Methicillin, Metronidazole, Mezlocillin, Minocycline, Mupirocin, Nafcillin, Neomycin, Netilmicin, Nitrofurantoin, Oxacillin, Oxytetracycline, Paromomycin, Penicillin G, Penicillin V, Piperacillin, Platensimycin, Polymyxin B, Pyrazinamide, Quinupristin, Rapamycin, Rifabutin, Rifampicin, Rifampin, Rifapentine, Rifaximin, Roxithromycin, Silver sulfadiazine, Spectinomycin, Streptomycin, Sulbactam, Sulfacetamide, Sulfadiazine, Sulfamethizole, Sulfamethoxazole, Sulfanilimide, Sulfasalazine, Sulfisoxazole, Tazobactam, Teicoplanin, Telavancin, Telithromycin, Temocillin, Tetracycline, Thiamphenicol, Ticarcillin, Tigecycline, Tinidazole, Tobramycin, Trimethoprim, Troleandomycin or Vancomycin.

Although certain embodiments have been described in detail above, those having ordinary skill in the art will clearly understand that many modifications are possible in the embodiments without departing from the teachings thereof.

## Claims

1. A compound of Formula (I), Formula (II) or Formula (III): 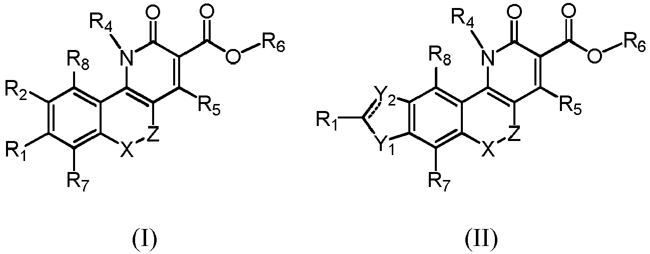 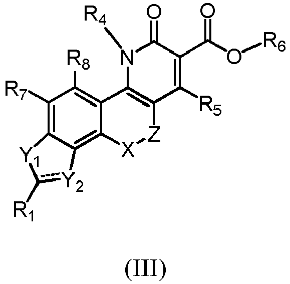 or a form thereof, wherein the form of the compound is selected from a free acid, free base, salt, hydrate, solvate, clathrate, isotopologue, racemate, enantiomer, diastereomer, stereoisomer, polymorph or tautomer form thereof, wherein:
X is a bond, N(R₁₄), S, O, -CH(R₉)-, -CH(R₉)-CH(R₁₀)-, -CH(R₉)-CH(R₁₀)-CH(R₁₁)-, -C(R₉)=C(R₁₀)-, -C(R₉)=C(R₁₀)-CH(R₁₁)-, -CH(R₉)-C(R₁₀)=C(R₁₁)-, -O-CH(R₁₀)-, -CH(R₉)-O-, -N(R₁₄)-CH(R₁₀)-, -CH(R₉)-N(R₁₄)-, -S-CH(R₁₀)-, -CH(R₉)-S-, -O-CH(R₁₀)-CH(R₁₀)-, -CH(R₉)-O-CH(R₁₁)-, -CH(R₉)-CH(R₁₀)-O-, -N(R₁₄)-CH(R₁₀)-CH(R₁₁)-, -CH(R₉)-N(R₁₄)-CH(R₁₁)-, -CH(R₉)-CH(R₁₀)-N(R₁₄)-, -S-CH(R₁₀)-CH(R₁₁)-, -CH(R₉)-S-CH(R₁₁)-, -CH(R₉)-CH(R₁₀)-S-; -O-C(O)-CH(R₁₁)-, -C(O)-O-CH(R₁₁)-, -CH(R₉)-O-C(O)-, -CH(R₉)-C(O)-O-, -N(R₁₄)-C(O)-CH(R₁₁)-, -C(O)-N(R₁₄)-CH(R₁₁)-, -CH(R₉)-N(R₁₄)-C(O)-, -CH(R₉)-C(O)-N(R₁₄)-, -S-C(O)-CH(R₁₁)-, -C(O)-S-CH(R₁₁)-, -CH(R₉)-S-C(O)- or -CH(R₉)-C(O)-S-;
Y₁ is -N(R₁₂)- or -O-;
Y₂ is -C(R₁₃)-, -N(R₁₂)- or -O-; wherein the dashed line represents a double bond that is present when Y₂ is -C(R₁₃)- and absent when Y₂ is -N(R₁₂)- or -O-;
Z is N(R₁₄), S, O, C(O) or -CH(R₃)-;
R₁ is halogen, hydroxyl, oxo, cyano, nitro, C₁₋₈alkyl, hydroxyl-C₁₋₈alkyl, halo-C₁₋₈alkyl, C₁₋₈alkoxy, halo-C₁₋₈alkoxy, C₁₋₈alkyl-thio, carboxyl, C₁₋₈alkyl-carbonyl, C₁₋₈alkoxy-carbonyl, amino-carbonyl, amino, C₁₋₈alkyl-amino, (C₁₋₈alkyl)₂-amino, C₂₋₈alkenyl-amino, (C₂₋₈alkenyl)₂-amino, C₂₋₈alkynyl-amino, (C₂₋₈alkynyl)₂-amino, amino-C₁₋₈alkyl, C₁₋₁₀alkyl-amino-C₁₋₈alkyl, (C₁₋₈alkyl)₂-amino-C₁₋₈alkyl, C₂₋₈alkenyl-amino-C₁₋₈alkyl, (C₂₋₈alkenyl)₂-amino-C₁₋₈alkyl, C₂₋₈alkynyl-amino-C₁₋₈alkyl, (C₂₋₈alkynyl)₂-amino-C₁₋₈alkyl, halo-C₁₋₈alkyl-amino, (halo-C₁₋₈alkyl)₂-amino, halo-C₁₋₈alkyl-amino-C₁₋₈alkyl, (halo-C₁₋₈alkyl)₂-amino-C₁₋₈alkyl, C₁₋₈alkoxy-C₁₋₈alkyl-amino, (C₁₋₈alkoxy-C₁₋₈alkyl,C₁₋₈alkyl)-amino, (C₁₋₈alkoxy-C₁₋₈alkyl)₂-amino, C₁₋₈alkoxy-C₁₋₈alkyl-amino-C₁₋₈alkyl, (C₁₋₈alkoxy-C₁₋₈alkyl,C₁₋₈alkyl)-amino-C₁₋₈alkyl, (C₁₋₈alkoxy-C₁₋₈alkyl)₂-amino-C₁₋₈alkyl, amino-C₁₋₈alkyl-amino, (amino-C₁₋₈alkyl,C₁₋₈alkyl)amino, C₁₋₈alkyl-amino-C₁₋₈alkyl-amino, (C₁₋₈alkyl-amino-C₁₋₈alkyl,C₁₋₈alkyl)amino, (C₁₋₈alkyl)₂-amino-C₁₋₈alkyl-amino, [(C₁₋₈alkyl)₂-amino-C₁₋₈alkyl,C₁₋₈alkyl]amino, amino-C₁₋₈alkyl-amino-C₁₋₈alkyl, (amino-C₁₋₈alkyl,C₁₋₈alkyl)amino-C₁₋₈alkyl, C₁₋₈alkyl-amino-C₁₋₈alkyl-amino-C₁₋₈alkyl, (C₁₋₈alkyl-amino-C₁₋₈alkyl,C₁₋₈alkyl)amino-C₁₋₈alkyl, (C₁₋₈alkyl)₂-amino-C₁₋₈alkyl-amino-C₁₋₈alkyl, [(C₁₋₈alkyl)₂-amino-C₁₋₈alkyl,C₁₋₈alkyl]amino-C₁₋₈alkyl, hydroxyl-amino, hydroxyl-C₁₋₈alkyl-amino, (hydroxyl-C₁₋₈alkyl,C₁₋₈alkyl)amino, (hydroxyl-C₁₋₈alkyl)₂-amino, hydroxyl-C₁₋₈alkyl-amino-C₁₋₈alkyl, (hydroxyl-C₁₋₈alkyl,C₁₋₈alkyl)amino-C₁₋₈alkyl, hydroxyl-C₁₋₈alkyl-amino-C₁₋₈alkyl-amino, (hydroxyl-C₁₋₈alkyl-amino-C₁₋₈alkyl,C₁₋₈alkyl)amino, (hydroxyl-C₁₋₈alkyl, C₁₋₈alkyl)amino-C₁₋₈alkyl-amino, [(hydroxyl-C₁₋₈alkyl,C₁₋₈alkyl)amino-C₁₋₈alkyl,C₁₋₈alkyl]amino, (C₁₋₈alkyl-carbonyl,C₁₋₈alkyl)amino-C₁₋₈alkyl, C₁₋₈alkyl-amino-carbonyl, (C₁₋₈alkyl)₂-amino-carbonyl, (C₁₋₈alkyl)₂-amino-carbonyl-C₁₋₈alkyl-amino-C₁₋₈alkyl, C₃₋₁₄cycloalkyl, C₃₋₁₄cycloalkyl-C₁₋₈alkyl, C₃₋₁₄cycloalkyl-oxy, C₃₋₁₄cycloalkyl-C₁₋₈alkoxy, C₃₋₁₄cycloalkyl-amino, C₃₋₁₄cycloalkyl-amino-C₁₋₈alkyl, (C₃₋₁₄cycloalkyl,C₁₋₈alkyl)amino-C₁₋₈alkyl, (C₃₋₁₄cycloalkyl)₂-amino-C₁₋₈alkyl, C₃₋₁₄cycloalkyl-C₁₋₈alkyl-amino-C₁₋₈alkyl, (C₃₋₁₄cycloalkyl-C₁₋₈alkyl,C₁₋₈alkyl)amino-C₁₋₈alkyl, (C₃₋₁₄cycloalkyl-C₁₋₈alkyl)₂-amino-C₁₋₈alkyl, aryl, aryl-C₁₋₈alkyl, aryl-amino, (aryl,C₁₋₈alkyl)amino, (aryl)₂-amino, aryl-amino-C₁₋₈alkyl, (aryl,C₁₋₈alkyl)amino-C₁₋₈alkyl, (aryl)₂-amino-C₁₋₈alkyl, aryl-C₁₋₈alkyl-amino, (aryl-C₁₋₈alkyl,C₁₋₈alkyl)amino, (aryl-C₁₋₈alkyl)₂-amino, aryl-C₁₋₈alkyl-amino-C₁₋₈alkyl, (aryl-C₁₋₈alkyl,C₁₋₈alkyl)amino-C₁₋₈alkyl, (aryl-C₁₋₈alkyl)₂-amino-C₁₋₈alkyl, heteroaryl selected from the group consisting of pyrrolyl, thiazolyl, 1H-1,2,3-triazolyl, 1H-tetrazolyl, 2H-tetrazolyl, imidazolyl, and pyridinyl, heteroaryl-C₁₋₈alkyl, heteroaryl-amino, heteroaryl-C₁₋₈alkyl-amino, (heteroaryl-C₁₋₈alkyl,C₁₋₈alkyl)amino, (heteroaryl-C₁₋₈alkyl)₂-amino, heteroaryl-C₁₋₈alkyl-amino-C₁₋₈alkyl, (heteroaryl-C₁₋₈alkyl,C₁₋₈alkyl)amino-C₁₋₈alkyl, heterocyclyl selected from the group consisting of azetidinyl, pyrrolidinyl, tetrahydrofuranyl, piperidinyl, piperazinyl, morpholinyl, 1,4-diazepanyl, 1,3-dioxolanyl, 2,5-dihydro-1H-pyrrolyl, dihydro-1H-imidazolyl, 1,4,5,6-tetrahydropyrimidiny, 1,2,3,6-tetrahydropyridinyl, tetrahydro-2H-pyranyl, indolinyl, 2,3-dihydrobenzo[d]oxazolyl, 3,4-dihydro-2H-benzo[b][1,4]oxazinyl, 3,4-dihydroisoquinolin-(1H)-yl, 1,2,3,4-tetrahydroisoquinolinyl, 1,2,3,4-tetrahydroquinoxalinyl, hexahydropyrrolo[3,4-b][1,4]oxazin-(2H)-yl, (4aR,7aS)-hexahydropyrrolo[3,4-b][1,4]oxazin-(4aH)-yl, 3,4-dihydro-2H-pyrido[3,2-b][1,4]oxazinyl, (cis)-octahydrocyclopenta[c]pyrrolyl, hexahydropyrrolo[3,4-b]pyrrol-(1H)-yl, (3aR,6aR)-hexahydropyrrolo[3,4-b]pyrrol-(1H)-yl, (3aR,6aS)-hexahydropyrrolo[3,4-c]pyrrol-(1H)-yl, 5H-pyrrolo[3,4-b]pyridin-(7H)-yl, 5,7-dihydro-6H-pyrrolo[3,4-b]pyridinyl, tetrahydro-1H-pyrrolo[3,4-b]pyridin-(2H,7H,7aH)-yl, hexahydro-1H-pyrrolo[3,4-b]pyridin-(2H)-yl, (4aR,7aR)-hexahydro-1H-pyrrolo[3,4-b]pyridin-(2H)-yl, octahydro-6H-pyrrolo[3,4-b]pyridinyl, 2,3,4,9-tetrahydro-1H-carbazolyl, 1,2,3,4-tetrahydropyrazino[1,2-a]indol-8-yl, 2,3-dihydro-1H-pyrrolo[1,2-a]indolyl, (3aR,6aR)-hexahydrocyclopenta[c]pyrrol-(1H)-yl, (3aR,4R,6aS)-hexahydrocyclopenta[c]pyrrol-(1H)-yl, (3aR,4S,6aS)-hexahydrocyclopenta[c]pyrrol-(1H)-yl, (3aR,5r,6aS)-hexahydrocyclopenta[c]pyrrol-(1H)-yl, 1,3-dihydro-2H-isoindolyl, octahydro-2H-isoindolyl, (3aS)-1,3,3a,4,5,6-hexahydro-2H-isoindolyl, (3aR,4R,7aS)-1H-isoindol-(3H,3aH,4H,5H,6H,7H,7aH)-yl, (3aR,7aS)-octahydro-2H-isoindolyl, (3aR,4R,7aS)-octahydro-2H-isoindolyl, (3aR,4S,7aS)-octahydro-2H-isoindolyl, 2,5-diazabicyclo[2.2.1]heptanyl, 2-azabicyclo[2.2.1]hept-5-enyl, 3-azabicyclo[3.1.0]hexanyl, (1R,SS,6s)-3-azabicyclo[3.1.0]hexanyl, (cis,cis)-3-azabicyclo[3.1.0]hexanyl, 3,6-diazabicyclo[3.1.0]hexanyl, (1S,SR,6R)-3-azabicyclo[3.2.0]heptanyl, (1S,SR,6S)-3-azabicyclo[3.2.0]heptanyl, 5-azaspiro[2.4]heptanyl, 2,6-diazaspiro[3.3]heptanyl, 2,5-diazaspiro[3.4]octanyl, 2,6-diazaspiro[3.4]octanyl, 2,7-diazaspiro[3.5]nonanyl, 2,7-diazaspiro[4.4]nonanyl, 2-azaspiro[4.5]decanyl, and 2,8-diazaspiro[4.5]decanyl, heterocyclyl-C₁₋₈alkyl, heterocyclyl-oxy, heterocyclyl-C₁₋₈alkoxy, heterocyclyl-amino, (heterocyclyl,C₁₋₈alkyl)amino, (heterocyclyl)₂-amino, heterocyclyl-amino-C₁₋₈alkyl, (heterocyclyl,C₁₋₈alkyl)amino-C₁₋₈alkyl, (heterocyclyl)₂-amino-C₁₋₈alkyl, (heterocyclyl,C₃₋₁₄cycloalkyl-C₁₋₈alkyl)amino-C₁₋₈alkyl, heterocyclyl-C₁₋₈alkyl-amino-C₁₋₈alkyl, (heterocyclyl-C₁₋₈alkyl,C₁₋₈alkyl)amino-C₁₋₈alkyl, (heterocyclyl-C₁₋₈alkyl)₂-amino-C₁₋₈alkyl, heterocyclyl-oxy-amino, (heterocyclyl-oxy,C₁₋₈alkyl)amino, (heterocyclyl-oxy)₂-amino, (heterocyclyl-oxy-C₁₋₈alkyl,C₁₋₈alkyl)amino, heterocyclyl-carbonyl or heterocyclyl-carbonyl-oxy;
wherein each instance of C₃₋₁₄cycloalkyl, aryl, heterocyclyl and heteroaryl is optionally substituted with one, two or three substituents each selected from R₁₅; and,
wherein each instance of aryl, heterocyclyl or heteroaryl is optionally substituted with one additional substituent selected from R₁₆;
R₂ is hydrogen, halogen, hydroxyl, C₁₋₈alkyl, amino, C₁₋₈alkyl-amino or (C₁₋₈alkyl)₂-amino;
R₃ is hydrogen, halogen, hydroxyl, C₁₋₈alkyl, amino, C₁₋₈alkyl-amino or (C₁₋₈alkyl)₂-amino;
R₄ is hydrogen, hydroxyl, aryl-C₁₋₈alkyl, wherein aryl is optionally substituted with one additional substituent selected from C₁₋₈alkyl, halo-C₁₋₈alkyl, C₁₋₈alkoxy or C₁₋₈alkoxy-C₁₋₈alkyl;
R₅ is hydrogen, halogen, hydroxyl, C₁₋₈alkyl, C₁₋₈alkoxy, carboxyl, amino, C₁₋₈alkyl-amino, (C₁₋₈alkyl)₂-amino or C₁₋₈alkyl-SO₂-amino;
R₆ is hydrogen or C₁₋₈alkyl;
R₇ is hydrogen, halogen, hydroxyl, C₁₋₈alkyl, amino, C₁₋₈alkyl-amino or (C₁₋₈alkyl)₂-amino;
R₈ is hydrogen, halogen, hydroxyl, C₁₋₈alkyl, amino, C₁₋₈alkyl-amino or (C₁₋₈alkyl)₂-amino;
R₉ is hydrogen, halogen, hydroxyl, C₁₋₈alkyl, amino, C₁₋₈alkyl-amino or (C₁₋₈alkyl)₂-amino;
R₁₀ is hydrogen, halogen, hydroxyl, C₁₋₈alkyl, amino, C₁₋₈alkyl-amino or (C₁₋₈alkyl)₂-amino;
R₁₁ is hydrogen, halogen, hydroxyl, C₁₋₈alkyl, amino, C₁₋₈alkyl-amino or (C₁₋₈alkyl)₂-amino;
R₁₂ is hydrogen, C₁₋₈alkyl, aryl or heteroaryl, wherein aryl and heteroaryl is optionally substituted with one additional substituent selected from C₁₋₈alkyl, halo-C₁₋₈alkyl, C₁₋₈alkoxy or C₁₋₈alkoxy-C₁₋₈alkyl;
R₁₃ is hydrogen, cyano, halogen, hydroxyl or C₁₋₈alkyl;
R₁₄ is hydrogen, C₁₋₈alkyl, C₁₋₈alkyl-carbonyl, C₁₋₈alkoxy-carbonyl, aryl or heteroaryl, wherein aryl and heteroaryl is optionally substituted with one additional substituent selected from C₁₋₈alkyl, halo-C₁₋₈alkyl, C₁₋₈alkoxy or C₁₋₈alkoxy-C₁₋₈alkyl;
R₁₅ is azido, halogen, hydroxyl, cyano, nitro, C₁₋₈alkyl, halo-C₁₋₈alkyl, hydroxyl-C₁₋₈alkyl, C₁₋₈alkoxy-C₁₋₈alkyl, C₁₋₈alkoxy, halo-C₁₋₈alkoxy, hydroxyl-C₁₋₈alkoxy, carboxyl, C₁₋₈alkyl-carbonyl, C₁₋₈alkoxy-carbonyl, amino, C₁₋₈alkyl-amino, (C₁₋₈alkyl)₂-amino, halo-C₁₋₈alkyl-amino, (halo-C₁₋₈alkyl)₂-amino, halo-C₁₋₈alkyl-amino-C₁₋₈alkyl, (halo-C₁₋₈alkyl)₂-amino-C₁₋₈alkyl, amino-C₁₋₈alkyl, C₁₋₈alkyl-amino-C₁₋₈alkyl, (C₁₋₈alkyl)₂-amino-C₁₋₈alkyl, [(C₁₋₈alkyl)₂-amino-C₁₋₈alkyl,C₁₋₈alkyl]amino-C₁₋₈alkyl C₁₋₈alkyl-thio, amino-carbonyl, C₁₋₈alkyl-amino-carbonyl, (C₁₋₈alkyl)₂-amino-carbonyl, C₁₋₈alkyl-carbonyl-amino or (carboxyl-C₁₋₈alkyl, C₁₋₈alkyl)amino-carbonyl-amino;
R₁₆ is C₃₋₁₄cycloalkyl, C₃₋₁₄cycloalkyl-amino, aryl, aryl-C₁₋₈alkyl, aryl-amino, (aryl,C₁₋₈alkyl)amino, (aryl)₂-amino, aryl-C₁₋₈alkyl-amino, (aryl-C₁₋₈alkyl,C₁₋₈alkyl)amino, (aryl-C₁₋₈alkyl)₂-amino, aryl-C₁₋₈alkyl-amino-C₁₋₈alkyl, (aryl-C₁₋₈alkyl,C₁₋₈alkyl)amino-C₁₋₈alkyl, (aryl-C₁₋₈alkyl)₂-amino-C₁₋₈alkyl, aryl-amino-C₁₋₈alkyl, (aryl,C₁₋₈alkyl)amino-C₁₋₈alkyl, (aryl)₂-amino-C₁₋₈alkyl, aryl-amino-carbonyl, aryl-C₁₋₈alkoxy, aryl-C₁₋₈alkoxy-carbonyl-amino, heteroaryl, heteroaryl-C₁₋₈alkyl, heteroaryl-amino, (heteroaryl)₂-amino, heteroaryl-C₁₋₈alkyl-amino, (heteroaryl-C₁₋₈alkyl,C₁₋₈alkyl)amino, (heteroaryl-C₁₋₈alkyl)₂-amino, heteroaryl-C₁₋₈alkyl-amino-C₁₋₈alkyl, (heteroaryl-C₁₋₈alkyl,C₁₋₈alkyl)amino-C₁₋₈alkyl, (heteroaryl-C₁₋₈alkyl)₂-amino-C₁₋₈alkyl, heterocyclyl, heterocyclyl-C₁₋₈alkyl, heterocyclyl-amino-C₁₋₈alkyl or heterocyclyl-oxy;
wherein each instance of C₃₋₁₄cycloalkyl is optionally substituted with one substituent selected from R₁₈; and,
wherein each instance of aryl is optionally substituted with one substituent selected from R₁₉;
R₁₈ is amino, C₁₋₈alkyl-amino, (C₁₋₈alkyl)₂-amino, amino-C₁₋₈alkyl, C₁₋₈alkyl-amino-C₁₋₈alkyl, (C₁₋₈alkyl)₂-amino-C₁₋₈alkyl or aryl-C₁₋₈alkyl-amino; and
R₁₉ is halogen.

2. The compound of claim 1, wherein R₁ is
C₃₋₁₄acycloalkyl selected in each instance, when present, from cyclopropyl, cyclobutyl, cyclopentyl, cyclohexyl or cycloheptyl;
aryl selected in each instance, when present, from phenyl;
heteroaryl selected in each instance, when present, from pyrrolyl, thiazolyl, 1H-1,2,3-triazolyl, 1H-tetrazolyl, 2H-tetrazolyl, imidazolyl or pyridinyl;
heterocyclyl selected in each instance, when present, from azetidinyl, pyrrolidinyl, tetrahydrofuranyl, piperidinyl, piperazinyl, morpholinyl, 1,4-diazepanyl, 1,3-dioxolanyl, 2,5-dihydro-1H-pyrrolyl, dihydro-1H-imidazolyl, 1,4,5,6-tetrahydropyrimidiny, 1,2,3,6-tetrahydropyridinyl, tetrahydro-2H-pyranyl, indolinyl, 2,3-dihydrobenzo[d]oxazolyl, 3,4-dihydro-2H-benzo[b][1,4]oxazinyl, 3,4-dihydroisoquinolin-(1H)-yl, 1,2,3,4-tetrahydroisoquinolinyl, 1,2,3,4-tetrahydroquinoxalinyl, hexahydropyrrolo[3,4-b][1,4]oxazin-(2H)-yl, (4aR,7aS)-hexahydropyrrolo[3,4-b][1,4]oxazin-(4aH)-yl, 3,4-dihydro-2H-pyrido[3,2-b][1,4]oxazinyl, (cis)-octahydrocyclopenta[c]pyrrolyl, hexahydropyrrolo[3,4-b]pyrrol-(1H)-yl, (3aR,6aR)-hexahydropyrrolo[3,4-b]pyrrol-(1H)-yl, (3aR,6aS)-hexahydropyrrolo[3,4-c]pyrrol-(1H)-yl, 5H-pyrrolo[3,4-b]pyridin-(7H)-yl, 5,7-dihydro-6H-pyrrolo[3,4-b]pyridinyl, tetrahydro-1H-pyrrolo[3,4-b]pyridin-(2H,7H,7aH)-yl, hexahydro-1H-pyrrolo[3,4-b]pyridin-(2H)-yl, (4aR,7aR)-hexahydro-1H-pyrrolo[3,4-b]pyridin-(2H)-yl, octahydro-6H-pyrrolo[3,4-b]pyridinyl, 2,3,4,9-tetrahydro-1H-carbazolyl, 1,2,3,4-tetrahydropyrazino[1,2-a]indol-8-yl, 2,3-dihydro-1H-pyrrolo[1,2-a]indolyl, (3aR,6aR)-hexahydrocyclopenta[c]pyrrol-(1H)-yl, (3aR,4R,6aS)-hexahydrocyclopenta[c]pyrrol-(1H)-yl, (3aR,4S,6aS)-hexahydrocyclopenta[c]pyrrol-(1H)-yl, (3aR,5r,6aS)-hexahydrocyclopenta[c]pyrrol-(1H)-yl, 1,3-dihydro-2H-isoindolyl, octahydro-2H-isoindolyl, (3aS)-1,3,3a,4,5,6-hexahydro-2H-isoindolyl, (3aR,4R,7aS)-1H-isoindol-(3H,3aH,4H,5H,6H,7H,7aH)-yl, (3aR,7aS)-octahydro-2H-isoindolyl, (3aR,4R,7aS)-octahydro-2H-isoindolyl, (3aR,4S,7aS)-octahydro-2H-isoindolyl, 2,5-diazabicyclo[2.2.1]heptanyl, 2-azabicyclo[2.2.1]hept-5-enyl, 3-azabicyclo[3.1.0]hexanyl, (1R,5S,6s)-3-azabicyclo[3.1.0]hexanyl, (cis,cis)-3-azabicyclo[3.1.0]hexanyl, 3,6-diazabicyclo[3.1.0]hexanyl, (1S,5R,6R)-3-azabicyclo[3.2.0]heptanyl, (1S,SR,6S)-3-azabicyclo[3.2.0]heptanyl, 5-azaspiro[2.4]heptanyl, 2,6-diazaspiro[3.3]heptanyl, 2,5-diazaspiro[3.4]octanyl, 2,6-diazaspiro[3.4]octanyl, 2,7-diazaspiro[3.5]nonanyl, 2,7-diazaspiro[4.4]nonanyl, 2-azaspiro[4.5]decanyl or 2,8-diazaspiro[4.5]decanyl.

3. The compound of claim 1, wherein
R₂ is hydrogen, C₁₋₈alkyl, amino, C₁₋₈alkyl-amino or (C₁₋₈alkyl)₂-amino;
R₃ is hydrogen, halogen, hydroxyl, C₁₋₈alkyl, amino, C₁₋₈alkyl-amino or (C₁₋₈alkyl)₂-amino; and
R₄ is hydrogen.

4. The compound of claim 1, wherein R₁ is
C₃₋₁₄cycloalkyl-amino-C₁₋₈alkyl, wherein C₃₋₁₄cycloalkyl is selected from cyclopropyl, cyclobutyl, cyclopentyl or cyclohexyl;
(C₃₋₁₄cycloalkyl,C₁₋₈alkyl)amino-C₁₋₈alkyl, wherein C₃-₁₄cycloalkyl is selected from cyclopropyl, cyclobutyl or cyclopentyl;
C₃₋₁₄cycloalkyl-C₁₋₈alkyl-amino-C₁₋₈alkyl, wherein C₃₋₁₄cycloalkyl is selected from cyclopropyl, cyclobutyl, cyclopentyl, cyclohexyl or cycloheptyl;
aryl, wherein aryl is selected from phenyl;
aryl-amino, wherein aryl is selected from phenyl;
(aryl,C₁₋₈alkyl)amino, wherein aryl is selected from phenyl;
aryl-amino-C₁₋₈alkyl, wherein aryl is selected from phenyl;
aryl-C₁₋₈alkyl-amino-C₁₋₈alkyl, wherein aryl is selected from phenyl;
(aryl-C₁₋₈alkyl,C₁₋₈alkyl)amino-C₁₋₈alkyl, wherein aryl is selected from phenyl;
(aryl-C₁₋₈alkyl)₂-amino-C₁₋₈alkyl, wherein aryl is selected from phenyl;
heteroaryl, wherein heteroaryl is selected from pyrrolyl, thiazolyl, 1H-1,2,3-triazolyl, 1H-tetrazolyl, 2H-tetrazolyl, imidazolyl or pyridinyl;
heteroaryl-C₁₋₈alkyl-amino-C₁₋₈alkyl, wherein heteroaryl is selected from pyridin-2-yl, pyridin-3-yl or pyridin-4-yl;
(heteroaryl-C₁₋₈alkyl,C₁₋₈alkyl)amino-C₁₋₈alkyl, wherein heteroaryl is selected from pyridin-3-yl or pyridin-4-yl;
heterocyclyl, wherein heterocyclyl is selected from azetidinyl, pyrrolidinyl, tetrahydrofuranyl, piperidinyl, piperazinyl, morpholinyl, 1,4-diazepanyl, 1,3-dioxolanyl, 2,5-dihydro-1H-pyrrolyl, dihydro-1H-imidazolyl, 1,4,5,6-tetrahydropyrimidinyl, 1,2,3,6-tetrahydropyridinyl, tetrahydro-2H-pyranyl, 3,4-dihydroisoquinolin-(1H)-yl, 1,2,3,4-tetrahydroisoquinolinyl, hexahydropyrrolo[3,4-b][1,4]oxazin-(2H)-yl, (4aR,7aS)-hexahydropyrrolo[3,4-b][1,4]oxazin-(4aH)-yl, (cis)-octahydrocyclopenta[c]pyrrolyl, hexahydropyrrolo[3,4-b]pyrrol-(1H)-yl, (3aR,6aR)-hexahydropyrrolo[3,4-b]pyrrol-(1H)-yl, (3aR,6aS)-hexahydropyrrolo[3,4-c]pyrrol-(1H)-yl, 5H-pyrrolo[3,4-b]pyridin-(7H)-yl, 5,7-dihydro-6H-pyrrolo[3,4-b]pyridinyl, tetrahydro-1H-pyrrolo[3,4-b]pyridin-(2H,7H,7aH)-yl, hexahydro-1H-pyrrolo[3,4-b]pyridin-(2H)-yl, (4aR,7aR)-hexahydro-1H-pyrrolo[3,4-b]pyridin-(2H)-yl, octahydro-6H-pyrrolo[3,4-b]pyridinyl, (3aR,6aR)-hexahydrocyclopenta[c]pyrrol-(1H)-yl, (3aR,4R,6aS)-hexahydrocyclopenta[c]pyrrol-(1H)-yl, (3aR,4S,6aS)-hexahydrocyclopenta[c]pyrrol-(1H)-yl, (3aR,5r,6aS)-hexahydrocyclopenta[c]pyrrol-2(1H)-yl, 1,3-dihydro-2H-isoindolyl, octahydro-2H-isoindolyl, (3aS)-1,3,3a,4,5,6-hexahydro-2H-isoindolyl, (3aR,4R,7aS)-1H-isoindol-(3H,3aH,4H,5H,6H,7H,7aH)-yl, (3aR,7aS)-octahydro-2H-isoindolyl, (3aR,4R,7aS)-octahydro-2H-isoindolyl, (3aR,4S,7aS)-octahydro-2H-isoindolyl, 2,5-diazabicyclo[2.2.1]heptanyl, 2-azabicyclo[2.2.1]hept-5-enyl, 3-azabicyclo[3.1.0]hexanyl, (1R,5S,6s)-3-azabicyclo[3.1.0]hexanyl, (cis,cis)-3-azabicyclo[3.1.0]hexanyl, 3,6-diazabicyclo[3.1.0]hexanyl, (1S,5R,6R)-3-azabicyclo[3.2.0]heptanyl, (1S,5R,6S)-3-azabicyclo[3.2.0]heptanyl, 5-azaspiro[2.4]heptanyl, 2,6-diazaspiro[3.3]heptanyl, 2,5-diazaspiro[3.4]octanyl, 2,6-diazaspiro[3.4]octanyl, 2,7-diazaspiro[3.5]nonanyl, 2,7-diazaspiro[4.4]nonanyl, 2-azaspiro[4.5]decanyl or 2,8-diazaspiro 4.5]decanyl;
heterocyclyl-C₁₋₈alkyl, wherein heterocyclyl is selected from azetidinyl, pyrrolidinyl, tetrahydrofuranyl, piperidinyl, piperazinyl, morpholinyl, 1,4-diazepanyl, 1,3-dioxolanyl, 2,5-dihydro-1H-pyrrolyl, dihydro-1H-imidazolyl, 1,4,5,6-tetrahydropyrimidinyl, 1,2,3,6-tetrahydropyridinyl, tetrahydro-2H-pyranyl, 3,4-dihydroisoquinolin-(1H)-yl, 1,2,3,4-tetrahydroisoquinolinyl, hexahydropyrrolo[3,4-b][1,4]oxazin-(2H)-yl, (4aR,7aS)-hexahydropyrrolo[3,4-b][1,4]oxazin-(4aH)-yl, (cis)-octahydrocyclopenta[c]pyrrolyl, hexahydropyrrolo[3,4-b]pyrrol-(1H)-yl, (3aR,6aR)-hexahydropyrrolo[3,4-b]pyrrol-(1H)-yl, (3aR,6aS)-hexahydropyrrolo[3,4-c]pyrrol-(1H)-yl, 5H-pyrrolo[3,4-b]pyridin-(7H)-yl, 5,7-dihydro-6H-pyrrolo[3,4-b]pyridinyl, tetrahydro-1H-pyrrolo[3,4-b]pyridin-(2H,7H,7aH)-yl, hexahydro-1H-pyrrolo[3,4-b]pyridin-(2H)-yl, (4aR,7aR)-hexahydro-1H-pyrrolo[3,4-b]pyridin-(2H)-yl, octahydro-6H-pyrrolo[3,4-b]pyridinyl, (3aR,6aR)-hexahydrocyclopenta[c]pyrrol-(1H)-yl, (3aR,4R,6aS)-hexahydrocyclopenta[c]pyrrol-(1H)-yl, (3aR,4S,6aS)-hexahydrocyclopenta[c]pyrrol-(1H)-yl, (3aR,5r,6aS)-hexahydrocyclopenta[c]pyrrol-2(1H)-yl, 1,3-dihydro-2H-isoindolyl, octahydro-2H-isoindolyl, (3aS)-1,3,3a,4,5,6-hexahydro-2H-isoindolyl, (3aR,4R,7aS)-1H-isoindol-(3H,3aH,4H,5H,6H,7H,7aH)-yl, (3aR,7aS)-octahydro-2H-isoindolyl, (3aR,4R,7aS)-octahydro-2H-isoindolyl, (3aR,4S,7aS)-octahydro-2H-isoindolyl, 2,5-diazabicyclo[2.2.1]heptanyl, 2-azabicyclo[2.2.1]hept-5-enyl, 3-azabicyclo[3.1.0]hexanyl, (1R,5S,6s)-3-azabicyclo[3.1.0]hexanyl, (cis,cis)-3-azabicyclo[3.1.0]hexanyl, 3,6-diazabicyclo[3.1.0]hexanyl, (1S,5R,6R)-3-azabicyclo[3.2.0]heptanyl, (1S,5R,6S)-3-azabicyclo[3.2.0]heptanyl, 5-azaspiro[2.4]heptanyl, 2,6-diazaspiro[3.3]heptanyl, 2,5-diazaspiro[3.4]octanyl, 2,6-diazaspiro[3.4]octanyl, 2,7-diazaspiro[3.5]nonanyl, 2,7-diazaspiro[4.4]nonanyl, 2-azaspiro[4.5]decanyl or 2,8-diazaspiro[4.5]decanyl;
heterocyclyl-amino-C₁₋₈alkyl, wherein heterocyclyl is selected from azetidin-1-yl or piperidin-4-yl;
(heterocyclyl,C₁₋₈alkyl)amino-C₁₋₈alkyl, wherein heterocyclyl is selected from piperidin-3-yl or piperidin-4-yl;
(heterocyclyl,C₃₋₁₄cycloalkyl-C₁₋₈alkyl)amino-C₁₋₈alkyl, wherein heterocyclyl is selected from piperidin-3-yl or piperidin-4-yl;
heterocyclyl-C₁₋₈alkyl-amino-C₁₋₈alkyl, wherein heterocyclyl is selected from pyrrolidin-2-yl, piperidin-2-yl, piperidin-3-yl, piperidin-4-yl or tetrahydro-2H-pyran-4-yl; and
(heterocyclyl-oxy-C₁₋₈alkyl,C₁₋₈alkyl)amino selected from tetrahydro-2H-pyran-2-yl-oxy-C₁₋₈alkyl,C₁₋₈alkyl)amino.

5. The compound of claim 1, wherein R₁₆ is
C₃₋₁₄cycloalkyl, wherein C₃₋₁₄cycloalkyl is selected from cyclopropyl or cyclobutyl;
C₃₋₁₄cycloalkyl-amino, wherein C₃₋₁₄cycloalkyl is selected from cyclopropyl; aryl, wherein aryl is selected from phenyl;
aryl-C₁₋₈alkyl, wherein aryl is selected from phenyl;
aryl-amino, wherein aryl is selected from phenyl;
(aryl,C₁₋₈alkyl)amino, wherein aryl is selected from phenyl;
aryl-C₁₋₈alkyl-amino, wherein aryl is selected from phenyl;
(aryl-C₁₋₈alkyl,C₁₋₈alkyl)amino, wherein aryl is selected from phenyl;
(aryl-C₁₋₈alkyl)₂-amino, wherein aryl is selected from phenyl;
aryl-C₁₋₈alkyl-amino-C₁₋₈alkyl, wherein aryl is selected from phenyl;
(aryl-C₁₋₈alkyl,C₁₋₈alkyl)amino-C₁₋₈alkyl, wherein aryl is selected from phenyl;
(aryl-C₁₋₈alkyl)₂-amino-C₁₋₈alkyl, wherein aryl is selected from phenyl;
aryl-C₁₋₈alkoxy, wherein aryl is selected from phenyl;
aryl-C₁₋₈alkoxy-carbonyl-amino, wherein aryl is selected from phenyl;
heteroaryl, wherein heteroaryl is selected from pyridin-2-yl, pyridin-4-yl, thiazol-2-yl or 1H-1,2,3-triazol-1-yl;
heteroaryl-C₁₋₈alkyl-amino-C₁₋₈alkyl, wherein heteroaryl is selected from pyridin-2-yl, pyridin-3-yl or pyridin-4-yl;
heterocyclyl, wherein heterocyclyl is selected from pyrrolidin-1-yl or morpholin-4-yl;
heterocyclyl-C₁₋₈alkyl, wherein heterocyclyl is selected from pyrrolidin-1-yl; and
heterocyclyl-oxy, wherein heterocyclyl is selected from tetrahydro-2H-pyran-2-yl-oxy.

6. The compound of claim 1, wherein the compound of Formula (I), Formula (II) or Formula (III) is a compound of Formula (Ia), Formula (IIa) or Formula (IIIa), respectively: 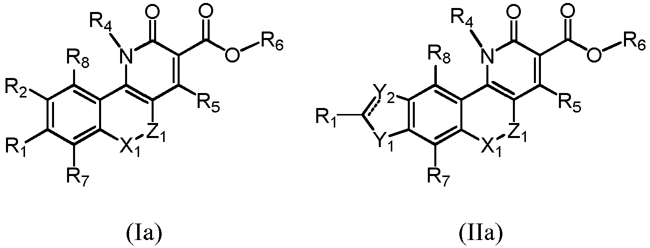 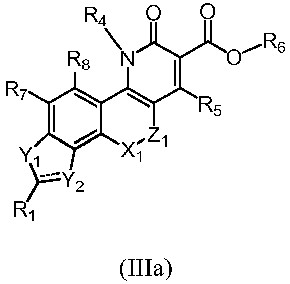 or a form thereof, wherein
the dashed line in Formula (IIa) and Formula (IIIa) represents an optionally present double bond;
X₁ is a bond, N(R₁₄), S, O or -CH(R₉)-;
Z₁ is N(R₁₄), S, O, C(O) or -CH(R₃)- when X₁ is a bond or -CH(R₉)- or, Z₁ is -CH(R₃)- when X₁ is N(R₁₄), S, or O; and
all other variables are as previously defined.

7. The compound of claim 1, wherein the compound or a form thereof is selected from:
8-(dimethylamino)-2-oxo-1,2,5,6-tetrahydrobenzo[h]quinoline-3-carboxylic acid,
9-(dimethylamino)-2-oxo-2,5,6,7-tetrahydro-1H-benzo[6,7]cyclohepta[1,2-b]pyridine-3-carboxylic acid,
9-(dimethylamino)-4-hydroxy-2-oxo-2,5,6,7-tetrahydro-1H-benzo[6,7]cyclohepta[1,2-b]pyridine-3-carboxylic acid,
10-(dimethylamino)-2-oxo-1,2,5,6,7,8-hexahydrobenzo[7,8]cycloocta[1,2-b]pyridine-3-carboxylic acid,
10-(dimethylamino)-4-hydroxy-2-oxo-1,2,5,6,7,8-hexahydrobenzo[7,8]cycloocta[1,2-b]pyridine-3-carboxylic acid,
4-hydroxy-2-oxo-9-(pyrrolidin-1-yl)-2,5,6,7-tetrahydro-1H-benzo[6,7]cyclohepta[1,2-b]pyridine-3-carboxylic acid,
5-methyl-2-oxo-8-(pyrrolidin-1-yl)-2,5-dihydro-1H-chromeno[4,3-b]pyridine-3-carboxylic acid,
9-(dimethylamino)-4-hydroxy-2-oxo-1,2,5,6-tetrahydrobenzo[2,3]oxepino[4,5-b]pyridine-3-carboxylic acid,
4-hydroxy-2-oxo-9-(pyrrolidin-1-yl)-1,2,5,6-tetrahydrobenzo[2,3]oxepino[4,5-b]pyridine-3-carboxylic acid,
2-oxo-9-(pyrrolidin-1-yl)-1,2,5,6-tetrahydrobenzo[2,3]oxepino[4,5-b]pyridine-3-carboxylic acid,
4-hydroxy-5-methyl-2-oxo-9-(pyrrolidin-1-yl)-1,2,5,6-tetrahydrobenzo[2,3]oxepino[4,5-b]pyridine-3-carboxylic acid,
4-hydroxy-7-methyl-2-oxo-9-(pyrrolidin-1-yl)-2,5,6,7-tetrahydro-1H-benzo[b]pyrido[2,3-d]azepine-3-carboxylic acid,
4-hydroxy-2-oxo-9-(pyrrolidin-1-yl)-2,5,6,7-tetrahydro-1H-benzo[b]pyrido[2,3-d]azepine-3-carboxylic acid,
4-hydroxy-5-methyl-2-oxo-8-(pyrrolidin-1-yl)-1,2,5,6-tetrahydrobenzo[h]quinoline-3-carboxylic acid,
8-(dimethylamino)-4-hydroxy-5-methyl-2-oxo-1,2,5,6-tetrahydrobenzo[h]quinoline-3-carboxylic acid,
4-hydroxy-2-oxo-10-(pyrrolidin-1-yl)-1,2,5,6,7,8-hexahydrobenzo[7,8]cycloocta[1,2-b]pyridine-3-carboxylic acid,
4-hydroxy-2-oxo-10-(propylamino)-1,2,5,6,7,8-hexahydrobenzo[7,8]cycloocta[1,2-b]pyridine-3-carboxylic acid,
10-(ethylamino)-4-hydroxy-2-oxo-1,2,5,6,7,8-hexahydrobenzo[7,8]cycloocta[1,2-b]pyridine-3-carboxylic acid,
9-(dimethylamino)-4-hydroxy-2-oxo-1,2,5,6-tetrahydrobenzo[2,3]thiepino[4,5-b]pyridine-3-carboxylic acid, and
4-hydroxy-2-oxo-9-(pyrrolidin-1-yl)-1,2,5,6-tetrahydrobenzo[2,3]thiepino[4,5-b]pyridine-3-carboxylic acid;
wherein the form of the compound is selected from a free acid, free base, salt, hydrate, solvate, clathrate, isotopologue, racemate, enantiomer, diastereomer, stereoisomer, polymorph or tautomer form thereof.

8. The compound of claim 1, wherein the compound or a form thereof is selected from:
9-(3-(dimethylamino)pyrrolidin-1-yl)-4-hydroxy-2-oxo-2,5,6,7-tetrahydro-1H-benzo[6,7]cyclohepta[1,2-b]pyridine-3-carboxylic acid hydrochloride,
4-hydroxy-9-(3-(methylamino)pyrrolidin-1-yl)-2-oxo-2,5,6,7-tetrahydro-1H-benzo[6,7]cyclohepta[1,2-b]pyridine-3-carboxylic acid hydrochloride,
9-((cis,cis)-6-amino-3-azabicyclo[3.1.0]hexan-3-yl)-4-hydroxy-2-oxo-2,5,6,7-tetrahydro-1H-benzo[6,7]cyclohepta[1,2-b]pyridine-3-carboxylic acid trifluoroacetate,
9-((cis,cis)-6-(benzyl(methyl)amino)-3-azabicyclo [3.1.0]hexan-3-yl)-4-hydroxy-2-oxo-2,5,6,7-tetrahydro-1H-benzo[6,7]cyclohepta[1,2-b]pyridine-3-carboxylic acid hydrochloride,
4-hydroxy-9-((cis,cis)-6-(methylamino)-3-azabicyclo[3.1.0]hexan-3-yl)-2-oxo-2,5,6,7-tetrahydro-1H-benzo[6,7]cyclohepta[1,2-b]pyridine-3-carboxylic acid hydrochloride,
9-(3-(dimethylamino)pyrrolidin-1-yl)-4-hydroxy-5-methyl-2-oxo-2,5,6,7-tetrahydro-1H-benzo[6,7]cyclohepta[1,2-b]pyridine-3-carboxylic acid hydrochloride,
9-((cis,cis)-6-amino-3-azabicyclo[3.1.0]hexan-3-yl)-4-hydroxy-5-methyl-2-oxo-2,5,6,7-tetrahydro-1H-benzo[6,7]cyclohepta[1,2-b]pyridine-3-carboxylic acid hydrochloride,
9-((cis,cis)-6-amino-3-azabicyclo[3.1.0]hexan-3-yl)-4,7-dihydroxy-2-oxo-2,5,6,7-tetrahydro-1H-benzo[6,7]cyclohepta[1,2-b]pyridine-3-carboxylic acid trifluoroacetate,
9-(3-(dimethylamino)pyrrolidin-1-yl)-4,7-dihydroxy-2-oxo-2,5,6,7-tetrahydro-1H-benzo[6,7]cyclohepta[1,2-b]pyridine-3-carboxylic acid hydrochloride,
2-((ethylamino)methyl)-8-hydroxy-1-methyl-6-oxo-1,5,6,9-tetrahydropyrido[3',2':4,5]cyclopenta[1,2-f]indole-7-carboxylic acid hydrochloride,
4-hydroxy-8-methyl-9-((methylamino)methyl)-2-oxo-2,5,6,8-tetrahydro-1H-indolo[6,5-h]quinoline-3-carboxylic acid hydrochloride,
9-(azetidin-1-ylmethyl)-4-hydroxy-8-methyl-2-oxo-2,5,6,8-tetrahydro-1H-indolo[6,5-h]quinoline-3-carboxylic acid hydrochloride,
4-hydroxy-9-methyl-10-((methylamino)methyl)-2-oxo-1,2,5,6,7,9-hexahydropyrido[3',2':6,7]cyclohepta[1,2-f]indole-3-carboxylic acid hydrochloride,
10-((cyclobutylamino)methyl)-4-hydroxy-9-methyl-2-oxo-1,2,5,6,7,9-hexahydropyrido[3',2':6,7]cyclohepta[1,2-f]indole-3-carboxylic acid hydrochloride,
10-(azetidin-1-ylmethyl)-4-hydroxy-9-methyl-2-oxo-1,2,5,6,7,9-hexahydropyrido[3',2':6,7]cyclohepta[1,2-f]indole-3-carboxylic acid hydrochloride,
10-((ethylamino)methyl)-4-hydroxy-9-methyl-2-oxo-1,2,5,6,7,9-hexahydropyrido[3',2':6,7]cyclohepta[1,2-f]indole-3-carboxylic acid hydrochloride,
4-hydroxy-10-((isopropylamino)methyl)-9-methyl-2-oxo-1,2,5,6,7,9-hexahydropyrido[3',2':6,7]cyclohepta[1,2-f]indole-3-carboxylic acid hydrochloride,
10-((tert-butylamino)methyl)-4-hydroxy-9-methyl-2-oxo-1,2,5,6,7,9-hexahydropyrido[3',2':6,7]cyclohepta[1,2-f]indole-3-carboxylic acid hydrochloride,
4-hydroxy-10-((4-hydroxypiperidin-1-yl)methyl)-9-methyl-2-oxo-1,2,5,6,7,9-exahydropyrido[3',2':6,7]cyclohepta[1,2-f]indole-3-carboxylic acid hydrochloride,
10-((4-aminopiperidin-1-yl)methyl)-4-hydroxy-9-methyl-2-oxo-1,2,5,6,7,9-exahydropyrido[3',2':6,7]cyclohepta[1,2-f]indole-3-carboxylic acid dihydrochloride,
10-((4-(dimethylamino)piperidin-1-yl)methyl)-4-hydroxy-9-methyl-2-oxo-1,2,5,6,7,9-hexahydropyrido[3',2':6,7]cyclohepta[1,2-f]indole-3-carboxylic acid dihydrochloride,
10-((3-aminopiperidin-1-yl)methyl)-4-hydroxy-9-methyl-2-oxo-1,2,5,6,7,9-hexahydropyrido[3',2':6,7]cyclohepta[1,2-f]indole-3-carboxylic acid dihydrochloride,
10-(((cyclopropylmethyl)amino)methyl)-4-hydroxy-9-methyl-2-oxo-1,2,5,6,7,9-hexahydropyrido[3',2':6,7]cyclohepta[1,2-f]indole-3-carboxylic acid hydrochloride,
4-hydroxy-9-methyl-10-(((1-methylcyclopropyl)amino)methyl)-2-oxo-1,2,5,6,7,9-hexahydropyrido[3',2':6,7]cyclohepta[1,2-f]indole-3-carboxylic acid hydrochloride,
10-((benzylamino)methyl)-4-hydroxy-9-methyl-2-oxo-1,2,5,6,7,9-hexahydropyrido[3',2':6,7]cyclohepta[1,2-f]indole-3-carboxylic acid hydrochloride,
10-(aminomethyl)-4-hydroxy-9-methyl-2-oxo-1,2,5,6,7,9-hexahydropyrido[3',2':6,7]cyclohepta[1,2-f]indole-3-carboxylic acid hydrochloride,
10-((cyclopropylamino)methyl)-4-hydroxy-9-methyl-2-oxo-1,2,5,6,7,9-hexahydropyrido[3',2':6,7]cyclohepta[1,2-f]indole-3-carboxylic acid hydrochloride,
10-(((cyclobutylmethyl)amino)methyl)-4-hydroxy-9-methyl-2-oxo-1,2,5,6,7,9-hexahydropyrido[3',2':6,7]cyclohepta[1,2-f]indole-3-carboxylic acid hydrochloride,
4-hydroxy-9-methyl-10-((((2-methylcyclopropyl)methyl)amino)methyl)-2-oxo-1,2,5,6,7,9-hexahydropyrido[3',2':6,7]cyclohepta[1,2-f]indole-3-carboxylic acid hydrochloride,
4-hydroxy-9-methyl-2-oxo-10-(((pyridin-4-ylmethyl)amino)methyl)-1,2,5,6,7,9-hexahydropyrido[3',2':6,7]cyclohepta[1,2-f]indole-3-carboxylic acid hydrochloride,
4-hydroxy-9-methyl-2-oxo-10-(piperidin-1-ylmethyl)-1,2,5,6,7,9-hexahydropyrido[3',2':6,7]cyclohepta[1,2-f]indole-3-carboxylic acid hydrochloride,
4-hydroxy-9-methyl-10-(morpholinomethyl)-2-oxo-1,2,5,6,7,9-hexahydropyrido[3',2':6,7]cyclohepta[1,2-f]indole-3-carboxylic acid hydrochloride,
4-hydroxy-9-methyl-10-((4-methylpip erazin-1-yl)methyl)-2-oxo-1,2,5,6,7,9-hexahydropyrido[3',2':6,7]cyclohepta[1,2-f]indole-3-carboxylic acid dihydrochloride,
10-((diethylamino)methyl)-4-hydroxy-9-methyl-2-oxo-1,2,5,6,7,9-hexahydropyrido[3',2':6,7]cyclohepta[1,2-f]indole-3-carboxylic acid hydrochloride,
4-hydroxy-9-methyl-2-oxo-10-((propylamino)methyl)-1,2,5,6,7,9-hexahydropyrido[3',2':6,7]cyclohepta[1,2-f]indole-3-carboxylic acid hydrochloride,
4-hydroxy-9-methyl-2-oxo-10-((prop-2-yn-1-ylamino)methyl)-1,2,5,6,7,9-hexahydropyrido[3',2':6,7]cyclohepta[1,2-f]indole-3-carboxylic acid hydrochloride,
(R)-10-((3-fluoropyrrolidin-1-yl)methyl)-4-hydroxy-9-methyl-2-oxo-1,2,5,6,7,9-hexahydropyrido[3',2':6,7]cyclohepta[1,2-f]indole-3-carboxylic acid hydrochloride,
10-((3-(dimethylamino)pyrrolidin-1-yl)methyl)-4-hydroxy-9-methyl-2-oxo-1,2,5,6,7,9-hexahydropyrido[3',2':6,7]cyclohepta[1,2-f]indole-3-carboxylic acid dihydrochloride,
10-((dimethylamino)methyl)-4-hydroxy-9-methyl-2-oxo-1,2,5,6,7,9-hexahydropyrido[3',2':6,7]cyclohepta[1,2-f]indole-3-carboxylic acid hydrochloride,
(S)-10-((3-aminopyrrolidin-1-yl)methyl)-4-hydroxy-9-methyl-2-oxo-1,2,5,6,7,9-hexahydropyrido[3',2':6,7]cyclohepta[1,2-f]indole-3-carboxylic acid dihydrochloride,
4-hydroxy-9-methyl-2-oxo-10-(pyrrolidin-1-ylmethyl)-1,2,5,6,7,9-hexahydropyrido[3',2':6,7]cyclohepta[1,2-f]indole-3-carboxylic acid hydrochloride,
4-hydroxy-10-((3-hydroxypyrrolidin-1-yl)methyl)-9-methyl-2-oxo-1,2,5,6,7,9-hexahydropyrido[3',2':6,7]cyclohepta[1,2-f]indole-3-carboxylic acid hydrochloride,
4-hydroxy-9-methyl-2-oxo-10-(((pyridin-3-ylmethyl)amino)methyl)-1,2,5,6,7,9-hexahydropyrido[3',2':6,7]cyclohepta[1,2-f]indole-3-carboxylic acid hydrochloride,
9-methyl-10-((methylamino)methyl)-2-oxo-1,2,5,6,7,9-hexahydropyrido[3',2':6,7]cyclohepta[1,2-f]indole-3-carboxylic acid hydrochloride,
10-((ethylamino)methyl)-9-methyl-2-oxo-1,2,5,6,7,9-hexahydropyrido[3',2':6,7]cyclohepta[1,2-f]indole-3-carboxylic acid hydrochloride,
10-((isopropylamino)methyl)-9-methyl-2-oxo-1,2,5,6,7,9-hexahydropyrido[3',2':6,7]cyclohepta[1,2-f]indole-3-carboxylic acid hydrochloride,
10-((tert-butylamino)methyl)-9-methyl-2-oxo-1,2,5,6,7,9-hexahydropyrido[3',2':6,7]cyclohepta[1,2-f]indole-3-carboxylic acid hydrochloride,
10-(azetidin-1-ylmethyl)-9-methyl-2-oxo-1,2,5,6,7,9-hexahydropyrido[3',2':6,7]cyclohepta[1,2-f]indole-3-carboxylic acid hydrochloride,
4-hydroxy-10-methyl-11-((methylamino)methyl)-2-oxo-2,5,6,7,8,10-hexahydro-1H-pyrido[3',2':7,8]cycloocta[1,2-f]indole-3-carboxylic acid hydrochloride,
11-((ethylamino)methyl)-4-hydroxy-10-methyl-2-oxo-2,5,6,7,8,10-hexahydro-1H-pyrido[3',2':7,8]cycloocta[1,2-f]indole-3-carboxylic acid hydrochloride,
7-hydroxy-1-methyl-2-((methylamino)methyl)-9-oxo-1,4,5,6,9,10-hexahydropyrido[2',3':3,4]cyclohepta[1,2-e]indole-8-carboxylic acid hydrochloride,
2-((ethylamino)methyl)-7-hydroxy-1-methyl-9-oxo-1,4,5,6,9,10-hexahydropyrido[2',3':3,4]cyclohepta[1,2-e]indole-8-carboxylic acid hydrochloride,
4-hydroxy-7,9-dimethyl-10-((methylamino)methyl)-2-oxo-1,2,5,6,7,9-hexahydropyrido[3',2':6,7]cyclohepta[1,2-f]indole-3-carboxylic acid hydrochloride,
10-((ethylamino)methyl)-4-hydroxy-7,9-dimethyl-2-oxo-1,2,5,6,7,9-hexahydropyrido[3',2':6,7]cyclohepta[1,2-f]indole-3-carboxylic acid hydrochloride,
4-hydroxy-10-((isopropylamino)methyl)-7,9-dimethyl-2-oxo-1,2,5,6,7,9-hexahydropyrido[3',2':6,7]cyclohepta[1,2-f]indole-3-carboxylic acid hydrochloride,
10-((tert-butylamino)methyl)-4-hydroxy-7,9-dimethyl-2-oxo-1,2,5,6,7,9-hexahydropyrido[3',2':6,7]cyclohepta[1,2-f]indole-3-carboxylic acid hydrochloride,
4-hydroxy-7,9-dimethyl-10-(((1-methylcyclopropyl)amino)methyl)-2-oxo-1,2,5,6,7,9-hexahydropyrido[3',2':6,7]cyclohepta[1,2-f]indole-3-carboxylic acid hydrochloride,
10-((ethylamino)methyl)-4-hydroxy-5,9-dimethyl-2-oxo-1,2,5,6,7,9-hexahydropyrido[3',2':6,7]cyclohepta[1,2-f]indole-3-carboxylic acid hydrochloride,
4-hydroxy-9-methyl-10-((methylamino)methyl)-2-oxo-1,2,5,9-tetrahydropyrido[3',2':6,7]cyclohepta[1,2-f]indole-3-carboxylic acid hydrochloride,
10-((ethylamino)methyl)-4-hydroxy-9-methyl-2-oxo-1,2,5,9-tetrahydropyrido[3',2':6,7]cyclohepta[1,2-f]indole-3-carboxylic acid hydrochloride,
10-((ethylamino)methyl)-4-hydroxy-7,9-dimethyl-2-oxo-1,2,5,9-tetrahydropyrido[3',2':6,7]cyclohepta[1,2-f]indole-3-carboxylic acid hydrochloride,
(cis)-10-((ethylamino)methyl)-4,6,7-trihydroxy-9-methyl-2-oxo-1,2,5,6,7,9-hexahydropyrido[3',2':6,7]cyclohepta[1,2-f]indole-3-carboxylic acid hydrochloride,
8-(3-(dimethylamino)pyrrolidin-1-yl)-5-methyl-2-oxo-2,5-dihydro-1H-chromeno[4,3-b]pyridine-3-carboxylic acid hydrochloride,
9-(3-(dimethylamino)pyrrolidin-1-yl)-4-hydroxy-2-oxo-1,2,5,6-tetrahydrobenzo[2,3]oxepino[4,5-b]pyridine-3-carboxylic acid hydrochloride,
9-((cis,cis)-6-(dibenzylamino)-3-azabicyclo[3.1.0]hexan-3-yl)-4-hydroxy-2-oxo-1,2,5,6-tetrahydrobenzo[2,3]oxepino[4,5-b]pyridine-3-carboxylic acid trifluoroacetate,
9-((cis,cis)-6-amino-3-azabicyclo[3.1.0]hexan-3-yl)-4-hydroxy-2-oxo-1,2,5,6-tetrahydrobenzo[2,3]oxepino[4,5-b]pyridine-3-carboxylic acid hydrochloride,
9-(3-(dimethylamino)pyrrolidin-1-yl)-2-oxo-1,2,5,6-tetrahydrobenzo[2,3]oxepino[4,5-b]pyridine-3-carboxylic acid hydrochloride,
9-(3-(dimethylamino)pyrrolidin-1-yl)-4-hydroxy-5-methyl-2-oxo-1,2,5,6-tetrahydrobenzo[2,3]oxepino[4,5-b]pyridine-3-carboxylic acid hydrochloride,
9-((cis,cis)-6-(dibenzylamino)-3-azabicyclo[3.1.0]hexan-3-yl)-4-hydroxy-5-methyl-2-oxo-1,2,5,6-tetrahydrobenzo[2,3]oxepino[4,5-b]pyridine-3-carboxylic acid hydrochloride,
9-((cis,cis)-6-amino-3-azabicyclo[3.1.0]hexan-3-yl)-4-hydroxy-5-methyl-2-oxo-1,2,5,6-tetrahydrobenzo[2,3]oxepino[4,5-b]pyridine-3-carboxylic acid hydrochloride,
4-hydroxy-9-methyl-10-((methylamino)methyl)-2-oxo-2,5,6,9-tetrahydro-1H-pyrido[2',3':4,5]oxepino[3,2-f]indole-3-carboxylic acid hydrochloride,
10-((ethylamino)methyl)-4-hydroxy-9-methyl-2-oxo-2,5,6,9-tetrahydro-1H-pyrido[2',3':4,5]oxepino[3,2-f]indole-3-carboxylic acid hydrochloride,
4-hydroxy-10-((isopropylamino)methyl)-9-methyl-2-oxo-2,5,6,9-tetrahydro-1H-pyrido[2',3':4,5]oxepino[3,2-f]indole-3-carboxylic acid hydrochloride,
10-(azetidin-1-ylmethyl)-4-hydroxy-9-methyl-2-oxo-2,5,6,9-tetrahydro-1H-pyrido[2',3':4,5]oxepino[3,2-f]indole-3-carboxylic acid hydrochloride,
10-((ethylamino)methyl)-4-hydroxy-5,9-dimethyl-2-oxo-2,5,6,9-tetrahydro-1H-pyrido[2',3':4,5]oxepino[3,2-f]indole-3-carboxylic acid hydrochloride,
9-(3-(dimethylamino)pyrrolidin-1-yl)-4-hydroxy-2-oxo-2,5,6,7-tetrahydro-1H-benzo[b]pyrido[2,3-d]azepine-3-carboxylic acid hydrochloride,
4-hydroxy-9-(4-methylpiperazin-1-yl)-2-oxo-2,5,6,7-tetrahydro-1H-benzo[b]pyrido[2,3-d]azepine-3-carboxylic acid hydrochloride,
9-((cis,cis)-6-amino-3-azabicyclo[3.1.0]hexan-3-yl)-4-hydroxy-2-oxo-2,5,6,7-tetrahydro-1H-benzo[b]pyrido[2,3-d]azepine-3-carboxylic acid hydrochloride,
9-(hexahydro-1H-pyrrolo[3,4-b]pyridin-6(2H)-yl)-4-hydroxy-2-oxo-2,5,6,7-tetrahydro-1H-benzo[b]pyrido[2,3-d]azepine-3-carboxylic acid hydrochloride,
4-hydroxy-9-methyl-10-((methylamino)methyl)-2-oxo-1,2,5,6,7,9-hexahydropyrido[2',3':4,5]azepino[3,2-f]indole-3-carboxylic acid hydrochloride,
10-((ethylamino)methyl)-4-hydroxy-9-methyl-2-oxo-1,2,5,6,7,9-hexahydropyrido[2',3':4,5]azepino[3,2-f]indole-3-carboxylic acid hydrochloride,
4-hydroxy-10-((isopropylamino)methyl)-9-methyl-2-oxo-1,2,5,6,7,9-hexahydropyrido[2',3':4,5]azepino[3,2-f]indole-3-carboxylic acid hydrochloride,
10-((tert-butylamino)methyl)-4-hydroxy-9-methyl-2-oxo-1,2,5,6,7,9-hexahydropyrido[2',3':4,5]azepino[3,2-f]indole-3-carboxylic acid hydrochloride,
4-hydroxy-10-methyl-11-((methylamino)methyl)-2-oxo-2,5,6,7,8,10-hexahydro-1H-pyrido[2',3':4,5]azocino[3,2-f]indole-3-carboxylic acid hydrochloride,
11-((ethylamino)methyl)-4-hydroxy-10-methyl-2-oxo-2,5,6,7,8,10-hexahydro-1h-pyrido[2',3':4,5]azocino[3,2-f]indole-3-carboxylic acid hydrochloride,
4-hydroxy-11-((isopropylamino)methyl)-10-methyl-2-oxo-2,5,6,7,8,10-hexahydro-1H-pyrido[2',3':4,5]azocino[3,2-f]indole-3-carboxylic acid hydrochloride,
8-(3-(dimethylamino)pyrrolidin-1-yl)-4-hydroxy-5-methyl-2-oxo-1,2,5,6-tetrahydrobenzo[h]quinoline-3-carboxylic acid hydrochloride,
8-((cis,cis)-6-amino-3-azabicyclo[3.1.0]hexan-3-yl)-4-hydroxy-5-methyl-2-oxo-1,2,5,6-tetrahydrobenzo[h]quinoline-3-carboxylic acid trifluoroacetate,
9-(1,4-diazepan-1-yl)-4-hydroxy-2-oxo-2,5,6,7-tetrahydro-1H-benzo[6,7]cyclohepta[1,2-b]pyridine-3-carboxylic acid hydrochloride,
4-hydroxy-9-(4-methyl-1,4-diazepan-1-yl)-2-oxo-2,5,6,7-tetrahydro-1H-benzo[6,7]cyclohepta[1,2-b]pyridine-3-carboxylic acid hydrochloride,
9-((2-(dimethylamino)ethyl)amino)-4-hydroxy-2-oxo-2,5,6,7-tetrahydro-1H-benzo[6,7]cyclohepta[1,2-b]pyridine-3-carboxylic acid hydrochloride,
9-((4aR,7aR)-hexahydro-1H-pyrrolo[3,4-b]pyridin-6(2H)-yl)-4-hydroxy-2-oxo-2,5,6,7-tetrahydro-1H-benzo[6,7]cyclohepta[1,2-b]pyridine-3-carboxylic acid hydrochloride,
4-hydroxy-9-((4aR,7aR)-1-methylhexahydro-1H-pyrrolo[3,4-b]pyridin-6(2H)-yl)-2-oxo-2,5,6,7-tetrahydro-1H-benzo[6,7]cyclohepta[1,2-b]pyridine-3-carboxylic acid hydrochloride,
9-(4-(dimethylamino)piperidin-1-yl)-4-hydroxy-2-oxo-2,5,6,7-tetrahydro-1H-benzo[6,7]cyclohepta[1,2-b]pyridine-3-carboxylic acid hydrochloride,
9-(3-(dimethylamino)piperidin-1-yl)-4-hydroxy-2-oxo-2,5,6,7-tetrahydro-1H-benzo[6,7]cyclohepta[1,2-b]pyridine-3-carboxylic acid hydrochloride,
4-hydroxy-2-oxo-9-(piperidin-4-ylamino)-2,5,6,7-tetrahydro-1H-benzo[6,7]cyclohepta[1,2-b]pyridine-3-carboxylic acid hydrochloride,
4-hydroxy-2-oxo-9-(piperazin-1-yl)-2,5,6,7-tetrahydro-1H-benzo[6,7]cyclohepta[1,2-b]pyridine-3-carboxylic acid hydrochloride,
4-hydroxy-9-(4-methylpiperazin-1-yl)-2-oxo-2,5,6,7-tetrahydro-1H-benzo[6,7]cyclohepta[1,2-b]pyridine-3-carboxylic acid hydrochloride,
4-hydroxy-2-oxo-9-(2,6-diazaspiro[3.4]octan-6-yl)-2,5,6,7-tetrahydro-1H-benzo[6,7]cyclohepta[1,2-b]pyridine-3-carboxylic acid hydrochloride,
4-hydroxy-2-oxo-9-(2,7-diazaspiro[4.4]nonan-2-yl)-2,5,6,7-tetrahydro-1H-benzo[6,7]cyclohepta[1,2-b]pyridine-3-carboxylic acid hydrochloride,
4-hydroxy-9-(7-methyl-2,7-diazaspiro[4.4]nonan-2-yl)-2-oxo-2,5,6,7-tetrahydro-1H-benzo[6,7]cyclohepta[1,2-b]pyridine-3-carboxylic acid hydrochloride,
9-((cis,cis)-6-amino-3-azabicyclo[3.1.0]hexan-3-yl)-10,11-difluoro-4-hydroxy-2-oxo-2,5,6,7-tetrahydro-1H-benzo[6,7]cyclohepta[1,2-b]pyridine-3-carboxylic acid hydrochloride,
9-((cis,cis)-6-amino-3-azabicyclo[3.1.0]hexan-3-yl)-11-fluoro-4-hydroxy-2-oxo-2,5,6,7-tetrahydro-1H-benzo[6,7]cyclohepta[1,2-b]pyridine-3-carboxylic acid hydrochloride,
10-((cis,cis)-6-amino-3-azabicyclo[3.1.0]hexan-3-yl)-4-hydroxy-2-oxo-1,2,5,6,7,8-hexahydrobenzo[7,8]cycloocta[1,2-b]pyridine-3-carboxylic acid hydrochloride,
10-((butylamino)methyl)-4-hydroxy-9-methyl-2-oxo-1,2,5,6,7,9-hexahydropyrido[3',2':6,7]cyclohepta[1,2-f]indole-3-carboxylic acid hydrochloride,
4-hydroxy-9-methyl-2-oxo-10-((pentylamino)methyl)-1,2,5,6,7,9-hexahydropyrido[3',2':6,7]cyclohepta[1,2-f]indole-3-carboxylic acid hydrochloride,
10-((hexylamino)methyl)-4-hydroxy-9-methyl-2-oxo-1,2,5,6,7,9-hexahydropyrido[3',2':6,7]cyclohepta[1,2-f]indole-3-carboxylic acid hydrochloride,
10-((heptylamino)methyl)-4-hydroxy-9-methyl-2-oxo-1,2,5,6,7,9-hexahydropyrido[3',2':6,7]cyclohepta[1,2-f]indole-3-carboxylic acid hydrochloride,
4-hydroxy-9-methyl-10-((octylamino)methyl)-2-oxo-1,2,5,6,7,9-hexahydropyrido[3',2':6,7]cyclohepta[1,2-f]indole-3-carboxylic acid hydrochloride,
4-hydroxy-9-methyl-10-((nonylamino)methyl)-2-oxo-1,2,5,6,7,9-hexahydropyrido[3',2':6,7]cyclohepta[1,2-f]indole-3-carboxylic acid hydrochloride,
10-(((2-(dimethylamino)ethyl)amino)methyl)-4-hydroxy-9-methyl-2-oxo-1,2,5,6,7,9-hexahydropyrido[3',2':6,7]cyclohepta[1,2-f]indole-3-carboxylic acid dihydrochloride,
4-hydroxy-9-methyl-10-(((2-(methylamino)ethyl)amino)methyl)-2-oxo-1,2,5,6,7,9-hexahydropyrido[3',2':6,7]cyclohepta[1,2-f]indole-3-carboxylic acid dihydrochloride,
10-(((2-aminoethyl)amino)methyl)-4-hydroxy-9-methyl-2-oxo-1,2,5,6,7,9-hexahydropyrido[3',2':6,7]cyclohepta[1,2-f]indole-3-carboxylic acid dihydrochloride,
10-(((2-(dimethylamino)ethyl)(methyl)amino)methyl)-4-hydroxy-9-methyl-2-oxo-1,2,5,6,7,9-hexahydropyrido[3',2':6,7]cyclohepta[1,2-f]indole-3-carboxylic acid dihydrochloride,
10-((sec-butylamino)methyl)-4-hydroxy-9-methyl-2-oxo-1,2,5,6,7,9-hexahydropyrido[3',2':6,7]cyclohepta[1,2-f]indole-3-carboxylic acid hydrochloride,
4-hydroxy-10-(((2-hydroxyethyl)amino)methyl)-9-methyl-2-oxo-1,2,5,6,7,9-hexahydropyrido[3',2':6,7]cyclohepta[1,2-f]indole-3-carboxylic acid hydrochloride,
4-hydroxy-10-(((2-methoxyethyl)amino)methyl)-9-methyl-2-oxo-1,2,5,6,7,9-hexahydropyrido[3',2':6,7]cyclohepta[1,2-f]indole-3-carboxylic acid hydrochloride,
4-hydroxy-10-(((2-hydroxyethyl)(methyl)amino)methyl)-9-methyl-2-oxo-1,2,5,6,7,9-hexahydropyrido[3',2':6,7]cyclohepta[1,2-f]indole-3-carboxylic acid hydrochloride,
4-hydroxy-10-(((1-methoxypropan-2-yl)amino)methyl)-9-methyl-2-oxo-1,2,5,6,7,9-hexahydropyrido[3',2':6,7]cyclohepta[1,2-f]indole-3-carboxylic acid hydrochloride,
4-hydroxy-10-(((1-hydroxypropan-2-yl)amino)methyl)-9-methyl-2-oxo-1,2,5,6,7,9-hexahydropyrido[3',2':6,7]cyclohepta[1,2-f]indole-3-carboxylic acid hydrochloride,
4-hydroxy-9-methyl-2-oxo-10-(((2-(pyrrolidin-1-yl)ethyl)amino)methyl)-1,2,5,6,7,9-hexahydropyrido[3',2':6,7]cyclohepta[1,2-f]indole-3-carboxylic acid dihydrochloride,
10-((allylamino)methyl)-4-hydroxy-9-methyl-2-oxo-1,2,5,6,7,9-hexahydropyrido[3',2':6,7]cyclohepta[1,2-f]indole-3-carboxylic acid hydrochloride,
4-hydroxy-10-((isobutylamino)methyl)-9-methyl-2-oxo-1,2,5,6,7,9-hexahydropyrido[3',2':6,7]cyclohepta[1,2-f]indole-3-carboxylic acid hydrochloride,
4-hydroxy-9-methyl-10-((neopentylamino)methyl)-2-oxo-1,2,5,6,7,9-hexahydropyrido[3',2':6,7]cyclohepta[1,2-f]indole-3-carboxylic acid hydrochloride,
4-hydroxy-9-methyl-10-((4-methyl-1,4-diazepan-1-yl)methyl)-2-oxo-1,2,5,6,7,9-hexahydropyrido[3',2':6,7]cyclohepta[1,2-f]indole-3-carboxylic acid dihydrochloride,
4-hydroxy-9-methyl-10-(((1-methylpiperidin-4-yl)amino)methyl)-2-oxo-1,2,5,6,7,9-hexahydropyrido[3',2':6,7]cyclohepta[1,2-f]indole-3-carboxylic acid dihydrochloride,
4-hydroxy-10-((3-methoxypyrrolidin-1-yl)methyl)-9-methyl-2-oxo-1,2,5,6,7,9-hexahydropyrido[3',2':6,7]cyclohepta[1,2-f]indole-3-carboxylic acid hydrochloride,
10-((3-acetamidopyrrolidin-1-yl)methyl)-4-hydroxy-9-methyl-2-oxo-1,2,5,6,7,9-hexahydropyrido[3',2':6,7]cyclohepta[1,2-f]indole-3-carboxylic acid hydrochloride,
10-(((1-(dimethylamino)propan-2-yl)amino)methyl)-4-hydroxy-9-methyl-2-oxo-1,2,5,6,7,9-hexahydropyrido[3',2':6,7]cyclohepta[1,2-f]indole-3-carboxylic acid dihydrochloride,
4-hydroxy-9-methyl-2-oxo-10-((((tetrahydrofuran-2-yl)methyl)amino)methyl)-1,2,5,6,7,9-hexahydropyrido[3',2':6,7]cyclohepta[1,2-f]indole-3-carboxylic acid hydrochloride,
4-hydroxy-9-methyl-10-((1-methylhexahydropyrrolo[3,4-b]pyrrol-5(1H)-yl)methyl)-2-oxo-1,2,5,6,7,9-hexahydropyrido[3',2':6,7]cyclohepta[1,2-f]indole-3-carboxylic acid dihydrochloride,
10-(((2-(dimethylamino)-2-oxoethyl)amino)methyl)-4-hydroxy-9-methyl-2-oxo-1,2,5,6,7,9-hexahydropyrido[3',2':6,7]cyclohepta[1,2-f]indole-3-carboxylic acid hydrochloride,
10-(2-(ethylamino)ethyl)-4-hydroxy-9-methyl-2-oxo-1,2,5,9-tetrahydropyrido[3',2':6,7]cyclohepta[1,2-f]indole-3-carboxylic acid trifluoroacetate,
10-(2-(ethylamino)ethyl)-4-hydroxy-9-methyl-2-oxo-1,2,5,6,7,9-hexahydropyrido[3',2':6,7]cyclohepta[1,2-f]indole-3-carboxylic acid trifluoroacetate,
4-hydroxy-9-(((cis)-octahydrocyclopenta[c]pyrrol-4-yl)-(cis)-amino)-2-oxo-1,2,5,6-tetrahydrobenzo[2,3]oxepino[4,5-b]pyridine-3-carboxylic acid hydrochloride,
4-hydroxy-9-methyl-10-(((1-methylcyclopropyl)amino)methyl)-2-oxo-2,5,6,9-tetrahydro-1H-pyrido[2',3':4,5]oxepino[3,2-f]indole-3-carboxylic acid hydrochloride,
10-((sec-butylamino)methyl)-4-hydroxy-9-methyl-2-oxo-2,5,6,9-tetrahydro-1H-pyrido[2',3':4,5]oxepino[3,2-f]indole-3-carboxylic acid hydrochloride,
4-hydro xy-9-methyl-2-oxo-10-((propylamino)methyl)-2,5,6,9-tetrahydro-1H-pyrido[2',3':4,5]oxepino[3,2-f]indole-3-carboxylic acid hydrochloride,
10-(((2,4-dimethoxybenzyl)amino)methyl)-4-hydroxy-9-methyl-2-oxo-2,5,6,9-tetrahydro-1H-pyrido[2',3':4,5]oxepino[3,2-f]indole-3-carboxylic acid hydrochloride,
10-((cyclopropylamino)methyl)-4-hydroxy-9-methyl-2-oxo-2,5,6,9-tetrahydro-1H-pyrido[2',3':4,5]oxepino[3,2-f]indole-3-carboxylic acid hydrochloride,
10-((cyclobutylamino)methyl)-4-hydroxy-9-methyl-2-oxo-2,5,6,9-tetrahydro-1H-pyrido[2',3':4,5]oxepino[3,2-f]indole-3-carboxylic acid hydrochloride,
10-((dimethylamino)methyl)-4-hydroxy-9-methyl-2-oxo-2,5,6,9-tetrahydro-1H-pyrido[2',3':4,5]oxepino[3,2-f]indole-3-carboxylic acid hydrochloride,
4-hydroxy-9-methyl-2-oxo-10-(pyrrolidin-1-ylmethyl)-2,5,6,9-tetrahydro-1H-pyrido[2',3':4,5]oxepino[3,2-f]indole-3-carboxylic acid hydrochloride,
10-((tert-butylamino)methyl)-4-hydroxy-9-methyl-2-oxo-2,5,6,9-tetrahydro-1H-pyrido[2',3':4,5]oxepino[3,2-f]indole-3-carboxylic acid hydrochloride,
4-hydroxy-9-methyl-10-((4-methylpiperazin-1-yl)methyl)-2-oxo-2,5,6,9-tetrahydro-1H-pyrido[2',3':4,5]oxepino[3,2-f]indole-3-carboxylic acid dihydrochloride,
4-hydroxy-10-(((2-hydroxyethyl)amino)methyl)-9-methyl-2-oxo-2,5,6,9-tetrahydro-1H-pyrido[2',3':4,5]oxepino[3,2-f]indole-3-carboxylic acid hydrochloride,
4-hydroxy-10-(((1-hydroxypropan-2-yl)amino)methyl)-9-methyl-2-oxo-2,5,6,9-tetrahydro-1H-pyrido[2',3':4,5]oxepino[3,2-f]indole-3-carboxylic acid hydrochloride,
4-hydroxy-9-methyl-2-oxo-10-(((2-(pyrrolidin-1-yl)ethyl)amino)methyl)-2,5,6,9-tetrahydro-1H-pyrido[2',3':4,5]oxepino[3,2-f]indole-3-carboxylic acid dihydrochloride,
10-(((2-aminoethyl)amino)methyl)-4-hydroxy-9-methyl-2-oxo-2,5,6,9-tetrahydro-1H-pyrido[2',3':4,5]oxepino[3,2-f]indole-3-carboxylic acid dihydrochloride,
4-hydroxy-9-methyl-10-(((2-(methylamino)ethyl)amino)methyl)-2-oxo-2,5,6,9-tetrahydro-1H-pyrido[2',3':4,5]oxepino[3,2-f]indole-3-carboxylic acid dihydrochloride,
10-(((2-(dimethylamino)ethyl) amino)methyl)-4-hydroxy-9-methyl-2-oxo-2,5,6,9-tetrahydro-1H-pyrido[2',3':4,5]oxepino[3,2-flindole-3-carboxylic acid dihydrochloride,
4-hydroxy-10-(((2-methoxyethyl)amino)methyl)-9-methyl-2-oxo-2,5,6,9-tetrahydro-1H-pyrido[2',3':4,5]oxepino[3,2-f]indole-3-carboxylic acid hydrochloride, and
4-hydroxy-10-(((1-methoxypropan-2-yl)amino)methyl)-9-methyl-2-oxo-2,5,6,9-tetrahydro-1H-pyrido[2',3':4,5]oxepino[3,2-f]indole-3-carboxylic acid hydrochloride;
wherein the form of the compound is selected from a free acid, free base, hydrate, solvate, clathrate, isotopologue, racemate, enantiomer, diastereomer, stereoisomer, polymorph or tautomer form thereof.

9. The compound of claim 1, for use in the treatment of a bacterial infection.

10. The compound for use according to claim 9, wherein the bacterial infection results from a bacteria that is a multi-drug resistant Gram-negative or Gram-positive type.

11. The compound for use according to claim 9, wherein the treatment is a combination therapy comprising an effective amount of a compound of claim 1 or a form thereof and an effective amount of an antibiotic or antibacterial agent.

12. A pharmaceutical composition comprising an effective amount of a compound of claim 1 or a form thereof in admixture with a pharmaceutically acceptable excipient.

13. The compound of claim 1, for use as a medicament.

## Patentansprüche

1. Verbindung mit der Formel (I), Formel (II) oder Formel (III) : 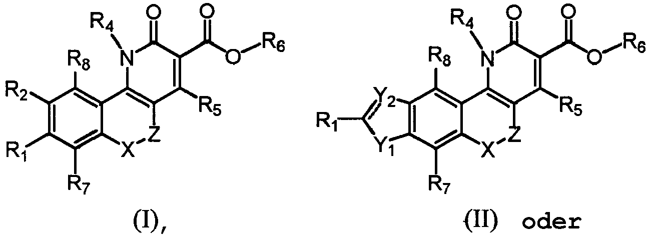 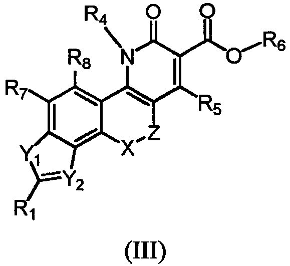 oder eine Form davon, wobei die Form der Verbindung aus einer isotopologischen, racemischen, enantiomeren, diastereomeren, stereoisomeren, polymorphen, tautomeren, Freien-Säure-, Freien-Basen-, Salz-, Hydrat-, Solvat- oder Clathratform davon ausgewählt ist, worin:
- X eine Bindung, N(R₁₄), S, O, -CH(R₉)-, -CH(R₉)-CH(R₁₀)-, -CH(R₉)-CH(R₁₀)-CH(R₁₁)-, -C(R₉) =C(R₁₀)-, -C(R₉) =C(R₁₀)-CH(11)-, -CH(R₉)-C(R₁₀) =C(R₁₁)-, -O-CH(R₁₀)-, -CH(R₉)-O-, -N(R₁₄)-CH(R₁₀)-, -CH(R₉)-N(R₁₄)-, -S-CH(R₁₀)-, -CH(R₉)-S-, -O-CH(R₁₀)-CH(R₁₁)-, -CH(R₉)-O-CH(R₁₁)-, -CH (R₉) -CH(R₁₀)-O-, -N(R₁₄)-CH(R₁₀)-CH(R₁₁)-, -CH (R₉)-N(R₁₄)-CH (R₁₁)-, -CH (R₉)-CH (R₁₀)-N(R₁₄)-, -S-CH(R₁₀)-CH(R₁₁)-, -CH(R₉)-S-CH(R₁₁)-, -CH(R₉)-CH(R₁₀)-S-, -O-C(O)-CH(R₁₁)-, -C(O)-O-CH(R₁₁)-, -CH(R₉)-O-C(O)-, -CH(R₉)-C(O)-O-, -N(R₁₄)-C(O)-CH(R₁₁)-, -C(O)-N(R₁₄)-CH(R₁₁)-, -CH(R₉)-N(R₁₄)-C(O)-, -CH(R₉)-C(O)-N(R₁₄)-, -S-C(O)-CH(R₁₁)-, C(O)-S-CH(R₁₁)-, CH(R₉)-S-C(O)-oder -CH(R₉)-C(O)-S-,
- Y₁ -N(R₁₂)- oder -O-,
- Y₂ -C(R₁₃)-, -N(R₁₂)- oder -O-, wobei die Strichellinie eine Doppelbindung bedeutet, die vorhanden ist, wenn Y₂ -C(R₁₃)- bedeutet, und fehlt, wenn Y₂ -N(R₁₂)- oder -O- bedeutet,
- Z N(R₁₄), S, O, C(O) oder -CH(R₃)- und
- R₁ Halogen, Hydroxyl, Oxo, Cyano, Nitro, C₁- bis C₈-Alkyl, Hydroxyl-C₁- bis C₈-alkyl, Halogen-C₁- bis C₈-alkyl, C₁- bis C₈-Alkoxy, Halogen-C₁- bis C₈-alkoxy, C₁- bis C₈-Alkylthio, Carboxyl, C₁- bis C₈-Alkyl-carbonyl, C₁- bis C₈-Alkoxy-carbonyl, Aminocarbonyl, Amino, C₁- bis C₈-Alkylamino, (C₁- bis C₈-Alkyl)2-amino, C₂- bis C₈-Alkenylamino, (C₂- bis C₈-Alkenyl)₂-amino, C₂- bis C₈-Alkinylamino, (C₂- bis C₈-Alkinyl)₂-amino, Amino-C₁- bis C₈-Alkyl, C₁- bis C₁₀-Alkyl-amino-C₁- bis C₈-alkyl, (C₁- bis C₈-Alkyl)₂-amino-C₁- bis C₈-alkyl, C₂- bis C₈-Alkenyl-amino-C₁- bis C₈-alkyl, (C₂- bis C₈-Alkenyl)₂-amino-C₁- bis C₈-alkyl, C₂- bis C₈-Alkinyl-amino-C₁- bis C₈-alkyl, (C₂- bis C₈-Alkinyl)₂-amino-C₁- bis C₈-alkyl, Halogen-C₁- bis C₈-alkylamino, (Halogen-C₁- bis C₈-alkyl)₂-amino, Halogen-C₁- bis C₈-alkyl-amino- C₁- bis C₈-alkyl, (Halogen-C₁- bis C₈-alkyl)₂-amino-C₁- bis C₈-alkyl, C₁- bis C₈-Alkoxy-C₁- bis C₈-alkylamino, (C₁- bis C₈-Alkoxy-C₁- bis C₈-alkyl, C₁- bis C₈-alkyl)-amino, (C₁- bis C₈-Alkoxy-C₁- bis C₈-Alkyl)₂-amino, C₁- bis C₈-Alkoxy-C₁- bis C₈-alkyl-amino-C₁- bis C₈-alkyl, (C₁- bis C₈-Alkoxy-C₁- bis C₈-alkyl,C₁- bis C₈-alkyl)-amino-C₁- bis C₈-alkyl, (C₁-bis C₈-Alkoxy-C₁- bis C₈-alkyl)₂-amino-C₁- bis C₈-alkyl, Amino-C₁-bis C₈-alkyl-amino, (Amino-C₁- bis C₈-alkyl,C₁- bis C₈-alkyl)amino, (C₁- bis C₈-Alkyl-amino-C₁- bis C₈-alkyl-amino, (C₁- bis C₈-Alkyl-amino-C₁- bis C₈-alkyl, C₁- bis C₈-alkyl)amino, (C₁- bis C₈-Alkyl)₂-amino-C₁- bis C₈-alkyl-amino, [(C₁- bis C₈-Alkyl)₂-amino-C₁- bis C₈-alkyl,C₁- bis C₈-alkyl]amino, Amino-C₁- bis C₈-alkyl-amino-C₁- bis C₈-alkyl, (Amino-C₁-bis C₈-alkyl, C₁- bis C₈-alkyl) amino-C₁- bis C₈-alkyl, C₁- bis C₈-Alkyl-amino-C₁- bis C₈-alkyl-amino-C₁- bis C₈-alkyl, (C₁- bis C₈-Alkyl-amino-C₁- bis C₈-alkyl,C₁- bis C₈-alkyl)amino-C₁- bis C₈-alkyl, (C₁- bis C₈-Alkyl)₂-amino, [(C₁- bis C₈-Alkyl)₂-amino-C₁- bis C₈-alkyl,C₁- bis C₈-alkyl]amino-C₁- bis C₈-alkyl, Hydroxyl-amino, Hydroxyl-C₁- bis C₈-alkylamino, (Hydroxyl-C₁- bis C₈-alkyl, C₁- bis C₈-alkyl)amino, (Hydroxyl-C₁- bis C₈-alkyl)₂-amino, Hydroxyl-C₁- bis C₈-alkyl-amino-C₁- bis C₈-alkyl, (Hydroxyl-C₁- bis C₈-alkyl,C₁- bis C₈-alkyl)amino-C₁-bis C₈-alkyl, Hydroxyl-C₁- bis C₈-alkylamino-C₁- bis C₈-alkylamino, (Hydroxyl-C₁- bis C₈-alkylamirio-C₁-bis C₈-alkyl,C₁- bis C₈-alkyl)amino, (Hydroxyl-C₁-bis C₈-alkyl, C₁- bis C₈-alkyl)amino-C₁- bis C₈-alkylamino, [(Hydroxyl-C₁- bis C₈-alkyl,C₁- bis C₈-alkyl)amino-C₁- bis C₈-alkyl,C₁- bis C₈-alkyl]amino, (C₁- bis C₈-Alkyl-carbonyl, C₁- bis C₈_alkyl) amino-C₁- bis C₈-alkyl, C₁- bis C₈-Alkylaminocarbonyl, (C₁- bis C₈-Alkyl)₂-amino-carbonyl, (C₁- bis C₈-Alkyl)₂-amino-carbonyl-C₁- bis C₈-alkylamino-C₁- bis C₈-alkyl, C₃- bis C₁₄-Cycloalkyl, C₃- bis C₁₄-Cycloalkyl-C₁- bis C₈-alkyl, C₃- bis C₁₄-Cycloalkyloxy, C₃- bis C₁₄-Cycloalkyl-C₁- bis C₈-alkoxy, C₃- bis C₁₄-Cycloalkylamino, C₃- bis C₁₄-Cycloalkylamino-C₁- bis C₈-alkyl, (C₃- bis C₁₄-Cycloalkyl, C₁- bis C₈-alkyl)amino-C₁- bis C₈-alkyl, (C₃- bis C₁₄-Cycloalkyl)₂-amino-C₁- bis C₈-alkyl), C₃- bis C₁₄-Cycloalkyl-C₁- bis C₈-alkylamino-C₁- bis C₈-alkyl, (C₃- bis C₁₄-Cycloalkyl-C₁- bis C₈-alkyl, C₁- bis C₈-alkyl)amino-C₁- bis C₈-alkyl, (C₃- bis C₁₄-Cycloalkyl-C₁- bis C₈-alkyl)₂-amino-C₁- bis C₈-alkyl, Aryl, Aryl-C₁- bis C₈-alkyl, Arylamino, (Aryl, C₁- bis C₈-alkyl)amino, (Aryl)₂-amino, Aryl-amino-C₁- bis C₈-alkyl, (Aryl, C₁- bis C₈-alkyl)amino-C₁- bis C₈-alkyl, (Aryl)₂-amino-C₁- bis C₈-alkyl, Aryl-C₁- bis C₈-alkylamino, (Aryl-C₁- bis C₈-alkyl, C₁- bis C₈-alkyl)amino, (Aryl-C₁- bis C₈-alkyl) 2-amino, Aryl-C₁- bis C₈-alkylamino-C₁- bis C₈-alkyl, (Aryl-C₁- bis C₈-alkyl, C₁- bis C₈-alkyl) amino-C₁- bis C₈-alkyl, (Aryl-C₁- bis C₈-alkyl)₂-amino-C₁- bis C₈-alkyl, Heteroaryl, das aus der Gruppe ausgewählt ist, die aus Pyrrolyl, Thiazolyl, 1H-1,2,3-Triazolyl, 1H-Tetrazolyl, 2H-Tetrazolyl, Imidazolyl und Pyridinyl besteht, Heteroaryl-C₁- bis C₈-alkyl, Heteroarylamino, Heteroaryl-C₁- bis C₈-alkylamino, (Heteroaryl-C₁- bis C₈-alkyl, C₁- bis C₈-alkyl) amino, (Heteroaryl-C₁- bis C₈-alkyl)₂-amino, Heteroaryl-C₁- bis C₈-alkylamino-C₁- bis C₈-alkyl, (Heteroaryl-C₁- bis C₈-alkyl, C₁- bis C₈-alkyl) amino-C₁- bis C₈-alkyl, Heterocyclyl, das aus der Gruppe ausgewählt ist, die aus Azetidinyl, Pyrrolidinyl, Tetrahydrofuranyl, Piperidinyl, Piperazinyl, Morpholinyl, 1,4-Diazepanyl, 1,3-Dioxolanyl, 2,5-Dihydro-1H-pyrrolyl, Dihydro-1H-imidazolyl, 1,4,5,6-Tetrahydropyrimidinyl, 1,2,3,6-Tetrahydropyridinyl, Tetrahydro-2H-pyranyl, Indolinyl, 2,3-Dihydrobenzo[*d*]oxazolyl, 3,4-Dihydro-2H-benzo[*b*][1,4]oxazinyl, 3,4-Dihydroisochinolin-(1H)-yl, 1,2,3,4-Tetrahydroisochinolinyl, 1,2,3,4-Tetrahydrochinoxalinyl, Hexahydropyrrolo[3,4-*b*][1,4]oxazin-(2H)-yl, (4aR,7aS)-Hexahydropyrrolo[3,4-*b*][1,4]oxazin-(4aH)-yl, 3,4-Dihydro-2H-pyrido[3,2-*b*][1,4]oxazinyl, (*cis*)-Octahydrocyclopenta[*c*]pyrrolyl, Hexahydropyrrolo[3,4-*b*]pyrrol-(1H)-yl, (3aR,6aR)-Hexahydropyrrolo[3,4-*b*]pyrrol-(1H)-yl, (3aR,6aS)-Hexahydropyrrolo[3,4-*c*]pyrrol-(1H)-yl, 5H-Pyrrolo[3,4-*b*]pyridin-(7H)-yl, 5,7-Dihydro-6H-pyrrolo[3,4-*b*]pyridinyl, Tetrahydro-1H-pyrrolo[3,4-*b*]pyridin-(2H,7H,7aH)-yl, Hexahydro-1H-pyrrolo[3,4-*b*]pyridin-(2H)-yl, (4aR,7aR)-Hexahydro-1H-pyrrolo[3,4-b]pyridin-(2H)-yl, Octahydro-6H-pyrrolo[3,4-*b*]pyridinyl, 2,3,4,9-Tetrahydro-1H-carbazolyl, 1,2,3,4-Tetrahydropyrazino[1,2-*a*]indol-8-yl, 2,3-Dihydro-1H-pyrrolo[1,2*a*]indolyl, (3aR,6aR)-Hexahydrocyclopenta[*c*]pyrrol-(1H)-yl, (3aR,4R,6aS)-Hexahydrocyclopenta[*c*]pyrrol-(1H)-yl, (3aR,4S,6aS)-Hexahydrocyclopenta[*c*]pyrrol-(1H)-yl, (3aR,5R,6aS)-Hexahydrocyclopenta[*c*]pyrrol-(1H)-yl, 1,3-Dihydro-2H-isoindolyl, Octahydro-2H-isoindolyl, (3aS)-1,3,3a,4,5,6-Hexahydro-2H-isoindolyl, (3aR,4R,7aS)-1H-Isoindolyl-(3H,3aH,4H,5H,6H,7H,7aH)-yl, (3aR,7aS)-Octahydro-2H-isoindolyl, (3aR,4R,7aS)-Octahydro-2H-isoindolyl, (3aR,4S,7aS)-Octahydro-2H-isoindolyl, 2,5-Diazabicyclo[2.2.1]heptanyl, 2-Azabicyclo[2.2.1]hept-5-enyl, 3-Azabicyclo[3.1.0]hexanyl, (1R,5S,6S)-3-Azabicyclo[3.1.0]hexanyl, (*cis,cis*)-3-Azabicyclo[3.1.0]hexanyl, 3,6-Diazabicyclo[3.1.0]hexanyl, (1S,5R,6R)-3-Azabicyclo[3.2.0]heptanyl, (1S,5R,6S)-3-Azabicyclo[3.2.0]heptanyl, 5-Azaspiro[2.4]heptanyl, 2,6-Diazaspiro[3.3]heptanyl, 2,5-Diazaspiro[3.4]octanyl, 2,6-Diazaspiro[3.4]octanyl, 2,7-Diazaspiro[3.5]nonanyl, 2,7-Diazaspiro[4.4]nonanyl, 2-Azaspiro[4.5]decanyl und 2,8-Diazaspiro[4.5]decanyl besteht, Heterocyclyl-C₁- bis C₈-alkyl, Heterocyclyloxy, Heterocyclyl-C₁- bis C₈-alkoxy, Heterocyclylamino, (Heterocyclyl,C₁- bis C₈-alkyl)amino, (Heterocyclyl)₂-amino, Heterocyclyl-amino-C₁- bis C₈-alkyl, (Heterocyclyl,C₁- bis C₈-alkyl) amino-C₁- bis C₈-alkyl, (Heterocyclyl)₂-amino-C₁- bis C₈-alkyl, (Heterocyclyl, C₃- bis C₁₄-cycloalkyl-C₁- bis C₈-alkyl)amino-C₁- bis C₈-alkyl, Heterocyclyl-C₁- bis C₈-alkyl-amino-C₁- bis C₈-alkyl, (Heterocyclyl-C₁- bis C₈-alkyl, C₁- bis C₈-alkyl) amino-C₁- bis C₈-alkyl, (Heterocyclyl-C₁- bis C₈-alkyl)₂-amino-C₁- bis C₈-alkyl, Heterocyclyl-oxy-amino, (Heterocyclyloxy, C₁- bis C₈-alkyl)amino, (Heterocyclyloxy)₂-amino, (Heterocyclyloxy-C₁- bis C₈-alkyl, C₁- bis C₈-alkyl)-amino, Heterocyclyl-carbonyl oder Heterocyclylcarbonyloxy, wobei jedes Vorkommen von C₃- bis C₁₄-Cycloalkyl, Aryl, Heterocyclyl und Heteroaryl wahlweise mit einem, zwei oder drei Substituenten, die jeweils aus R₁₅ ausgewählt sind, substituiert ist und wobei jedes Vorkommen von Aryl, Heterocyclyl oder Heteroaryl wahlweise mit einem weiteren Substituenten, der aus R₁₆ ausgewählt ist, substituiert ist,
- R₂ Wasserstoff, Halogen, Hydroxyl, C₁- bis C₈-Alkyl, Amino, C₁- bis C₈-Alkylamino oder (C₁- bis C₈-Alkyl)₂-amino,
- R₃ Wasserstoff, Halogen, Hydroxyl, C₁- bis C₈-Alkyl, Amino, C₁- bis C₈-Alkylamino oder (C₁- bis C₈-Alkyl)₂-amino,
- R₄ Wasserstoff, Hydroxyl oder Aryl-C₁- bis C₈-Alkyl, wobei Aryl wahlweise mit einem weiteren Substituenten, der aus C₁- bis C₈-Alkyl, Halogen-C₁- bis C₈-Alkyl, C₁- bis C₈-Alkoxy oder C₁- bis C₈-Alkoxy-C₁- bis C₈-alkyl ausgewählt ist, substituiert ist,
- R₅ Wasserstoff, Halogen, Hydroxyl, C₁- bis C₈-Alkyl, C₁- bis C₈-Alkoxy, Carboxyl, Amino, C₁- bis C₈-Alkylamino, (C₁- bis C₈-Alkyl)₂-amino oder C₁- bis C₈-Alkyl-SO₂-amino,
- R₆ Wasserstoff oder C₁- bis C₈-Alkyl,
- R₇ Wasserstoff, Halogen, Hydroxyl, C₁- bis C₈-Alkyl, Amino, C₁- bis C₈-Alkylamino oder (C₁- bis C₈-Alkyl)₂-amino,
- R₈ Wasserstoff, Halogen, Hydroxyl, C₁- bis C₈-Alkyl, Amino, C₁- bis C₈-Alkylamino oder (C₁- bis C₈-Alkyl)₂-amino,
- R₉ Wasserstoff, Halogen, Hydroxyl, C₁- bis C₈-Alkyl, Amino, C₁- bis C₈-Alkylamino oder (C₁- bis C₈-Alkyl)₂-amino,
- R₁₀ Wasserstoff, Halogen, Hydroxyl, C₁- bis C₈-Alkyl, Amino, C₁- bis C₈-Alkylamino oder (C₁- bis C₈-Alkyl)₂-amino,
- R₁₁ Wasserstoff, Halogen, Hydroxyl, C₁- bis C₈-Alkyl, Amino, C₁- bis C₈-Alkylamino oder (C₁- bis C₈-Alkyl)₂-amino,
- R₁₂ Wasserstoff, C₁- bis C₈-Alkyl oder Heteroaryl, wobei Aryl und Heteroaryl wahlweise mit einem weiteren Substituenten, der aus C₁- bis C₈-Alkyl, Halogen-C₁- bis C₈-Alkyl, C₁- bis C₈-Alkoxy oder C₁- bis C₈-Alkoxy-C₁- bis C₈-alkyl, ausgewählt ist, substituiert sind,
- R₁₃ Wasserstoff, Cyano, Halogen, Hydroxyl oder C₁- bis C₈-Alkyl,
- R₁₄ Wasserstoff, C₁- bis C₈-Alkyl, C₁- bis C₈-Alkylcarbonyl, C₁- bis C₈-Alkoxy-carbonyl, Aryl oder Heteroaryl, wobei Aryl und Heteroaryl wahlweise mit einem weiteren Substituenten, der aus C₁- bis C₈-Alkyl, Halogen-C₁- bis C₈-Alkyl, C₁- bis C₈-Alkoxy oder C₁- bis C₈-Alkoxy-C₁- bis C₈-Alkyl ausgewählt ist, substituiert sind,
- R₁₅ Azido, Halogen, Hydroxyl, Cyano, Nitro, C₁- bis C₈-Alkyl, Halogen-C₁- bis C₈-Alkyl, Hydroxyl-C₁- bis C₈-Alkyl, C₁- bis C₈-Alkoxy-C₁- bis C₈-Alkyl, C₁- bis C₈-Alkoxy, Halogen-C₁- bis C₈-Alkoxy, Hydroxyl-C₁- bis C₈-Alkoxy, Carboxyl, C₁- bis C₈-Alkyl-carbonyl, C₁- bis C₈-Alkoxy-carbonyl, Amino, C₁- bis C₈-Alkylamino, (C₁- bis C₈-Alkyl)₂-amino, Halogen-C₁- bis C₈-Alkylamino, (Halogen-C₁- bis C₈-alkyl)₂-amino, Halogen-C₁- bis C₈-alkyl-amino-C₁- bis C₈-alkyl, (Halogen-C₁- bis C₈-alkyl)₂-amino-C₁- bis C₈-Alkyl, Amino-C₁- bis C₈-alkyl, C₁- bis C₈-Alkylamino-C₁- bis C₈-Alkyl, (C₁- bis C₈-Alkyl)₂-amino-C₁- bis C₈-alkyl, [(C₁- bis C₈-Alkyl)₂-amino-C₁- bis C₈-alkyl, C₁- bis C₈-alkyl] amino-C₁- bis C₈-alkyl-C₁- bis C₈-alkylthio, Aminocarbonyl, C₁- bis C₈-Alkyl-amino-carbonyl, (C₁- bis C₈-Alkyl)₂-amino-carbonyl, C₁- bis C₈-Alkylcarbonyl-amino oder (Carboxyl-C₁- bis C₈-alkyl, C₁- bis C₈-alkyl) amino-carbonyl-amino,
- R₁₆ C₃- bis C₁₄-Cycloalkyl, C₃- bis C₁₄-Cycloalkylamino, Aryl, Aryl-C₁- bis C₈-alkyl, Arylamino, (Aryl, C₁- bis C₈-alkyl) amino, (Aryl)₂-amino, Aryl-C₁- bis C₈-alkyl-amino, (Aryl-C₁- bis C₈-alkyl, C₁- bis C₈-alkyl) amino, (Aryl-C₁- bis C₈-alkyl) 2-amino, Aryl-C₁- bis C₈-alkyl-amino-C₁- bis C₈-alkyl, (Aryl-C₁- bis C₈-alkyl, C₁- bis C₈-alkyl)-amino-C₁- bis C₈-alkyl, (Aryl-C₁- bis C₈-alkyl)₂-amino-C₁- bis C₈-alkyl, Aryl-amino-C₁- bis C₈-alkyl, (Aryl, C₁- bis C₈-alkyl)-amino-C₁- bis C₈-alkyl, (Aryl)₂-amino-C₁- bis C₈-alkyl, Arylaminocarbonyl, Aryl-C₁- bis C₈-alkoxy, Aryl-C₁- bis C₈-alkoxy-carbonyl-amino, Heteroaryl, Heteroaryl-C₁- bis C₈-alkyl, Heteroarylamino, (Heteroaryl)₂-amino, Heteroaryl-C₁- bis C₈-alkylamino, (Heteroaryl-C₁- bis C₈-alkyl, C₁- bis C₈-alkyl) amino, (Heteroaryl-C₁- bis C₈-alkyl)₂-amino, Heteroaryl-C₁- bis C₈-alkylamino-C₁- bis C₈-alkyl, (Heteroaryl-C₁- bis C₈-alkyl, C₁- bis C₈-alkyl)amino-C₁- bis C₈-alkyl, (Heteroaryl-C₁- bis C₈-alkyl)₂-amino-C₁- bis C₈-alkyl, Heterocyclyl, Heterocyclyl-C₁- bis C₈-alkyl, Heterocyclylamino-C₁- bis C₈-alkyl oder Heterocyclyloxy,
wobei jedes Vorkommen von C₃- bis C₁₄-Cycloalkyl wahlweise mit einem Substituenten, der aus R₁₈ ausgewählt ist, substituiert ist und
wobei jedes Vorkommen von Aryl wahlweise mit einem Substituenten, der aus R₁₉ ausgewählt ist, substituiert ist,
- R₁₈ Amino, C₁- bis C₈-Alkyl-amino, (C₁- bis C₈-Alkyl)₂-amino, Amino-C₁- bis C₈-alkyl, C₁- bis C₈-Alkyl-amino-C₁- bis C₈-alkyl, (C₁- bis C₈-Alkyl)₂-amino-C₁- bis C₈-alkyl oder Aryl-C₁- bis C₈-alkylamino und
- R₁₉ Halogen bedeutet.

2. Verbindung nach Anspruch 1, wobei R₁ :
- C₃- bis C₁₄-Cycloalkyl, das, falls vorhanden, bei jedem Vorkommen aus Cyclopropyl, Cyclobutyl, Cyclopentyl, Cyclohexyl oder Cycloheptyl ausgewählt ist,
- Aryl, das, falls vorhanden, bei jedem Vorkommen aus Phenyl ausgewählt ist,
- Heteroaryl, das, falls vorhanden, bei jedem Vorkommen aus Pyrrolyl, Thiazolyl, 1H-1,2,3-Triazolyl, 1H-Tetrazolyl, 2H-Tetrazolyl, Imidazolyl oder Pyridinyl ausgewählt ist, oder
- Heterocyclyl, das, falls vorhanden, bei jedem Vorkommen aus Azetidinyl, Pyrrolidinyl, Tetrahydrofuranyl, Piperidinyl, Piperazinyl, Morpholinyl, 1,4-Diazepanyl, 1,3-Dioxolanyl, 2,5-Dihydro-1H-pyrrolyl, Dihydro-1H-imidazolyl, 1,4,5,6-Tetrahydropyrimidinyl, 1,2,3,6-Tetrahydropyridinyl, Tetrahydro-2H-pyranyl, Indolinyl, 2,3-Dihydrobenzo[d]oxazolyl, 3,4-Dihydro-2H-benzo[*b*][1,4]oxazinyl, 3,4-Dihydroisochinolin-(1H)-yl, 1,2,3,4-Tetrahydroisochinolinyl, 1,2,3,4-Tetrahydrochinoxalinyl, Hexahydropyrrolo[3,4-*b*][1,4]oxazin-(2H)-yl, (4aR,7aS)-Hexahydropyrrolo[3,4-*b*][1,4]oxazin-(4aH)-yl, 3,4-Dihydro-2H-pyrido[3,2-*b*][1,4]oxazinyl, (*cis*)-Octahydrocyclopenta[*c*]pyrrolyl, Hexahydropyrrolo[3,4-*b*]pyrrol-(1H)-yl, (3aR,6aR)-Hexahydropyrrolo[3,4-*b*]pyrrol-(1H)-yl, (3aR,6aS)-Hexahydropyrrolo[3,4-*c*]pyrrol-(1H)-yl, 5H-Pyrrolo[3,4-*b*]pyridin-(7H)-yl, 5,7-Dihydro-6H-pyrrolo[3,4-*b*]pyridinyl, Tetrahydro-1H-pyrrolo[3,4-*b*]pyridin-(2H,7H,7aH)-yl, Hexahydro-1H-pyrrolo[3,4-*b*]pyridin-(2H)-yl, (4aR,7aR)-Hexahydro-1H-pyrrolo[3,4-b]pyridin-(2H)-yl, Octahydro-6H-pyrrolo[3,4-*b*]pyridinyl, 2,3,4,9-Tetrahydro-1H-carbazolyl, 1,2,3,4-Tetrahydropyrazino[1,2-*a*]indol-8-yl, 2,3-Dihydro-1H-pyrrolo[1,2a]indolyl, (3aR,6aR)-Hexahydrocyclopenta[*c*]pyrrol-(1H)-yl, (3aR,4R,6aS)-Hexahydrocyclopenta[*c*]pyrrol-(1H)-yl, (3aR,4S,6aS)-Hexahydrocyclopenta[*c*]pyrrol-(1H)-yl, (3aR,5R,6aS)-Hexahydrocyclopenta[*c*]pyrrol-(1H)-yl, 1,3-Dihydro-2H-isoindolyl, Octahydro-2H-isoindolyl, (3aS)-1,3,3a,4,5,6-Hexahydro-2H-isoindolyl, (3aR,4R,7aS)-1H-Isoindolyl-(3H,3aH,4H,5H,6H,7H,7aH)-yl, (3aR,7aS)-Octahydro-2H-isoindolyl, (3aR,4R,7aS)-Octahydro-2H-isoindolyl, (3aR,4S,7aS)-Octahydro-2H-isoindolyl, 2,5-Diazabicyclo[2.2.1]heptanyl, 2-Azabicyclo[2.2.1]hept-5-enyl, 3-Azabicyclo[3.1.0]hexanyl, (1R,5S,6S)-3-Azabicyclo[3.1.0]hexanyl, (*cis,cis*)-3-Azabicyclo[3.1.0]hexanyl, 3,6-Diazabicyclo[3.1.0]hexanyl, (1S,5R,6R)-3-Azabicyclo[3.2.0]heptanyl, (1S,5R,6S)-3-Azabicyclo[3.2.0]heptanyl, 5-Azaspiro[2.4]heptanyl, 2,6-Diazaspiro[3.3]heptanyl, 2,5-Diazaspiro[3.4]octanyl, 2,6-Diazaspiro[3.4]octanyl, 2,7-Diazaspiro[3.5]nonanyl, 2,7-Diazaspiro[4.4]nonanyl, 2-Azaspiro[4.5]decanyl oder 2,8-Diazaspiro[4.5]decanyl ausgewählt ist, bedeutet.

3. Verbindung nach Anspruch 1, wobei:
- R₂ Wasserstoff, C₁- bis C₈-Alkyl, Amino, C₁- bis C₈-Alkylamino oder (C₁- bis C₈-Alkyl)₂-amino,
- R₃ Wasserstoff, Halogen, Hydroxyl, C₁- bis C₈-Alkyl, Amino, C₁- bis C₈-Alkylamino oder (C₁- bis C₈-Alkyl)₂-amino und
- R₄ Wasserstoff
bedeutet.

4. Verbindung nach Anspruch 1, wobei R₁ :
- C₃- bis C₁₄-Cycloalkylamino-C₁- bis C₈-alkyl, wobei C₃- bis C₁₄-Cycloalkyl aus Cyclopropyl, Cyclobutyl, Cyclopentyl oder Cyclohexyl ausgewählt ist,
- (C₃- bis C₁₄-Cycloalkyl, C₁- bis C₈-alkyl)amino-C₁- bis C₈-alkyl, wobei C₃- bis C₁₄-Cycloalkyl aus Cyclopropyl, Cyclobutyl oder Cyclopentyl ausgewählt ist,
- C₃- bis C₁₄-Cycloalkyl-C₁- bis C₈-alkylamino-C₁- bis C₈-alkyl, wobei C₃- bis C₁₄-Cycloalkyl aus Cyclopropyl, Cyclobutyl, Cyclopentyl, Cyclohexyl oder Cycloheptyl ausgewählt ist,
- Aryl, wobei Aryl aus Phenyl ausgewählt ist,
- Arylamino, wobei Aryl aus Phenyl ausgewählt ist,
- (Aryl, C₁- bis C₈-alkyl)amino, wobei Aryl aus Phenyl ausgewählt ist,
- Arylamino-C₁- bis C₈-alkyl, wobei Aryl aus Phenyl ausgewählt ist,
- Aryl-C₁- bis C₈-alkylamirio-C₁- bis C₈-alkyl, wobei Aryl aus Phenyl ausgewählt ist,
- (Aryl-C₁- bis C₈-alkyl, C₁- bis C₈-alkyl) amino-C₁- bis C₈-alkyl, wobei Aryl aus Phenyl ausgewählt ist,
- (Aryl-C₁- bis C₈-alkyl)₂-amino-C₁- bis C₈-alkyl, wobei Aryl aus Phenyl ausgewählt ist,
- Heteroaryl, wobei das Heteroaryl aus Pyrrolyl, Thiazolyl, 1H-1,2,3-Triazolyl, 1H-Tetrazolyl, 2H-Tetrazolyl, Imidazolyl oder Pyridinyl ausgewählt ist,
- Heteroaryl-C₁- bis C₈-alkylamino-C₁- bis C₈-alkyl, wobei das Heteroaryl aus Pyridin-2-yl, Pyridin-3-yl oder Pyridin-4-yl ausgewählt ist,
- (Heteroaryl-C₁- bis C₈-alkyl, C₁- bis C₈-alkyl)amino-C₁- bis C₈-alkyl, wobei das Heteroaryl aus Pyridin-3-yl oder Pyridin-4-yl ausgewählt ist,
- Heterocyclyl, wobei das Heterocyclyl aus Azetidinyl, Pyrrolidinyl, Tetrahydrofuranyl, Piperidinyl, Piperazinyl, Morpholinyl, 1,4-Diazepanyl, 1,3-Dioxolanyl, 2,5-Dihydro-1H-pyrrolyl, Dihydro-1H-imidazolyl, 1,4,5,6-Tetrahydropyrimidinyl, 1,2,3,6-Tetrahydropyridinyl, Tetrahydro-2H-pyranyl, 3,4-Dihydroisochinolin-(1H)-yl, 1,2,3,4-Tetrahydroisochinolinyl, Hexahydropyrrolo[3,4-b][1,4]oxazin-(2H)-yl, (4aR,7aS)-Hexahydropyrrolo[3,4-*b*][1,4]oxazin-(4aH)-yl, (*cis*)-Octahydrocyclopenta[*c*]pyrrolyl, Hexahydropyrrolo[3,4-*b*]pyrrol-(1H)-yl, (3aR,6aR)-Hexahydropyrrolo[3,4-*b*]pyrrol-(1H)-yl, (3aR,6aS)-Hexahydropyrrolo[3,4-*c*]pyrrol-(1H)-yl, 5H-Pyrrolo[3,4-*b*]pyridin-(7H)-yl, 5,7-Dihydro-6H-pyrrolo[3,4-*b*]pyridinyl, Tetrahydro-1H-pyrrolo[3,4-*b*]pyridin-(2H,7H,7aH)-yl, Hexahydro-1H-pyrrolo[3,4-*b*]pyridin-(2H)-yl, (4aR,7aR)-Hexahydro-1H-pyrrolo[3,4-b]pyridin-(2H)-yl, Octahydro-6H-pyrrolo[3,4-*b*]pyridinyl, (3aR,6aR)-Hexahydrocyclopenta[*c*]pyrrol-(1H)-yl, (3aR,4R,6aS)-Hexahydrocyclopenta[*c*]pyrrol-(1H)-yl, (3aR,4S,6aS)-Hexahydrocyclopenta[*c*]pyrrol-(1H)-yl, (3aR,5R,6aS)-Hexahydrocyclopenta[*c*]pyrrol-2(1H)-yl, 1,3-Dihydro-2H-isoindolyl, Octahydro-2H-isoindolyl, (3aS)-1,3,3a,4,5,6-Hexahydro-2H-isoindolyl, (3aR,4R,7aS)-1H-Isoindolyl-(3H,3aH,4H,5H,6H,7H,7aH)-yl, (3aR,7aS)-Octahydro-2H-isoindolyl, (3aR,4R,7aS)-Octahydro-2H-isoindolyl, (3aR,4S,7aS)-Octahydro-2H-isoindolyl, 2,5-Diazabicyclo[2.2.1]heptanyl, 2-Azabicyclo[2.2.1]hept-5-enyl, 3-Azabicyclo[3.1.0]hexanyl, (1R,5S,6S)-3-Azabicyclo[3.1.0]hexanyl, (*cis,cis*)-3-Azabicyclo[3.1.0]hexanyl, 3,6-Diazabicyclo[3.1.0]hexanyl, (1S,5R,6R)-3-Azabicyclo[3.2.0]heptanyl, (1S,5R,6S)-3-Azabicyclo[3.2.0]heptanyl, 5-Azaspiro[2.4]heptanyl, 2,6-Diazaspiro[3.3]heptanyl, 2,5-Diazaspiro[3.4]octanyl, 2,6-Diazaspiro[3.4]octanyl, 2,7-Diazaspiro[3.5]nonanyl, 2,7-Diazaspiro[4.4]nonanyl, 2-Azaspiro[4.5]decanyl oder 2,8-Diazaspiro[4.5]decanyl ausgewählt ist,
- Heterocyclyl-C₁- bis C₈-alkyl, wobei das Heterocyclyl aus Azetidinyl, Pyrrolidinyl, Tetrahydrofuranyl, Piperidinyl, Piperazinyl, Morpholinyl, 1,4-Diazepanyl, 1,3-Dioxolanyl, 2,5-Dihydro-1H-pyrrolyl, Dihydro-1H-imidazolyl, 1,4,5,6-Tetrahydropyrimidinyl, 1,2,3,6-Tetrahydropyridinyl, Tetrahydro-2H-pyranyl, 3,4-Dihydroisochinolin-(1H)-yl, 1,2,3,4-Tetrahydroisochinolinyl, Hexahydropyrrolo[3,4-*b*][1,4]oxazin-(2H)-yl, (4aR,7aS)-Hexahydropyrrolo[3,4-*b*][1,4]oxazin-(4aH)-yl, (*cis*)-Octahydrocyclopenta[*c*]pyrrolyl, Hexahydropyrrolo[3,4-*b*]pyrrol-(1H)-yl, (3aR,6aR)-Hexahydropyrrolo[3,4-*b*]pyrrol-(1H)-yl, (3aR,6aS)-Hexahydropyrrolo[3,4-*c*]pyrrol-(1H)-yl, 5H-Pyrrolo[3,4-*b*]pyridin-(7H)-yl, 5,7-Dihydro-6H-pyrrolo[3,4-*b*]pyridinyl, Tetrahydro-1H-pyrrolo[3,4-*b*]pyridin-(2H, 7H, 7aH)-yl, Hexahydro-1H-pyrrolo[3,4-*b*]pyridin-(2H)-yl, (4aR,7aR)-Hexahydro-1H-pyrrolo[3,4-b]pyridin-(2H)-yl, Octahydro-6H-pyrrolo[3,4-*b*]pyridinyl, (3aR,6aR)-Hexahydrocyclopenta[*c*]pyrrol-(1H)-yl, (3aR,4R,6aS)-Hexahydrocyclopenta[*c*]pyrrol-(1H)-yl, (3aR,4S,6aS)-Hexahydrocyclopenta[*c*]pyrrol-(1H)-yl, (3aR,5R,6aS)-Hexahydrocyclopenta[*c*]pyrrol-2(1H)-yl, 1,3-Dihydro-2H-isoindolyl, Octahydro-2H-isoindolyl, (3aS)-1,3,3a,4,5,6-Hexahydro-2H-isoindolyl, (3aR,4R,7aS)-1H-Isoindolyl-(3H,3aH,4H,5H,6H,7H,7aH)-yl, (3aR,7aS)-Octahydro-2H-isoindolyl, (3aR,4R,7aS)-Octahydro-2H-isoindolyl, (3aR,4S,7aS)-Octahydro-2H-isoindolyl, 2,5-Diazabicyclo[2.2.1]heptanyl, 2-Azabicyclo[2.2.1]hept-5-enyl, 3-Azabicyclo[3.1.0]hexanyl, (1R,5S,6S)-3-Azabicyclo[3.1.0]hexanyl, (*cis,cis*)-3-Azabicyclo[3.1.0]hexanyl, 3,6-Diazabicyclo[3.1.0]hexanyl, (1S,5R,6R)-3-Azabicyclo[3.2.0]heptanyl, (1S,5R,6S)-3-Azabicyclo[3.2.0]heptanyl, 5-Azaspiro[2.4]heptanyl, 2,6-Diazaspiro[3.3]heptanyl, 2,5-Diazaspiro[3.4]octanyl, 2,6-Diazaspiro[3.4]octanyl, 2,7-Diazaspiro[3.5]nonanyl, 2,7-Diazaspiro[4.4]nonanyl, 2-Azaspiro[4.5]decanyl oder 2,8-Diazaspiro[4.5]decanyl ausgewählt ist,
- Heterocyclylamino-C₁- bis C₈-alkyl, wobei das Heterocyclyl aus Azetidin-1-yl oder Piperidin-4-yl ausgewählt ist,
- (Heterocyclyl,C₁- bis C₈-alkyl) amino-C₁- bis C₈-alkyl, wobei das Heterocyclyl aus Piperidin-3-yl oder Piperidin-4-yl ausgewählt ist,
- (Heterocyclyl, C₃- bis C₁₄-cycloalkyl-C₁- bis C₈-alkyl) amino-C₁- bis C₈-alkyl, wobei das Heterocyclyl aus Piperidin-3-yl oder Piperidin-4-yl ausgewählt ist,
- Heterocyclyl-C₁- bis C₈-alkylamino-C₁- bis C₈-alkyl, wobei das Heterocyclyl aus Pyrrolidin-2-yl, Piperidin-2-yl, Piperidin-3-yl, Piperidin-4-yl oder Tetrahydro-2H-pyran-4-yl ausgewählt ist, und
- (Heterocyclyloxy-C₁- bis C₈-alkyl, C₁- bis C₈-alkyl)amino, das aus Tetrahydro-2H-pyran-2-yl-oxy-C₁- bis C₈-alkyl, C₁- bis C₈-alkyl)amino ausgewählt ist, bedeutet.

5. Verbindung nach Anspruch 1, wobei R₁₆ :
- C₃- bis C₁₄-Cycloalkyl, wobei C₃- bis C₁₄-Cycloalkyl aus Cyclopropyl oder Cyclobutyl ausgewählt ist,
- C₃- bis C₁₄-Cycloalkylamino, wobei C₃- bis C₁₄-Cycloalkyl aus Cyclopropyl ausgewählt ist,
- Aryl, wobei Aryl aus Phenyl ausgewählt ist,
- Aryl-C₁- bis C₈-alkyl, wobei Aryl aus Phenyl ausgewählt ist,
- Arylamino, wobei Aryl aus Phenyl ausgewählt ist,
- (Aryl, C₁- bis C₈-alkyl) amino, wobei Aryl aus Phenyl ausgewählt ist,
- Aryl-C₁- bis C₈-alkylamino, wobei Aryl aus Phenyl ausgewählt ist,
- (Aryl-C₁- bis C₈-alkyl, C₁- bis C₈-alkyl)amino, wobei Aryl aus Phenyl ausgewählt ist,
- (Aryl-C₁- bis C₈-alkyl)₂-amino, wobei Aryl aus Phenyl ausgewählt ist,
- Aryl-C₁- bis C₈-alkylamino-C₁- bis C₈-alkyl, wobei Aryl aus Phenyl ausgewählt ist,
- (Aryl-C₁- bis C₈-alkyl, C₁- bis C₈-alkyl)amino-C₁- bis C₈-alkyl, wobei Aryl aus Phenyl ausgewählt ist,
- (Aryl-C₁- bis C₈-alkyl)₂-amino-C₁- bis C₈-alkyl, wobei Aryl aus Phenyl ausgewählt ist,
- Aryl-C₁- bis C₈-Alkoxy, wobei Aryl aus Phenyl ausgewählt ist,
- Aryl-C₁- bis C₈-Alkoxy-carbonylamino, wobei Aryl aus Phenyl ausgewählt ist,
- Heteroaryl, wobei das Heteroaryl aus Pyridin-2-yl, Pyridin-4-yl, Thiazol-2-yl oder 1H-1,2,3-Triazol-1-yl ausgewählt ist,
- Heteroaryl-C₁- bis C₈-alkylamino-C₁- bis C₈-alkyl, wobei das Heteroaryl aus Pyridin-2-yl, Pyridin-3-yl oder Pyridin-4-yl ausgewählt ist,
- Heterocyclyl, wobei das Heterocyclyl aus Pyrrolidin-1-yl oder Morpholin-4-yl ausgewählt ist,
- Heterocyclyl-C₁- bis C₈-alkyl, wobei das Heterocyclyl aus Pyrrolidin-1-yl ausgewählt ist, und
- Heterocyclyloxy, wobei Heterocyclyl aus Tetrahydro-2H-pyran-2-yl-oxy ausgewählt ist,
bedeutet.

6. Verbindung nach Anspruch 1, wobei die Verbindung mit der Formel (I), Formel (II) oder Formel (III) eine Verbindung mit der Formel (Ia), Formel (IIa) bzw. Formel (IIIa) : 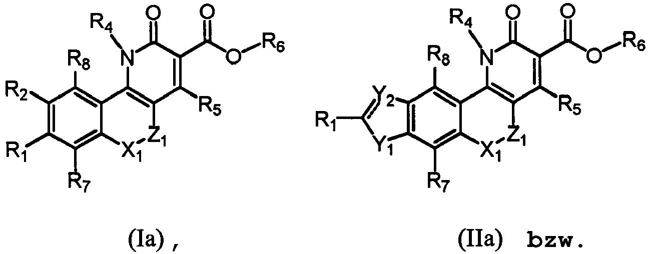 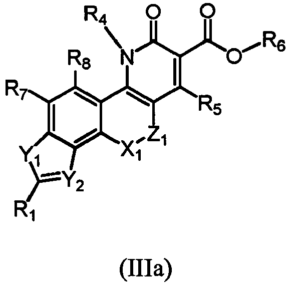 oder eine Form davon ist, worin:
- die Strichellinie in Formel (IIa) und Formel (IIIa) eine wahlweise vorhandene Doppelbindung,
- X₁ eine Bindung, N(R₁₄), S, O oder -CH(R₉)- und
- Z₁ N(R₁₄), S, O, C(O) oder -CH(R₃)- bedeutet, wenn X₁ eine Bindung bzw. -CH(R₉)- bedeutet, oder Z₁ -CH(R₃)- bedeutet, wenn X₁ N(R₁₄), S bzw. O bedeutet, und
- alle anderen Variablen wie weiter oben definiert sind.

7. Verbindung nach Anspruch 1, wobei die Verbindung oder eine Form davon aus:
- 8-(Dimethylamino)-2-oxo-1,2,5,6-tetrahydrobenzo[h]chinolin-3-carbonsäure,
- 9-(Dimethylamino)-2-oxo-2,5,6,7-tetrahydro-1H-benzo[6,7]cyclohepta[1,2-b]pyridin-3-carbonsäure,
- 9-(Dimethylamino)-4-hydroxy-2-oxo-2,5,6,7-tetrahydro-1H-benzo[6,7]cyclohepta[1,2-b]pyridin-3-carbonsäure,
- 10-(Dimethylamino)-2-oxo-1,2,5,6,7,8-hexahydrobenzo[7,8]cycloocta[1,2-b]pyridin-3-carbonsäure,
- 10-(Dimethylamino)-4-hydroxy-2-oxo-1,2,5,6,7,8-hexahydrobenzo[7,8]cycloocta[1,2-b]pyridin-3-carbonsäure,
- 4-Hydroxy-2-oxo-9-(pyrrolidin-1-yl)-2,5,6,7-tetrahydro-1H-benzo[6,7]cyclohepta[1,2-b]pyridin-3-carbonsäure,
- 5-Methyl-2-oxo-8-(pyrrolidin-1-yl)-2,5-dihydro-1H-chromeno[4,3-b]pyridin-3-carbonsäure,
- 9-(Dimethylamino)-4-hydroxy-2-oxo-1,2,5,6-tetrahydrobenzo[2,3]oxepino[4,5-b]pyridin-3-carbonsäure,
- 4-Hydroxy-2-oxo-9-(pyrrolidin-1-yl)-1,2,5,6-tetrahydrobenzo[2,3]oxepino[4,5-b]pyridin-3-carbonsäure,
- 2-Oxo-9-(pyrrolidin-1-yl)-1,2,5,6-tetrahydrobenzo[2,3]oxepino[4,5-b]pyridin-3-carbonsäure,
- 4-Hydroxy-5-methyl-2-oxo-9-(pyrrolidin-1-yl)-1,2,5,6-tetrahydrobenzo[2,3]oxepino[4,5-b]pyridin-3-carbonsäure,
- 4-Hydroxy-7-methyl-2-oxo-9-(pyrrolidin-1-yl)-2,5,6,7-tetrahydro-1H-benzo[b]pyrido[2,3-d]azepin-3-carbonsäure,
- 4-Hydroxy-2-oxo-9-(pyrrolidin-1-yl)-2,5,6,7-tetrahydro-1H-benzo[b]pyrido[2,3-d]azepin-3-carbonsäure,
- 4-Hydroxy-5-methyl-2-oxo-8-(pyrrolidin-1-yl)-1,2,5,6-tetrahydrobenzo[h]chinolin-3-carbonsäure,
- 8-(Dimethylamino)-4-hydroxy-5-methyl-2-oxo-1,2,5,6-tetrahydrobenzo[h]chinolin-3-carbonsäure,
- 4-Hydroxy-2-oxo-10-(pyrrolidin-1-yl)-1,2,5,6,7,8-hexahydrobenzo[7,8]cycloocta[1,2-b]pyridin-3-carbonsäure,
- 4-Hydroxy-2-oxo-10-(propylamino)-1,2,5,6,7,8-hexahydrobenzo[7,8]cycloocta[1,2-b]pyridin-3-carbonsäure,
- 10-(Ethylamino)-4-hydroxy-2-oxo-1,2,5,6,7,8-hexahydrobenzo[7,8]cycloocta[1,2-b]pyridin-3-carbonsäure,
- 9-(Dimethylamino)-4-hydroxy-2-oxo-1,2,5,6-tetrahydrobenzo[2,3]thiepino[4,5-b]pyridin-3-carbonsäure und
- 4-Hydroxy-2-oxo-9-(pyrrolidin-1-yl)-1,2,5,6-tetrahydrobenzo[2,3]thiepino[4,5-b]pyridin-3-carbonsäure ausgewählt ist,
wobei die Form der Verbindung aus einer isotopologischen, racemischen, enantiomeren, diastereomeren, stereoisomeren, polymorphen, tautomeren, Freien-Säure-, Freien-Basen-, Salz-, Hydrat-, Solvat- oder Clathratform davon ausgewählt ist.

8. Verbindung nach Anspruch 1, wobei die Verbindung oder eine Form davon aus:
- 9-(3-(Dimethylamino)pyrrolidin-1-yl)-4-hydroxy-2-oxo-2,5,6,7-tetrahydro-1H-benzo[6,7]cyclohepta[1,2-b]pyridin-3-carbonsäurehydrochlorid,
- 4-Hydroxy-9-(3-(methylamino)pyrrolidin-1-yl)-2-oxo-2,5,6,7-tetrahydro-1H-benzo[6,7]cyclohepta[1,2-b]pyridin-3-carbonsäurehydrochlorid,
- 9-((*cis,cis*)-6-Amino-3-azabicyclo[3.1.0]hexan-3-yl)-4-hydroxy-2-oxo-2,5,6,7-tetrahydro-1H-benzo[6,7]cyclohepta[1,2-b]pyridin-3-carbonsäuretrifluoracetat,
- 9-((*cis*,*cis*)-6-(Benzyl(methyl)amino)-3-azabicyclo[3.1.0]hexan-3-yl)-4-hydroxy-2-oxo-2,5,6,7-tetrahydro-1H-benzo[6,7]cyclohepta[1,2-b]pyridin-3-carbonsäurehydrochlorid,
- 4-Hydroxy-9-((*cis*,*cis*)-6-(methylamino)-3-azabicyclo[3.1.0]hexan-3-yl)-2-oxo-2,5,6,7-tetrahydro-1H-benzo[6,7]cyclohepta[1,2-b]pyridin-3-carbonsäurehydrochlorid,
- 9-(3-(Dimethylamino)pyrrolidin-1-yl)-4-hydroxy-5-methyl-2-oxo-2,5,6,7-tetrahydro-1H-benzo[6,7]cyclohepta[1,2-b]pyridin-3-carbonsäurehydrochlorid,
- 9-((*cis*,*cis*)-6-Amino-3-azabicyclo[3.1.0]hexan-3-yl)-4-hydroxy-5-methyl-2-oxo-2,5,6,7-tetrahydro-1H-benzo[6,7]cyclohepta[1,2-b]pyridin-3-carbonsäurehydrochlorid,
- 9-((*cis,cis*)-6-Amino-3-azabicyclo[3.1.0]hexan-3-yl)-4,7-dihydroxy-2-oxo-2,5,6,7-tetrahydro-1H-benzo[6,7]cyclohepta[1,2-b]pyridin-3-carbonsäuretrifluoracetat,
- 9-(3-(Dimethylamino)pyrrolidin-1-yl)-4,7-dihydroxy-2-oxo-2,5,6,7-tetrahydro-1H-benzo[6,7]cyclohepta[1,2-b]pyridin-3-carbonsäurehydrochlorid,
- 2-((Ethylamino)methyl)-8-hydroxy-l-methyl-6-oxo-1,5,6,9-tetrahydropyrido[3',2':4,5]cyclopenta[1,2-f]indol-7-carbonsäurehydrochlorid,
- 4-Hydroxy-8-methyl-9-((methylamino)methyl)-2-oxo-2,5,6,8-tetrahydro-1H-indolo[6,5-h]chinolin-3-carbonsäurehydrochlorid,
- 9-(Azetidin-1-ylmethyl)-4-hydroxy-8-methyl-2-oxo-2,5,6,8-tetrahydro-1H-indolo[6,5-h]chinolin-3-carbonsäurehydrochlorid,
- 4-Hydroxy-9-methyl-10-((methylamino)methyl)-2-oxo-1,2,5,6,7,9-hexahydropyrido[3',2':6,7]cyclohepta[1,2-f]indol-3-carbonsäurehydrochlorid,
- 10-((Cyclobutylamino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 10-(Azetidin-1-ylmethyl)-4-hydroxy-9-methyl-2-oxo-1,2,5,6,7,9-hexahydropyrido[3',2':6,7]cyclohepta[1,2-f]indol-3-carbonsäurehydrochlorid,
- 10-((Ethylamino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 4-Hydroxy-10-((isopropylamino)methyl)-9-methyl-2-oxo-1,2,5,6,7,9-hexahydropyrido[3',2':6,7]cyclohepta[1,2-f]indol-3-carbonsäurehydrochlorid,
- 10-((*tert*.-Butylamino)methyl)-4-hydroxy-9-methyl-2-oxo-1,2,5,6,7,9-hexahydropyrido[3',2':6,7]cyclohepta-[1,2-f]indol-3-carbonsäurehydrochlorid,
- 4-Hydroxy-10-((4-hydroxypiperidin-1-yl)methyl)-9-methyl-2-oxo-1,2,5,6,7,9-hexahydropyrido[3',2':6,7]cyclohepta[1,2-f]indol-3-carbonsäurehydrochlorid,
- 10-((4-Aminopiperidin-1-yl)methyl)-4-hydroxy-9-methyl-2-oxo-1,2,5,6,7,9-hexahydropyrido[3',2':6,7]cyclohepta[1,2-f]indol-3-carbonsäuredihydrochlorid,
- 10-((4-(Dimethylamino)piperidin-1-yl)methyl)-4-hydroxy-9-methyl-2-oxo-1,2,5,6,7,9-hexahydropyrido[3',2':6,7]cyclohepta[1,2-f]indol-3-carbonsäuredihydrochlorid,
- 10-((3-Aminopiperidin-1-yl)methyl)-4-hydroxy-9-methyl-2-oxo-1,2,5,6,7,9-hexahydropyrido[3',2':6,7]cyclohepta[1,2-f]indol-3-carbonsäuredihydrochlorid,
- 10-(((Cyclopropylmethyl)amino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 4-Hydroxy-9-methyl-10-(((1-methylcyclopropyl)amino)methyl)-2-oxo-1,2,5,6,7,9-hexahydropyrido[3',2':6,7]cyclohepta[1,2-f]indol-3-carbonsäurehydrochlorid,
- 10-((Benzylamino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 10-(Aminomethyl)-4-hydroxy-9-methyl-2-oxo-1,2,5,6,7,9-hexahydropyrido[3',2':6,7]cyclohepta[1,2-f]indol-3-carbonsäurehydrochlorid,
- 10-((Cyclopropylamino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 10-(((Cyclobutylmethyl)amino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 4-Hydroxy-9-methyl-10-((((2-methylcyclopropyl)methyl)amino)methyl)-2-oxo-1,2,5,6,7,9-hexahydropyrido[3',2':6,7]cyclohepta[1,2-f]indol-3-carbonsäurehydrochlorid,
- 4-Hydroxy-9-methyl-2-oxo-10-(((pyridin-4-ylmethyl)amino)methyl)-1,2,5,6,7,9-hexahydropyrido[3',2':6,7]cyclohepta[1,2-f]indol-3-carbonsäurehydrochlorid,
- 4-Hydroxy-9-methyl-2-oxo-10-(piperidin-1-ylmethyl)-1,2,5,6,7,9-hexahydropyrido[3',2':6,7]cyclohepta[1,2-f]indol-3-carbonsäurehydrochlorid,
- 4-Hydroxy-9-methyl-10-(morpholinomethyl)-2-oxo-1,2,5,6,7,9-hexahydropyrido[3',2':6,7]cyclohepta[1,2-f]indol-3-carbonsäurehydrochlorid,
- 4-Hydroxy-9-methyl-10-((4-methylpiperazin-1-yl)methyl)-2-oxo-1,2,5,6,7,9-hexahydropyrido[3',2':6,7]cyclohepta[1,2-f]indol-3-carbonsäuredihydrochlorid,
- 10-((Diethylamino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 4-Hydroxy-9-methyl-2-oxo-10-((propylamino)methyl)-1,2,5,6,7,9-hexahydropyrido[3',2':6,7]cyclohepta[1,2-f]indol-3-carbonsäurehydrochlorid,
- 4-Hydroxy-9-methyl-2-oxo-10-((prop-2-in-1-ylamino)methyl)-1,2,5,6,7,9-hexahydropyrido[3',2':6,7]cyclohepta[1,2-f]indol-3-carbonsäurehydrochlorid,
- (R)-10-((3-Fluorpyrrolidin-1-yl)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 10-((3-(Dimethylamino)pyrrolidin-1-yl)methyl)-4-hydroxy-9-methyl-2-oxo-1,2,5,6,7,9-hexahydropyrido[3',2':6,7]cyclohepta[1,2-f]indol-3-carbonsäuredihydrochlorid,
- 10-((Dimethylamino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- (S)-10-((3-Aminopyrrolidin-1-yl)methyl)-4-hydroxy-9-methyl-2-oxo-1,2,5,6,7,9-hexahydropyrido[3',2':6,7]cyclohepta[1,2-f]indol-3-carbonsäuredihydrochlorid,
- 4-Hydroxy-9-methyl-2-oxo-10-(pyrrolidin-1-ylmethyl)-1,2,5,6,7,9-hexahydropyrido[3',2':6,7]cyclohepta[1,2-f]indol-3-carbonsäurehydrochlorid,
- 4-Hydroxy-10-((3-hydroxypyrrolidin-1-yl)methyl)-9-methyl-2-oxo-1,2,5,6,7,9-hexahydropyrido[3',2':6,7]cyclohepta[1,2-f]indol-3-carbonsäurehydrochlorid,
- 4-Hydroxy-9-methyl-2-oxo-10-(((pyridin-3-ylmethyl)amino)methyl)-1,2,5,6,7,9-hexahydropyrido[3',2':6,7]cyclohepta[1,2-f]indol-3-carbonsäurehydrochlorid,
- 9-Methyl-10-((methylamino)methyl)-2-oxo-1,2,5,6,7,9-hexahydropyrido[3',2':6,7]cyclohepta[1,2-f]indol-3-carbonsäurehydrochlorid,
- 10-((Ethylamino)methyl)-9-methyl-2-oxo-1,2,5,6,7,9-hexahydropyrido[3',2':6,7]cyclohepta[1,2-f]indol-3-carbonsäurehydrochlorid,
- 10-((Isopropylamino)methyl)-9-methyl-2-oxo-1,2,5,6,7,9-hexahydropyrido[3',2':6,7]cyclohepta[1,2-f]indol-3-carbonsäurehydrochlorid,
- 10-((*tert*.-Butylamino)methyl)-9-methyl-2-oxo-1,2,5,6,7,9-hexahydropyrido[3',2':6,7]cyclohepta[1,2-f]indol-3-carbonsäurehydrochlorid,
- 10-(Azetidin-1-ylmethyl)-9-methyl-2-oxo-1,2,5,6,7,9-hexahydropyrido[3',2':6,7]cyclohepta[1,2-f]indol-3-carbonsäurehydrochlorid,
- 4-Hydroxy-10-methyl-11-((methylamino)methyl)-2-oxo-2,5,6,7,8,10-hexahydro-1H-pyrido[3',2':7,8]cycloocta[1,2-f]indol-3-carbonsäurehydrochlorid,
- 11-((Ethylamino)methyl)-4-hydroxy-10-methyl-2-oxo-2,5,6,7,8,10-hexahydro-1H-pyrido[3',2':7,8]cycloocta[1,2-f]indol-3-carbonsäurehydrochlorid,
- 7-Hydroxy-l-methyl-2-((methylamino)methyl)-9-oxo-1,4,5,6,9,10-hexahydropyrido[2',3':3,4]cyclohepta[1,2-e]indol-8-carbonsäurehydrochlorid,
- 2-((Ethylamino)methyl)-7-hydroxy-l-methyl-9-oxo-1,4,5,6,9,10-hexahydropyrido[2',3':3,4]cyclohepta[1,2-e]indol-8-carbonsäurehydrochlorid,
- 4-Hydroxy-7,9-dimethyl-10-((methylamino)methyl)-2-oxo-1,2,5,6,7,9-hexahydropyrido[3',2':6,7]cyclohepta[1,2-f]indol-3-carbonsäurehydrochlorid,
- 10-((Ethylamino)methyl)-4-hydroxy-7,9-dimethyl-2-oxo-1,2,5,6,7,9-hexahydropyrido[3',2':6,7]cyclohepta[1,2-f]indol-3-carbonsäurehydrochlorid,
- 4-Hydroxy-10-((isopropylamino)methyl)-7,9-dimethyl-2-oxo-1,2,5,6,7,9-hexahydropyrido[3',2':6,7]cyclohepta[1,2-f]indol-3-carbonsäurehydrochlorid,
- 10-((*tert*.-Butylamino)methyl)-4-hydroxy-7,9dimethyl-2-oxo-1,2,5,6,7,9-hexahydropyrido[3',2':6,7]cyclohepta[1,2-f]indol-3-carbonsäurehydrochlorid,
- 4-Hydroxy-7,9-dimethyl-10-(((1-methylcyclopropyl)amino)methyl)-2-oxo-1,2,5,6,7,9-hexahydropyrido[3',2':6,7]cyclohepta[1,2-f]indol-3-carbonsäurehydrochlorid,
- 10-((Ethylamino)methyl)-4-hydroxy-5,9-dimethyl-2-oxo-1,2,5,6,7,9-hexahydropyrido[3',2':6,7]cyclohepta[1,2-f]indol-3-carbonsäurehydrochlorid,
- 4-Hydroxy-9-methyl-10-((methylamino)methyl)-2-oxo-1,2,5,9-tetrahydropyrido[3',2':6,7]cyclohepta[1,2-f]indol-3-carbonsäurehydrochlorid,
- 10-((Ethylamino)methyl)-4-hydroxy-9-methyl-2-oxo-1,2,5,9-tetrahydropyrido[3',2':6,7]cyclohepta[1,2-f]indol-3-carbonsäurehydrochlorid,
- 10-((Ethylamino)methyl)-4-hydroxy-7,9-dimethyl-2-oxo-1,2,5,9-tetrahydropyrido[3',2':6,7]cyclohepta[1,2-f]indol-3-carbonsäurehydrochlorid,
- (*cis*)-10-((Ethylamino)methyl)-4,6,7-trihydroxy-9-methyl-2-oxo-1,2,5,6,7,9-hexahydropyrido[3',2':6,7]cyclohepta[1,2-f]indol-3-carbonsäurehydrochlorid,
- 8-(3-(Dimethylamino)pyrrolidin-1-yl)-5-methyl-2-oxo-2,5-dihydro-1H-chromeno[4,3-b]pyridin-3-carbonsäurehydrochlorid,
- 9-(3-(Dimethylamino)pyrrolidin-1-yl)-4-hydroxy-2-oxo-1,2,5,6-tetrahydrobenzo[2,3]oxepino[4,5-b]pyridin-3-carbonsäurehydrochlorid,
- 9-((*cis,cis*)-6-(Dibenzylamino)-3-azabicyclo[3.1.0]hexan-3-yl)-4-hydroxy-2-oxo-1,2,5,6-tetrahydrobenzo[2,3]oxepino[4,5-b]pyridin-3-carbonsäuretrifluoracetat,
- 9-((*cis,cis*)-6-Amino-3-azabicyclo[3.1.0]hexan-3-yl)-4-hydroxy-2-oxo-1,2,5,6-tetrahydrobenzo[2,3]oxepino[4,5-b]pyridin-3-carbonsäurehydrochlorid,
- 9-(3-(Dimethylamino)pyrrolidin-1-yl)-2-oxo-1,2,5,6-tetrahydrobenzo[2,3]oxepino[4,5-b]pyridin-3-carbonsäurehydrochlorid,
- 9-(3-(Dimethylamino)pyrrolidin-1-yl)-4-hydroxy-5-methyl-2-oxo-1,2,5,6-tetrahydrobenzo[2,3]oxepino[4,5-b]pyridin-3-carbonsäurehydrochlorid,
- 9-((*cis,cis*)-6-(Dibenzylamino)-3-azabicyclo[3.1.0]hexan-3-yl)-4-hydroxy-5-methyl-2-oxo-1,2,5,6-tetrahydrobenzo[2,3]oxepino[4,5-b]pyridin-3-carbonsäurehydrochlorid,
- 9-((*cis,cis*)-6-Amino-3-azabicyclo[3.1.0]hexan-3-yl)-4-hydroxy-5-methyl-2-oxo-1,2,5,6-tetrahydrobenzo[2,3]oxepino[4,5-b]pyridin-3-carbonsäurehydrochlorid,
- 4-Hydroxy-9-methyl-10-((methylamino)methyl)-2-oxo-2,5,6,9-tetrahydro-1H-pyrido[2',3':4,5]oxepino[3,2-f]indol-3-carbonsäurehydrochlorid,
- 10-((Ethylamino)methyl)-4-hydroxy-9-methyl-2-oxo-2,5,6,9-tetrahydro-1H-pyrido[2',3':4,5]oxepino[3,2-f]indol-3-carbonsäurehydrochlorid,
- 4-Hydroxy-10-((isopropylamino)methyl)-9-methyl-2-oxo-2,5,6,9-tetrahydro-1H-pyrido[2',3':4,5]oxepino[3,2-f]indol-3-carbonsäurehydrochlorid,
- 10-(Azetidin-1-ylmethyl)-4-hydroxy-9-methyl-2-oxo-2,5,6,9-tetrahydro-1H-pyrido[2',3':4,5]oxepino[3,2-f]indol-3-carbonsäurehydrochlorid,
- 10-((Ethylamino)methyl)-4-hydroxy-5,9-dimethyl-2-oxo-2,5,6,9-tetrahydro-1H-pyrido[2',3':4,5]oxepino[3,2-f]indol-3-carbonsäurehydrochlorid,
- 9-(3-(Dimethylamino)pyrrolidin-1-yl)-4-hydroxy-2-oxo-2,5,6,7-tetrahydro-1H-benzo[b]pyrido[2,3-d]azepin-3-carbonsäurehydrochlorid,
- 4-Hydroxy-9-(4-methylpiperazin-1-yl)-2-oxo-2,5,6,7-tetrahydro-1H-benzo[b]pyrido[2,3-d]azepin-3-carbonsäurehydrochlorid,
- 9-((*cis,cis*)-6-Amino-3-azabicyclo[3.1.0]hexan-3-yl)-4-hydroxy-2-oxo-2,5,6,7-tetrahydro-1H-benzo[b]pyrido[2,3-d]azepin-3-carbonsäurehydrochlorid,
- 9-(Hexahydro-1H-pyrrolo[3,4-b]pyridin-6(2H)-yl)-4-hydroxy-2-oxo-2,5,6,7-tetrahydro-1H-benzo[b]pyrido[2,3-d]azepin-3-carbonsäurehydrochlorid,
- 4-Hydroxy-9-methyl-10-((methylamino)methyl)-2-oxo-1,2,5,6,7,9-hexahydropyrido[2',3':4,5]azepino[3,2-f]indol-3-carbonsäurehydrochlorid,
- 10-((Ethylamino)methyl)-4-hydroxy-9-methyl-2-oxo-1,2,5,6,7,9-hexahydropyrido[2',3':4,5]azepino[3,2-f]indol-3-carbonsäurehydrochlorid,
- 4-Hydroxy-10-((isopropylamino)methyl)-9-methyl-2-oxo-1,2,5,6,7,9-hexahydropyrido[2',3':4,5]azepino[3,2-f]indol-3-carbonsäurehydrochlorid,
- 10-((tert.-Butylamino)methyl)-4-hydroxy-9-methyl-2-oxo-1,2,5,6,7,9-hexahydropyrido[2',3':4,5]azepino[3,2-f]indol-3-carbonsäurehydrochlorid,
- 4-Hydroxy-10-methyl-11-((methylamino)methyl)-2-oxo-2,5,6,7,8,10-hexahydro-1H-pyrido[2',3':4,5]azocino[3,2-f]indol-3-carbonsäurehydrochlorid,
- 11-((Ethylamino)methyl)-4-hydroxy-10-methyl-2-oxo-2,5,6,7,8,10-hexahydro-1H-pyrido[2',3':4,5]azocino[3,2-f]indol-3-carbonsäurehydrochlorid,
- 4-Hydroxy-11-((isopropylamino)methyl)-10-methyl-2-oxo-2,5,6,7,8,10-hexahydro-1H-pyrido[2',3':4,5]azocino[3,2-f]indol-3-carbonsäurehydrochlorid,
- 8-(3-(Dimethylamino)pyrrolidin-1-yl)-4-hydroxy-5-methyl-2-oxo-1,2,5,6-tetrahydrobenzo[h]chinolin-3-carbonsäurehydrochlorid,
- 8-((*cis,cis*)-6-Amino-3-azabicyclo[3.1.0]hexan-3-yl)-4-hydroxy-5-methyl-2-oxo-1,2,5,6-tetrahydrobenzo[h]chinolin-3-carbonsäuretrifluoracetat,
- 9-(1,4-Diazepan-1-yl)-4-hydroxy-2-oxo-2,5,6,7-tetrahydro-1H-benzo[6,7]cyclohepta[1,2-b]pyridin-3-carbonsäurehydrochlorid,
- 4-Hydroxy-9-(4-methyl-1,4-diazepan-1-yl)-2-oxo-2,5,6,7-tetrahydro-1H-benzo[6,7]cyclohepta[1,2-b]pyridin-3-carbonsäurehydrochlorid,
- 9-((2-(Dimethylamino)ethyl)amino)-4-hydroxy-2-oxo-2,5,6,7-tetrahydro-1H-benzo[6,7]cyclohepta[1,2-b]pyridin-3-carbonsäurehydrochlorid,
- 9-((4aR,7aR)-Hexahydro-1H-pyrrolo[3,4-b]pyridin-6(2H)-yl)-4-hydroxy-2-oxo-2,5,6,7-tetrahydro-1H-benzo[6,7]cyclohepta[1,2-b]pyridin-3-carbonsäurehydrochlorid,
- 4-Hydroxy-9-((4aR,7aR)-1-methylhexahydro-1H-pyrrolo[3,4-b]pyridin-6(2H)-yl)-2-oxo-2,5,6,7-tetrahydro-1H-benzo[6,7]cyclohepta[1,2-b]pyridin-3-carbonsäurehydrochlorid,
- 9-(4-(Dimethylamino)piperidin-1-yl)-4-hydroxy-2-oxo-2,5,6,7-tetrahydro-1H-benzo[6,7]cyclohepta[1,2-b]pyridin-3-carbonsäurehydrochlorid,
- 9-(3-(Dimethylamino)piperidin-1-yl)-4-hydroxy-2-oxo-2,5,6,7-tetrahydro-1H-benzo[6,7]cyclohepta[1,2-b]pyridin-3-carbonsäurehydrochlorid,
- 4-Hydroxy-2-oxo-9-(piperidin-4-ylamino)-2,5,6,7-tetrahydro-1H-benzo[6,7]cyclohepta[1,2-b]pyridin-3-carbonsäurehydrochlorid,
- 4-Hydroxy-2-oxo-9-(piperazin-1-yl)-2,5,6,7-tetrahydro-1H-benzo[6,7]cyclohepta[1,2-b]pyridin-3-carbonsäurehydrochlorid,
- 4-Hydroxy-9-(4-methylpiperazin-1-yl)-2-oxo-2,5,6,7-tetrahydro-1H-benzo[6,7]cyclohepta[1,2-b]pyridin-3-carbonsäurehydrochlorid,
- 4-Hydroxy-2-oxo-9-(2,6-diazaspiro[3.4]octan-6-yl)-2,5,6,7-tetrahydro-1H-benzo[6,7]cyclohepta[1,2-b]pyridin-3-carbonsäurehydrochlorid,
- 4-Hydroxy-2-oxo-9-(2,7-diazaspiro[4.4]nonan-2-yl)-2,5,6,7-tetrahydro-1H-benzo[6,7]cyclohepta[1,2-b]pyridin-3-carbonsäurehydrochlorid,
- 4-Hydroxy-9-(7-methyl-2,7-diazaspiro[4.4]nonan-2-yl)-2-oxo-2,5,6,7-tetrahydro-1H-benzo[6,7]cyclohepta[1,2-b]pyridin-3-carbonsäurehydrochlorid,
- 9-((*cis,cis*)-6-Amino-3-azabicyclo[3.1.0]hexan-3-yl)-10,11-difluor-4-hydroxy-2-oxo-2,5,6,7-tetrahydro-1H-benzo[6,7]cyclohepta[1,2-b]pyridin-3-carbonsäurehydrochlorid,
- 9-((*cis,cis*)-6-Amino-3-azabicyclo[3.1.0]hexan-3-yl)-11-fluor-4-hydroxy-2-oxo-2,5,6,7-tetrahydro-1H-benzo[6,7]cyclohepta[1,2-b]pyridin-3-carbonsäurehydrochlorid,
- 10-((*cis,cis*)-6-Amino-3-azabicyclo[3.1.0]hexan-3-yl)-4-hydroxy-2-oxo-1,2,5,6,7,8-hexahydrobenzo[7,8]cycloocta[1,2-b]pyridin-3-carbonsäurehydrochlorid,
- 10-((Butylamino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 4-Hydroxy-9-methyl-2-oxo-10-((pentylamino)methyl)-1,2,5,6,7,9-hexahydropyrido[3',2':6,7]cyclohepta[1,2-f]indol-3-carbonsäurehydrochlorid,
- 10-((Hexylamino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 10-((Heptylamino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 4-Hydroxy-9-methyl-10-((octylamino)methyl)-2-oxo-1,2,5,6,7,9-hexahydropyrido[3',2':6,7]cyclohepta[1,2-f]indol-3-carbonsäurehydrochlorid,
- 4-Hydroxy-9-methyl-10-((nonylamino)methyl)-2-oxo-1,2,5,6,7,9-hexahydropyrido[3',2':6,7]cyclohepta[1,2-f]indol-3-carbonsäurehydrochlorid,
- 10-(((2-(Dimethylamino)ethyl)amino)methyl)-4-hydroxy-9-methyl-2-oxo-1,2,5,6,7,9-hexahydropyrido[3',2':6,7]cyclohepta[1,2-f]indol-3-carbonsäuredihydrochlorid,
- 4-Hydroxy-9-methyl-10-(((2-(methylamino)ethyl)amino)methyl)-2-oxo-1,2,5,6,7,9-hexahydropyrido[3',2':6,7]cyclohepta[1,2-f]indol-3-carbonsäuredihydrochlorid,
- 10-(((2-Aminoethyl)amino)methyl)-4-hydroxy-9-methyl-2-oxo-1,2,5,6,7,9-hexahydropyrido[3',2':6,7]cyclohepta[1,2-f]indol-3-carbonsäuredihydrochlorid,
- 10-(((2-(Dimethylamino)ethyl)(methyl)amino)methyl)-4-hydroxy-9-methyl-2-oxo-1,2,5,6,7,9-hexahydropyrido[3',2':6,7]cyclohepta[1,2-f]indol-3-carbonsäuredihydrochlorid,
- 10-((sek.-Butylamino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 4-Hydroxy-10-(((2-hydroxyethyl)amino)methyl)-9-methyl-2-oxo-1,2,5,6,7,9-hexahydropyrido[3',2':6,7]cyclohepta[1,2-f]indol-3-carbonsäurehydrochlorid,
- 4-Hydroxy-10-(((2-methoxyethyl)amino)methyl)-9-methyl-2-oxo-1,2,5,6,7,9-hexahydropyrido[3',2':6,7]cyclohepta[1,2-f]indol-3-carbonsäurehydrochlorid,
- 4-Hydroxy-10-(((2-hydroxyethyl)(methyl)amino)methyl)-9-methyl-2-oxo-1,2,5,6,7,9-hexahydropyrido[3',2':6,7]cyclohepta[1,2-f]indol-3-carbonsäurehydrochlorid,
- 4-Hydroxy-10-(((1-methoxypropan-2-yl)amino)methyl)-9-methyl-2-oxo-1,2,5,6,7,9-hexahydropyrido[3',2':6,7]cyclohepta[1,2-f]indol-3-carbonsäurehydrochlorid,
- 4-Hydroxy-10-(((1-hydroxypropan-2-yl)amino)methyl)-9-methyl-2-oxo-1,2,5,6,7,9-hexahydropyrido[3',2':6,7]cyclohepta[1,2-f]indol-3-carbonsäurehydrochlorid,
- 4-Hydroxy-9-methyl-2-oxo-10-(((2-(pyrrolidin-1-yl)ethyl)amino)methyl)-1,2,5,6,7,9-hexahydropyrido[3',2':6,7]cyclohepta[1,2-f]indol-3-carbonsäuredihydrochlorid,
- 10-((Allylamino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 4-Hydroxy-10-((isobutylamino)methyl)-9-methyl-2-oxo-1,2,5,6,7,9-hexahydropyrido[3',2':6,7]cyclohepta[1,2-f]indol-3-carbonsäurehydrochlorid,
- 4-Hydroxy-9-methyl-10-((neopentylamino)methyl)-2-oxo-1,2,5,6,7,9-hexahydropyrido[3',2':6,7]cyclohepta[1,2-f]indol-3-carbonsäurehydrochlorid,
- 4-Hydroxy-9-methyl-10-((4-methyl-1,4-diazepan-1-yl)methyl)-2-oxo-1,2,5,6,7,9-hexahydropyrido[3',2':6,7]cyclohepta[1,2-f]indol-3-carbonsäuredihydrochlorid,
- 4-Hydroxy-9-methyl-10-(((1-methylpiperidin-4-yl)amino)methyl)-2-oxo-1,2,5,6,7,9-hexahydropyrido[3',2':6,7]cyclohepta[1,2-f]indol-3-carbonsäuredihydrochlorid,
- 4-Hydroxy-10-((3-methoxypyrrolidin-1-yl)methyl)-9-methyl-2-oxo-1,2,5,6,7,9-hexahydropyrido[3',2':6,7]cyclohepta[1,2-f]indol-3-carbonsäurehydrochlorid,
- 10-((3-Acetamidopyrrolidin-1-yl)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 10-(((1-(Dimethylamino)propan-2-yl)amino)methyl)-4-hydroxy-9-methyl-2-oxo-1,2,5,6,7,9-hexahydropyrido[3',2':6,7]cyclohepta[1,2-f]indol-3-carbonsäuredihydrochlorid,
- 4-Hydroxy-9-methyl-2-oxo-10-((((tetrahydrofuran-2-yl)methyl)amino)methyl)-1,2,5,6,7,9-hexahydropyrido[3',2':6,7]cyclohepta[1,2-f]indol-3-carbonsäurehydrochlorid,
- 4-Hydroxy-9-methyl-10-((1-methylhexahydropyrrolo[3,4-b]pyrrol-5(1H)-yl)methyl)-2-oxo-1,2,5,6,7,9-hexahydropyrido[3',2':6,7]cyclohepta[1,2-f]indol-3-carbonsäuredihydrochlorid,
- 10-(((2-(Dimethylamino)-2-oxoethyl)amino)methyl)-4-hydroxy-9-methyl-2-oxo-1,2,5,6,7,9-hexahydropyrido[3',2':6,7]cyclohepta[1,2-f]indol-3-carbonsäurehydrochlorid,
- 10-(2-(Ethylamino)ethyl)-4-hydroxy-9-methyl-2-oxo-1,2,5,9-tetrahydropyrido[3',2':6,7]cyclohepta[1,2-f]indol-3-carbonsäuretrifluoracetat,
- 10-(2-(Ethylamino)ethyl)-4-hydroxy-9-methyl-2-oxo-1,2,5,6,7,9-hexahydropyrido[3',2':6,7]cyclohepta[1,2-f]indol-3-carbonsäuretrifluoracetat,
- 4-Hydroxy-9-(((*cis*)-octahydrocyclopenta[c]pyrrol-4-yl)-(*cis*)-amino)-2-oxo-1,2,5,6-tetrahydrobenzo[2,3]oxepino[4,5-b]pyridin-3-carbonsäurehydrochlorid,
- 4-Hydroxy-9-methyl-10-(((1-methylcyclopropyl)amino)methyl)-2-oxo-2,5,6,9-tetrahydro-1H-pyrido[2',3':4,5]oxepino[3,2-f]indol-3-carbonsäurehydrochlorid,
- 10-((*sek*.-Butylamino)methyl)-4-hydroxy-9-methyl-2-oxo-2,5,6,9-tetrahydro-1H-pyrido[2',3':4,5]oxepino[3,2-f]indol-3-carbonsäurehydrochlorid,
- 4-Hydroxy-9-methyl-2-oxo-10-((propylamino)methyl)-2,5,6,9-tetrahydro-1H-pyrido[2',3':4,5]oxepino[3,2-f]indol-3-carbonsäurehydrochlorid,
- 10-(((2,4-Dimethoxybenzyl)amino)methyl)-4-hydroxy-9-methyl-2-oxo-2,5,6,9-tetrahydro-1H-pyrido[2',3':4,5]oxepino[3,2-f]indol-3-carbonsäurehydrochlorid,
- 10-((Cyclopropylamino)methyl)-4-hydroxy-9-methyl-2-oxo-2,5,6,9-tetrahydro-1H-pyrido[2',3':4,5]oxepino[3,2-f]indol-3-carbonsäurehydrochlorid,
- 10-((Cyclobutylamino)methyl)-4-hydroxy-9-methyl-2-oxo-2,5,6,9-tetrahydro-1H-pyrido[2',3':4,5]oxepino[3,2-f]indol-3-carbonsäurehydrochlorid,
- 10-((Dimethylamino)methyl)-4-hydroxy-9-methyl-2-oxo-2,5,6,9-tetrahydro-1H-pyrido[2',3':4,5]oxepino[3,2-f]indol-3-carbonsäurehydrochlorid,
- 4-Hydroxy-9-methyl-2-oxo-10-(pyrrolidin-1-ylmethyl)-2,5,6,9-tetrahydro-1H-pyrido[2',3':4,5]oxepino[3,2-f]indol-3-carbonsäurehydrochlorid,
- 10-((tert.-Butylamino)methyl)-4-hydroxy-9-methyl-2-oxo-2,5,6,9-tetrahydro-1H-pyrido[2',3':4,5]oxepino[3,2-f]indol-3-carbonsäurehydrochlorid,
- 4-Hydroxy-9-methyl-10-((4-methylpiperazin-1-yl)methyl)-2-oxo-2,5,6,9-tetrahydro-1H-pyrido[2',3':4,5]oxepino[3,2-f]indol-3-carbonsäuredihydrochlorid,
- 4-Hydroxy-10-(((2-hydroxyethyl)amino)methyl)-9-methyl-2-oxo-2,5,6,9-tetrahydro-1H-pyrido[2',3':4,5]oxepino[3,2-f]indol-3-carbonsäurehydrochlorid,
- 4-Hydroxy-10-(((1-hydroxypropan-2-yl)amino)methyl)-9-methyl-2-oxo-2,5,6,9-tetrahydro-1H-pyrido[2',3':4,5]oxepino[3,2-f]indol-3-carbonsäurehydrochlorid,
- 4-Hydroxy-9-methyl-2-oxo-10-(((2-(pyrrolidin-1-yl)ethyl)amino)methyl)-2,5,6,9-tetrahydro-1H-pyrido[2',3':4,5]oxepino[3,2-f]indol-3-carbonsäuredihydrochlorid,
- 10-(((2-Aminoethyl)amino)methyl)-4-hydroxy-9-methyl-2-oxo-2,5,6,9-tetrahydro-1H-pyrido[2',3':4,5]oxepino[3,2-f]indol-3-carbonsäuredihydrochlorid,
- 4-Hydroxy-9-methyl-10-(((2-(methylamino)ethyl)amino)methyl)-2-oxo-2,5,6,9-tetrahydro-1H-pyrido[2',3':4,5]oxepino[3,2-f]indol-3-carbonsäuredihydrochlorid,
- 10-(((2-(Dimethylamino)ethyl)amino)methyl)-4-hydroxy-9-methyl-2-oxo-2,5,6,9-tetrahydro-1H-pyrido[2',3':4,5]oxepino[3,2-f]indol-3-carbonsäuredihydrochlorid,
- 4-Hydroxy-10-(((2-methoxyethyl)amino)methyl)-9-methyl-2-oxo-2,5,6,9-tetrahydro-1H-pyrido[2',3':4,5]oxepino[3,2-f]indol-3-carbonsäurehydrochlorid und
- 4-Hydroxy-10-(((1-methoxypropan-2-yl)amino)methyl)-9-methyl-2-oxo-2,5,6,9-tetrahydro-1H-pyrido[2',3':4,5]oxepino[3,2-f]indol-3-carbonsäurehydrochlorid
ausgewählt ist, wobei die Form der Verbindung aus einer isotopologischen, racemischen, enantiomeren, diastereomeren, stereoisomeren, polymorphen, tautomeren, Freien-Säure-, Freien-Basen-, Hydrat-, Solvat- oder Clathratform davon ausgewählt ist

9. Verbindung nach Anspruch 1 zur Verwendung bei der Behandlung einer bakteriellen Infektion.

10. Verbindung zur Verwendung nach Anspruch 9, wobei die bakterielle Infektion von einem Bakterium, das ein multiarzneimittelresistentes gramnegatives oder grampositives Bakterium ist, verursacht worden ist.

11. Verbindung zur Verwendung nach Anspruch 9, wobei die Behandlung eine Kombinationstherapie ist, die einen wirksamen Anteil an einer Verbindung nach Anspruch 1 oder einer Form davon und einem wirksamen Anteil an einem antibiotischen oder antibakteriellen Mittel umfasst.

12. Pharmazeutische Zubereitung, die einen wirksamen Anteil an einer Verbindung nach Anspruch 1 oder einer Form davon zusammen mit einem pharmazeutisch verträglichen Arzneimittelträger umfasst.

13. Verbindung nach Anspruch 1 zur Verwendung als Arzneimittel.

## Revendications

1. Composé de formule (I), de formule (II) ou de formule (III) : 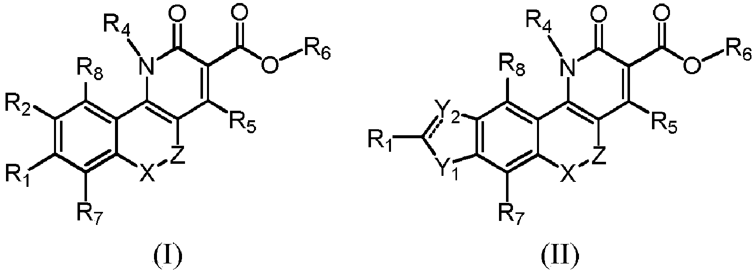 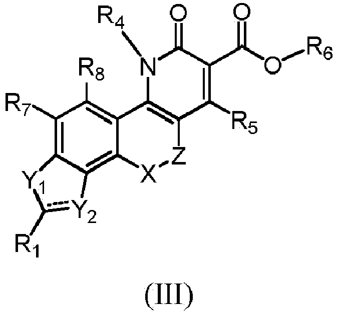 ou l'une de leurs formes, dans lequel la forme du composé est choisie parmi une forme d'acide libre, de base libre, de sel, d'hydrate, de solvate, de clathrate, d'isotopologue,
de racémate, d'énantiomètre, de diastéréomère, de stéréoisomère, polymorphe ou tautomérique de celui-ci, dans lequel :
N(R₁₄), S, O, -CH(R₉)-, -CH(R₉)-CH(R₁₀)-,
X est une liaison, -CH(R₉)-CH(R₁₀)-CH(R₁₁)-, -C(R₉)=C(R₁₀)-, -C(R₉)=C(R₁₀)-CH(R₁₁)-, -CH(R₉)-C(R₁₀)=C(R₁₁)-, -O-CH(R₁₀)-, -CH(R₉)-O-, -N(R₁₄)-CH(R₁₀)-, -CH(R₉)-N(R₁₄)-, -S-CH(R₁₀)-, -CH(R₉)-S-, -O-CH(R₁₀)-CH(R₁₁)-, -CH(R₉)-O-CH(R₁₁)-, -CH(R₉)-CH(R₁₀)-O-, -N(R₁₄)-CH(R₁₀)-CH(R₁₁)-, -CH(R₉)-N(R₁₄)-CH(R₁₁)-, -CH(R₉)-CH(R₁₀)-N(R₁₄)-, -S-CH(R₁₀)-CH(R₁₁)-, -CH(R₉)-S-CH(R₁₁)-, -CH(R₉)-CH(R₁₀)-S-; -O-C(O)-CH(R₁₁)-, -C(O)-O-CH(R₁₁)-, -CH(R₉)-O-C(O)-, -CH(R₉)-C(O)-O-, -N(R₁₄)-C(O)-CH(R₁₁)-, -C(O)-N(R₁₄)-CH(R₁₁)-, -CH(R₉)-N(R₁₄)-C(O)-, -CH(R₉)-C(O)-N(R₁₄)-, -S-C(O)-CH(R₁₁)-, -C(O)-S-CH(R₁₁)-, -CH(R₉)-S-C(O)- ou -CH(R₉)-C(O)-S-;
Y₁ est -N(R₁₂)- ou -O- ;
Y₂ est -C(R₁₃)-, -N(R₁₂)- ou -O- ; dans lequel les tirets représentent une double liaison présente quand Y₂ est -C(R₁₃)-et absente quand Y₂ est -N(R₁₂)- ou -O- ;
Z est N(R₁₄), S, O, C(O) ou -CH(R₃)- ;
R₁ est un halogène, hydroxyle, oxo, cyano, nitro, C₁₋₈ alkyle, hydroxyle-C₁₋₈ alkyle, halo-C₁₋₈ alkyle, C₁₋₈ alcoxy, halo-C₁₋₈ alcoxy, C₁₋₈ alkyl-thio, carboxyle, C₁₋₈ alkyl-carbonyle, C₁₋₈ alcoxy-carbonyle, amino-carbonyle, amino, C₁₋₈ alkyl-amino, (C₁₋₈ alkyl)₂-amino, C₂₋₈ alkényl-amino, (C₂₋₈ alkényl)₂-amino, C₂₋₈ alkynyl-amino, (C₂₋₈ alkynyl)₂-amino, amino-C₁₋₈ alkyle, C₁₋₁₀ alkyl-amino-C₁₋₈ alkyle, (C₁₋₈ alkyl)₂-amino-C₁₋₈ alkyle, C₂₋₈ alkényl-amino-C₁₋₈ alkyle, (C₂₋₈ alkényl)₂-amino-C₁₋₈ alkyle, C₂₋₈ alkynyl-amino-C₁₋₈ alkyle, (C₂₋₈ alkynyl)₂-amino-C₁₋₈ alkyle, halo-C₁₋₈ alkyl-amino, halo-(C₁₋₈ alkyl)₂-amino, halo-C₁₋₈ alkyl-amino-C₁₋₈ alkyle, (halo-C₁₋₈ alkyl)₂-amino-C₁₋₈ alkyle, C₁₋₈ alcoxy-C₁₋₈ alkyl-amino, (C₁₋₈ alcoxy-C₁₋₈ alkyle, C₁₋₈ alkyl)-amino, (C₁₋₈ alcoxy-C₁₋₈ alkyl)₂-amino, C₁₋₈ alcoxy-C₁₋₈ alkyl-amino-C₁₋₈ alkyle, (C₁₋₈ alcoxy-C₁₋₈ alkyl, C₁₋₈ alkyl)-amino-C₁₋₈ alkyle, (C₁₋₈ alcoxy-C₁₋₈ alkyl)₂-amino-C₁₋₈ alkyle, amino-C₁₋₈ alkyl-amino, (amino-C₁₋₈ alkyle, C₁₋₈ alkyl) -amino, C₁₋₈ alkyl-amino-C₁₋₈ alkyl-amino, (C₁₋₈ alkyl-amino-C₁₋₈ alkyle, C₁₋₈ alkyl)amino, (C₁₋₈ alkyl)₂-amino-C₁₋₈ alkyl-amino, [(C₁₋₈ alkyl)₂-amino-C₁₋₈ alkyle, C₁₋₈ alkyl]amino, amino-C₁₋₈ alkyl-amino-C₁₋₈ alkyle, (amino-C₁₋₈ alkyle, C₁₋₈ alkyl)amino-C₁₋₈ alkyle, C₁₋₈ alkyl-amino-C₁₋₈ alkyl-amino-C₁₋₈ alkyle, (C₁₋₈ alkyl-amino-C₁₋₈ alkyle, C₁₋₈ alkyl)amino-C₁₋₈ alkyle, (C₁₋₈ alkyl)₂-amino-C₁₋₈ alkyl-amino-C₁₋₈ alkyle, [(C₁₋₈ alkyl)₂-amino-C₁₋₈ alkyle, C₁₋₈ alkyl]amino-C₁₋₈ alkyle, hydroxyl-amino, hydroxyl-C₁₋₈ alkyl-amino, (hydroxyl-C₁₋₈ alkyle, C₁₋₈ alkyl)amino, (hydroxyl-C₁₋₈ alkyl)₂-amino, hydroxyl-C₁₋₈ alkyl-amino-C₁₋₈ alkyle, (hydroxyl-C₁₋₈ alkyle, C₁₋₈ alkyl)amino-C₁₋₈ alkyle, hydroxyl-C₁₋₈ alkyl-amino-C₁₋₈ alkyl-amino, (hydroxyl-C₁₋₈ alkyl-amino-C₁₋₈ alkyle, C₁₋₈ alkyl)amino, (hydroxyl-C₁₋₈ alkyle, C₁₋₈ alkyl)amino-C₁₋₈ alkyl-amino, [(hydroxyl-C₁₋₈ alkyle, C₁₋₈ alkyl)amino-C₁₋₈ alkyle, C₁₋₈ alkyl]amino, (C₁₋₈ alkyl-carbonyl, C₁₋₈ alkyl)amino-C₁₋₈ alkyle, C₁₋₈ alkyl-amino-carbonyl, (C₁₋₈ alkyl)₂-amino-carbonyl, (C₁₋₈ alkyl)₂-amino-carbonyl-C₁₋₈ alkyl-amino-C₁₋₈ alkyle, C₃₋₁₄ cycloalkyle, C₃₋₁₄ cycloalkyl-C₁₋₈ alkyle, C₃₋₁₄ cycloalkyl-oxy, C₃₋₁₄ cycloalkyl-C₁₋₈ alcoxy, C₃₋₁₄ cycloalkyl-amino, C₃₋₁₄ cycloalkyl-amino-C₁₋₈ alkyle, (C₃₋₁₄ cycloalkyle, C₁₋₈ alkyl)-amino-C₁₋₈ alkyle, (C₃-₁₄ cycloalkyl)₂-amino-C₁₋₈ alkyle, C₃₋₁₄ cycloalkyl-C₁₋₈ alkyl-amino-C₁₋₈ alkyle, (C₃₋₁₄ cycloalkyl-C₁₋₈ alkyl, C₁₋₈ alkyl)-amino-C₁₋₈ alkyle, (C₃₋₁₄ cycloalkyl-C₁₋₈ alkyl)₂-amino-C₁₋₈ alkyle, aryle, aryl-C₁₋₈ alkyle, aryl-amino, (aryl, C₁₋₈ alkyl)amino, (aryl)₂-amino, aryl-amino-C₁₋₈ alkyle, (aryl, C₁₋₈ alkyl)-amino-C₁₋₈ alkyle, (aryl)₂-amino-C₁₋₈ alkyle, aryl-C₁₋₈ alkyl-amino, (aryl-C₁₋₈ alkyl, C₁₋₈ alkyl)amino, (aryl-C₁₋₈ alkyl)₂-amino, aryl-C₁₋₈ alkyl-amino-C₁₋₈ alkyle, (aryl-C₁₋₈ alkyl, C₁₋₈ alkyl)amino-C₁₋₈ alkyle, (aryl-C₁₋₈ alkyl)₂-amino-C₁₋₈ alkyle, un hétéroaryle choisi dans le groupe comprenant le pyrrolyle, le thiazolyle, le 1H-1,2,3-triazolyle, le 1H-tétrazolyle, le 2H-tétrazolyle, l'imidazolyle et le pyridinyle, les hétéroaryl-C₁₋₈ alkyle, hétéroaryl-amino, hétéroaryl-C₁₋₈ alkyl-amino, (hétéroaryl-C₁₋₈ alkyl, C₁₋₈ alkyl)amino, hétéroaryl-C₁₋₈ alkyl)₂-amino, hétéroaryl-C₁₋₈ alkyl-amino-C₁₋₈ alkyle, (hétéroaryl-C₁₋₈ alkyl, C₁₋₈ alkyl)amino-C₁₋₈ alkyle, un hétérocyclyle choisi dans le groupe comprenant l'azétidinyle, le pyrrolidinyle, le tétrahydrofuranyle,
le pipéridinyle, le pipérazinyle, le morpholinyle, le 1,4-diazépanyle, le 1,3 dioxolanyle, le 2,5-dihydro-1H-pyrrolyle, le dihydro-1H-imidazolyle, le 1,4,5,6-tétrahydropyrimidinyle, le 1,2,3,6-tétrahydropyridinyle, le tétrahydro-2H-pyranyle, l'indolinyle, le 2,3-dihydrobenzo[d]oxazolyle, le 3,4-dihydro-2H-benzo[b][1,4]oxazinyle, le 3,4-dihydroisoquinolin-(1H)-yle, le 1,2,3,4-tétrahydroisoquinolinyle, le 1,2,3,4-tétrahydroquinoxalinyle, l'hexahydropyrrolo[3,4-b][1,4]oxazin-(2H)-yle,
le (4aR,7aS)-hexahydropyrrolo[3,4-b][1,4]oxazin-(4aH)-yle, le 3,4-dihydro-2H-pyrido[3,2-b][1,4]oxazinyle, le (cis)-octahydrocyclopenta[c]pyrrolyl, l'hexahydropyrrolo[3,4-b]pyrrol-(1H)-yle, le (3aR,6aR)-hexahydropyrrolo[3,4-b]pyrrol-(1H)-yle, le (3aR,6aS)-hexahydropyrrolo[3,4-c]pyrrol-(1H)-yle, le 5H-pyrrolo[3,4-b]pyridin-(7H)-yle, le 5,7-dihydro-6H-pyrrolo[3,4-b]pyridinyle,
le tétrahydro-1H-pyrrolo[3,4-b]pyridin-(2H,7H,7aH)-yle, l'hexahydro-1H-pyrrolo[3,4-b]pyridin-(2H)-yle, le (4aR,7aR)-hexahydro-1H-pyrrolo[3,4-b]pyridin-(2H)-yle, l'octahydro-6H-pyrrolo[3,4-b]pyridinyle, le 2,3,4,9-tétrahydro-1H-carbazolyle, le 1,2,3,4-tétrahydropyrazino[1,2-a]indol-8-yle, le 2,3-dihydro-1H-pyrrolo[1,2,a]indolyle, le (3aR,6aR)-hexahydrocyclopenta[c]pyrrol-(1H)-yle, le (3aR,4R,6aS)-hexahydrocyclopenta[c]pyrrol-(1H)-yle, le (3aR,4S,6aS)-hexahydrocyclopenta[c]pyrrol-(1H)-yle, le (3aR,5r,6aS)-hexahydrocyclopenta[c]pyrrol-(1H)-yle, le 1,3-dihydro-2H-isoindolyle, l'octahydro-2H-isoindolyle, le (3aS)-1,3,3a,4,5,6-hexahydro-2H-isoindolyle,
le (3aR,4R,7aS)-1H-isoindol-(3H,3aH,4H,5H,6H,7H,7aH)-yle, le (3aR,7aS)-octahydro-2H-isoindolyle, le (3aR,4R,7aS)-octahydro-2H-isoindolyle,
le (3aR,4S,7aS)-octahydro-2H-isoindolyle, le 2,5-diazabicyclo[2.2.1]heptanyl, le 2-azabicyclo[2.2.1]hept-5-enyle, le 3-azabicyclo[3.1.0]hexanyle, le (1R,5S,6s)-3-azabicyclo[3.1.0]hexanyle, le (cis,cis)-3-azabicyclo[3.1.0]hexanyle, le 3,6-diazabicyclo[3.1.0]hexanyle, le (1S,5R,6R)-3-azabicyclo[3.2.0]heptanyle,
le (1S,5R,6S)-3-azabicyclo[3.2.0]heptanyle, le 5-azaspiro[2.4]heptanyle, le 2,6-diazaspiro[3.3]heptanyle, le 2,5-diazaspiro[3.4]octanyle, le 2,6-diazaspiro[3.4]octanyle, le 2,7-diazaspiro[3.5]nonanyle, le 2,7-diazaspiro[4.4]nonanyle, le 2-azaspiro[4.5]décanyle et le 2,8-diazaspiro[4.5]décanyle, les hétérocyclyl-C₁₋₈ alkyle, hétérocyclyle oxy, hétérocyclyl-C₁₋₈ alcoxy, hétérocyclyl-amino, (hétérocyclyl, C₁₋₈ alkyl)amino, (hétérocyclyl)₂-amino, hétérocyclyl-amino-C₁₋₈ alkyle, (hétérocyclyl, C₁₋₈ alkyl)amino-C₁₋₈ alkyle, (hétérocyclyl)₂-amino-C₁₋₈ alkyle, (hétérocyclyl, C₃₋₁₄ cycloalkyl-C₁₋₈ alkyl)amino-C₁₋₈ alkyle, hétérocyclyl-C₁₋₈ alkyl-amino-C₁₋₈ alkyle, (hétérocyclyl-C₁₋₈ alkyl,C₁₋₈ alkyl)-amino-C₁₋₈ alkyle, (hétérocyclyl-C₁₋₈ alkyl)₂-amino-C₁₋₈ alkyle, hétérocyclyl-oxy-amino, (hétérocyclyl-oxy,C₁₋₈ alkyl)-amino, (hétérocyclyl-oxy)₂-amino, (hétérocyclyl-oxy-C₁₋₈ alkyl,C₁₋₈ alkyl)-amino, hétérocyclyl-carbonyle ou hétérocyclyl-carbonyl-oxy ;
dans lequel chaque instance de C₃₋₁₄ cycloalkyle, aryle, hétérocyclyle et hétéroaryle est facultativement substituée par un, deux ou trois substituants, chacun choisi parmi R₁₅ ; et
dans lequel chaque instance d'aryle, hétérocyclyle ou hétéroaryle est facultativement substituée par un substituant supplémentaire choisi parmi R₁₆ ;
R₂ est l'hydrogène, un halogène, un hydroxyle, un C₁₋₈ alkyle, un amino, un C₁₋₈ alkyl-amino ou un (C₁₋₈ alkyl)₂-amino ;
R₃ est l'hydrogène, un halogène, un hydroxyle, un C₁₋₈ alkyle, un amino, un C₁₋₈ alkyl-amino ou un (C₁₋₈ alkyl)₂-amino ;
R₄ est l'hydrogène, un hydroxyle, un aryl-C₁₋₈ alkyle, dans lequel l'aryle est facultativement substitué par un substituant supplémentaire choisi parmi un C₁₋₈ alkyle, un halo-C₁₋₈ alkyle, un C₁₋₈ alcoxy ou un C₁₋₈ alcoxy-C₁₋₈ alkyle ;
R₅ est l'hydrogène, un halogène, un hydroxyle, un C₁₋₈ alkyle, un C₁₋₈ alcoxy, un carboxyle, un amino, un C₁₋₈ alkyl-amino, un (C₁₋₈ alkyl)₂-amino ou un C₁₋₈ alkyl-SO₂-amino ;
R₆ est l'hydrogène ou un C₁₋₈ alkyle ;
R₇ est l'hydrogène, un halogène, un hydroxyle, un C₁₋₈ alkyle, un amino, un C₁₋₈ alkyl-amino ou un (C₁₋₈ alkyl)₂-amino ;
R₈ est l'hydrogène, un halogène, un hydroxyle, un C₁₋₈ alkyle, un amino, un C₁₋₈ alkyl-amino ou un (C₁₋₈ alkyl)₂-amino ;
R₉ est l'hydrogène, un halogène, un hydroxyle, un C₁₋₈ alkyle, un amino, un C₁₋₈ alkyl-amino ou un (C₁₋₈ alkyl)₂-amino ;
R₁₀ est l'hydrogène, un halogène, un hydroxyle, un C₁₋₈ alkyle, un amino, un C₁₋₈ alkyl-amino ou un (C₁₋₈ alkyl)₂-amino ;
R₁₁ est l'hydrogène, un halogène, un hydroxyle, un C₁₋₈ alkyle, un amino, un C₁₋₈ alkyl-amino ou un (C₁₋₈ alkyl)₂-amino ;
R₁₂ est l'hydrogène, un C₁₋₈ alkyle, un aryle ou un hétéroaryle, dans lequel l'aryle et l'hétéroaryle sont facultativement substitués par un substituant supplémentaire choisi parmi un C₁₋₈ alkyle, un halo-C₁₋₈ alkyle, un C₁₋₈ alcoxy ou un C₁₋₈ alcoxy-C₁₋₈ alkyle ;
R₁₃ est l'hydrogène, un cyano, un halogène, un hydroxyle ou un C₁₋₈ alkyle ;
R₁₄ est l'hydrogène, un C₁₋₈ alkyle, un C₁₋₈ alkyl-carbonyle, un C₁₋₈ alcoxy-carbonyle, un aryle ou un hétéroaryle, dans lequel l'aryle et l'hétéroaryle sont facultativement substitués par un substituant supplémentaire choisi parmi un C₁₋₈ alkyle, un halo-C₁₋₈ alkyle, un C₁₋₈ alcoxy ou un C₁₋₈ alcoxy-C₁₋₈ alkyle ;
R₁₅ est un azido, un halogène, un hydroxyle, un cyano, un nitro, un C₁₋₈ alkyle, un halo-C₁₋₈ alkyle,
un hydroxyl-C₁₋₈ alkyle, un C₁₋₈ alcoxy-C₁₋₈ alkyle, un C₁₋₈ alcoxy, un halo-C₁₋₈ alcoxy, un hydroxyl-C₁₋₈ alcoxy, un carboxyle, un C₁₋₈ alkyl-carbonyle, un C₁₋₈ alcoxy-carbonyle, un amino, un C₁₋₈ alkyl-amino, un (C₁₋₈ alkyl)₂-amino, un halo-C₁₋₈ alkyl-amino,
un (halo-C₁₋₈ alkyl)₂-amino, un halo-C₁₋₈ alkyl-amino-C₁₋₈ alkyle,
un (halo-C₁₋₈ alkyl)₂-amino-C₁₋₈ alkyle, amino-C₁₋₈ alkyle,
un C₁₋₈ alkyl-amino-C₁₋₈ alkyle, un (C₁₋₈ alkyl)₂-amino-C₁₋₈ alkyle,
un [(C₁₋₈ alkyl)₂-amino-C₁₋₈ alkyle, C₁₋₈ alkyl]amino-C₁₋₈ alkyle, un C₁₋₈ alkyl-thio, un amino-carbonyle, un C₁₋₈ alkyl-amino-carbonyle, un (C₁₋₈ alkyl)₂-amino-carbonyle,
un C₁₋₈ alkyl-carbonyl-amino ou
un (carboxyl-C₁₋₈ alkyle, C₁₋₈ alkyl)amino-carbonyl-amino ;
R₁₆ est un C₃₋₁₄ cycloalkyle, un C₃₋₁₄ cycloalkyl-amino, un aryle, un aryl-C₁₋₈ alkyle, un aryl-amino,
un (aryl, C₁₋₈ alkyl)amino, (aryl)₂-amino, un aryl-C₁₋₈ alkyl-amino,
un (aryl-C₁₋₈ alkyl, C₁₋₈ alkyl)amino, un (aryl-C₁₋₈ alkyl)₂-amino,
un aryl-C₁₋₈ alkyl-amino-C₁₋₈ alkyle, un (aryl-C₁₋₈ alkyl, C₁₋₈ alkyl)amino-C₁₋₈ alkyle,
un (aryl-C₁₋₈ alkyl)₂-amino-C₁₋₈ alkyle, un aryl-amino-C₁₋₈ alkyle,
un (aryl-C₁₋₈ alkyl)amino-C₁₋₈ alkyle, un (aryl)₂-amino-C₁₋₈ alkyle,
un aryl-amino-carbonyle, un aryl-C₁₋₈ alcoxy, un aryl-C₁₋₈ alcoxy-carbonyl-amino,
un hétéroaryle, un hétéroaryl-C₁₋₈ alkyle, un hétéroaryl-amino, un (hétéroaryl)₂-amino,
un hétéroaryl-C₁₋₈ alkyl-amino, un (hétéroaryl-C₁₋₈ alkyle, C₁₋₈ alkyl)amino,
un (hétéroaryl-C₁₋₈ alkyl)₂-amino, un hétéroaryl-C₁₋₈ alkyl-amino-C₁₋₈ alkyle,
un (hétéroaryl-C₁₋₈ alkyle, C₁₋₈ alkyl)amino-C₁₋₈ alkyle,
un (hétéroaryl-C₁₋₈ alkyl)₂-amino-₁₋₈ alkyle, un hétérocyclyle,
un hétérocyclyl-C₁₋₈ alkyle, un hétérocyclyl-amino-C₁₋₈ alkyle ou un hétérocyclyl-oxy ;
dans lequel chaque instance de C₃₋₁₄ cycloalkyle est facultativement substituée par un substituant choisi parmi R₁₈ ; et
dans lequel chaque instance d'aryle est facultativement substituée par un substituant choisi parmi R₁₉ ;
R₁₈ est un amino, un C₁₋₈ alkyl-amino, un (C₁₋₈ alkyl)₂-amino, un amino-C₁₋₈ alkyle,
un C₁₋₈ alkyl-amino-C₁₋₈ alkyle, un (C₁₋₈ alkyl)₂-amino-C₁₋₈ alkyle ou
un aryl-C₁₋₈ alkyl-amino ; et
R₁₉ est un halogène.

2. Composé selon la revendication 1, dans lequel R₁ est
un C₃₋₁₄ cycloalkyle choisi pour chaque instance, le cas échéant, parmi le cyclopropyle, le cyclobutyle, le cyclopentyle, le cyclohexyle ou le cycloheptyle ;
un aryle pour lequel on choisit, pour chaque instance, le cas échéant, un phényle ;
un hétéroaryle choisi pour chaque instance, le cas échéant, parmi le pyrrolyle, le thiazolyle, le 1H-1,2,3-triazolyle, le 1H-tétrazolyle, le 2H-tétrazolyle, l'imidazolyle ou le pyridinyle ;
un hétérocyclyle choisi pour chaque instance, le cas échéant, parmi l'azétidinyle,
le pyrrolidinyle, le tétrahydrofuranyle, le pipéridinyle, le pipérazinyle, le morpholinyle, le 1,4-diazépanyle, le 1,3 dioxolanyle, le 2,5-dihydro-1H-pyrrolyle, le dihydro-1H-imidazolyle, le 1,4,5,6-tétrahydropyrimidinyle, le 1,2,3,6-tétrahydropyridinyle,
le tétrahydro-2H-pyranyle, l'indolinyle, le 2,3-dihydrobenzo[d]oxazolyle, le 3,4-dihydro-2H-benzo[b][1,4]oxazinyle, le 3,4-dihydroisoquinolin-(1H)-yle,
le 1,2,3,4-tétrahydroisoquinolinyle, le 1,2,3,4-tétrahydroquinoxalinyle, l'hexahydropyrrolo[3,4-b][1,4]oxazin-(2H)-yle, le (4aR,7aS)-hexahydropyrrolo[3,4-b][1,4]oxazin-(4aH)-yle, le 3,4-dihydro-2H-pyrido[3,2-b][1,4]oxazinyle, le (cis)-octahydrocyclopenta[c]pyrrolyl, l'hexahydropyrrolo[3,4-b]pyrrol-(1H)-yle, le (3aR,6aR)-hexahydropyrrolo[3,4-b]pyrrol-(1H)-yle, le (3aR,6aS)-hexahydropyrrolo[3,4-c]pyrrol-(1H)-yle, le 5H-pyrrolo[3,4-b]pyridin-(7H)-yle, le 5,7-dihydro-6H-pyrrolo[3,4-b]pyridinyle,
le tétrahydro-1H-pyrrolo[3,4-b]pyridin-(2H,7H,7aH)-yle, l'hexahydro-1H-pyrrolo[3,4-b]pyridin-(2H)-yle, le (4aR,7aR)-hexahydro-1H-pyrrolo[3,4-b]pyridin-(2H)-yle, l'octahydro-6H-pyrrolo[3,4-b]pyridinyle, le 2,3,4,9-tétrahydro-1H-carbazolyle, le 1,2,3,4-tétrahydropyrazino[1,2-a]indol-8-yle, le 2,3-dihydro-1H-pyrrolo[1,2,a]indolyle, le (3aR,6aR)-hexahydrocyclopenta[c]pyrrol-(1H)-yle, le (3aR,4R,6aS)-hexahydrocyclopenta[c]pyrrol-(1H)-yle, le (3aR,4S,6aS)-hexahydrocyclopenta[c]pyrrol-(1H)-yle, le (3aR,5r,6aS)-hexahydrocyclopenta[c]pyrrol-(1H)-yle, le 1,3-dihydro-2H-isoindolyle, l'octahydro-2H-isoindolyle, le (3aS)-1,3,3a,4,5,6-hexahydro-2H-isoindolyle,
le (3aR,4R,7aS)-1H-isoindol-(3H,3aH,4H,5H,6H,7H,7aH)-yle, le (3aR,7aS)-octahydro-2H-isoindolyle, le (3aR,4R,7aS)-octahydro-2H-isoindolyle,
le (3aR,4S,7aS)-octahydro-2H-isoindolyle, le 2,5-diazabicyclo[2.2.1]heptanyl, le 2-azabicyclo[2.2.1]hept-5-enyle, le 3-azabicyclo[3.1.0]hexanyle, le (1R,5S,6s)-3-azabicyclo[3.1.0]hexanyle, le (cis,cis)-3-azabicyclo[3.1.0]hexanyle, le 3,6-diazabicyclo[3.1.0]hexanyle, le (1S,5R,6R)-3-azabicyclo[3.2.0]heptanyle,
le (1S,5R,6S)-3-azabicyclo[3.2.0]heptanyle, le 5-azaspiro[2.4]heptanyle, le 2,6-diazaspiro[3.3]heptanyle, le 2,5-diazaspiro[3.4]octanyle, le 2,6-diazaspiro[3.4]octanyle, le 2,7-diazaspiro[3.5]nonanyle, le 2,7-diazaspiro[4.4]nonanyle, le 2-azaspiro[4.5]décanyle et le 2,8-diazaspiro[4.5]décanyle.

3. Composé selon la revendication 1, dans lequel :
R₂ est l'hydrogène, un C₁₋₈ alkyle, un amino, un C₁₋₈ alkyl-amino ou un (C₁₋₈ alkyl)₂-amino ;
R₃ est l'hydrogène, un halogène, un hydroxyle, un C₁₋₈ alkyle, un amino, un C₁₋₈ alkyl-amino ou
un (C₁₋₈ alkyl)₂-amino ; et
R₄ est l'hydrogène.

4. Composé selon la revendication 1, dans lequel R₁ est
un C₃₋₁₄ cycloalkyl-amino-C₁₋₈ alkyle, dans lequel le C₃₋₁₄ cycloalkyle est choisi parmi le cyclopropyle, le cyclobutyle, le cyclopentyle ou le cyclohexyle ;
un (C₃₋₁₄ cycloalkyle, C₁₋₈ alkyl)-amino-C₁₋₈ alkyle, dans lequel le C₃₋₁₄ cycloalkyle est choisi parmi le cyclopropyle, le cyclobutyle ou le cyclopentyle ;
un C₃₋₁₄ cycloalkyl-C₁₋₈ alkyl-amino-C₁₋₈ alkyle, dans lequel le C₃₋₁₄ cycloalkyle est choisi parmi le cyclopropyle, le cyclobutyle, le cyclopentyle, le cyclohexyle ou le cycloheptyle ;
un aryle, dans lequel l'aryle est un phényle ;
un aryl-amino, dans lequel l'aryle est un phényle ;
un (aryl, C₁₋₈ alkyl)amino, dans lequel l'aryle est un phényle ;
un aryl-amino-C₁₋₈ alkyle, dans lequel l'aryle est un phényle ;
un aryl-C₁₋₈ alkyl-amino-C₁₋₈ alkyle, dans lequel l'aryle est un phényle ;
un (aryl-C₁₋₈ alkyl, C₁₋₈ alkyl)amino-C₁₋₈ alkyle, dans lequel l'aryle est un phényle ;
un (aryl-C₁₋₈ alkyl)₂-amino-C₁₋₈ alkyle, dans lequel l'aryle est un phényle ;
un hétéroaryle, dans lequel l'hétéroaryle est choisi parmi le pyrrolyle, le thiazolyle, le 1H-1,2,3-triazolyle, le 1H-tétrazolyle, le 2H-tétrazolyle, l'imidazolyle ou le pyridinyle ;
un hétéroaryl-C₁₋₈ alkyl-amino-C₁₋₈ alkyle, dans lequel l'hétéroaryle est choisi parmi le pyridin-2-yle, le pyridin-3-yle ou le pyridin-4-yle ;
un (hétéroaryl-C₁₋₈ alkyl, C₁₋₈ alkyl)amino-C₁₋₈ alkyle, dans lequel l'hétéroaryle est choisi parmi le pyridin-3-yle ou le pyridin-4-yle ;
un hétérocyclyle, dans lequel l'hétérocyclyle est choisi parmi l'azétidinyle, le pyrrolidinyle, le tétrahydrofuranyle, le pipéridinyle, le pipérazinyle, le morpholinyle, le 1,4-diazépanyle,
le 1,3 dioxolanyle, le 2,5-dihydro-1H-pyrrolyl, le dihydro-1H-imidazolyle, le 1,4,5,6-tétrahydropyrimidinyle, le 1,2,3,6-tétrahydropyridinyle, le tétrahydro-2H-pyranyle,
le 3,4-dihydroisoquinolin-(1H)-yle, le 1,2,3,4-tétrahydroisoquinolinyle, l'hexahydropyrrolo[3,4-b][1,4]oxazin-(2H)-yle, le (4aR,7aS)-hexahydropyrrolo[3,4-b][1,4]oxazin-(4aH)-yle, le (cis)-octahydrocyclopenta[c]pyrrolyl, l'hexahydropyrrolo[3,4-b]pyrrol-(1H)-yle,
le (3aR,6aR)-hexahydropyrrolo[3,4-b]pyrrol-(1H)-yle, le (3aR,6aS)-hexahydropyrrolo[3,4-c]pyrrol-(1H)-yle, le 5H-pyrrolo[3,4-b]pyridin-(7H)-yle,
le 5,7-dihydro-6H-pyrrolo[3,4-b]pyridinyle, le tétrahydro-1H-pyrrolo[3,4-b]pyridin-(2H,7H,7aH)-yle, l'hexahydro-1H-pyrrolo[3,4-b]pyridin-(2H)-yle,
le (4aR,7aR)-hexahydro-1H-pyrrolo[3,4-b]pyridin-(2H)-yle, l'octahydro-6H-pyrrolo[3,4-b]pyridinyle, le (3aR,6aR)-hexahydrocyclopenta[c]pyrrol-(1H)-yle,
le (3aR,4R,6aS)-hexahydrocyclopenta[c]pyrrol-(1H)-yle, le (3aR,4S,6aS)-hexahydrocyclopenta[c]pyrrol-(1H)-yle, le (3aR,5r,6aS)-hexahydrocyclopenta[c]pyrrol-2(1H)-yle, le 1,3-dihydro-2H-isoindolyle, l'octahydro-2H-isoindolyle, le (3aS)-1,3,3a,4,5,6-hexahydro-2H-isoindolyle,
le (3aR,4R,7aS)-1H-isoindol-(3H,3aH,4H,5H,6H,7H,7aH)-yle, le (3aR,7aS)-octahydro-2H-isoindolyle, le (3aR,4R,7aS)-octahydro-2H-isoindolyle,
le (3aR,4S,7aS)-octahydro-2H-isoindolyle, le 2,5-diazabicyclo[2.2.1]heptanyl, le 2-azabicyclo[2.2.1]hept-5-enyle, le 3-azabicyclo[3.1.0]hexanyle, le (1R,5S,6s)-3-azabicyclo[3.1.0]hexanyle, le (cis,cis)-3-azabicyclo[3.1.0]hexanyle, le 3,6-diazabicyclo[3.1.0]hexanyle, le (1S,5R,6R)-3-azabicyclo[3.2.0]heptanyle,
le (1S,5R,6S)-3-azabicyclo[3.2.0]heptanyle, le 5-azaspiro[2.4]heptanyle, le 2,6-diazaspiro[3.3]heptanyle, le 2,5-diazaspiro[3.4]octanyle, le 2,6-diazaspiro[3.4]octanyle, le 2,7-diazaspiro[3.5]nonanyle, le 2,7-diazaspiro[4.4]nonanyle, le 2-azaspiro[4.5]décanyle et le 2,8-diazaspiro[4.5]décanyle ;
un hétérocyclyl-C₁₋₈ alkyle, dans lequel l'hétérocyclyle est choisi parmi l'azétidinyle,
le pyrrolidinyle, le tétrahydrofuranyle, le pipéridinyle, le pipérazinyle, le morpholinyle, le 1,4-diazépanyle, le 1,3 dioxolanyle, le 2,5-dihydro-1H-pyrrolyle, le dihydro-1H-imidazolyle, le 1,4,5,6-tétrahydropyrimidinyle, le 1,2,3,6-tétrahydropyridinyle,
le tétrahydro-2H-pyranyle, le 3,4-dihydroisoquinolin-(1H)-yle, le 1,2,3,4-tétrahydroisoquinolinyle, l'hexahydropyrrolo[3,4-b][1,4]oxazin-(2H)-yle, le (4aR,7aS)-hexahydropyrrolo[3,4-b][1,4]oxazin-(4aH)-yle, le (cis)-octahydrocyclopenta[c]pyrrolyle, l'hexahydropyrrolo[3,4-b]pyrrol-(1H)-yle,
le (3aR,6aR)-hexahydropyrrolo[3,4-b]pyrrol-(1H)-yle, le (3aR,6aS)-hexahydropyrrolo[3,4-c]pyrrol-(1H)-yle, le 5H-pyrrolo[3,4-b]pyridin-(7H)-yle,
le 5,7-dihydro-6H-pyrrolo[3,4-b]pyridinyle, le tétrahydro-1H-pyrrolo[3,4-b]pyridin-(2H,7H,7aH)-yle, l'hexahydro-1H-pyrrolo[3,4-b]pyridin-(2H)-yle,
le (4aR,7aR)-hexahydro-1H-pyrrolo[3,4-b]pyridin-(2H)-yle, l'octahydro-6H-pyrrolo[3,4-b]pyridinyle, le (3aR,6aR)-hexahydrocyclopenta[c]pyrrol-(1H)-yle,
le (3aR,4R,6aS)-hexahydrocyclopenta[c]pyrrol-(1H)-yle, le (3aR,4S,6aS)-hexahydrocyclopenta[c]pyrrol-(1H)-yle, le (3aR,5r,6aS)-hexahydrocyclopenta[c]pyrrol-2(1H)-yle, le 1,3-dihydro-2H-isoindolyle, l'octahydro-2H-isoindolyle, le (3aS)-1,3,3a,4,5,6-hexahydro-2H-isoindolyle,
le (3aR,4R,7aS)-1H-isoindol-(3H,3aH,4H,5H,6H,7H,7aH)-yle, le (3aR,7aS)-octahydro-2H-isoindolyle, le (3aR,4R,7aS)-octahydro-2H-isoindolyle,
le (3aR,4S,7aS)-octahydro-2H-isoindolyle, le 2,5-diazabicyclo[2.2.1]heptanyl, le 2-azabicyclo[2.2.1]hept-5-enyle, le 3-azabicyclo[3.1.0]hexanyle, le (1R,5S,6s)-3-azabicyclo[3.1.0]hexanyle, le (cis,cis)-3-azabicyclo[3.1.0]hexanyle, le 3,6-diazabicyclo[3.1.0]hexanyle, le (1S,5R,6R)-3-azabicyclo[3.2.0]heptanyle,
le (1S,5R,6S)-3-azabicyclo[3.2.0]heptanyle, le 5-azaspiro[2.4]heptanyle, le 2,6-diazaspiro[3.3]heptanyle, le 2,5-diazaspiro[3.4]octanyle, le 2,6-diazaspiro[3.4]octanyle, le 2,7-diazaspiro[3.5]nonanyle, le 2,7-diazaspiro[4.4]nonanyle, le 2-azaspiro[4.5]décanyle et le 2,8-diazaspiro[4.5]décanyle ;
un hétérocyclyl-amino-C₁₋₈ alkyle, dans lequel l'hétérocyclyle est choisi parmi l'azétidin-1-yle ou la pipéridin-4-yle ;
un (hétérocyclyl, C₁₋₈ alkyl)amino-C₁₋₈ alkyle, dans lequel l'hétérocyclyle est choisi parmi le pipéridin-3-yle ou le pipéridin-4-yle ;
un (hétérocyclyl, C₃₋₁₄ cycloalkyl, C₁₋₈ alkyl)amino-C₁₋₈ alkyle, dans lequel l'hétérocyclyle est choisi parmi le pipéridin-3-yle ou le pipéridin-4-yle ;
un hétérocyclyl-C₁₋₈ alkyl-amino-C₁₋₈ alkyle, dans lequel l'hétérocyclyle est choisi parmi
le pyrrolidin-2-yle, le pipéridin-2-yle, le pipéridin-3-yle, le pipéridin-4-yle ou le tétrahydro-2H-pyran-4-yle ; et
un (hétérocyclyl-oxy-C₁₋₈ alkyl, C₁₋₈ alkyl)-amino choisi parmi les tétrahydro-2H-pyran-2-yl-oxy-C₁₋₈ alkyl, C₁₋₈ alkyl)amino.

5. Composé selon la revendication 1, dans lequel R₁₆ est
un C₃₋₁₄ cycloalkyle, dans lequel le C₃₋₁₄ cycloalkyle est choisi parmi le cyclopropyle ou le cyclobutyle ;
un C₃₋₁₄ cycloalkyl-amino, dans lequel le C₃₋₁₄ cycloalkyle est un cyclopropyle ;
un aryle, dans lequel l'aryle est un phényle ;
un aryl-C₁₋₈ alkyle, dans lequel l'aryle est un phényle ;
un aryl-amino, dans lequel l'aryle est un phényle ;
un (aryl, C₁₋₈ alkyl)amino, dans lequel l'aryle est un phényle ;
un aryl-C₁₋₈ alkyl-amino, dans lequel l'aryle est un phényle ;
un (aryl, C₁₋₈ alkyl, C₁₋₈ alkyl)amino, dans lequel l'aryle est un phényle ;
un (aryl-C₁₋₈ alkyl)₂-amino, dans lequel l'aryle est un phényle ;
un aryl-C₁₋₈ alkyl-amino-C₁₋₈ alkyle, dans lequel l'aryle est un phényle ;
un (aryl-C₁₋₈ alkyl, C₁₋₈ alkyl)amino-C₁₋₈ alkyle, dans lequel l'aryle est un phényle ;
un (aryl-C₁₋₈ alkyl)₂-amino-C₁₋₈ alkyle, dans lequel l'aryle est un phényle ;
un aryl-C₁₋₈ alcoxy, dans lequel l'aryle est un phényle ;
un aryl-C₁₋₈ alcoxy-carbonyl-amino, dans lequel l'aryle est un phényle ;
un hétéroaryle, dans lequel l'hétéroaryle est choisi parmi le pyridin-2-yle, le pyridin-4-yle, le thiazol-2-yle et 1H-1,2,3-triazol-1-yle ;
un hétéroaryl-C₁₋₈ alkyl-amino-C₁₋₈ alkyle, dans lequel l'hétéroaryle est choisi parmi le pyridin-2-yle, le pyridin-3-yle et le pyridin-4-yle ;
un hétérocyclyle, dans lequel l'hétérocyclyle est choisi parmi le pyrrolidin-1-yle et le morpholin-4-yle ;
un hétérocyclyl-C₁₋₈ alkyle, dans lequel l'hétérocyclyle est le pyrrolidin-1-yle ;
un hétérocyclyl-oxy, dans lequel l'hétérocyclyle est le tétrahydro-2H-pyran-2-yl-oxy.

6. Composé selon la revendication 1, dans lequel le composé de formule (I), de formule (II) ou de formule (III) est un composé de formule (Ia), de formule (IIa) ou de formule (IIIa), respectivement : 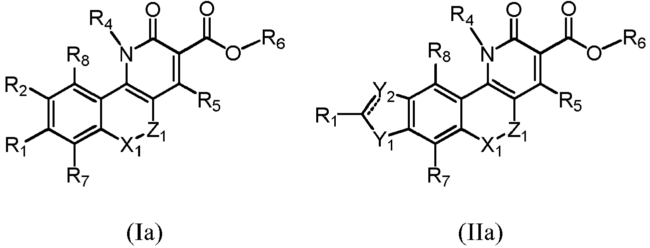 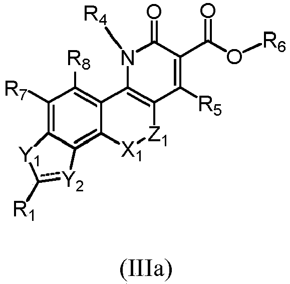 ou d'une de leurs formes, dans lequel
le tiret dans la formule (IIa) et la formule (IIIa) représente une double liaison facultativement présente ;
X₁ est une liaison, N(R₁₄), S, O ou -CH(R₉)- ;
Z₁ est N(R₁₄), S, O, C(O) ou -CH(R₃)- quand X₁ est une liaison ou -CH(R₉)-, Z₁ est-CH(R₃)- quand X₁ est N(R₁₄), S, ou O ; et
toutes les autres variables sont telles que définies précédemment.

7. Composé selon la revendication 1, dans lequel le composé ou l'une de ses formes est choisi parmi :
l'acide 8-(diméthylamino)-2-oxo-1,2,5,6-tétrahydrobenzo[h]quinoline-3-carboxylique,
l'acide 9-(diméthylamino)-2-oxo-2,5,6,7-tétrahydro-1H-benzo[6,7]cyclohepta[1,2-b]pyridine-3-carboxylique,
l'acide 9-(diméthylamino)-4-hydroxy-2-oxo-2,5,6,7-tétrahydro-1H-benzo[6,7]cyclohepta[1,2-b]pyridine-3-carboxylique,
l'acide 10-(diméthylamino)-2-oxo-1,2,5,6,7,8-hexahydrobenzo[7,8]cycloocta[1,2-b]pyridine-3-carboxylique,
l'acide 10-(diméthylamino)-4-hydroxy-2-oxo-1,2,5,6,7,8-hexahydrobenzo[7,8]cycloocta[1,2-b]pyridine-3-carboxylique,
l'acide 4-hydroxy-2-oxo-9-(pyrrolidin-1-yl)-2,5,6,7-tétrahydro-1H-benzo[6,7]cyclohepta[1,2-b]pyridine-3-carboxylique,
l'acide 5-méthyl-2-oxo-8-(pyrrolidin-1-yl)-2,5-dihydro-1H-chroméno[4,3-b]pyridine-3-carboxylique,
l'acide 9-(diméthylamino)-4-hydroxy-2-oxo-1,2,5,6-tétrahydrobenzo[2,3]oxepino[4,5-b]pyridine-3-carboxylique,
l'acide 4-hydroxy-2-oxo-9-(pyrrolidin-1-yl)-1,2,5,6-tétrahydrobenzo[2,3]oxepino[4,5-b]pyridine-3-carboxylique,
l'acide 2-oxo-9-(pyrrolidin-1-yl)-1,2,5,6-tétrahydrobenzo[2,3]oxepino[4,5-b]pyridine-3-carboxylique,
l'acide 4-hydroxy-5-méthyl-2-oxo-9-(pyrrolidin-1-yl)-1,2,5,6-tétrahydrobenzo[2,3]oxepino[4,5-b]pyridine-3-carboxylique,
l'acide 4-hydroxy-7-méthyl-2-oxo-9-(pyrrolidin-1-yl)-2,5,6,7-tétrahydro-1H-benzo[b]pyrido[2,3-d]azépine-3-carboxylique,
l'acide 4-hydroxy-2-oxo-9-(pyrrolidin-1-yl)-2,5,6,7-tétrahydro-1H-benzo[b]pyrido[2,3-d]azépine-3-carboxylique,
l'acide 4-hydroxy-5-méthyl-2-oxo-8-(pyrrolidin-1-yl)-1,2,5,6-tétrahydrobenzo[h]quinoline-3-carboxylique,
l'acide 8-(diméthylamino)-4-hydroxy-5-méthyl-2-oxo-1,2,5,6-tétrahydrobenzo[h]quinoline-3-carboxylique,
l'acide 4-hydroxy-2-oxo-10-(pyrrolidin-1-yl)-1,2,5,6,7,8-hexahydrobenzo[7,8]cycloocta[1,2-b]pyridine-3-carboxylique,
l'acide 4-hydroxy-2-oxo-10-(propylamino)-1,2,5,6,7,8-hexahydrobenzo[7,8]cycloocta[1,2-b]pyridine-3-carboxylique,
l'acide 10-(éthylamino)-4-hydroxy-2-oxo-1,2,5,6,7,8-hexahydrobenzo[7,8]cycloocta[1,2-b]pyridine-3-carboxylique,
l'acide 9-(diméthylamino)-4-hydroxy-2-oxo-1,2,5,6-tétrahydrobenzo[2,3]thiépino[4,5-b]pyridine-3-carboxylique, et
l'acide 4-hydroxy-2-oxo-9-(pyrrolidin-1-yl)-1,2,5,6-tétrahydrobenzo[2,3]thiépino[4,5-b]pyridine-3-carboxylique ;
dans lequel la forme du composé est choisie parmi une forme d'acide libre, de base libre, de sel, d'hydrate, de solvate, de clathrate, d'isotopologue, de racémate, d'énantiomètre, de diastéréomère, de stéréoisomère, polymorphe ou tautomérique de celui-ci.

8. Composé selon la revendication 1, dans lequel le composé ou l'une de ses formes est choisi parmi :
l'hydrochlorure d'acide 9-(3-(diméthylamino)pyrrolidin-1-yl)-4-hydroxy-2-oxo-2,5,6,7-tétrahydro-1H-benzo[6,7]cyclohepta[1,2-b]pyridine-3-carboxylique,
l'hydrochlorure d'acide 4-hydroxy-9-(3-(méthylamino)pyrrolidin-1-yl)-2-oxo-2,5,6,7-tétrahydro-1H-benzo[6,7]cyclohepta[1,2-b]pyridine-3-carboxylique,
le trifluoroacétate d'acide 9-((cis,cis)-6-amino)-3-azabicyclo[3.1.0]hexan-3-yl)-4-hydroxy-2-oxo-2,5,6,7-tétrahydro-1H-benzo[6,7]cyclohepta[1,2-b]pyridine-3-carboxylique,
l'hydrochlorure d'acide 9-((cis,cis)-6-(benzyl(méthyl)amino)-3-azabicyclo[3.1.0]hexan-3-yl)-4-hydroxy-2-oxo-2,5,6,7-tétrahydro-lH-benzo[6,7]cyclohepta[1,2-b]pyridine-3-carboxylique,
l'hydrochlorure d'acide 4-hydroxy-9-((cis,cis)-6-(méthylamino)-3-azabicyclo[3.1.0]hexan-3-yl)-2-oxo-2,5,6,7-tétrahydro-lH-benzo[6,7]cyclohepta[1,2-b]pyridine-3-carboxylique,
l'hydrochlorure d'acide 9-(3-(diméthylamino)pyrrolidin-1-yl)-4-hydroxy-5-méthyl-2-oxo-2,5,6,7-tétrahydro-1H-benzo[6,7]cyclohepta[1,2-b]pyridine-3-carboxylique,
l'hydrochlorure d'acide 9-((cis,cis)-6-amino-3-azabicyclo[3.1.0]hexan-3-yl)-4-hydroxy-5-méthyl-2-oxo-2,5,6,7-tétrahydro-1H-benzo[6,7]cyclohepta[1,2-b]pyridine-3-carboxylique,
le trifluoroacétate d'acide 9-((cis,cis)-6-amino)-3-azabicyclo[3.1.0]hexan-3-yl)-4,7-dihydroxy-2-oxo-2,5,6,7-tétrahydro-1H-benzo[6,7]cyclohepta[1,2-b]pyridine-3-carboxylique,
l'hydrochlorure d'acide 9-(3-(diméthylamino)pyrrolidin-1-yl)-4,7-dihydroxy-2-oxo-2,5,6,7-tétrahydro-1H-benzo[6,7]cyclohepta[1,2-b]pyridine-3-carboxylique,
l'hydrochlorure d'acide 2-((éthylamino)méthyl)-8-hydroxy-1-méthyl-6-oxo-1,5,6,9-tétrahydropyrido[3',2':4,5]cyclopenta[1,2-f]indole-7-carboxylique,
l'hydrochlorure d'acide 4-hydroxy-8-méthyl-9-((méthylamino)méthyl)-2-oxo-2,5,6,8-tétrahydro-1H-indolo[6,5-h]quinoline-3-carboxylique,
l'hydrochlorure d'acide 9-(azétidin-1-ylméthyl)-4-hydroxy-8-méthyl-2-oxo-2,5,6,8-tétrahydro-1H-indolo[6,5-h]quinoline-3-carboxylique,
l'hydrochlorure d'acide 4-hydroxy-9-méthyl-10-((méthylamino)méthyl)-2-oxo-1,2,5,6,7,9-hexahydropyrido[3',2':6,7]cyclohepta[1,2-f]indole-3-carboxylique,
l'hydrochlorure d'acide 10-((cyclobutylamino)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 10(azétidin-1-yl-méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 10-((éthylamino)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-10-((isopropylamino)méthyl)-9-méthyl-2-oxo-1,2,5,6,7,9-hexahydropyrido[3',2':6,7]cyclohepta[1,2-f]indole-3-carboxylique,
l'hydrochlorure d'acide 10-((tert-butylamino)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-10-((4-hydroxypipéridin-1-yl)méthyl)-9-méthyl-2-oxo-1,2,5,6,7,9-hexahydropyrido[3',2':6,7]cyclohepta[1,2-f]indole-3-carboxylique,
le dihydrochlorure d'acide 10-((4-aminopipéridin-1-yl)méthyl)-4-hydroxy-9-méthyl-2-oxo-1,2,5,6,7,9-hexahydropyrido[3',2':6,7]cyclohepta[1,2-f]indole-3-carboxylique,
le dihydrochlorure d'acide 10-((4-(dimétylamino)pipéridin-1-yl)méthyl)-4-hydroxy-9-méthyl-2-oxo-1,2,5,6,7,9-hexahydropyrido[3',2':6,7]cyclohepta[1,2-f]indole-3-carboxylique,
le dihydrochlorure d'acide 10-((3-aminopipéridin-1-yl)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 10-(((cyclopropylméthyl)amino)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-9-méthyl-10-(((1-méthylcyclopropyl)amino)méthyl)-2-oxo-1,2,5,6,7,9-hexahydropyrido[3',2':6,7]cyclohepta[1,2-f]indole-3-carboxylique,
l'hydrochlorure d'acide 10-((benzylamino)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 10-(aminométhyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 10-((cyclopropylamino)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 10-(((cyclobutylmethyl)amino)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-9-méthyl-10-((((2-méthylcyclopropyl)méthyl)amino)méthyl)-2-oxo-1,2,5,6,7,9-hexahydropyrido[3',2':6,7]cyclohepta[1,2-f]indole-3-carboxylique,
l'hydrochlorure d'acide 4-hydroxy-9-méthyl-2-oxo-10-(((pyridin-4-ylméthyl)amino)méthyl)-1,2,5,6,7,9-hexahydropyrido[3',2':6,7]cyclohepta[1,2-f]indo le-3 -carboxylique,
l'hydrochlorure d'acide 4-hydroxy-9-méthyl-2-oxo-10-(pipéridin-1-ylméthyl)-1,2,5,6,7,9-hexahydropyrido[3',2':6,7]cyclohepta[1,2-f]indole-3-carboxylique,
l'hydrochlorure d'acide 4-hydroxy-9-méthyl-10-(morpholinométhyl)-2-oxo-1,2,5,6,7,9-hexahydropyrido[3',2':6,7]cyclohepta[1,2-f]indole-3-carboxylique,
l'hydrochlorure d'acide 4-hydroxy-9-méthyl-10-((4-méthylpipérazin-1-yl)méthyl)-2-oxo-1,2,5,6,7,9-hexahydropyrido[3',2':6,7]cyclohepta[1,2-f]indole-3-carboxylique,
l'hydrochlorure d'acide 10-((diéthylamino)méthyl-4-hydroxy-9-méthyl-2-oxo-1,2,5,6,7,9-hexahydropyrido[3',2'6,7]cyclohepta[1,2-f]indole-3-carboxylique,
l'hydrochlorure d'acide 4-hydroxy-9-méthyl-2-oxo-10-((propylamino)méthyl)-1,2,5,6,7,9-hexahydropyrido[3',2':6,7]cyclohepta[1,2-f]indole-3-carboxylique,
l'hydrochlorure d'acide 4-hydroxy-9-méthyl-2-oxo-10-((prop-2-yl-1-amino)méthyl)-1,2,5,6,7,9-hexahydropyrido[3',2':6,7]cyclohepta[1,2-f]indole-3-carboxylique,
l'hydrochlorure d'acide (R)-10-((3-fluoropyrrolidin-1-yl)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 10-((3-(diméthylamino)pyrrolidin-1-yl)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 10-((diméthylamino)méthyl)-4-hydroxy-9-méthyl-2-oxo-1,2,5,6,7,9-hexahydropyrido[3',2':6,7]cyclohepta[1,2-f]indole-3-carboxylique,
le dihydrochlorure d'acide (S)-10-((3-aminopyrrolidin-1-yl)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-9-méthyl-2-oxo-10-(pyrrolidin-1-ylméthyl)-1,2,5,6,7,9-hexahydropyrido[3',2':6,7]cyclohepta[1,2-f]indole-3-carboxylique,
l'hydrochlorure d'acide 4-hydroxy-10-((3-hydroxypyrrolidin-1-yl)méthyl)-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-9-méthyl-2-oxo-10(((pyridin-3-ylméthyl)amino)méthyl)-1,2,5,6,7,9-hexahydropyrido[3',2':6,7]cyclohepta[1,2-f]indole-3-carboxylique,
l'hydrochlorure d'acide 9-méthyl-10-((méthylamino)méthyl)-2-oxo-1,2,5,6,7,9-hexahydropyrido[3',2':6,7]cyclohepta[1,2-f]indole-3-carboxylique,
l'hydrochlorure d'acide 10-((éthylamino)méthyl)-9-méthyl-2-oxo-1,2,5,6,7,9-hexahydropyrido[3',2':6,7]cyclohepta[1,2-f]indole-3-carboxylique,
l'hydrochlorure d'acide 10-((isopropylamino)méthyl)-9-méthyl-2-oxo-1,2,5,6,7,9-hexahydropyrido[3',2':6,7]cyclohepta[1,2-f]indole-3-carboxylique,
l'hydrochlorure d'acide 10-((tert-butylamino)méthyl)-9-méthyl-2-oxo-1,2,5,6,7,9-hexahydropyrido[3',2':6,7]cyclohepta[1,2-f]indole-3-carboxylique,
l'hydrochlorure d'acide 10-(azétidin-1-ylméthyl)-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-10-méthyl-11-((méthylamino)méthyl)-2-oxo-2,5,6,7,8,10-hexahydro-1H-pyrido[3',2':7,8]cycloocta[1,2-f]indole-3-carboxylique,
l'hydrochlorure d'acide 11-((éthylamino)méthyl)-4-hydroxy-10-méthyl-2-oxo-2,5,6,7,8,10-hexahydro-1H-pyrido[3',2':7,8]cycloocta[1,2-f]indole-3-carboxylique,
l'hydrochlorure d'acide 7-hydroxy-1-méthyl-2-((méthylamino)méthyl)-9-oxo-1,4,5,6,9,10-hexahydropyrido[2',3':3,4]cyclohepta[1,2-e]indole-8-carboxylique,
l'hydrochlorure d'acide 2-((éthylamino)méthyl)-7-hydroxy-1-méthyl-9-oxo-1,4,5,6,9,10-hexahydropyrido[2',3':3,4]cyclohepta[1,2-e]indole-8-carboxylique,
l'hydrochlorure d'acide 4-hydroxy-7,9-diméthyl-10-((méthylamino)méthyl)-2-oxo-1,2,5,6,7,9-hexahydropyrido[3',2':6,7]cyclohepta[1,2-f]indole-3-carboxylique,
l'hydrochlorure d'acide 10-((éthylamino)méthyl)-4-hydroxy-7,9-diméthyl-2-oxo-1,2,5,6,7,9-hexahydropyrido[3',2':6,7]cyclohepta[1,2-f]indole-3-carboxylique,
l'hydrochlorure d'acide 4-hydroxy-10-((isopropylamino)méthyl)-7,9-diméthyl-2-oxo-1,2,5,6,7,9-hexahydropyrido[3',2':6,7]cyclohepta[1,2-f]indole-3-carboxylique,
l'hydrochlorure d'acide 10-((tert-butylamino)méthyl)-4-hydroxy-7,9-diméthyl-2-oxo-1,2,5,6,7,9-hexahydropyrido[3',2':6,7]cyclohepta[1,2-f]indole-3-carboxylique,
l'hydrochlorure d'acide 4-hydroxy-7,9-diméthyl-10-(((1-méthylcyclopropyl)amino)méthyl)-2-oxo-1,2,5,6,7,9-hexahydropyrido[3',2':6,7]cyclohepta[1,2-f]indole-3-carboxylique,
l'hydrochlorure d'acide 10-((éthylamino)méthyl)-4-hydroxy-5,9-diméthyl-2-oxo-1,2,5,6,7,9-hexahydropyrido[3',2':6,7]cyclohepta[1,2-f]indole-3-carboxylique,
l'hydrochlorure d'acide 4-hydroxy-9-méthyl-10-((méthylamino)méthyl)-2-oxo-1,2,5,6,7,9-hexahydropyrido[3',2':6,7]cyclohepta[1,2-f]indole-3-carboxylique,
l'hydrochlorure d'acide 10-((éthylamino)méthyl)-4-hydroxy-9-méthyl-2-oxo-1,2,5,9-tétrahydropyrido[3',2':6,7]cyclohepta[1,2-f]indole-3-carboxylique,
l'hydrochlorure d'acide 10-((éthylamino)méthyl)-4-hydroxy-7,9-diméthyl-2-oxo-1,2,5,9-tétrahydropyrido[3',2':6,7]cyclohepta[1,2-f]indole-3-carboxylique,
l'hydrochlorure d'acide (cis)-10-((éthylamino)méthyl)-4,6,7-trihydroxy-9-méthyl-2-oxo-1,2,5,6,7,9-hexahydropyrido[3',2':6,7]cyclohepta[1,2-f]indole-3-carboxylique,
l'hydrochlorure d'acide 8-(3-(diméthylamino)pyrrolidin-1-yl)-5-méthyl-2-oxo-2,5-dihydro-1H-chromeno[4,3-b]pyridine-3-carboxylique,
l'hydrochlorure d'acide 9-(3-(diméthylamino)pyrrolidin-1-yl)-4-hydroxy-2-oxo-1,2,5,6-tétrahydrobenzo[2,3]oxepino[4,5-b]pyridine-3-carboxylique,
le trifluoroacétate d'acide 9-((cis,cis)-6-(dibenzylamino)-3-azabicyclo[3.1.0]hexan-3-yl)-4-hydroxy-2-oxo-1,2,5,6-tétrahydrobenzo[2,3]oxepino[4,5-b]pyridine-3-carboxylique,
l'hydrochlorure d'acide 9-((cis,cis)-6-amino-3-azabicyclo[3.1.0]hexan-3-yl)-4-hydroxy-2-oxo-1,2,5,6-tétrahydrobenzo[2,3]oxepino[4,5-b]pyridine-3-carboxylique,
l'hydrochlorure d'acide 9-(3-(diméthylamino)pyrrolidin-1-yl)-2-oxo-1,2,5,6-tétrahydrobenzo[2,3]oxepino[4,5-b]pyridine-3-carboxylique,
l'hydrochlorure d'acide 9-(3-(diméthylamino)pyrrolidin-1-yl)-4-hydroxy-5-méthyl-2-oxo-1,2,5,6-tétrahydrobenzo[2,3]oxepino[4,5-b]pyridine-3-carboxylique,
l'hydrochlorure d'acide 9-((cis,cis)-6-(dibenzylamino)-3-azabicyclo[3.1.0]hexan-3-yl)-4-hydroxy-5-méthyl-2-oxo-1,2,5,6-tétrahydrobenzo[2,3]oxepino[4,5-b]pyridine-3-carboxylique,
l'hydrochlorure d'acide 9-((cis,cis)-6-amino-3-azabicyclo[3.1.0]hexan-3-yl)-4-hydroxy-5-méthyl-2-oxo-1,2,5,6-tétrahydrobenzo[2,3]oxepino[4,5-b]pyridine-3-carboxylique,
l'hydrochlorure d'acide 4-hydroxy-9-méthyl-10-((méthylamino)méthyl)-2-oxo-2,5,6,9-tétrahydro-1H-pyrido[2',3':4,5]oxepino[3,2-f]indole-3-carboxylique,
l'hydrochlorure d'acide 10-((éthylamino)méthyl)-4-hydroxy-9-méthyl-2-oxo-2,5,6,9-tétrahydro-1H-pyrido[2',3':4,5]oxepino[3,2-f]indole-3-carboxylique,
l'hydrochlorure d'acide 4-hydroxy-10-((isopropylamino)méthyl)-9-méthyl-2-oxo-2,5,6,9-tétrahydro-1H-pyrido[2',3':4,5]oxepino[3,2-f]indole-3-carboxylique,
l'hydrochlorure d'acide 10-(azétidin-1-ylméthyl)-4-hydroxy-9-méthyl-2-oxo-2,5,6,9-tétrahydro-1H-pyrido[2',3':4,5]oxepino[3,2-f]indole-3-carboxylique,
l'hydrochlorure d'acide 10-((éthylamino)méthyl)-4-hydroxy-5,9-diméthyl-2-oxo-2,5,6,9-tétrahydro-1H-pyrido[2',3':4,5]oxepino[3,2-f]indole-3-carboxylique,
l'hydrochlorure d'acide 9-(3-(diméthylamino)pyrrolidin-1-yl)-4-hydroxy-2-oxo-2,5,6,7-tétrahydro-1H-benzo[b]pyrido[2,3-d]azépine-3-carboxylique,
l'hydrochlorure d'acide 4-hydroxy-9-(4-méthylpipérazin-1-yl)-2-oxo-2,5,6,7-tétrahydro-1H-benzo[b]pyrido[2,3-d]azépine-3-carboxylique,
l'hydrochlorure d'acide 9-((cis,cis)-6-amino-3-azabicyclo[3.1.0]hexan-3-yl)-4-hydroxy-2-oxo-2,5,6,7-tétrahydro-1H-benzo[b]pyrido[2,3-d]azépine-3-carboxylique,
l'hydrochlorure d'acide 9-(hexahydro-1H-pyrrolo[3,4-b]pyridin-6(2H)-yl)-4-hydroxy-2-oxo-2,5,6,7-tétrahydro-1H-benzo[b]pyrido[2,3-d]azépine-3-carboxylique,
l'hydrochlorure d'acide 4-hydroxy-9-méthyl-10-((méthylamino)méthyl)-2-oxo-1,2,5,6,7,9-hexahydropyrido[2',3':4,5]azepino[3,2-f]indole-3-carboxylique,
l'hydrochlorure d'acide 10-((éthylamino)méthyl)-4-hydroxy-9-méthyl-2-oxo-1,2,5,6,7,9-hexahydropyrido[2',3':4,5]azepino[3,2-f]indole-3-carboxylique,
l'hydrochlorure d'acide 4-hydroxy-10-((isopropylamino)méthyl)-9-méthyl-2-oxo-1,2,5,6,7,9-hexahydropyrido[2',3':4,5]azepino[3,2-f]indole-3-carboxylique,
l'hydrochlorure d'acide 10-((tert-butylamino)méthyl)-4-hydroxy-9-méthyl-2-oxo-1,2,5,6,7,9-hexahydropyrido[2',3':4,5]azepino[3,2-f]indole-3-carboxylique,
l'hydrochlorure d'acide 4-hydroxy-10-méthyl-11-((méthylamino)méthyl)-2-oxo-2,5,6,7,8,10-hexahydro-1H-pyrido[2',3':4,5]azocino[3,2-f]indole-3-carboxylique,
l'hydrochlorure d'acide 11-((éthylamino)méthyl)-4-hydroxy-10-méthyl-2-oxo-2,5,6,7,8,10-hexahydro-1H-pyrido[2',3':4,5]azocino[3,2-f]indole-3-carboxylique,
l'hydrochlorure d'acide 4-hydroxy-11-((isopropylamino)méthyl)-10-méthyl-2-oxo-2,5,6,7,8,10-hexahydro-1H-pyrido[2',3':4,5]azocino[3,2-f]indole-3-carboxylique,
l'hydrochlorure d'acide 8-(3-diméthylamino)pyrrolidin-1-yl)-4-hydroxy-5-méthyl-2-oxo-1,2,5,6-tétrahydrobenzo[h]quinoline-3-carboxylique,
le trifluoroacétate d'acide 8-((cis,cis)-6-amino-3-azabicyclo[3.1.0]hexan-3-yl)-4-hydroxy-5-méthyl-2-oxo-1,2,5,6-tétrahydrobenzo[h]quinoline-3-carboxylique,
l'hydrochlorure d'acide 9-(1,4-diazépan-1-yl)-4-hydroxy-2-oxo-2,5,6,7-tétrahydro-1H-benzo[6,7]cyclohepta[1,2-b]pyridine-3-carboxylique,
l'hydrochlorure d'acide 4-hydroxy-9-(4-méthyl-1,4-diazépan-1-yl)-2-oxo-2,5,6,7-tétrahydro-1H-benzo[6,7]cyclohepta[1,2-b]pyridine-3-carboxylique,
l'hydrochlorure d'acide 9-((2-(diméthylamino)éthyl)amino)-4-hydroxy-2-oxo-2,5,6,7-tétrahydro-1H-benzo[6,7]cyclohepta[1,2-b]pyridine-3-carboxylique,
l'hydrochlorure d'acide 9-((4aR,7aR)-hexahydro-1H-pyrrolo[3,4-b]pyridin-6(2H)-yl)-4-hydroxy-2-oxo-2,5,6,7-tétrahydro-1H-benzo[6,7]cyclohepta[1,2-b]pyridine-3-carboxylique,
l'hydrochlorure d'acide 4-hydroxy-9-((4aR,7aR)-1-méthylhexahydro-1H-pyrrolo[3,4-b]pyridin-6(2H)-yl)-2-oxo-2,5,6,7-tétrahydro-1H-benzo[6,7]cyclohepta[1,2-b]pyridine-3-carboxylique,
l'hydrochlorure d'acide 9-(4-(diméthylamino)pipéridin-1-yl)-4-hydroxy-2-oxo-2,5,6,7-tétrahydro-1H-benzo[6,7]cyclohepta[1,2-b]pyridine-3-carboxylique,
l'hydrochlorure d'acide 9-(3-(diméthylamino)pipéridin-1-yl)-4-hydroxy-2-oxo-2,5,6,7-tétrahydro-1H-benzo[6,7]cyclohepta[1,2-b]pyridine-3-carboxylique,
l'hydrochlorure d'acide 4-hydroxy-2-oxo-9-(pipéridin-4-ylamino)-2,5,6,7-tétrahydro-1H-benzo[6,7]cyclohepta[1,2-b]pyridine-3-carboxylique,
l'hydrochlorure d'acide 4-hydroxy-2-oxo-9-(pipérazin-1-yl)-2,5,6,7-tétrahydro-1H-benzo[6,7]cyclohepta[1,2-b]pyridine-3-carboxylique,
l'hydrochlorure d'acide 4-hydroxy-9-(4-méthylpipérazin-1-yl)-2-oxo-2,5,6,7-tétrahydro-1H-benzo[6,7]cyclohepta[1,2-b]pyridine-3-carboxylique,
l'hydrochlorure d'acide 4-hydroxy-2-oxo-9-(2,6-diazaspiro[3,4]octan-6-yl)-2,5,6,7-tétrahydro-1H-benzo[6,7]cyclohepta[1,2-b]pyridine-3-carboxylique,
l'hydrochlorure d'acide 4-hydroxy-2-oxo-9-(2,7-diazaspiro[4,4]nonan-2-yl)-2,5,6,7-tétrahydro-1H-benzo[6,7]cyclohepta[1,2-b]pyridine-3-carboxylique,
l'hydrochlorure d'acide 4-hydroxy-9-(7-méthyl-2,7-diazaspiro[4,4]nonan-2-yl)-2-oxo-2,5,6,7-tétrahydro-1H-benzo[6,7]cyclohepta[1,2-b]pyridine-3-carboxylique,
l'hydrochlorure d'acide 9-((cis,cis)-6-amino-3-azabicyclo[3.1.0]hexan-3-yl)-10,11-difluoro-4-hydroxy-2-oxo-2,5,6,7-tétrahydro-1H-benzo[6,7]cyclohepta[1,2-b]pyridine-3-carboxylique,
l'hydrochlorure d'acide 9-((cis,cis)-6-amino-3-azabicyclo[3.1.0]hexan-3-yl)-11-fluoro-4-hydroxy-2-oxo-2,5,6,7-tétrahydro-1H-benzo[6,7]cyclohepta[1,2-b]pyridine-3-carboxylique,
l'hydrochlorure d'acide 10-((cis,cis)-6-amino-3-azabicyclo[3.1.0]hexan-3-yl)-4-hydroxy-2-oxo-1,2,5,6,7,8-hexahydrobenzo[7,8]cycloocta[1,2-b]pyridine-3-carboxylique,
l'hydrochlorure d'acide 10-((butylamino)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-9-méthyl-2-oxo-10-((pentylamino)méthyl)-1,2,5,6,7,9-hexahydropyrido[3',2':6,7]cyclohepta[1,2-f]indole-3-carboxylique,
l'hydrochlorure d'acide 10-((hexylamino)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 10-((heptylamino)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-9-méthyl-10-((octylamino)méthyl)-2-oxo-1,2,5,6,7,9-hexahydropyrido[3',2':6,7]cyclohepta[1,2-f]indole-3-carboxylique,
l'hydrochlorure d'acide 4-hydroxy-9-méthyl-10-((nonylamino)méthyl)-2-oxo-1,2,5,6,7,9-hexahydropyrido[3',2':6,7] cyclohepta[1,2-f]indole-3-carboxylique,
le dihydrochlorure d'acide 10-(((2-(diméthylamino)éthyl)amino)méthyl)-4-hydroxy-9-méthyl-2-oxo-1,2,5,6,7,9-hexahydropyrido[3',2':6,7]cyclohepta[1,2-f]indole-3-carboxylique,
le dihydrochlorure d'acide 4-hydroxy-9-méthyl-10-(((2-(méthylamino)éthyl)amino)méthyl)-2-oxo-1,2,5,6,7,9-hexahydropyrido[3',2':6,7]cyclohepta[1,2-f]indole-3-carboxylique,
le dihydrochlorure d'acide 10-(((2-aminoéthyl)amino)méthyl)-4-hydroxy-9-méthyl-2-oxo-1,2,5,6,7,9-hexahydropyrido[3',2':6,7]cyclohepta[1,2-f]indole-3-carboxylique,
le dihydrochlorure d'acide 10-(((2-(diméthylamino)éthyl)(méthyl)amino)méthyl)-4-hydroxy-9-méthyl-2-oxo-1,2,5,6,7,9-hexahydropyrido[3',2':6,7]cyclohepta[1,2-b]indole-3-carboxylique,
l'hydrochlorure d'acide 10-((sec-butylamino)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-10-(((hydroxyéthyl)amino)méthyl)-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-10-(((2-méthoxyéthyl)amino)méthyl)-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-10-(((2-hydroxyéthyl)(méthyl)amino)méthyl)-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-10-(((1-méthoxypropan-2-yl)amino)méthyl)-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-10-(((1-hydroxypropan-2-yl)amino)méthyl)-9-méthyl-2-oxo-1,2,5,6,7,9-hexahydropyrido[3',2':6,7]cyclohepta[1,2-f]indole-3-carboxylique,
le dihydrochlorure d'acide 4-hydroxy-9-méthyl-2-oxo-10-(((2-(pyrrolidin-1-yl)éthyl)amino)méthyl)-1,2,5,6,7,9-hexahydropyrido[3',2':6,7]cyclohepta[1,2-f]indole-3-carboxylique,
l'hydrochlorure d'acide 10-((allylamino)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-10-((isobutylamino)méthyl)-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-9-méthyl-10-((neopentylamino)méthyl)-2-oxo-1,2,5,6,7,9-hexahydropyrido[3',2':6,7]cyclohepta[1,2-f]indole-3-carboxylique,
le dihydrochlorure d'acide 4-hydroxy-9-méthyl-10-((4-méthyl-1,4-diazépan-1-yl)-2-oxo-1,2,5,6,7,9-hexahydropyrido[3',2':6,7]cyclohepta[1,2-f]indole-3-carboxylique,
le dihydrochlorure d'acide 4-hydroxy-9-méthyl-10-(((1-méthylpipéridin-4-yl)amino)méthyl)-2-oxo-1,2,5,6,7,9-hexahydropyrido[3',2':6,7]cyclohepta[1,2-f]indo le-3 -carboxylique,
l'hydrochlorure d'acide 4-hydroxy-10-((3-méthoxypyrrolidin-1-yl)méthyl)-9-méthyl-2-oxo-1,2,5,6,7,9-hexahydropyrido[3',2':6,7]cyclohepta[1,2-f]indole-3-carboxylique,
l'hydrochlorure d'acide 10-((3-acétamidopyrrolidin-1-yl)méthyl)-4-hydroxy-9-méthyl-2-oxo-1,2,5,6,7,9-hexahydropyrido[3',2':6,7]cyclohepta[1,2-f]indole-3-carboxylique,
le dihydrochlorure d'acide 10-(((1-(diméthylamino)propan-2-yl)amino)méthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-9-méthyl-2-oxo-10-((((tétrahydrofuran-2-yl)méthyl)amino)méthyl)-1,2,5,6,7,9-hexahydropyrido[3',2':6,7]cyclohepta[1,2-f]indole-3-carboxylique,
le dihydrochlorure d'acide 4-hydroxy-9-méthyl-10-((1-méthylhexahydropyrrolo[3,4-b]pyrrol-5(1H)-yl)méthyl)-2-oxo-1,2,5,6,7,9-hexahydropyrido[3',2':6,7]cyclohepta[1,2-f]indole-3-carboxylique,
l'hydrochlorure d'acide 10-(((2-(diméthylamino)-2-oxoéthyl)amino)méthyl)-4-hydroxy-9-méthyl-2-oxo-1,2,5,6,7,9-hexahydropyrido[3',2':6,7]cyclohepta[1,2-f]indole-3-carboxylique,
le trifluoracétate d'acide 10-(2-(éthylamino)éthyl)-4-hydroxy-9-méthyl-2-oxo-1,2,5,9-tétrahydropyrido[3',2':6,7]cyclohepta[1,2-f]indole-3-carboxylique,
le trifluoracétate d'acide 10-(2-(éthylamino)éthyl)-4-hydroxy-9-méthyl-2-oxo-1,2,5,6,7,9-hexahydropyrido[3',2':6,7]cyclohepta[1,2-f]indole-3-carboxylique,
l'hydrochlorure d'acide 4-hydroxy-9-(((cis)-octahydrocyclopenta[c]pyrrol-4-yl)-(cis)-amino)-2-oxo-1,2,5,6-tétrahydrobenzo[2,3]oxepino[4,5-b]pyridine-3-carboxylique,
l'hydrochlorure d'acide 4-hydroxy-9-méthyl-10-(((1-méthylcyclopropyl)amino)méthyl)-2-oxo-2,5,6,9-tétrahydro-1H-pyrido[2',3':4,5]oxepino[3,2-f]indole-3-carboxylique,
l'hydrochlorure d'acide 10-((sec-butylamino)méthyl)-4-hydroxy-9-méthyl-2-oxo-2,5,6,9-tétrahydro-1H-pyrido[2',3':4,5]oxepino[3,2-f]indole-3-carboxylique,
l'hydrochlorure d'acide 4-hydroxy-9-méthyl-2-oxo-10-((propylamino)méthyl)-2,5,6,9-tétrahydro-1H-pyrido[2',3':4,5]oxepino[3,2-f]indole-3-carboxylique,
l'hydrochlorure d'acide 10-(((2,4-diméthoxybenzyl)amino)méthyl)-4-hydroxy-9-méthyl-2-oxo-2,5,6,9-tétrahydro-1H-pyrido[2',3':4,5]oxepino[3,2-f]indole-3-carboxylique,
l'hydrochlorure d'acide 10-((cyclopropylamino)méthyl)-4-hydroxy-9-méthyl-2-oxo-2,5,6,9-tétrahydro-1H-pyrido[2',3':4,5]oxepino[3,2-f]indole-3-carboxylique,
l'hydrochlorure d'acide 10-((cyclobutylamino)méthyl)-4-hydroxy-9-méthyl-2-oxo-2,5,6,9-tétrahydro-1H-pyrido[2',3':4,5]oxepino[3,2-f]indole-3-carboxylique,
l'hydrochlorure d'acide 10-((diméthylamino)méthyl)-4-hydroxy-9-méthyl-2-oxo-2,5,6,9-tétrahydro-1H-pyrido[2',3':4,5]oxepino[3,2-f]indole-3-carboxylique,
l'hydrochlorure d'acide 4-hydroxy-9-méthyl-2-oxo-10-(pyrrolidin-1-ylméthyl)-2,5,6,9-tétrahydro-1H-pyrido[2',3':4,5]oxepino[3,2-f]indole-3-carboxylique,
l'hydrochlorure d'acide 10-((tert-butylamino)méthyl)-4-hydroxy-9-méthyl-2-oxo-2,5,6,9-tétrahydro-1H-pyrido[2',3':4,5]oxepino[3,2-f]indole-3-carboxylique,
le dihydrochlorure d'acide 4-hydroxy-9-méthyl-10-(4-méthylpipérazin-1-yl)méthyl)-2-oxo-2,5,6,9-tétrahydro-1H-pyrido[2',3':4,5]oxepino[3,2-f]indole-3-carboxylique,
l'hydrochlorure d'acide 4-hydroxy-10-(((2-hydroxyéthyl)amino)méthyl)-9-méthyl-2-oxo-2,5,6,9-tétrahydro-1H-pyrido[2',3':4,5]oxepino[3,2-f]indole-3-carboxylique,
l'hydrochlorure d'acide 4-hydroxy-10-(((1-hydroxypropan-2-yl)amino)méthyl)-9-méthyl-2-oxo-2,5,6,9-tétrahydro-1H-pyrido[2',3':4,5]oxepino[3,2-f]indole-3-carboxylique,
le dihydrochlorure d'acide 4-hydroxy-9-méthyl-2-oxo-10-(((2-pyrrolidin-1-yl)éthyl)amino)méthyl)-2,5,6,9-tétrahydro-1H-pyrido[2',3':4,5]oxepino[3,2-f]indole-3-carboxylique,
le dihydrochlorure d'acide 10-(((2-aminoéthyl)amino)méthyl)-4-hydroxy-9-méthyl-2-oxo-2,5,6,9-tétrahydro-1H-pyrido[2',3':4,5]oxepino[3,2-f]indole-3-carboxylique,
le dihydrochlorure d'acide 4-hydroxy-9-méthyl-10-(((2-(méthylaminol)éthyl)amino)méthyl)-2-oxo-2,5,6,9-tétrahydro-1H-pyrido[2',3':4,5]oxepino[3,2-f]indole-3-carboxylique,
le dihydrochlorure d'acide 10-(((2-(diméthylaminol)éthyl)amino)méthyl)-4-hydroxy-9-méthyl-2-oxo-2,5,6,9-tétrahydro-1H-pyrido[2',3':4,5]oxepino[3,2-f]indole-3-carboxylique,
l'hydrochlorure d'acide 4-hydroxy-10-(((2-(méthoxyéthyl)aminol)méthyl)-9-méthyl-2-oxo-2,5,6,9-tétrahydro-1H-pyrido[2',3':4,5]oxepino[3,2-f]indole-3-carboxylique,
l'hydrochlorure d'acide 4-hydroxy-10-(((1-(méthoxypropan-2-yl)amino)méthyl)-9-méthyl-2-oxo-2,5,6,9-tétrahydro-1H-pyrido[2',3':4,5]oxepino[3,2-f]indole-3-carboxylique,
dans lequel la forme du composé est choisie parmi une forme d'acide libre, de base libre, d'hydrate, de solvate, de clathrate, d'isotopologue, de racémate, d'énantiomère, de diastéréomère, de stéréoisomère, polymorphe ou tautomérique de celui-ci.

9. Composé selon la revendication 1, destiné à être utilisé pour le traitement d'une infection bactérienne.

10. Composé destiné à être utilisé selon la revendication 9, dans lequel l'infection bactérienne résulte d'une bactérie multirésistante de type gram-négative ou gram-positive.

11. Composé destiné à être utilisé selon la revendication 9, dans lequel le traitement est un traitement combiné comprenant un quantité efficace d'un composé selon la revendication 1 ou d'une forme de celui-ci et une quantité efficace d'un antibiotique ou d'un agent antibactérien.

12. Composition pharmaceutique comprenant une quantité efficace d'un composé selon la revendication 1 ou une forme de celui-ci mélangé à un excipient acceptable en pharmacie.

13. Composé selon la revendication 1, destiné à être utilisé en tant que médicament.
